# EUROPEAN PATENT APPLICATION

(11) **EP 3 219 308 A1**
(43) Date of publication of application: **20.09.2017**
(21) Application number: 17169667.7
(22) Date of filing: 05.10.2011
(51) Int. Cl.: A61K 9/14, A61K 9/16, A61K 9/20, A61K 31/437, A61K 31/496, A61K 31/5377, A61K 31/541, A61K 47/02, A61K 47/22, A61K 47/26, A61K 47/32

(54) **MELT-EXTRUDED SOLID DISPERSIONS CONTAINING AN APOPTOSIS-INDUCING AGENT**

(30) Priority: 29.10.2010 US 408527 P
(62) Divisional of application: 11770975.8
(71) Applicant: AbbVie Ireland Unlimited Company, Dublin 2 (IE)
(72) Inventor: RÖSCH, Esther, 76185 Karlsruhe (DE); HOELIG, Peter, 63607 Waechtersbach (DE); LINDLEY, David J., Bristol, WI 55184-5922 (US); SANZGIRI, Yeshwant D., Gurnee, IL Illinois 60031 (US); TONG, Ping, Libertyville, IL Illinois 60048 (US)
(74) Representative: Modiano, Micaela Nadia

(57) **Abstract**

A pro-apoptotic solid dispersion comprises, in essentially non-crystalline form, a Bcl-2 family protein inhibitory compound of Formula I as defined herein, dispersed in a solid matrix that comprises (a) a pharmaceutically acceptable water-soluble polymeric carrier and (b) a pharmaceutically acceptable surfactant. A process for preparing such a solid dispersion comprises subjecting to elevated temperature the compound of Formula I, the water-soluble polymeric carrier and the surfactant, to provide an extrudable semi-solid mixture; extruding the semi-solid mixture; and cooling the resulting extrudate to to provide a solid matrix comprising the polymeric carrier and the surfactant and having the compound dispersed in essentially non-crystalline form therein. The solid dispersion is suitable for oral administration to a subject in need thereof for treatment of a disease characterized by overexpression of one or more anti-apoptotic Bcl-2 family proteins, for example cancer or an immune or autoimmune disease.

## Description

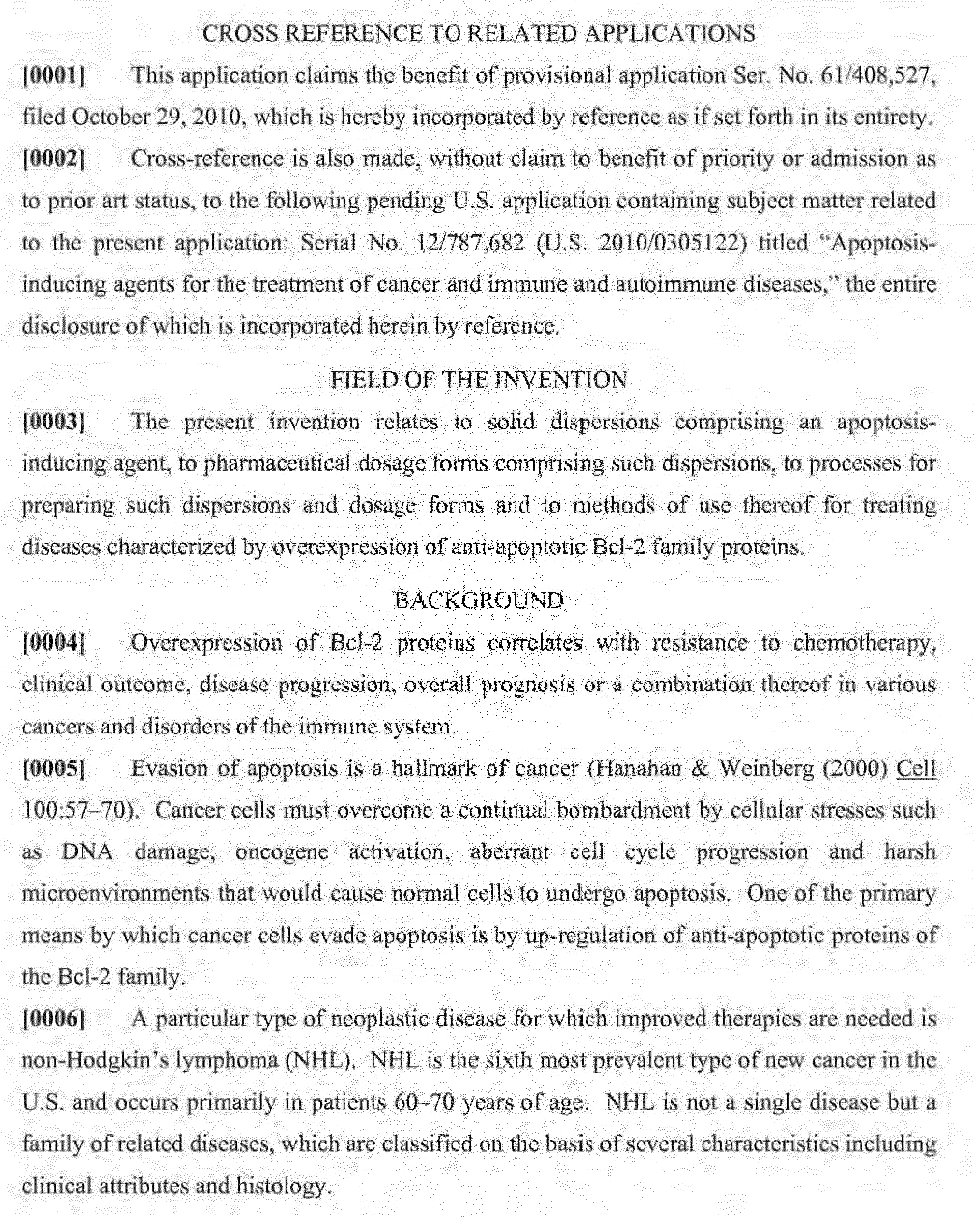

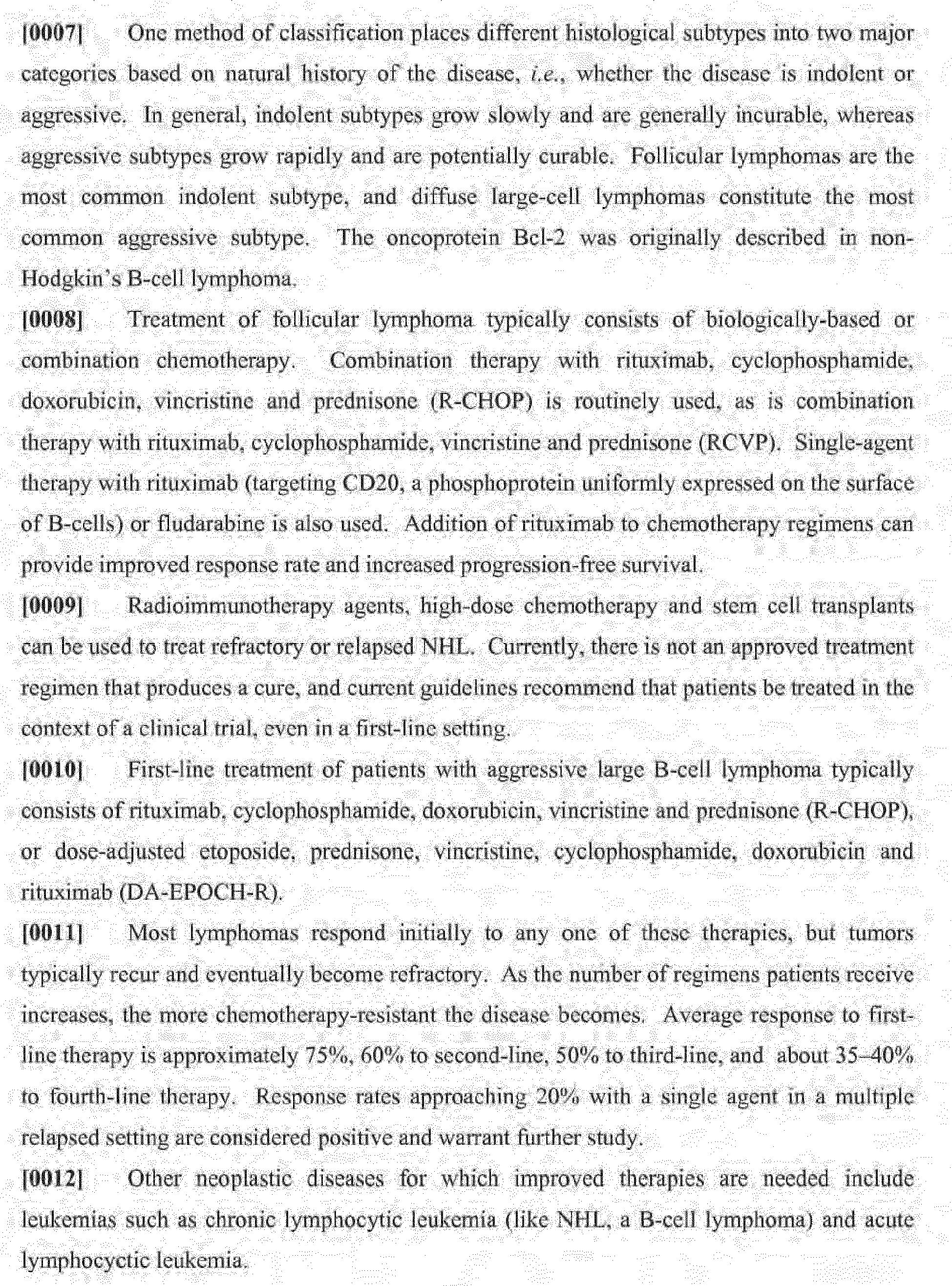

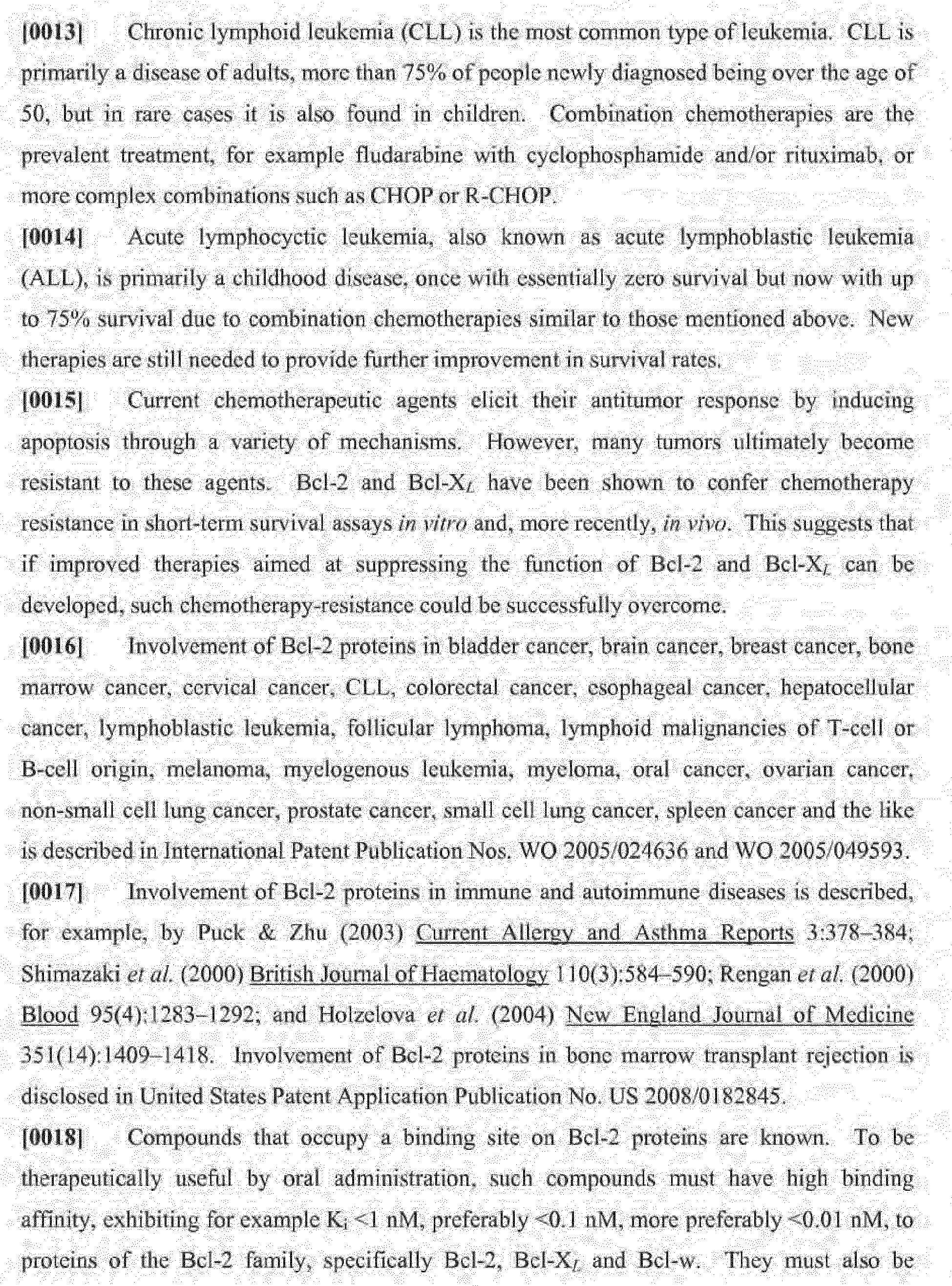

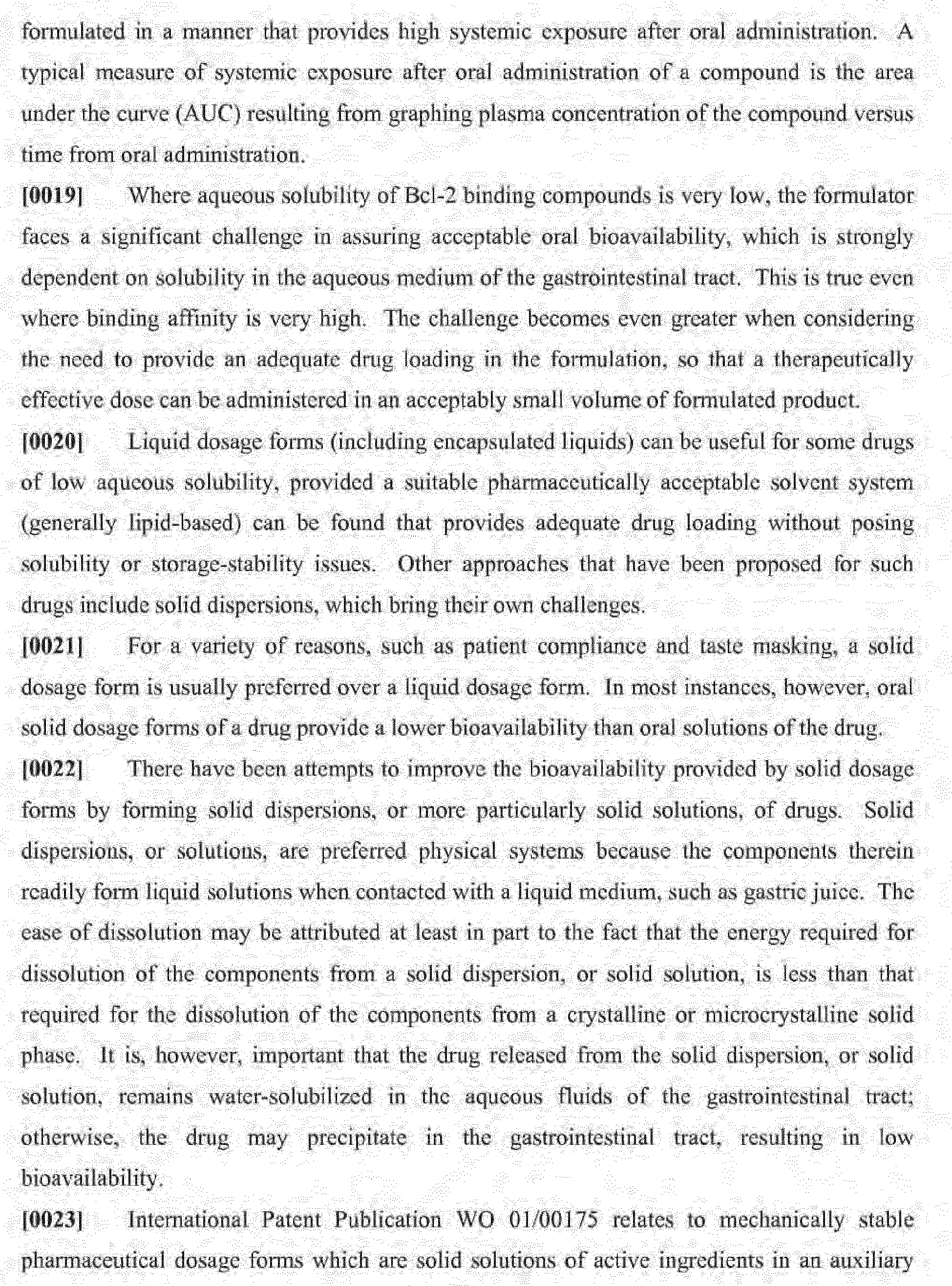

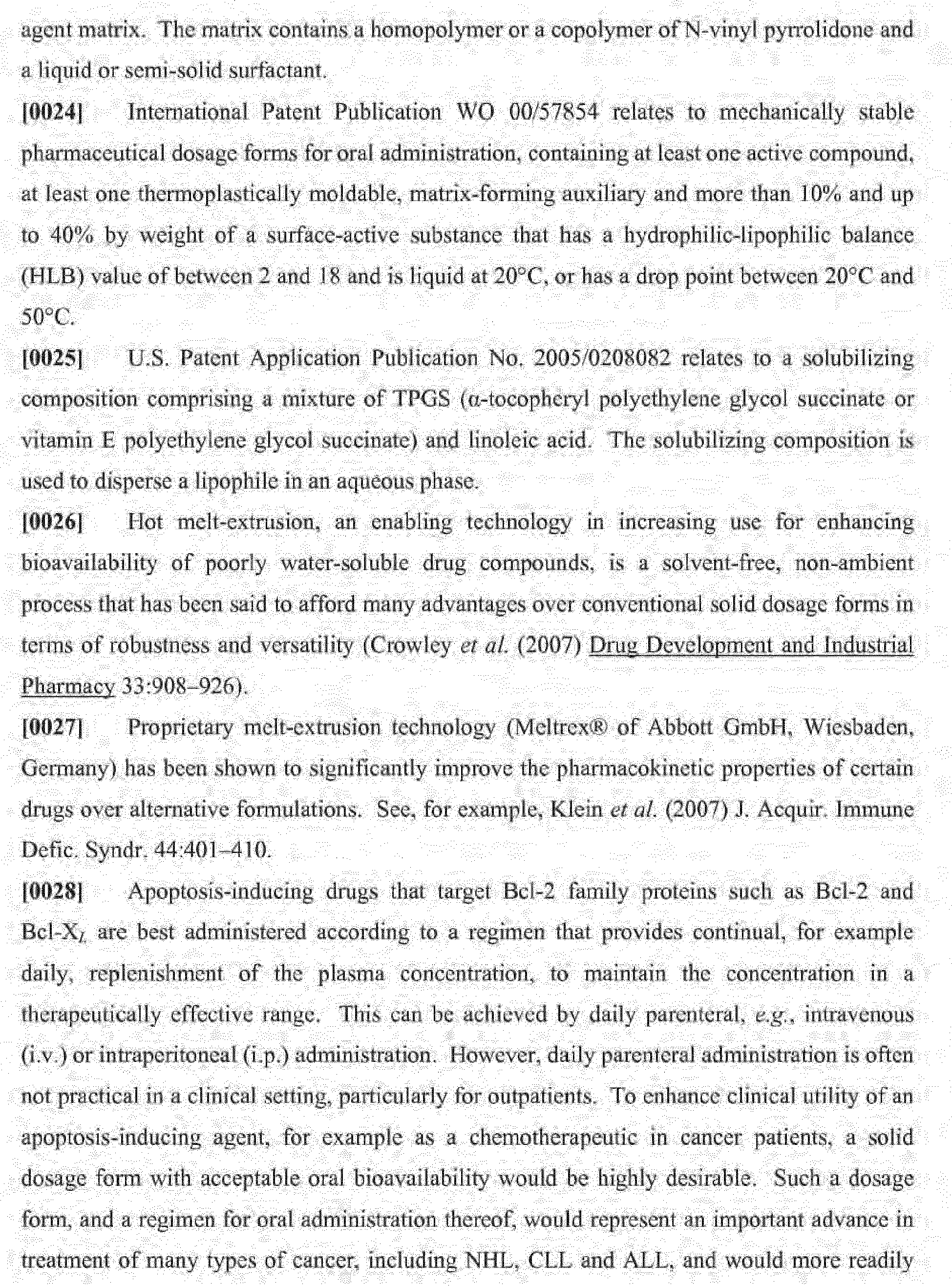

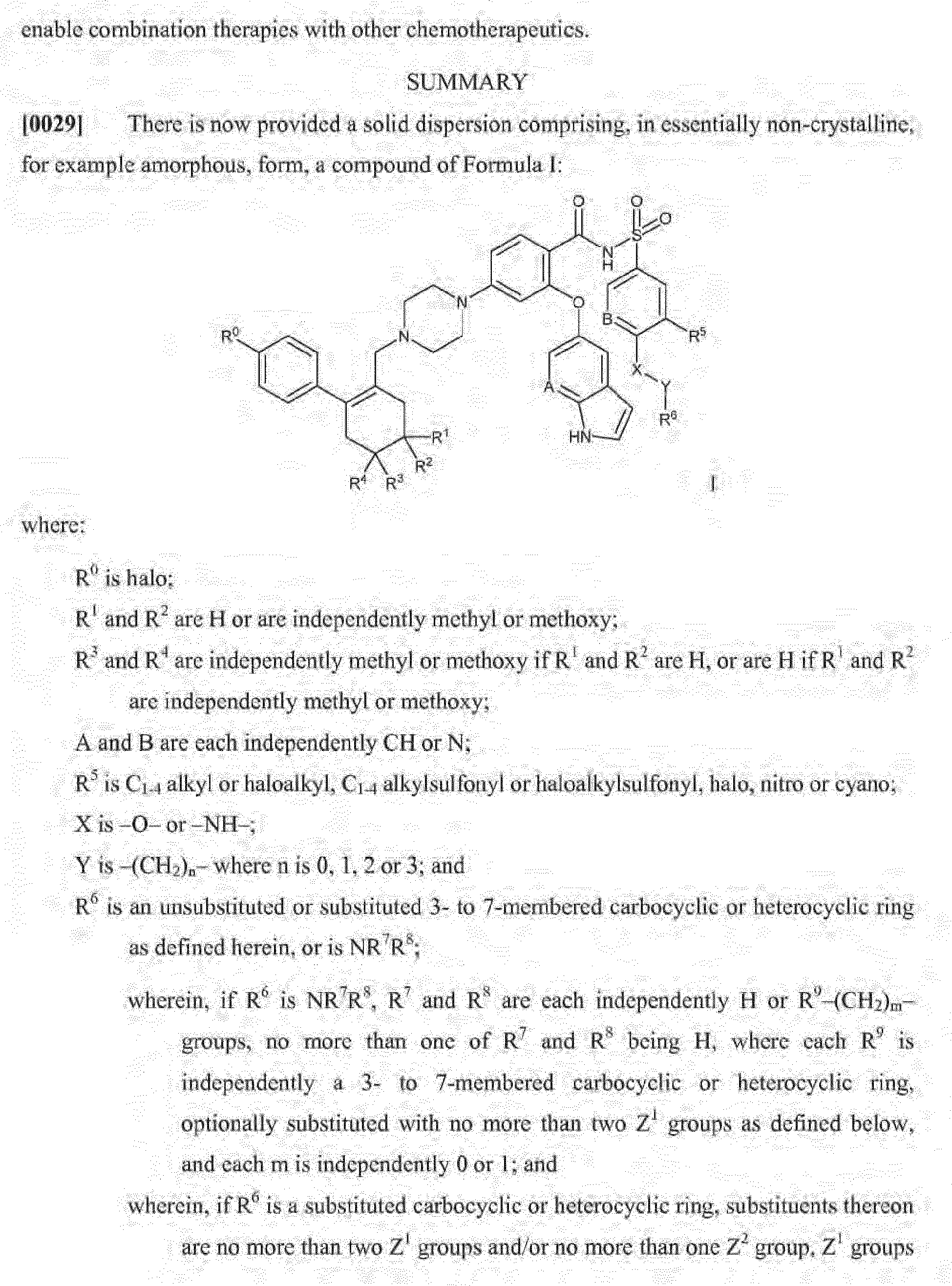

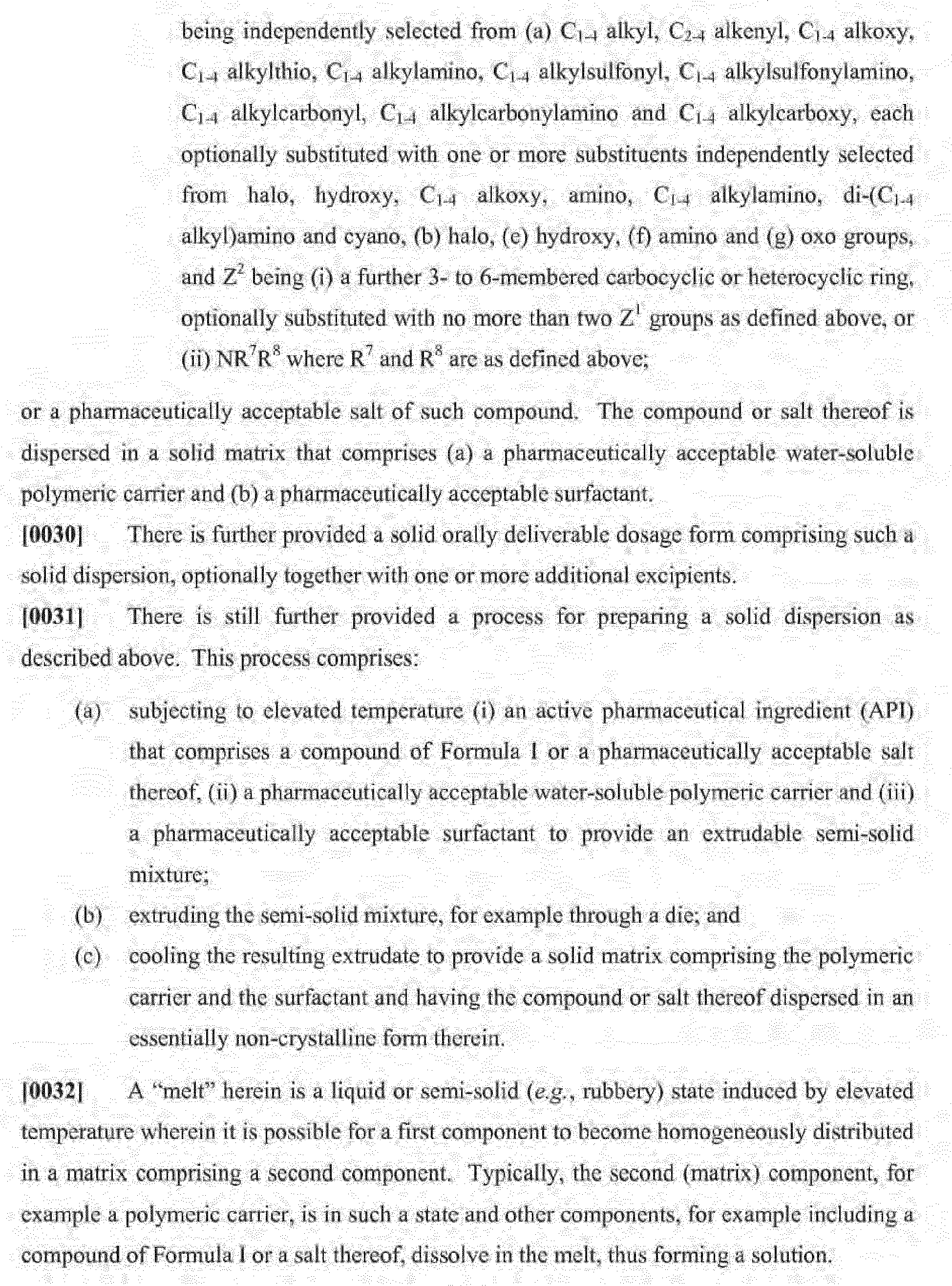

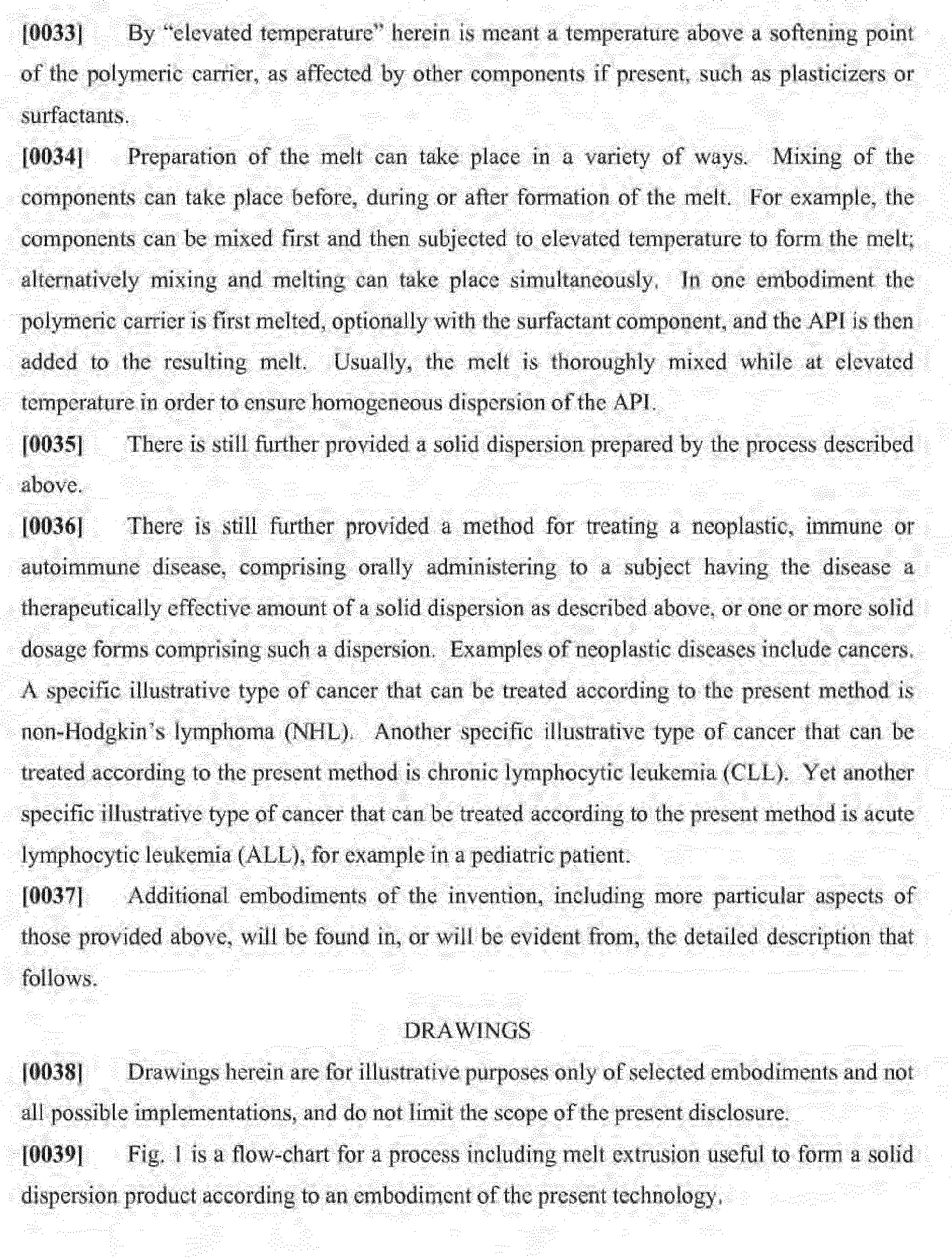

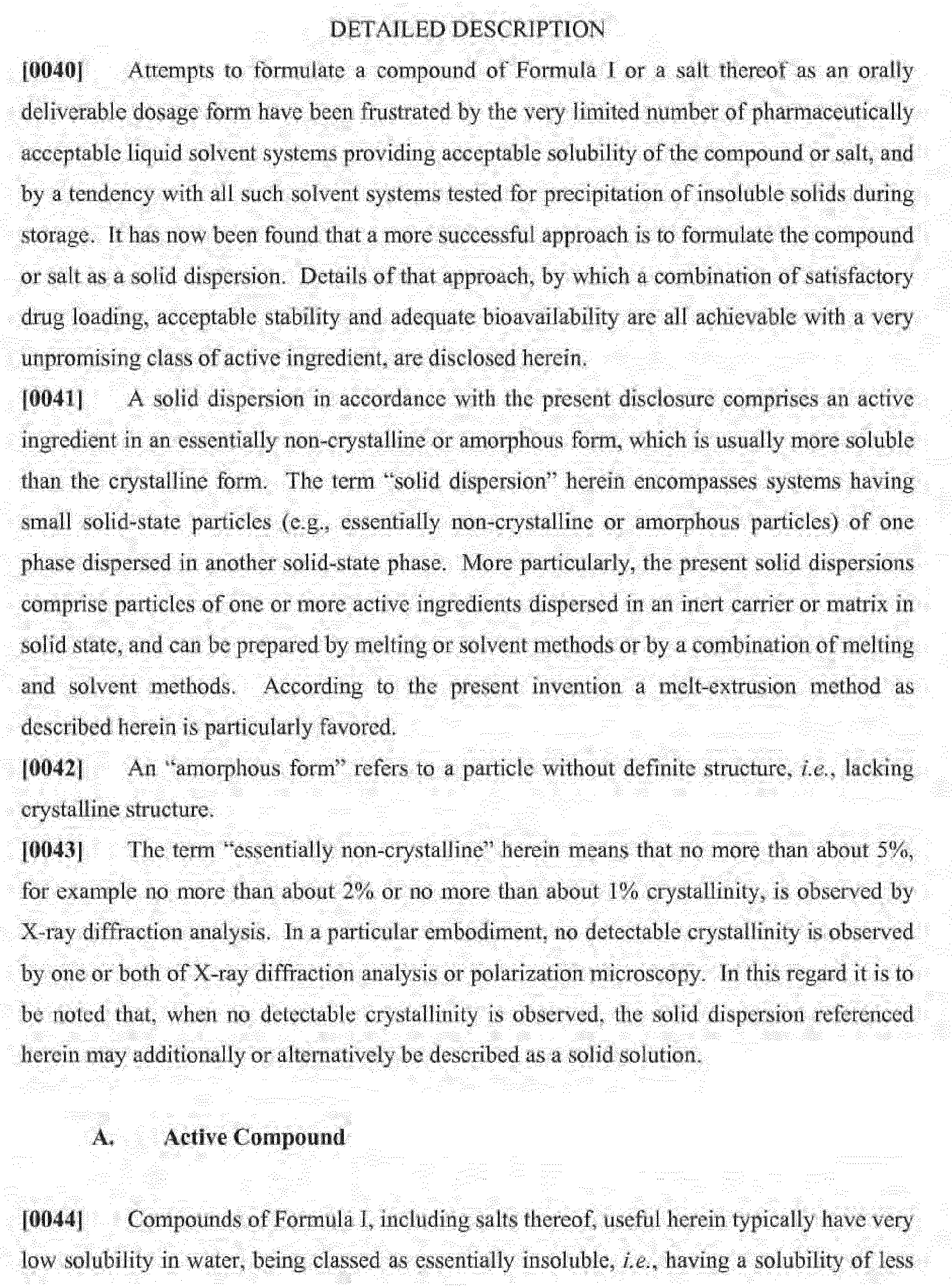

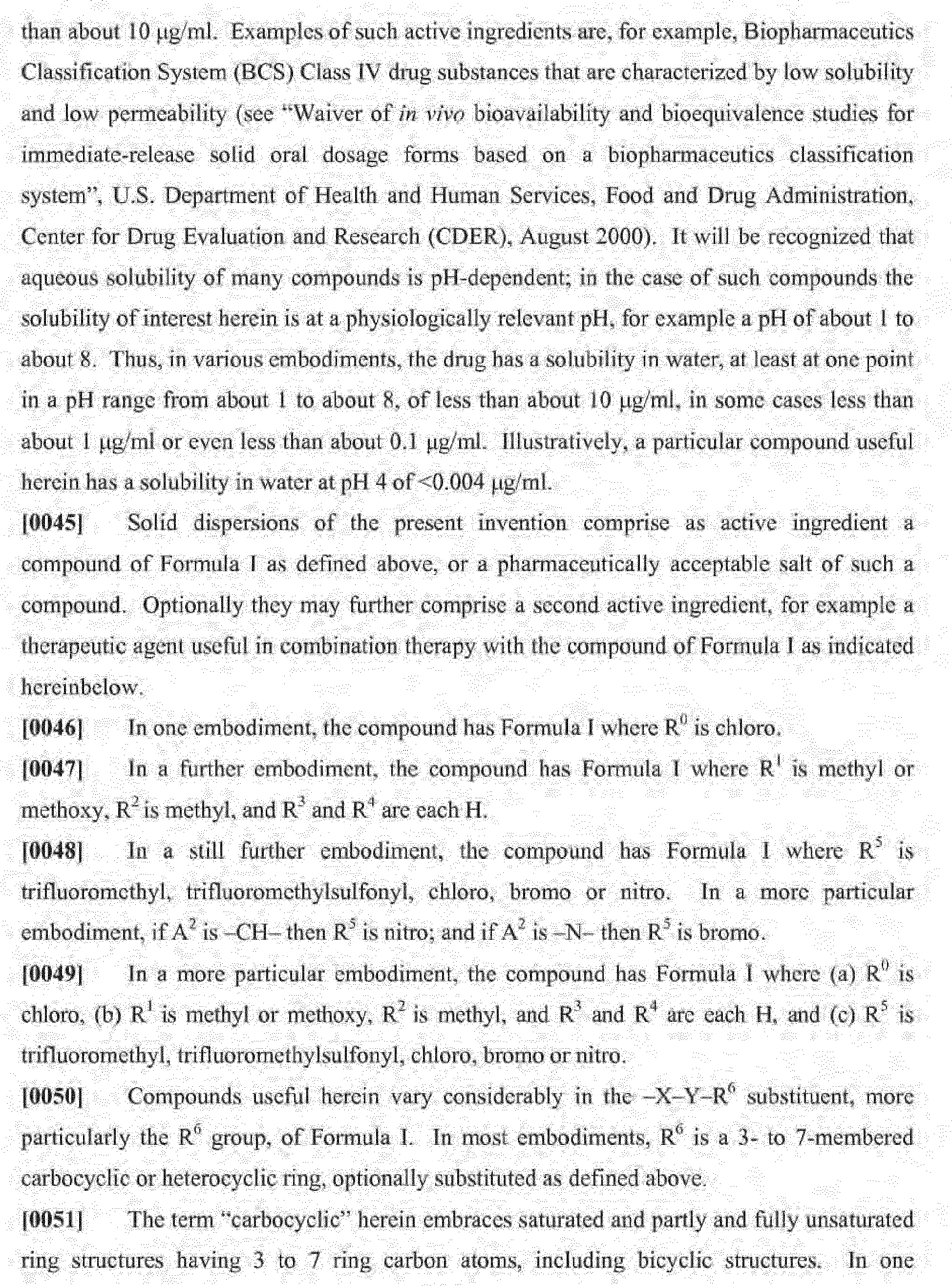

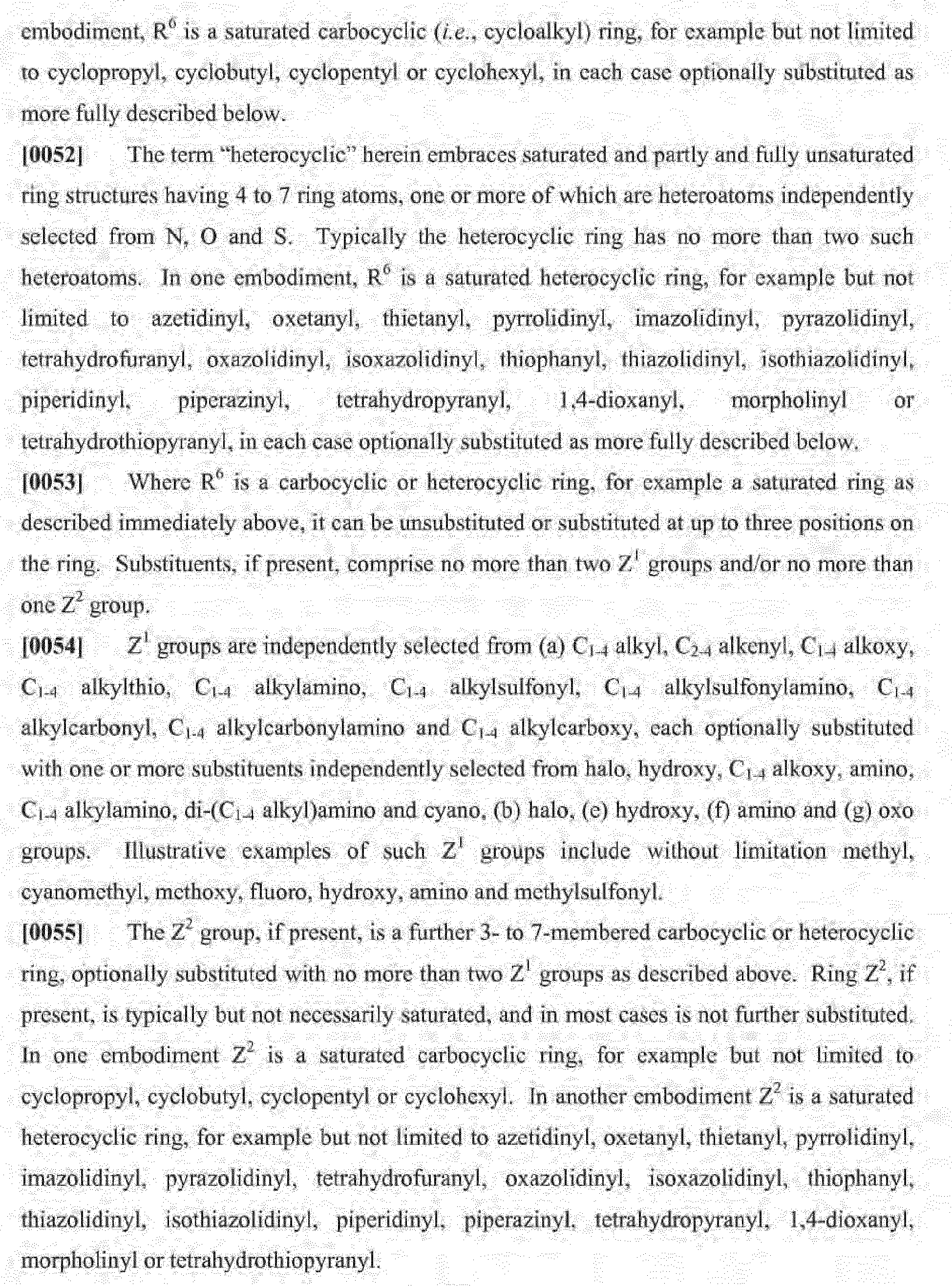

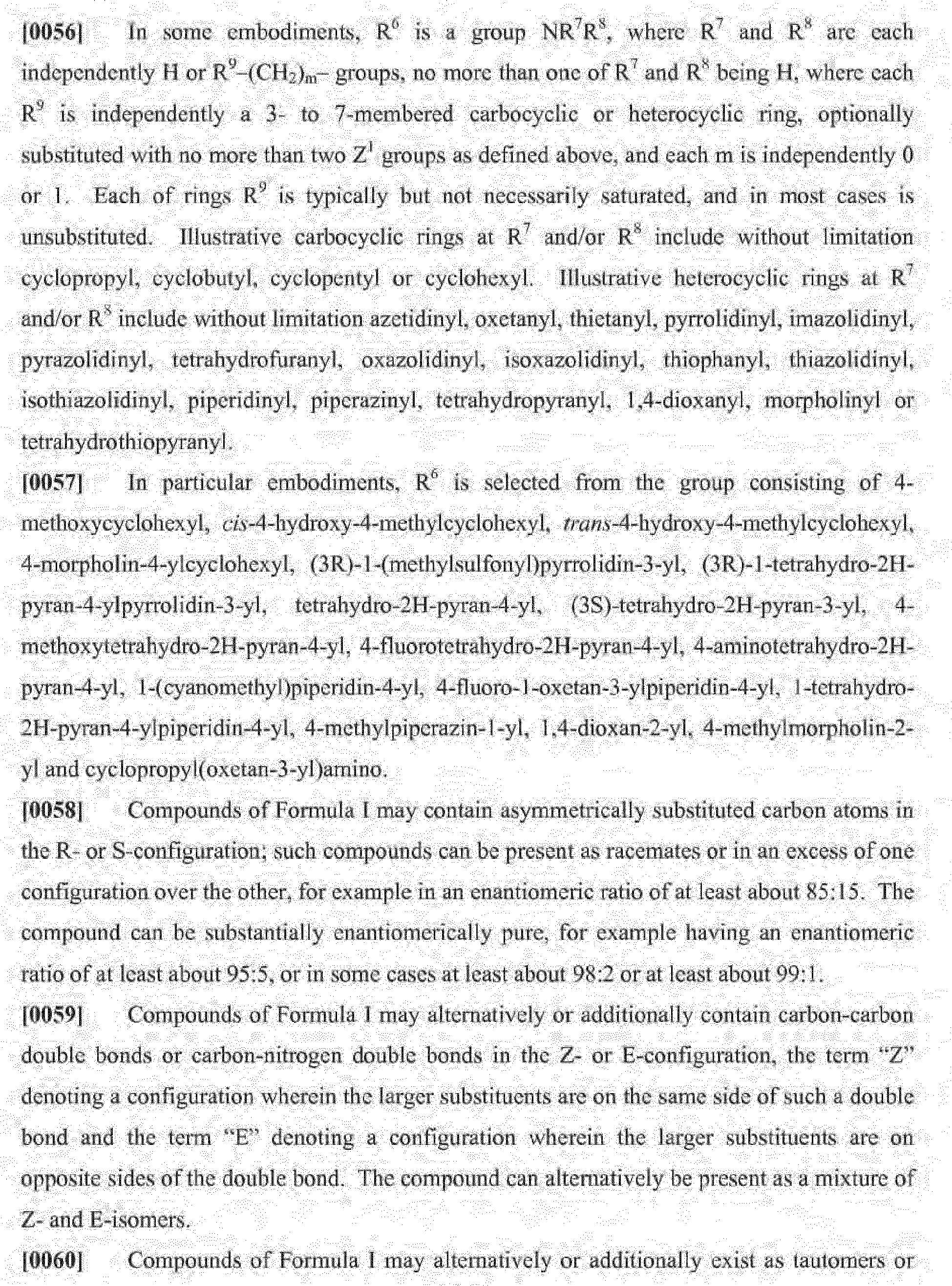

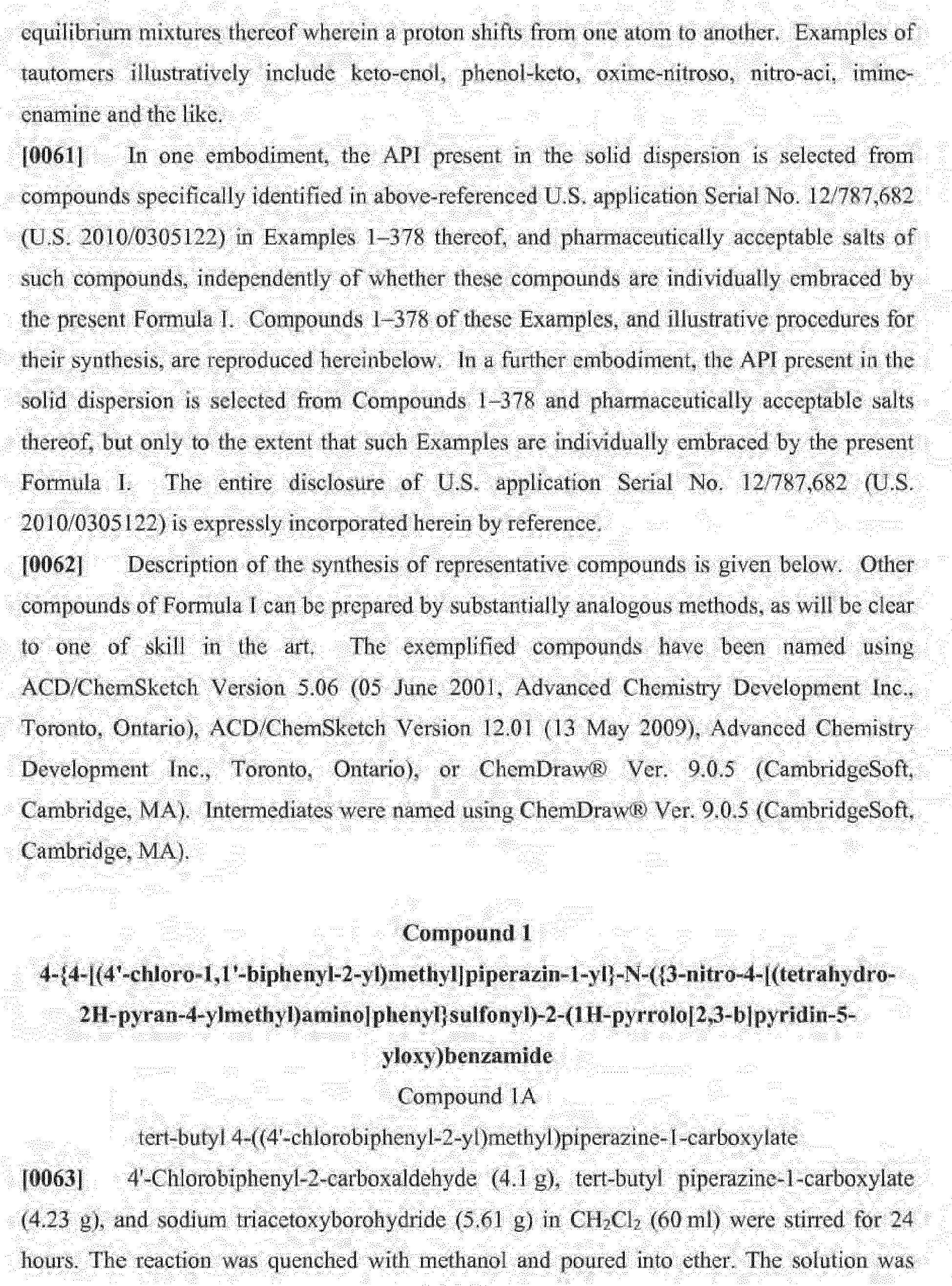

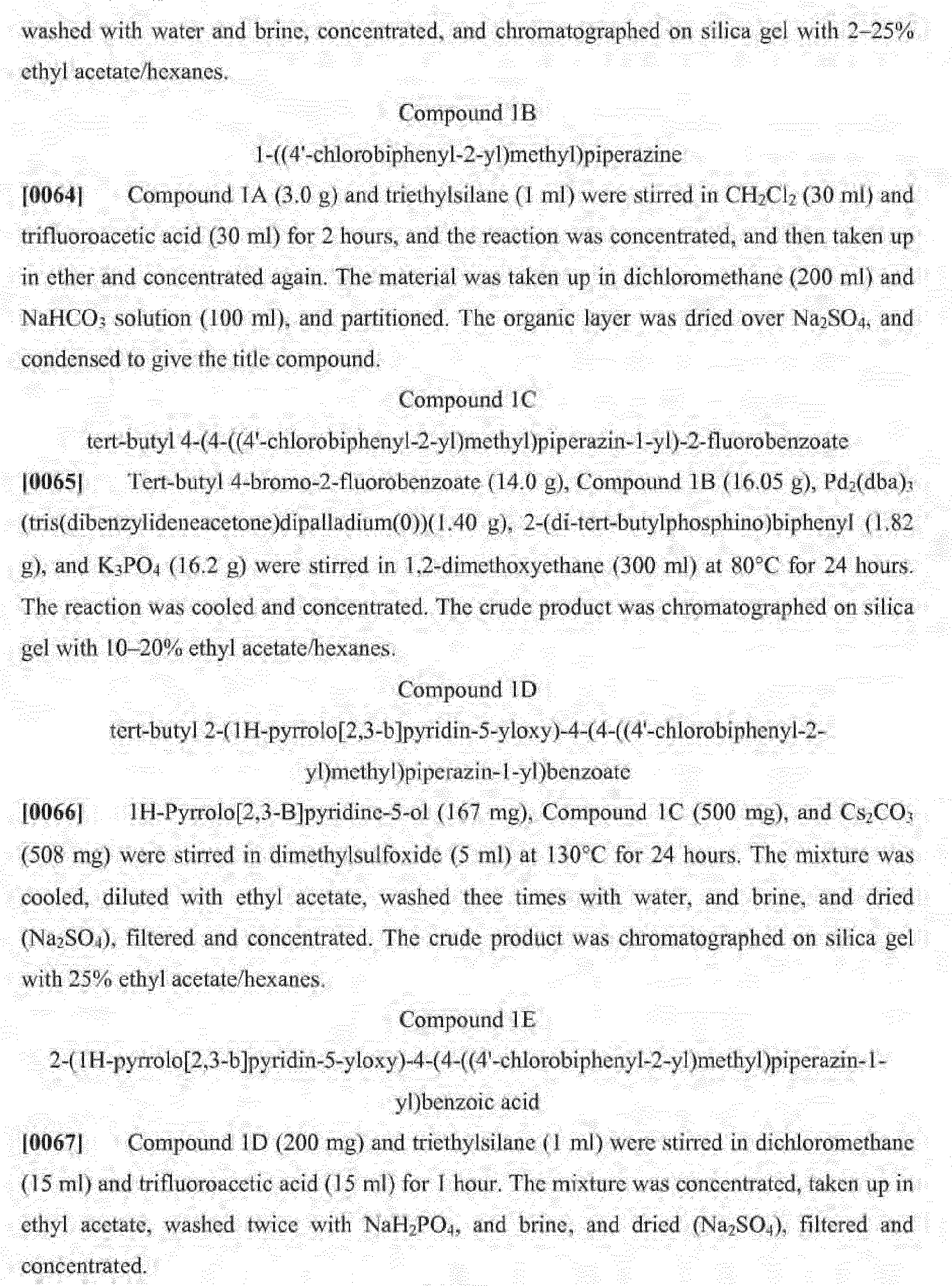

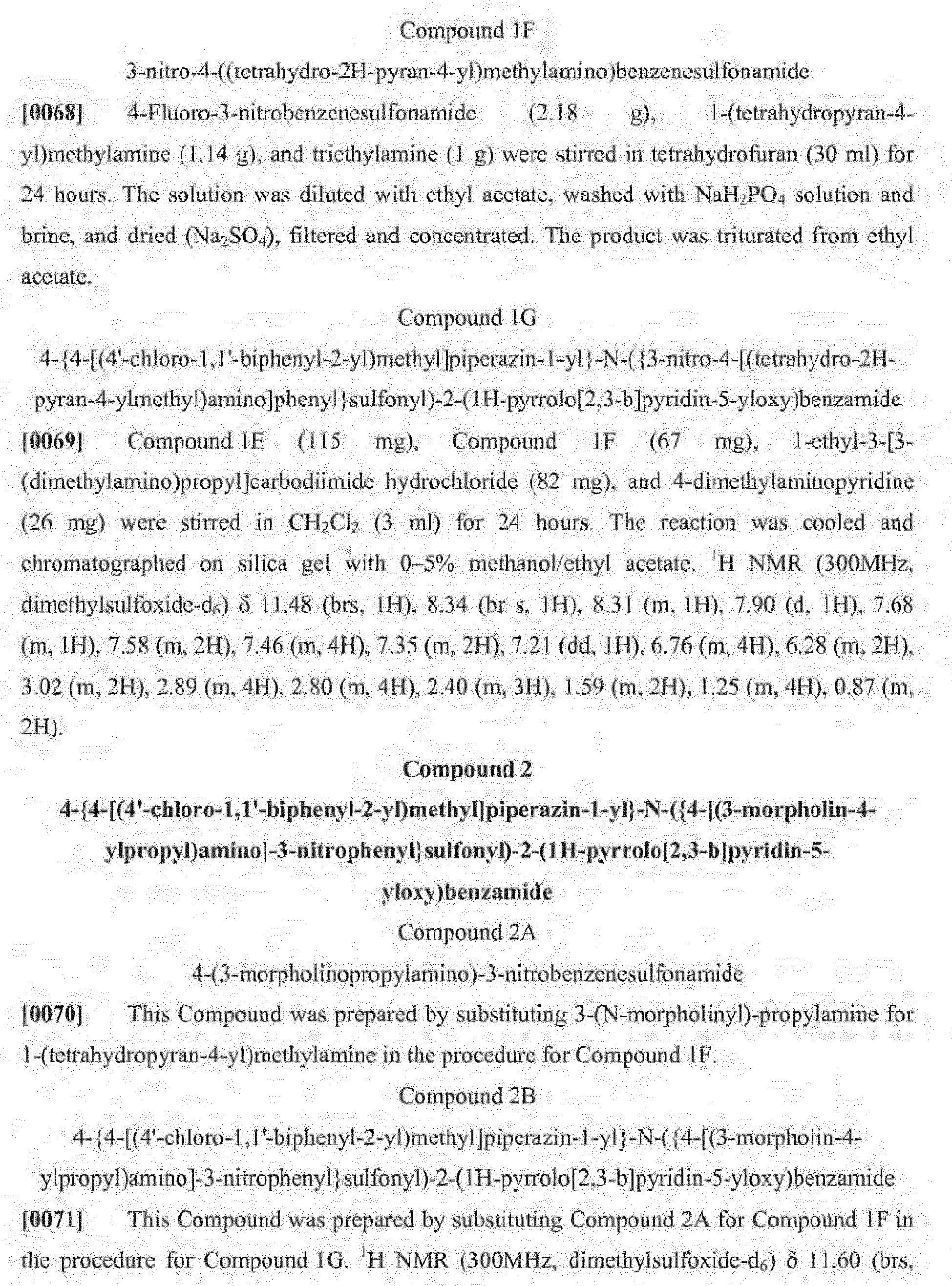

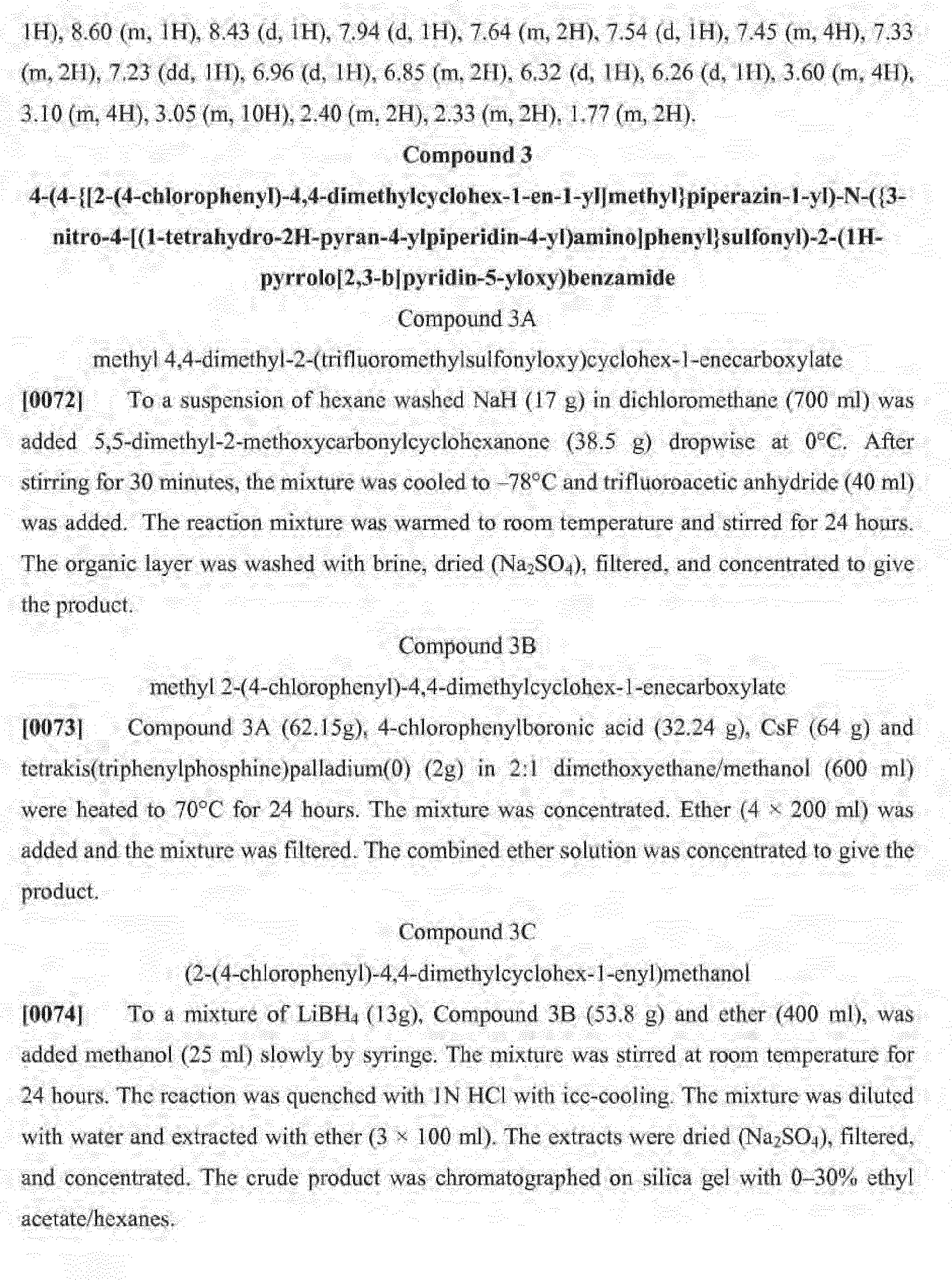

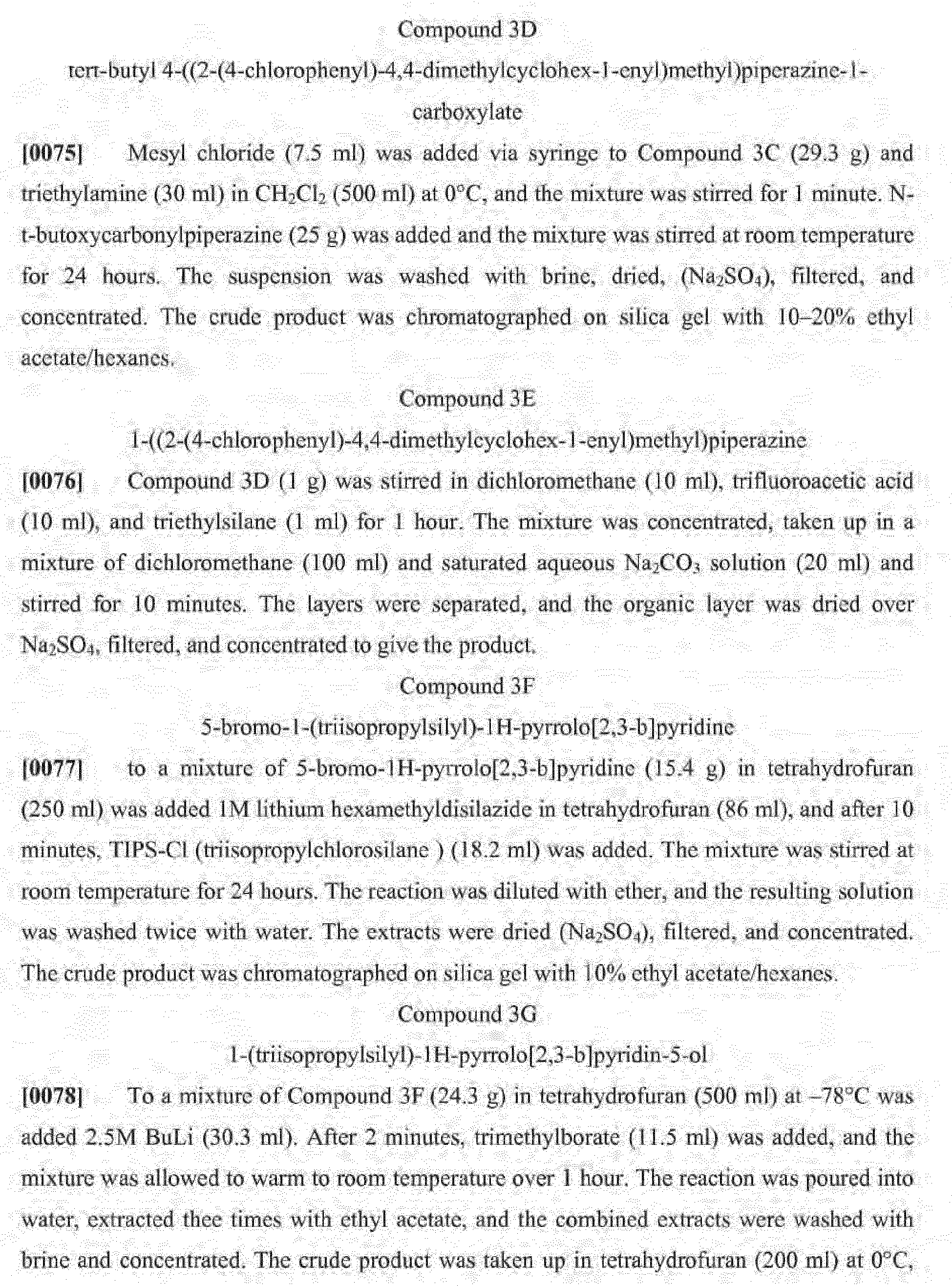

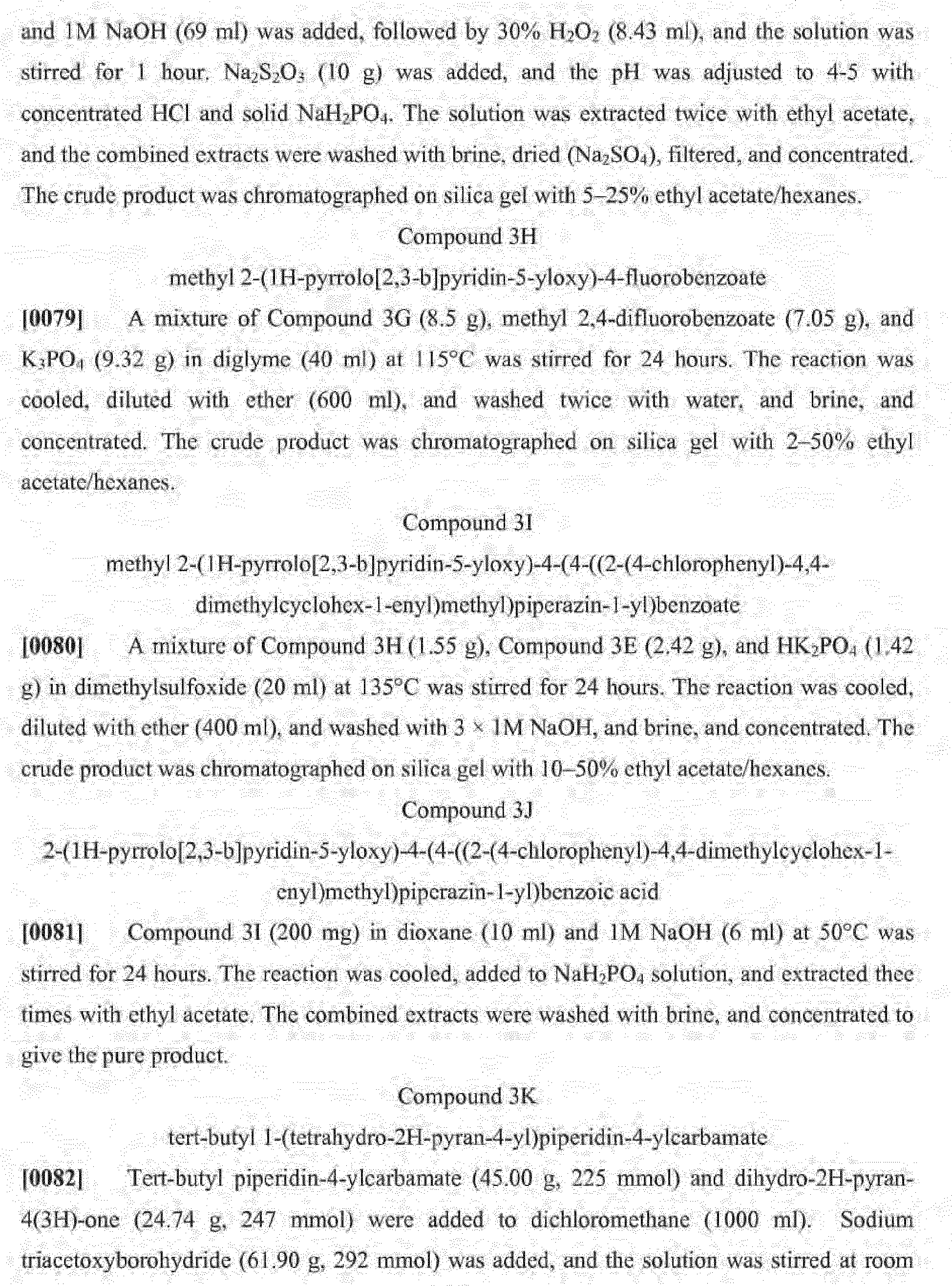

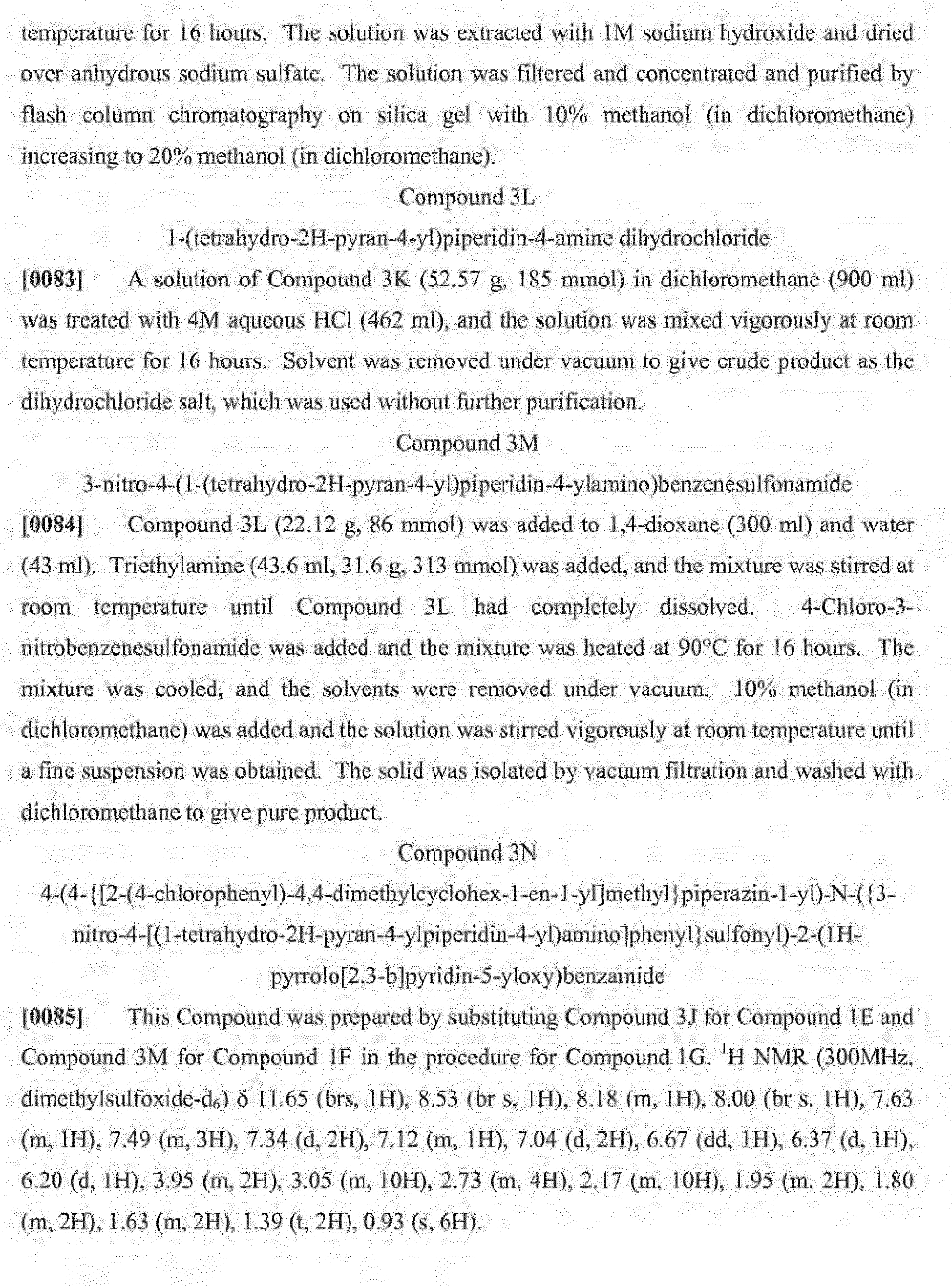

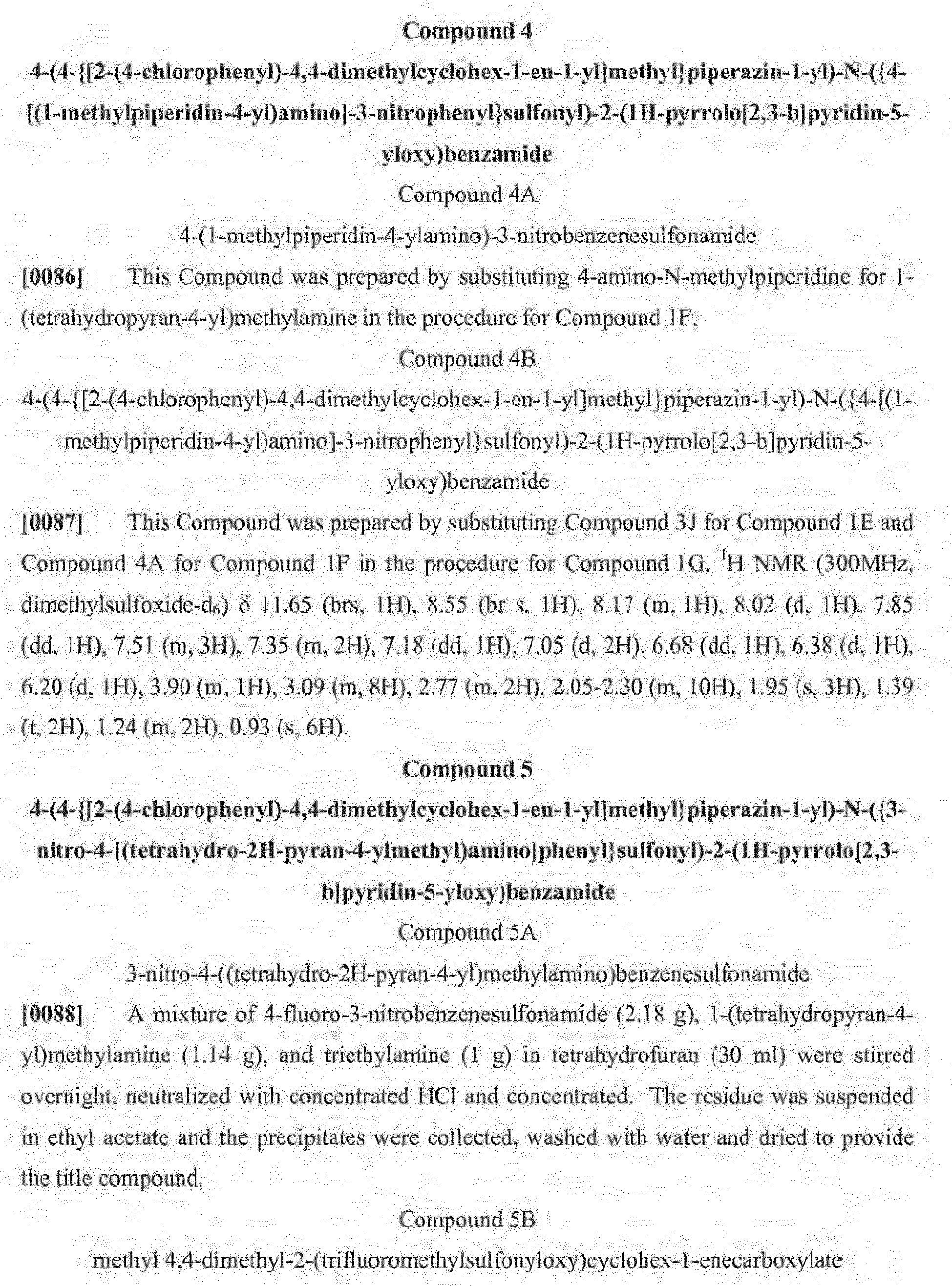

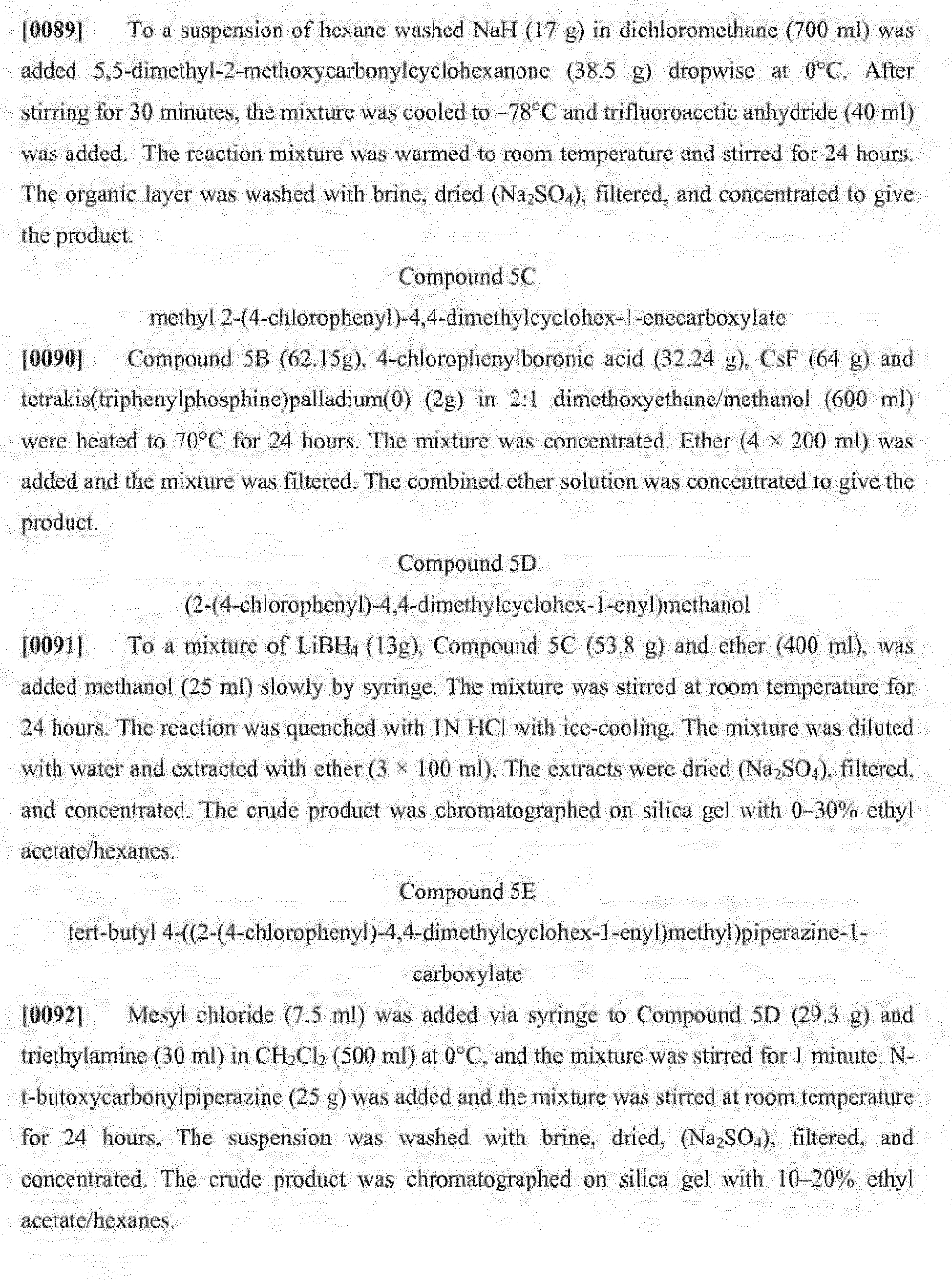

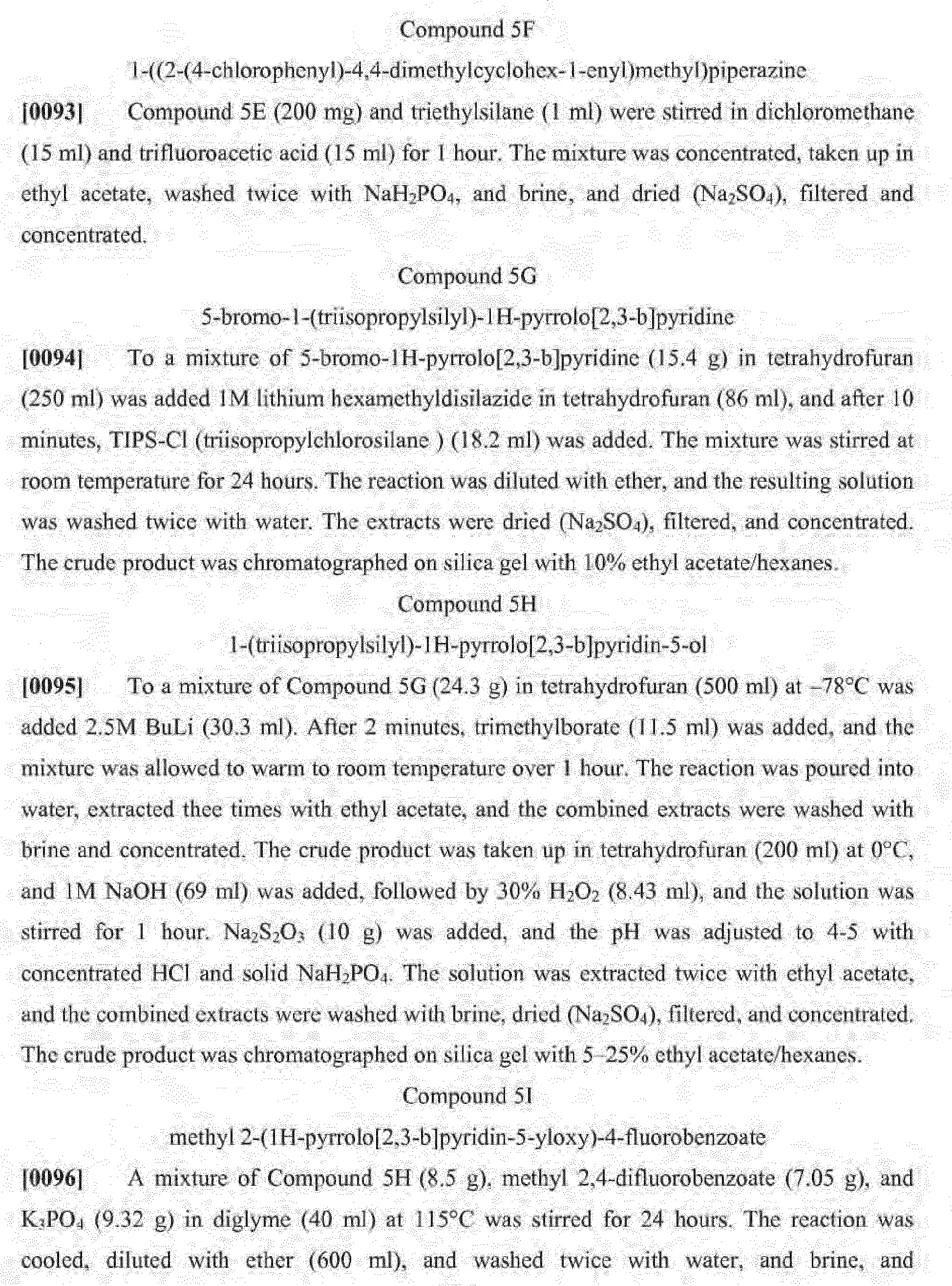

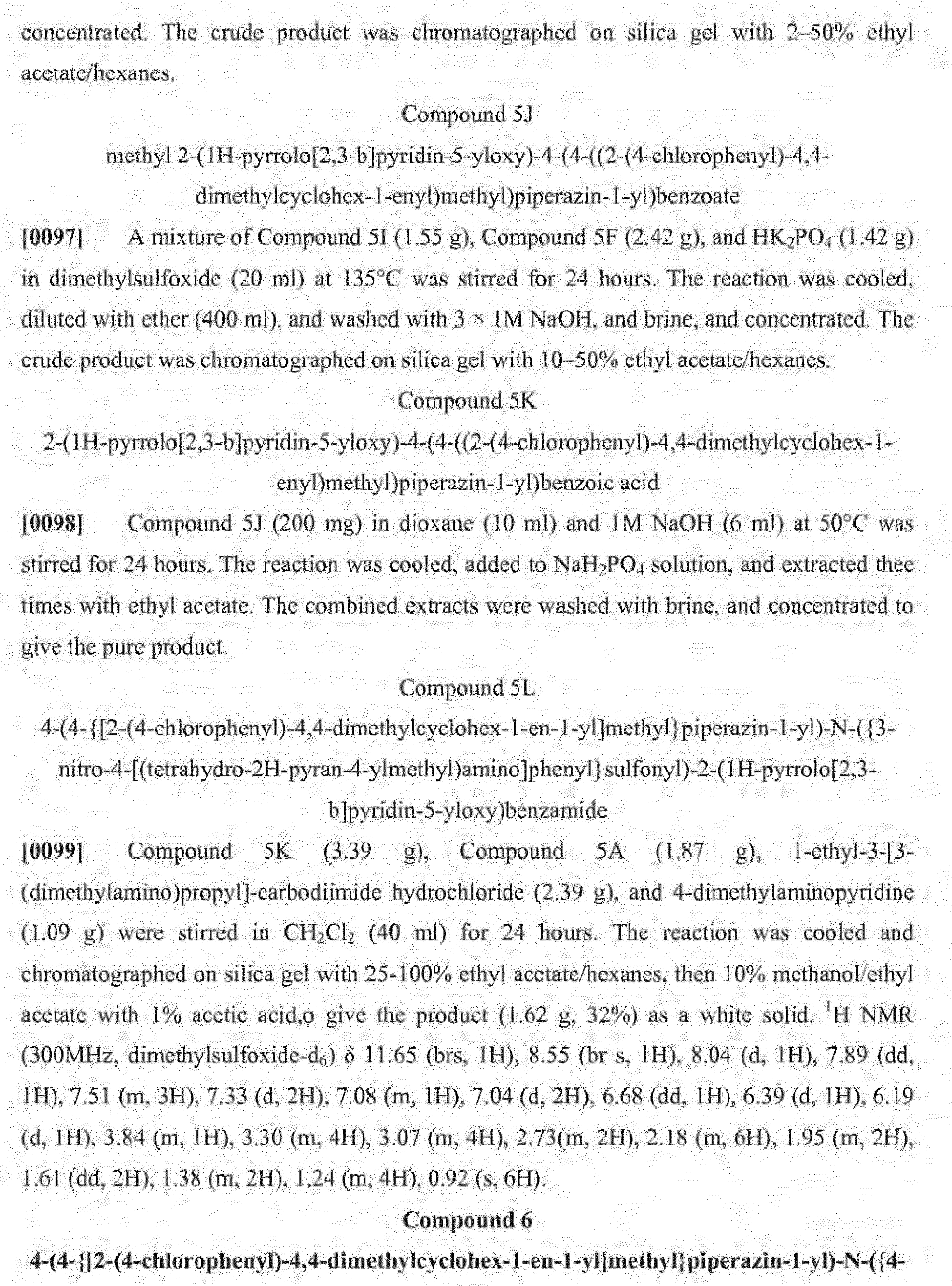

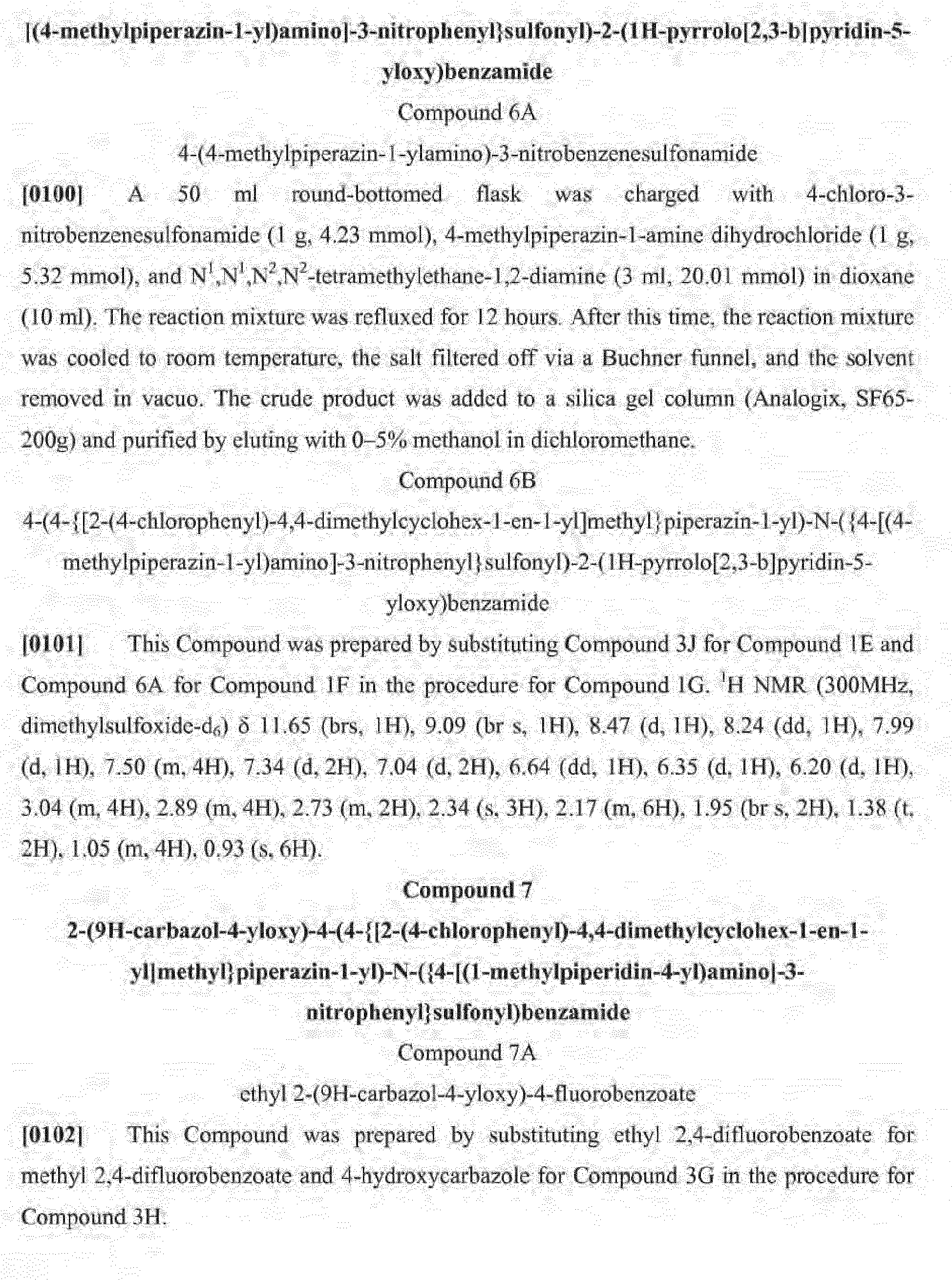

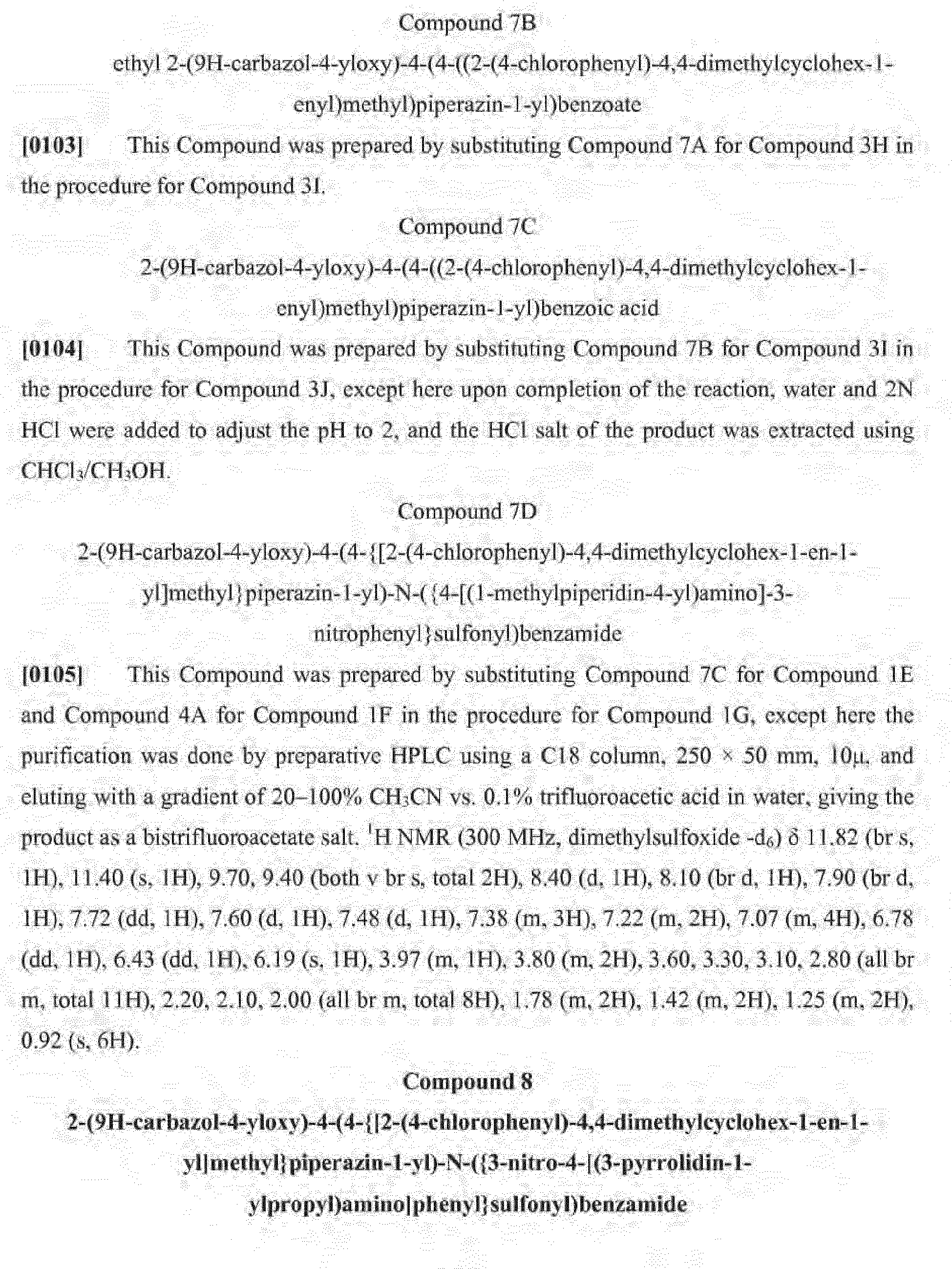

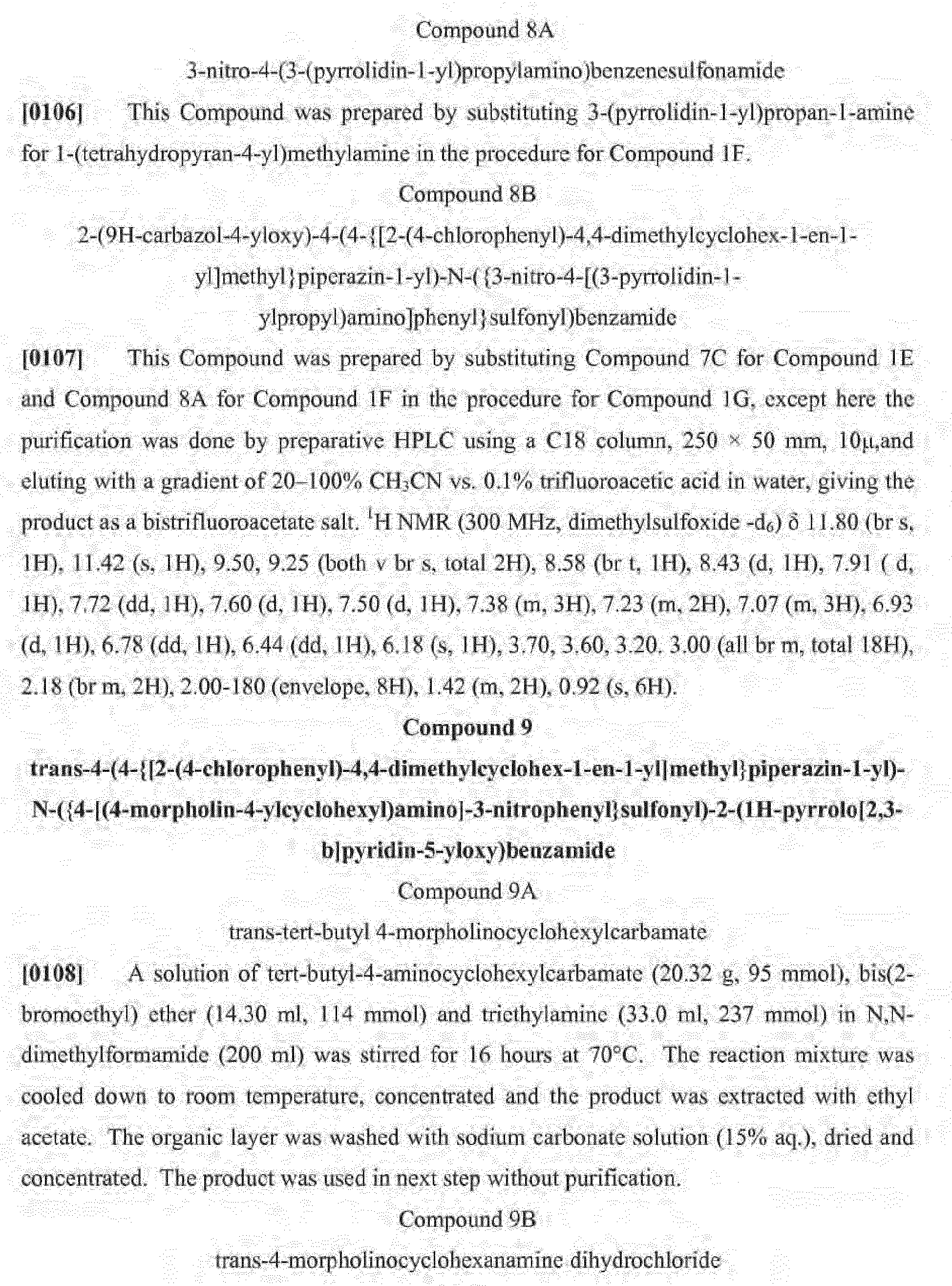

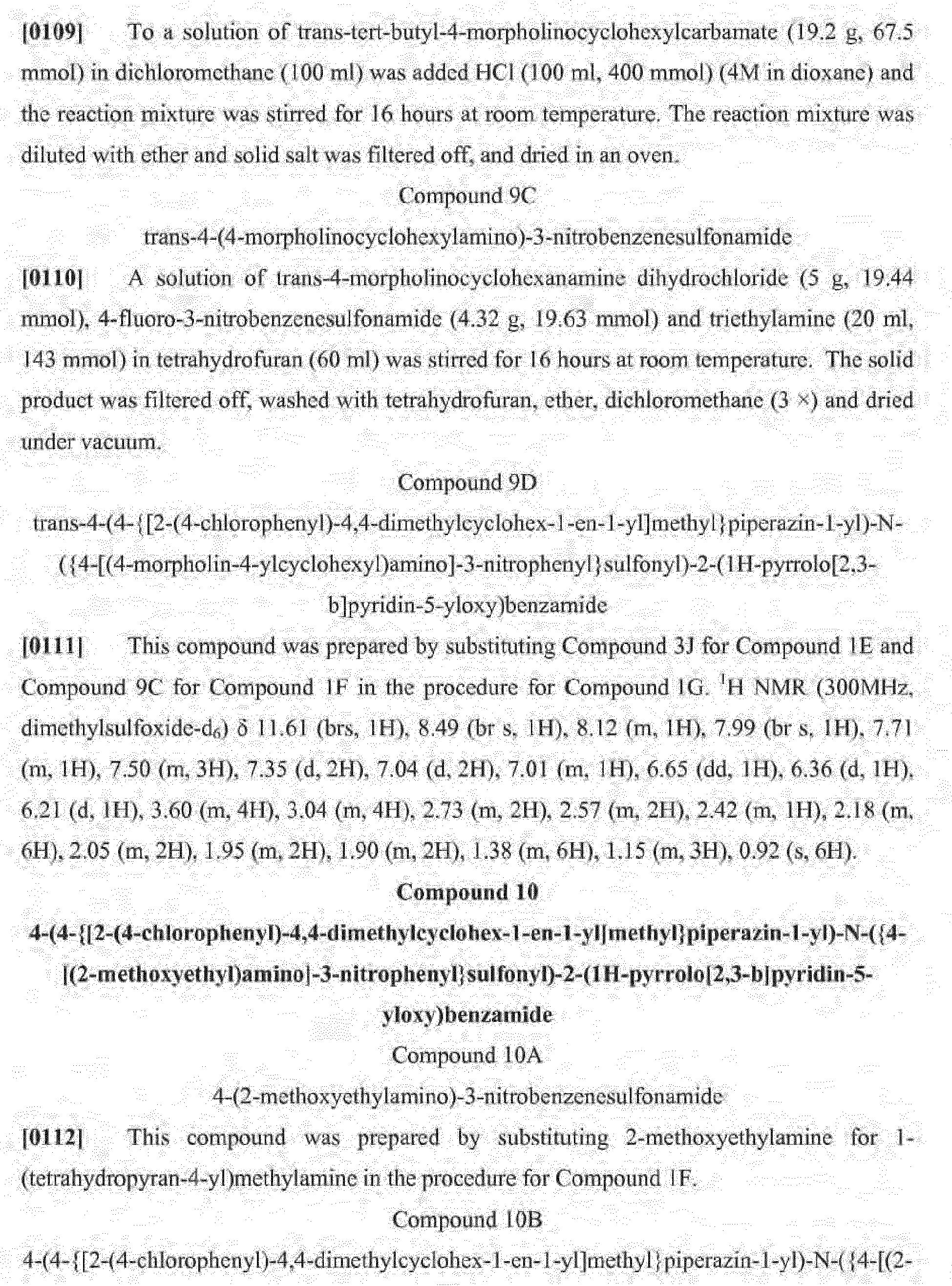

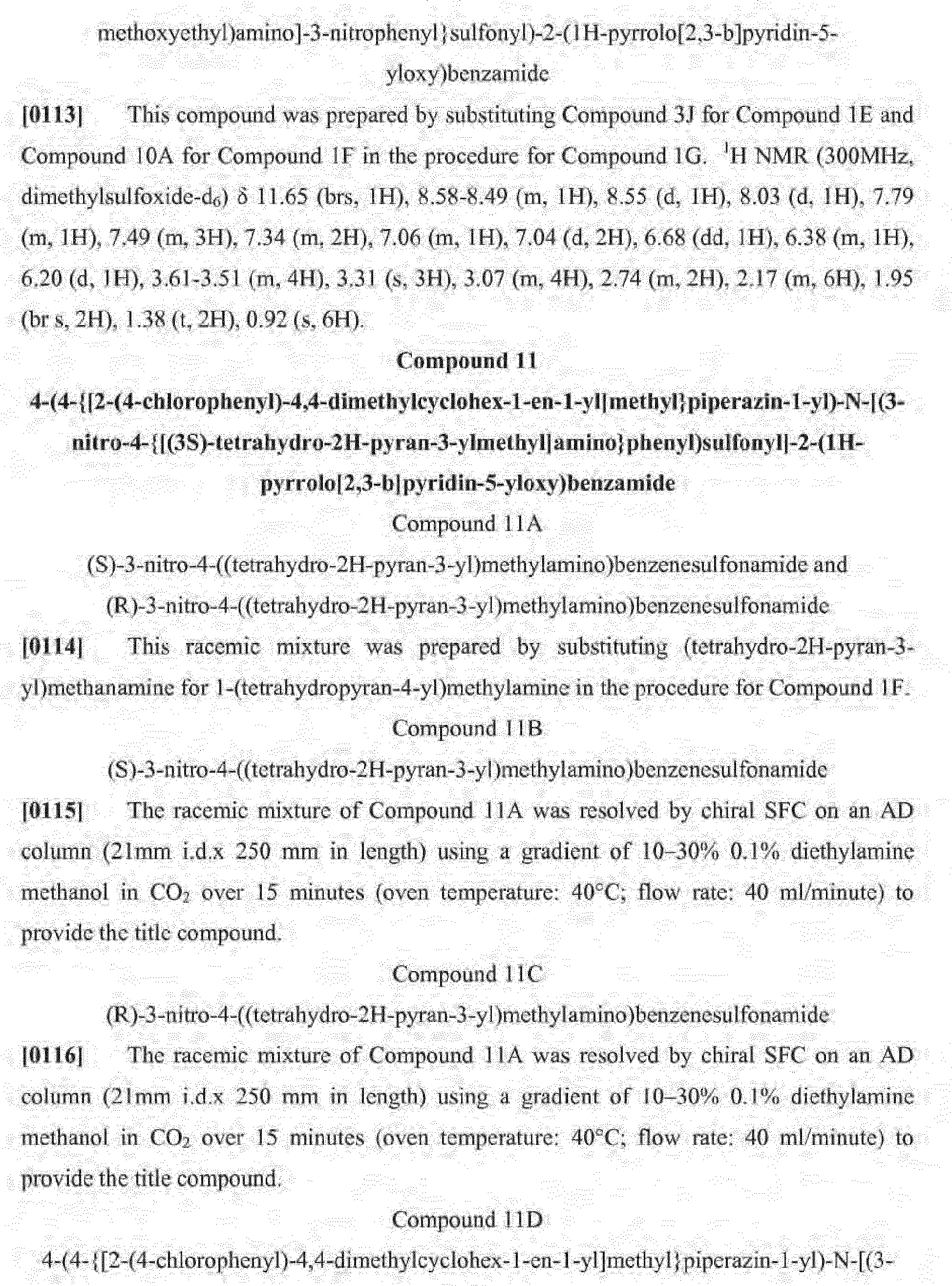

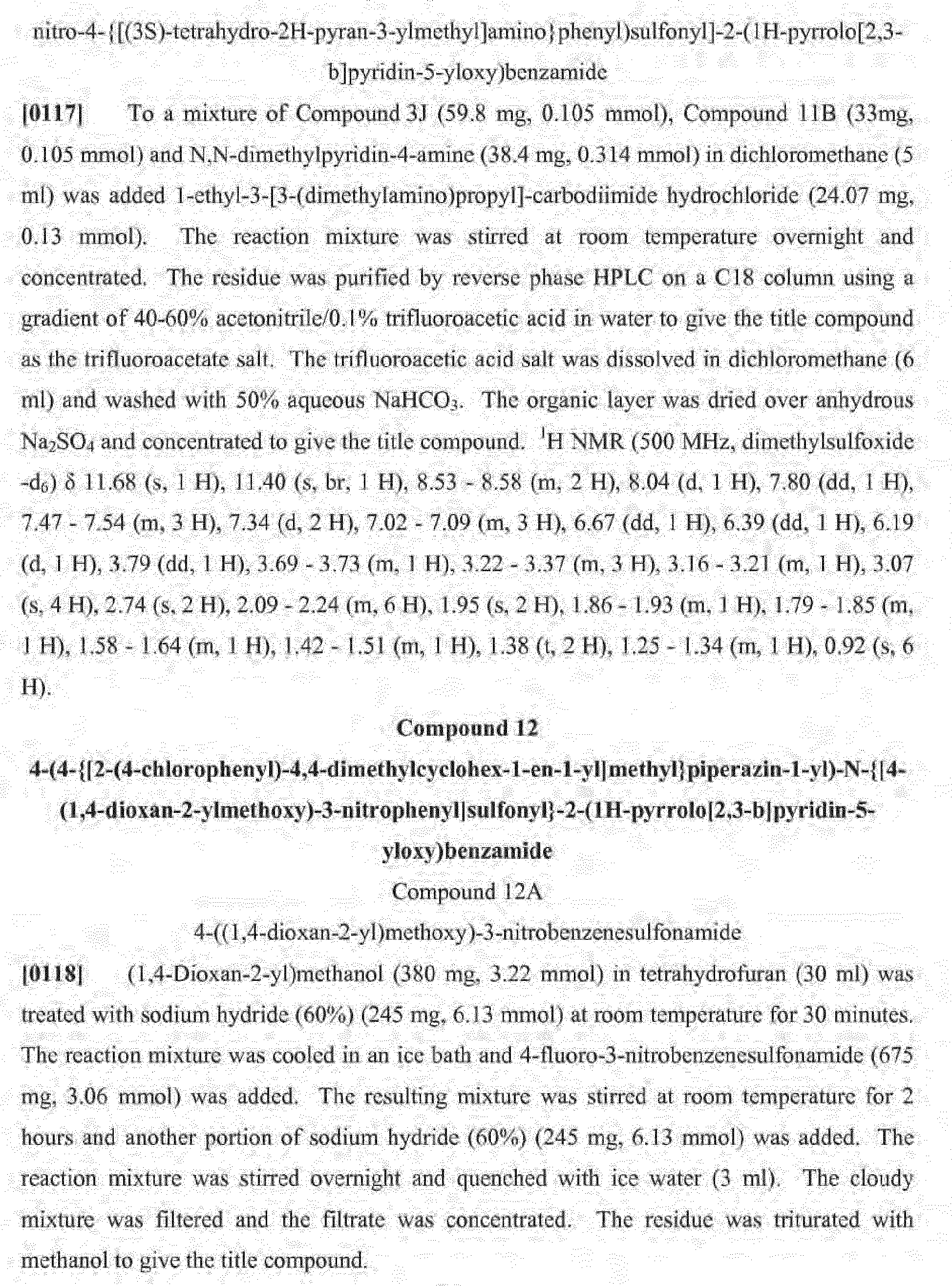

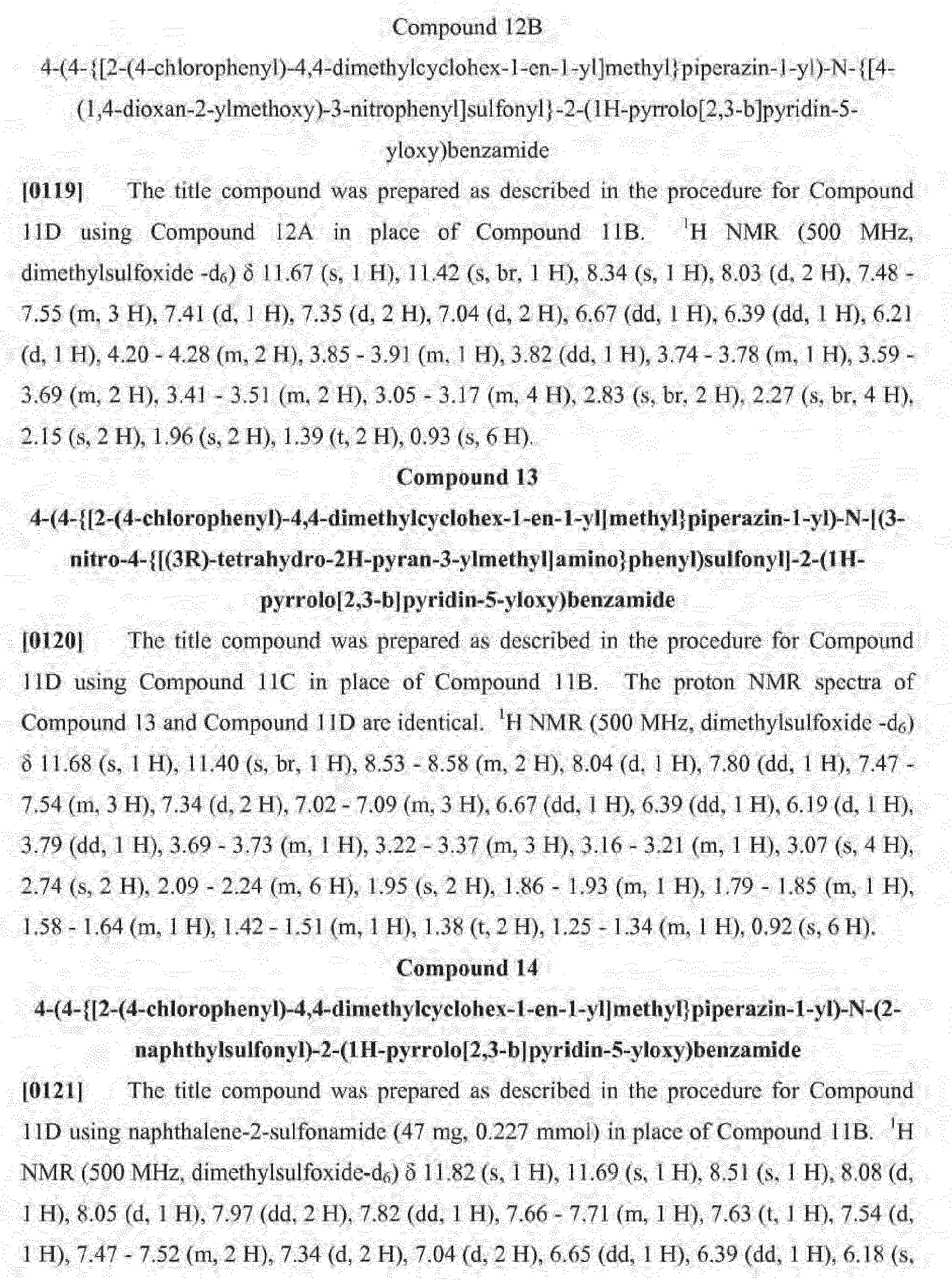

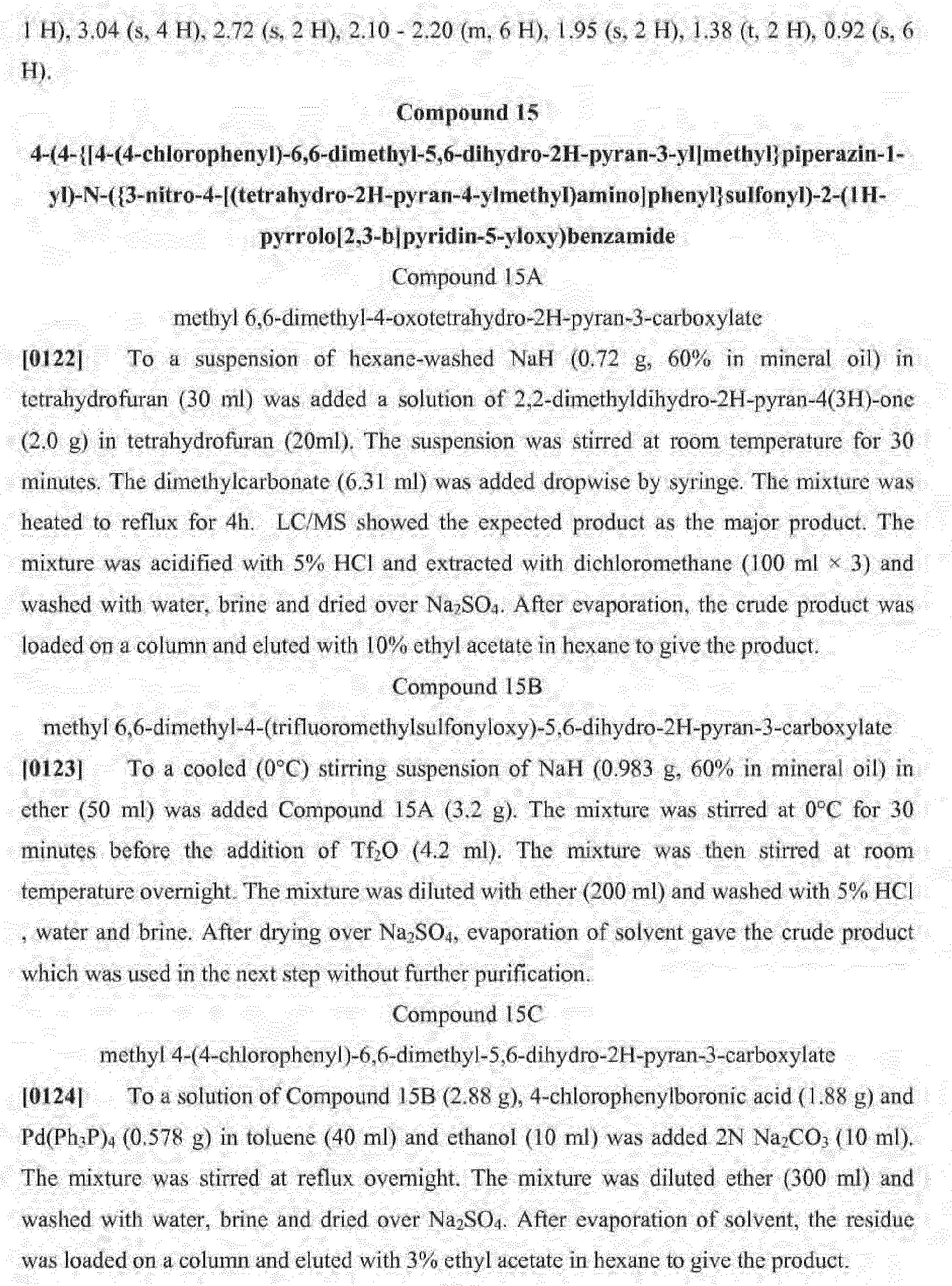

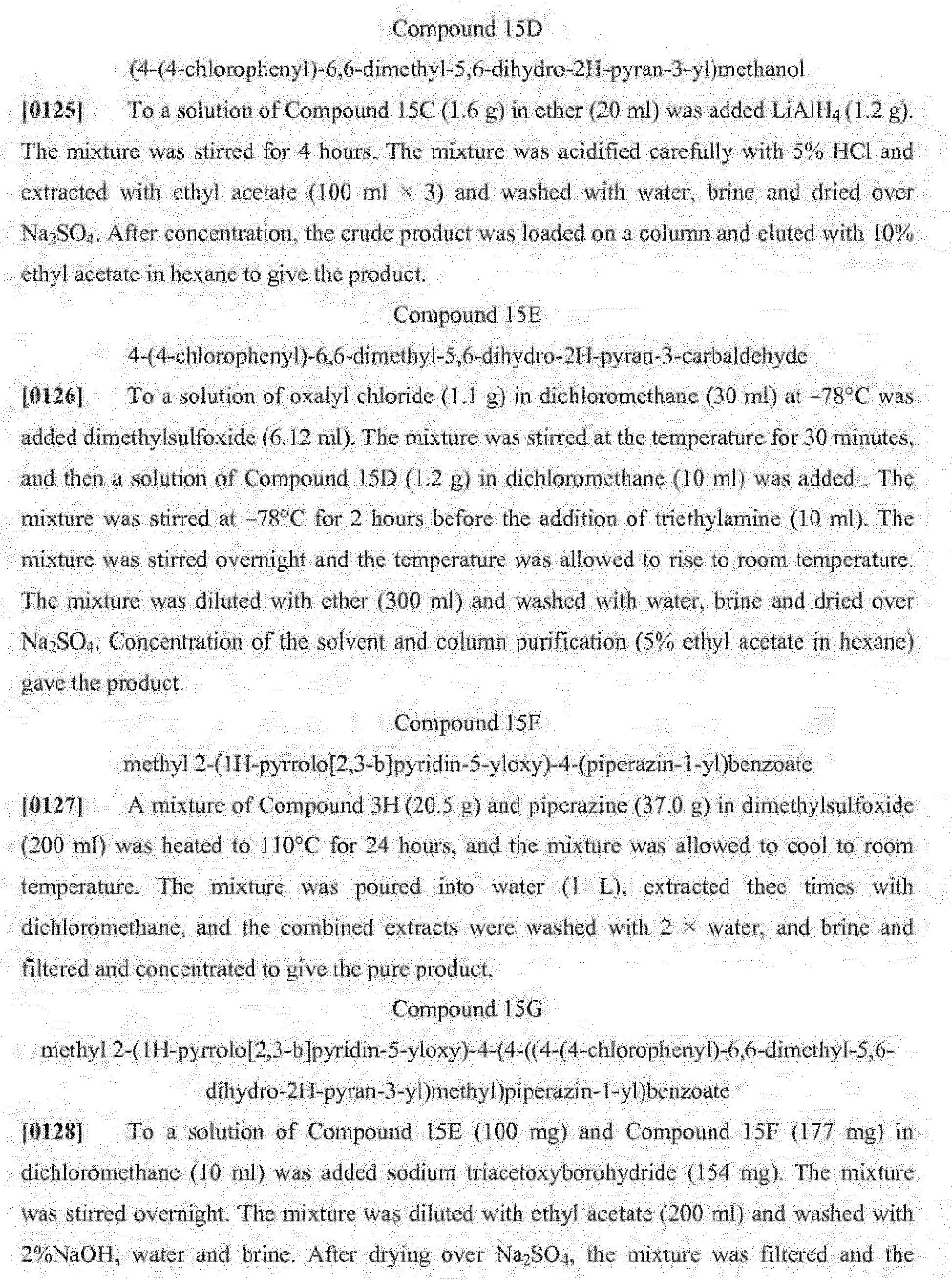

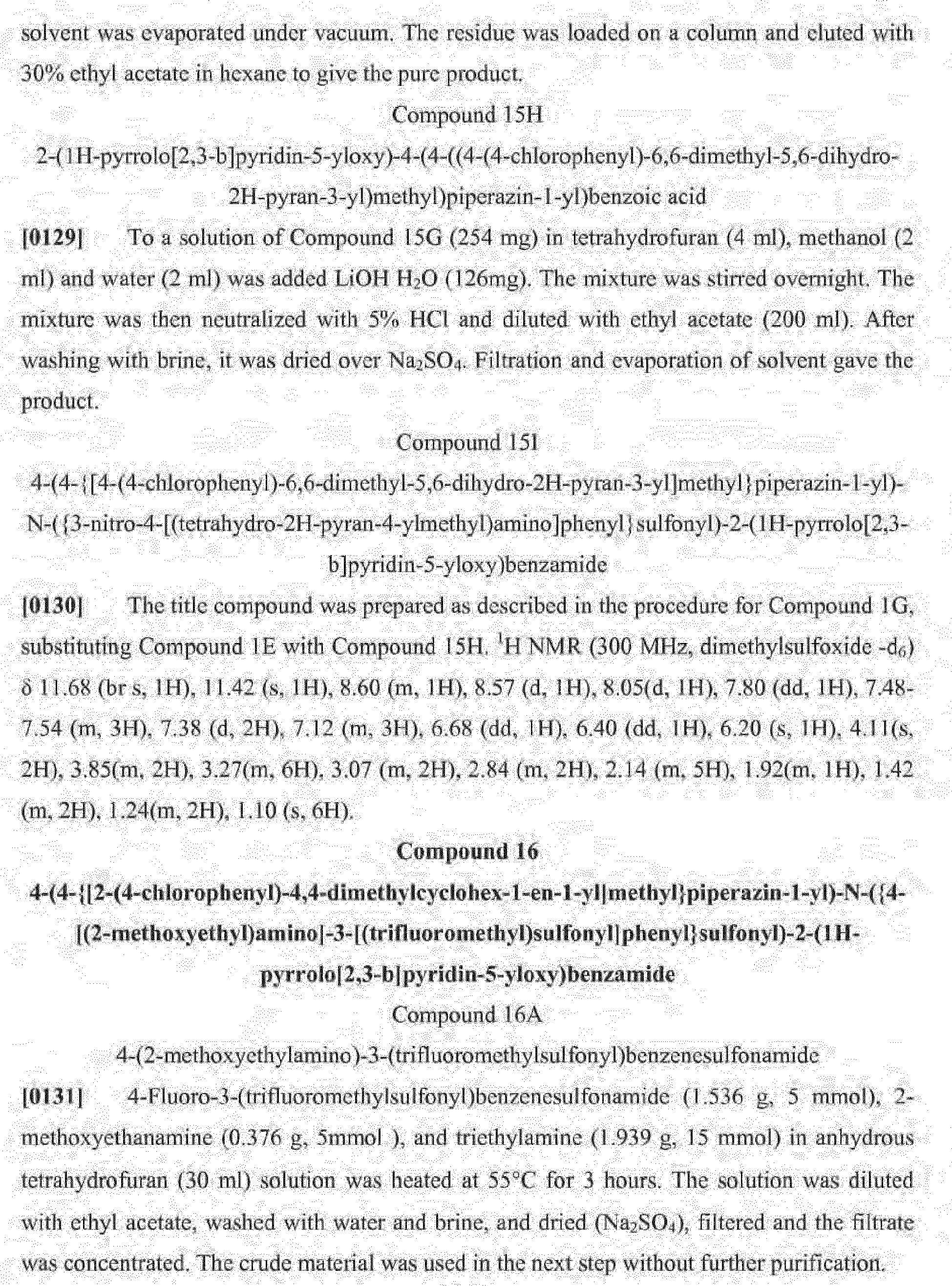

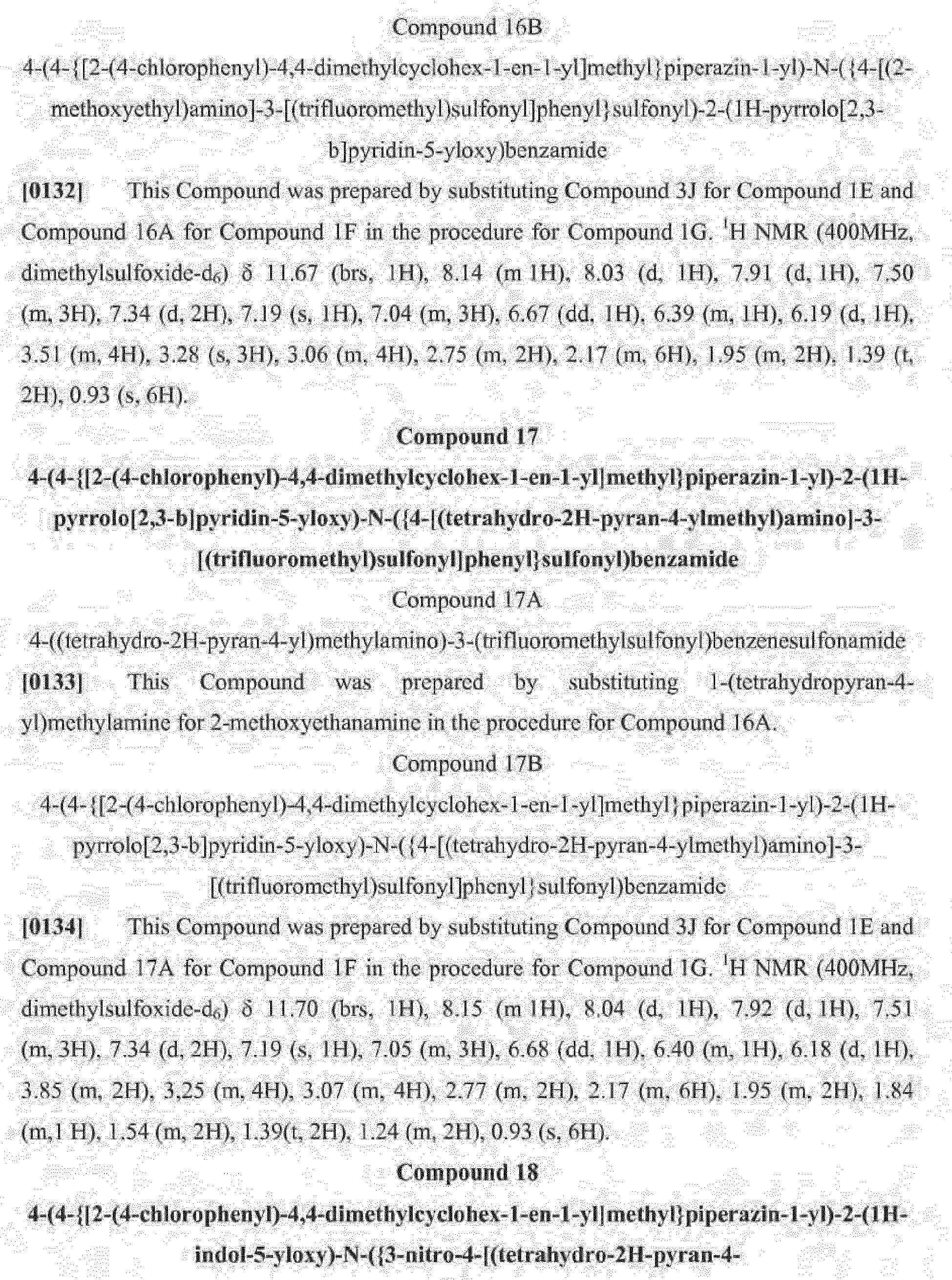

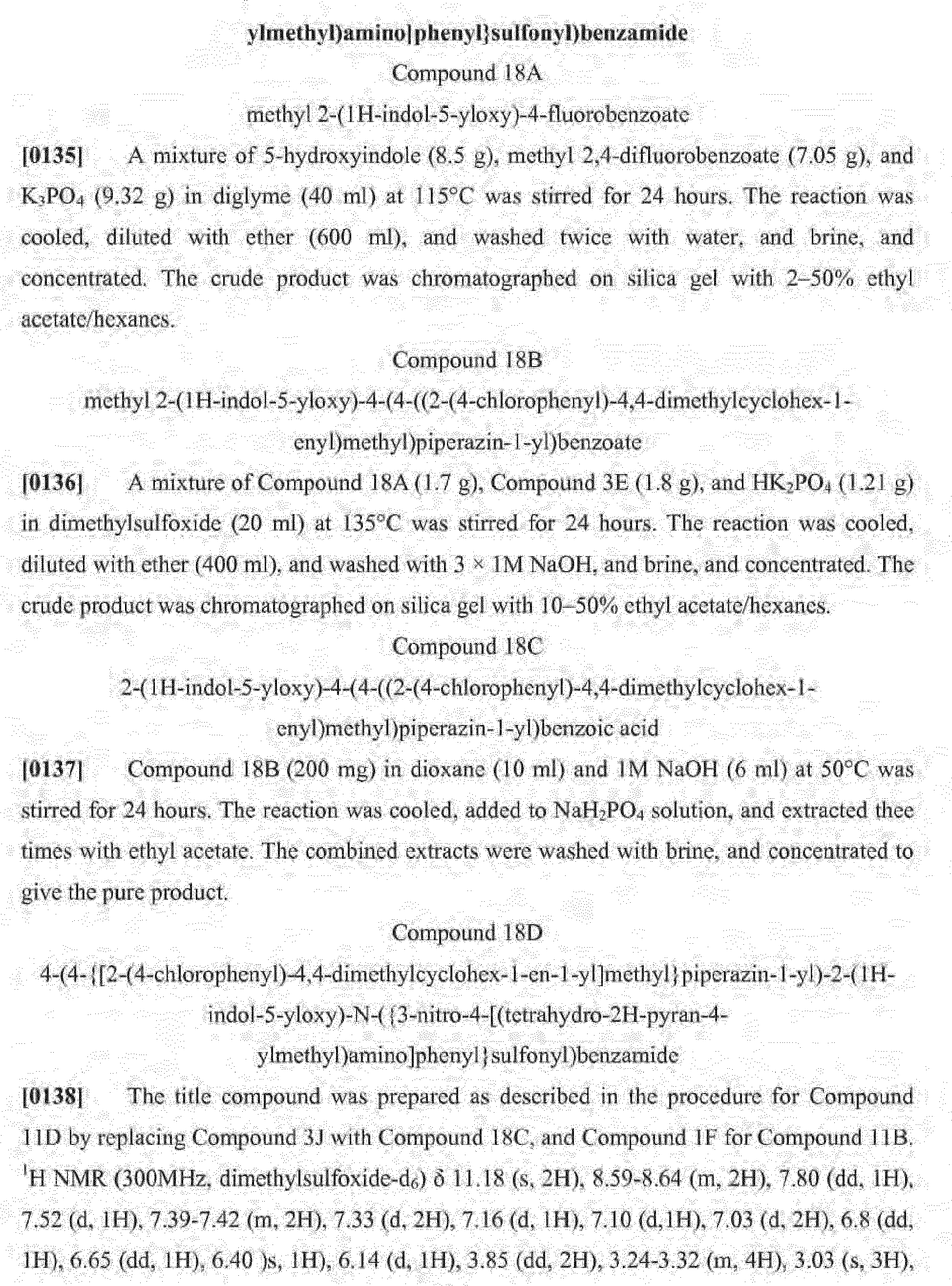

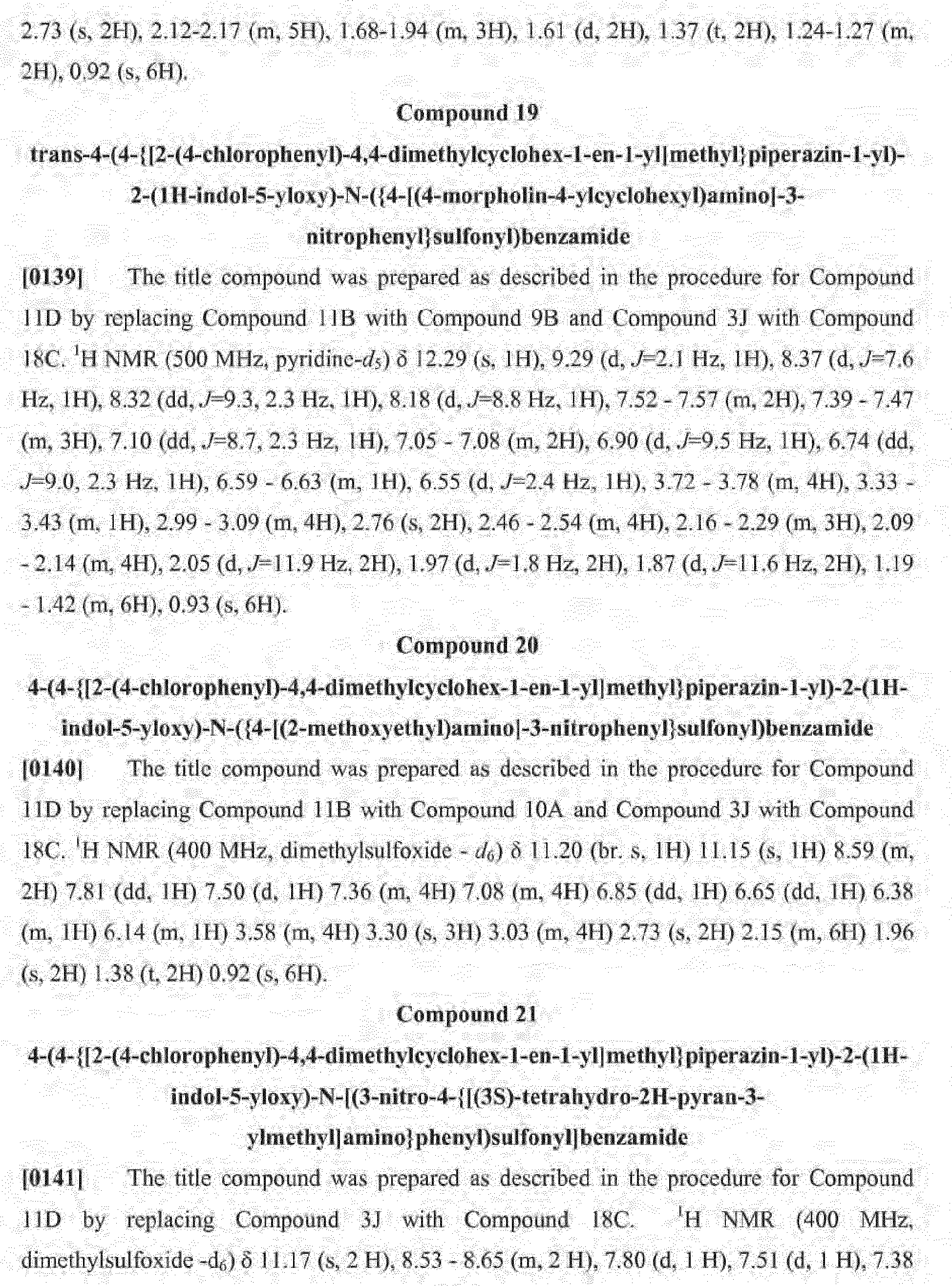

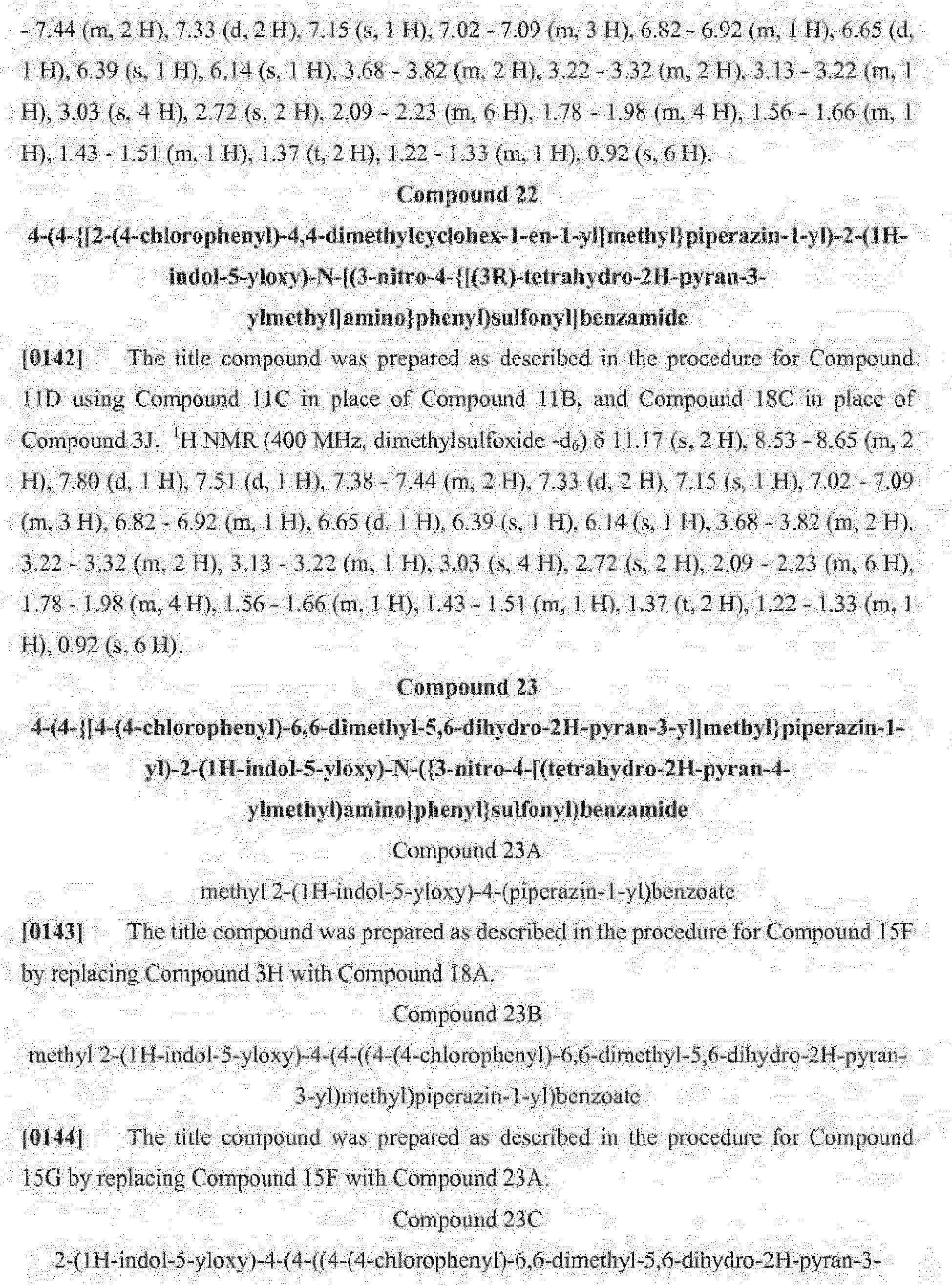

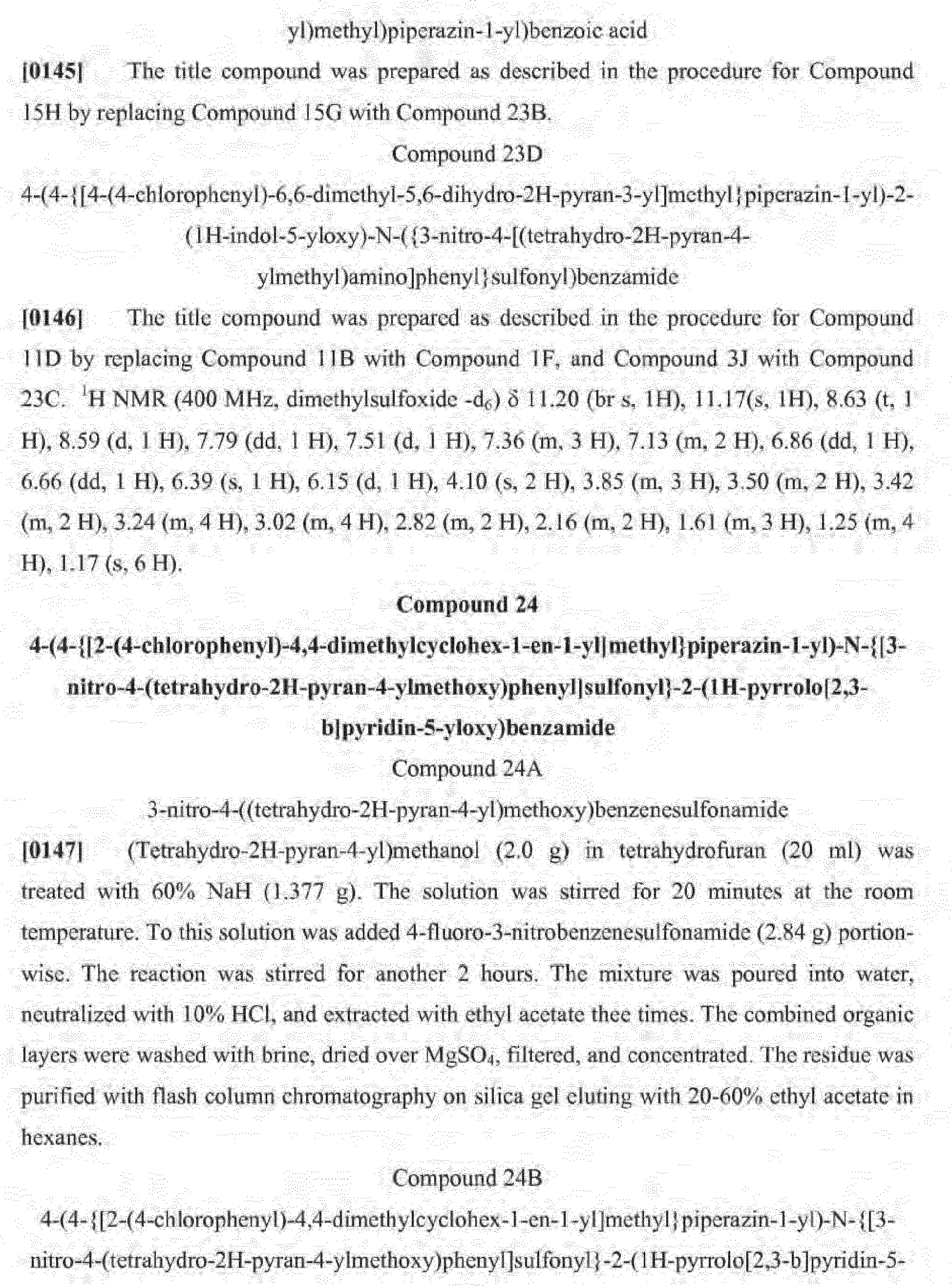

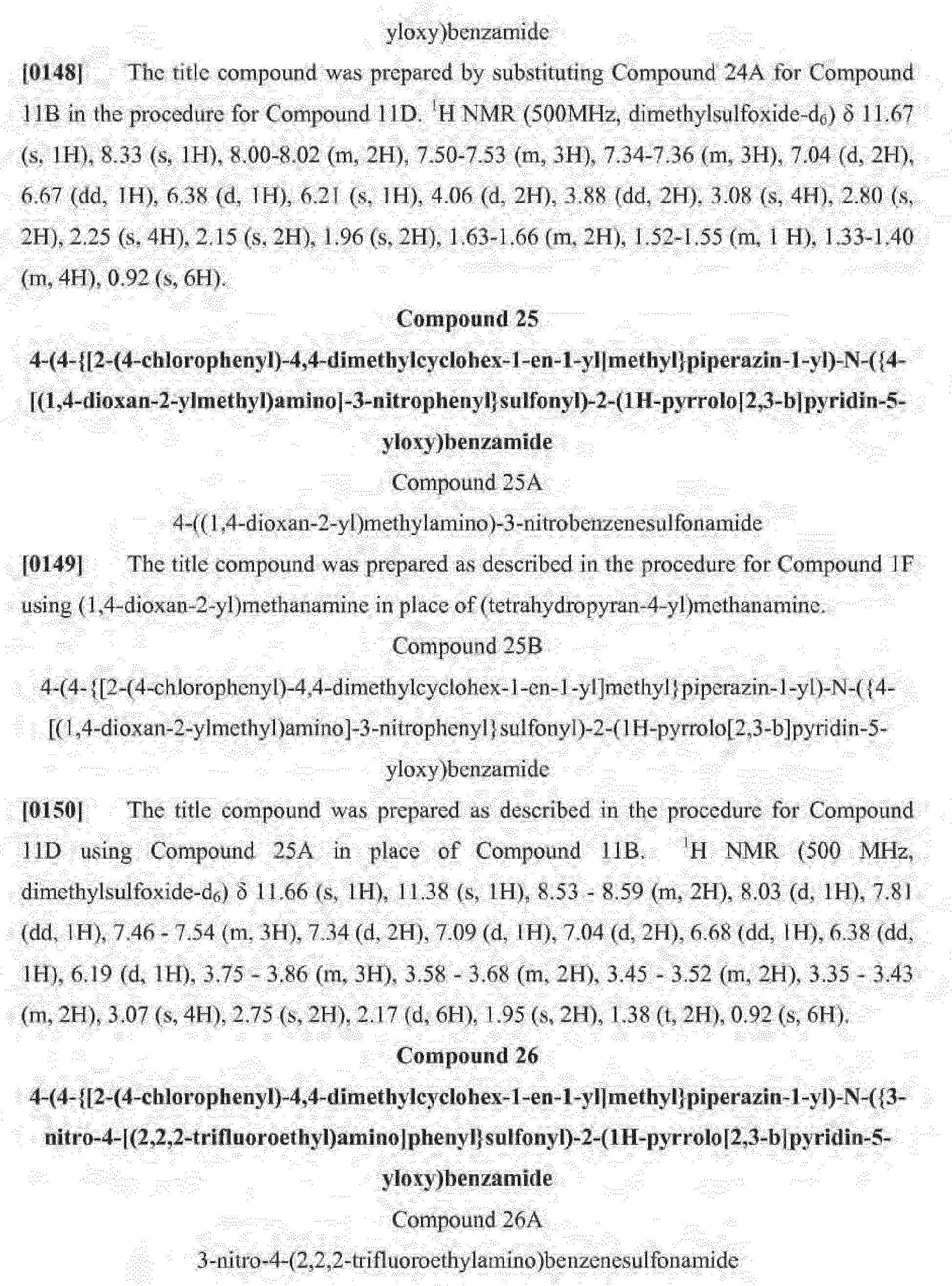

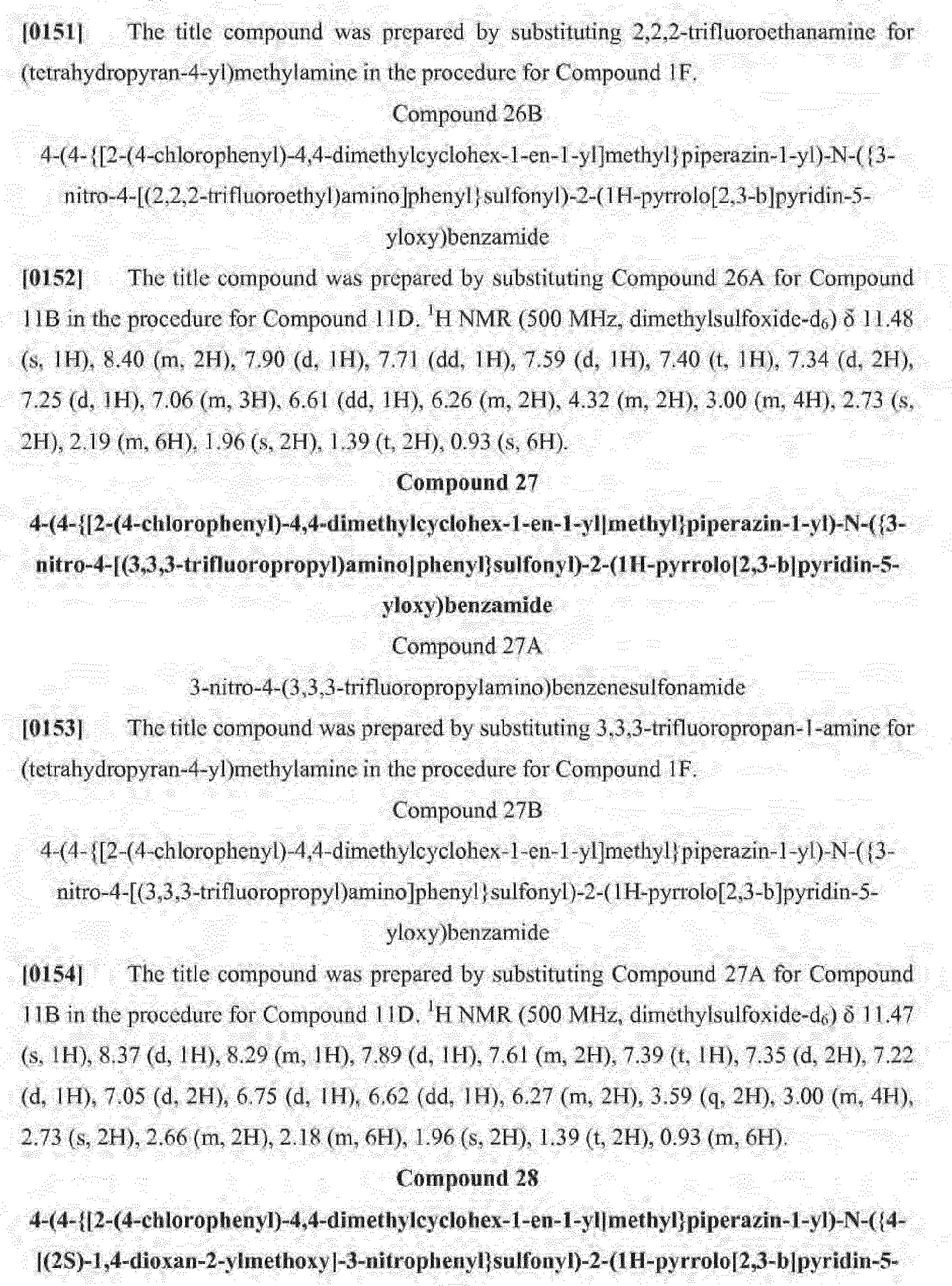

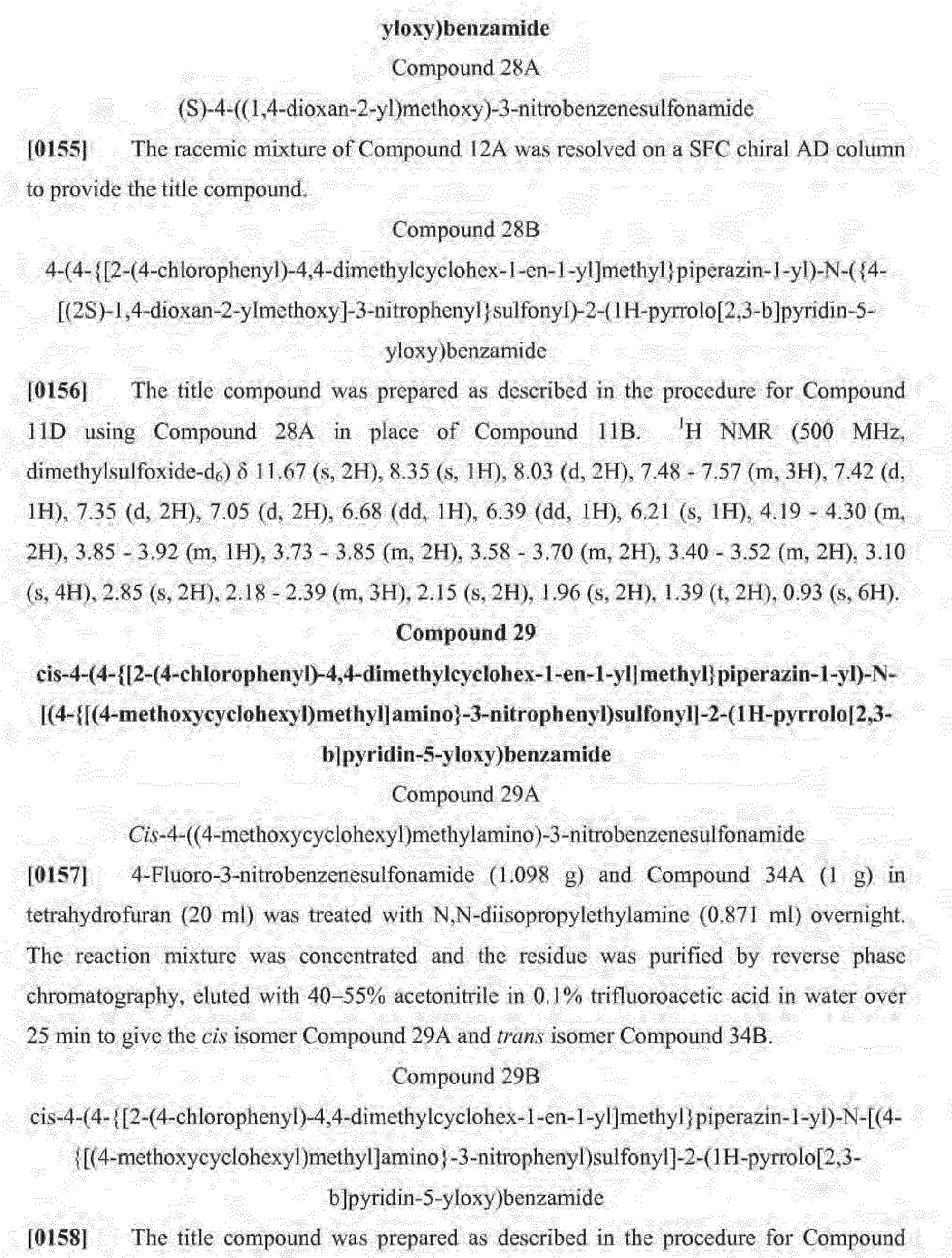

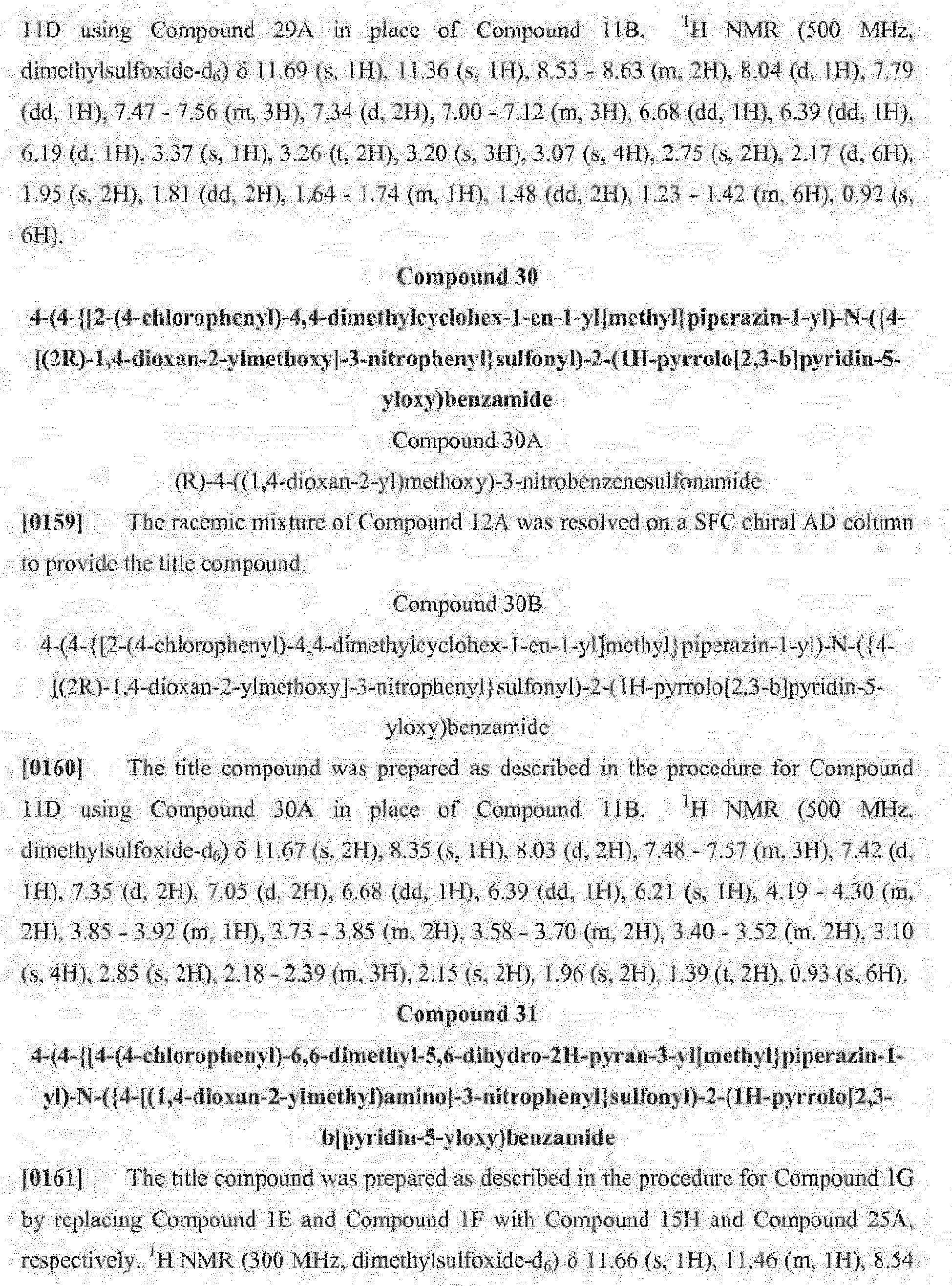

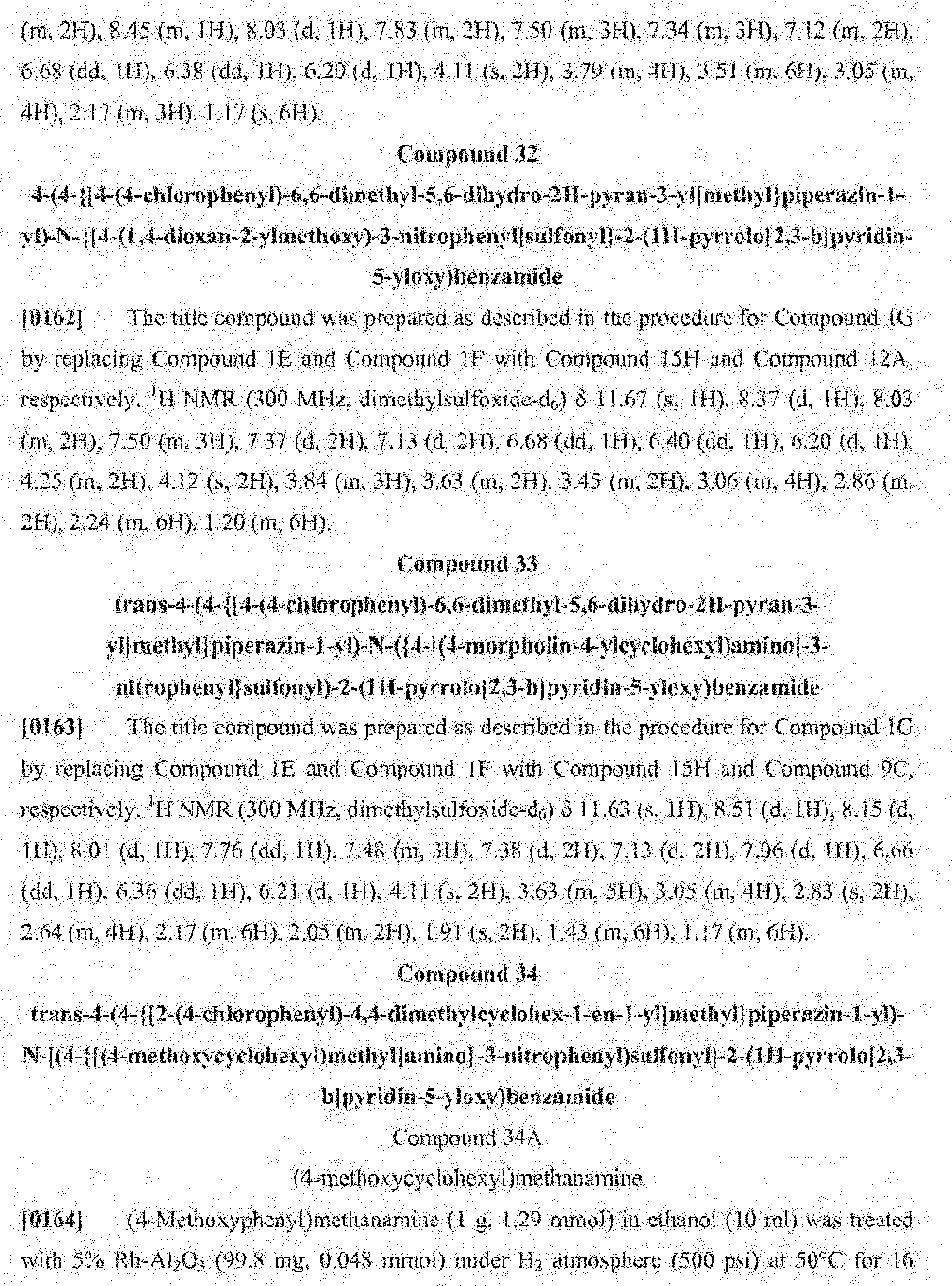

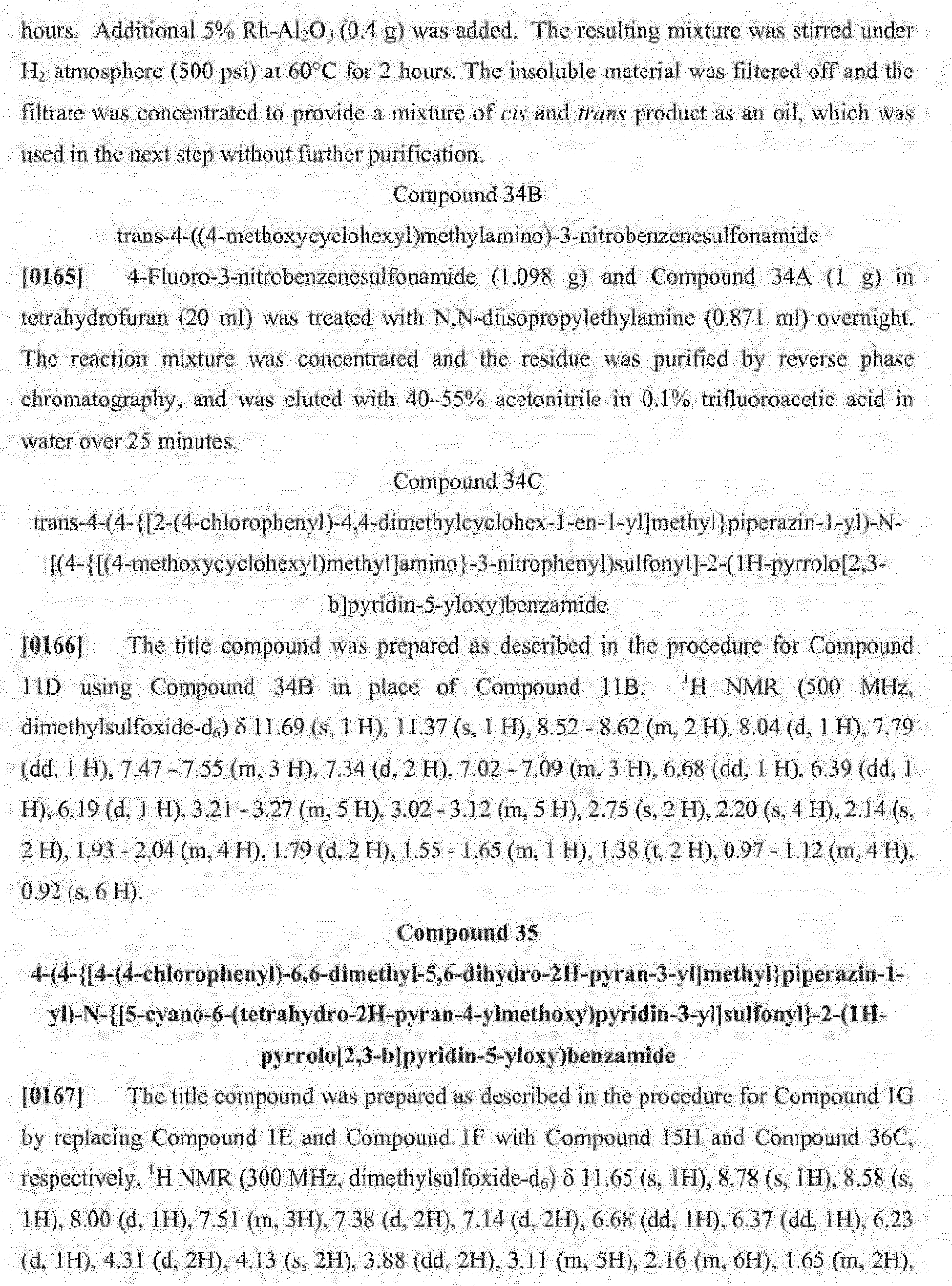

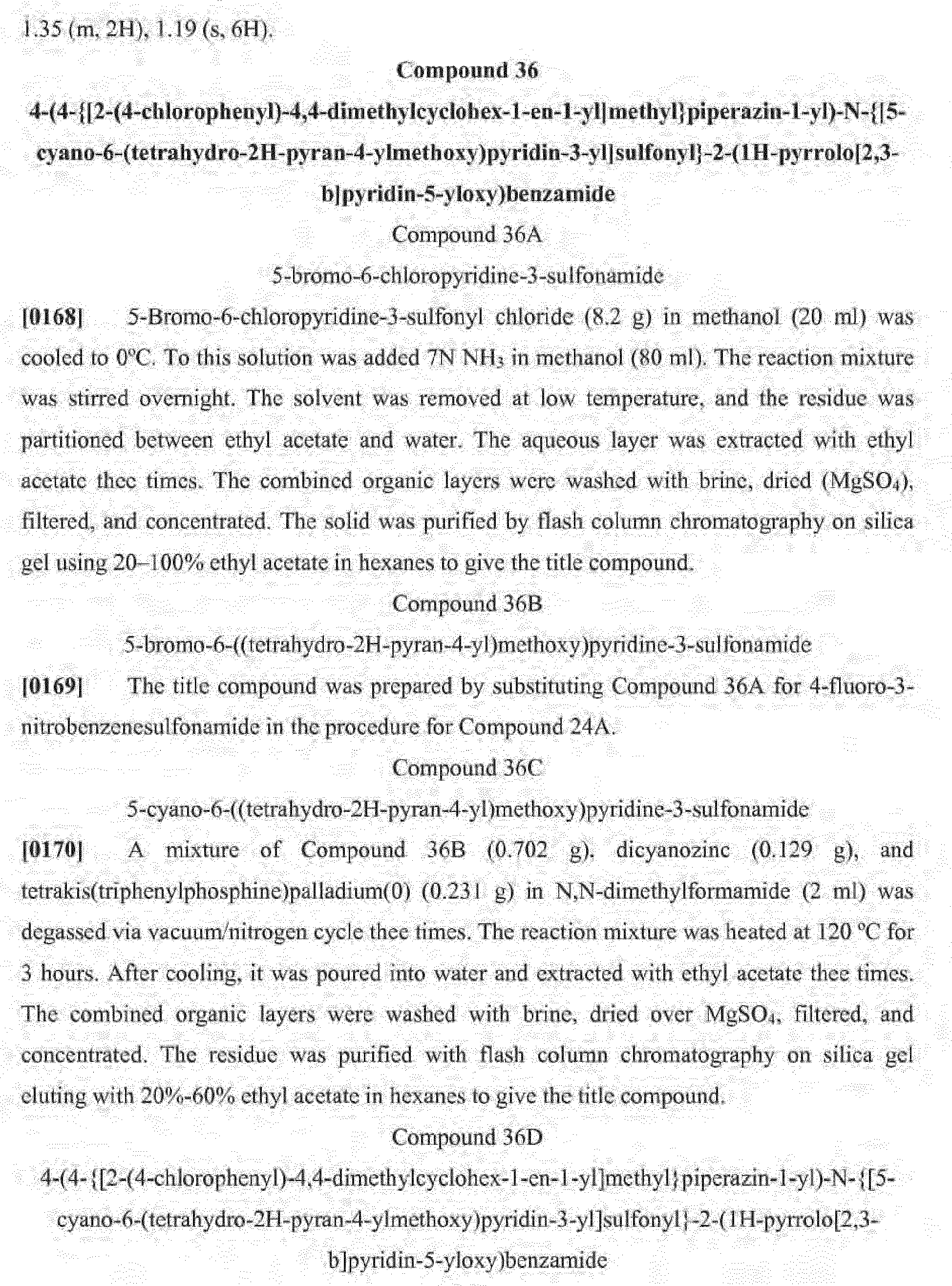

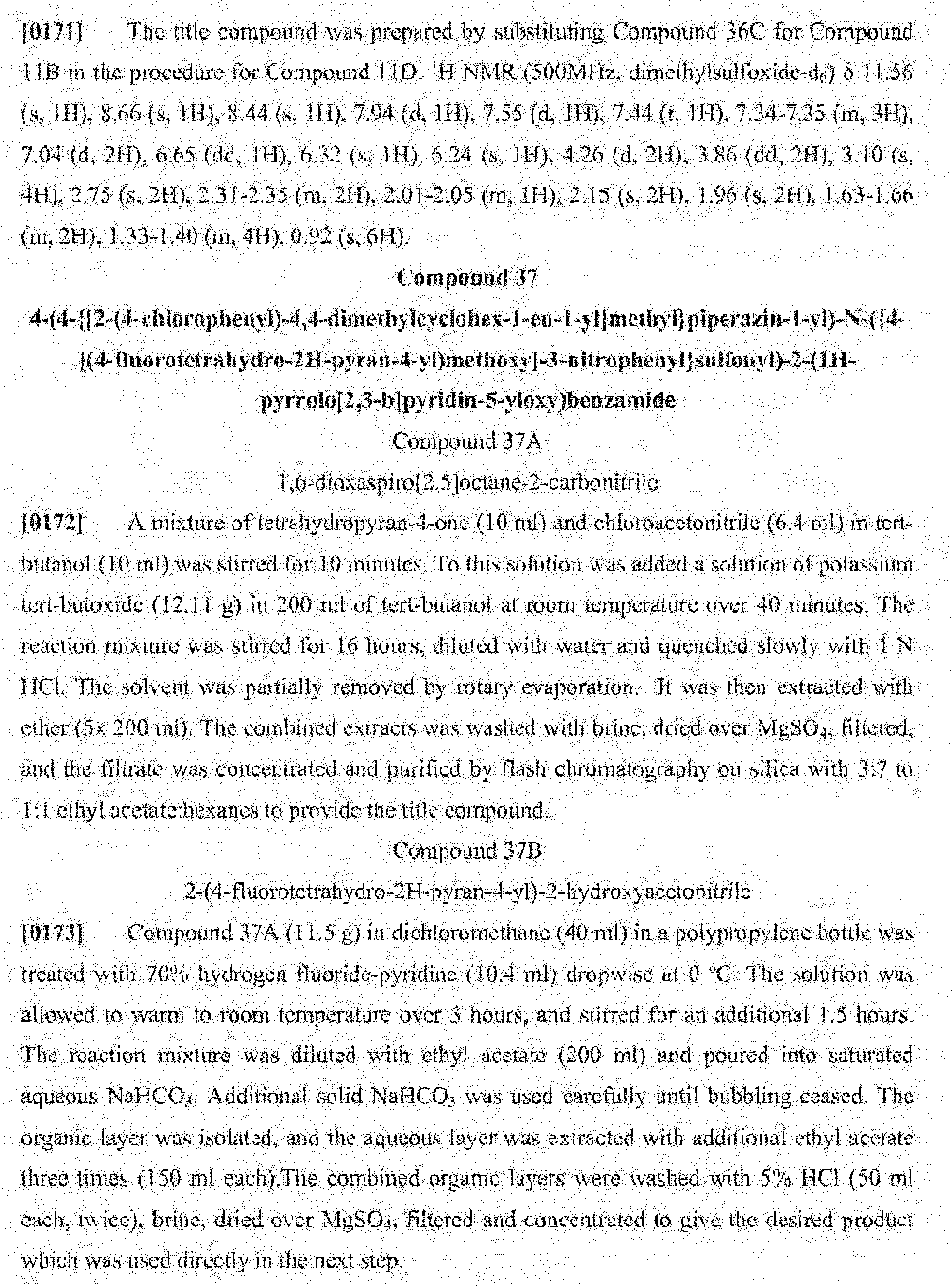

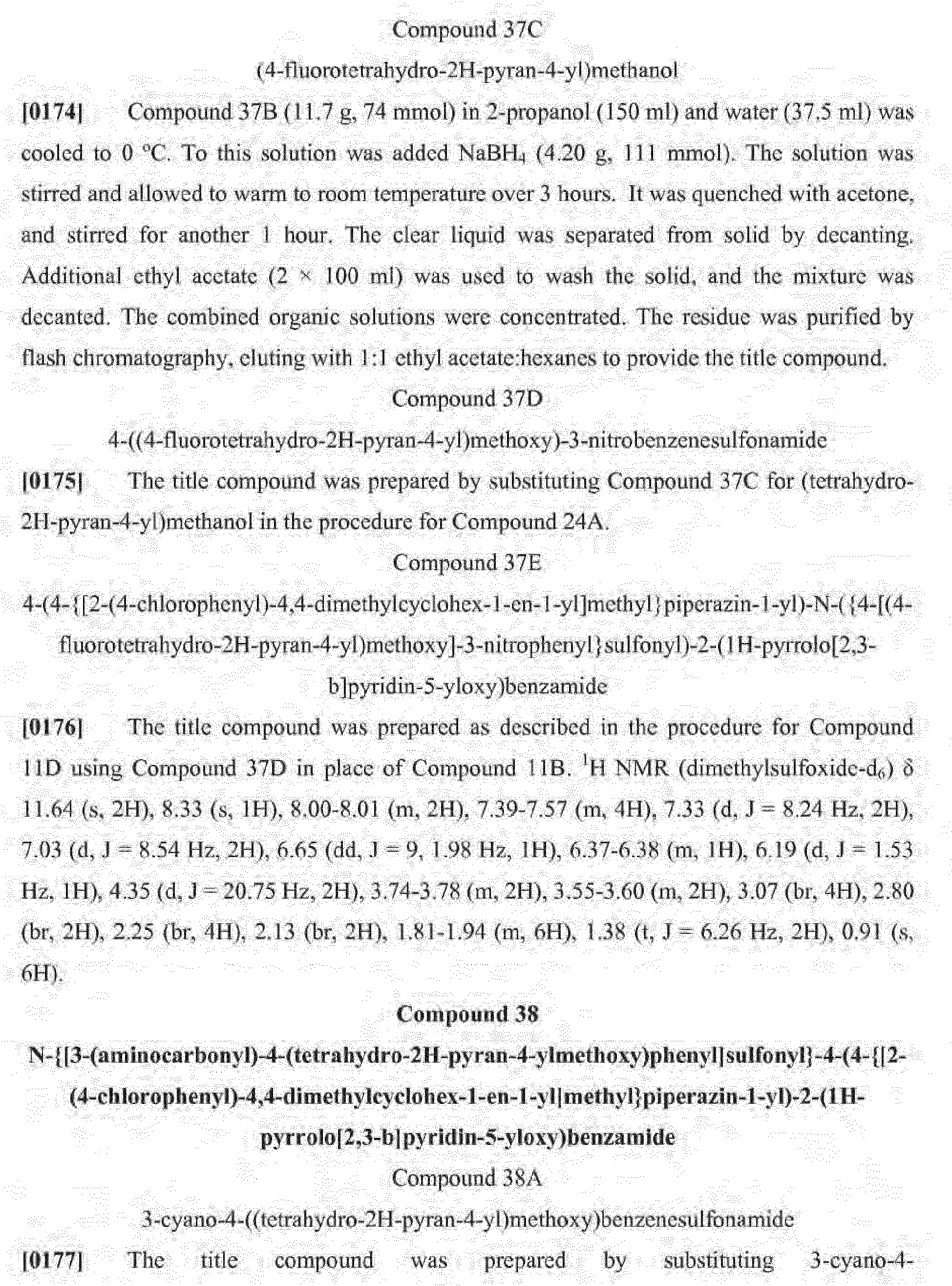

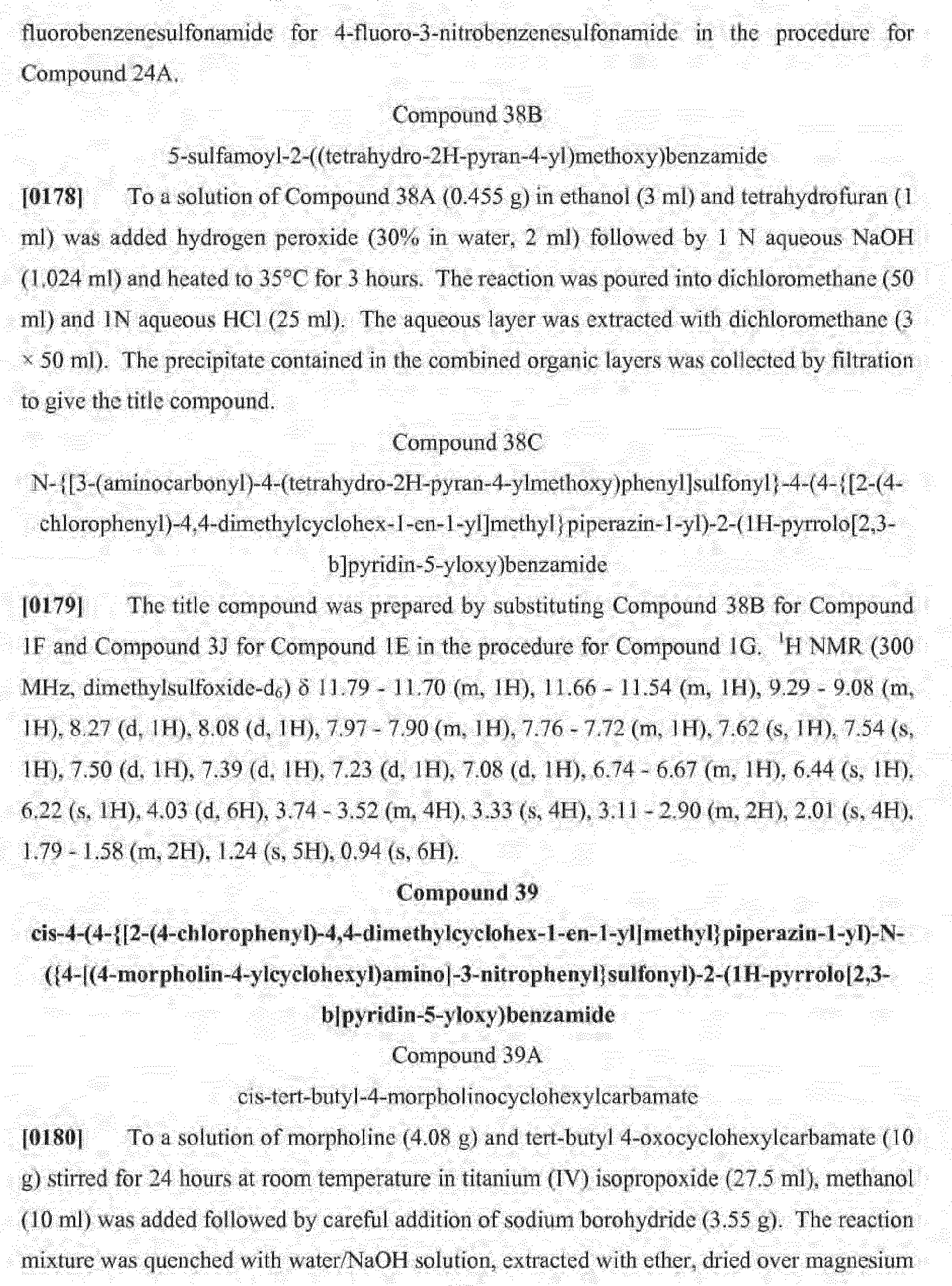

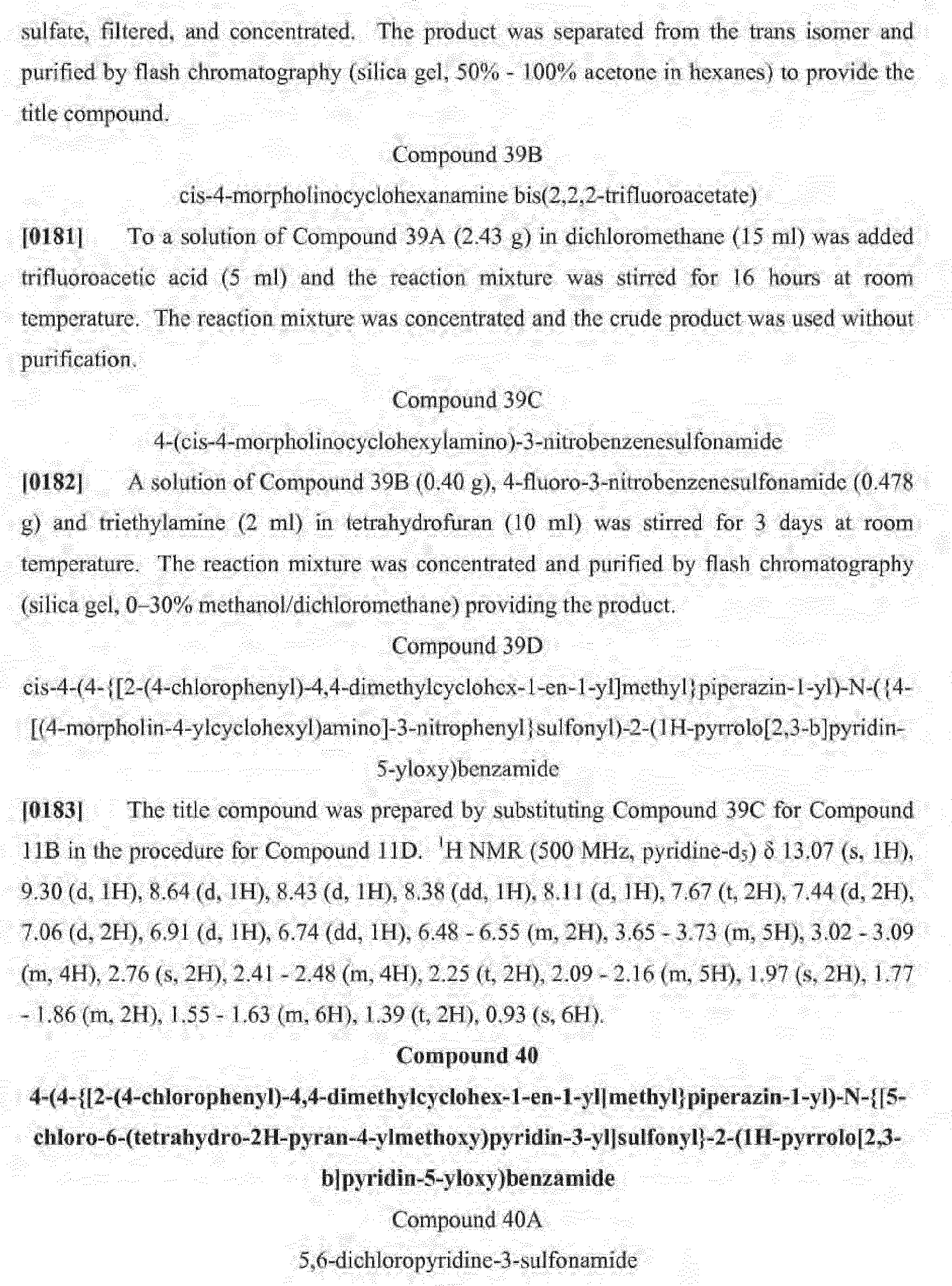

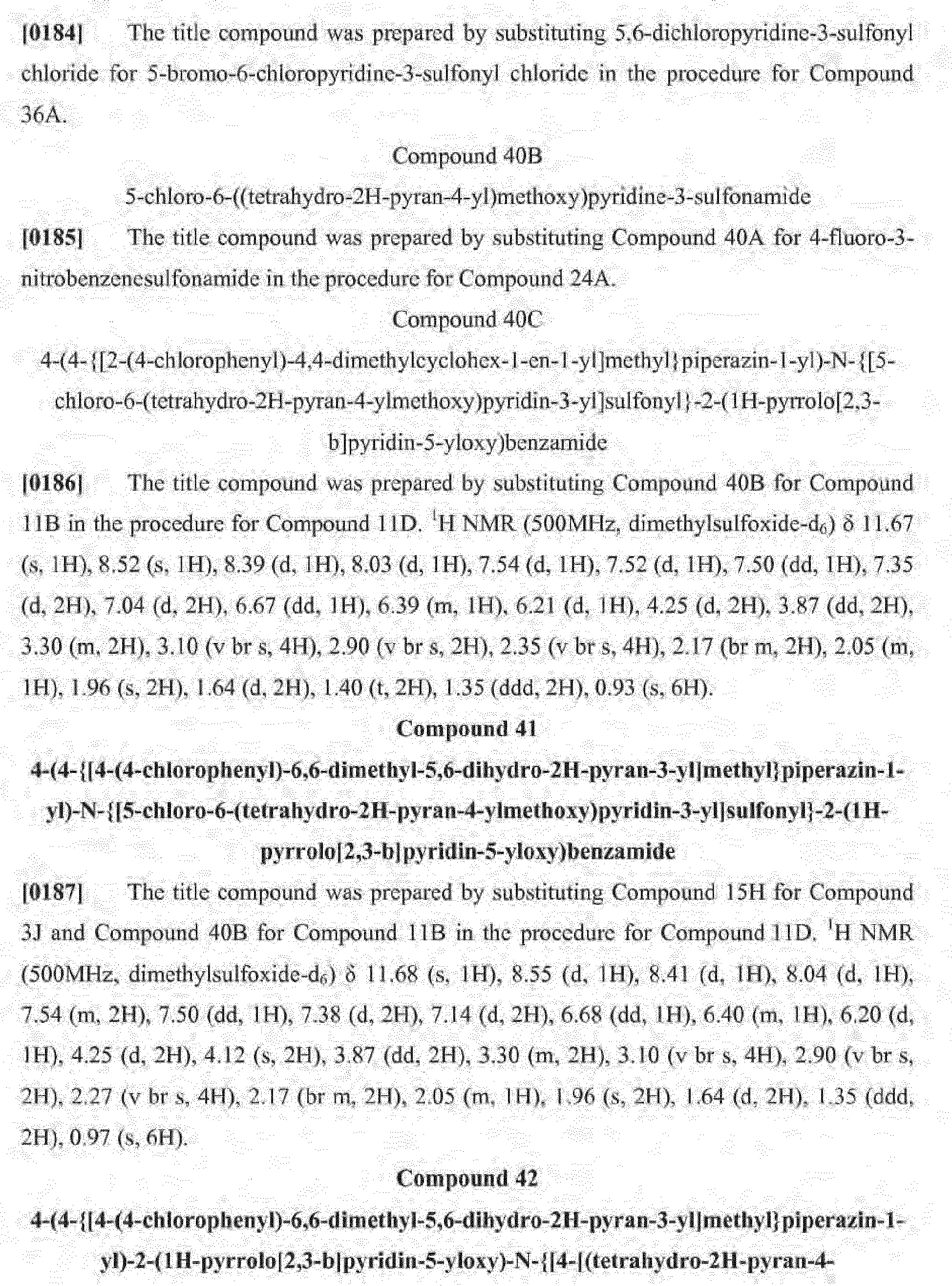

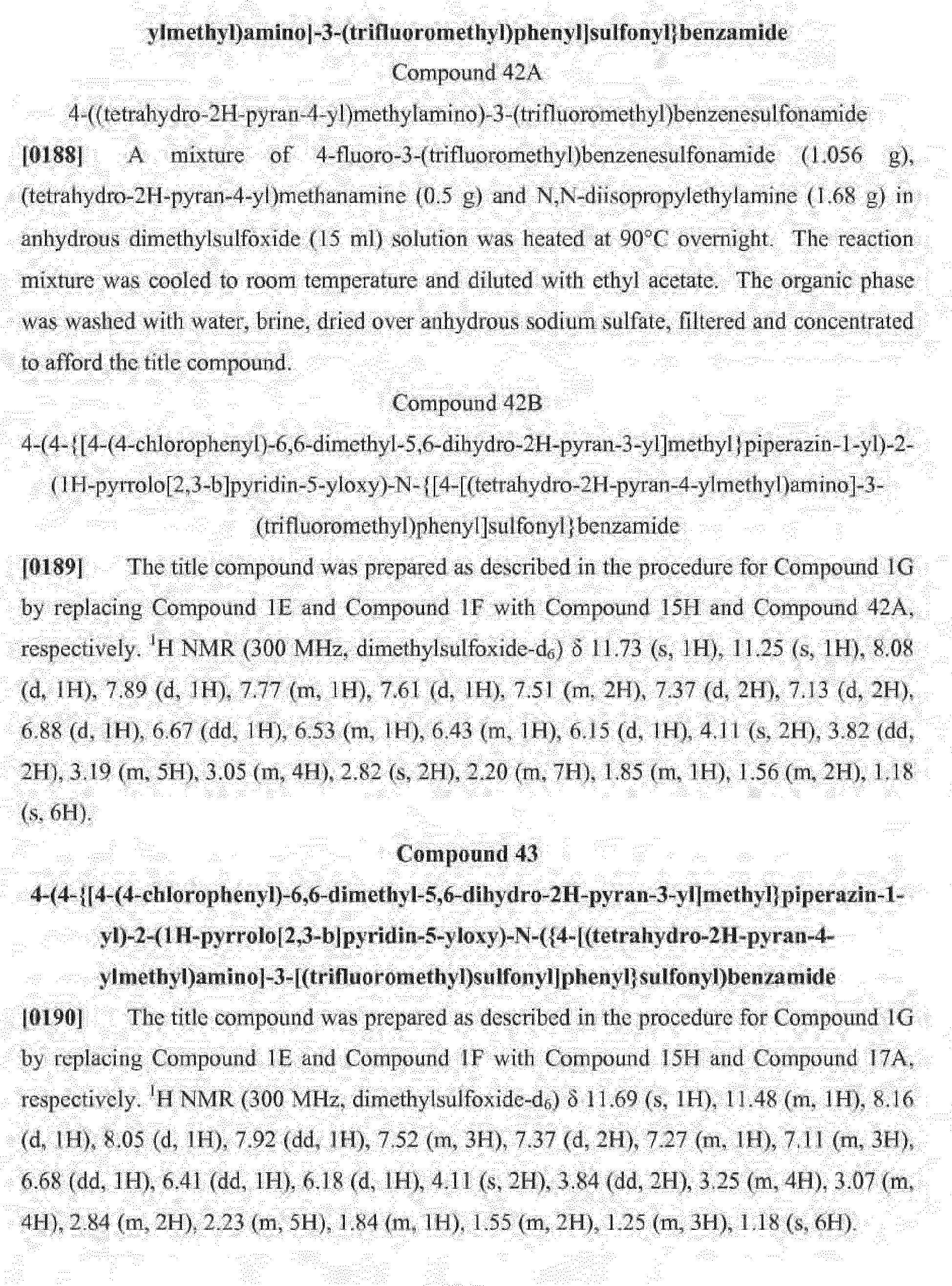

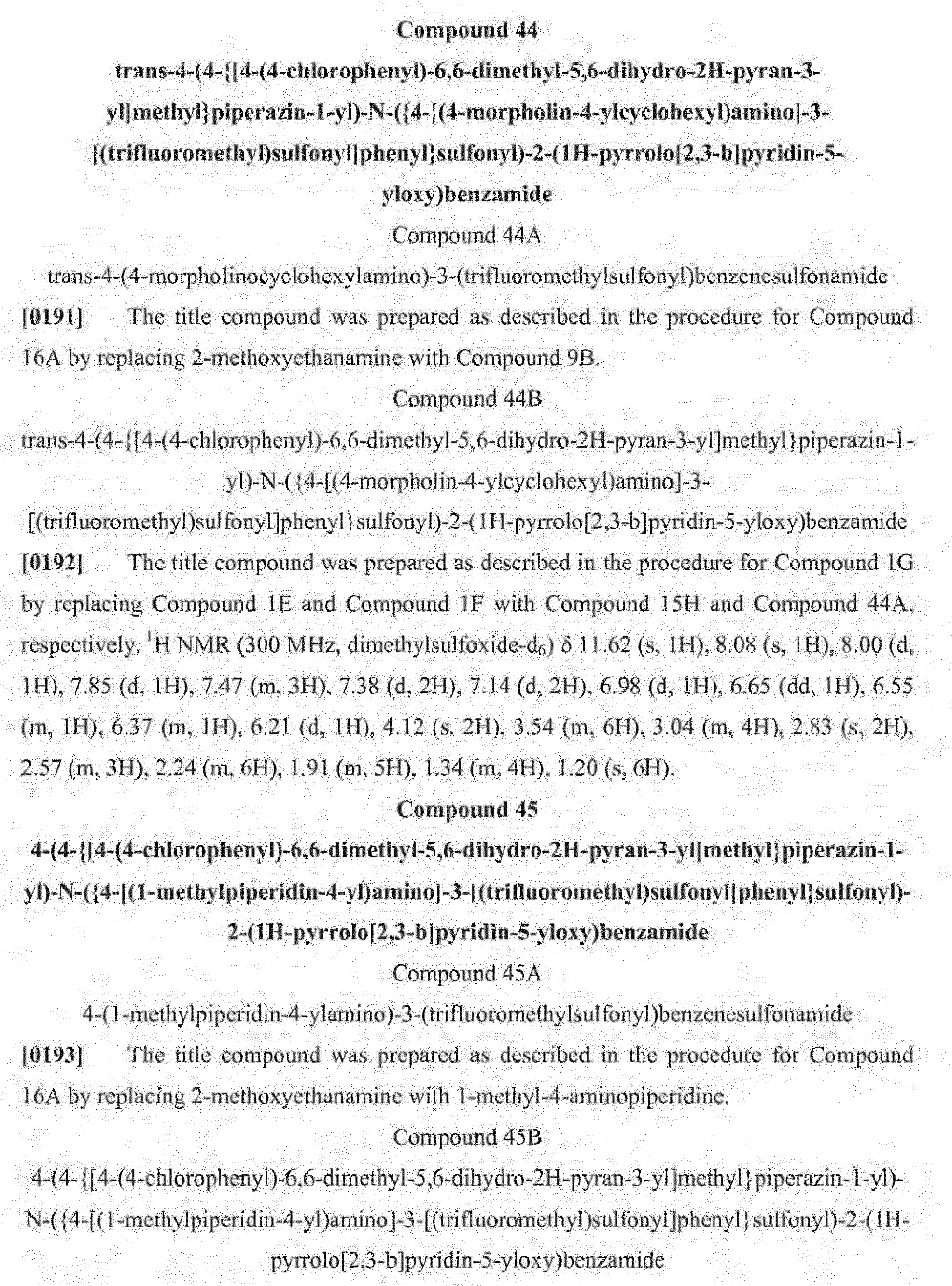

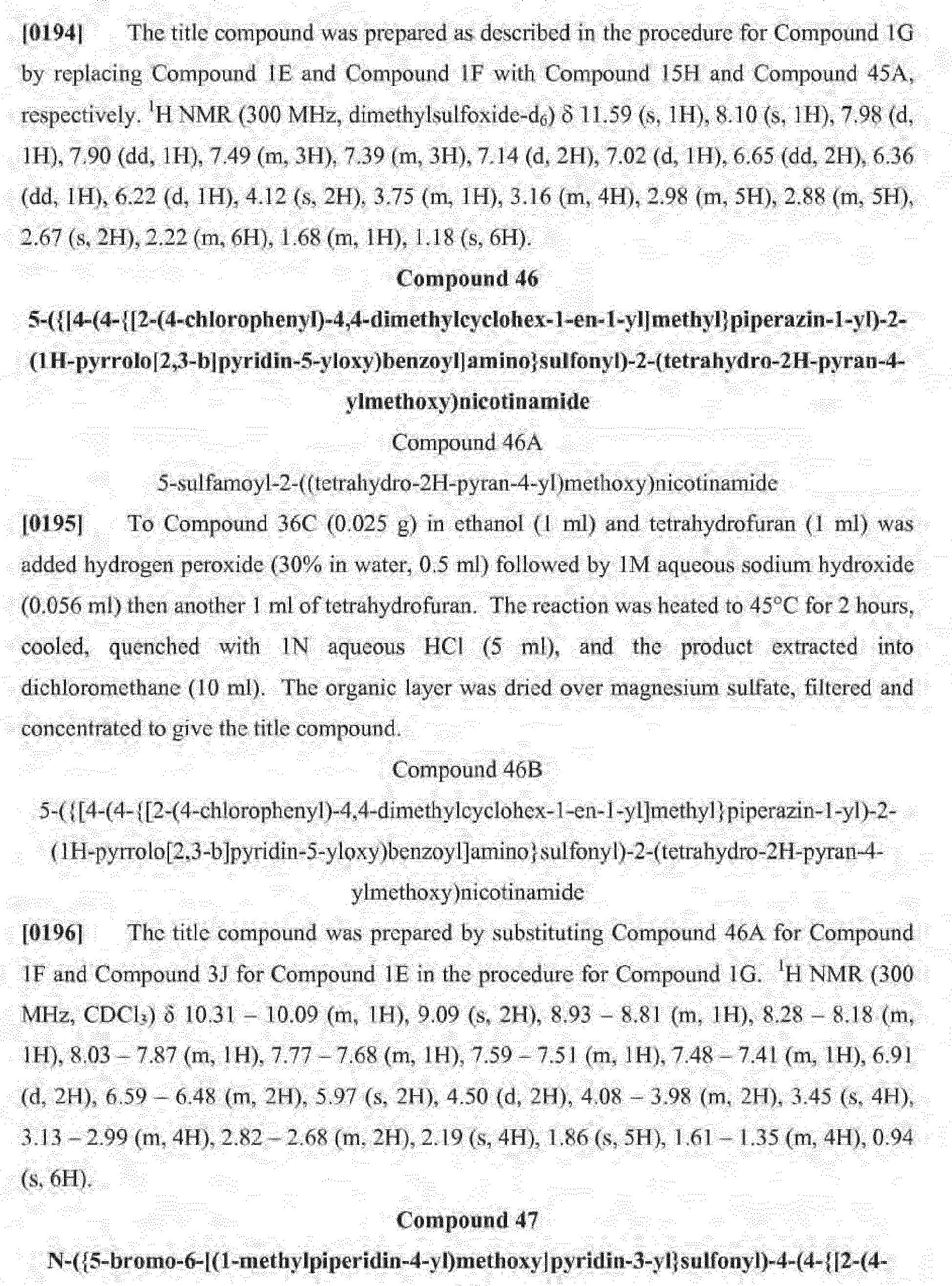

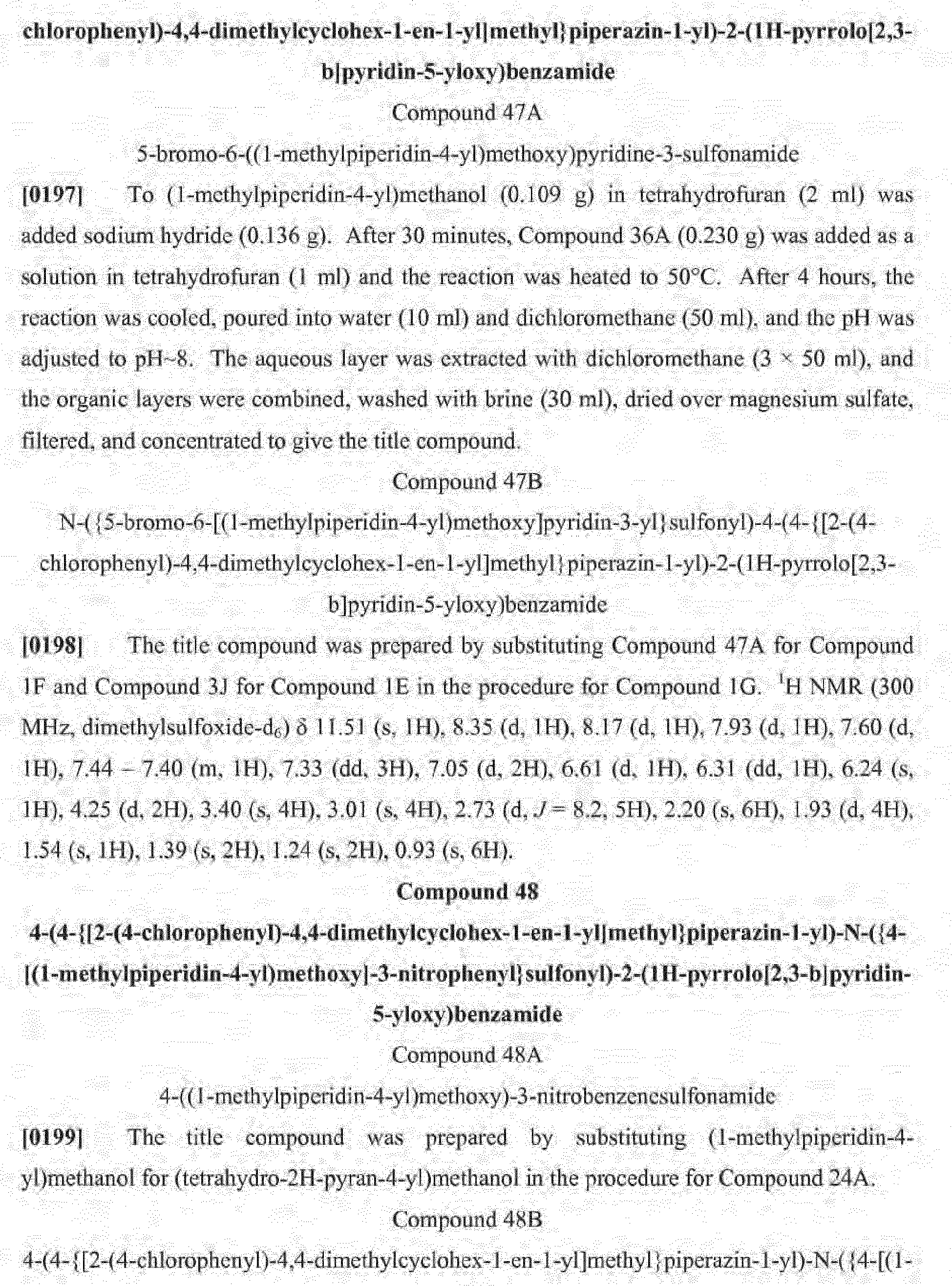

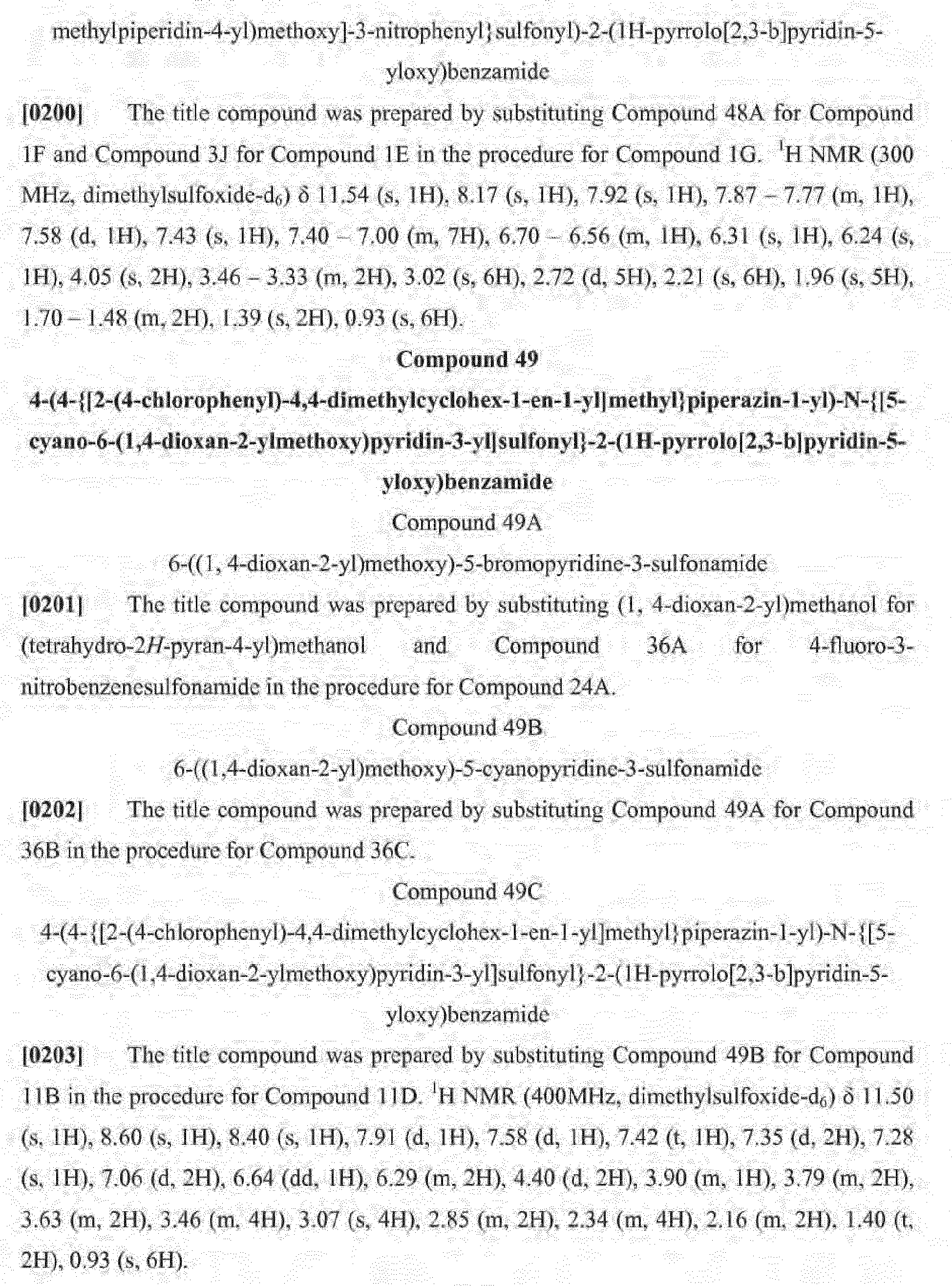

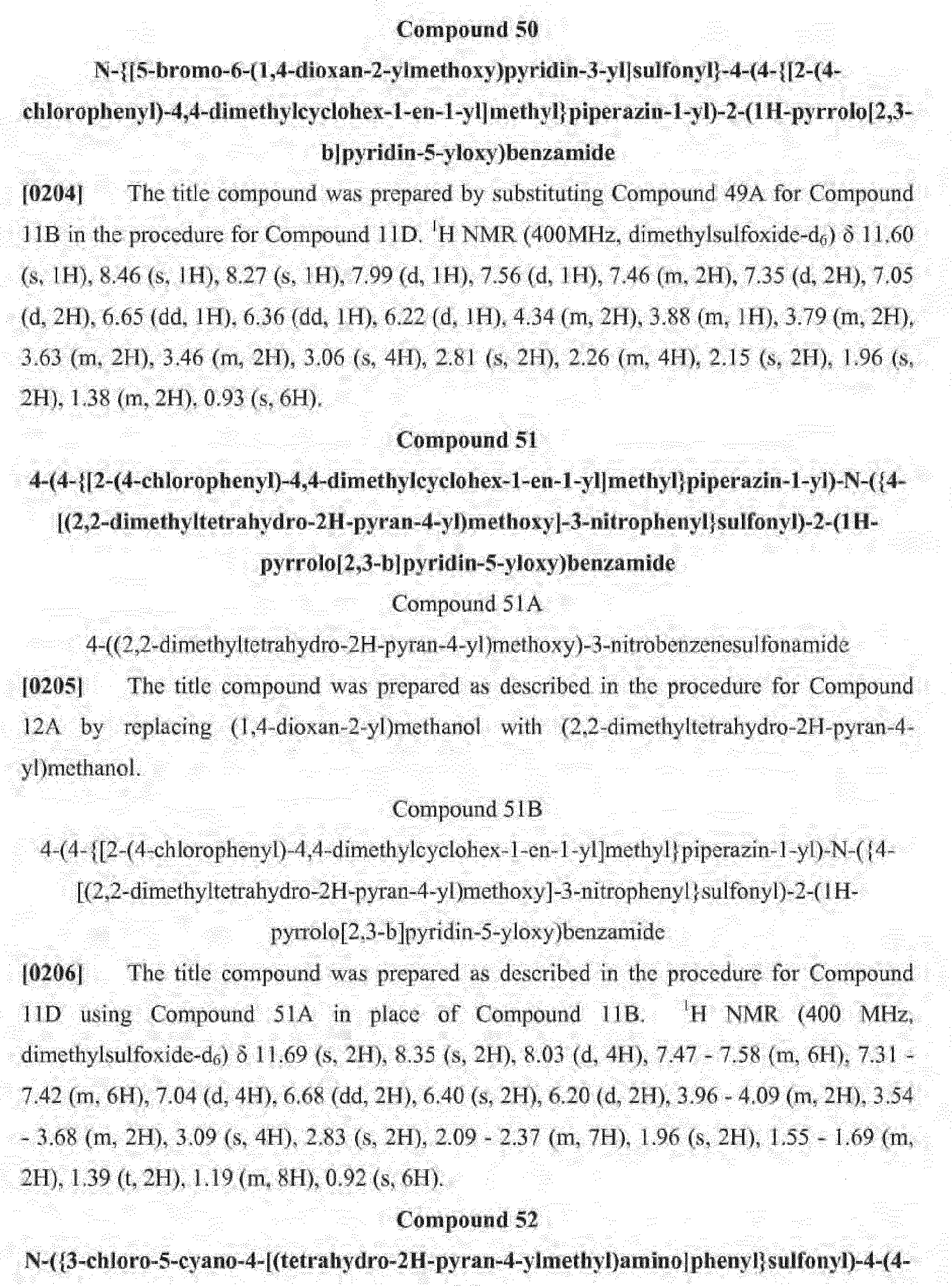

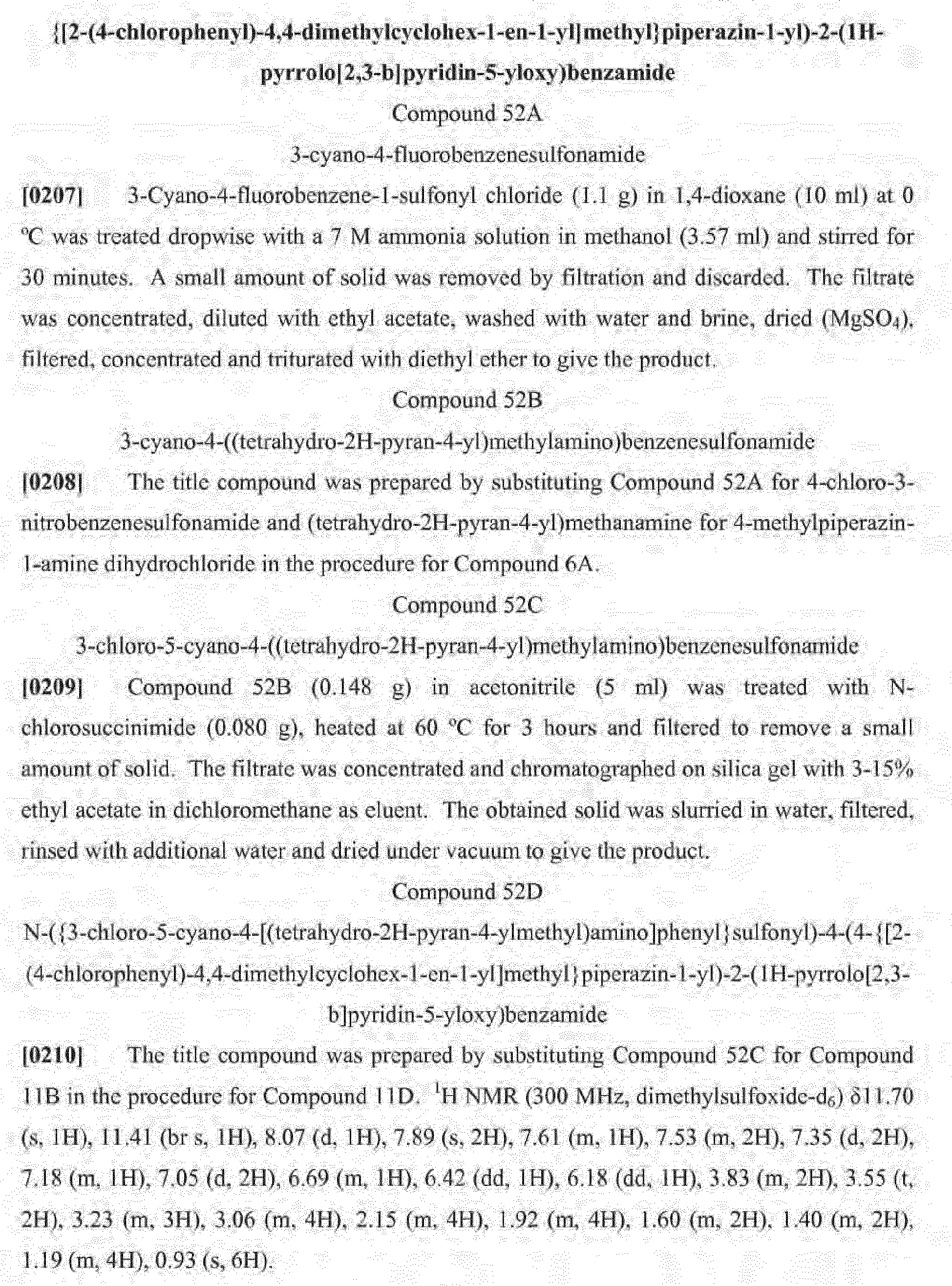

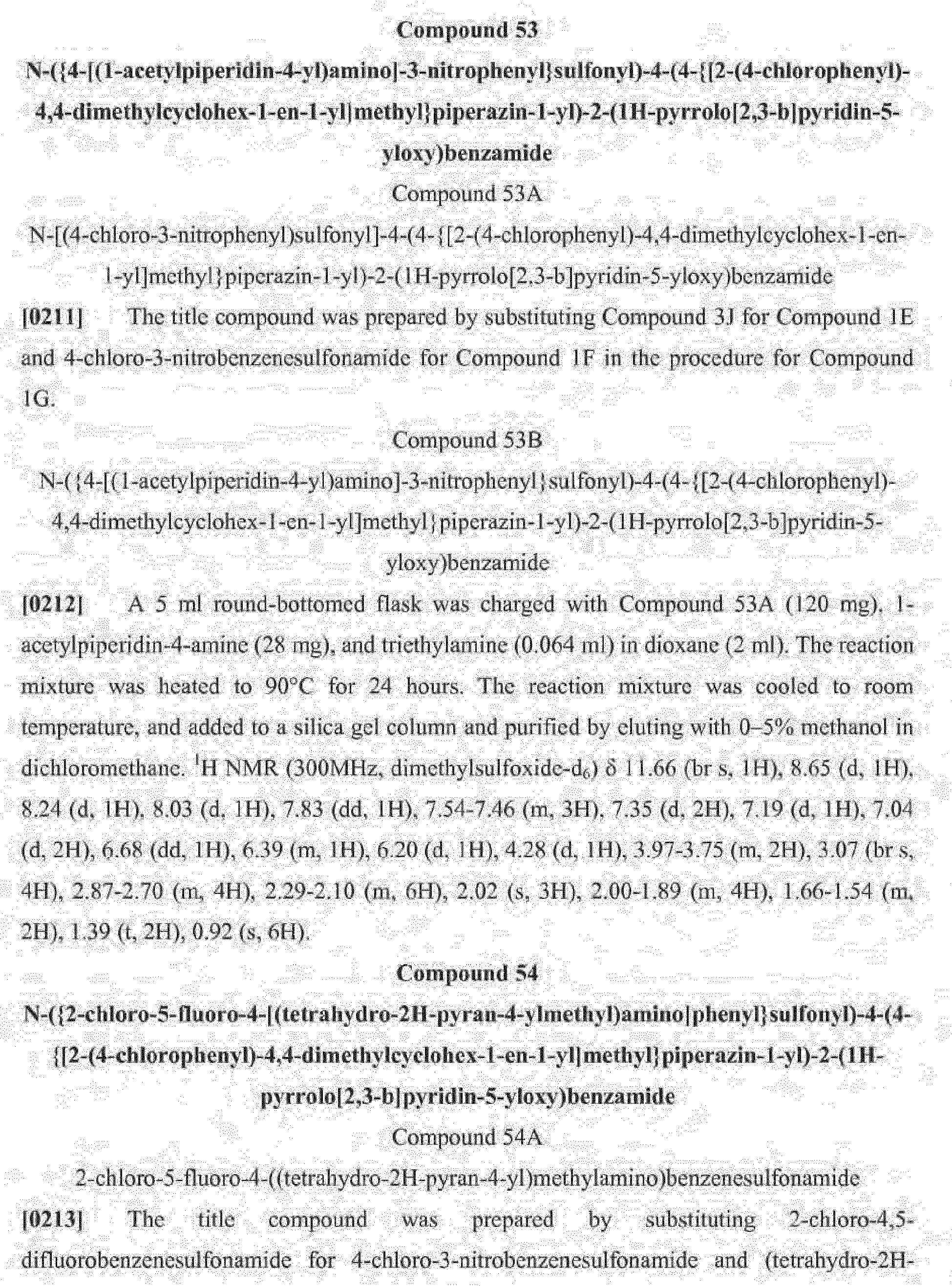

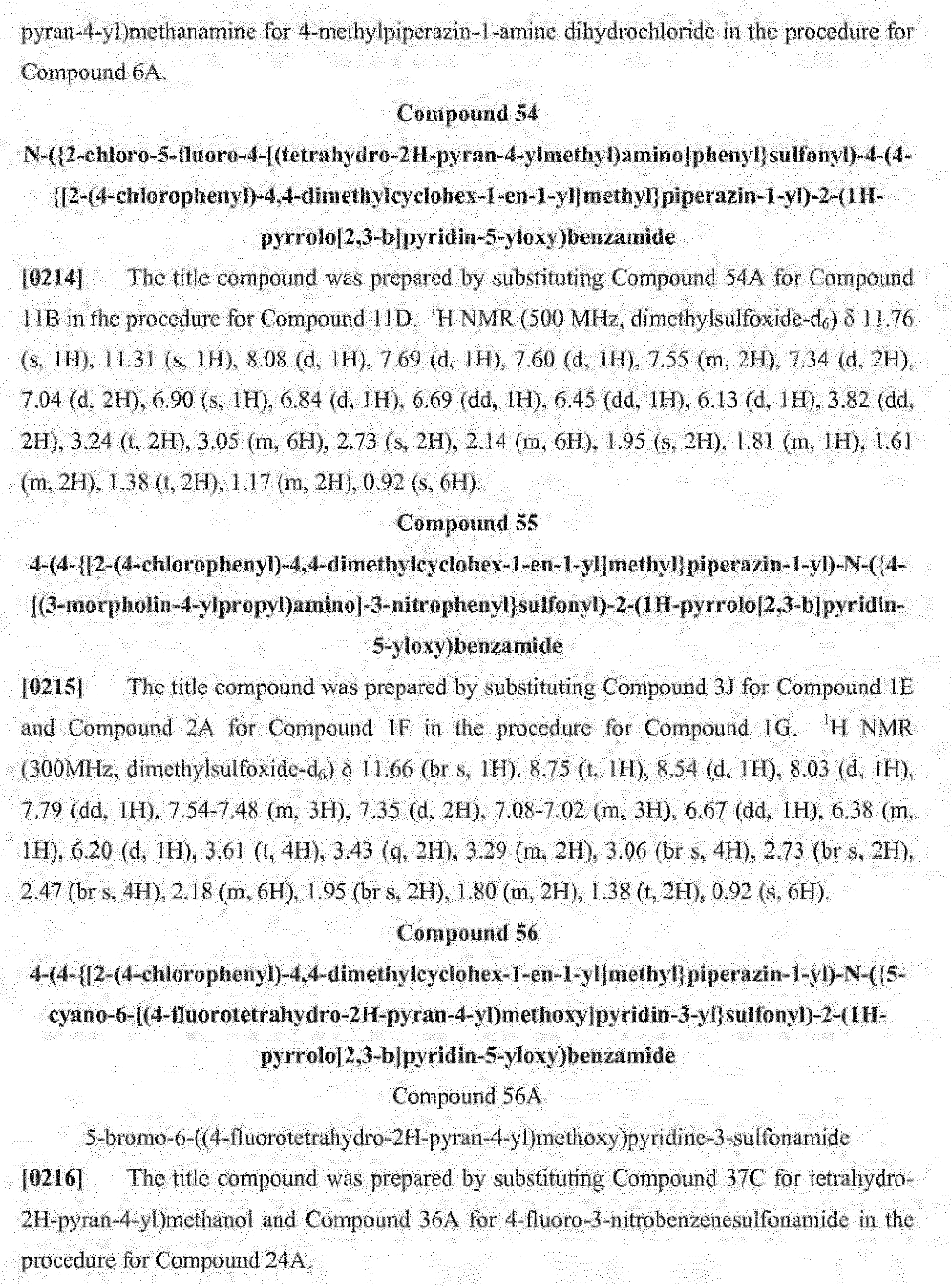

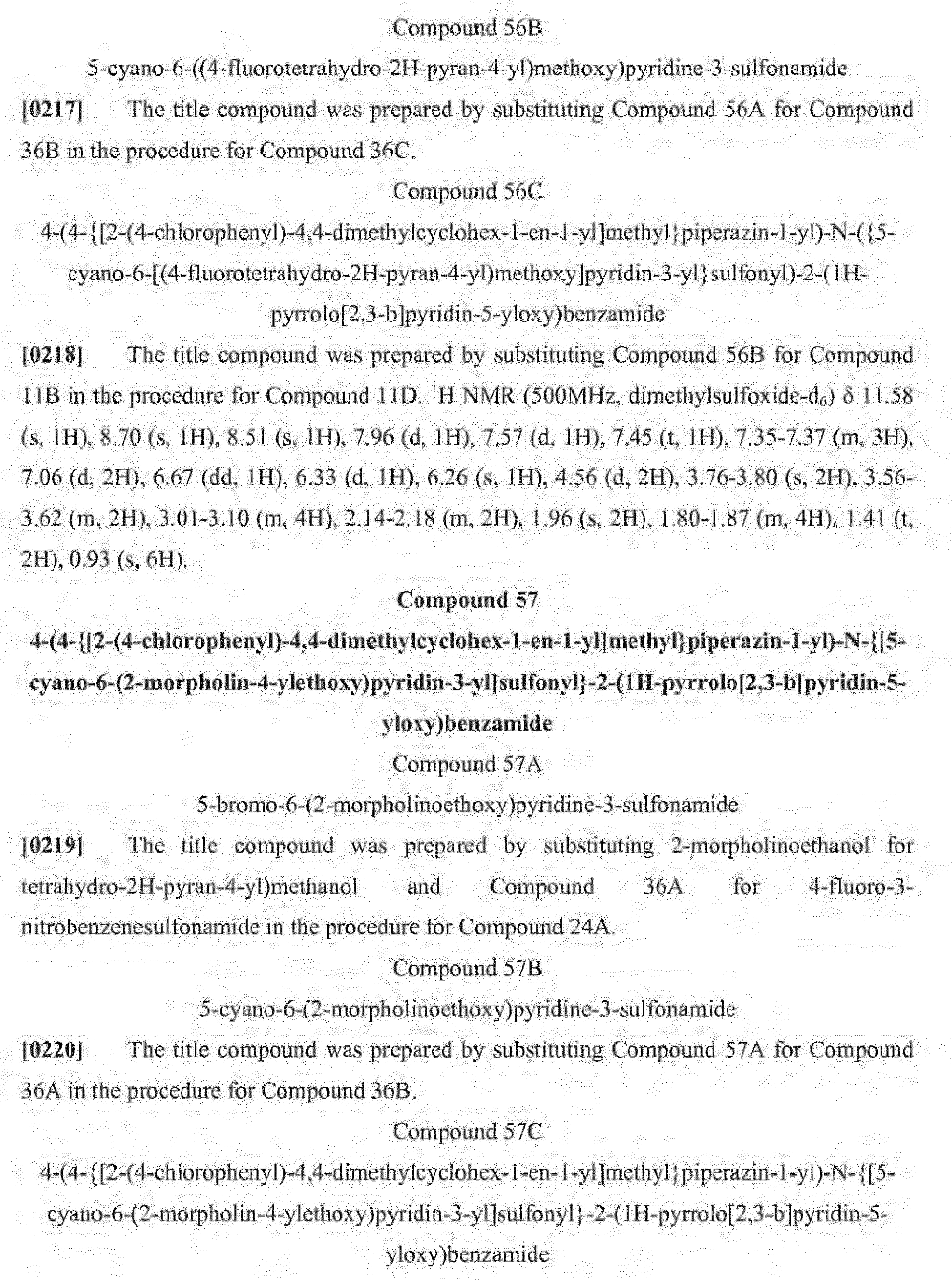

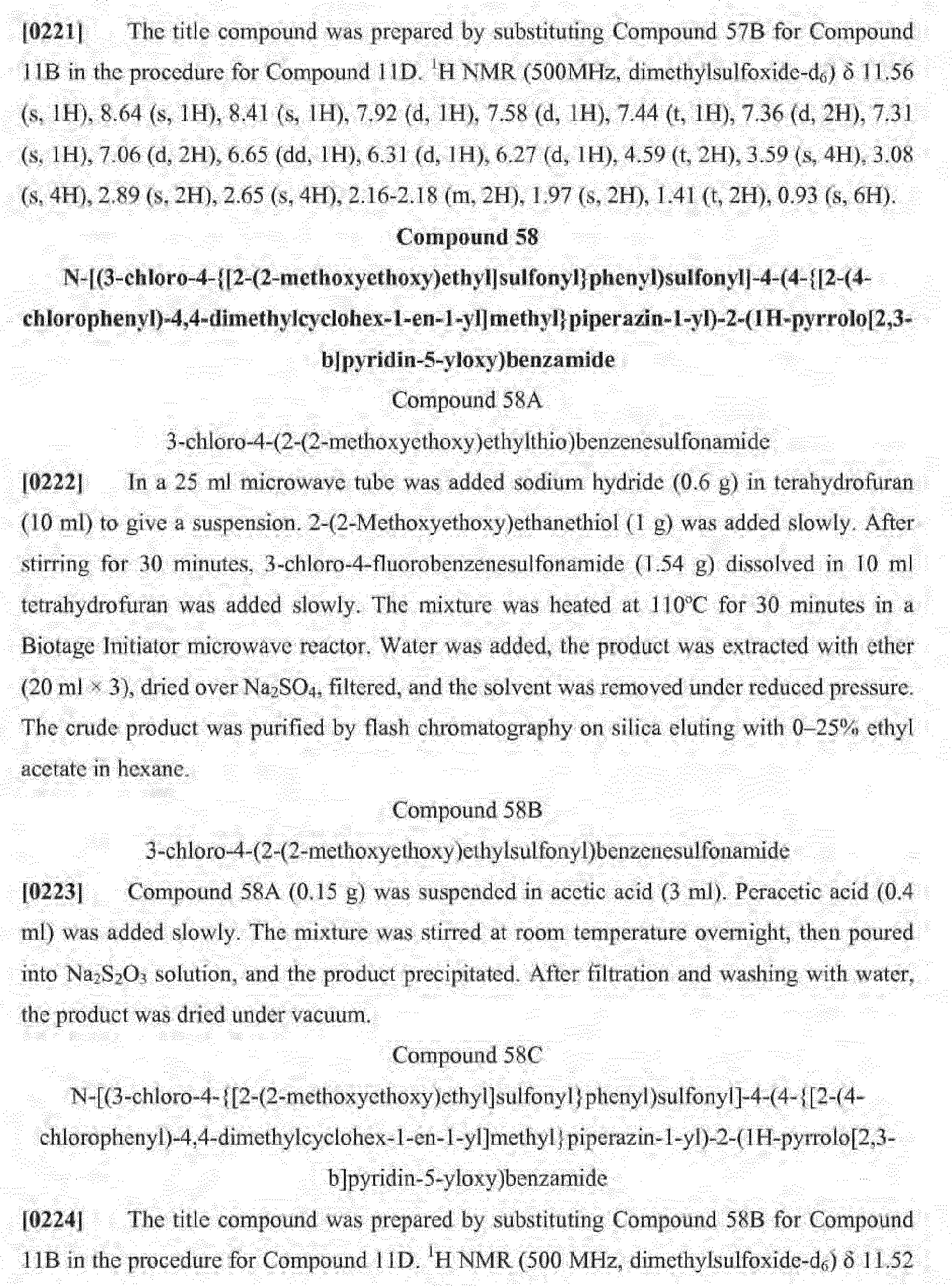

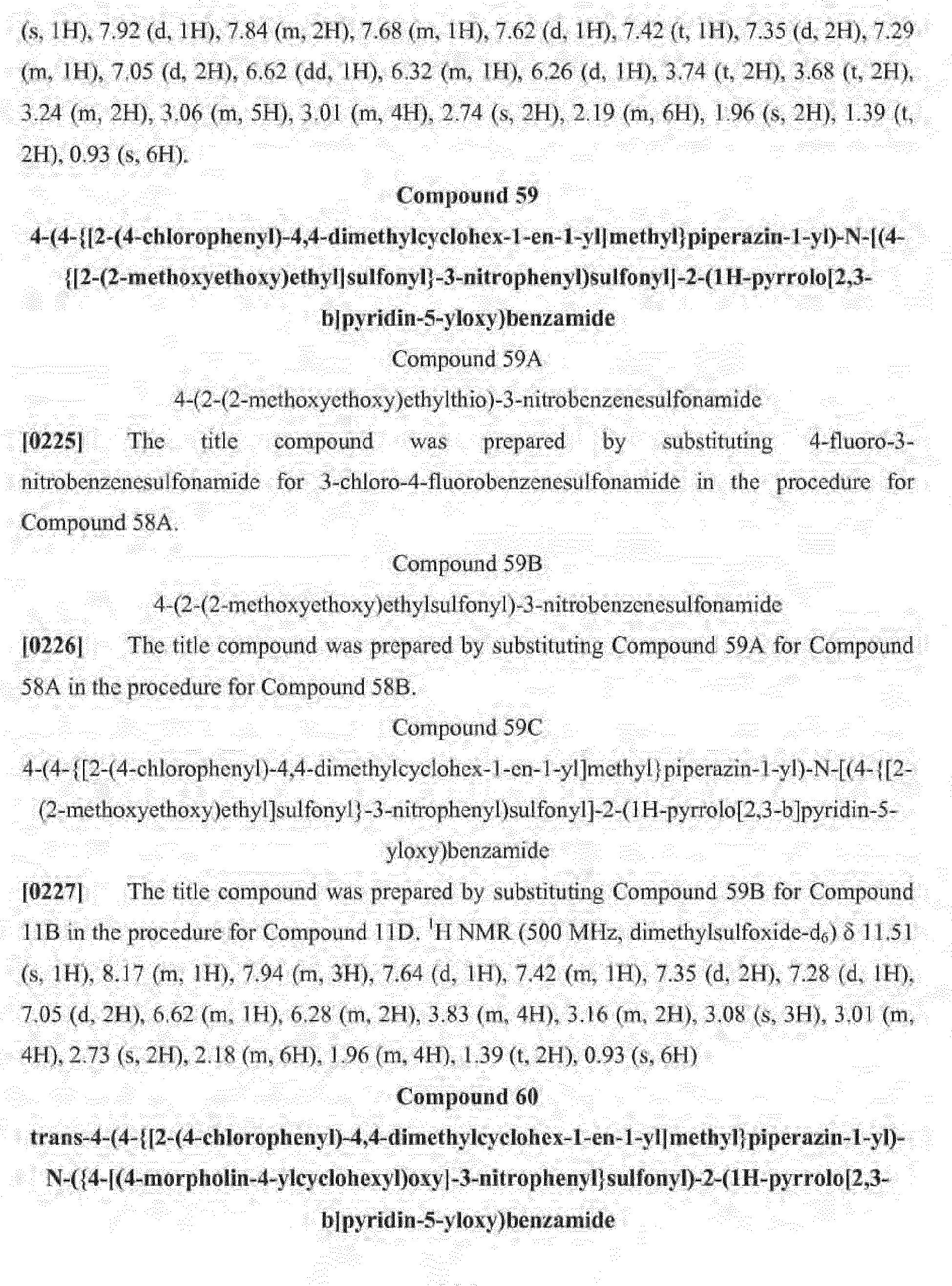

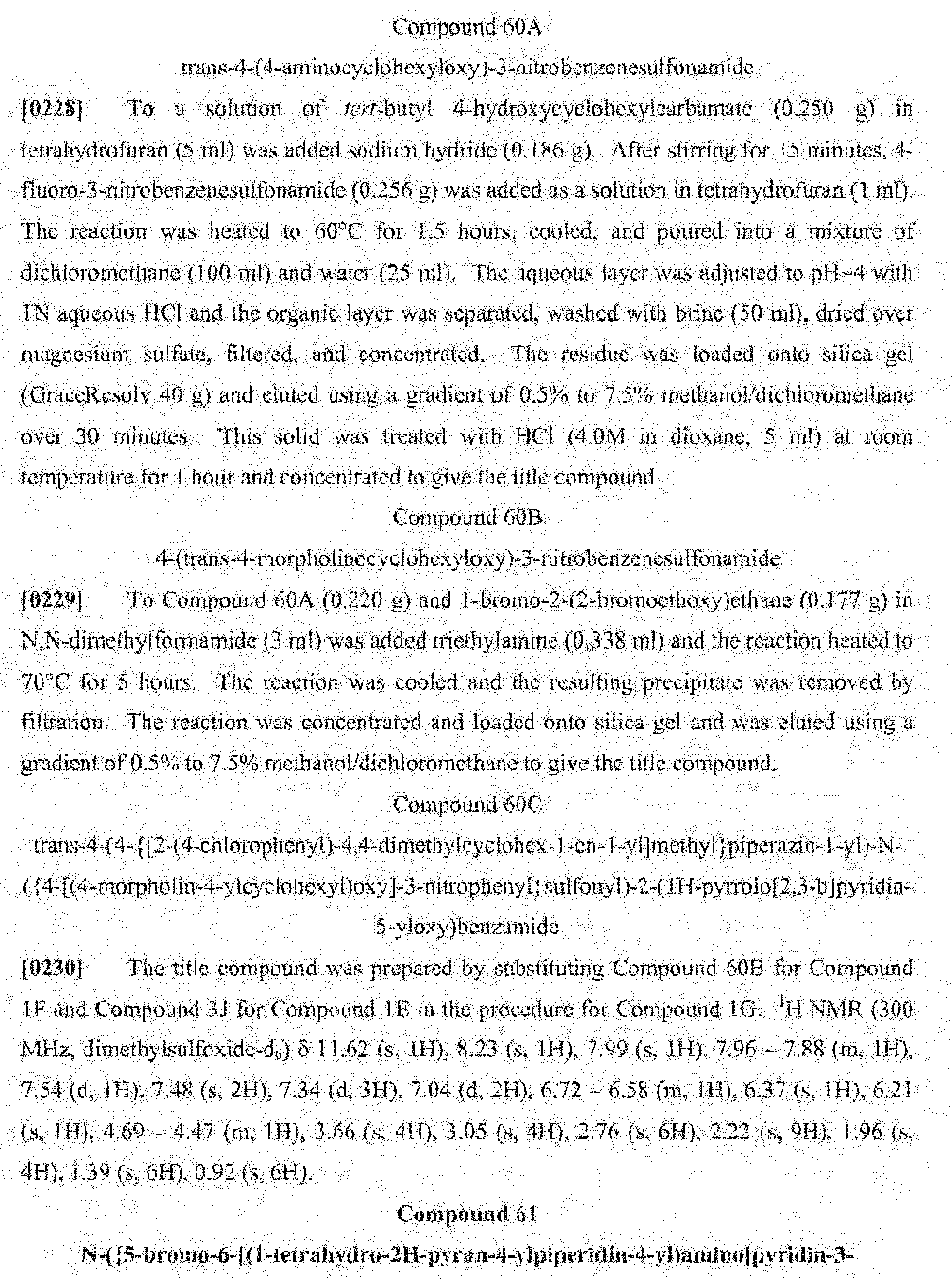

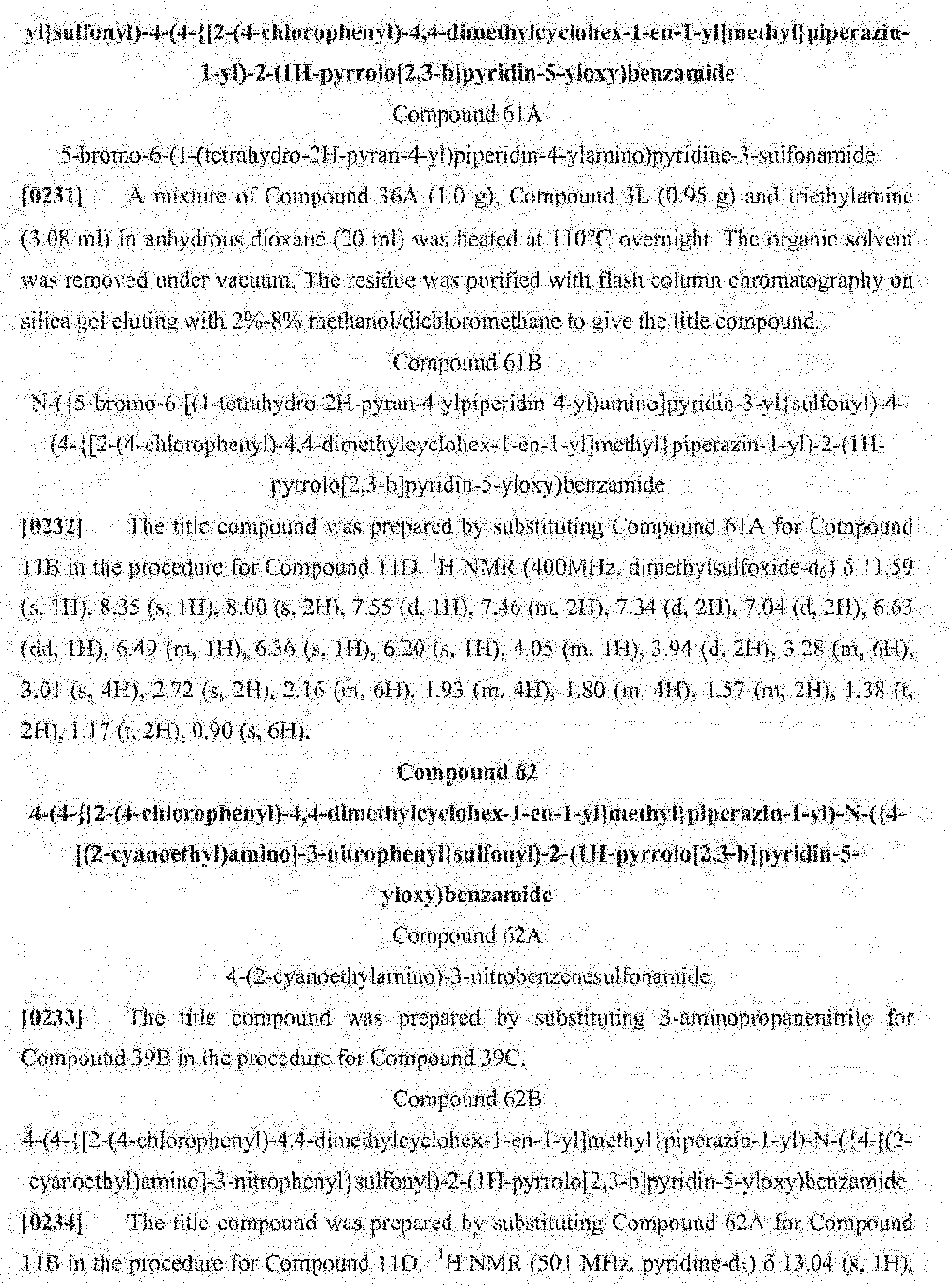

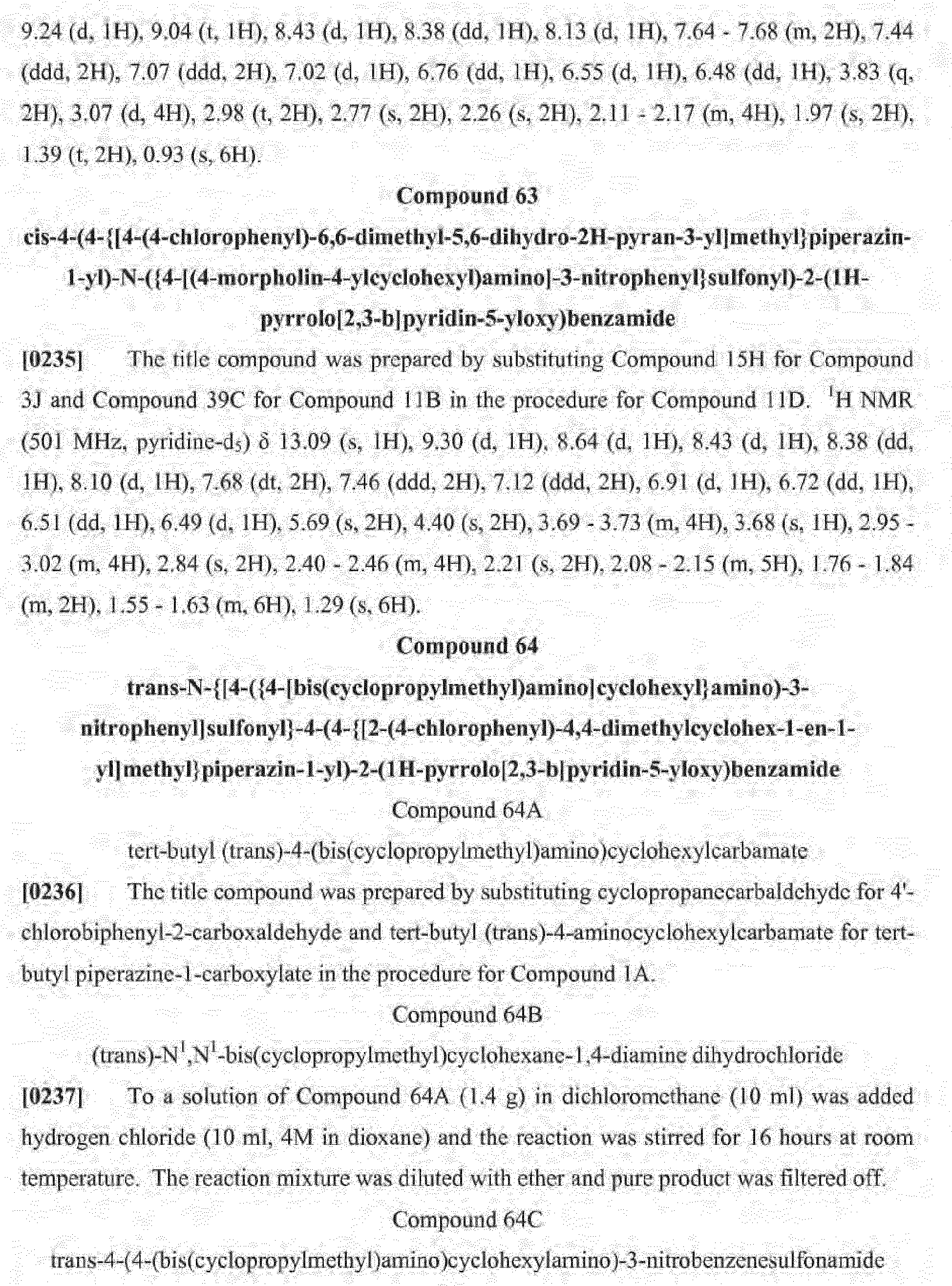

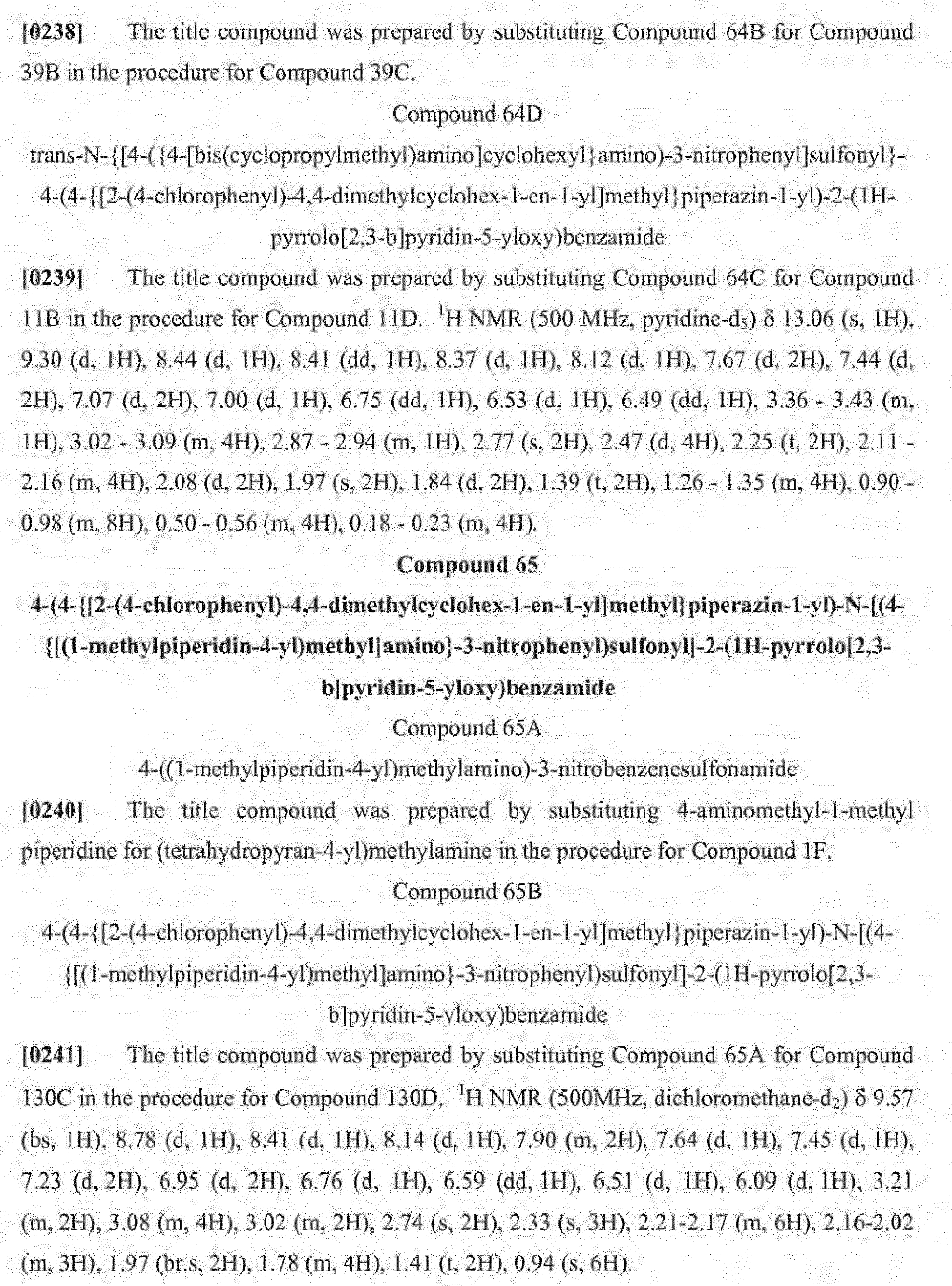

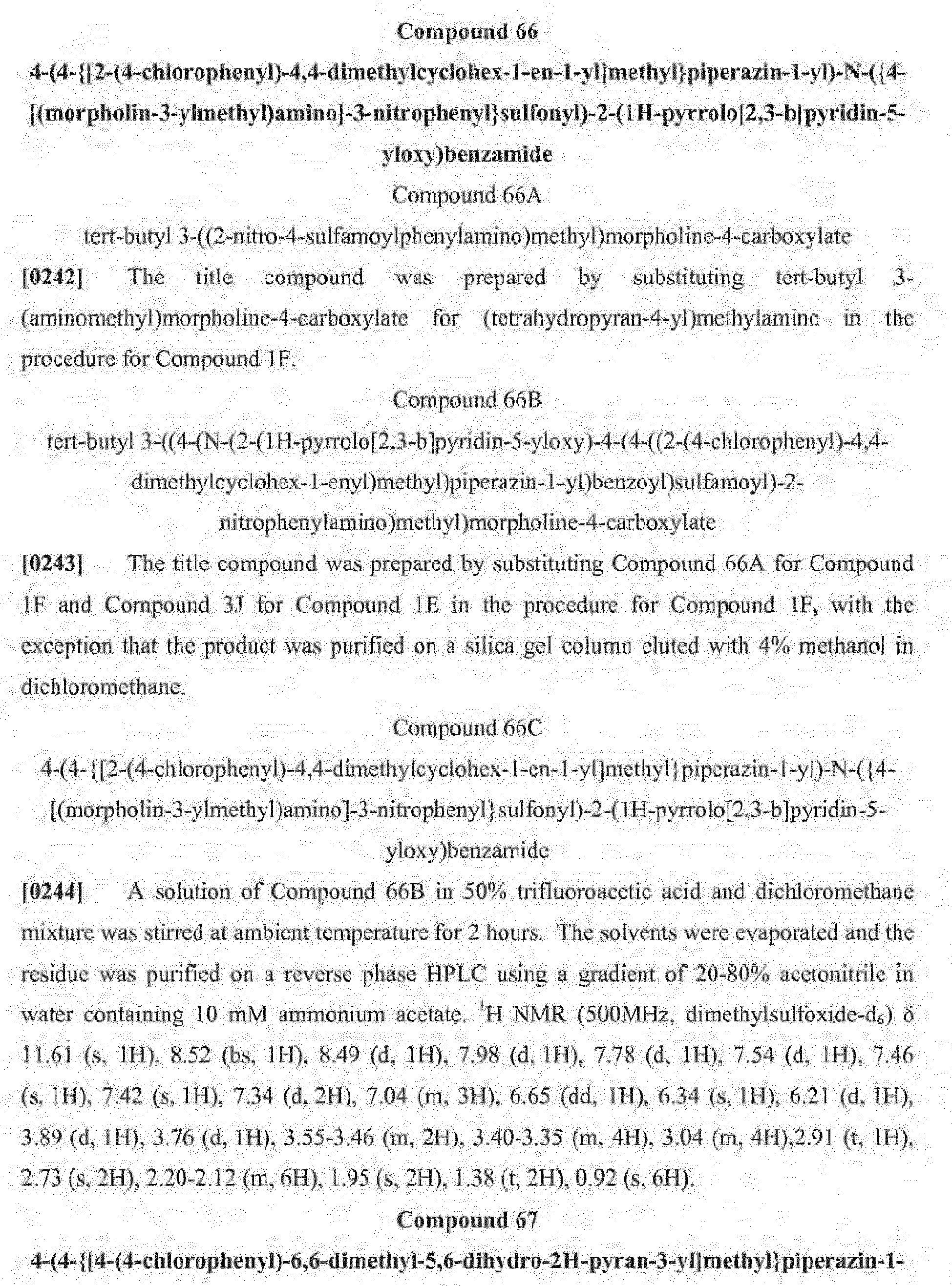

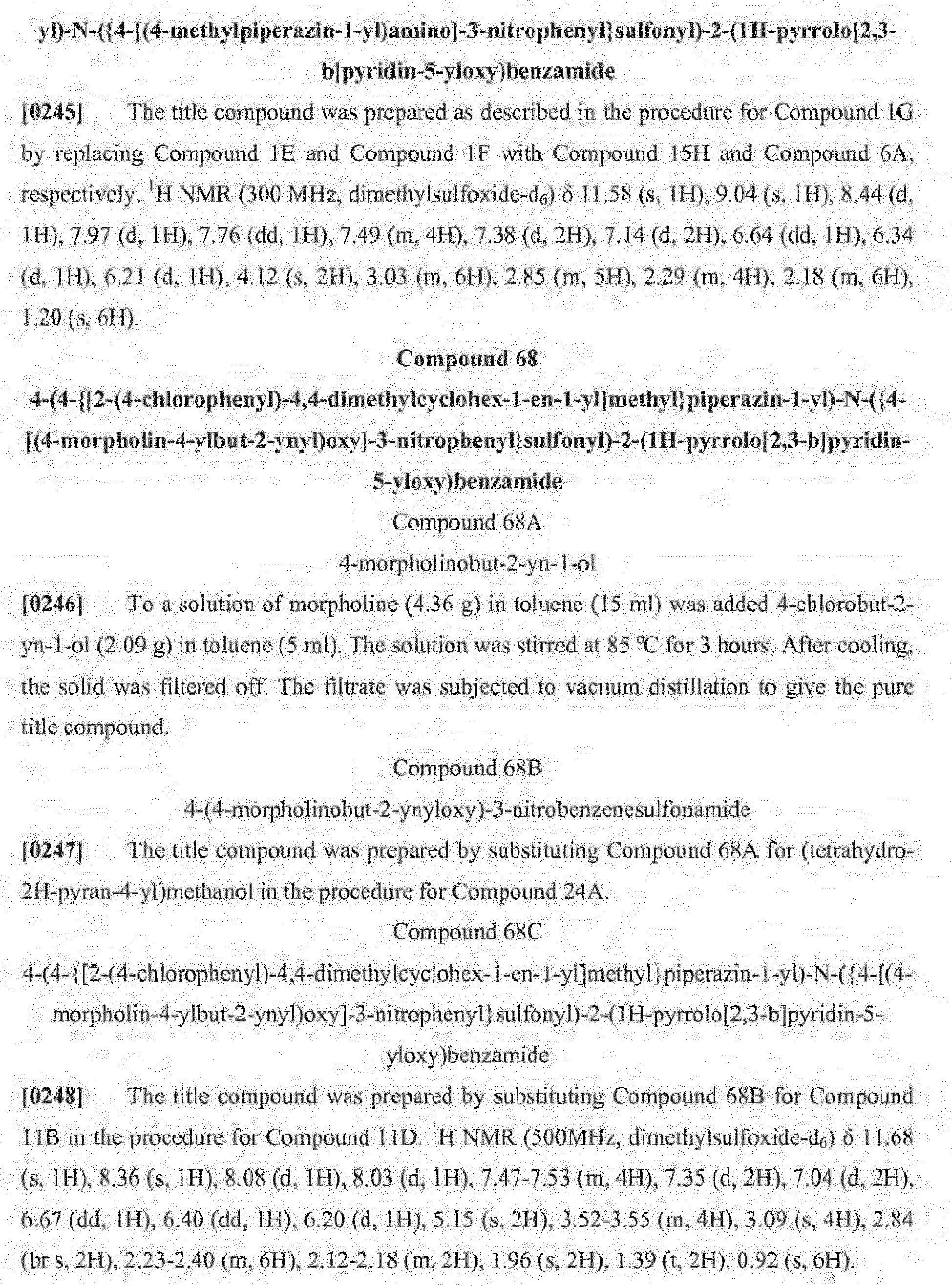

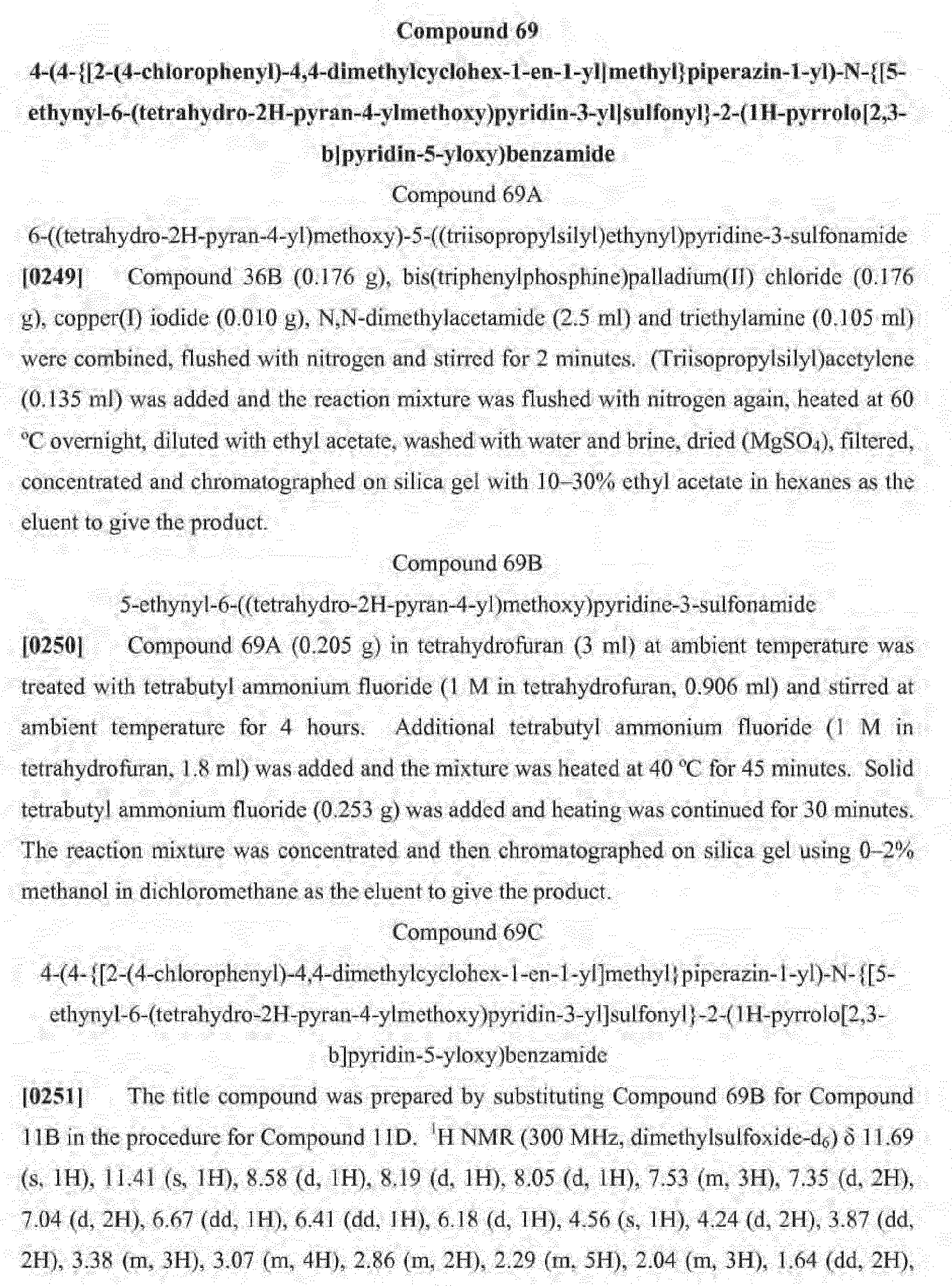

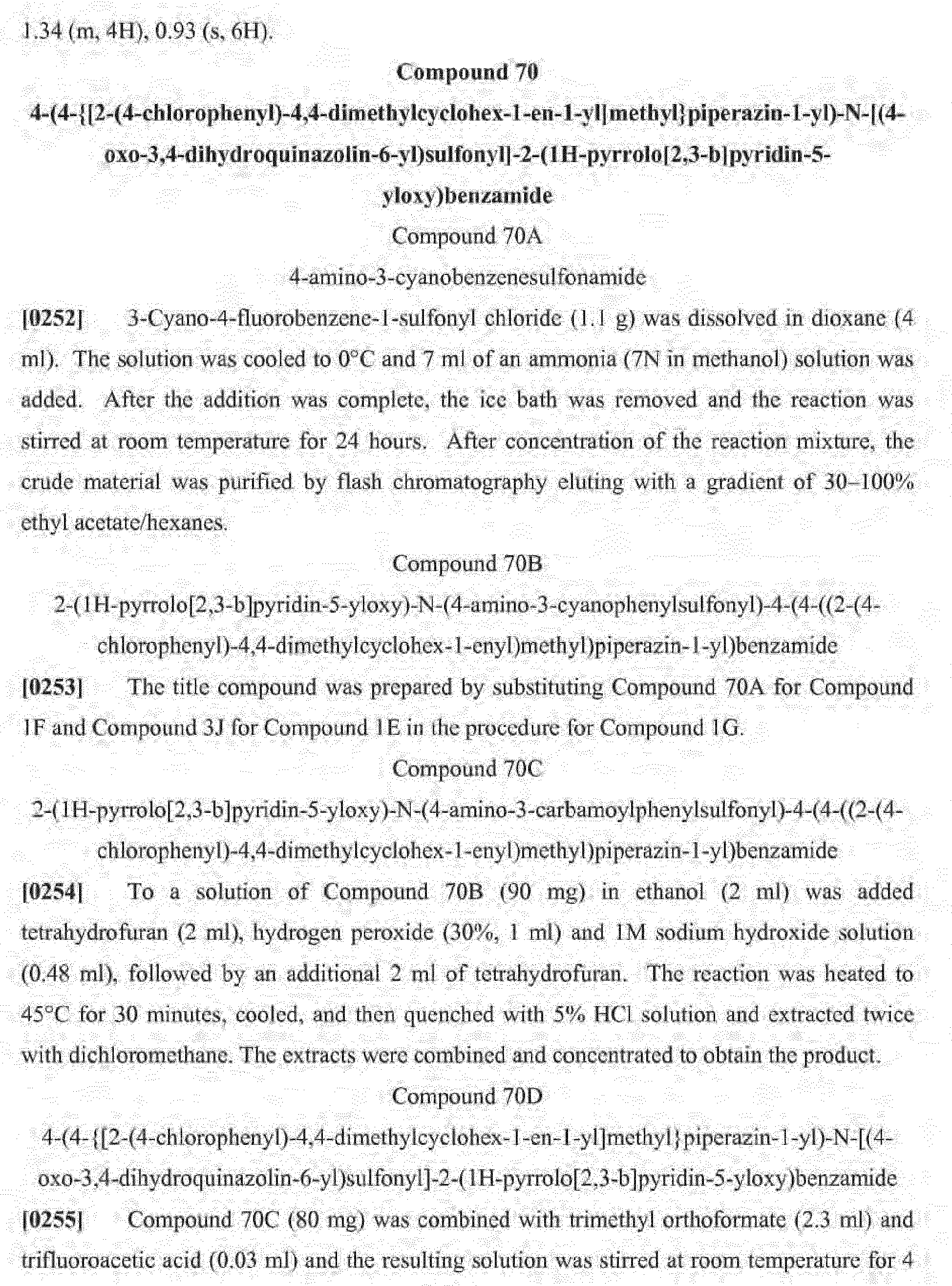

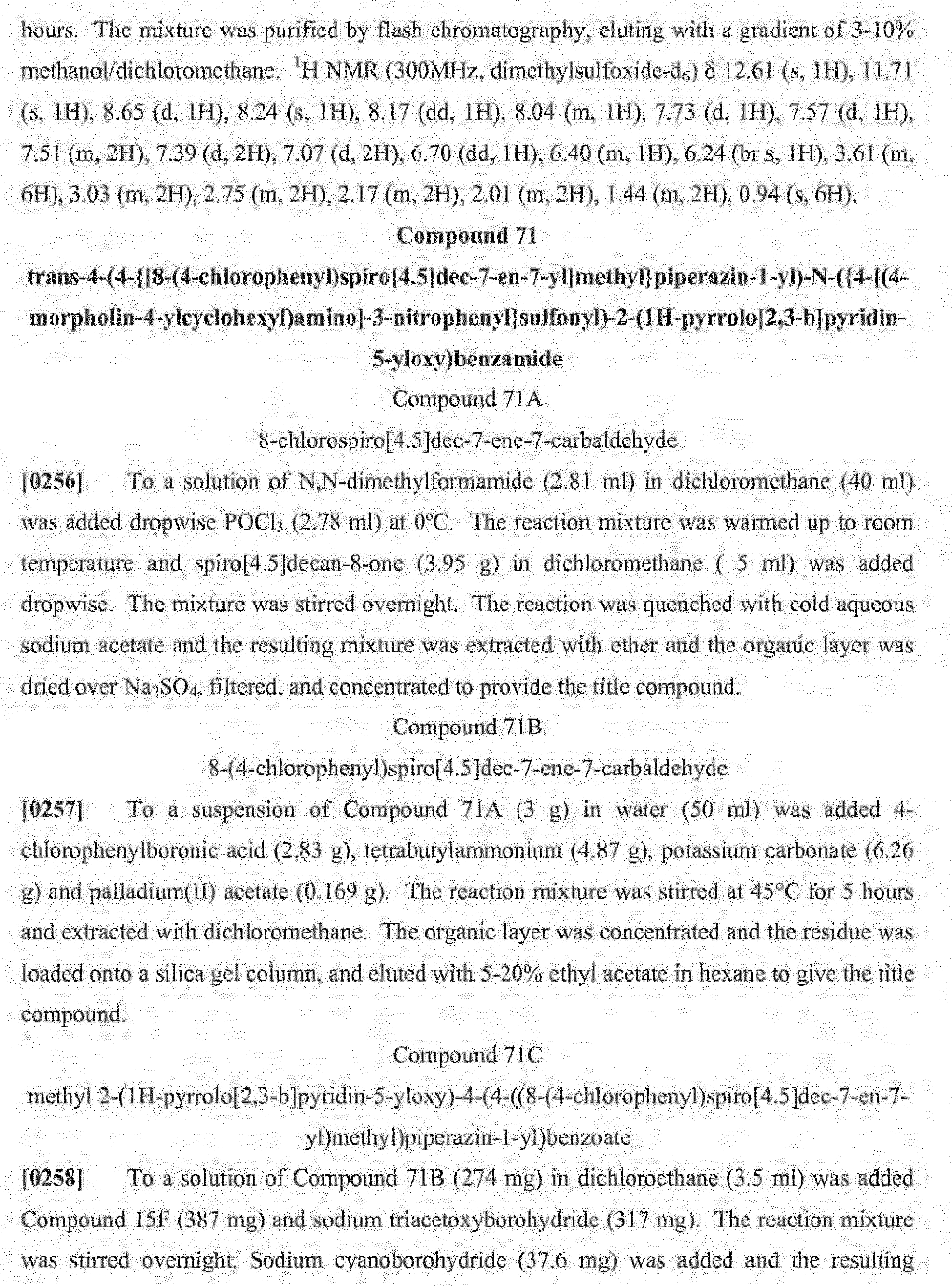

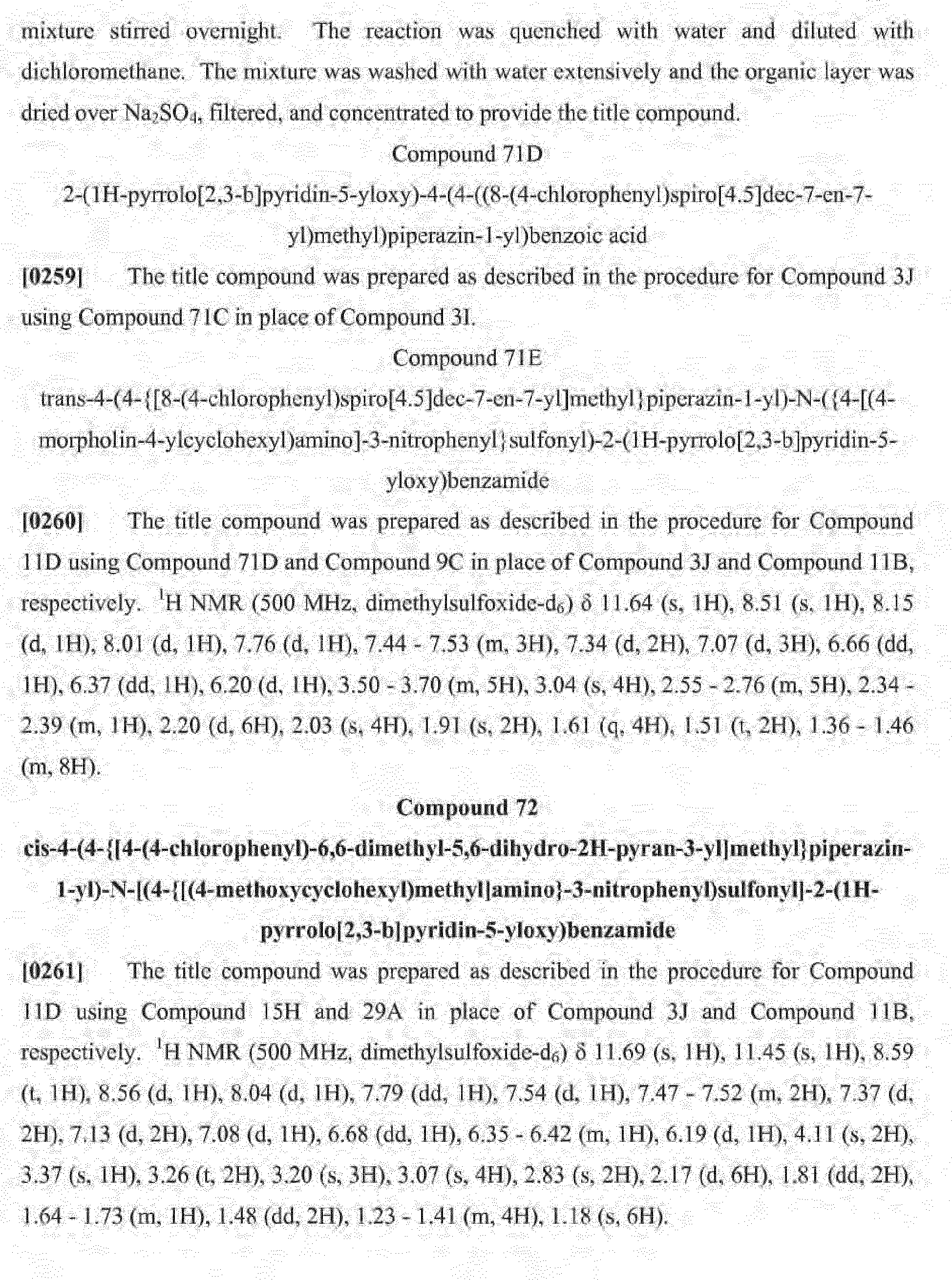

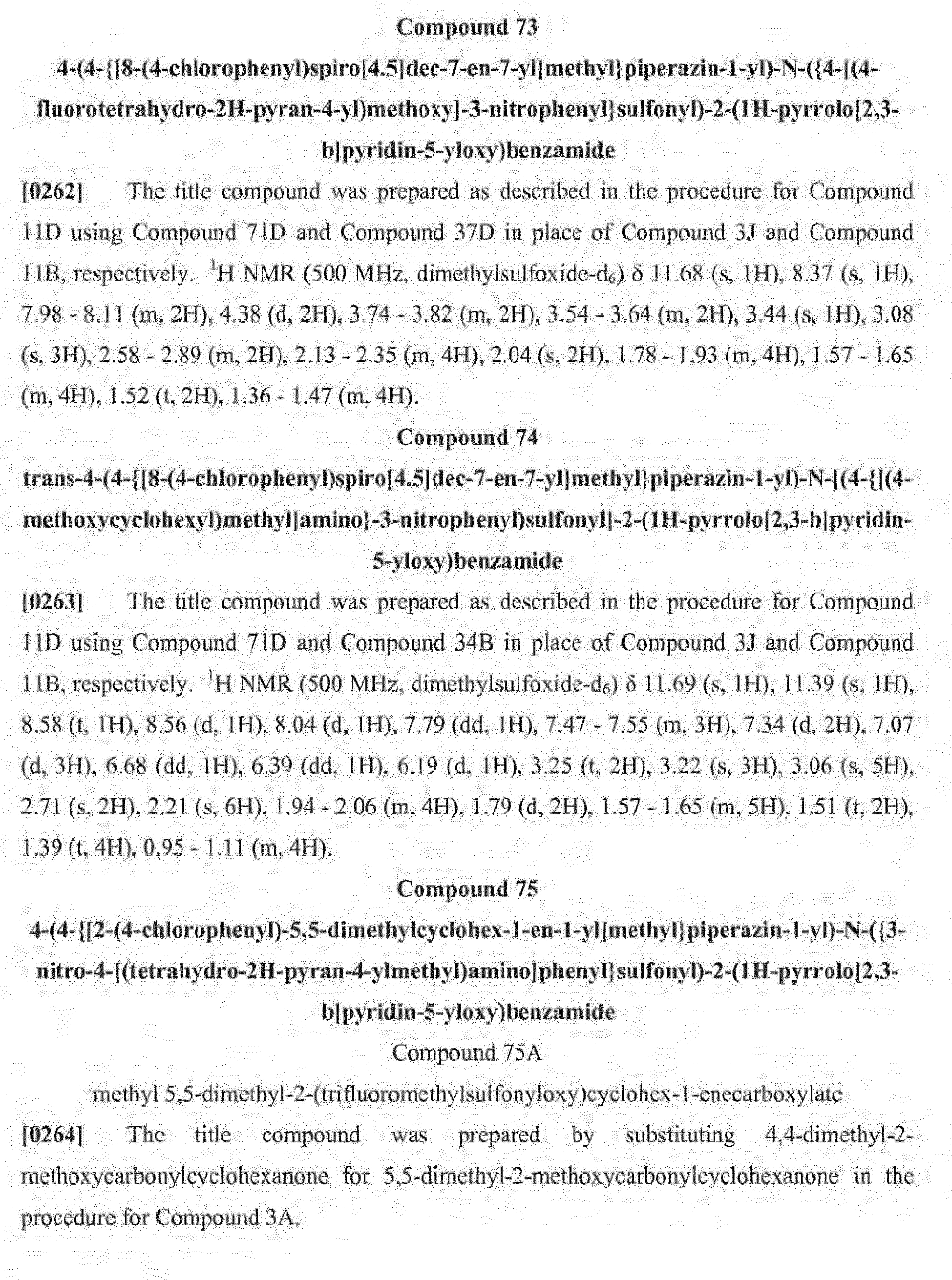

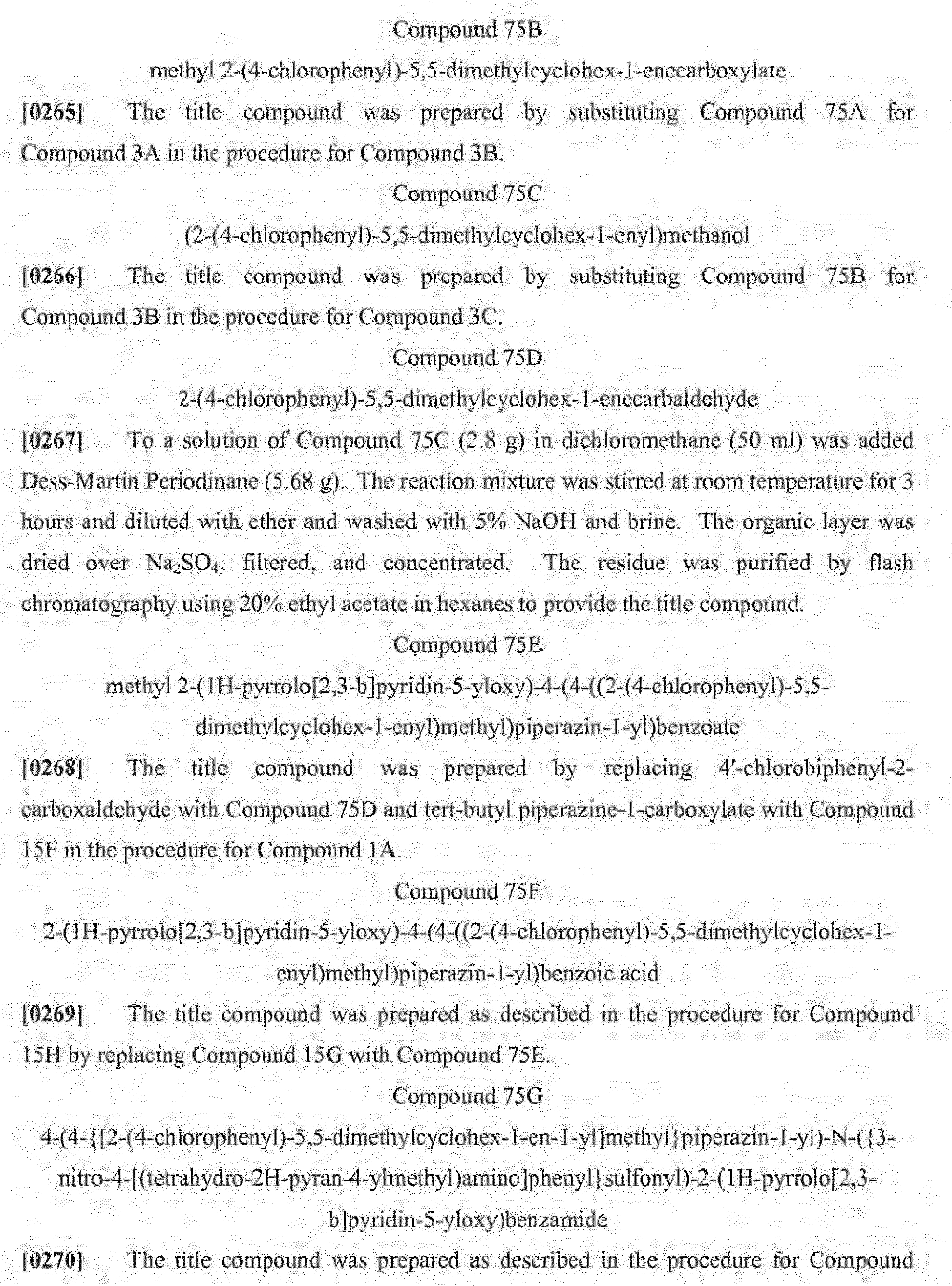

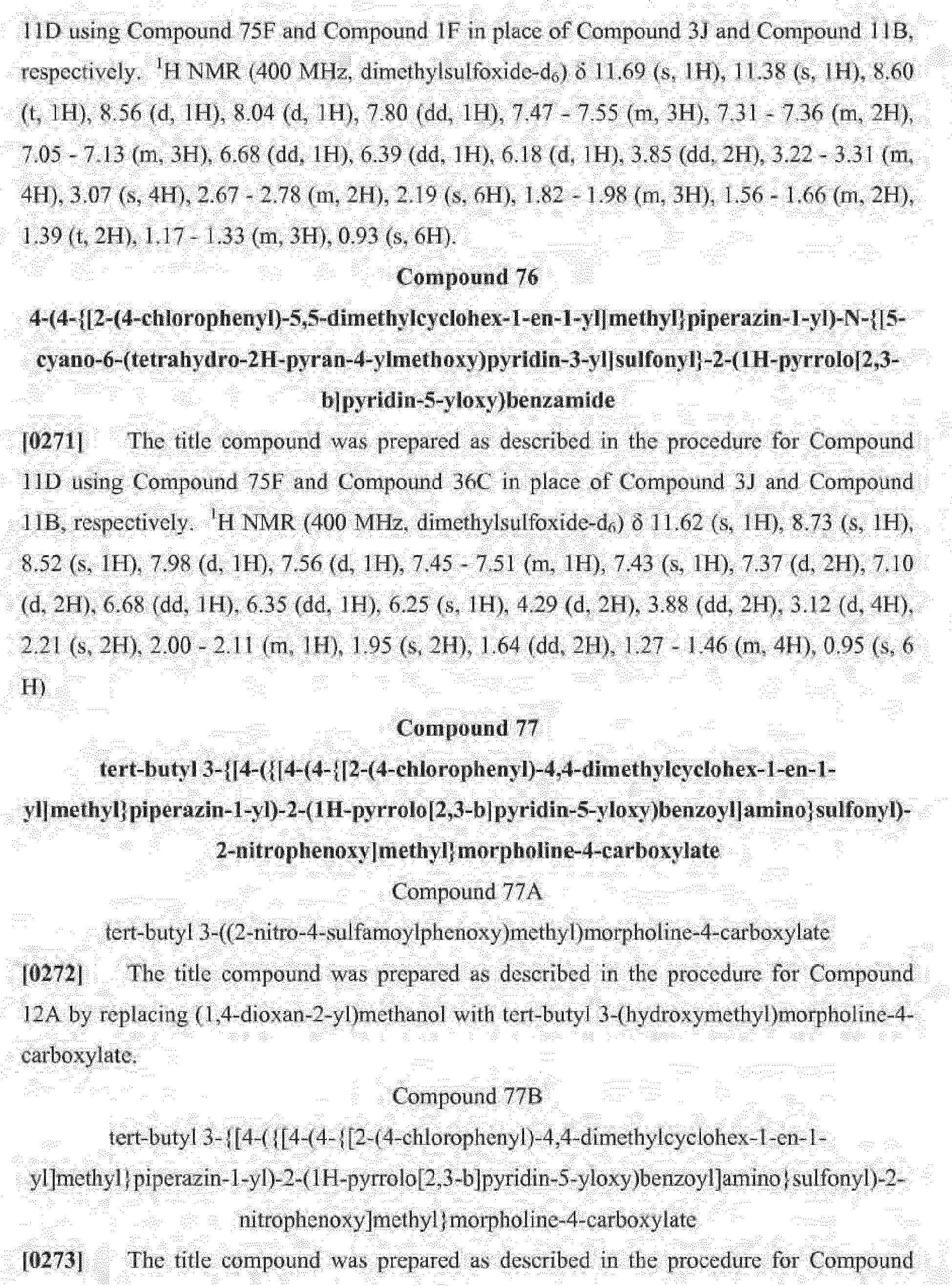

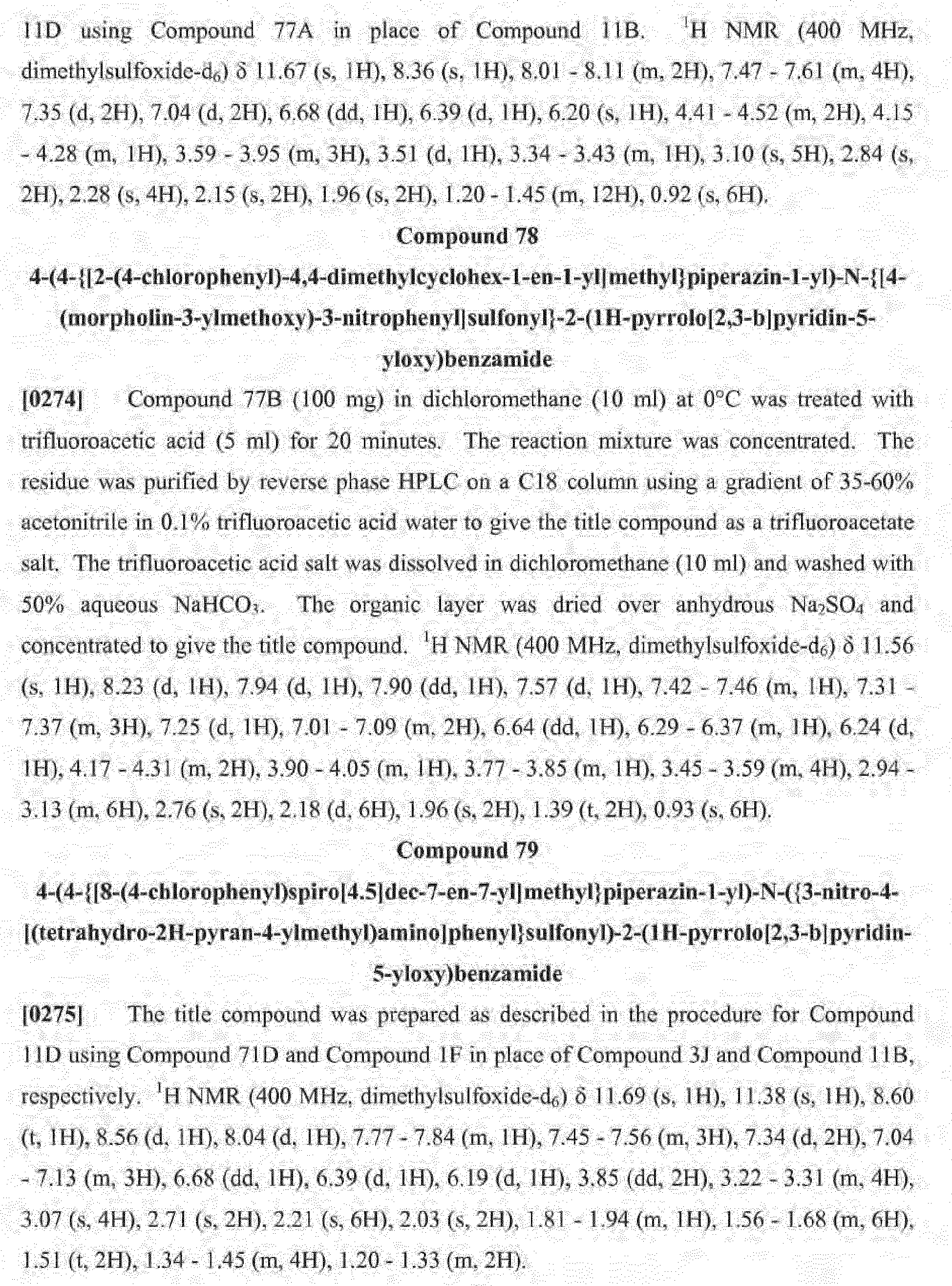

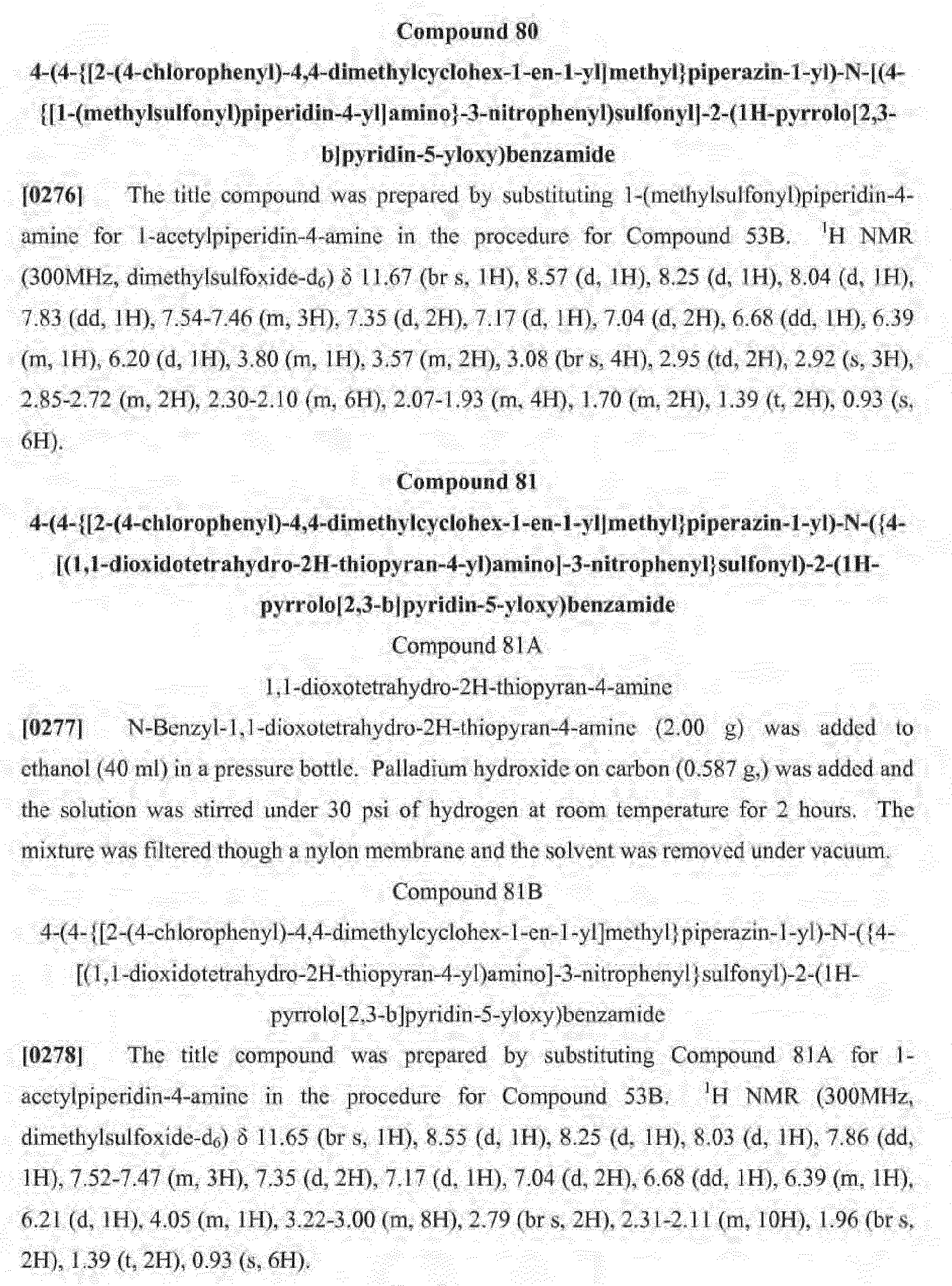

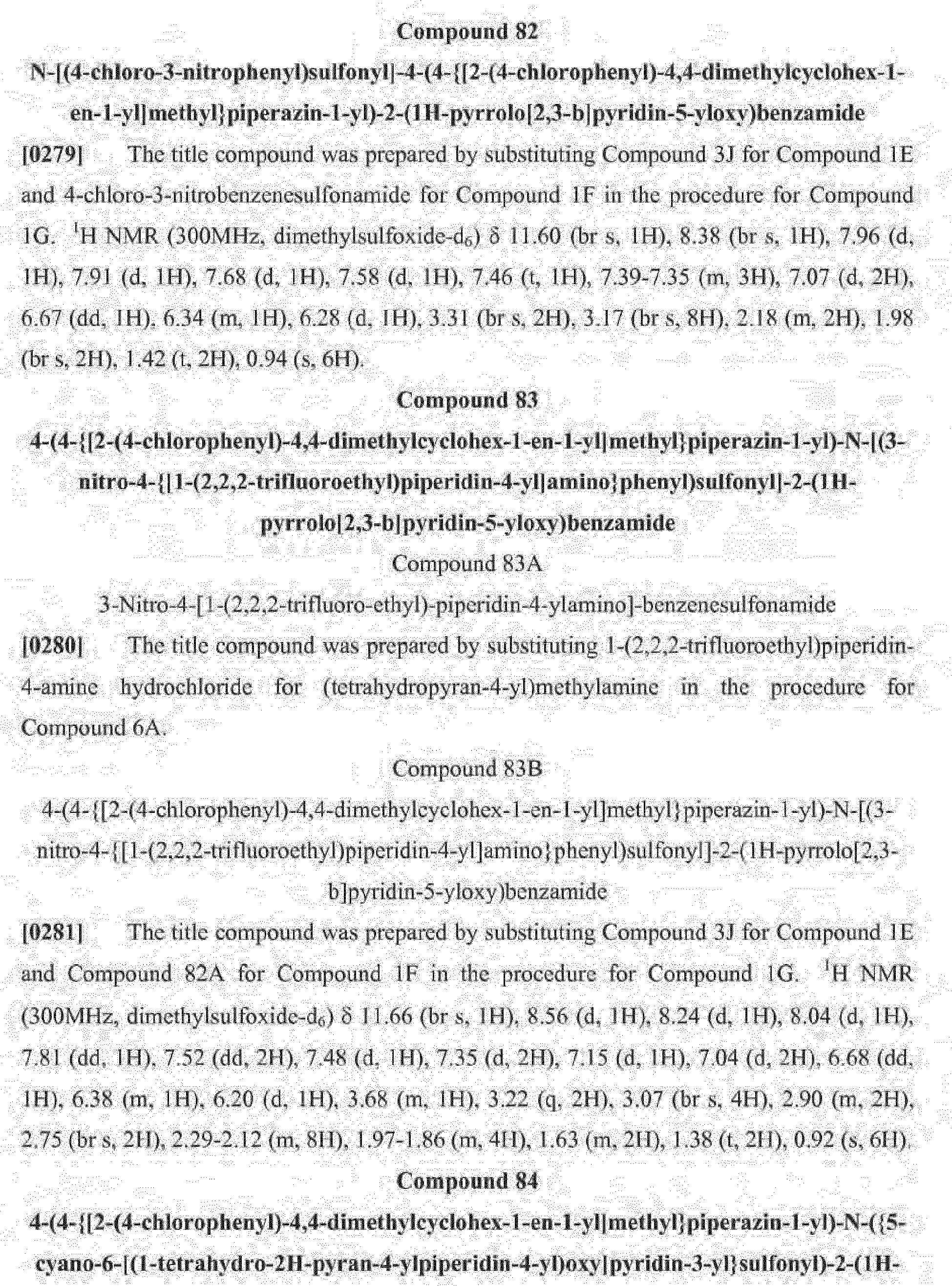

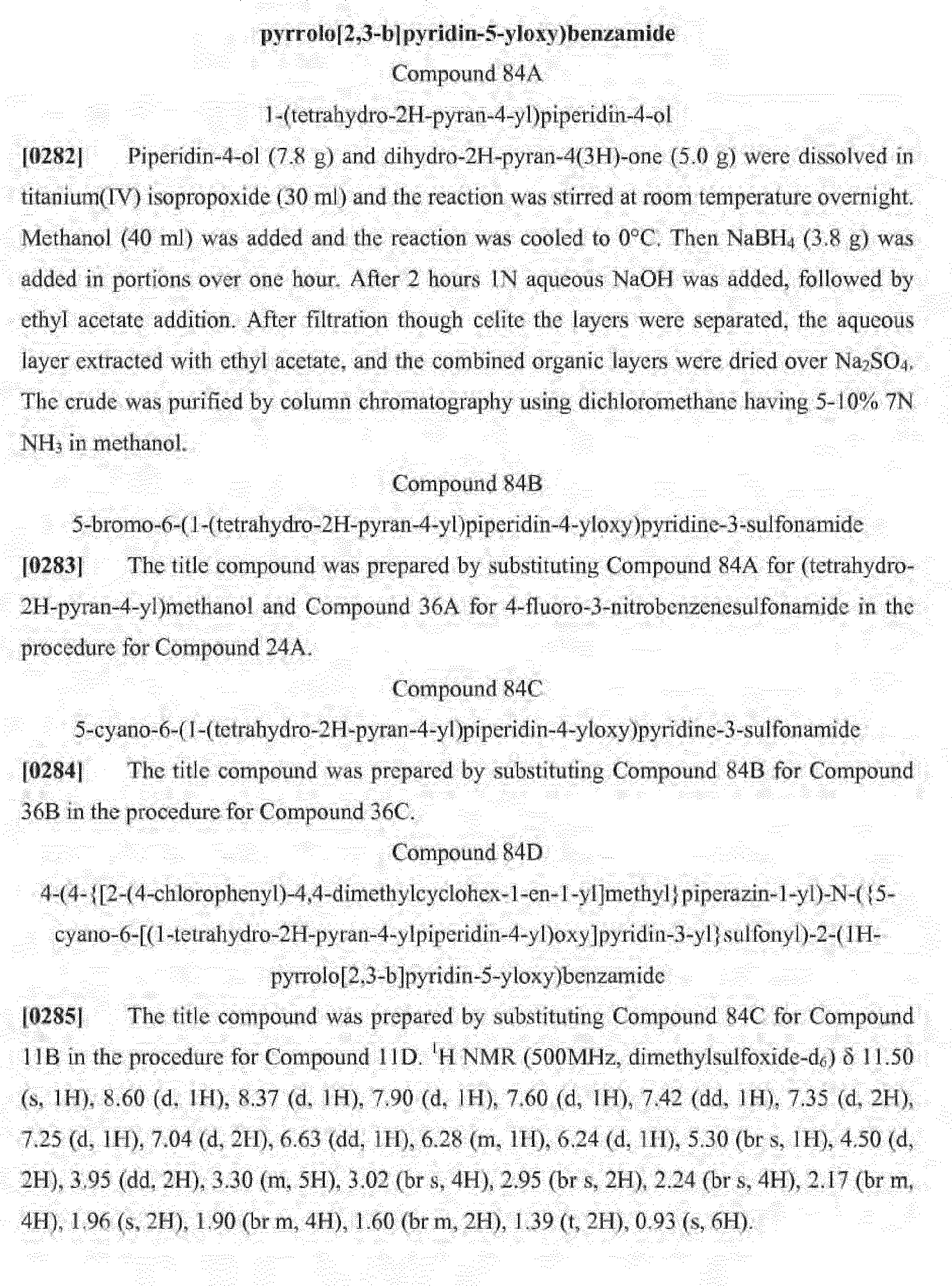

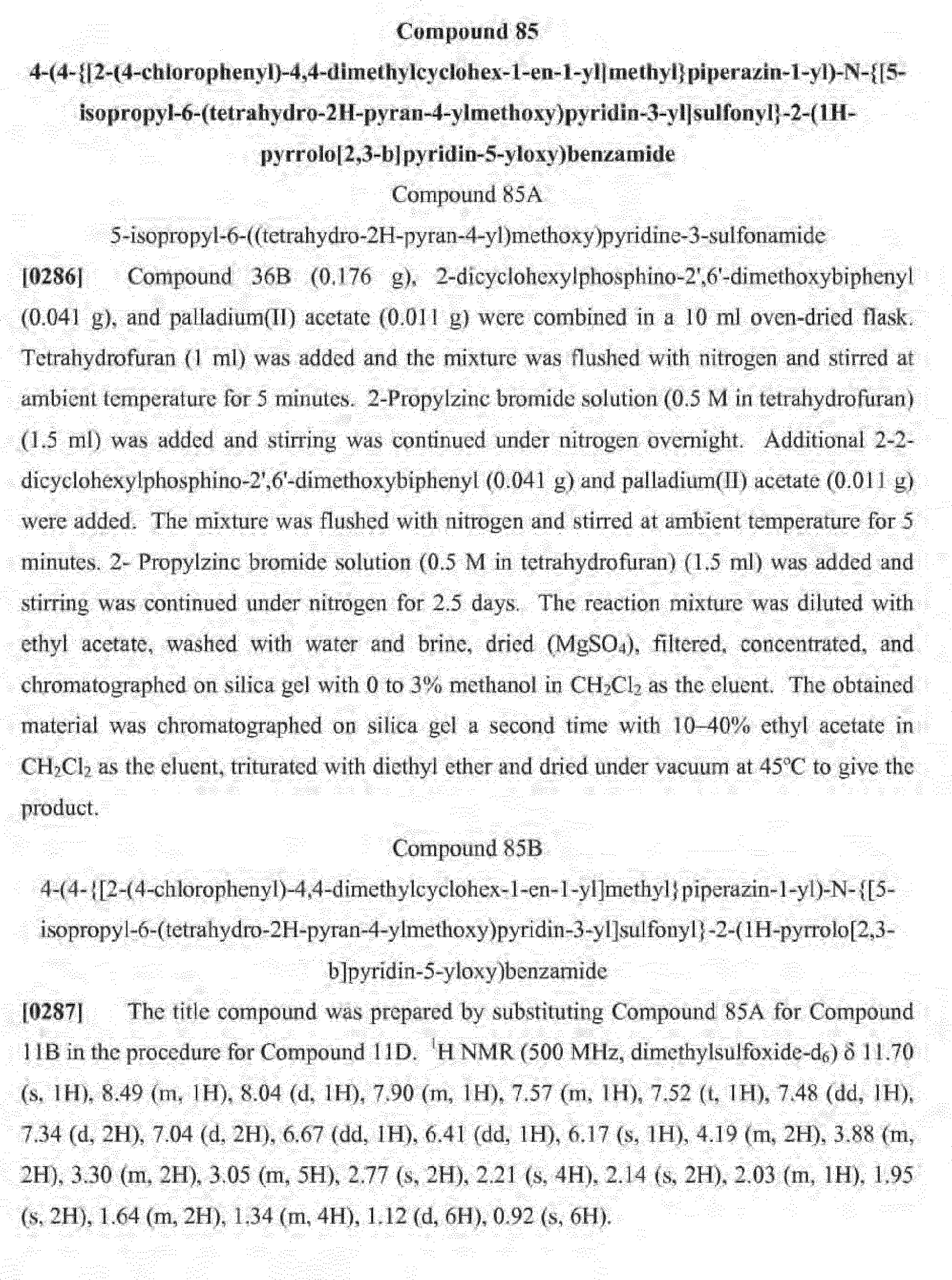

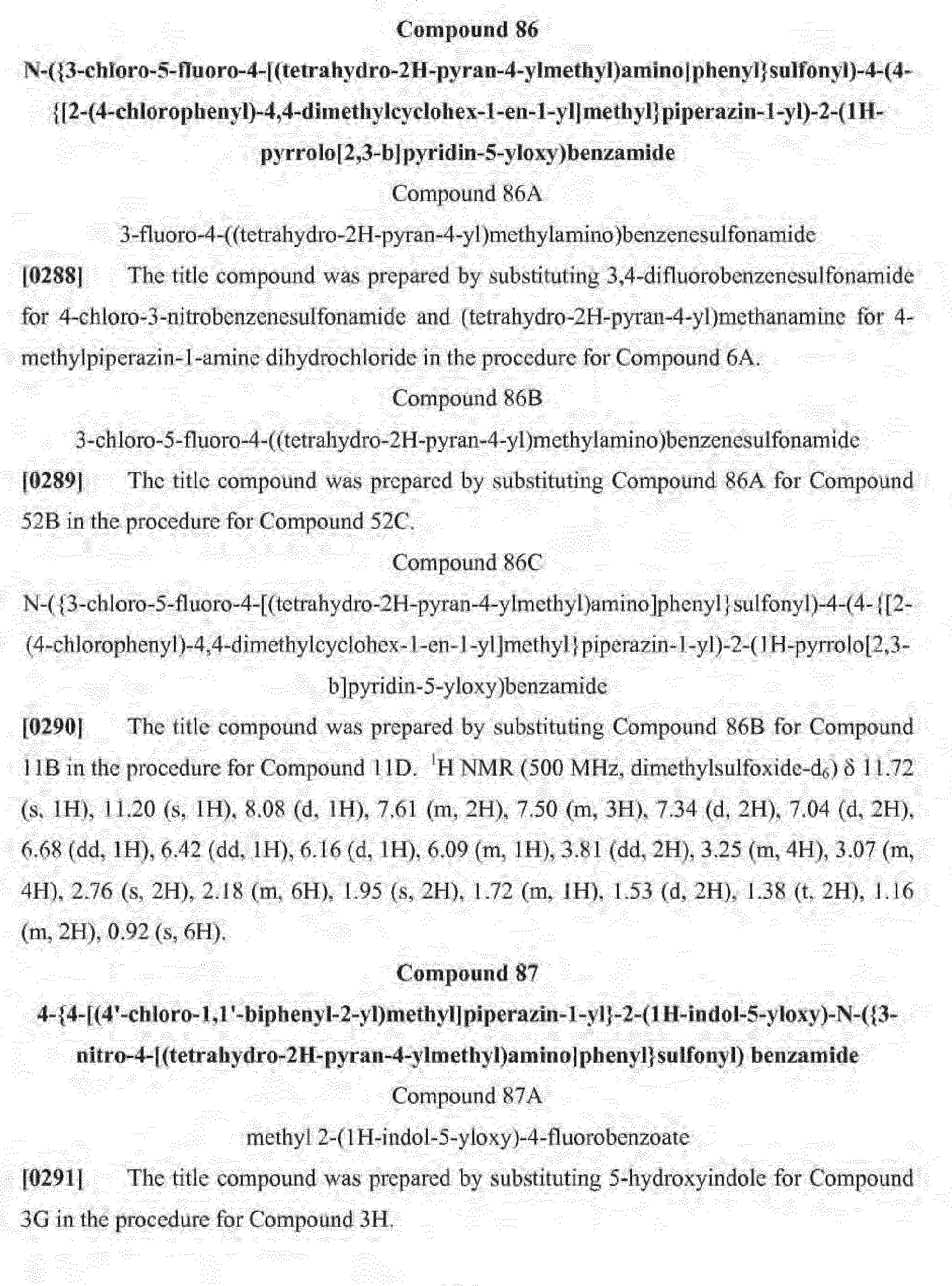

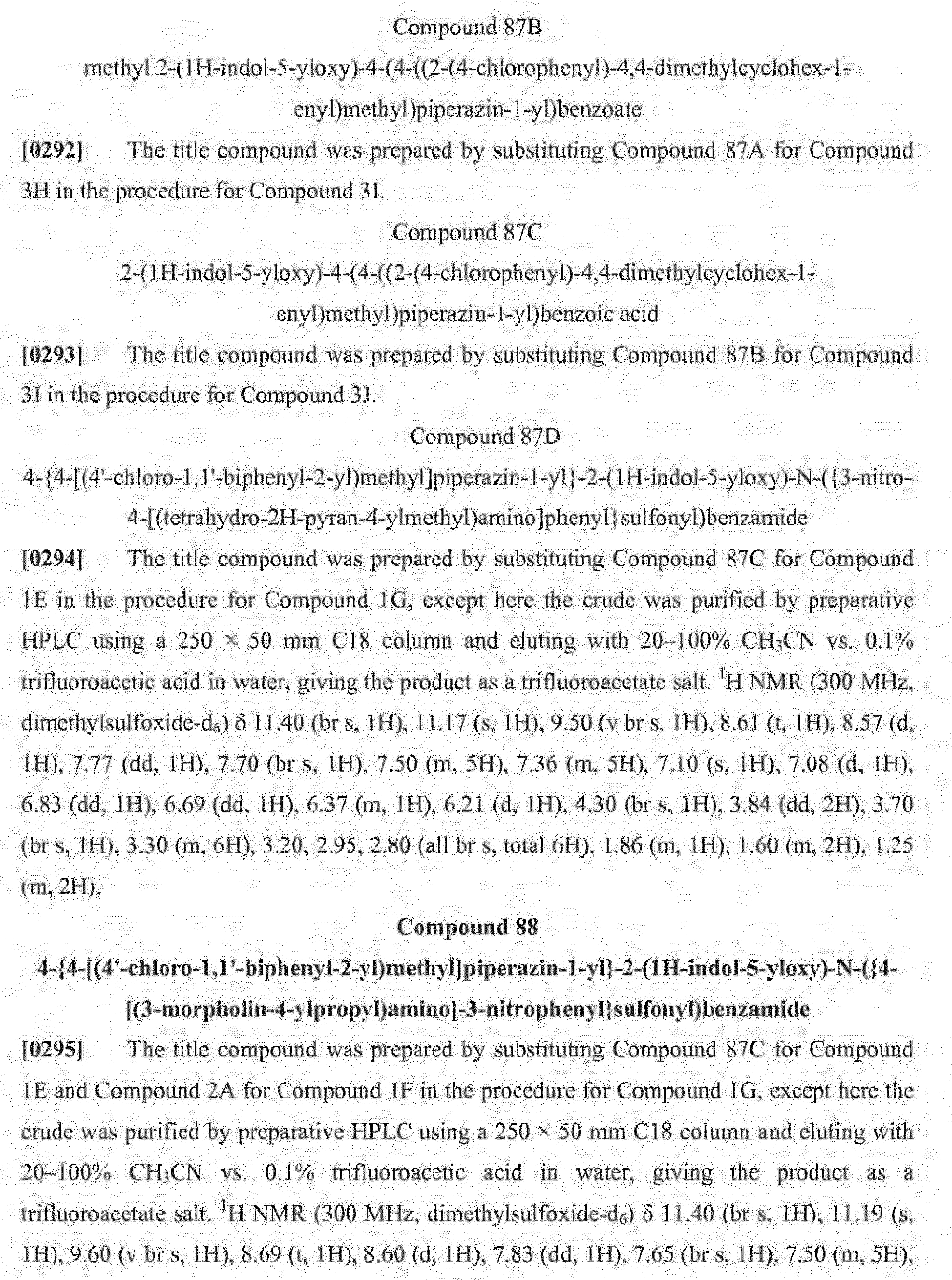

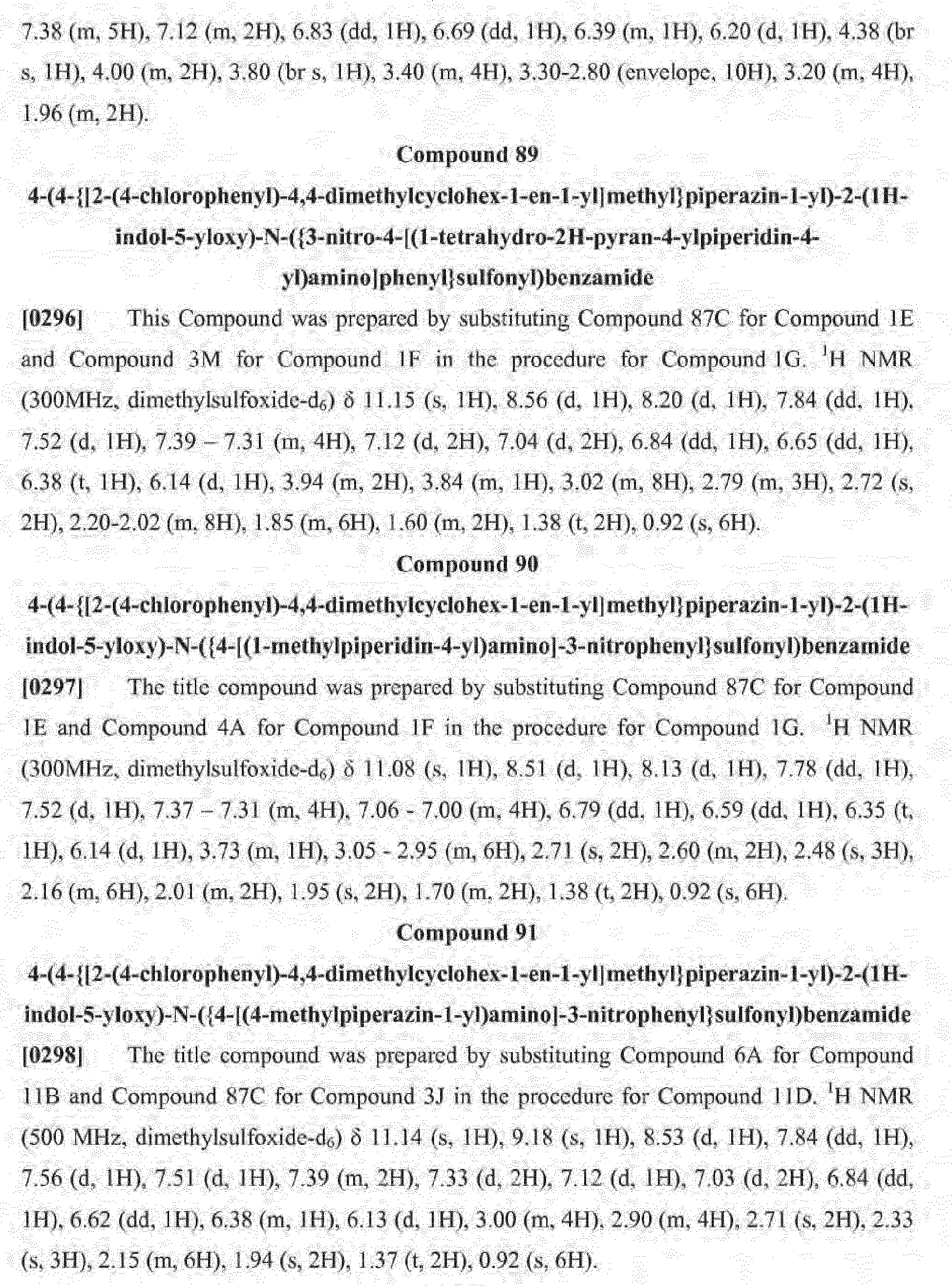

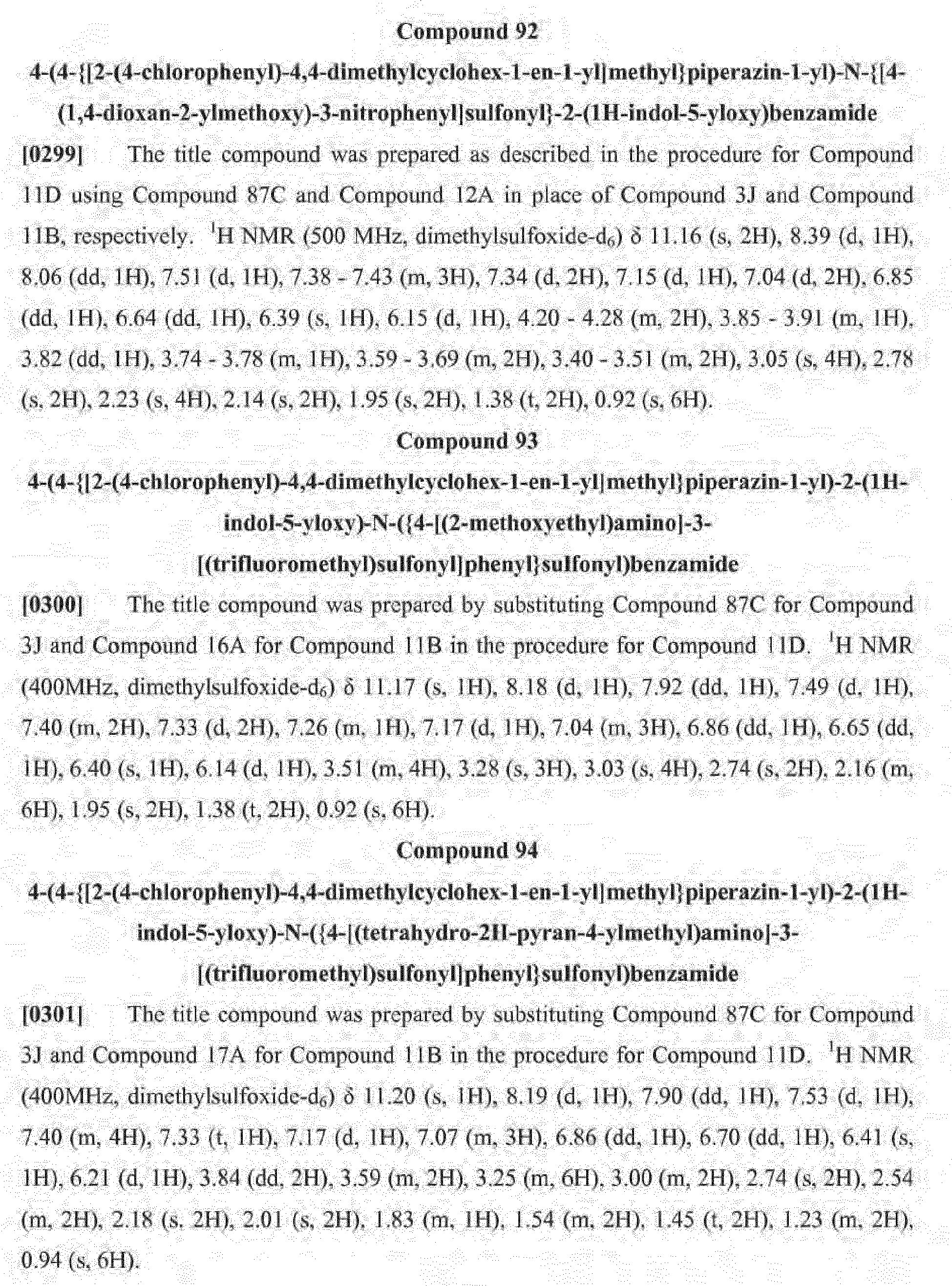

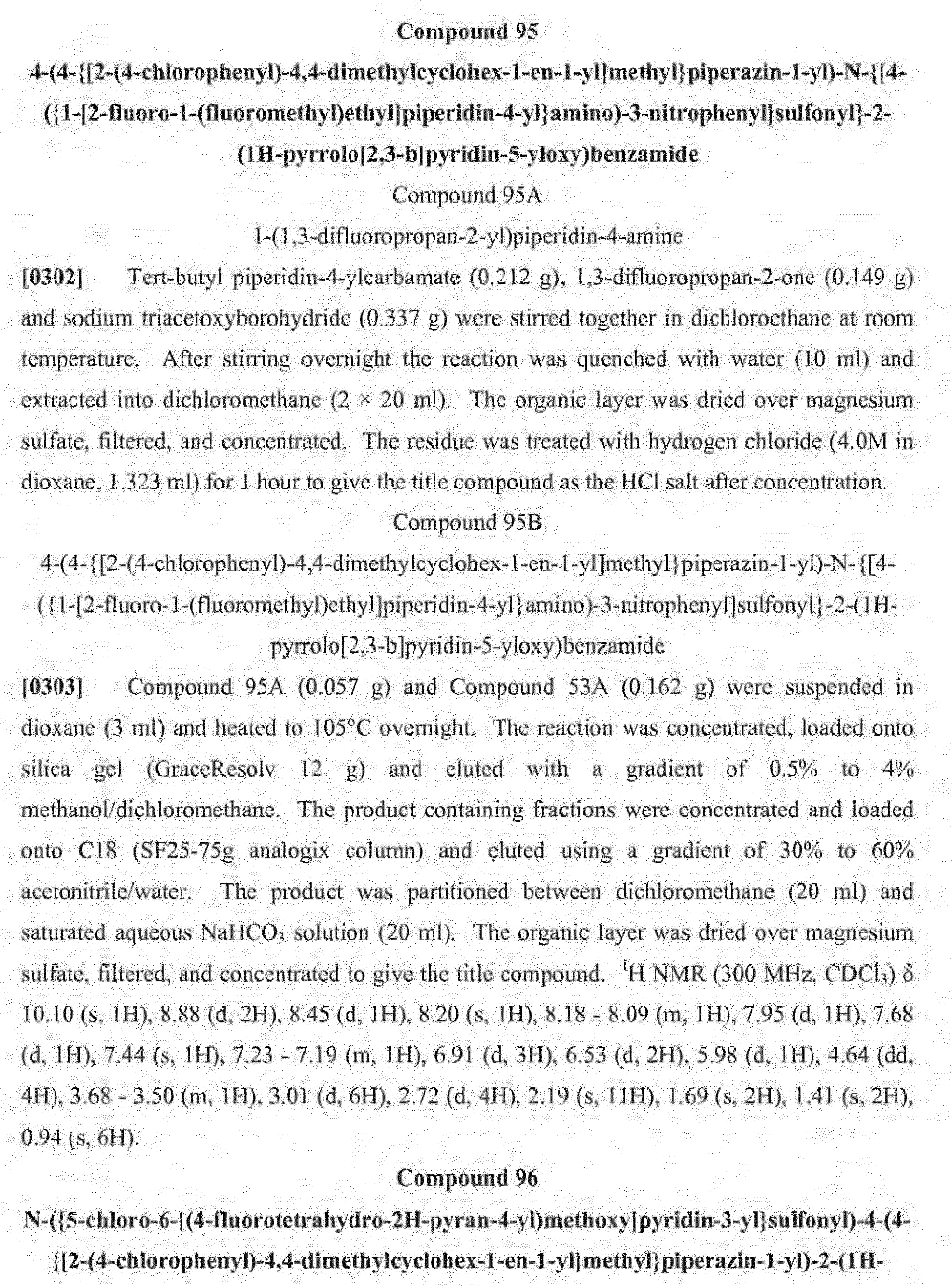

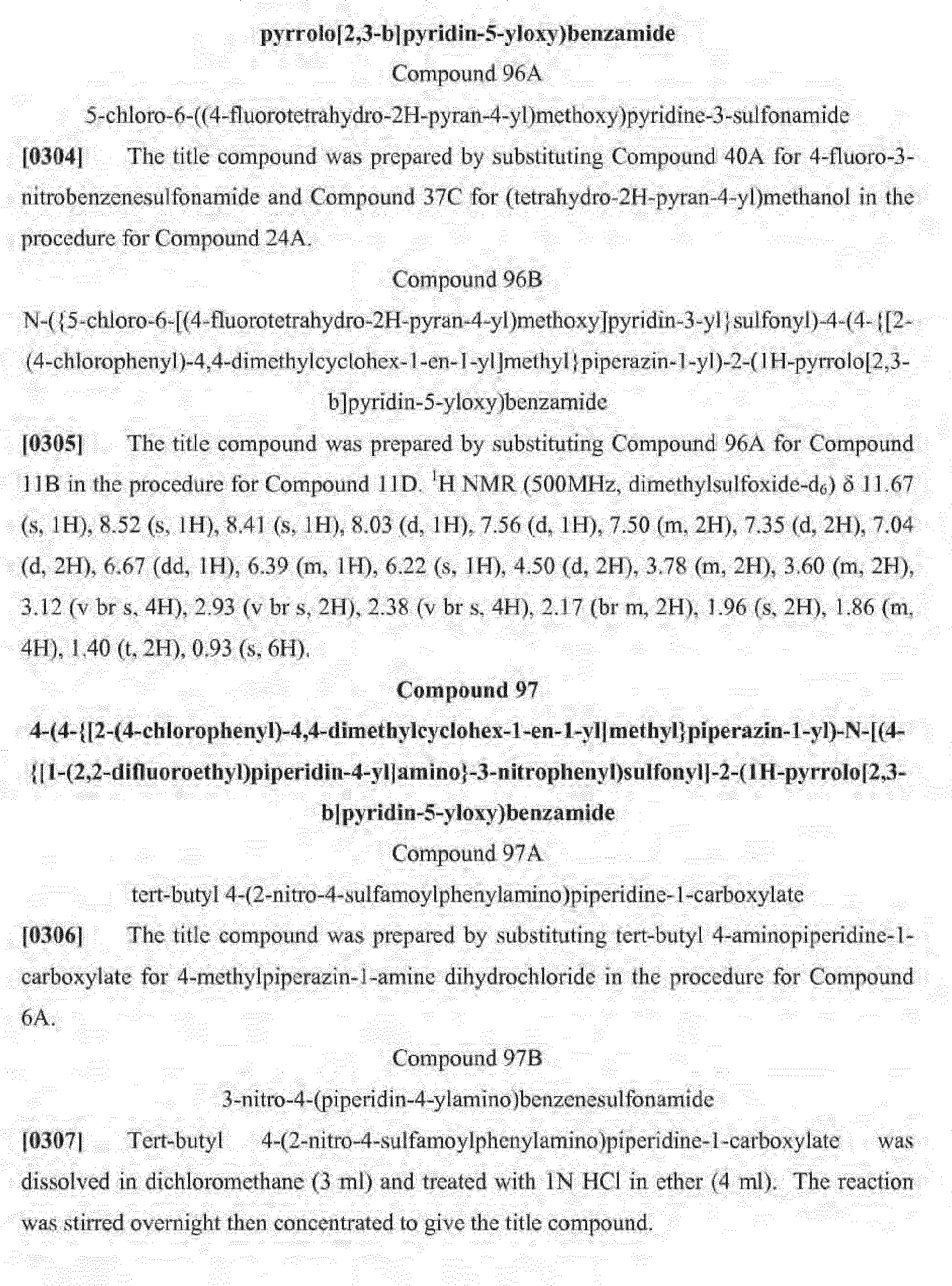

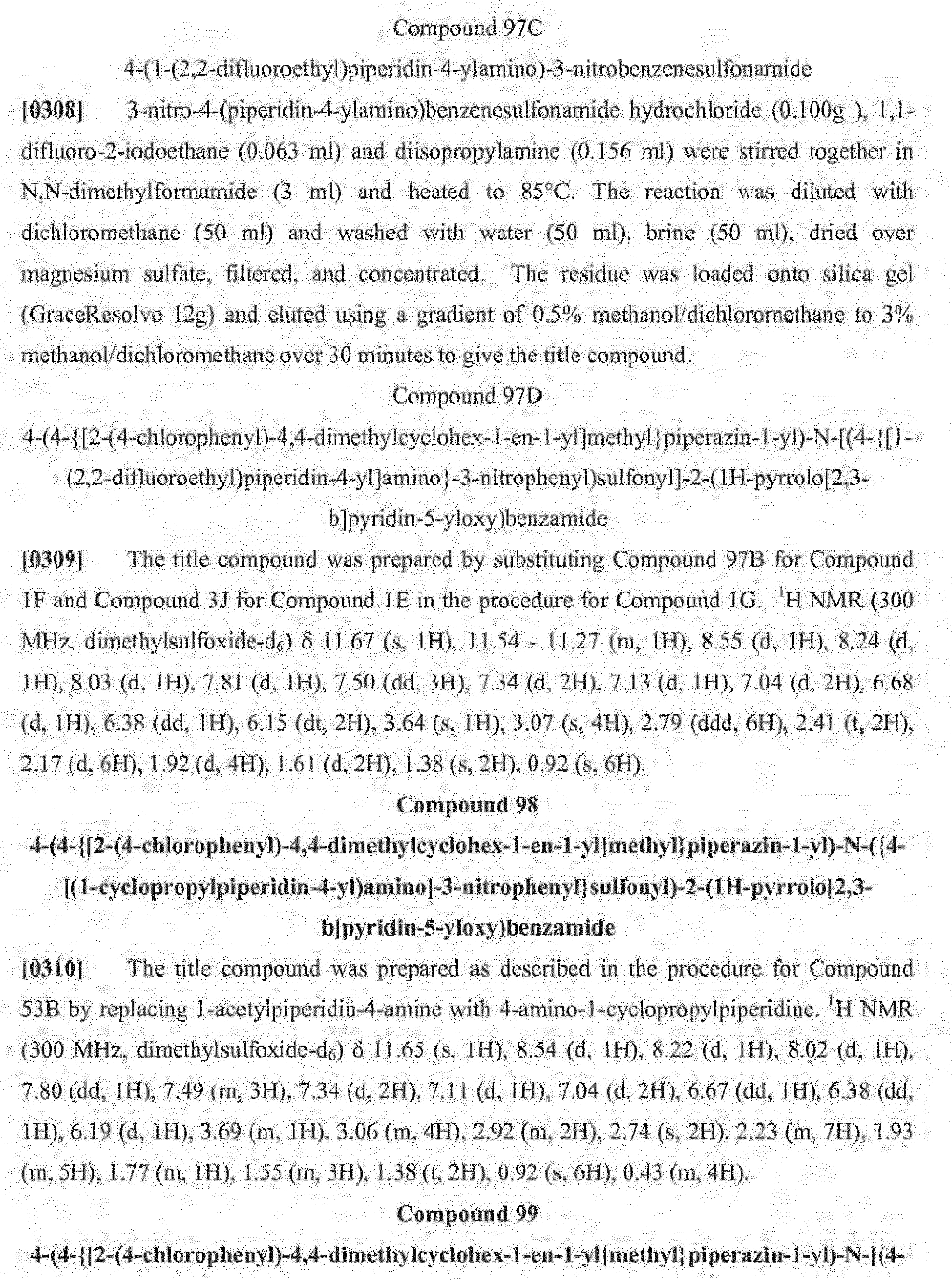

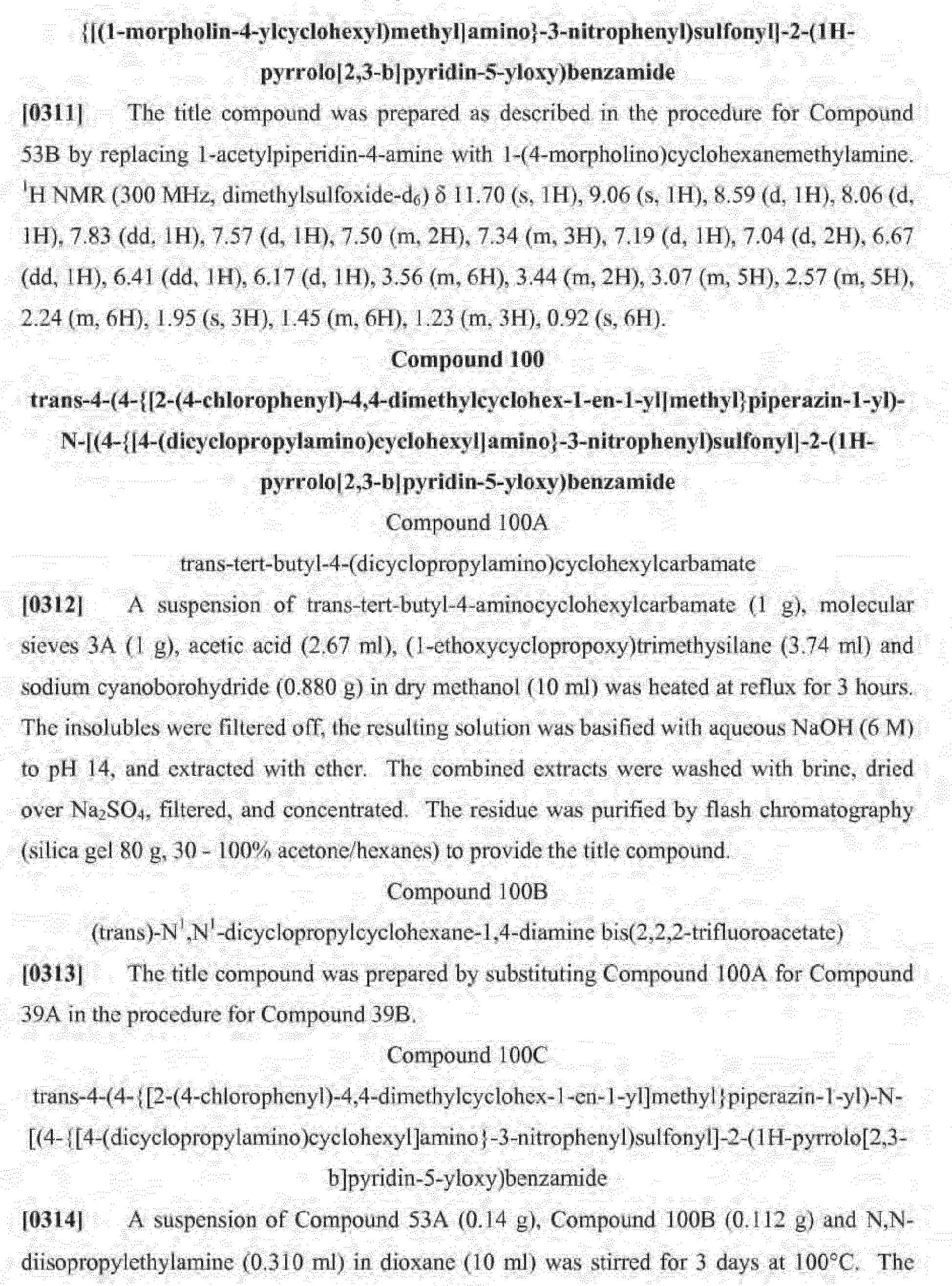

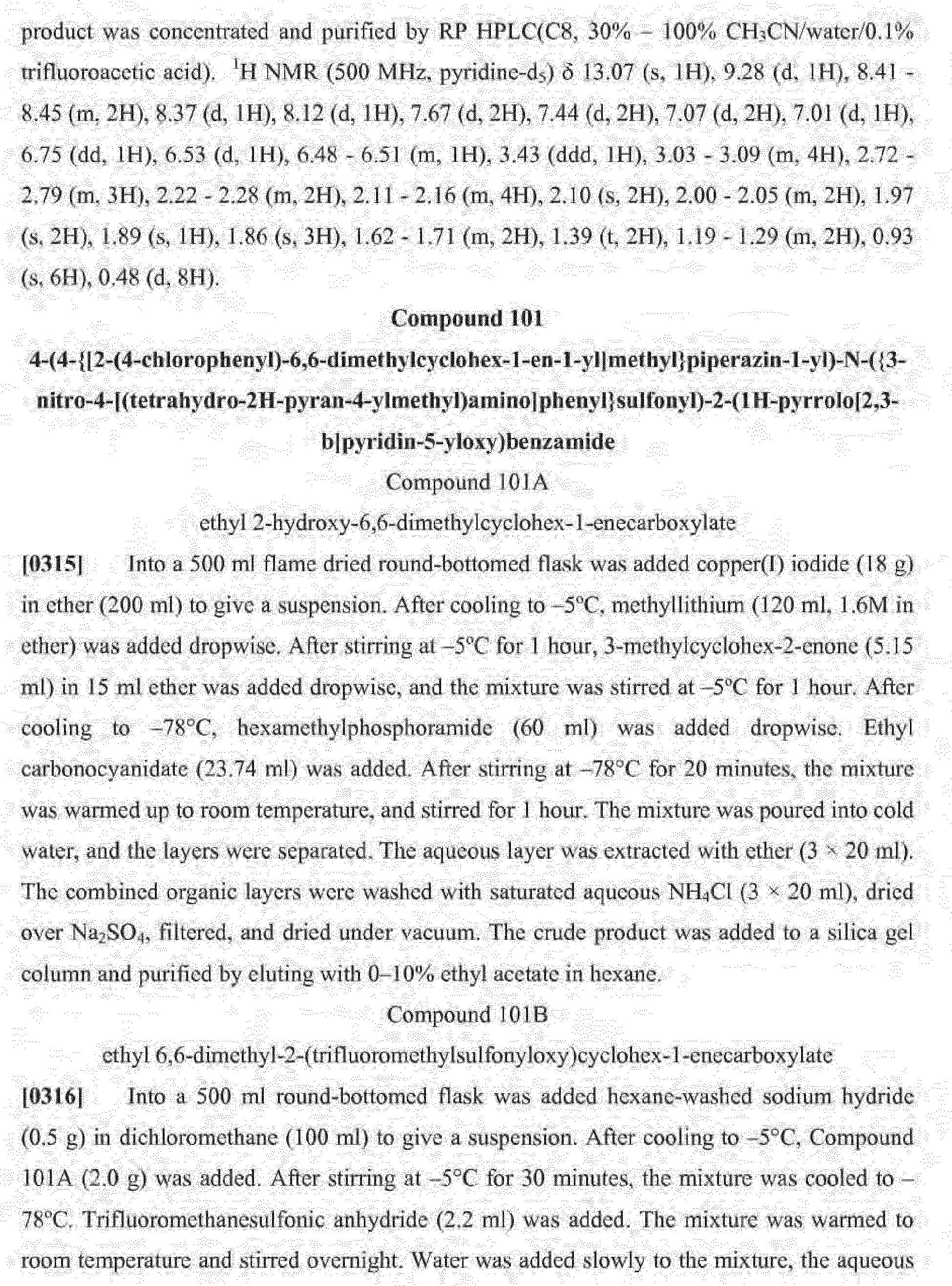

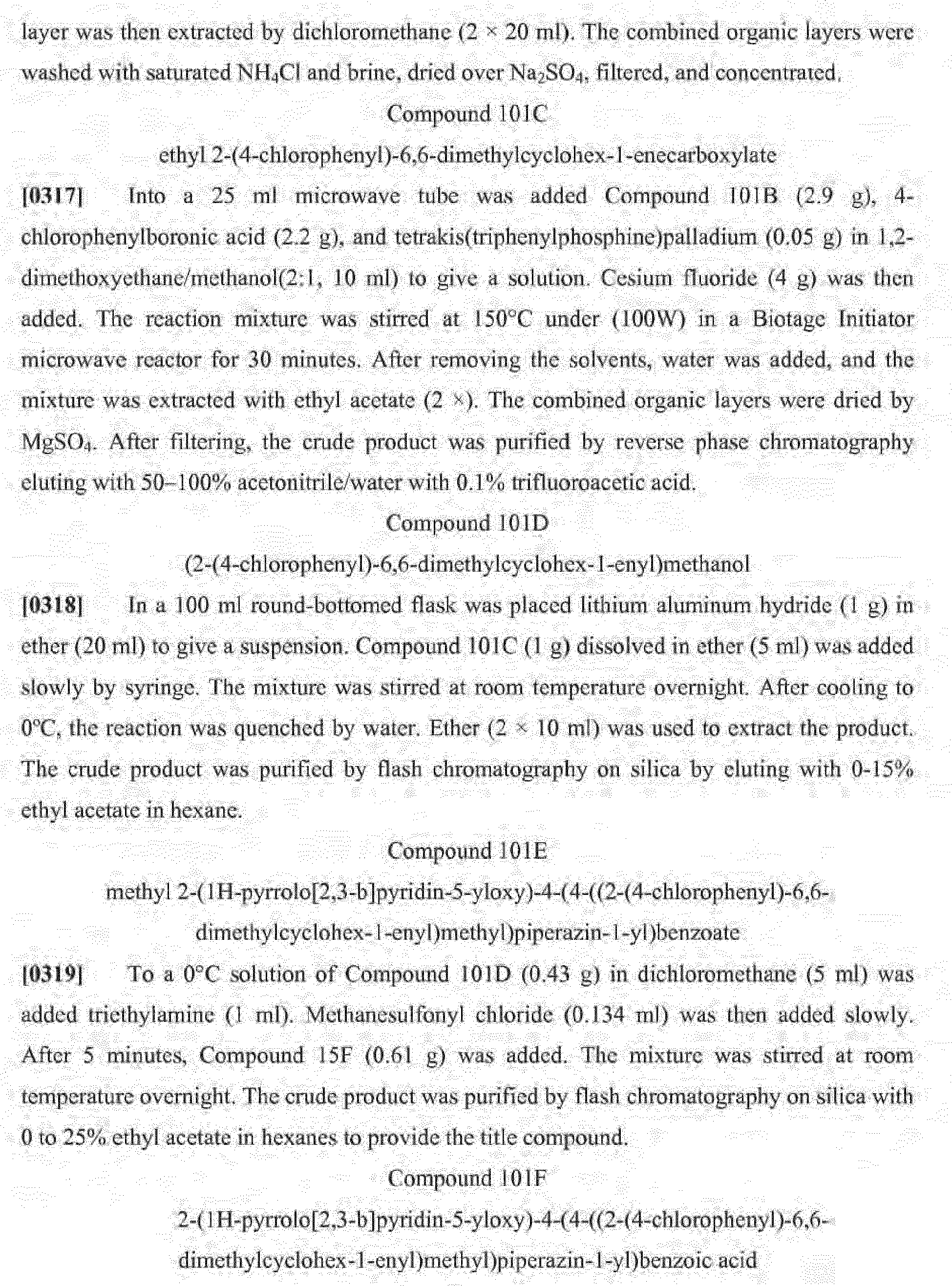

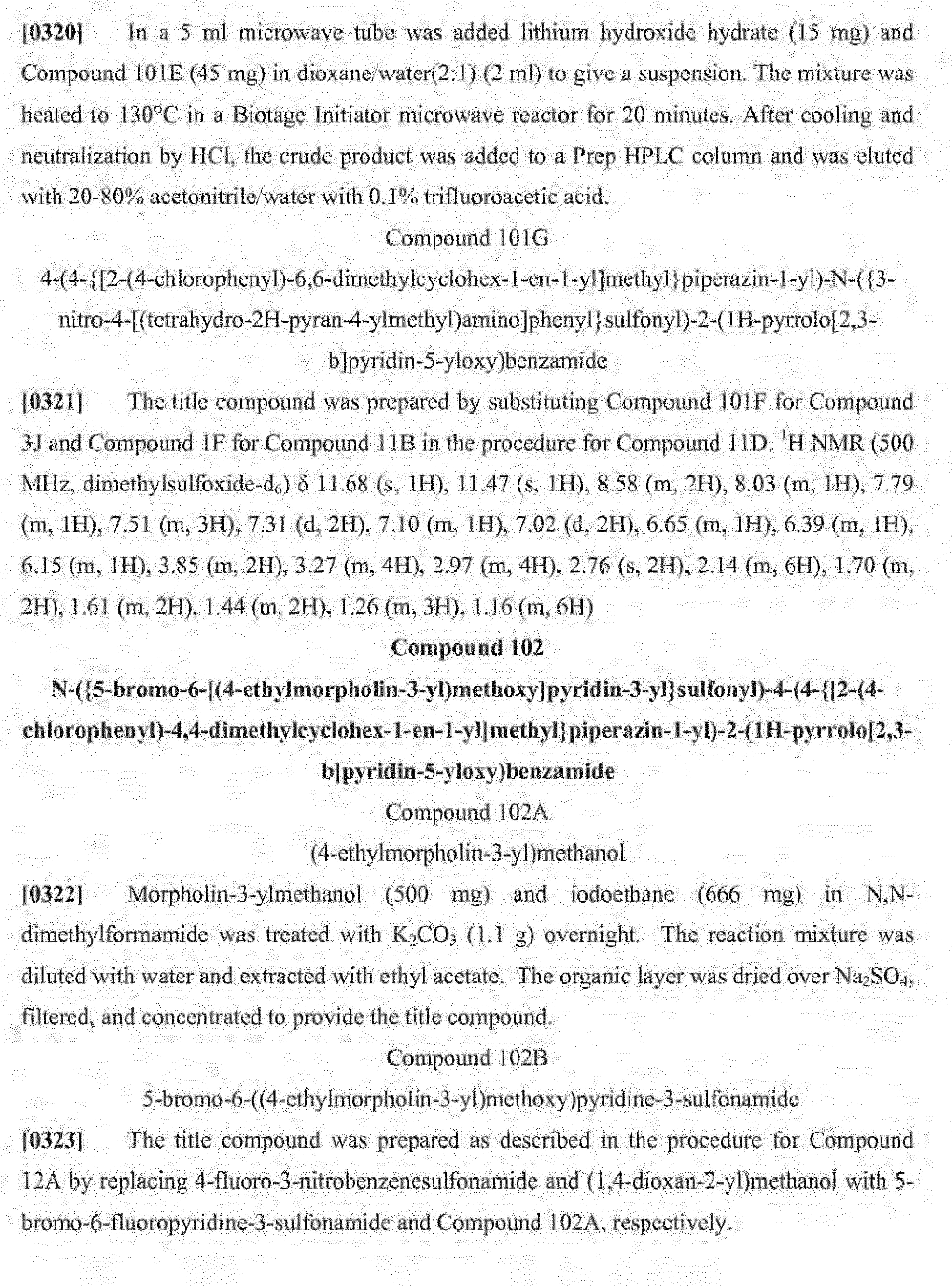

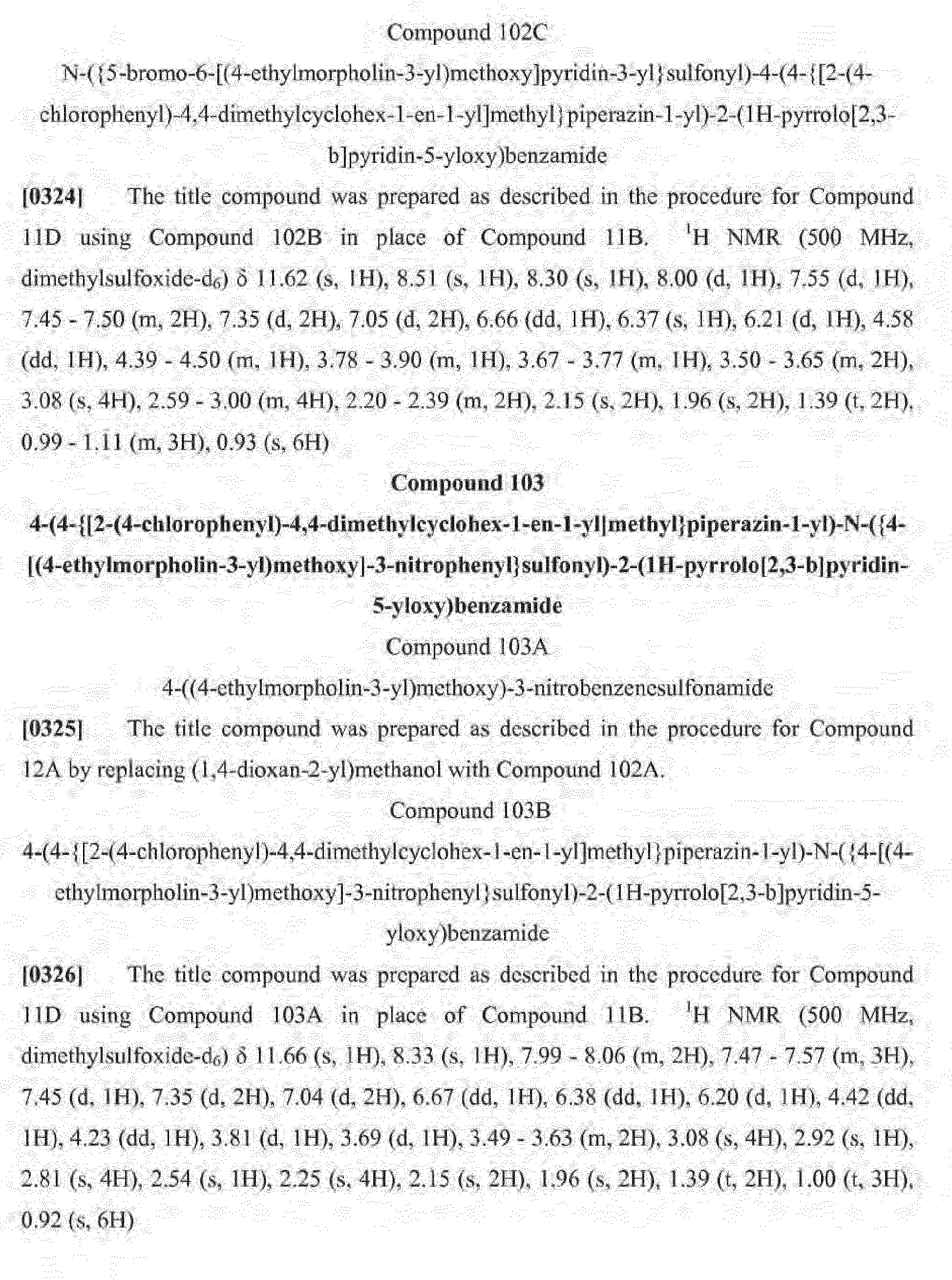

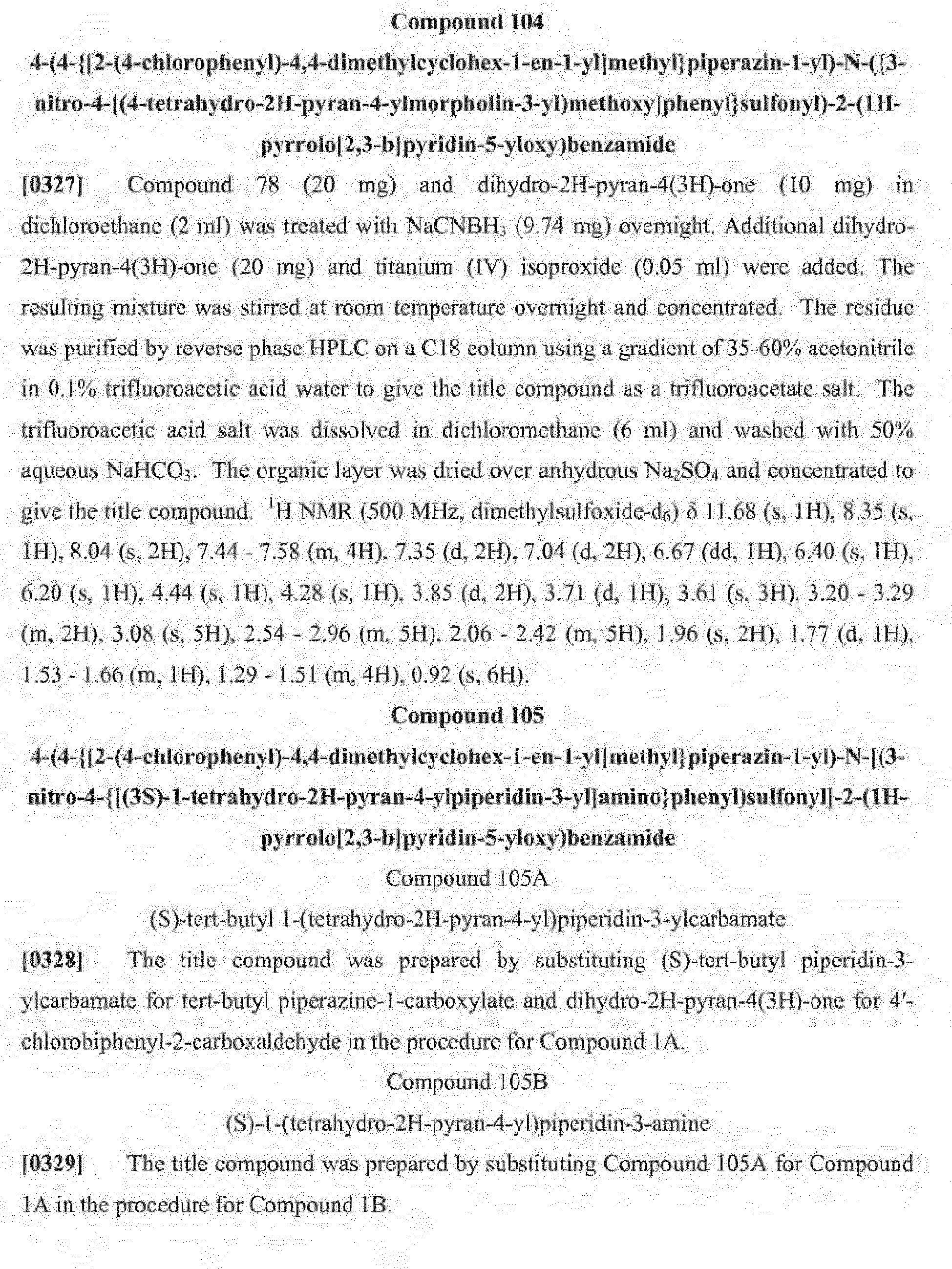

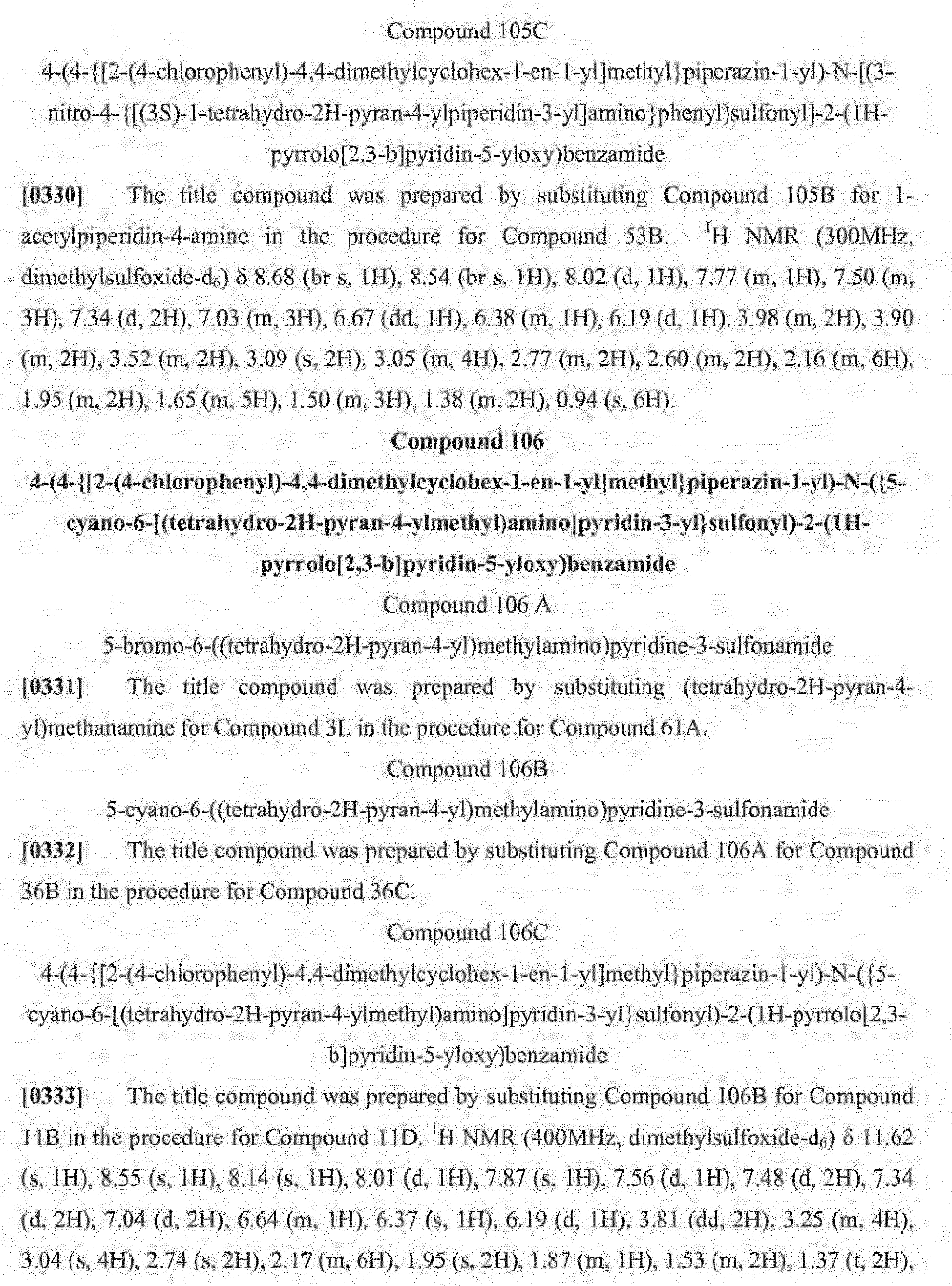

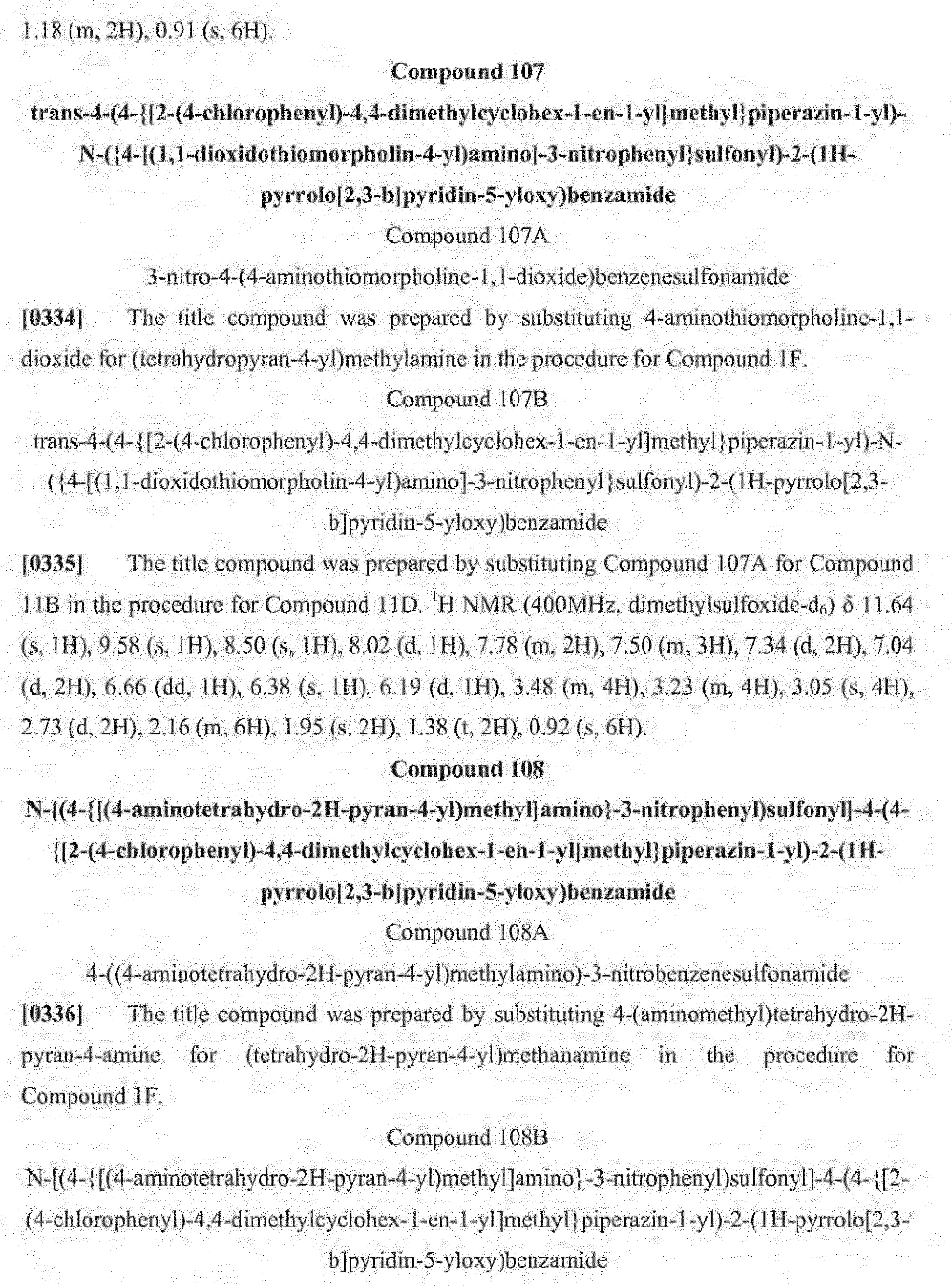

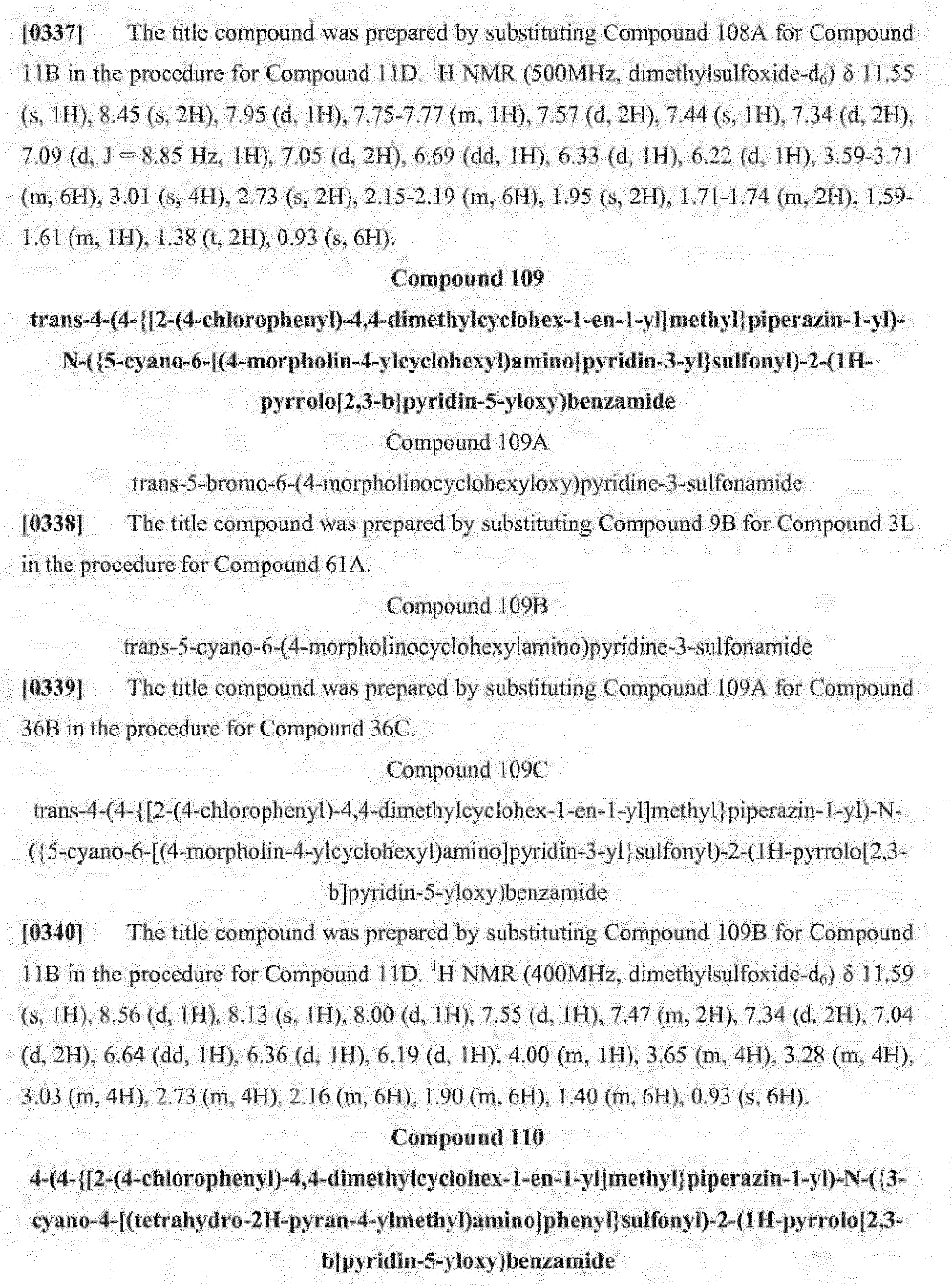

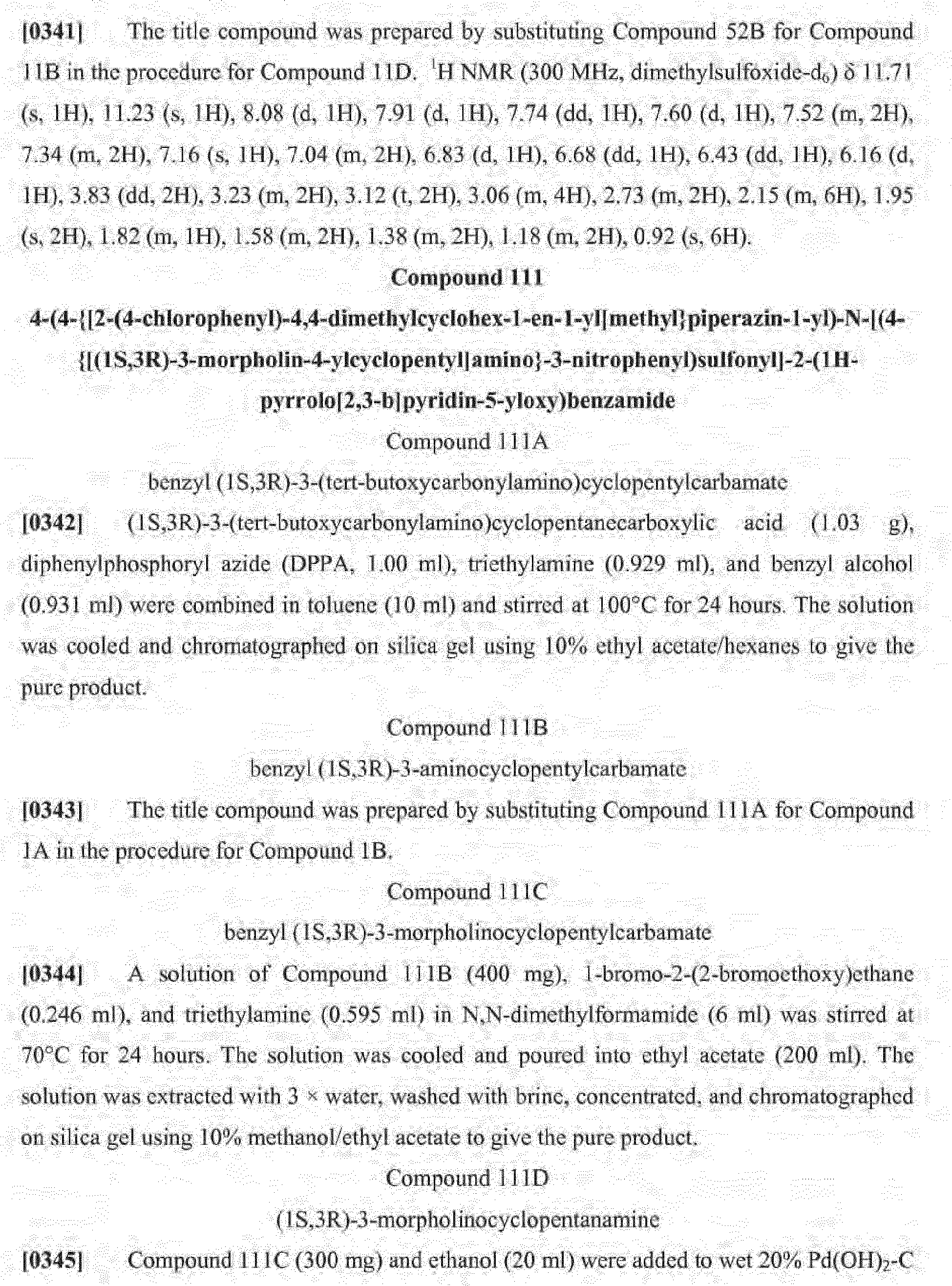

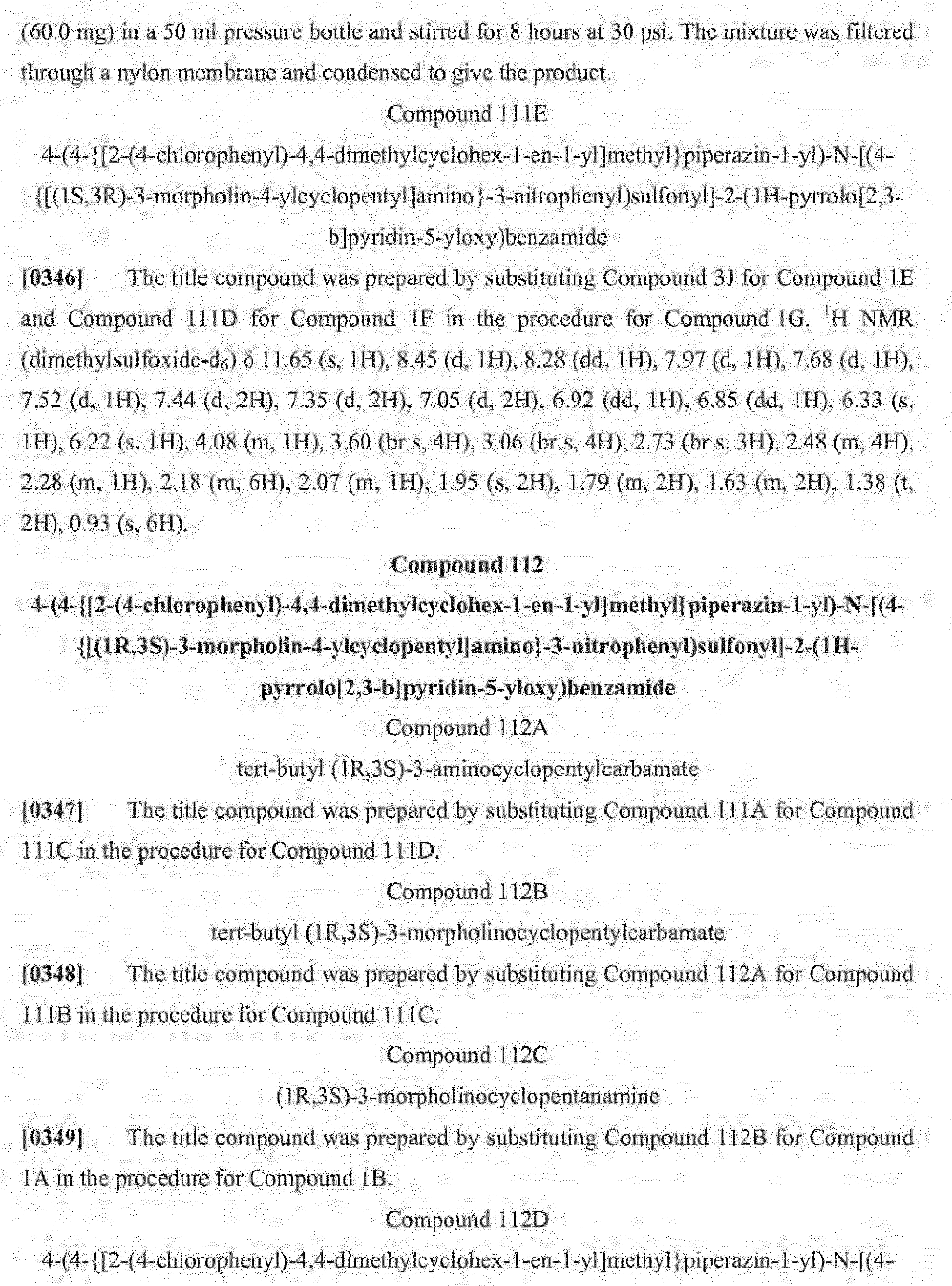

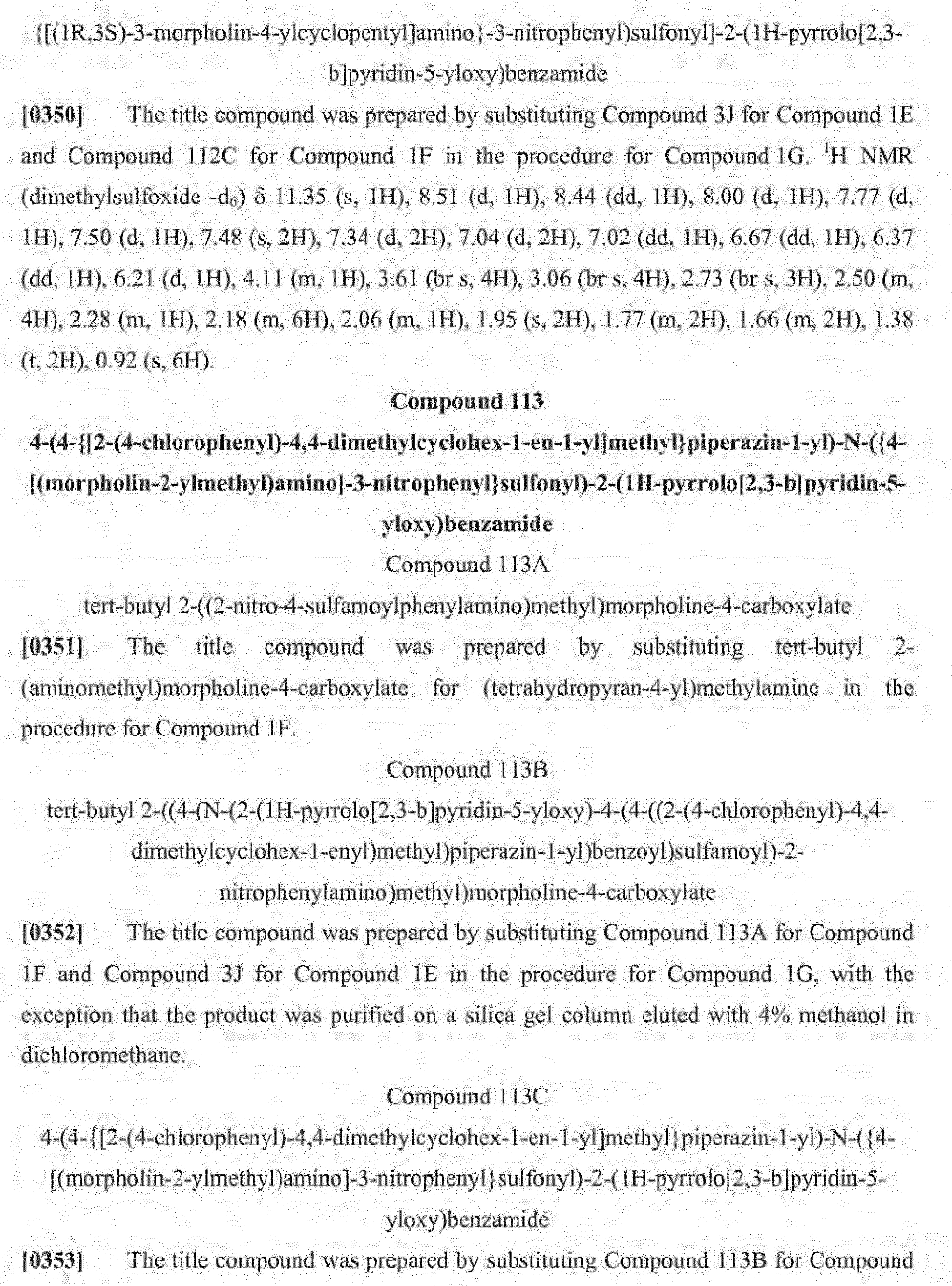

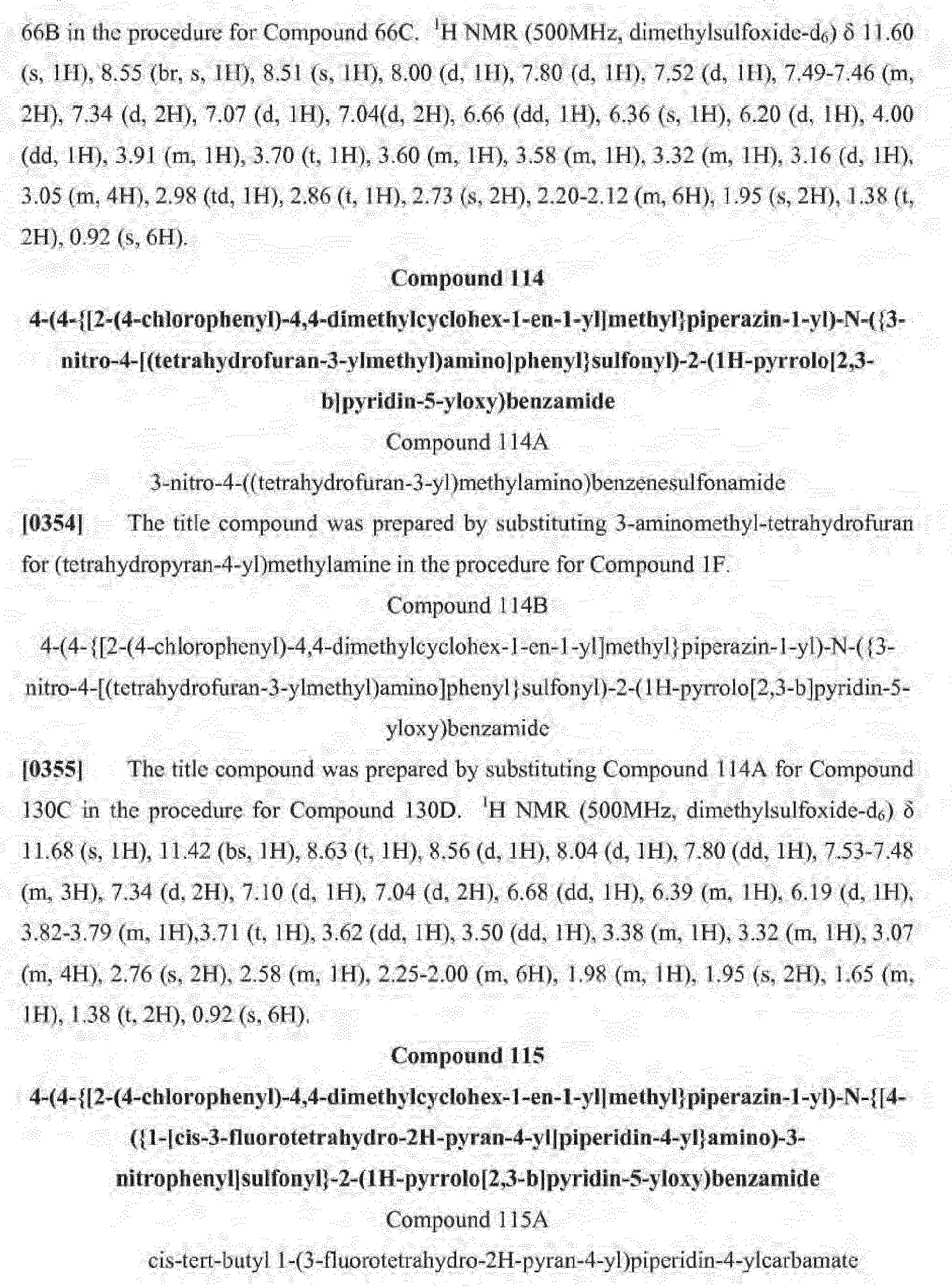

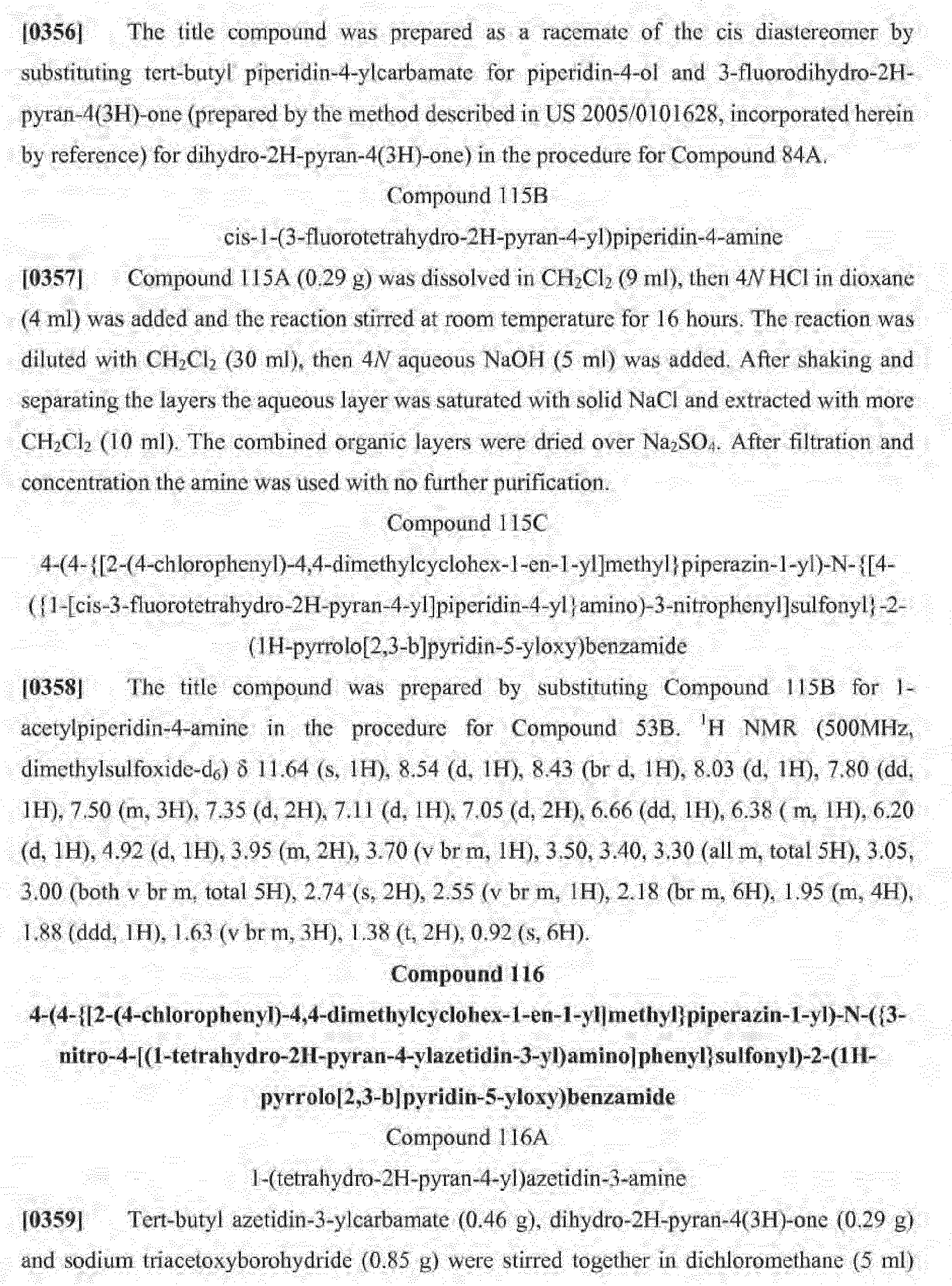

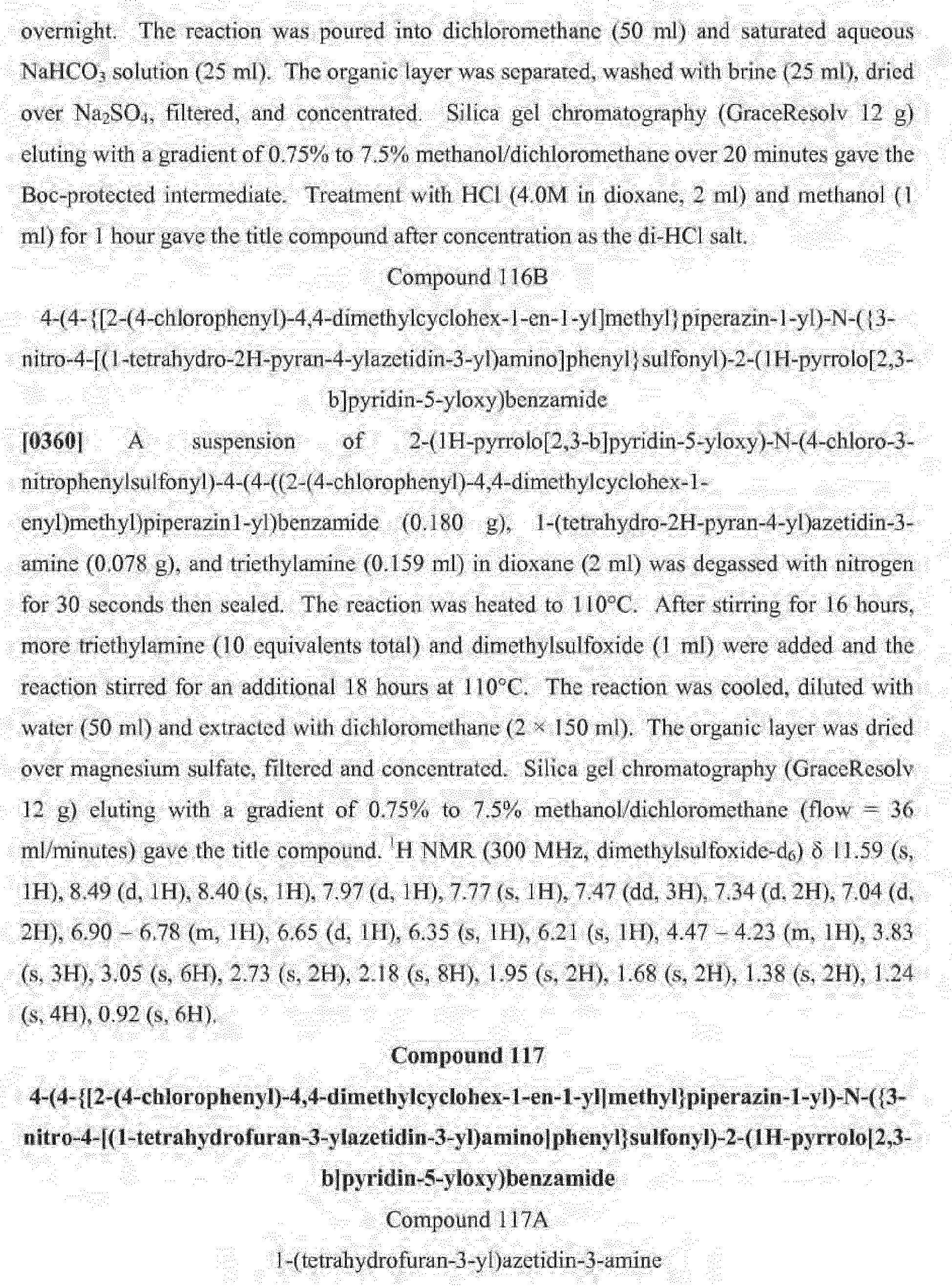

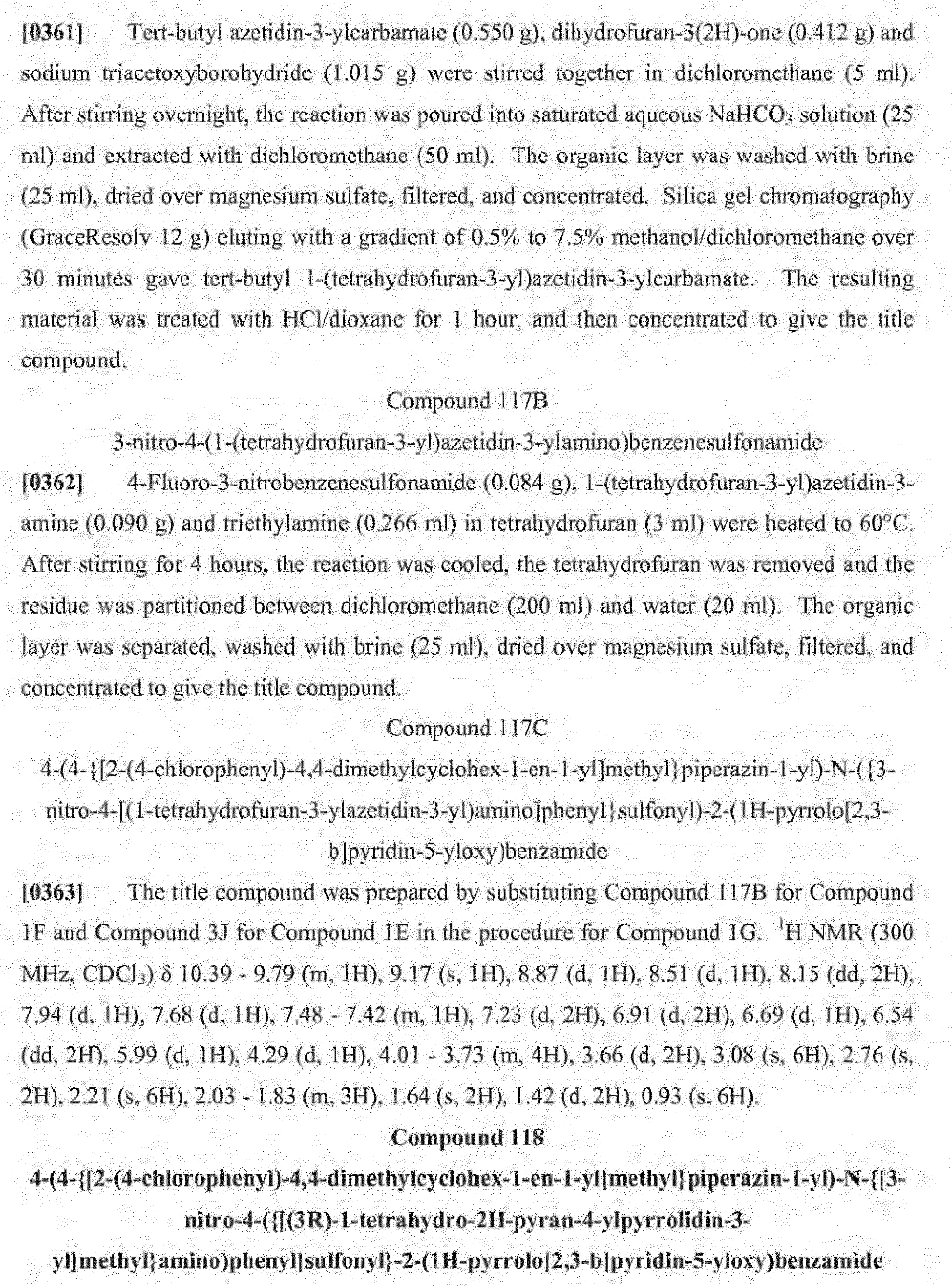

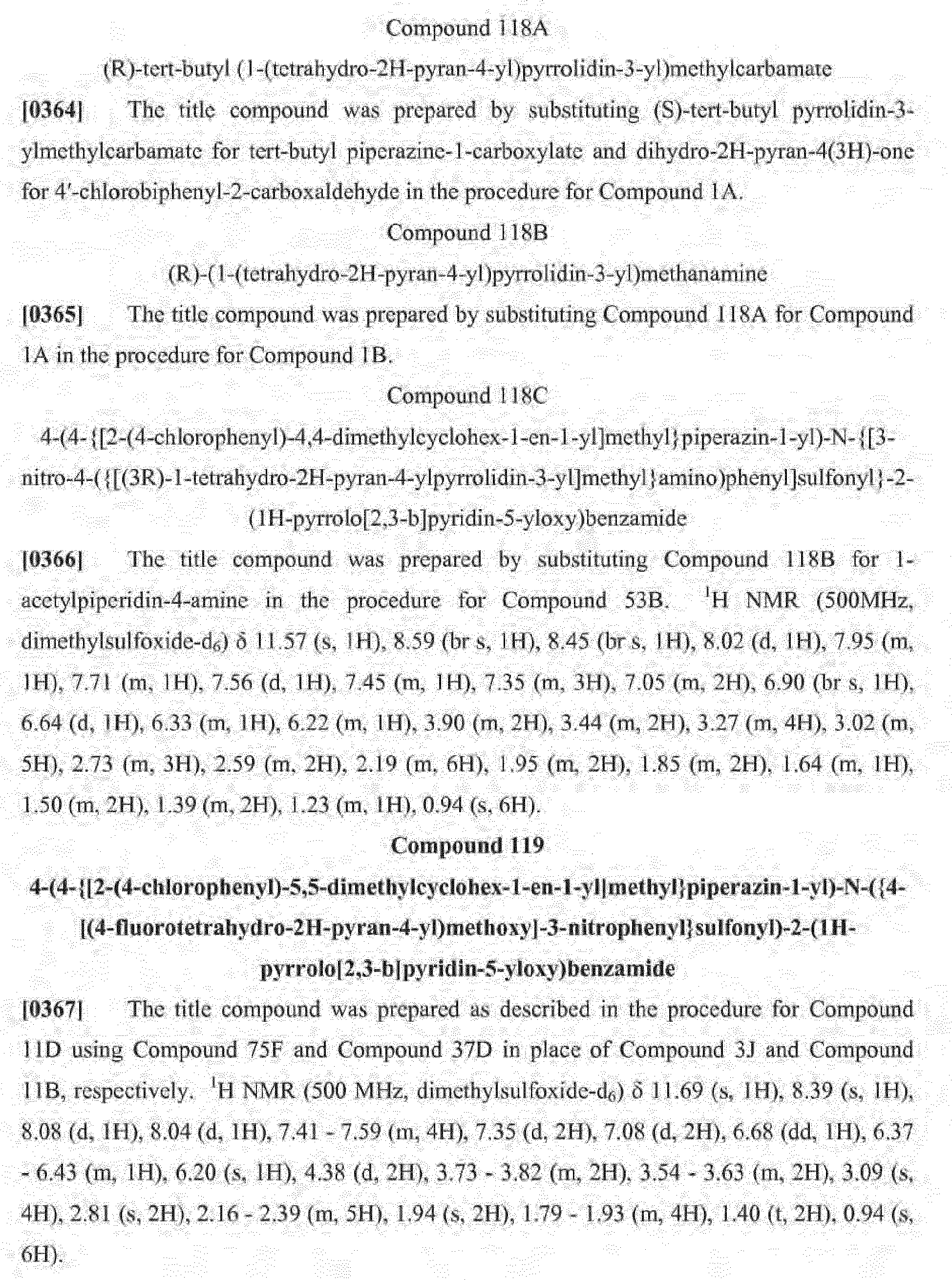

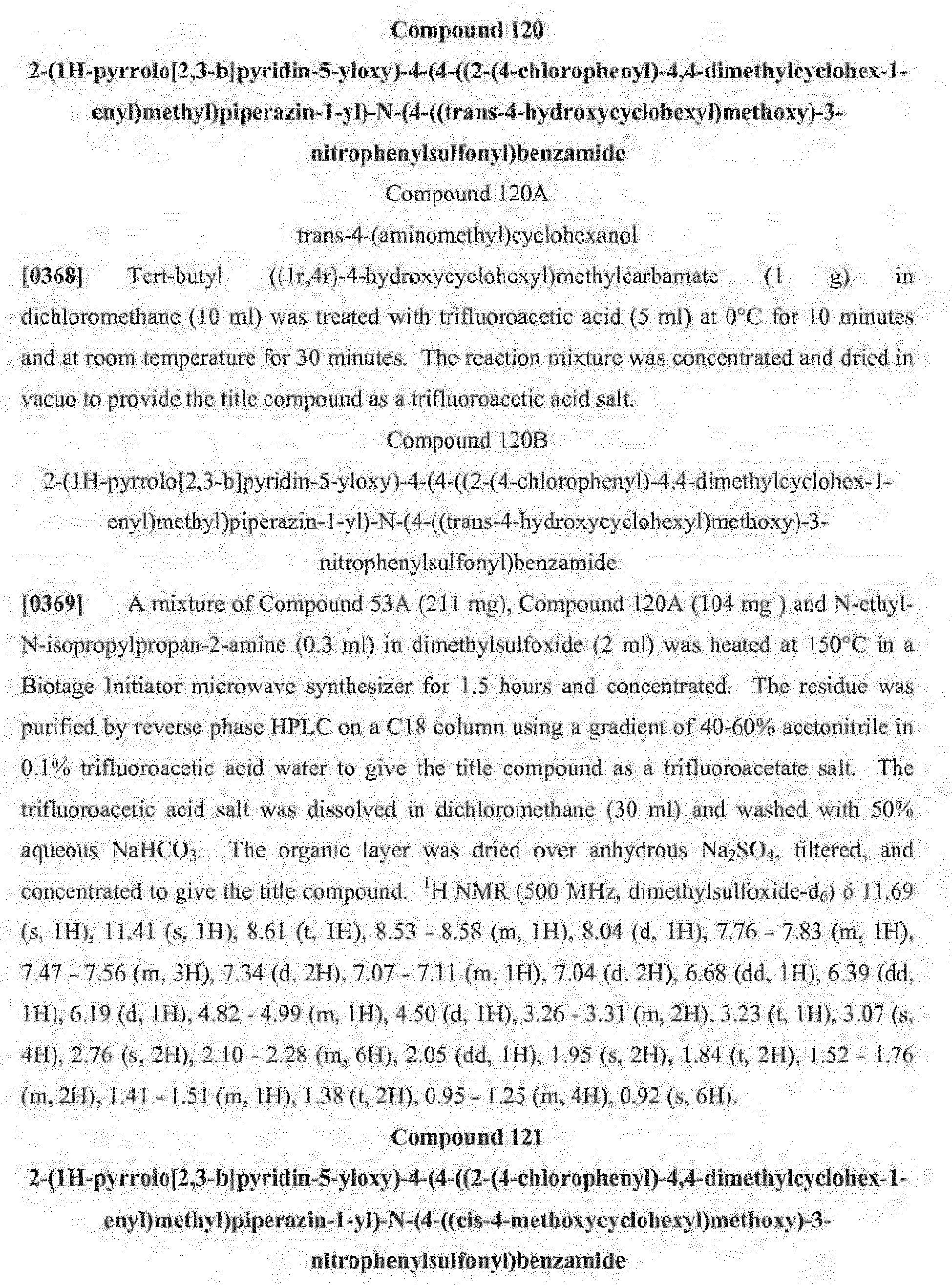

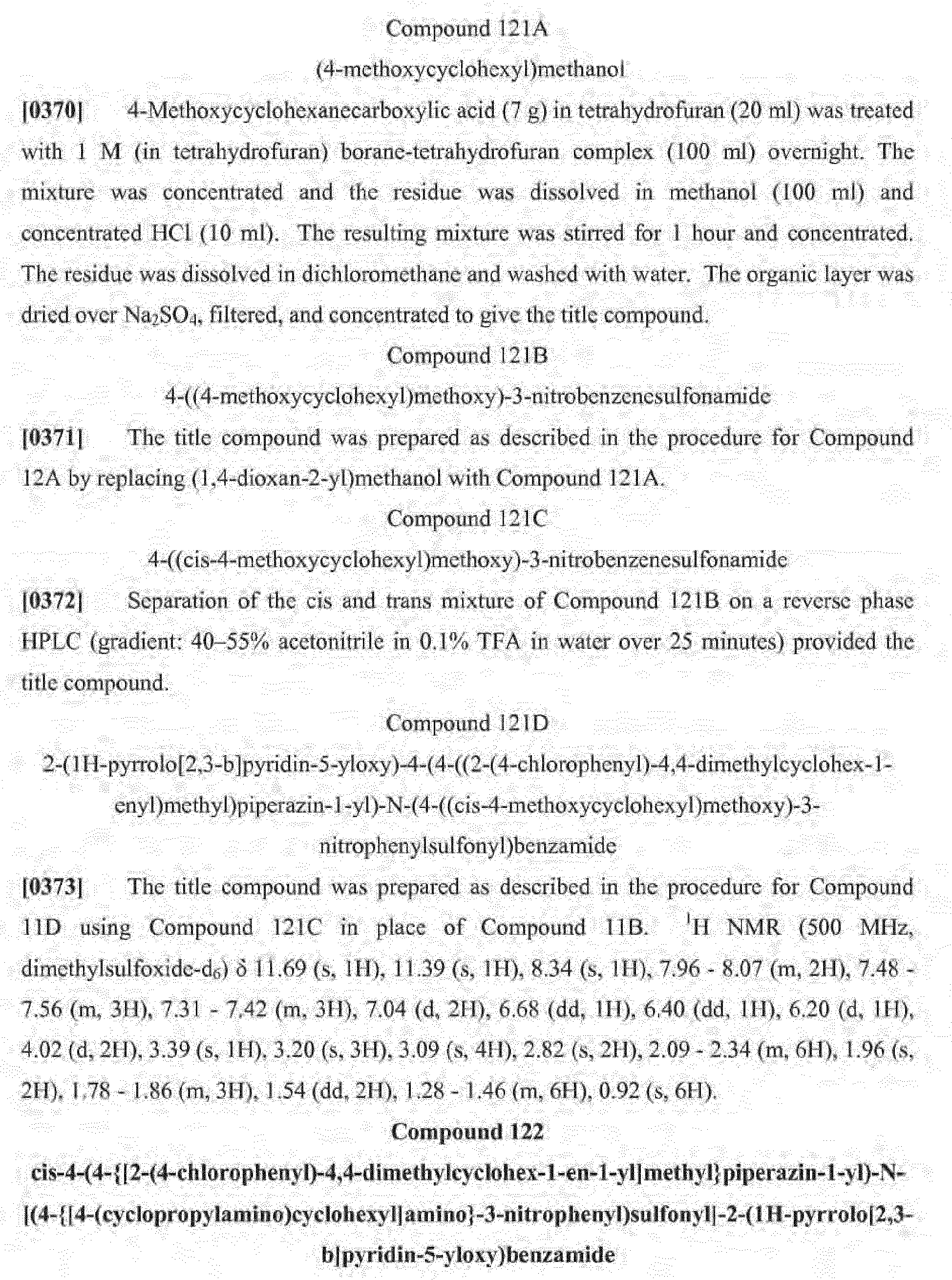

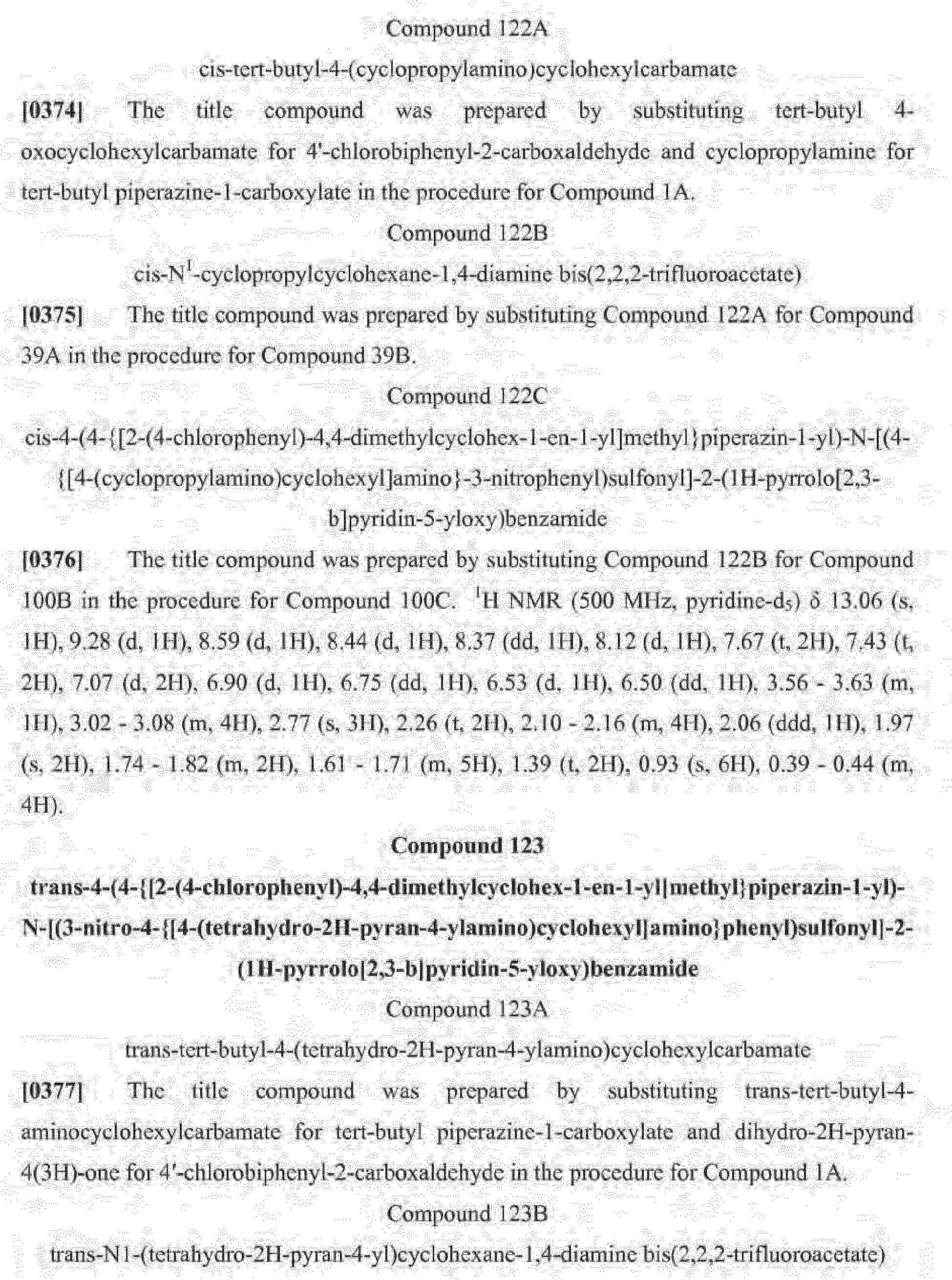

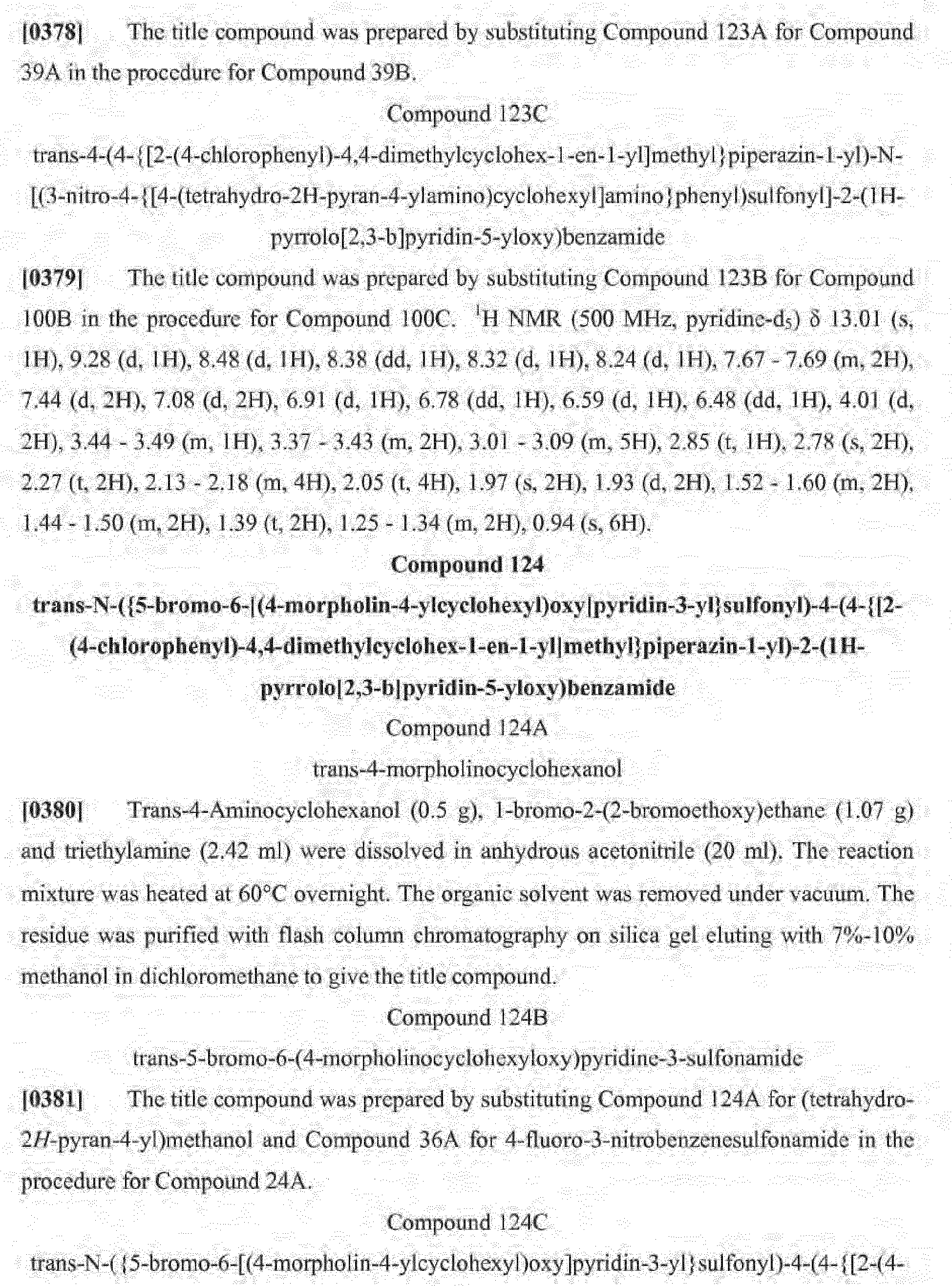

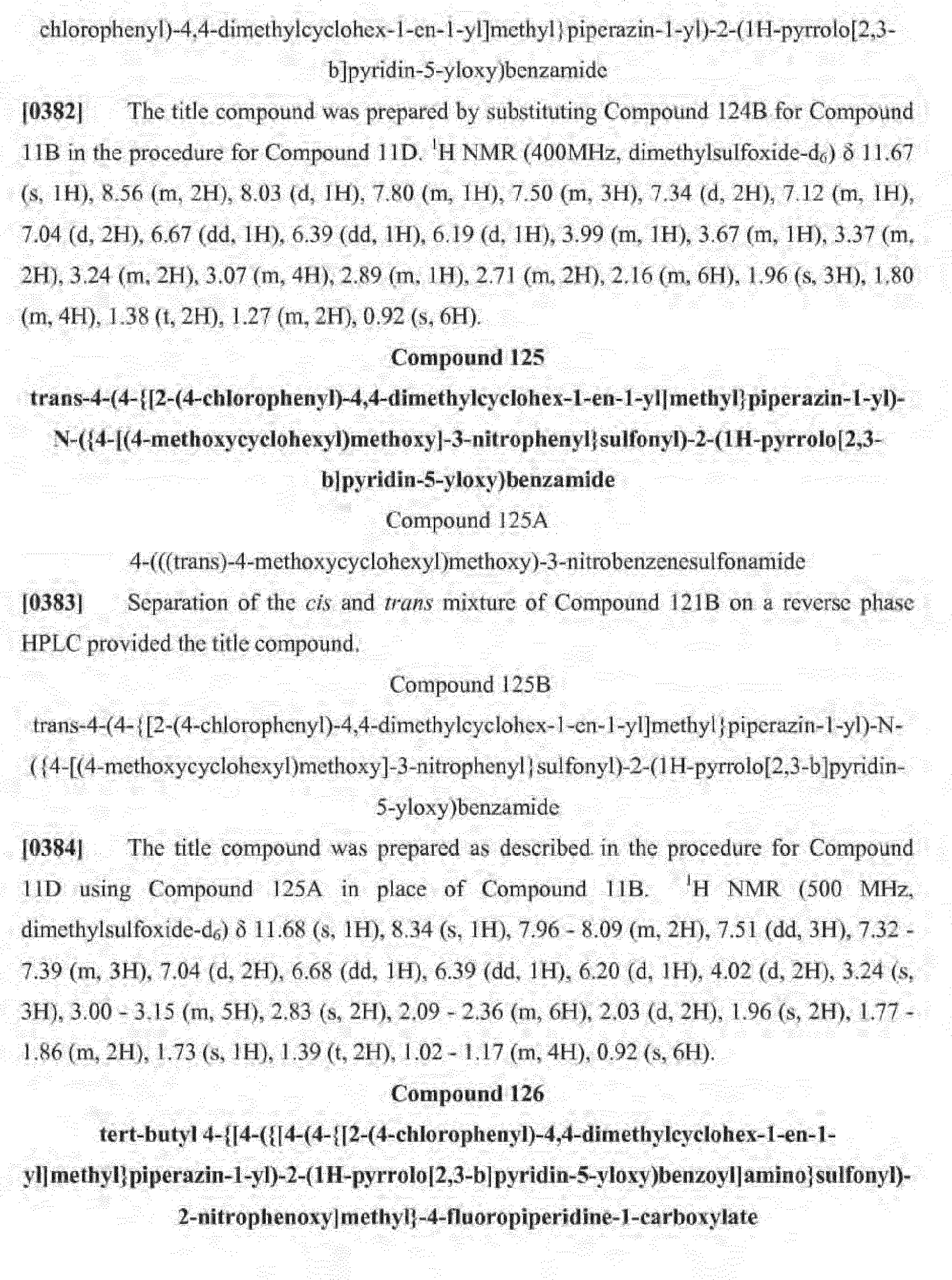

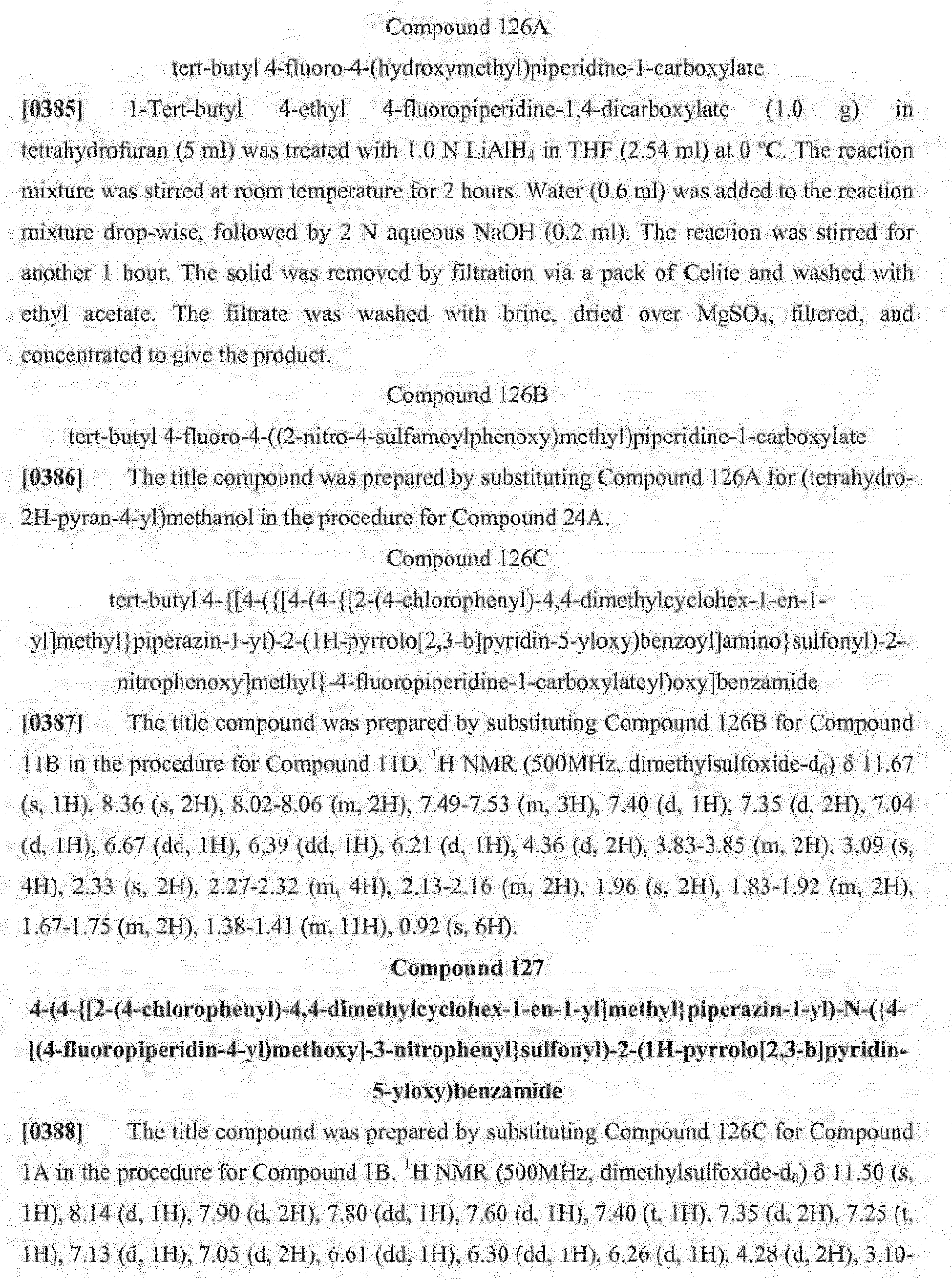

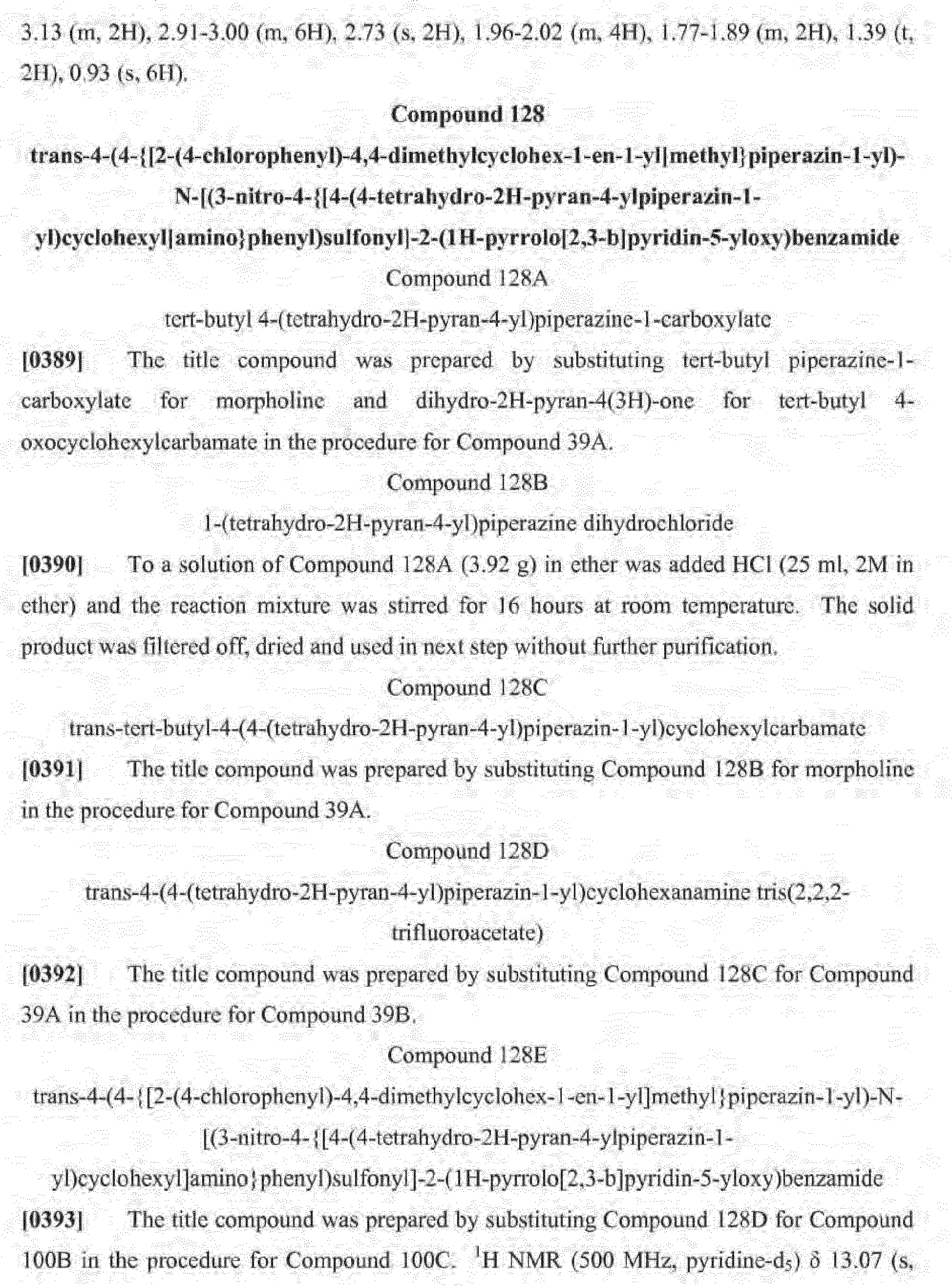

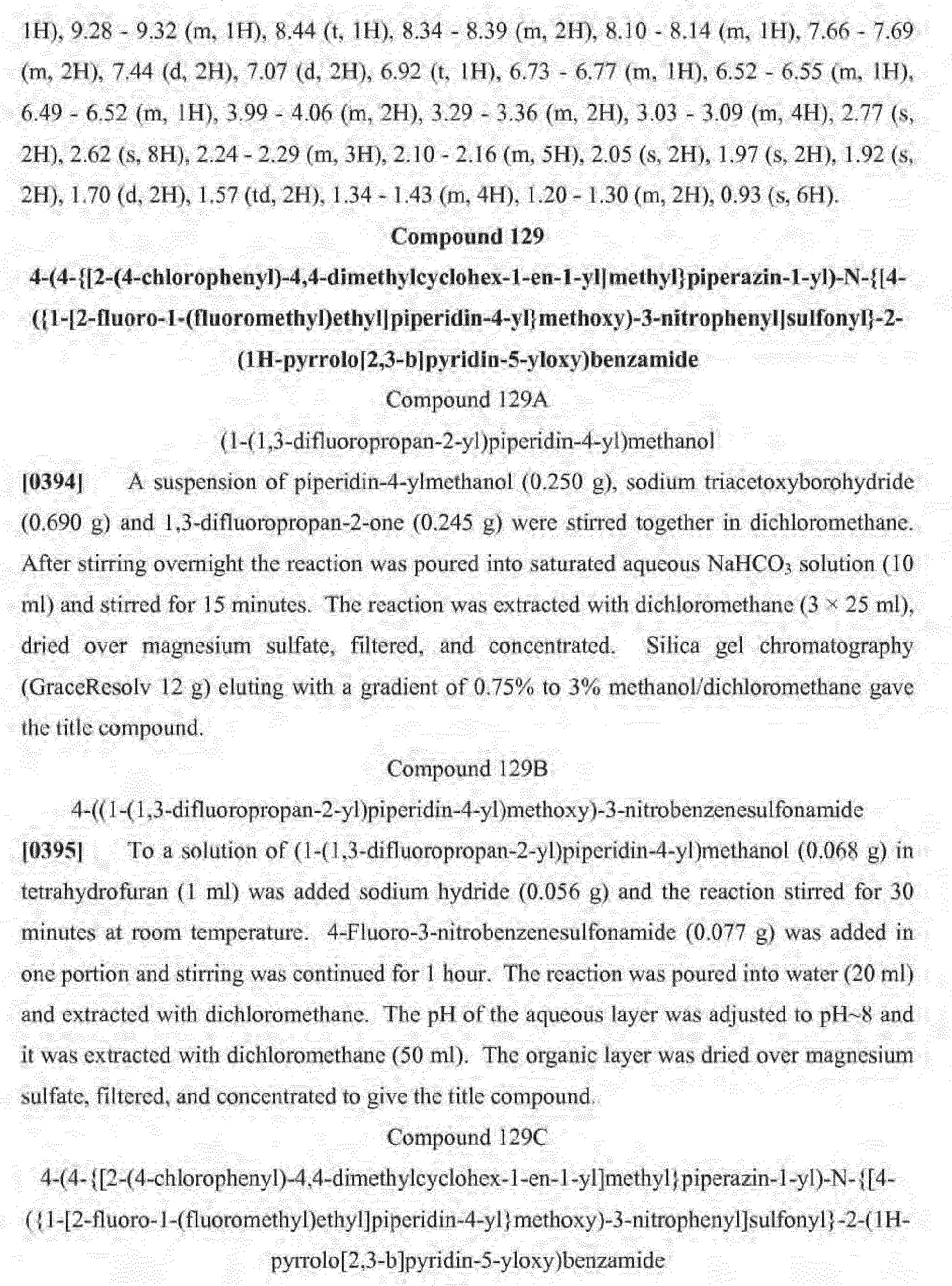

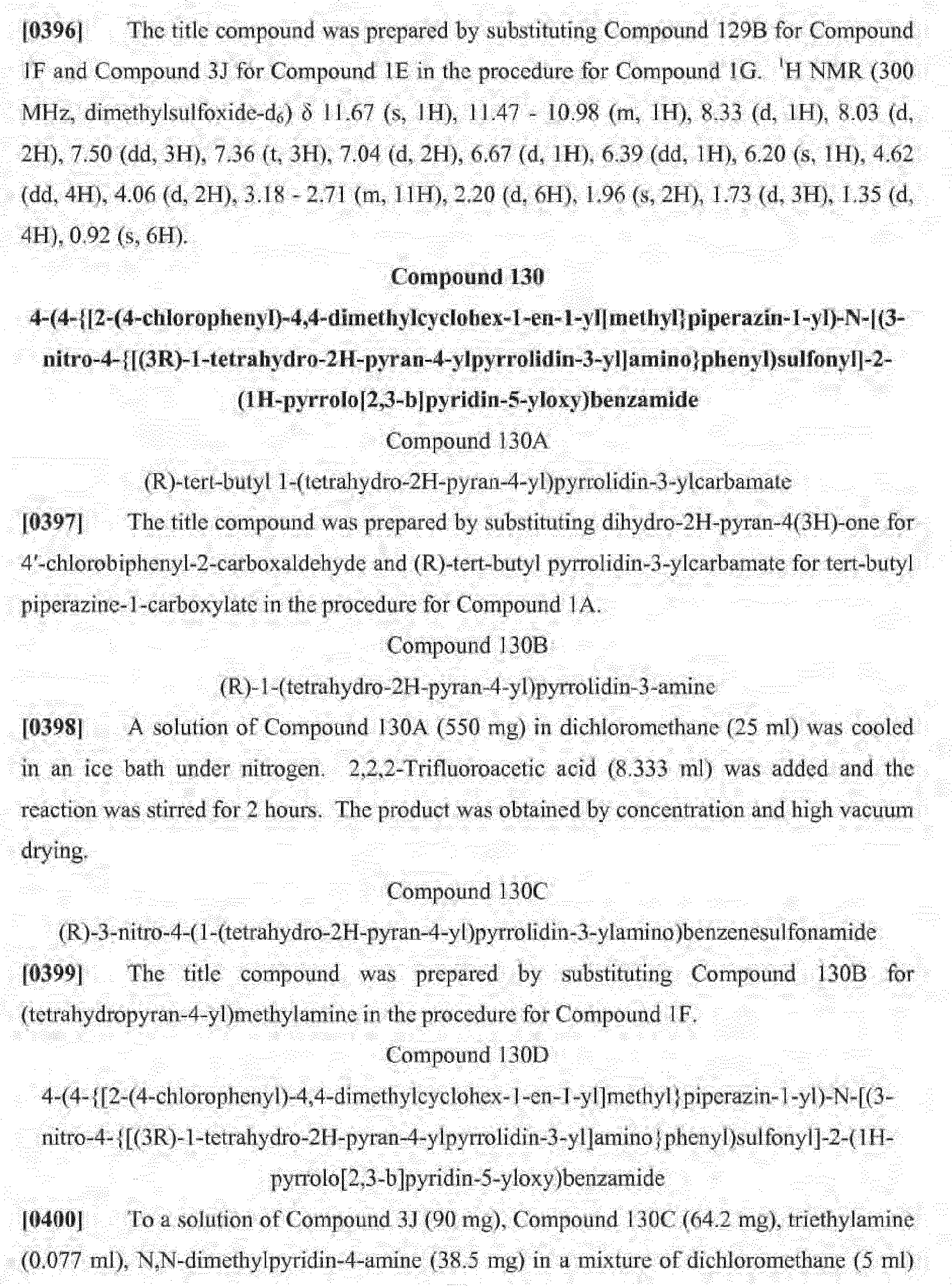

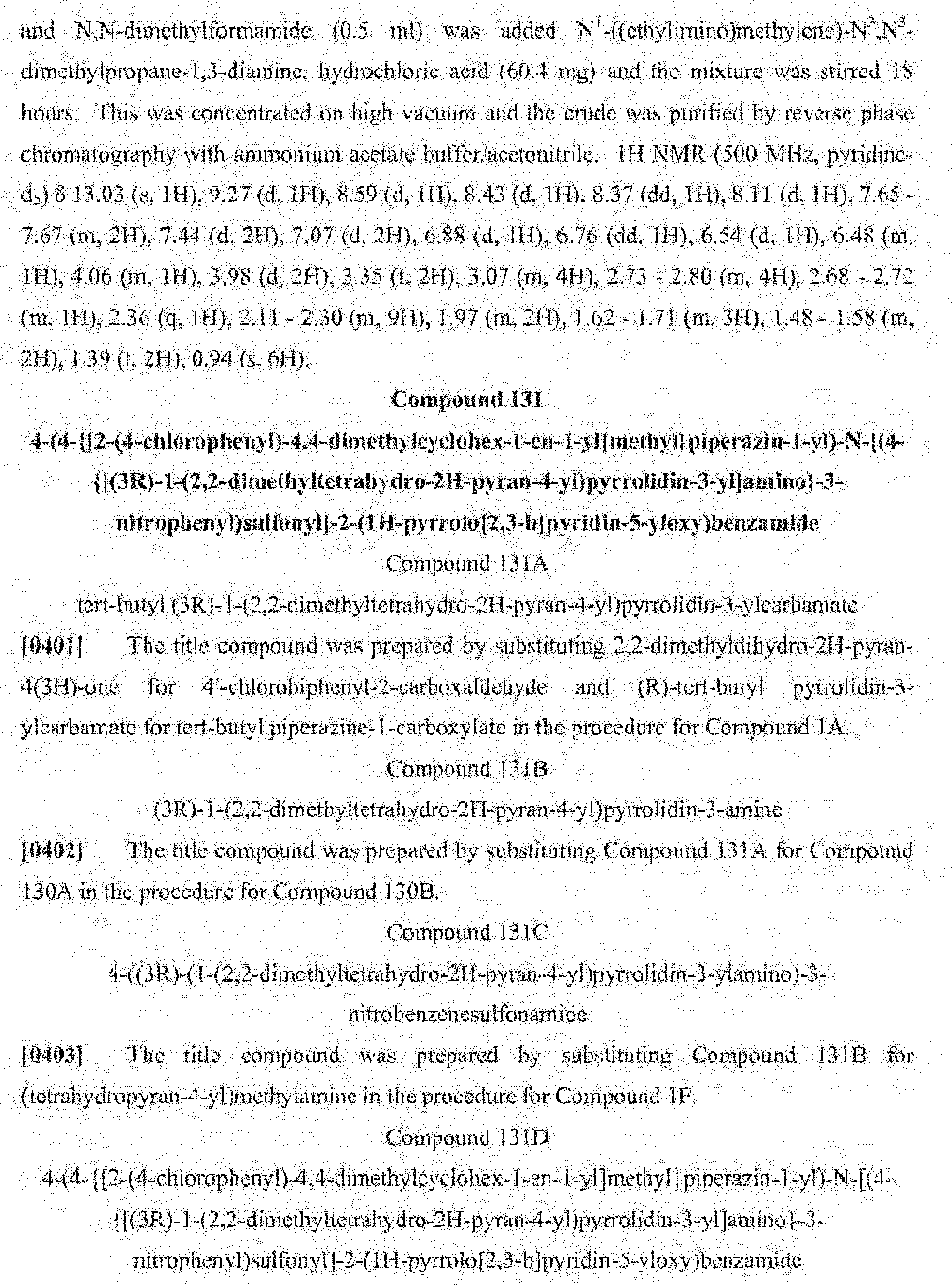

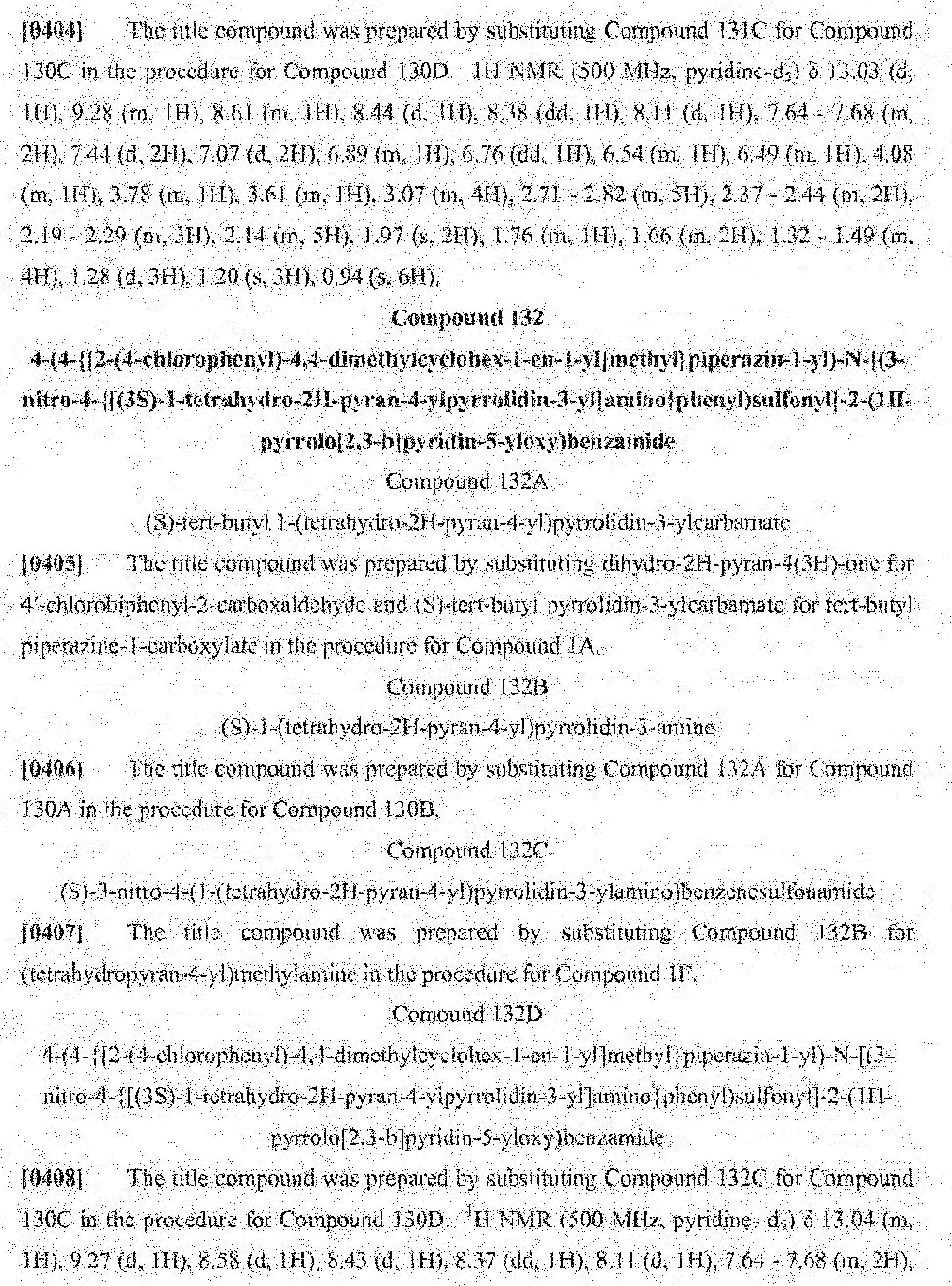

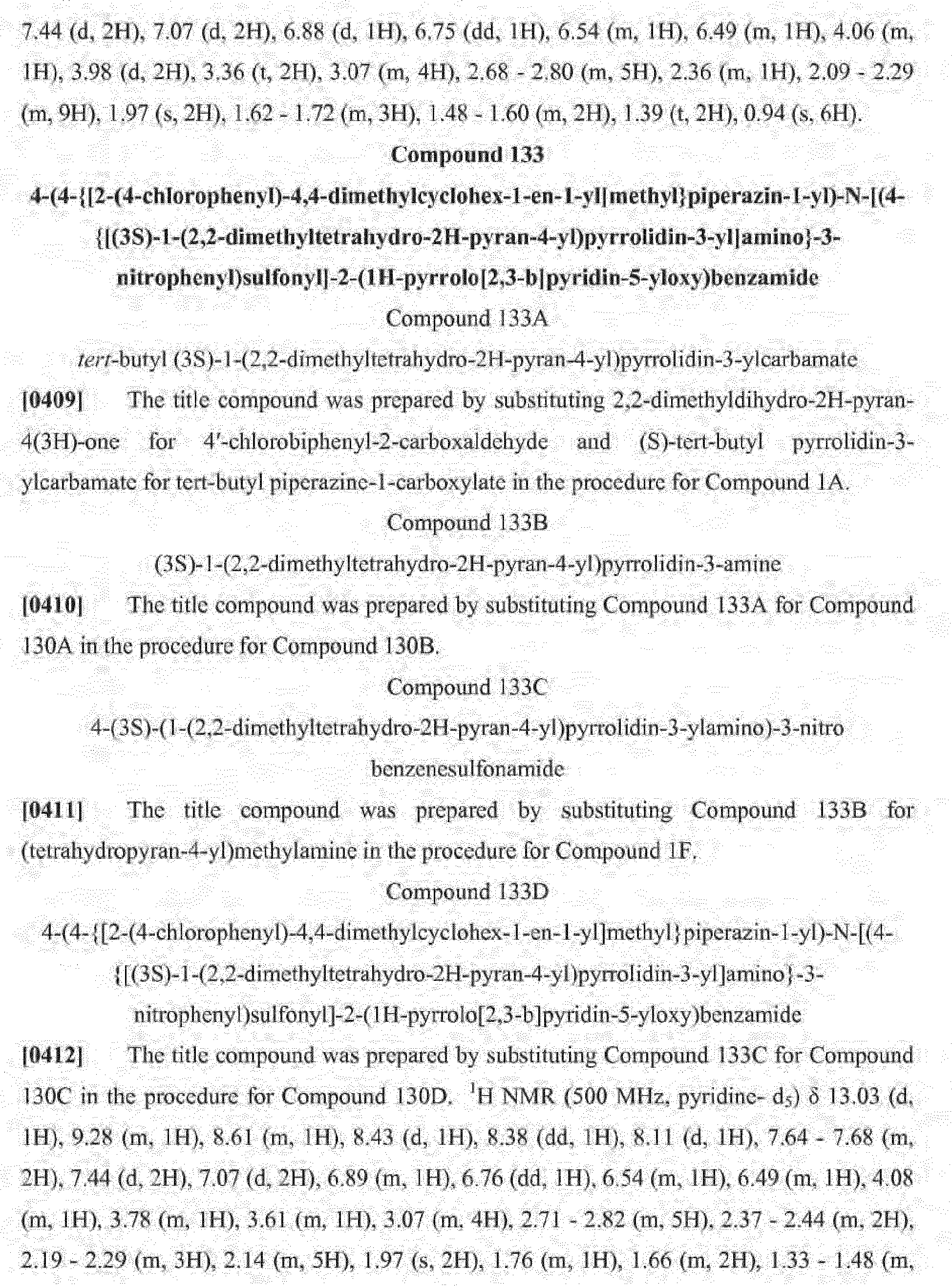

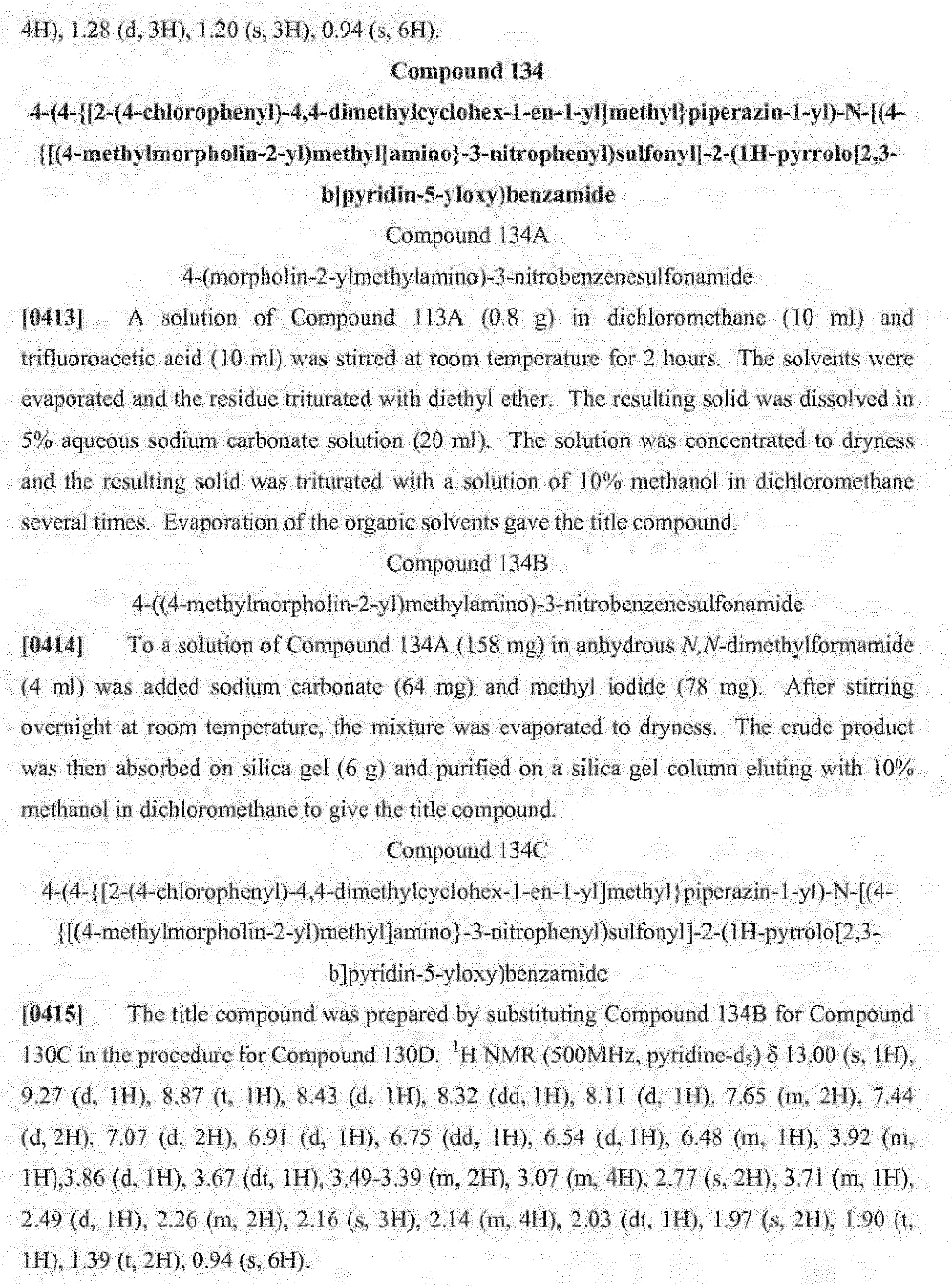

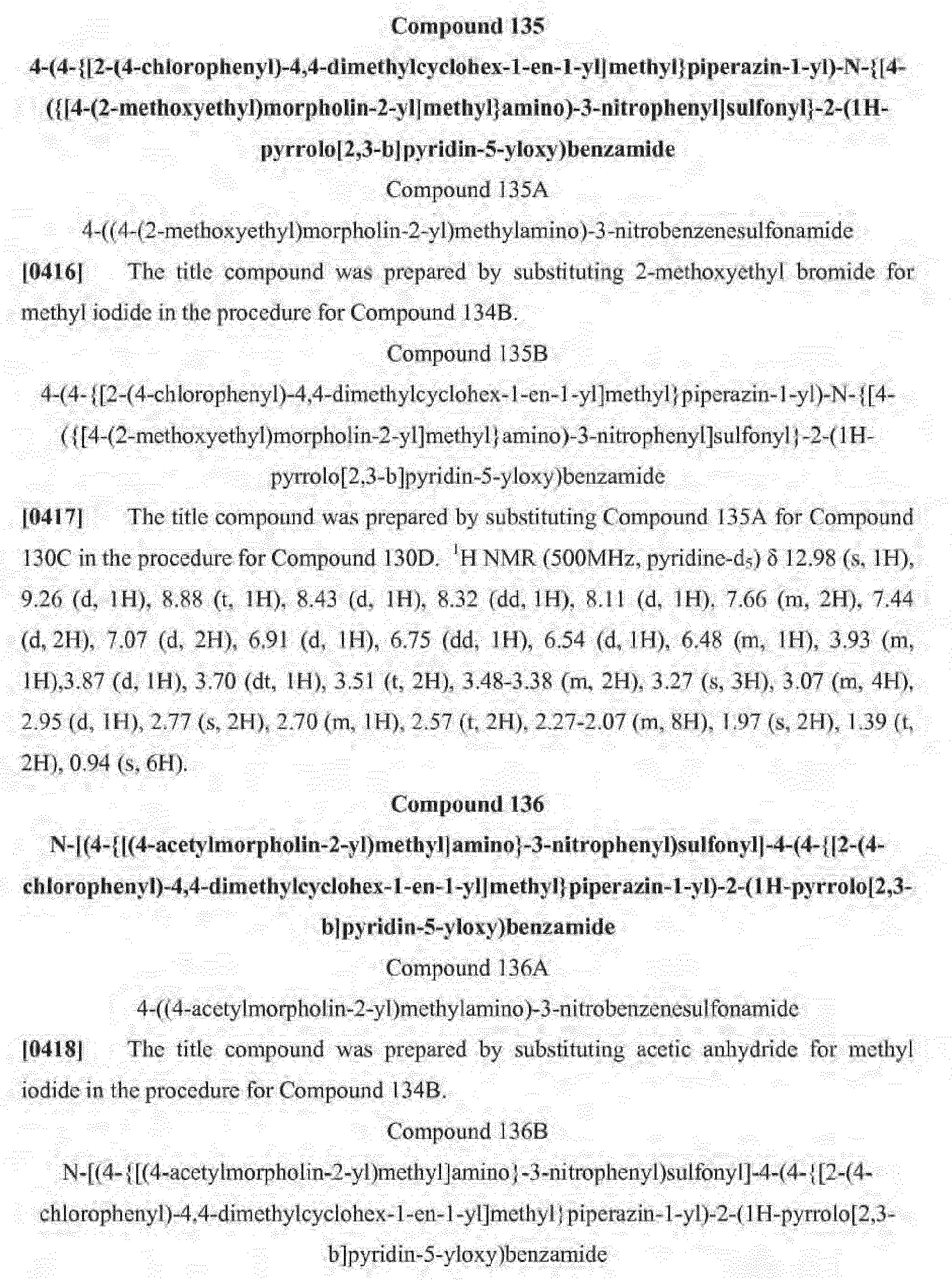

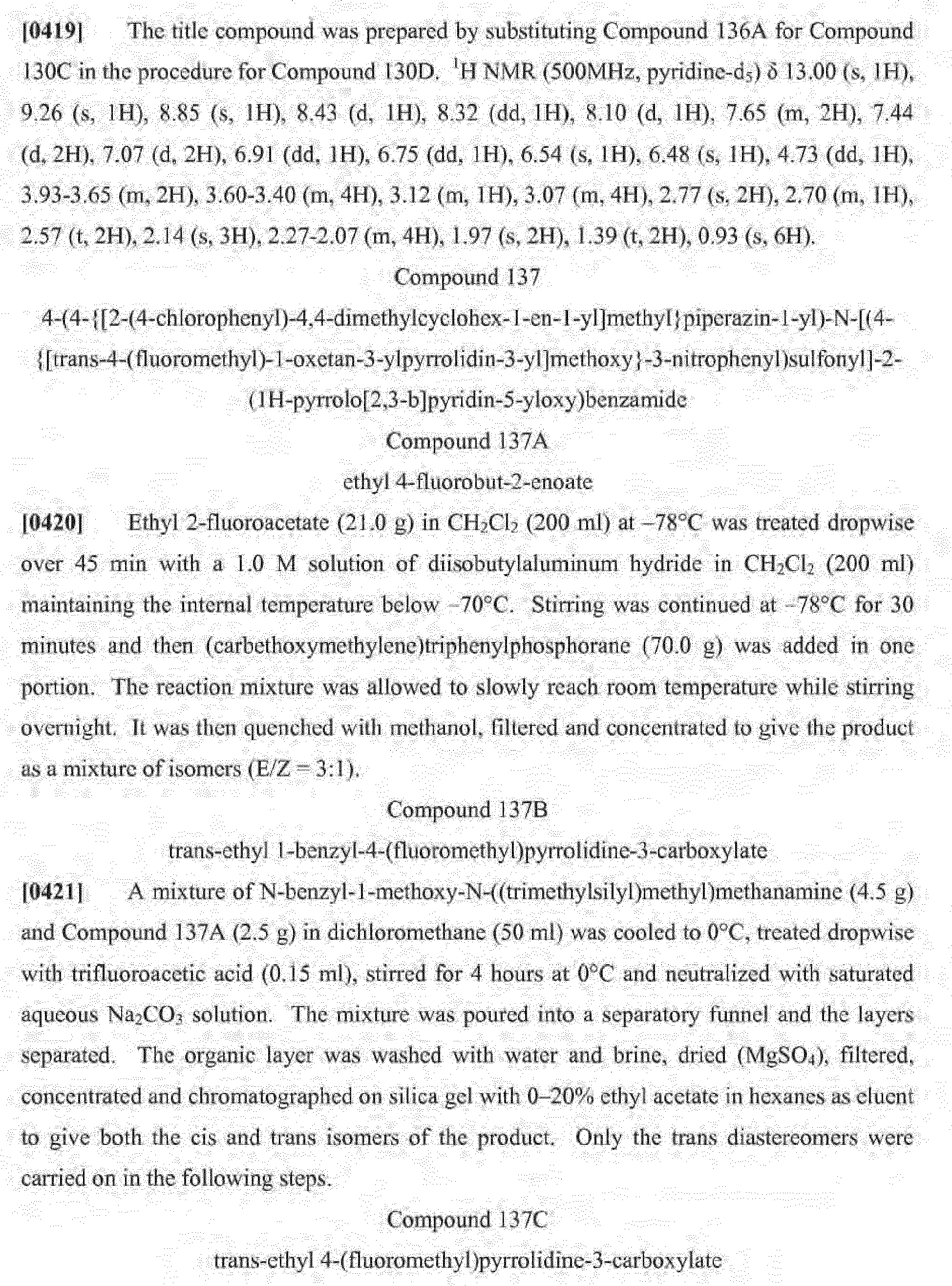

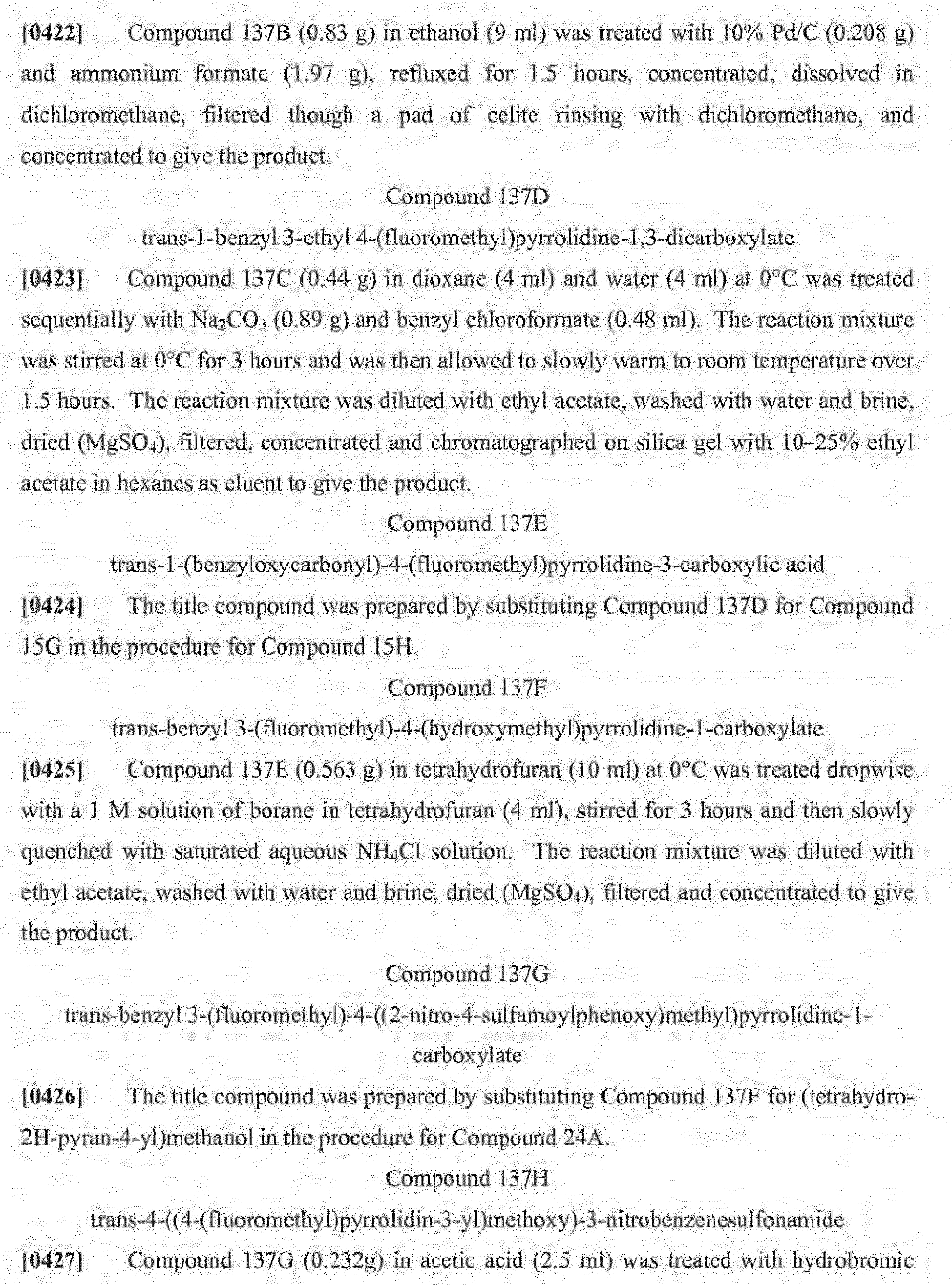

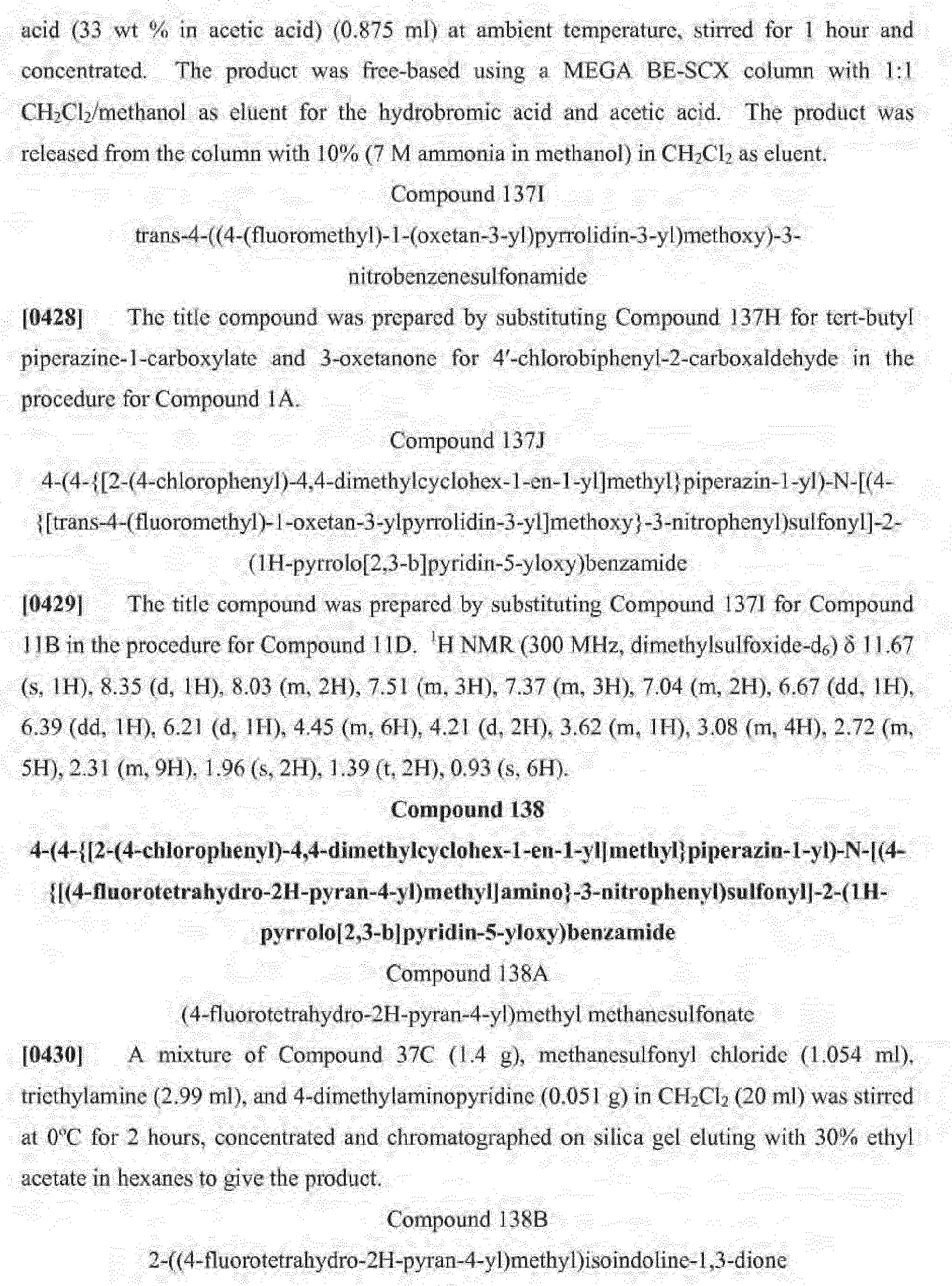

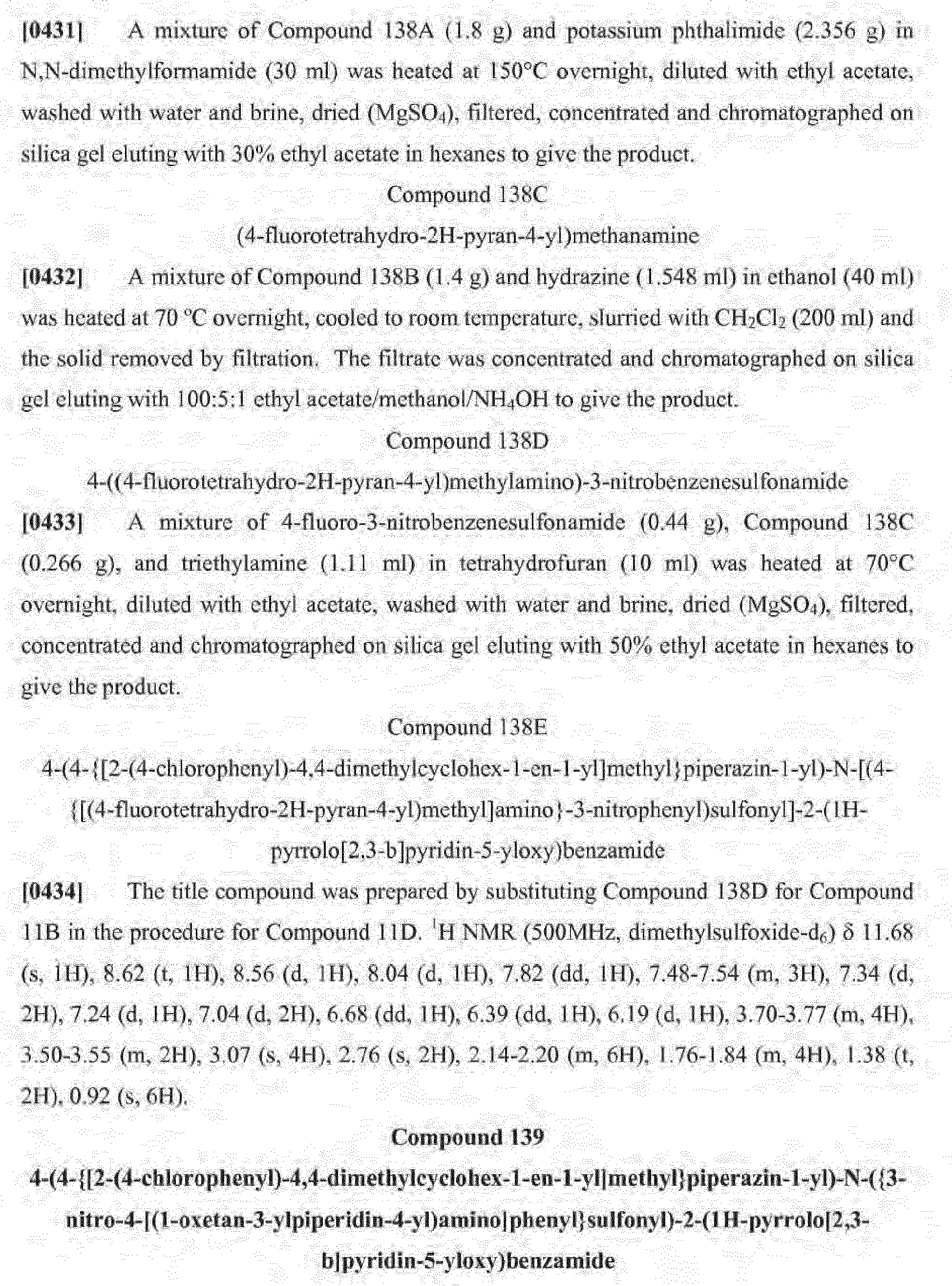

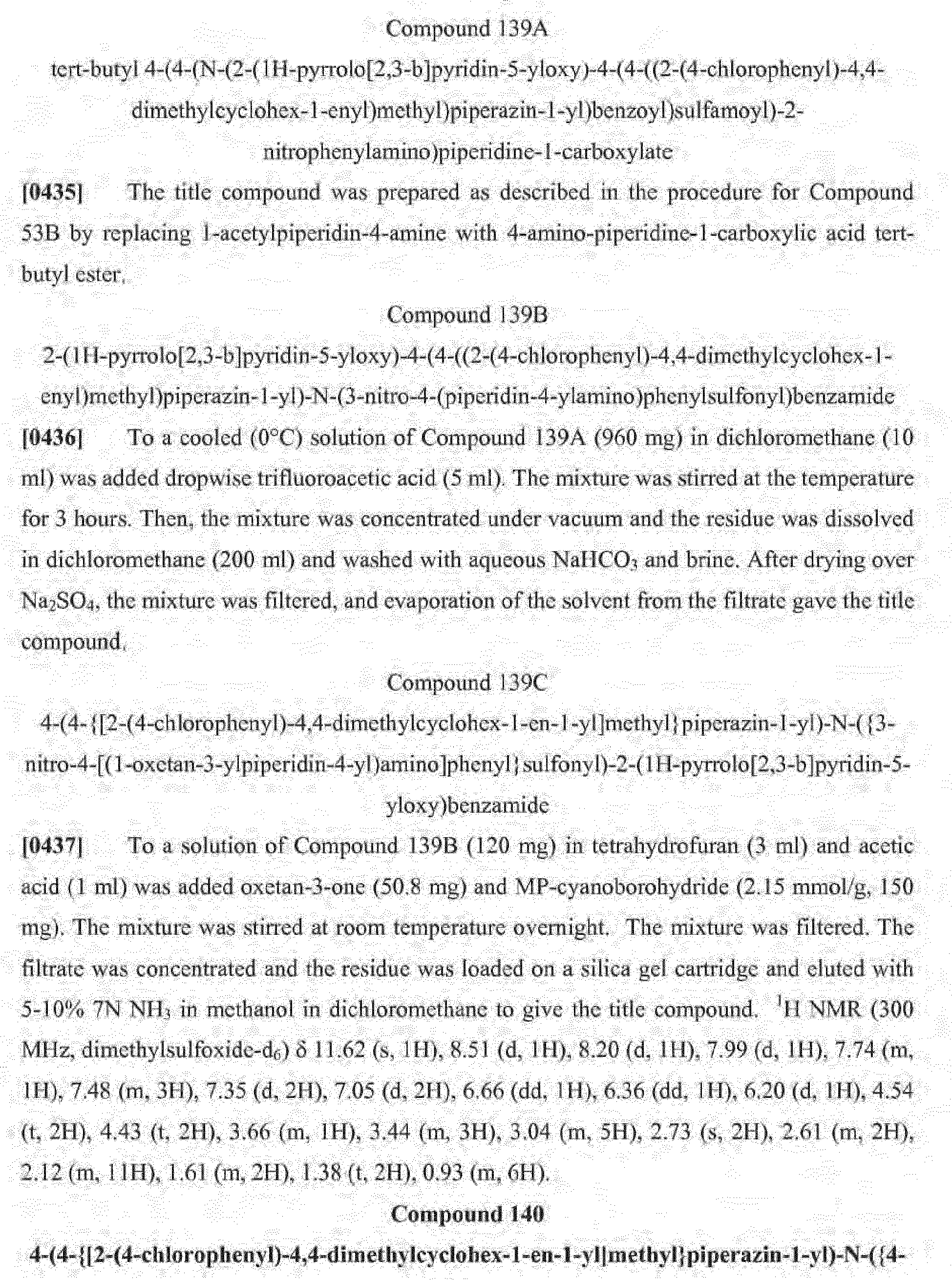

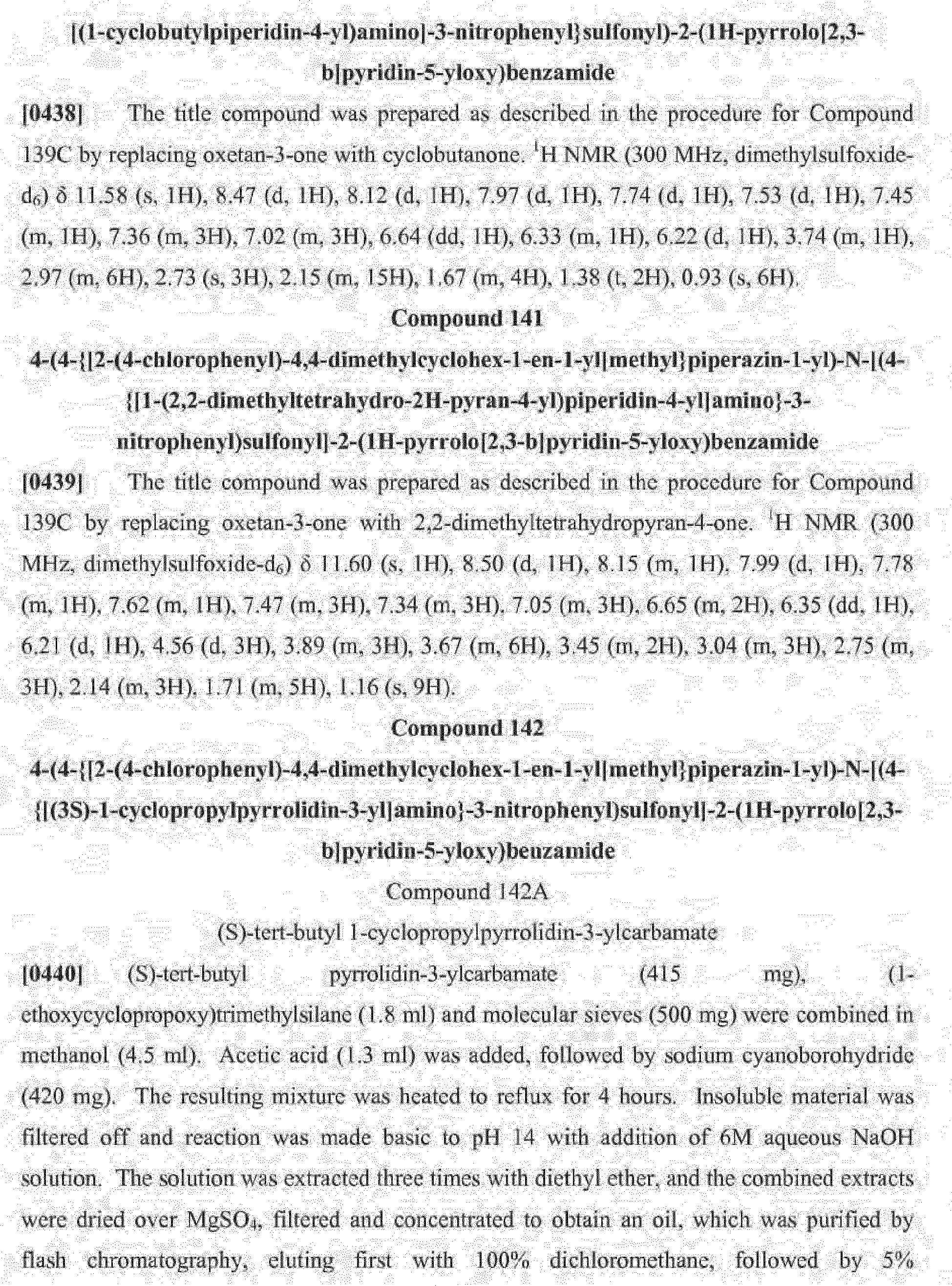

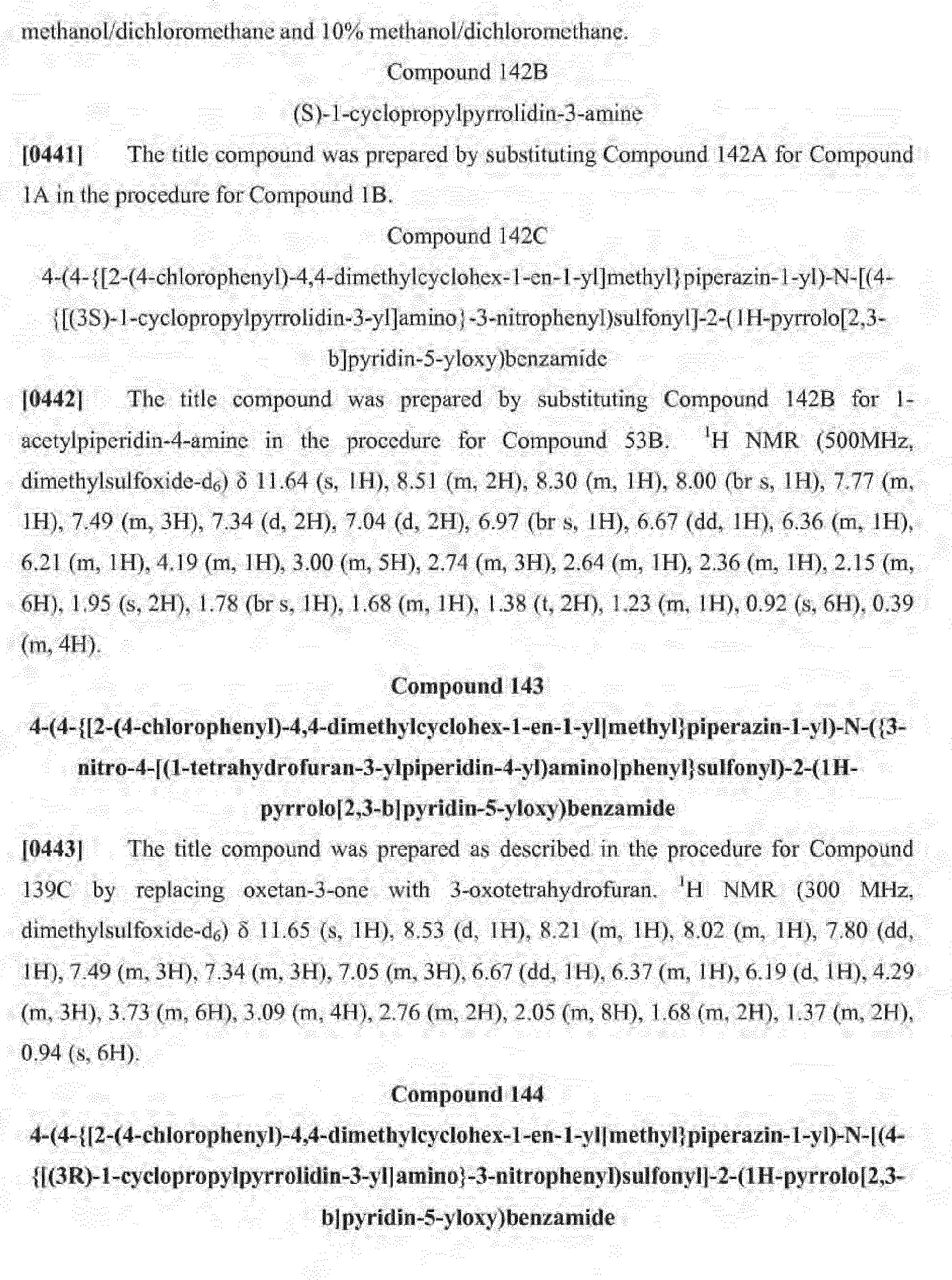

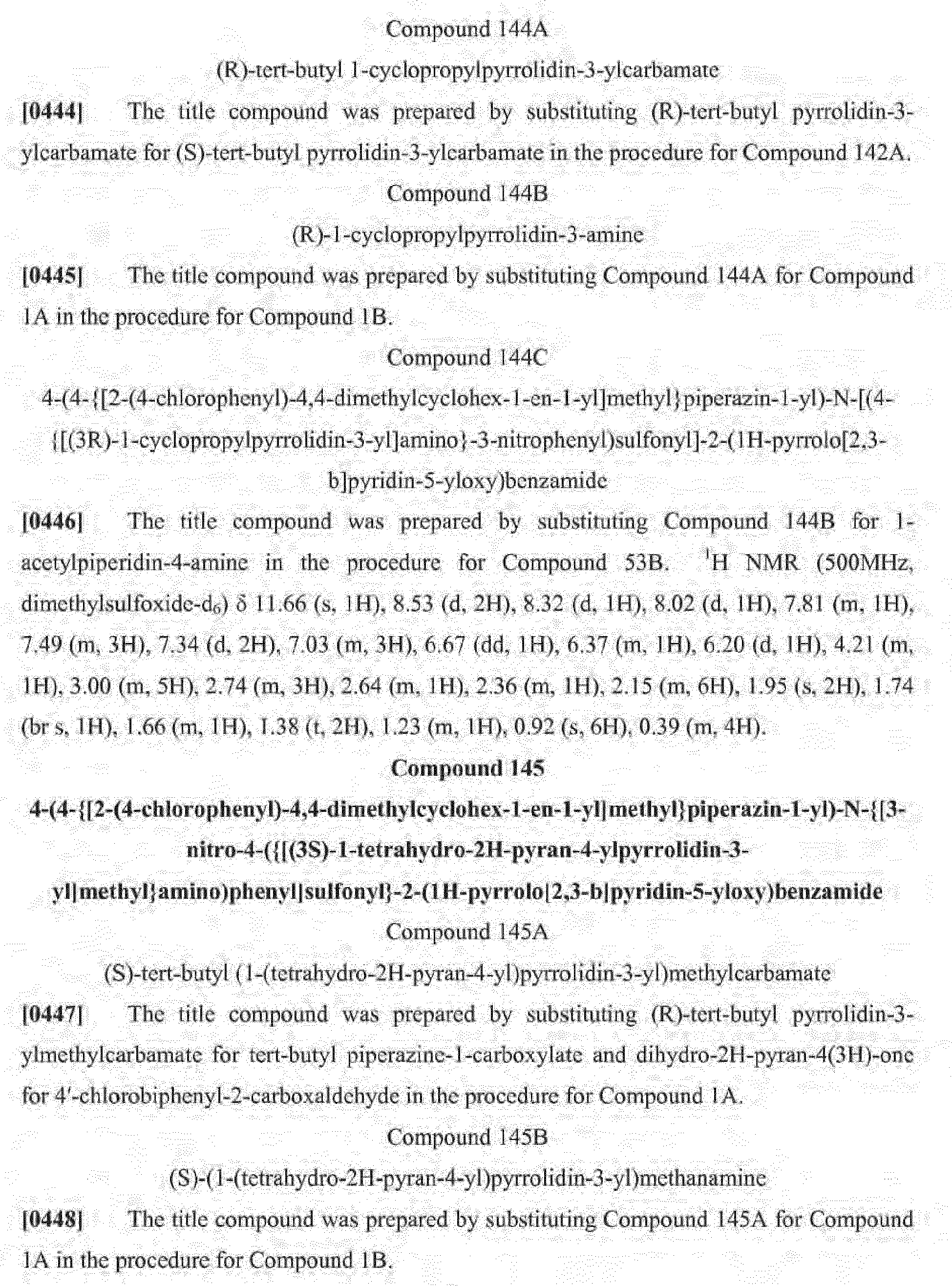

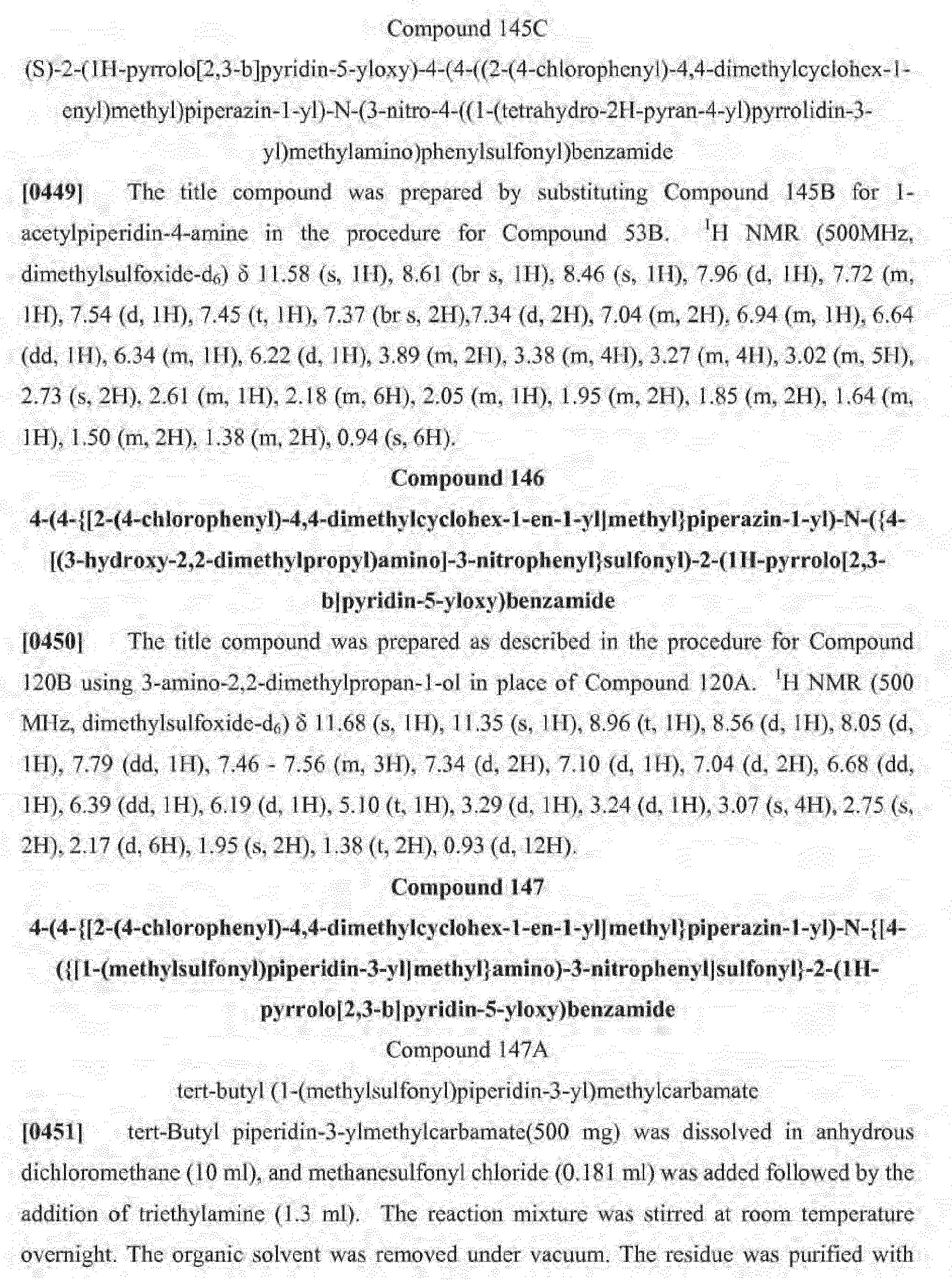

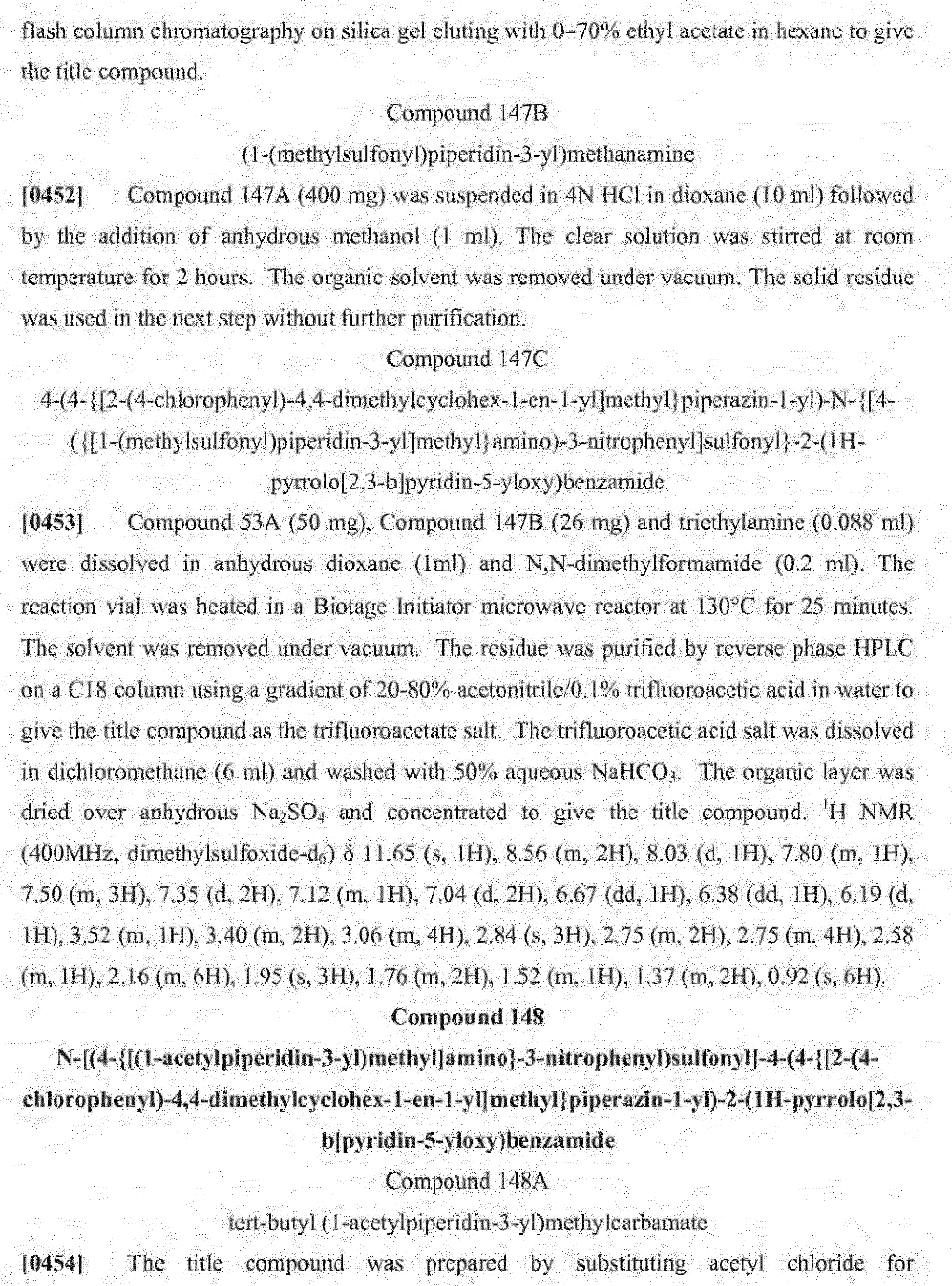

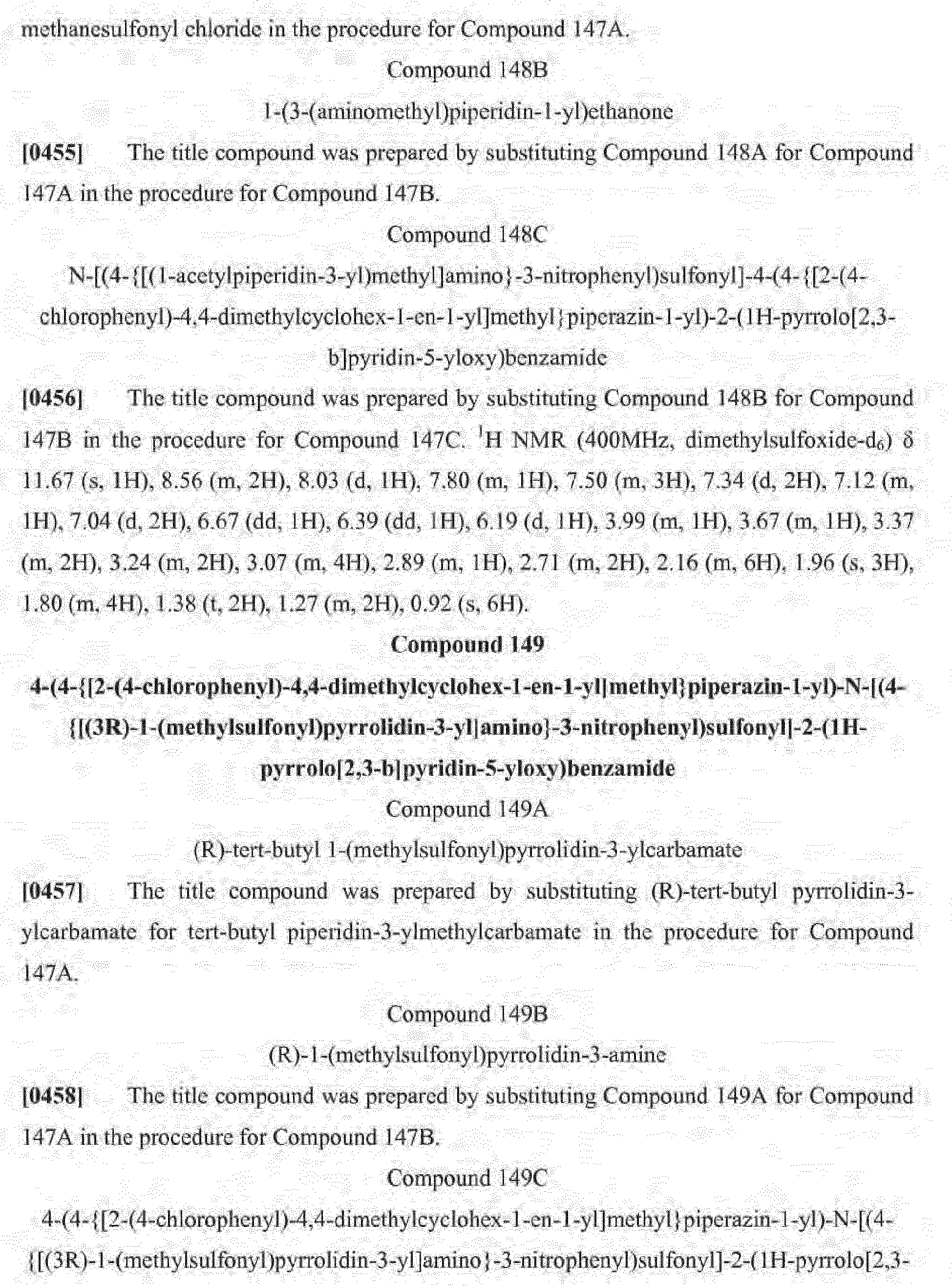

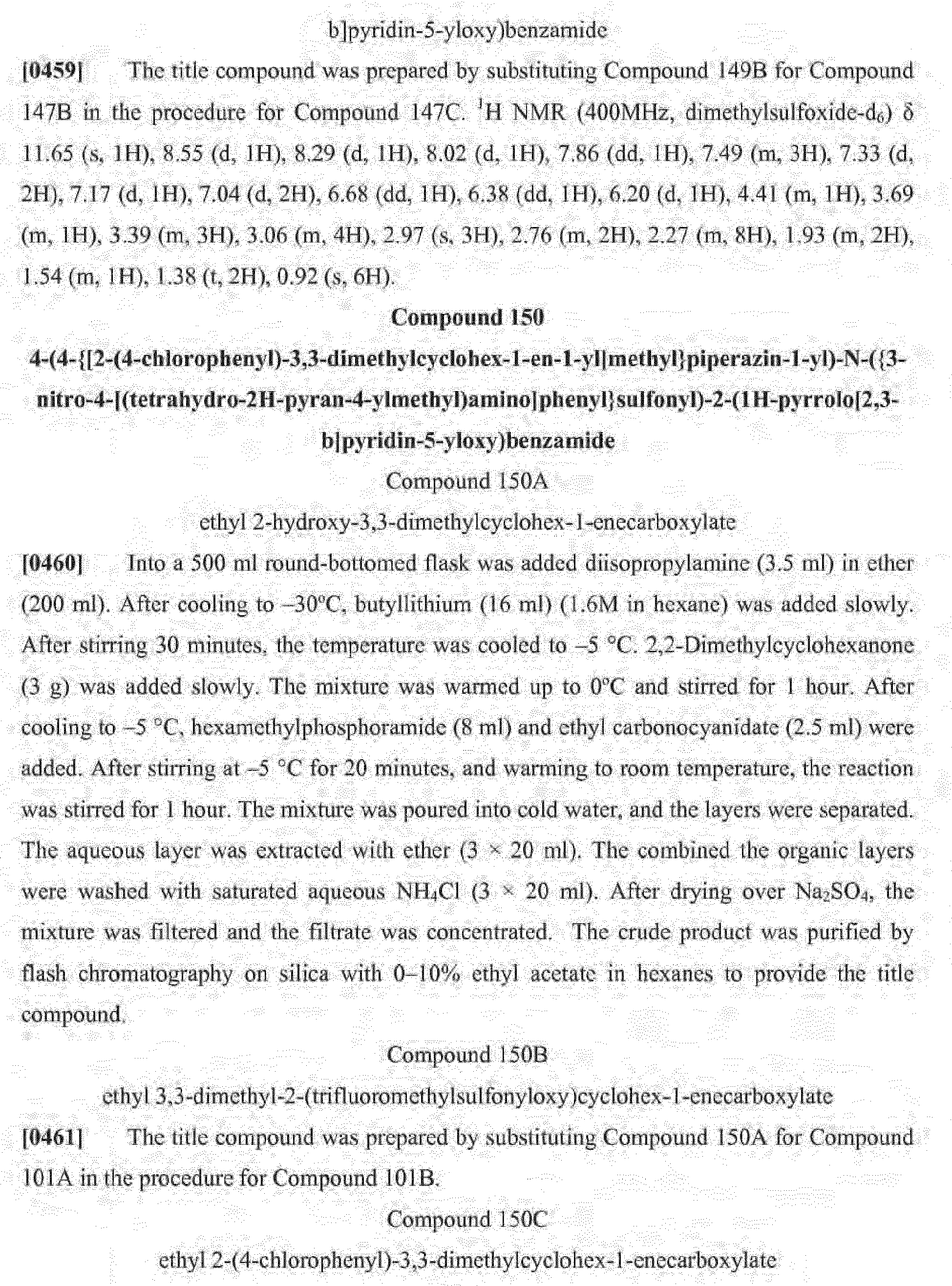

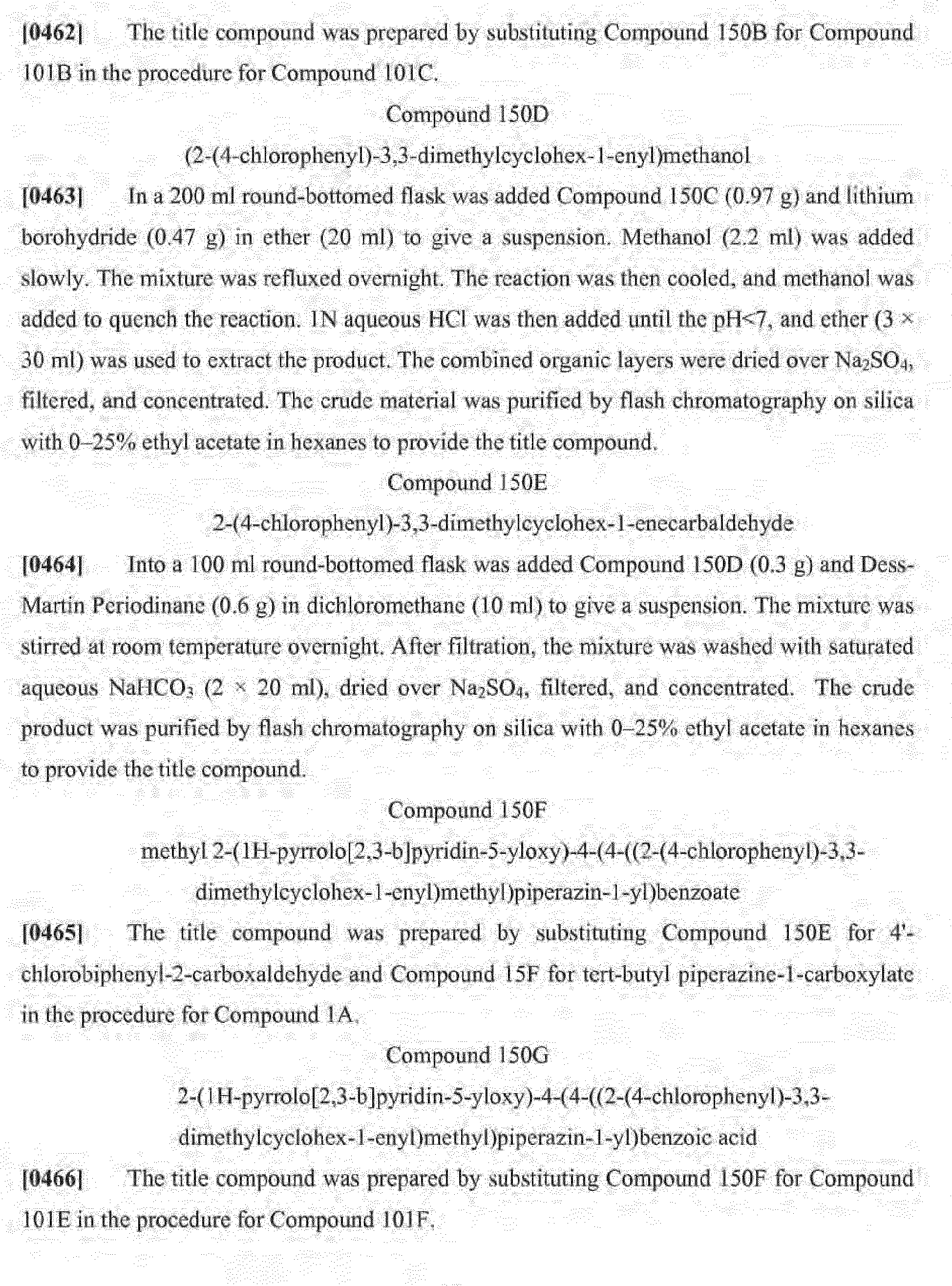

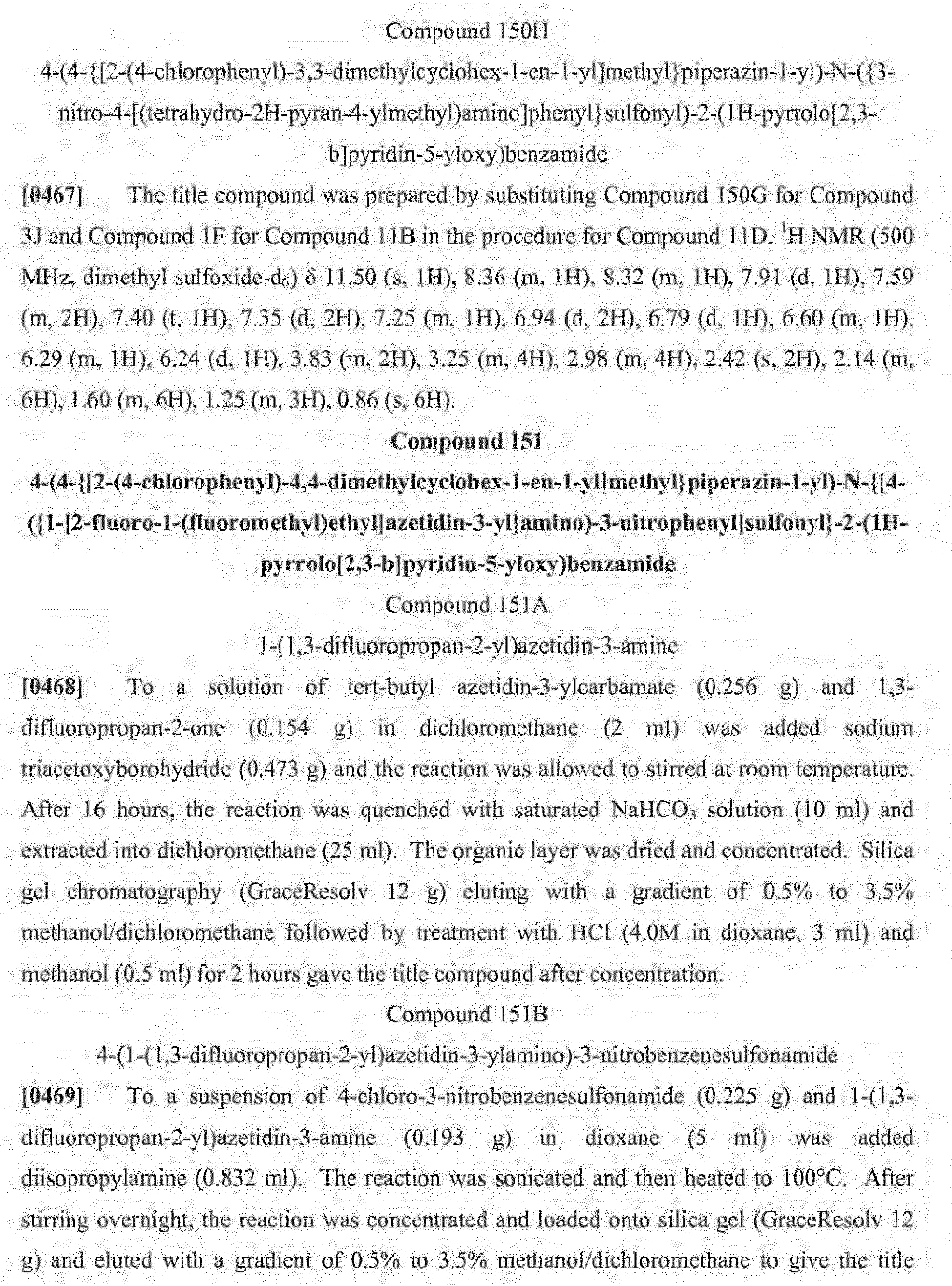

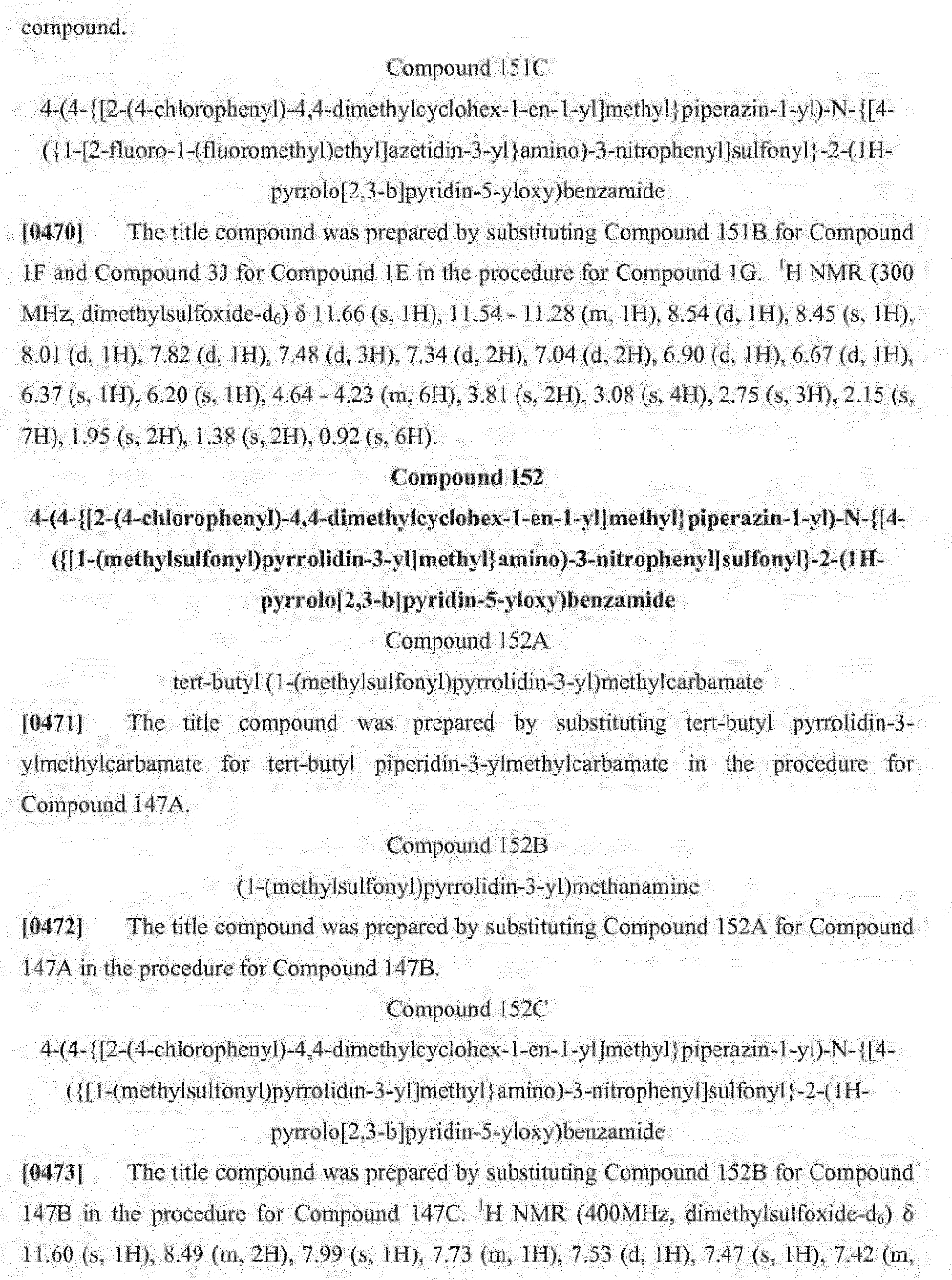

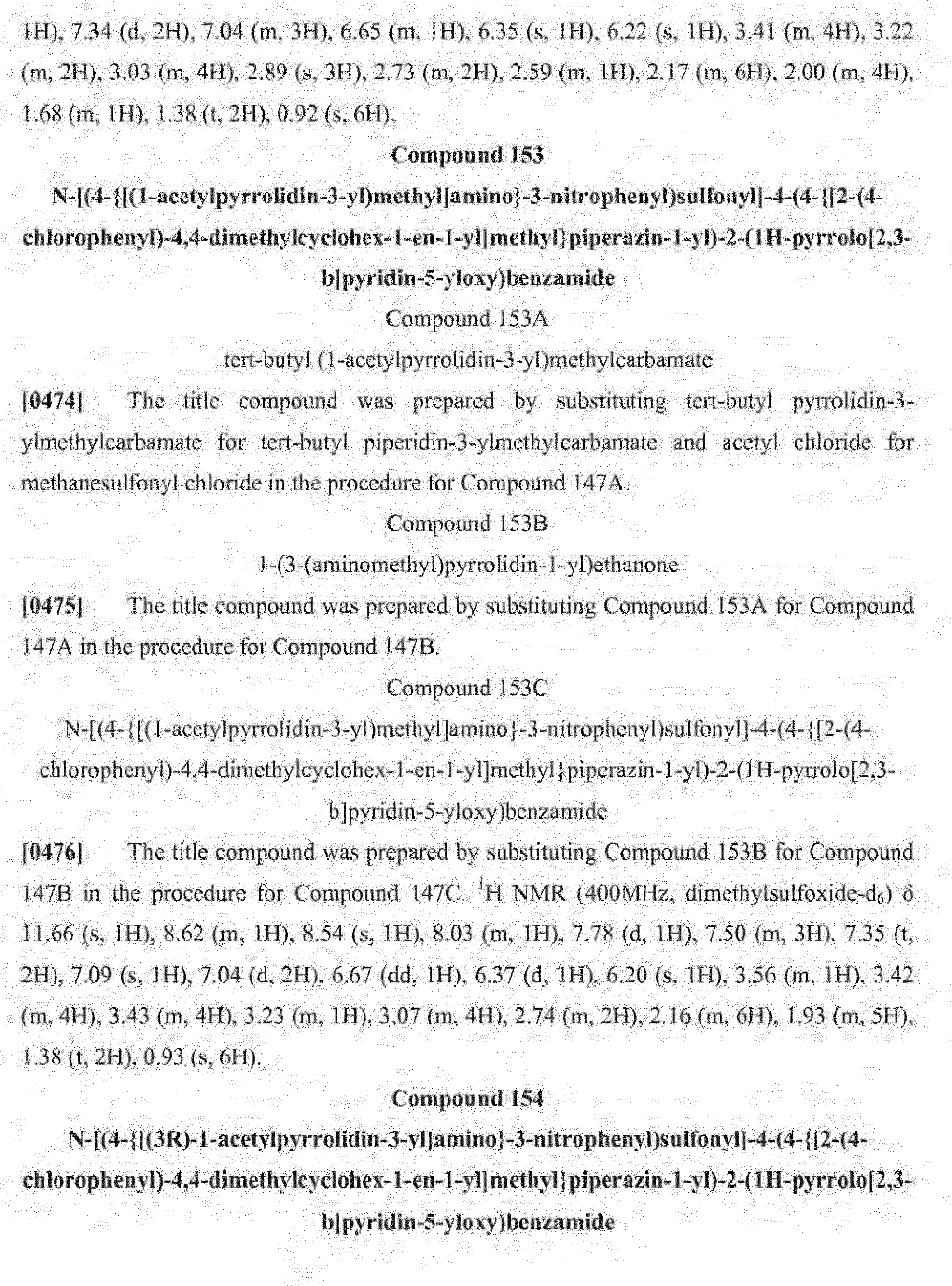

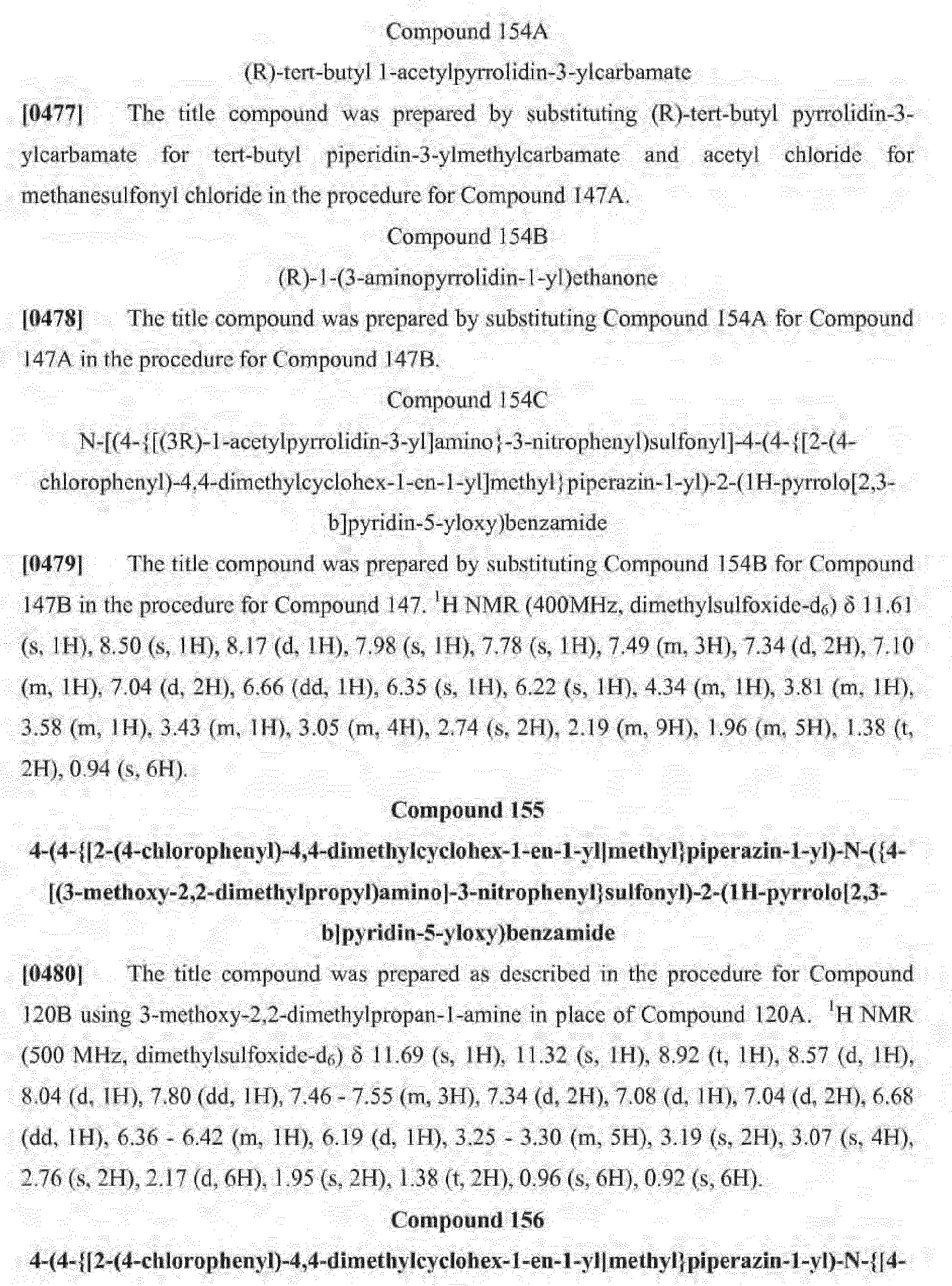

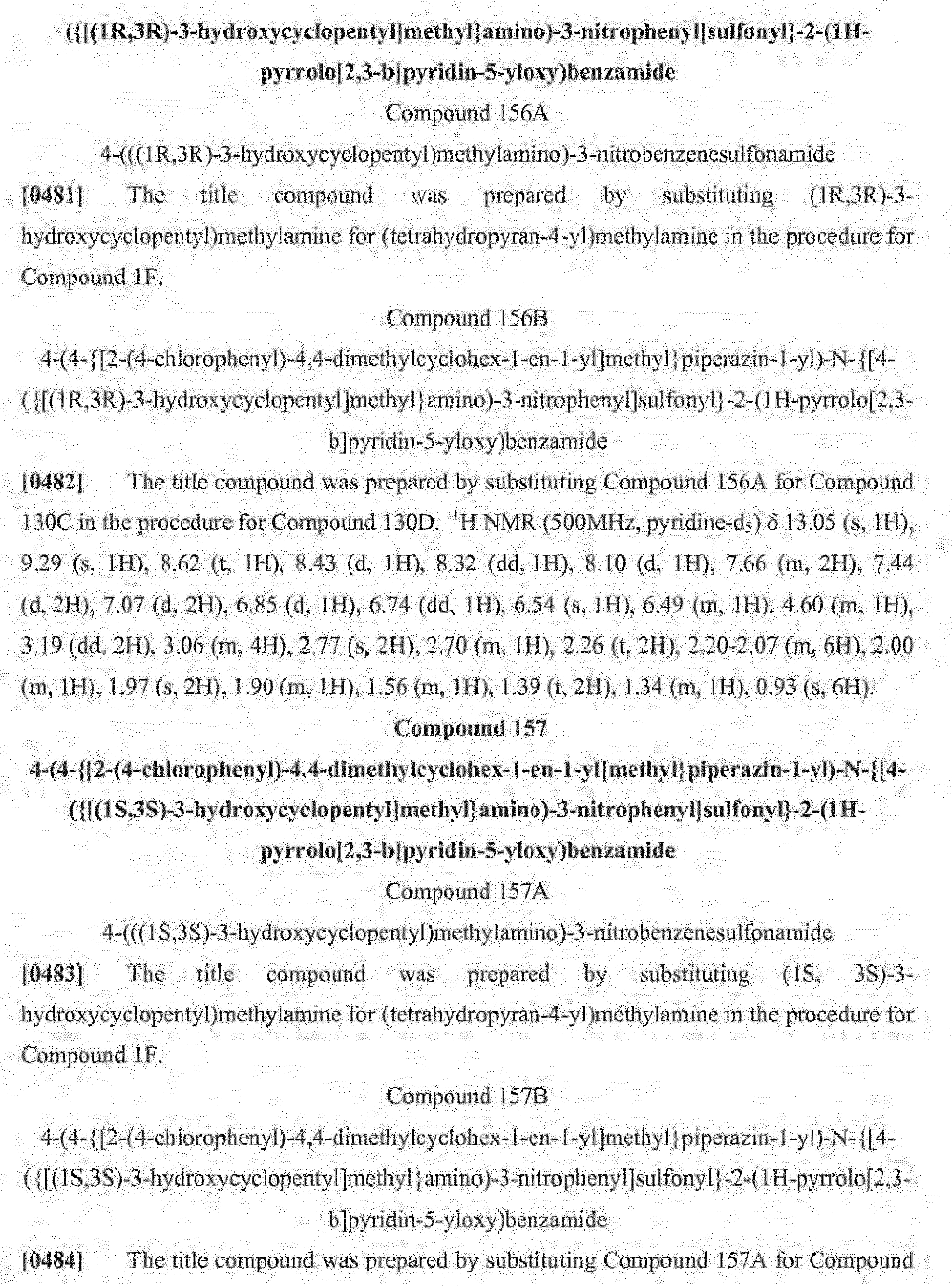

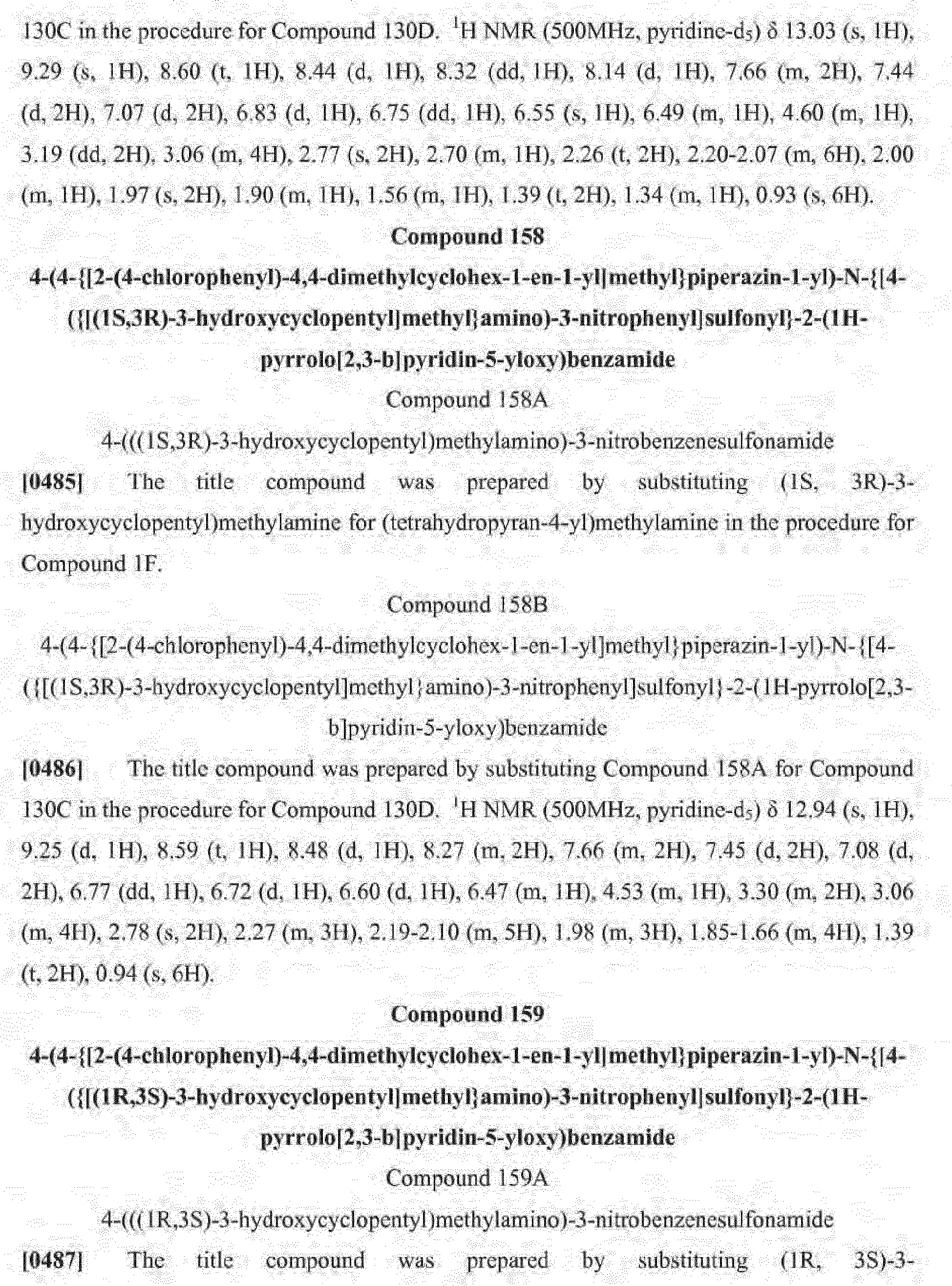

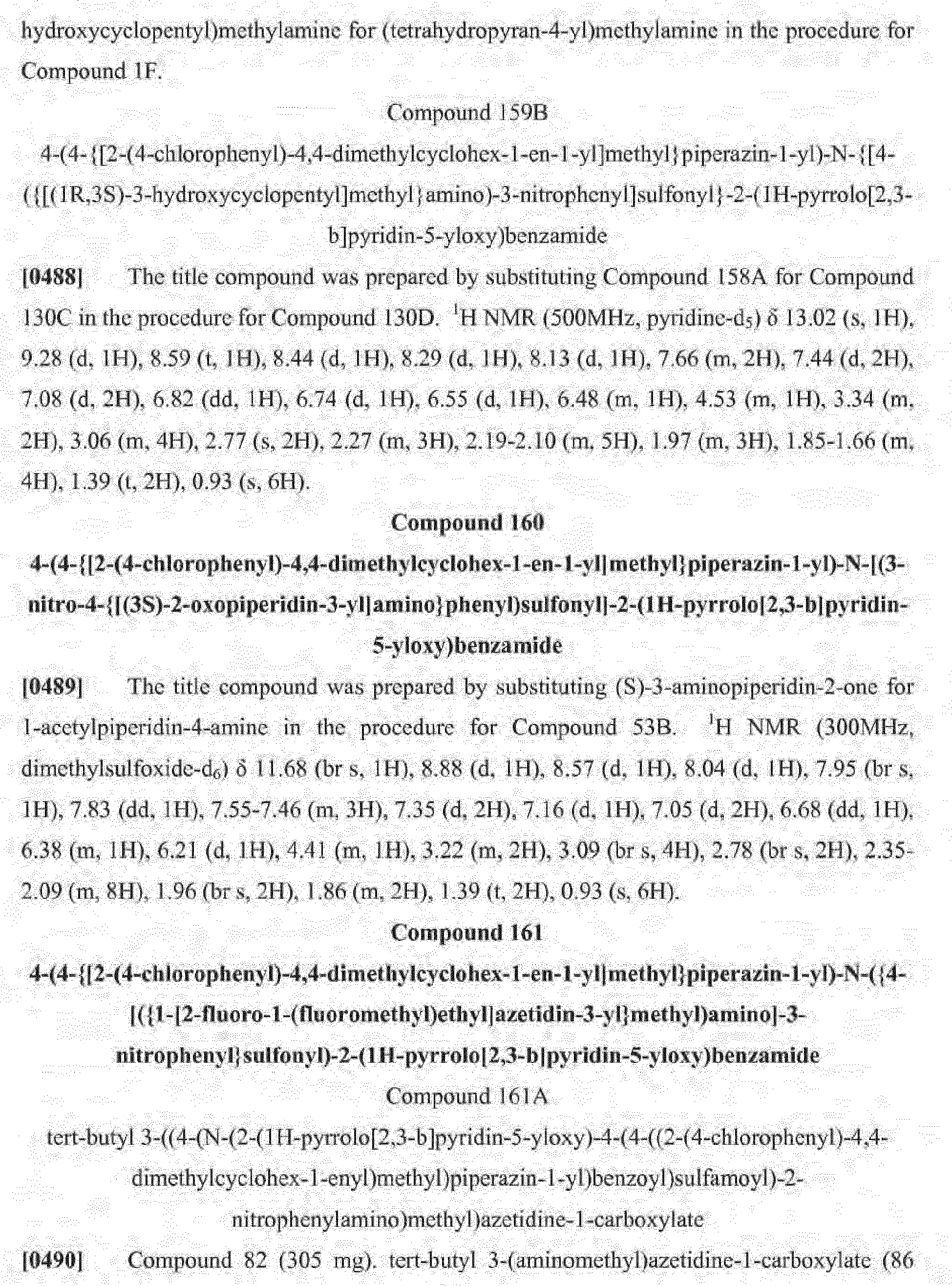

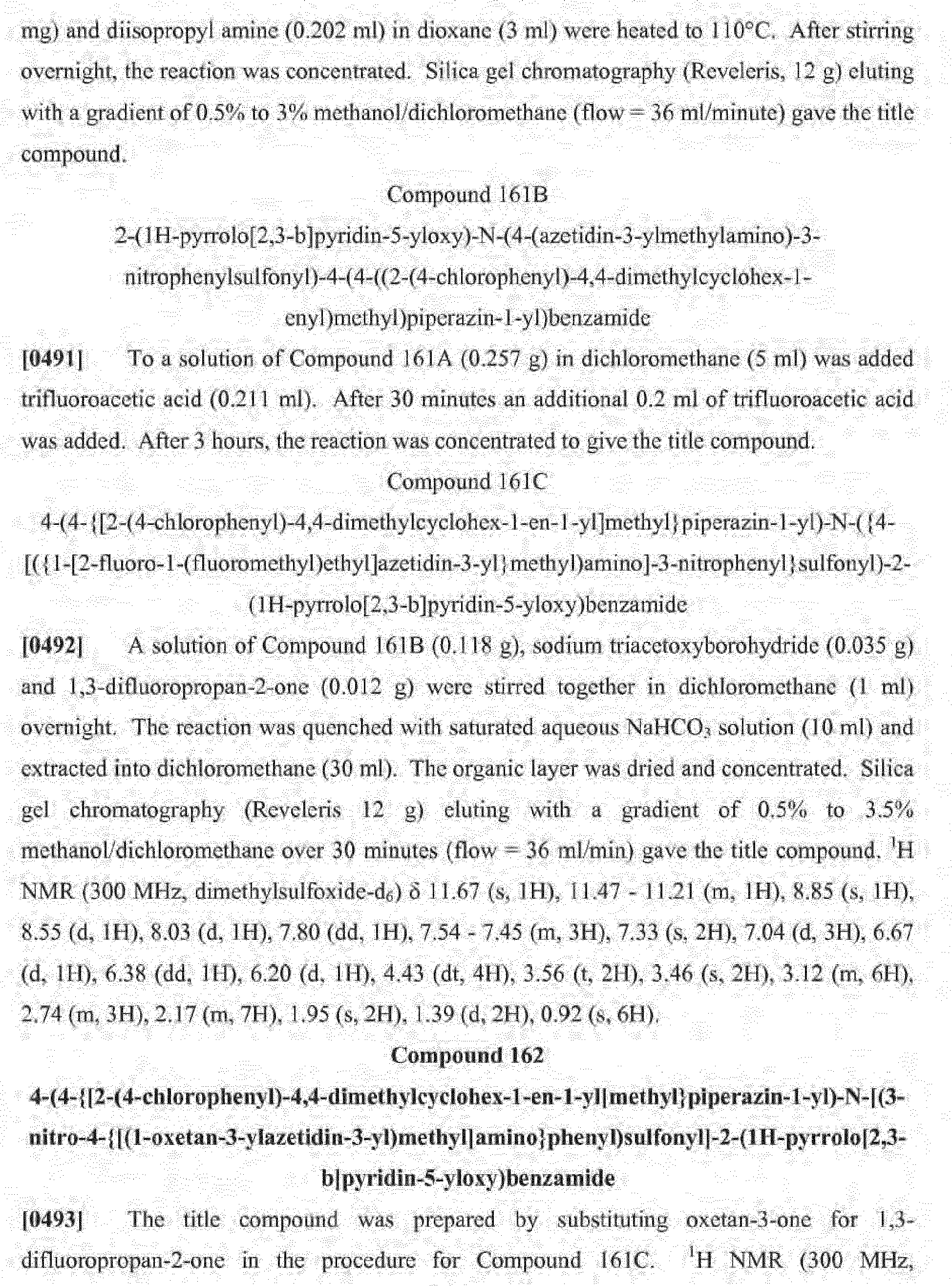

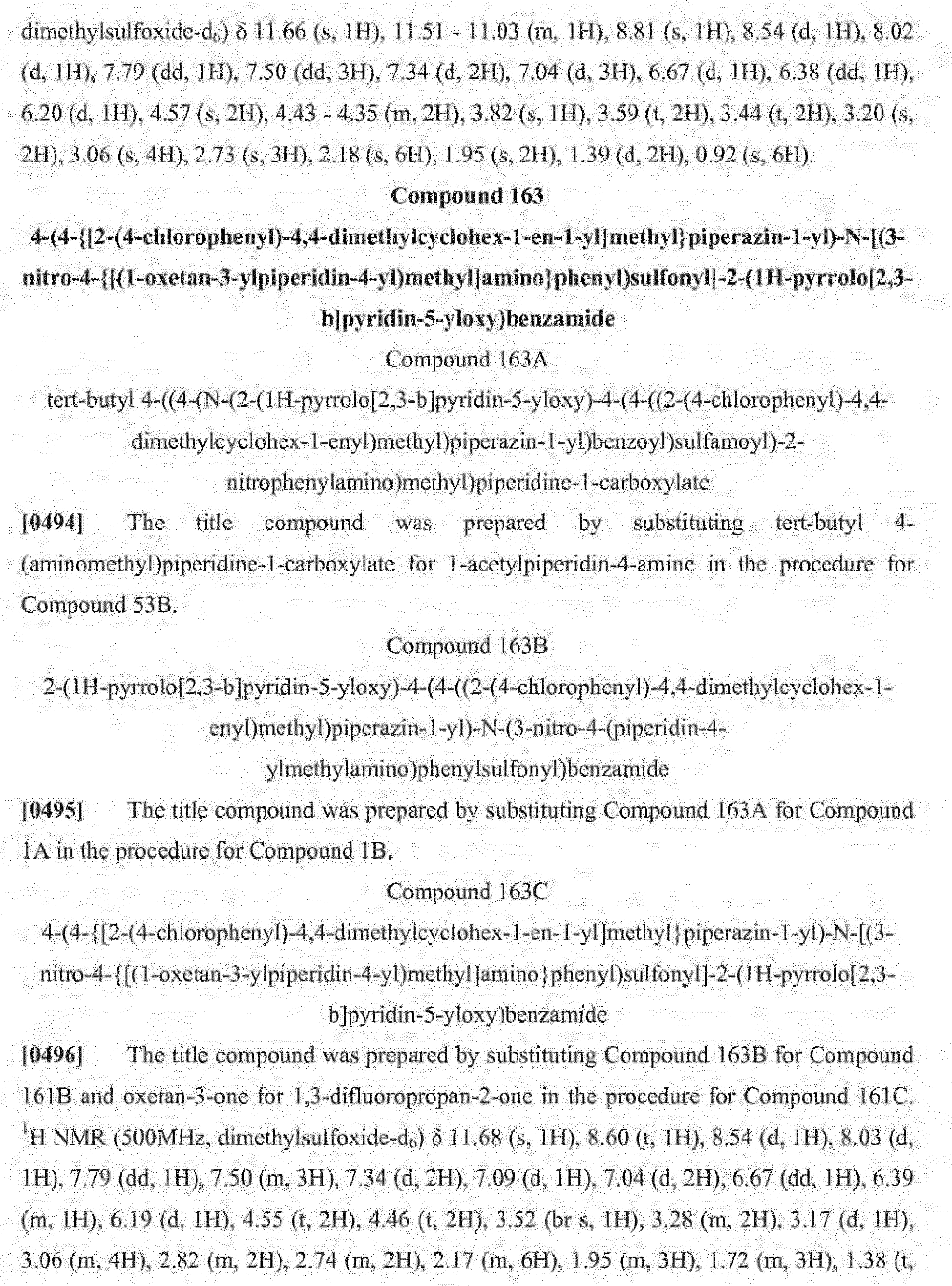

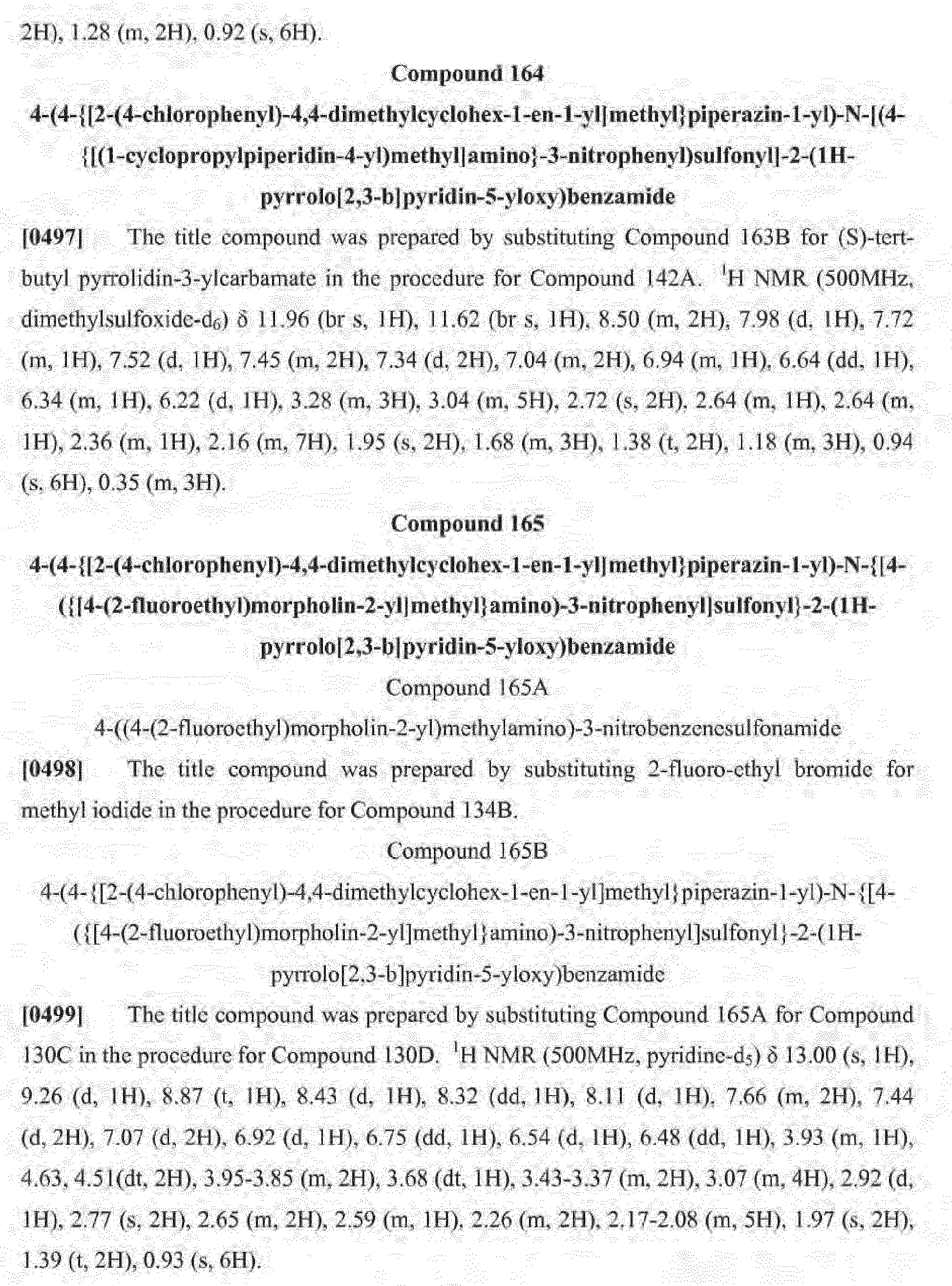

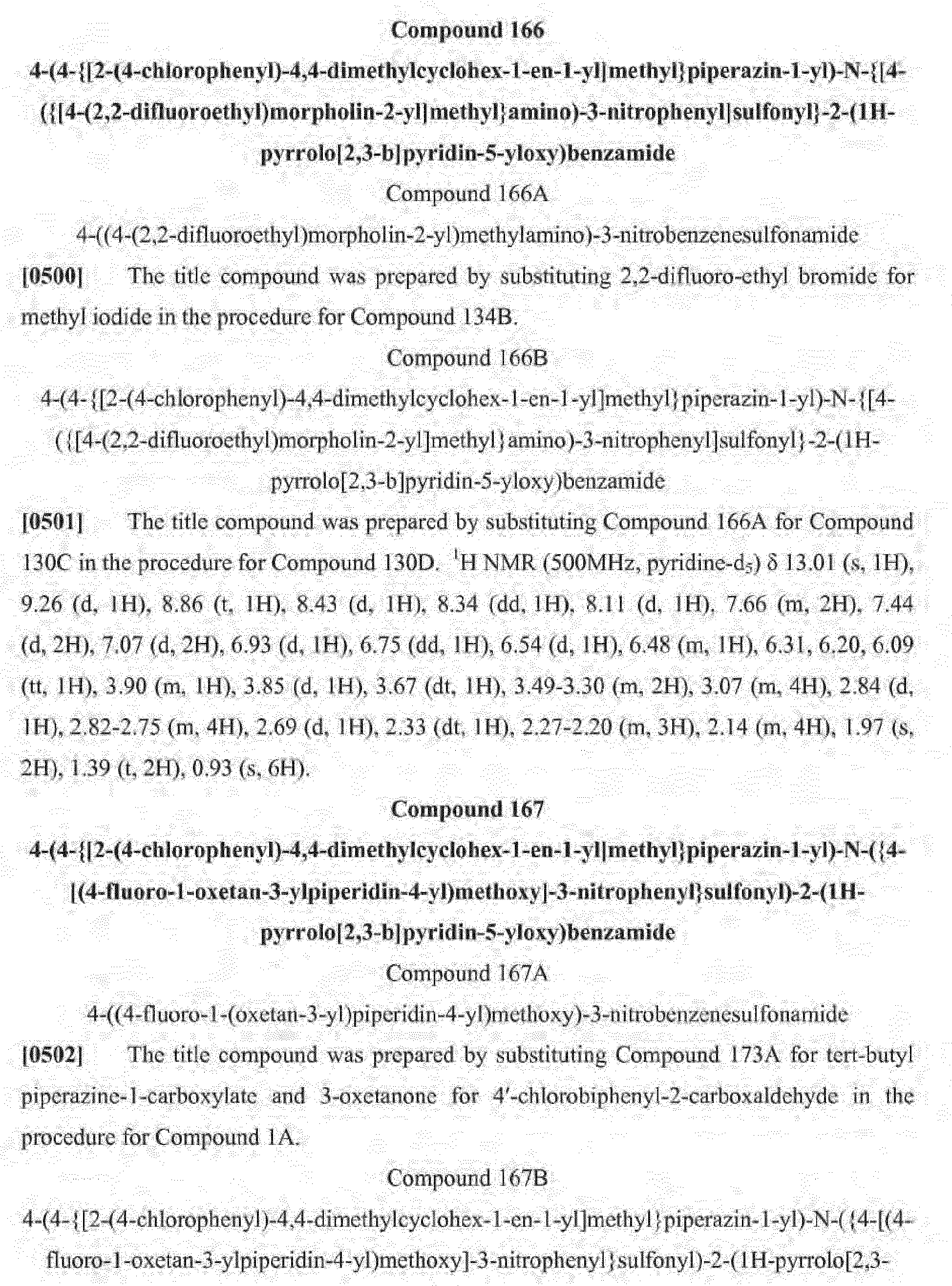

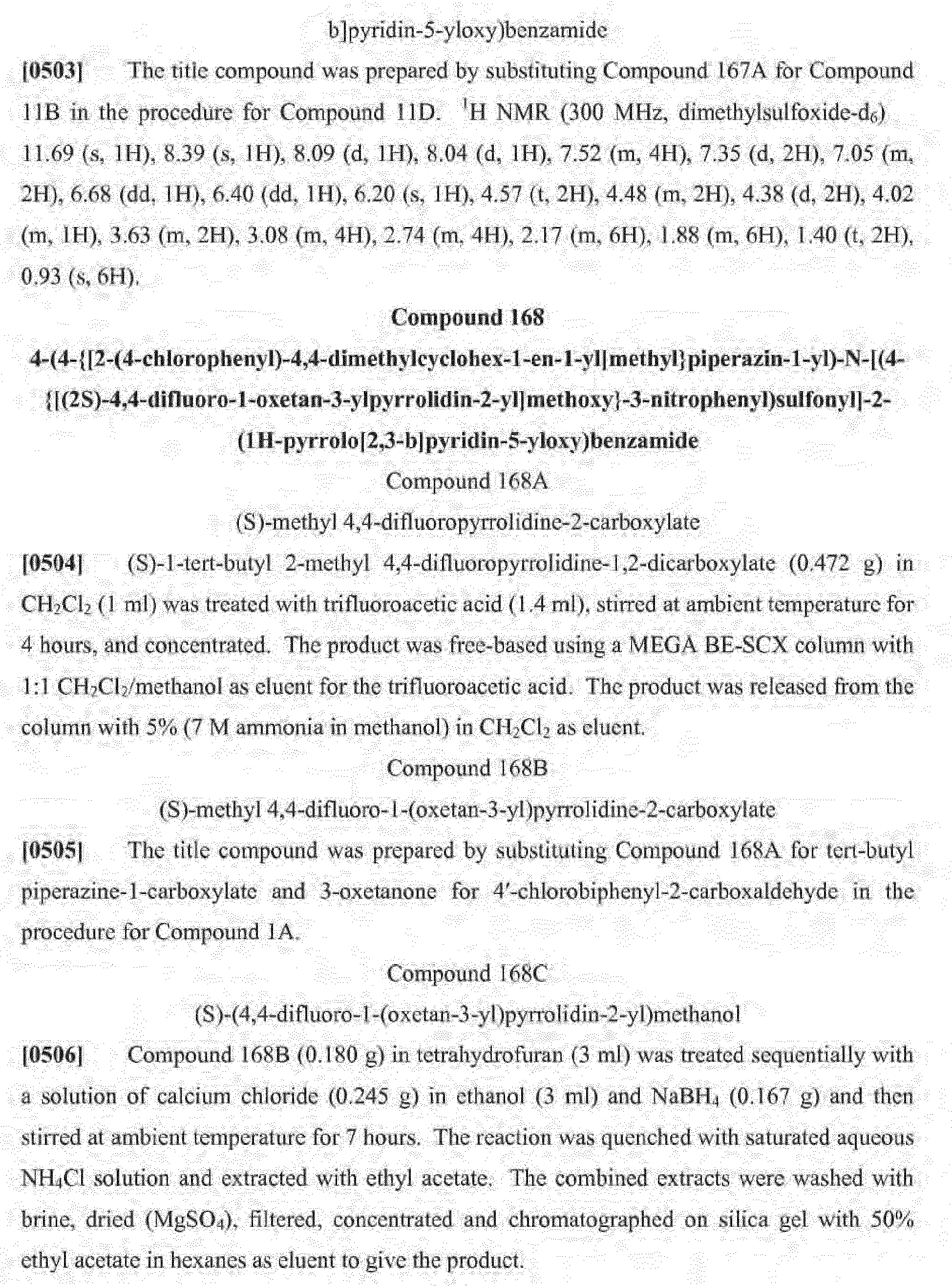

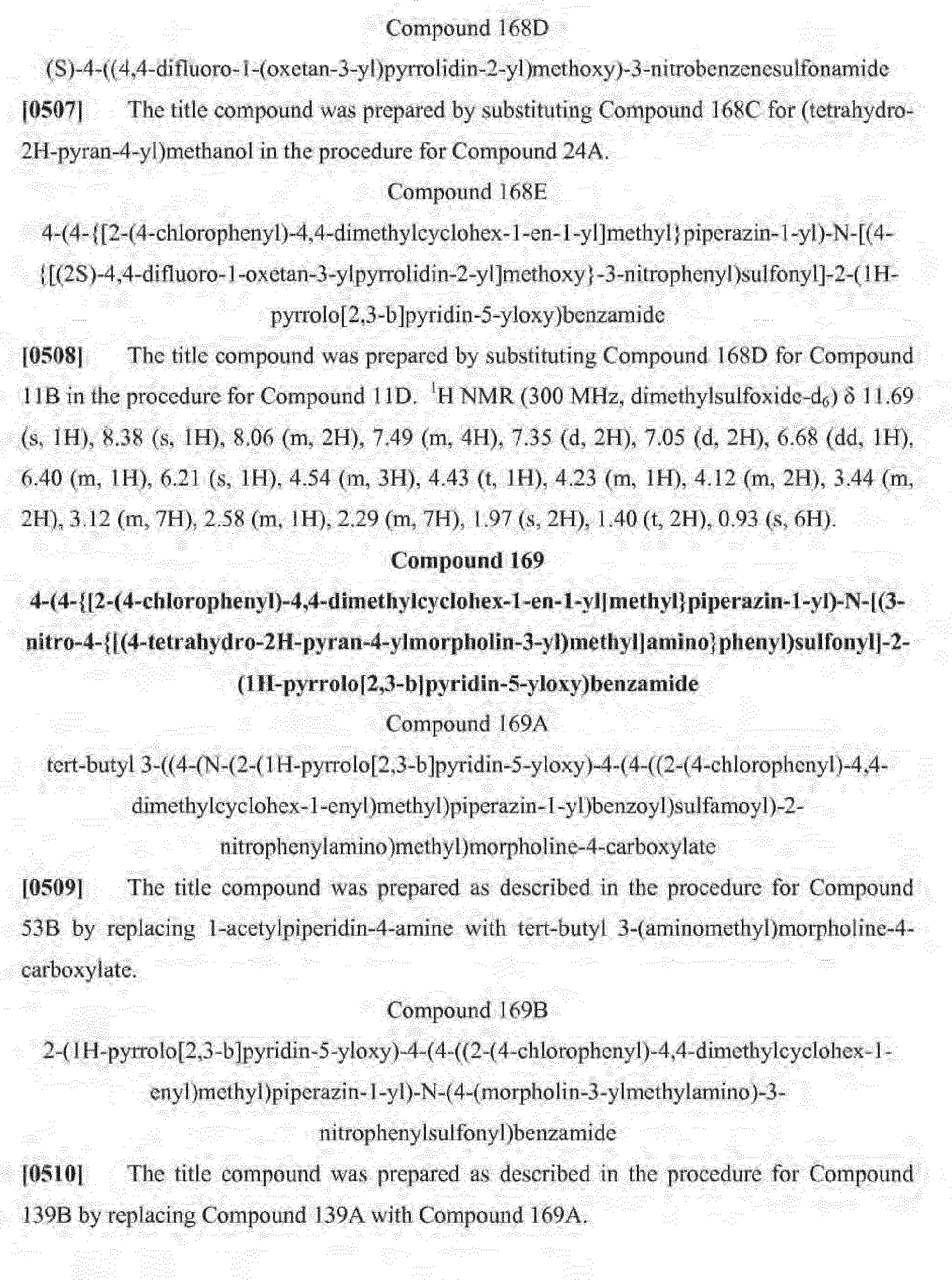

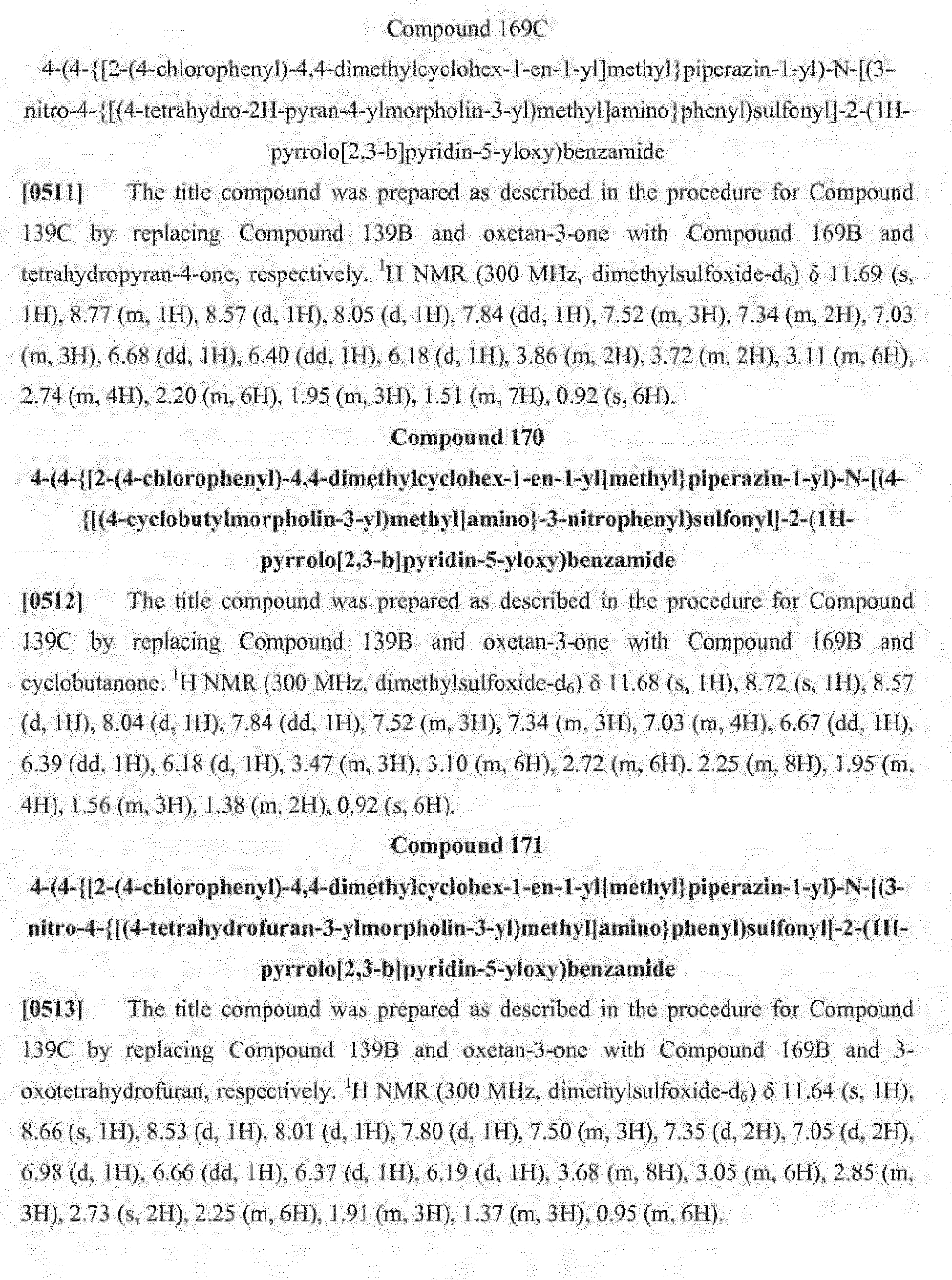

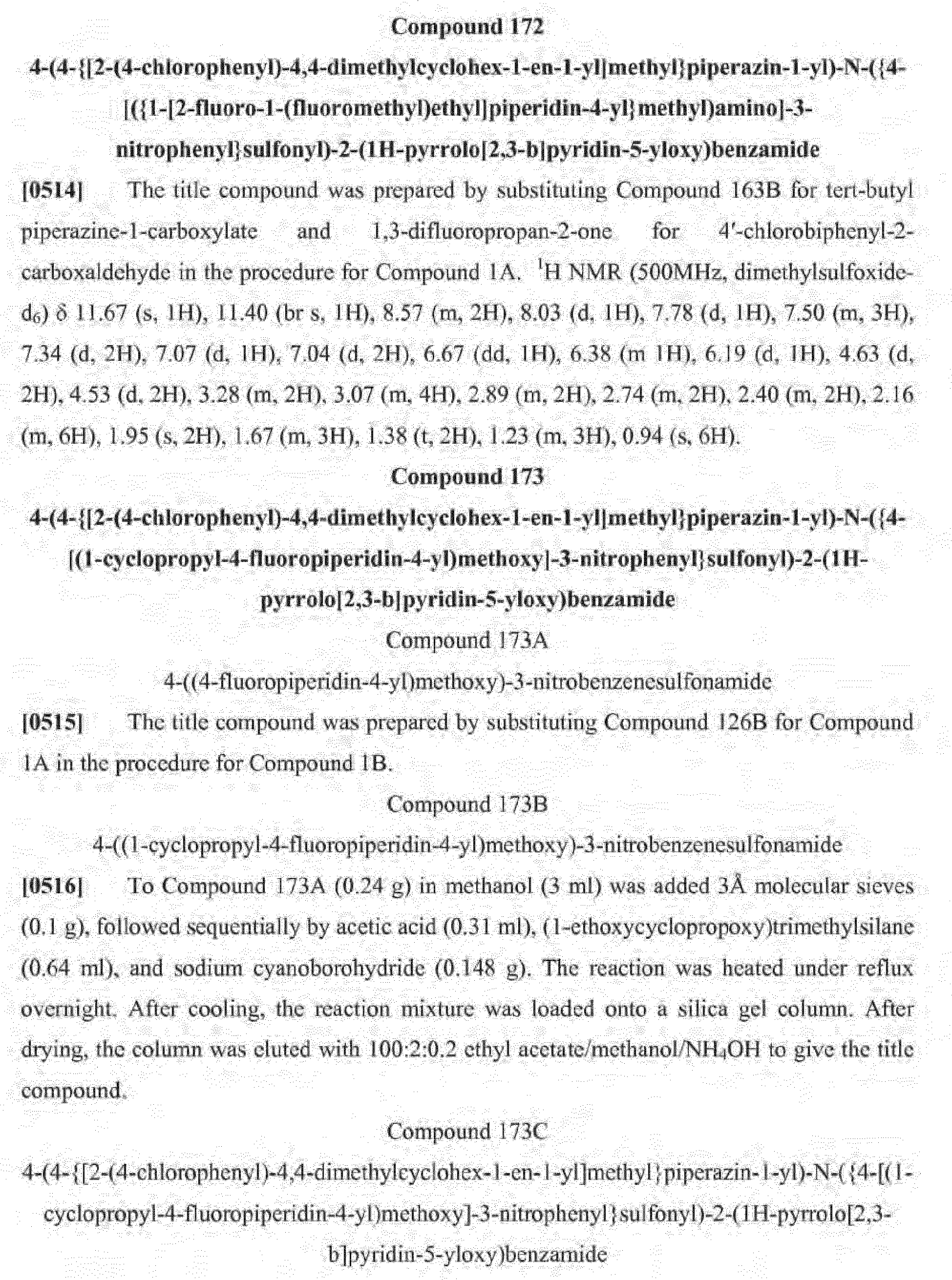

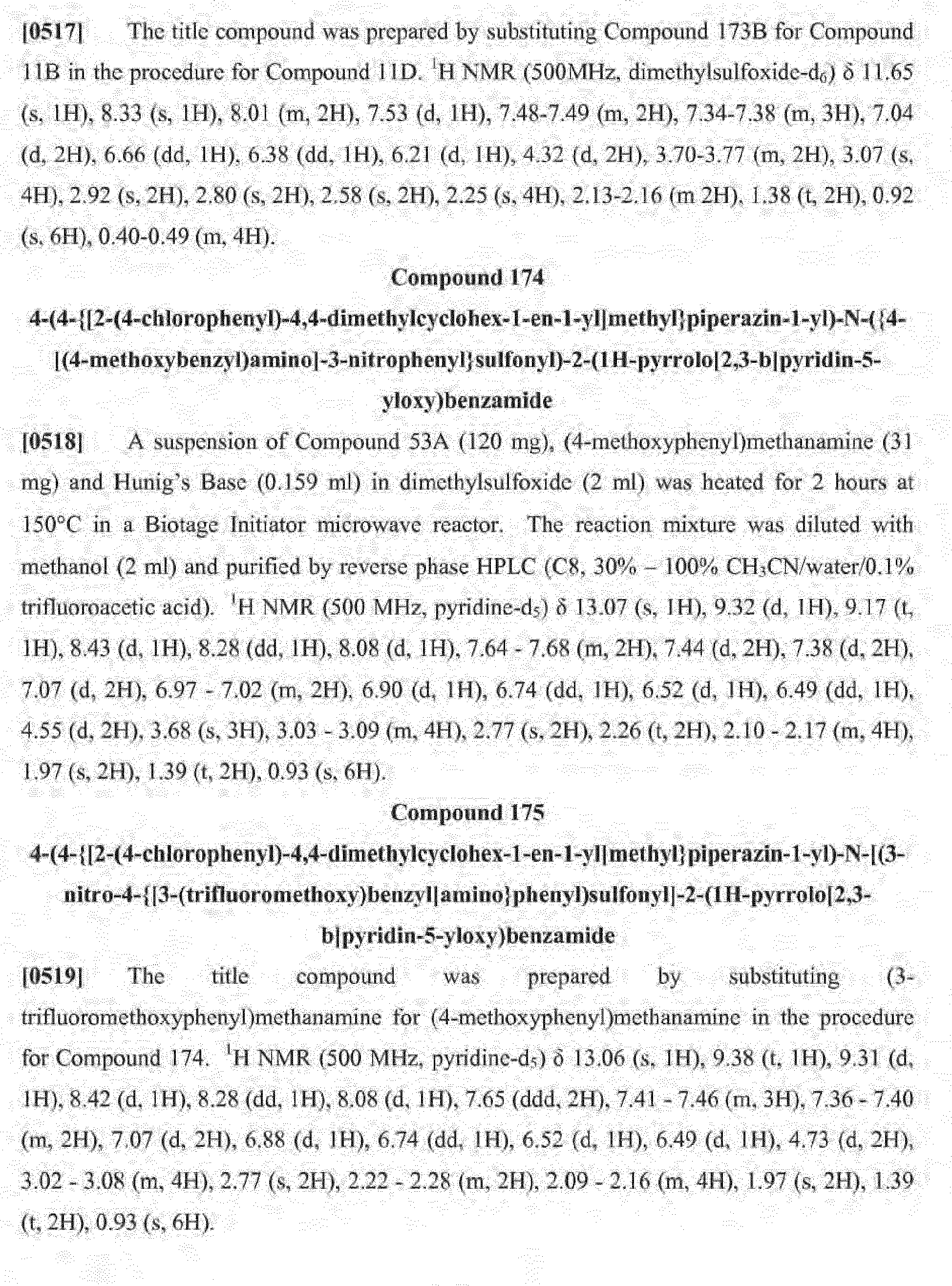

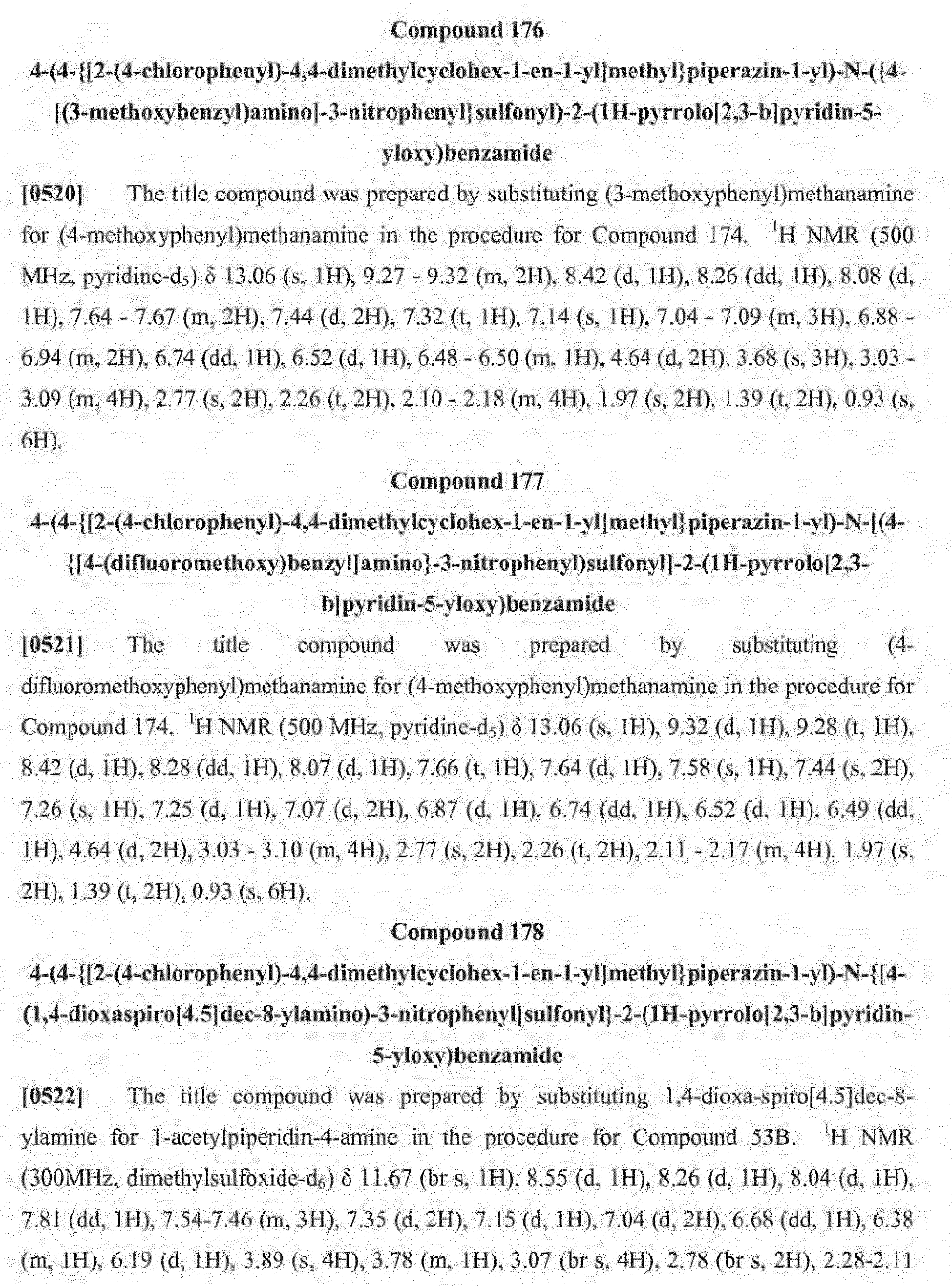

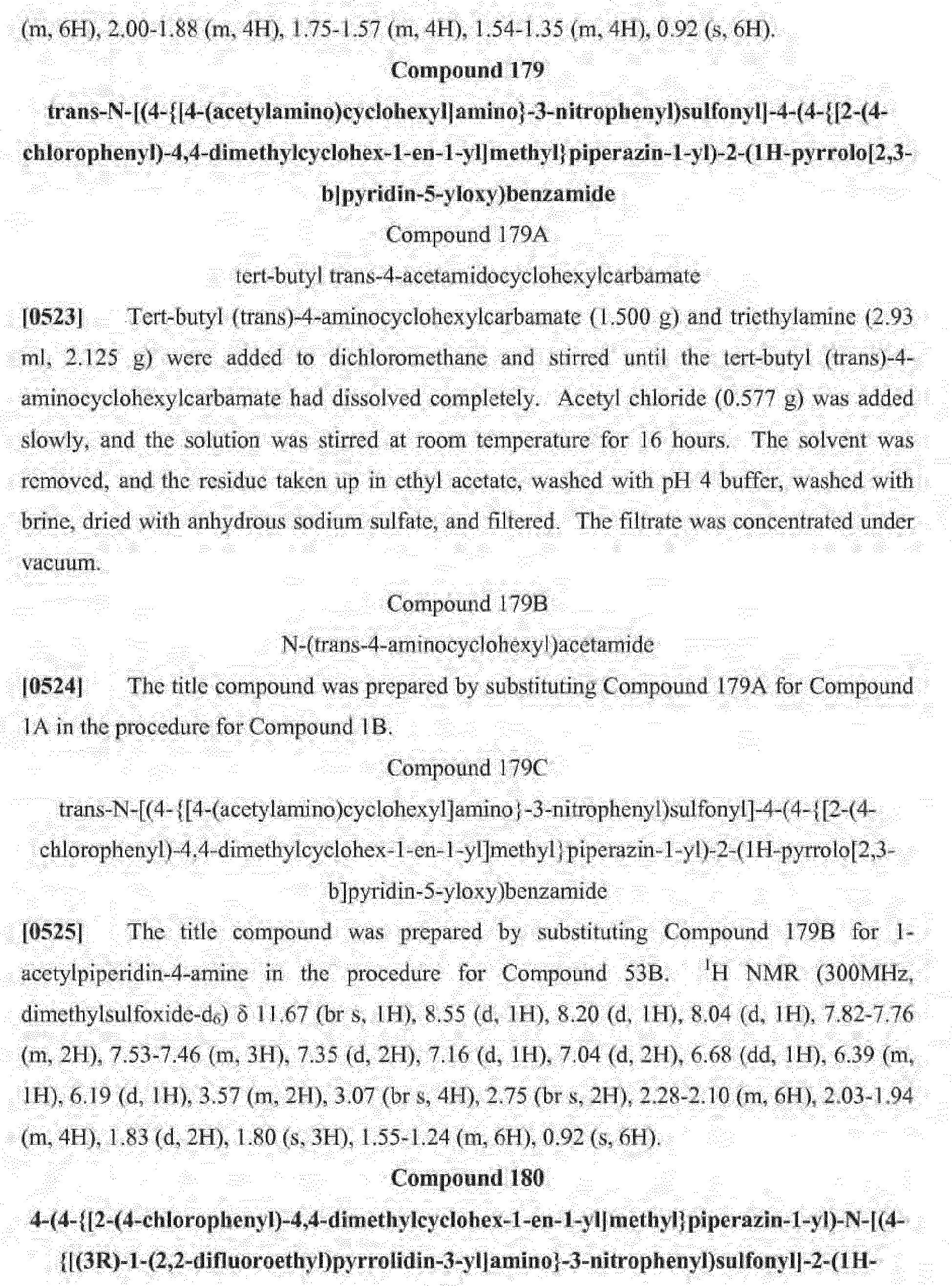

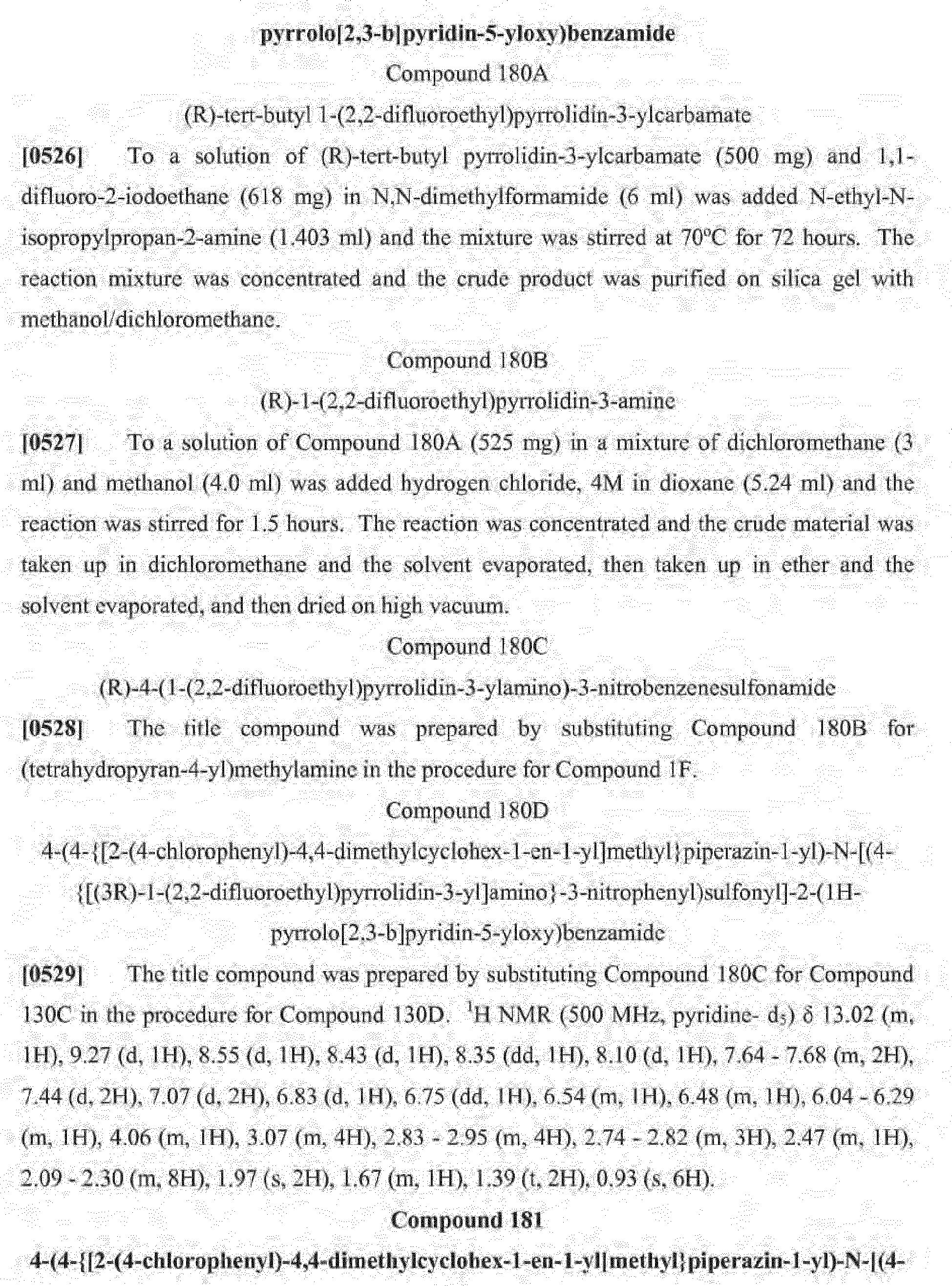

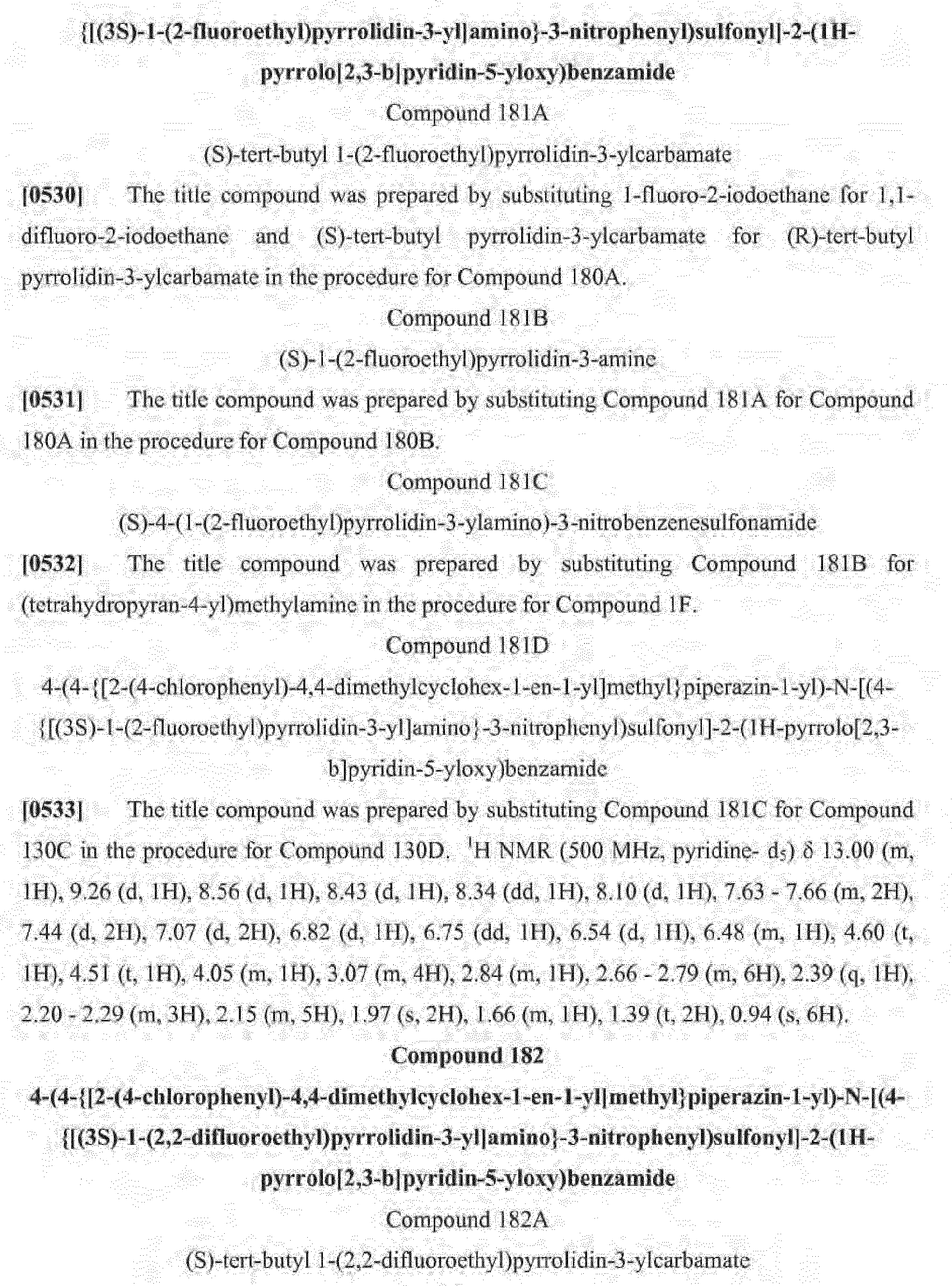

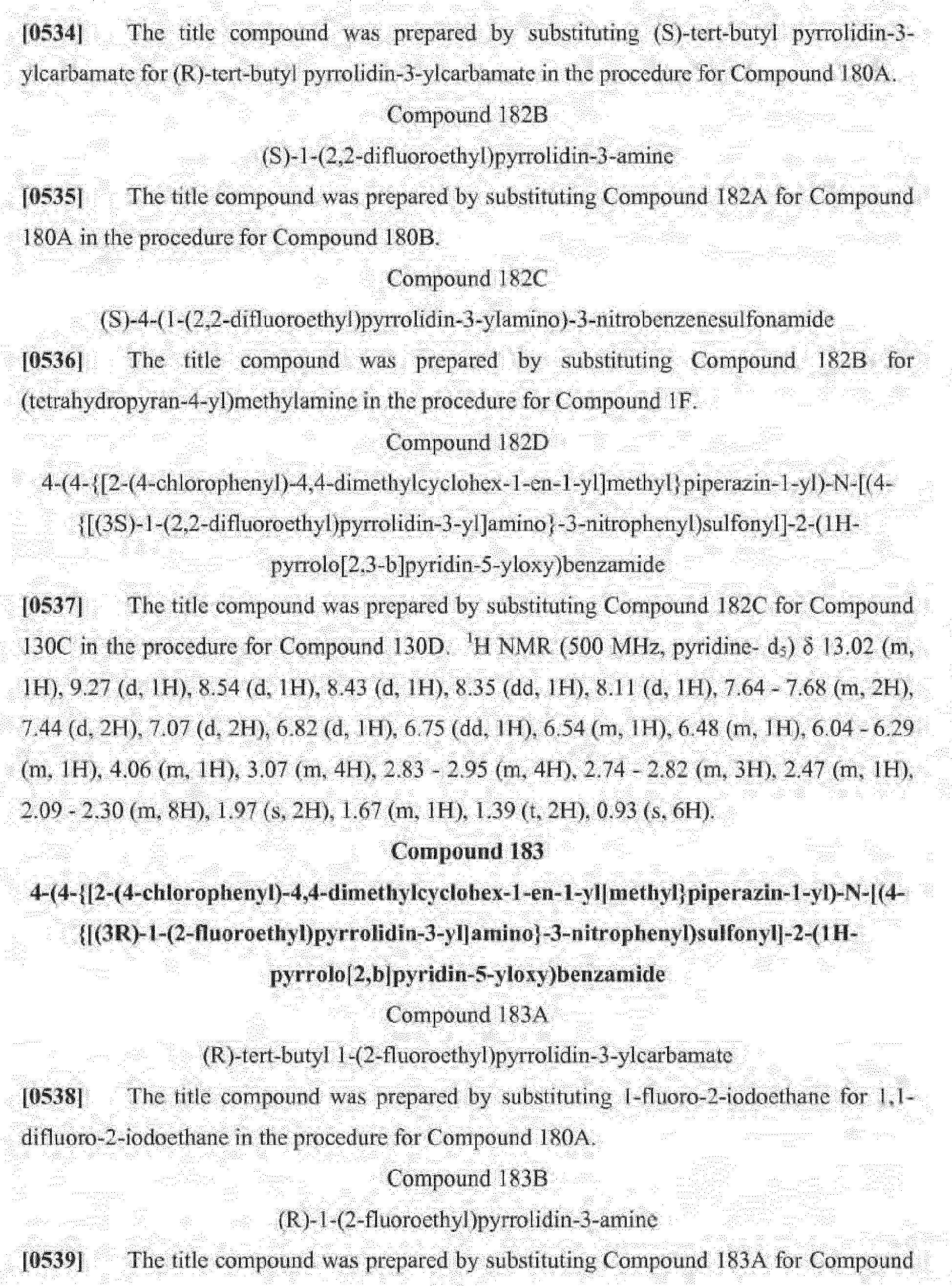

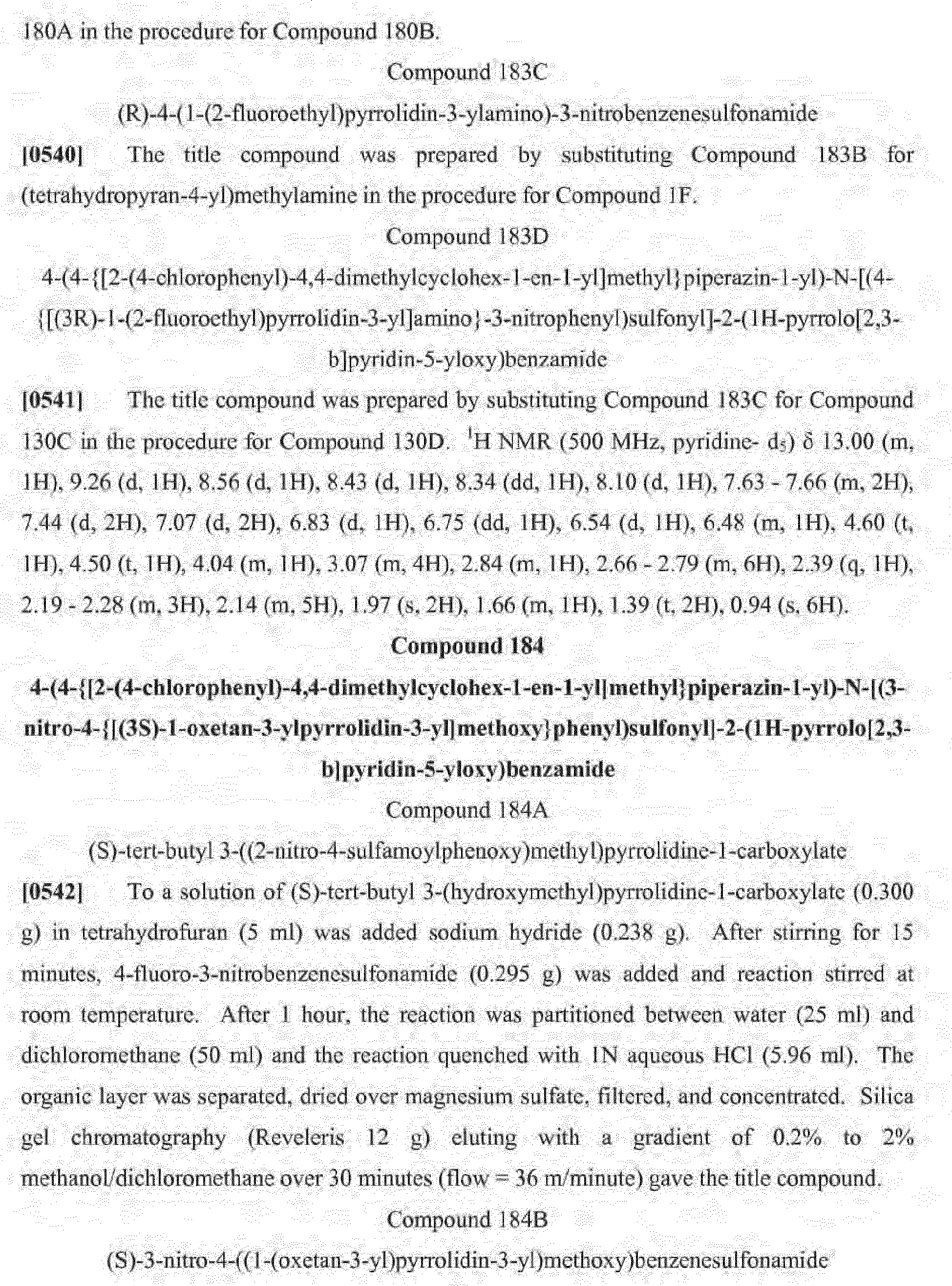

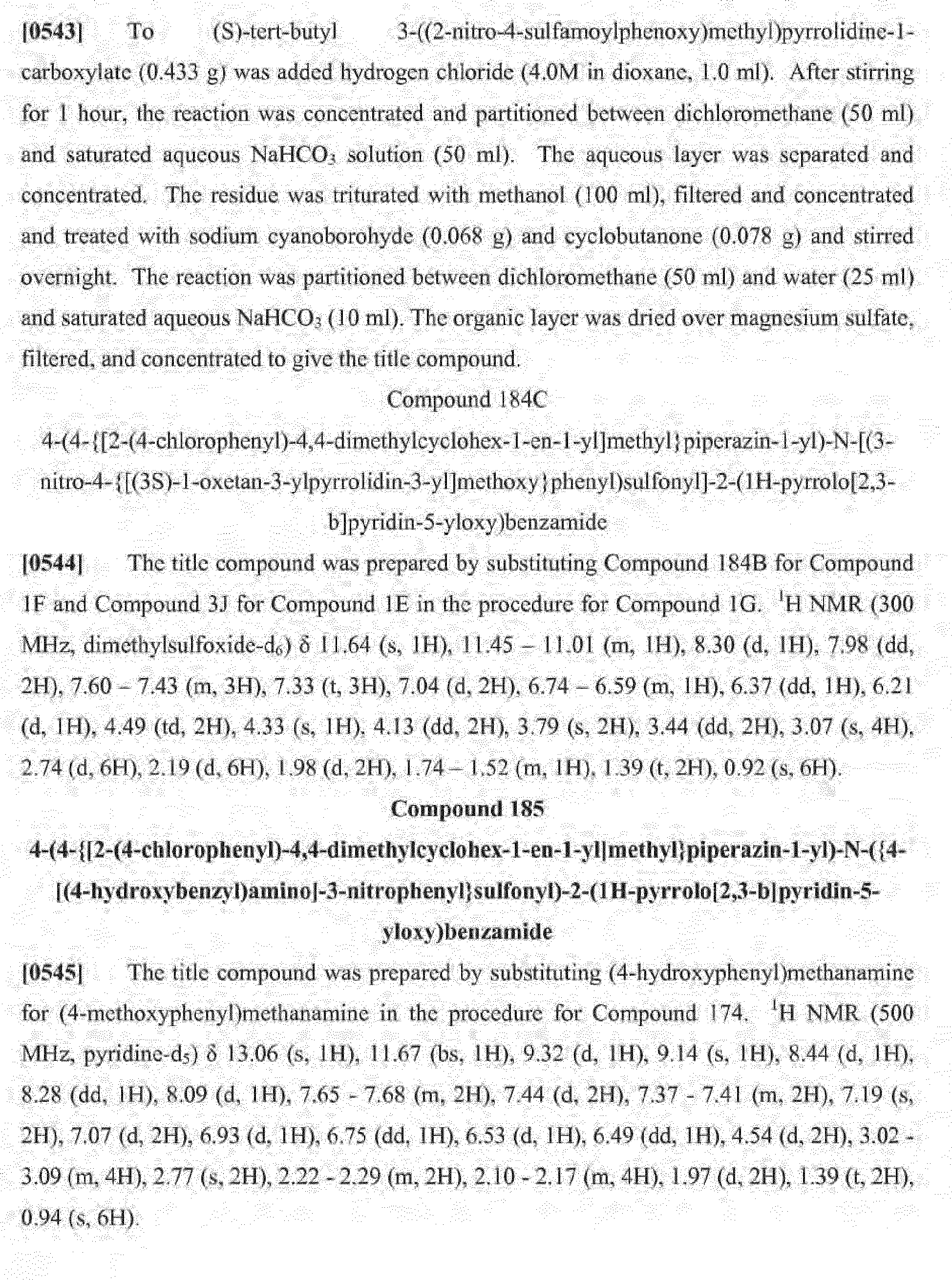

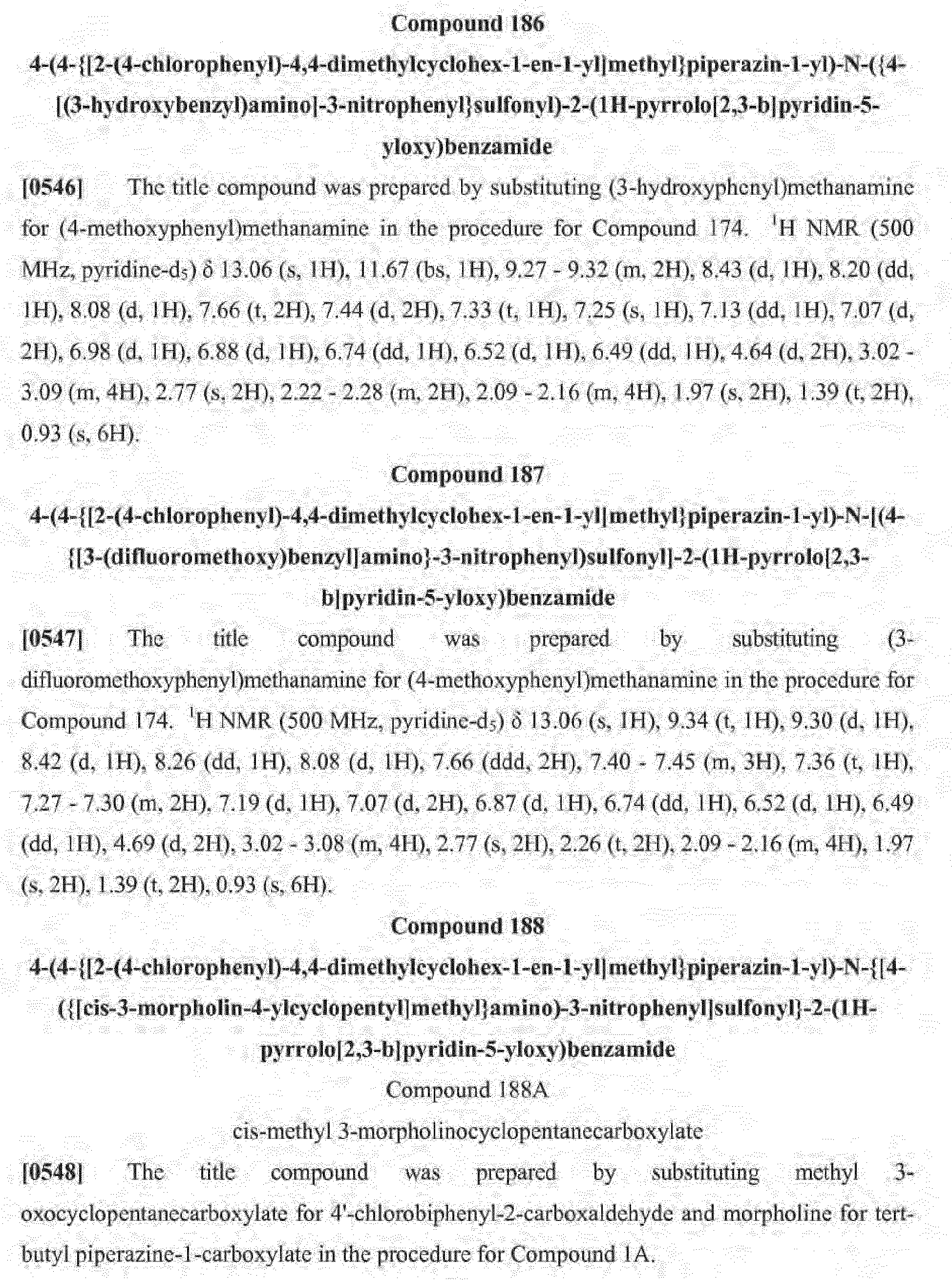

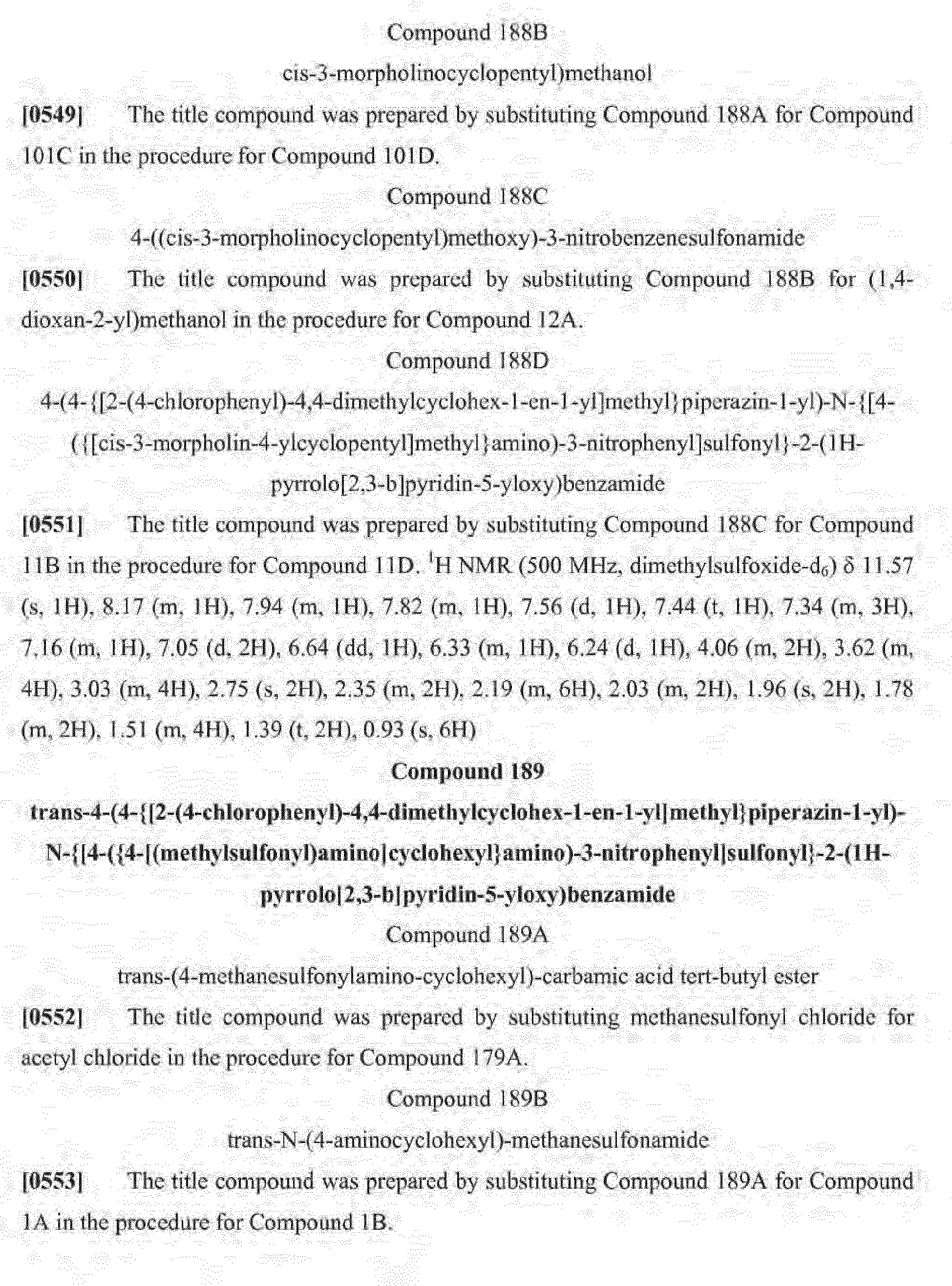

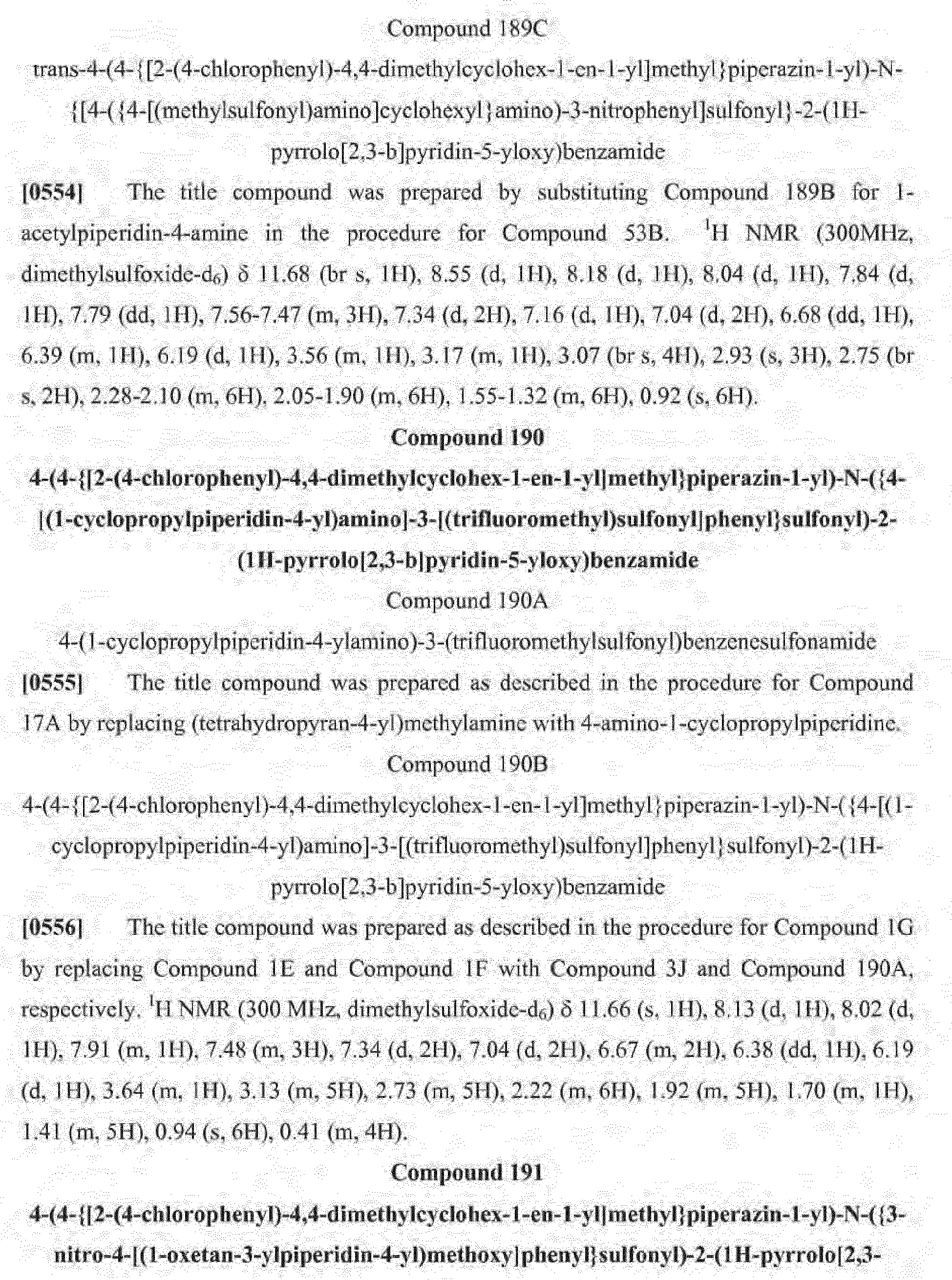

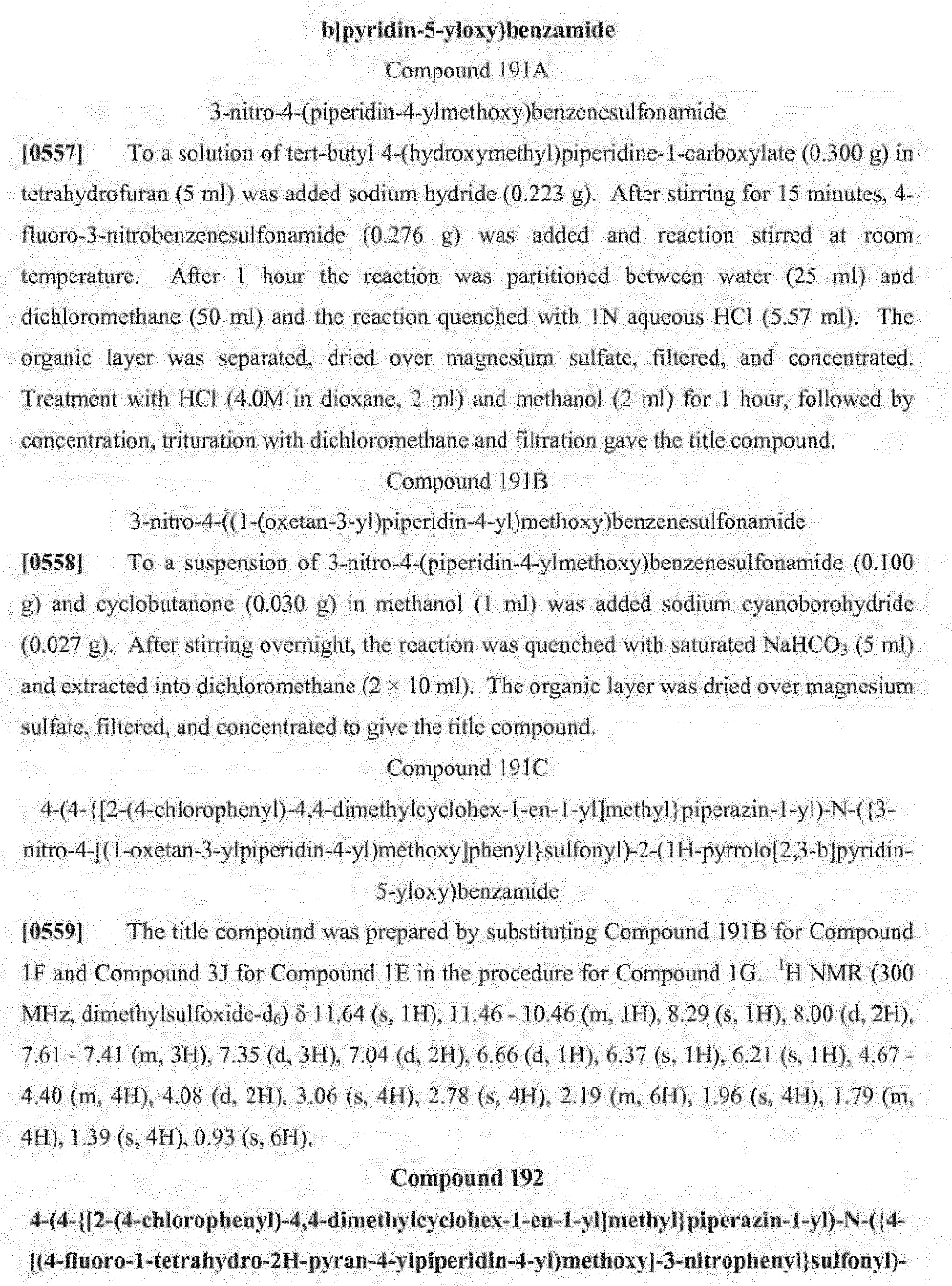

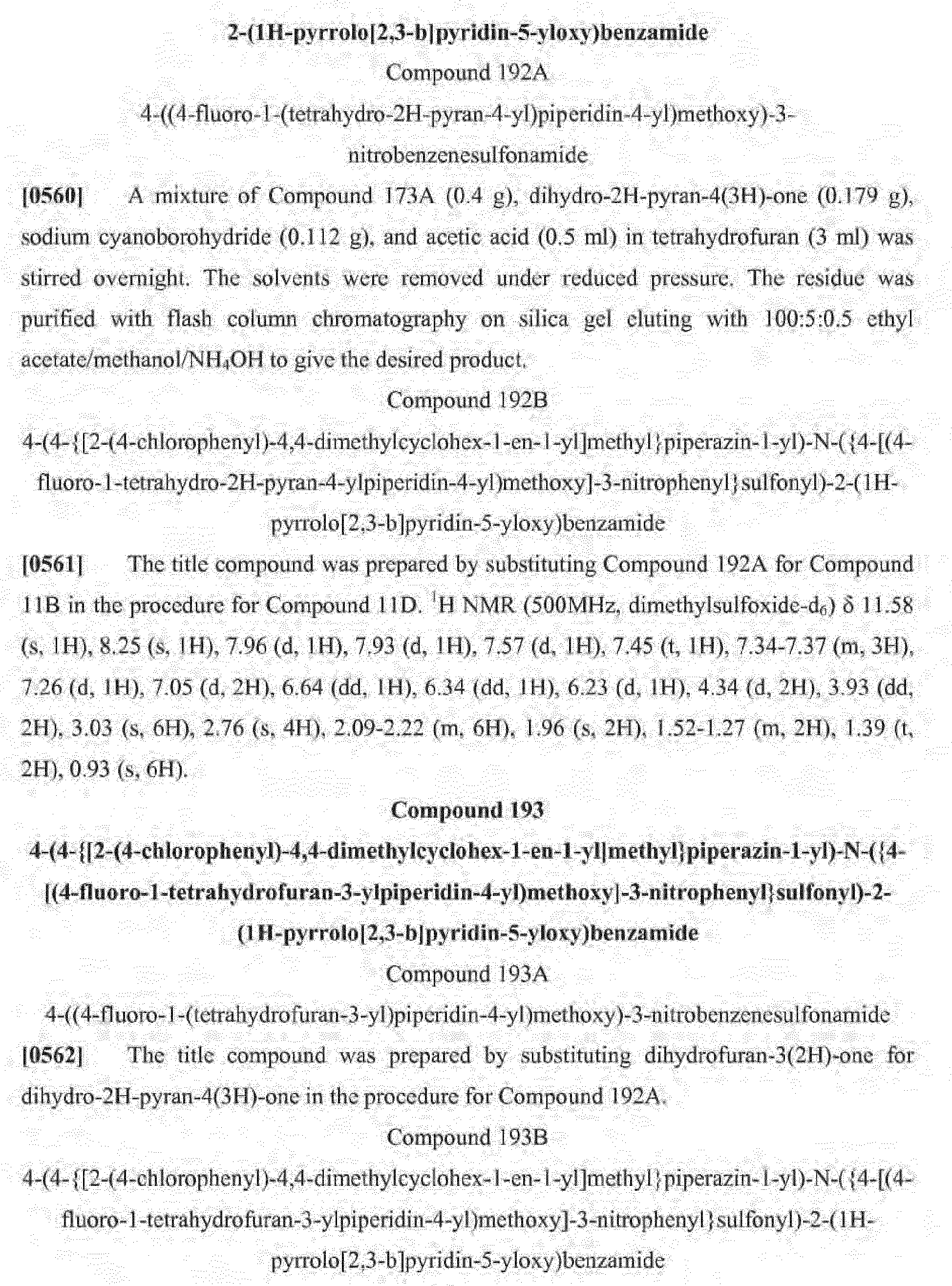

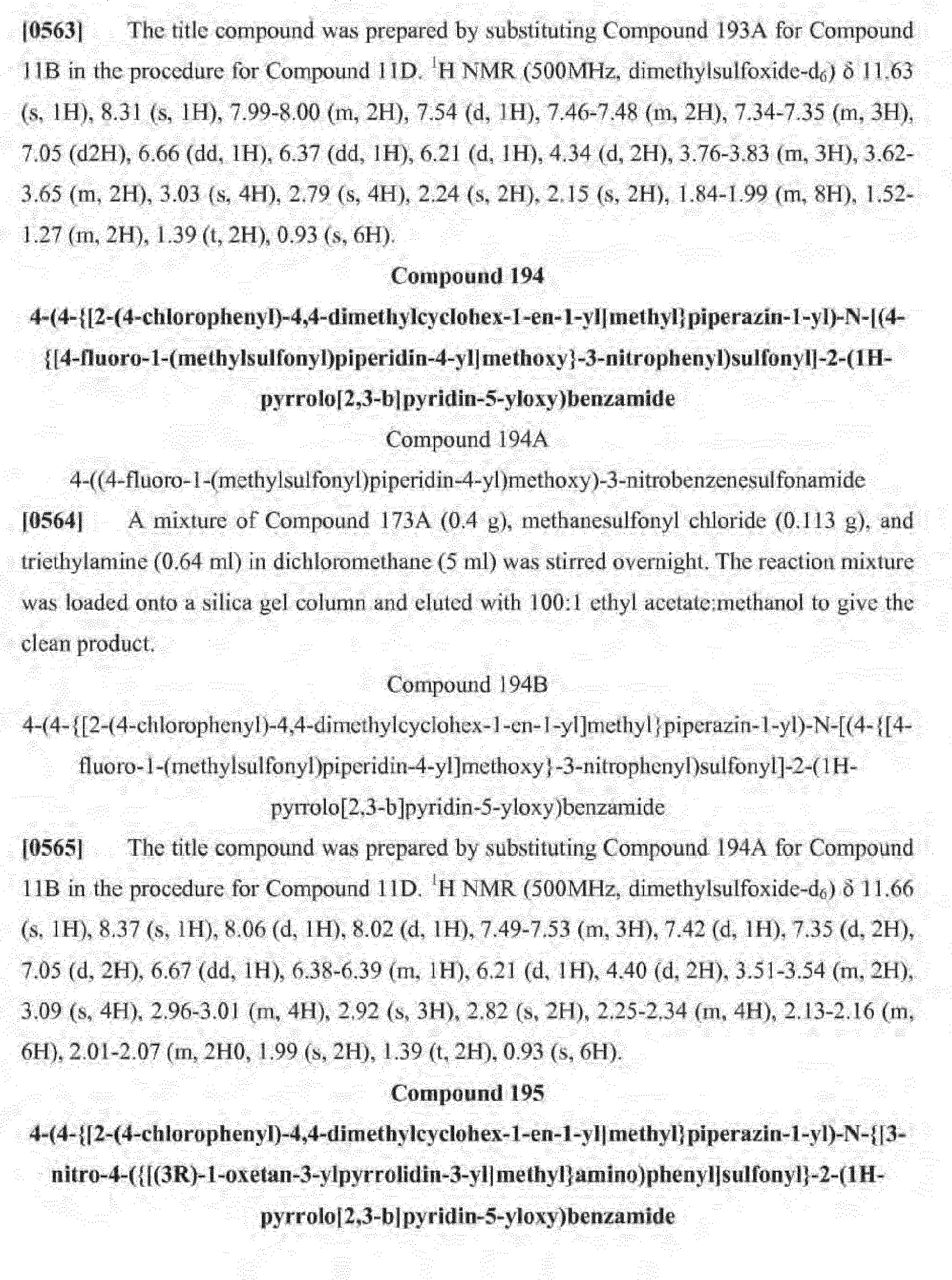

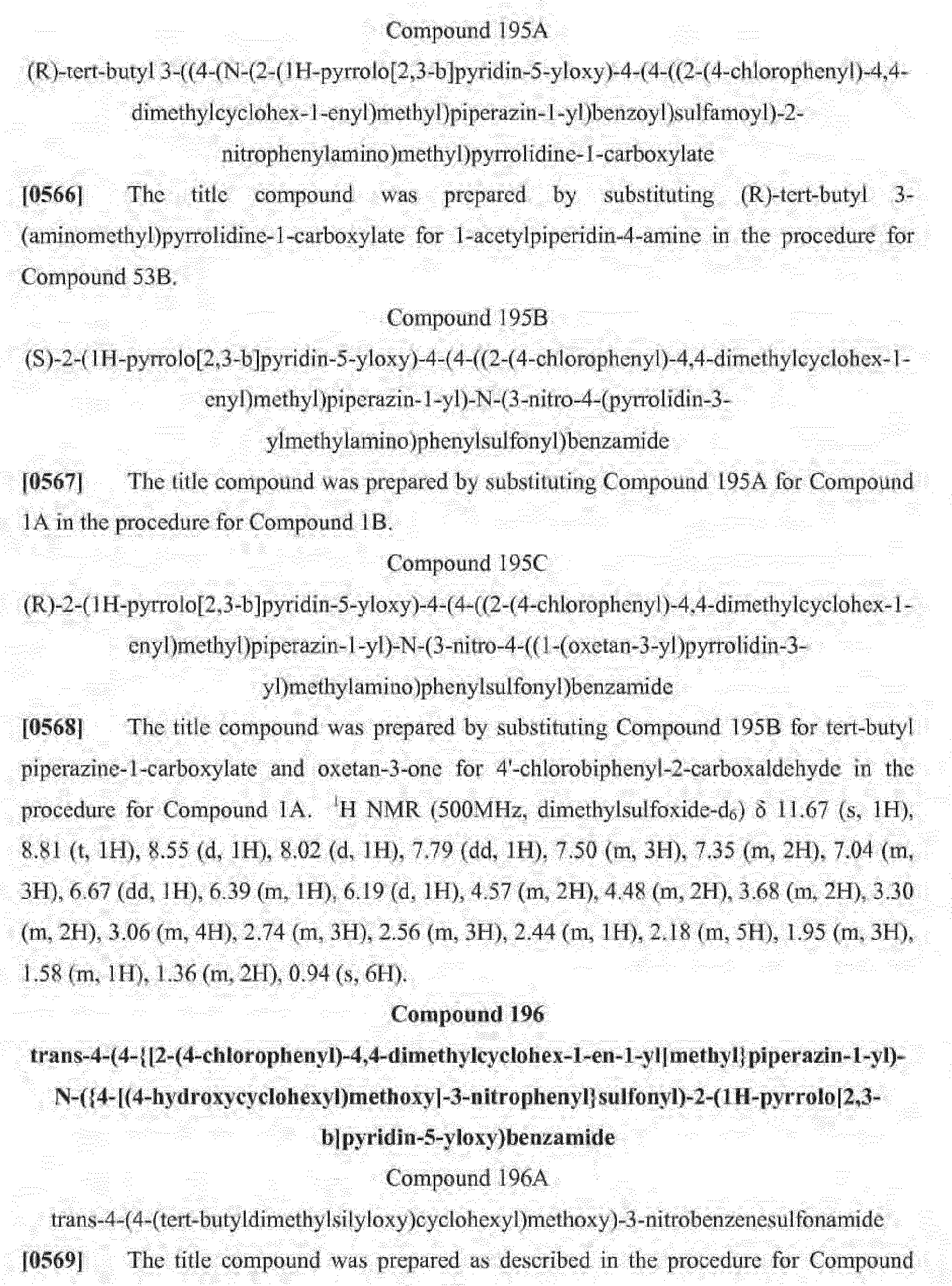

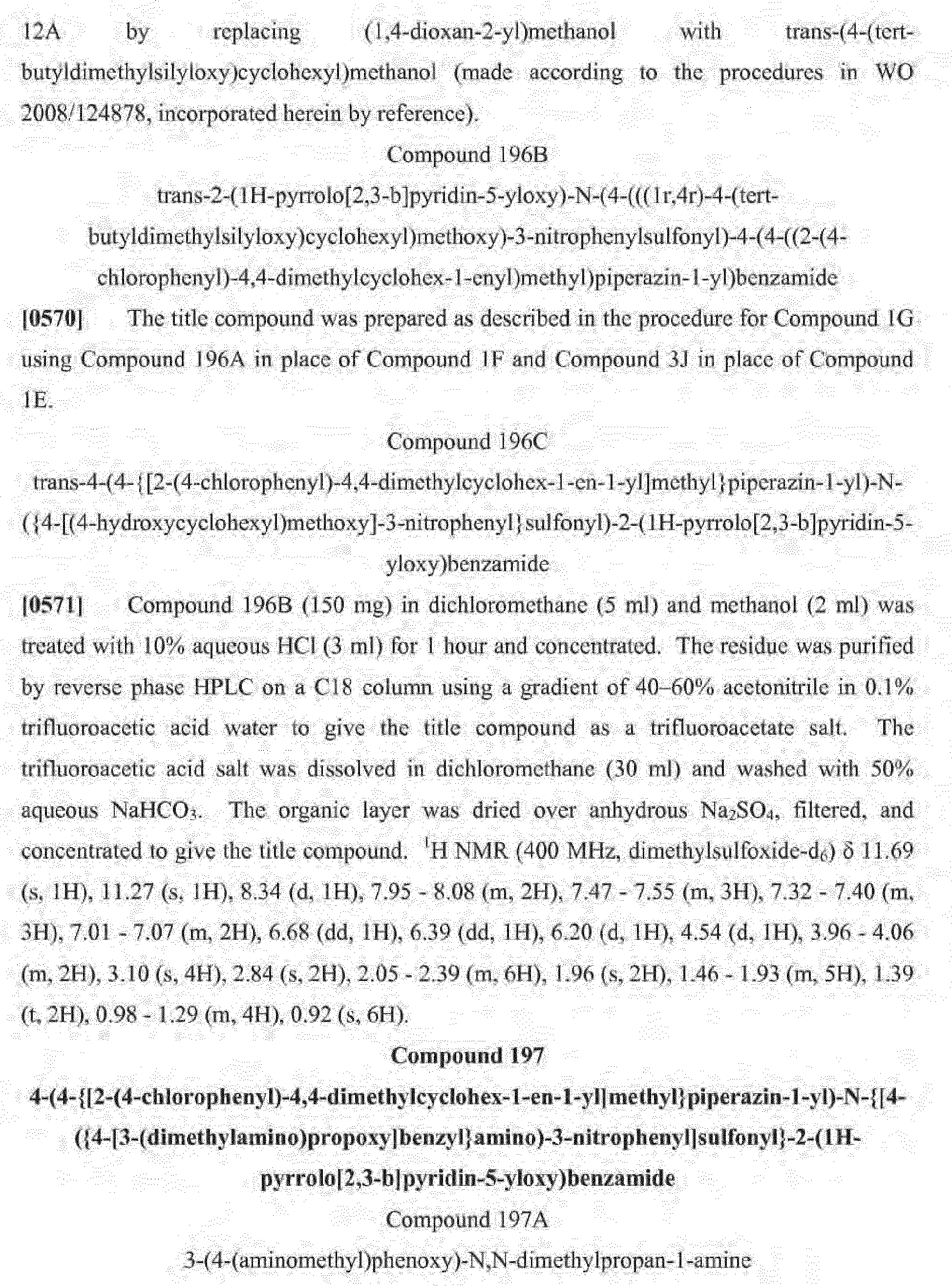

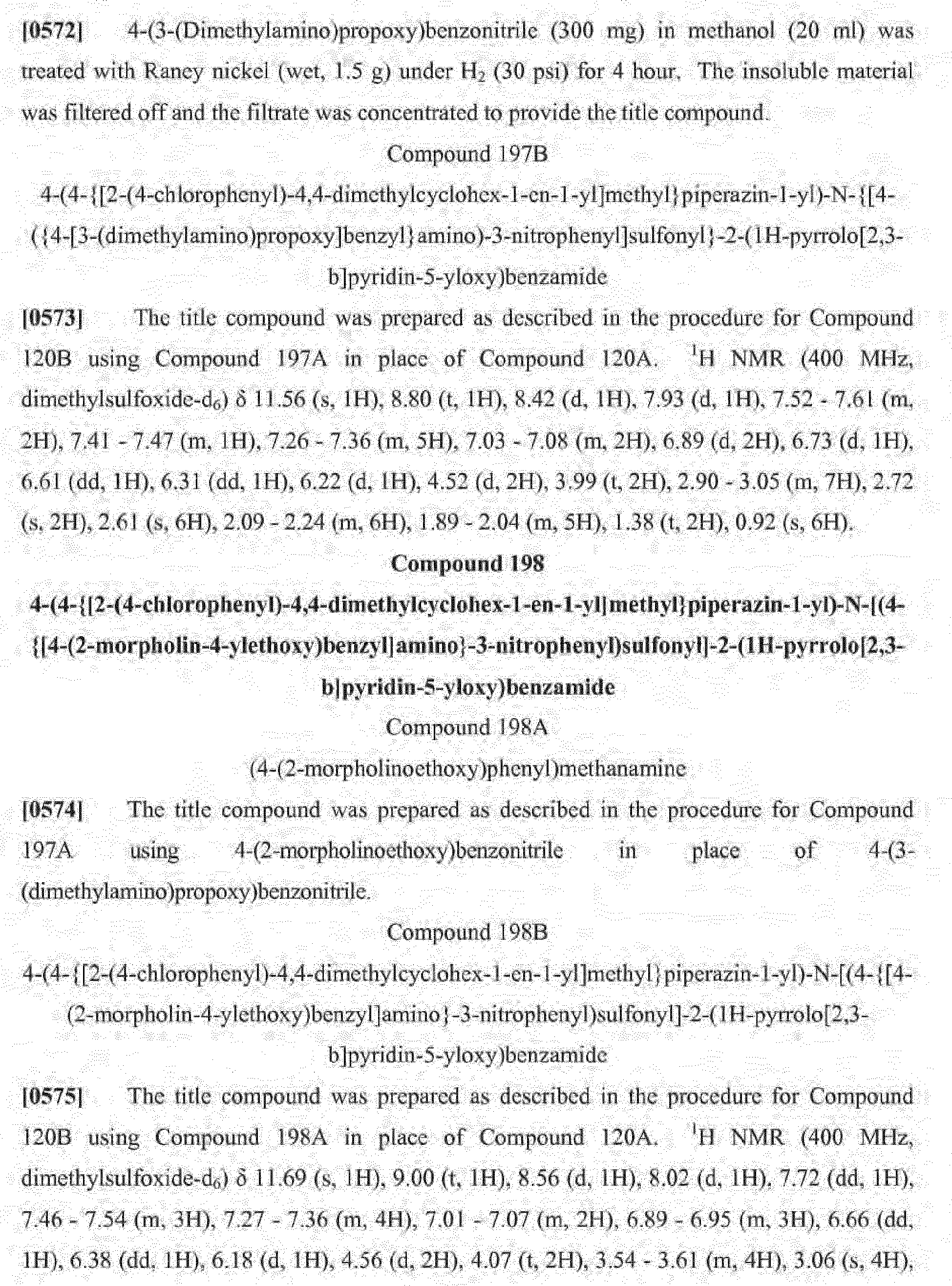

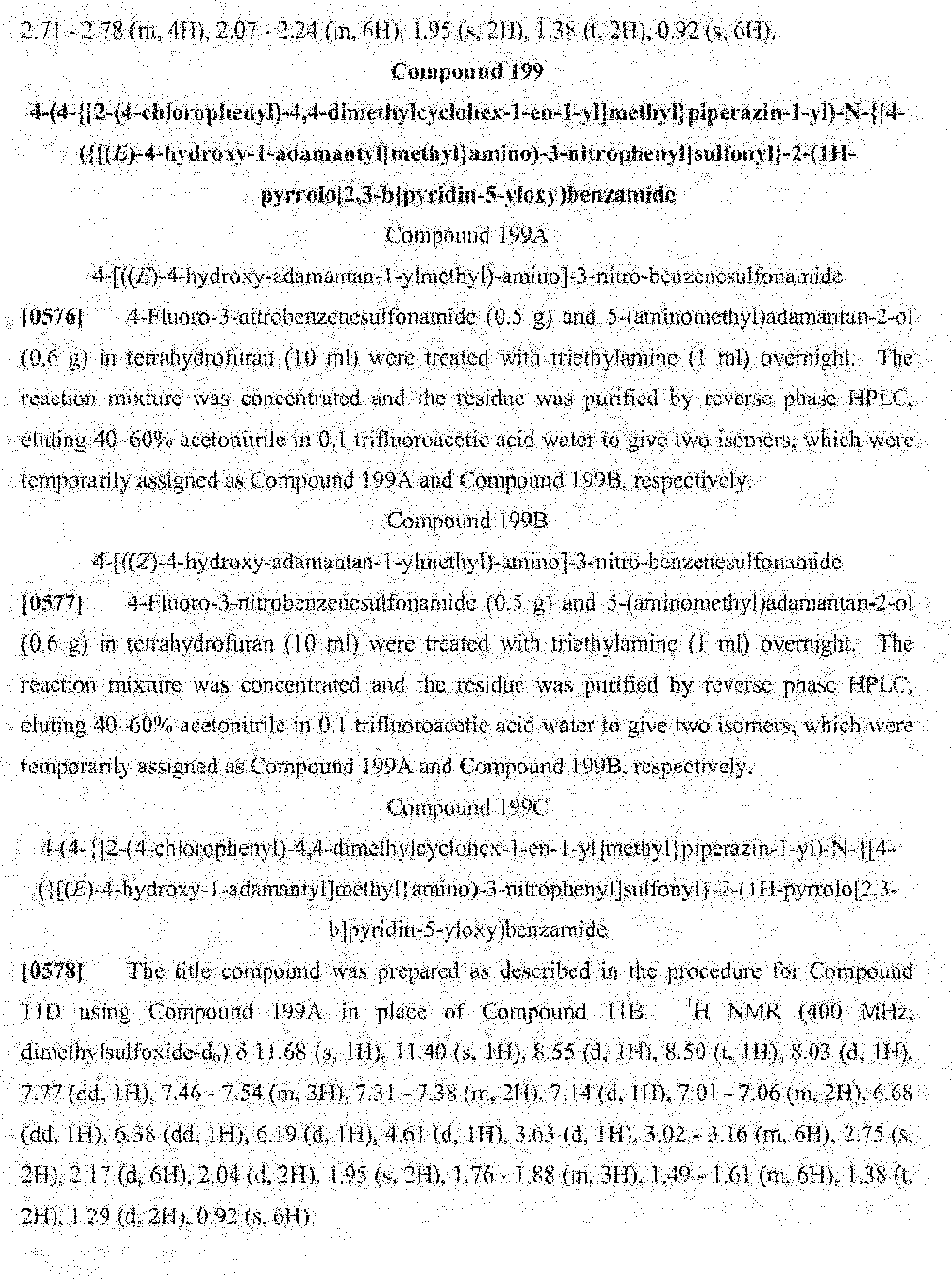

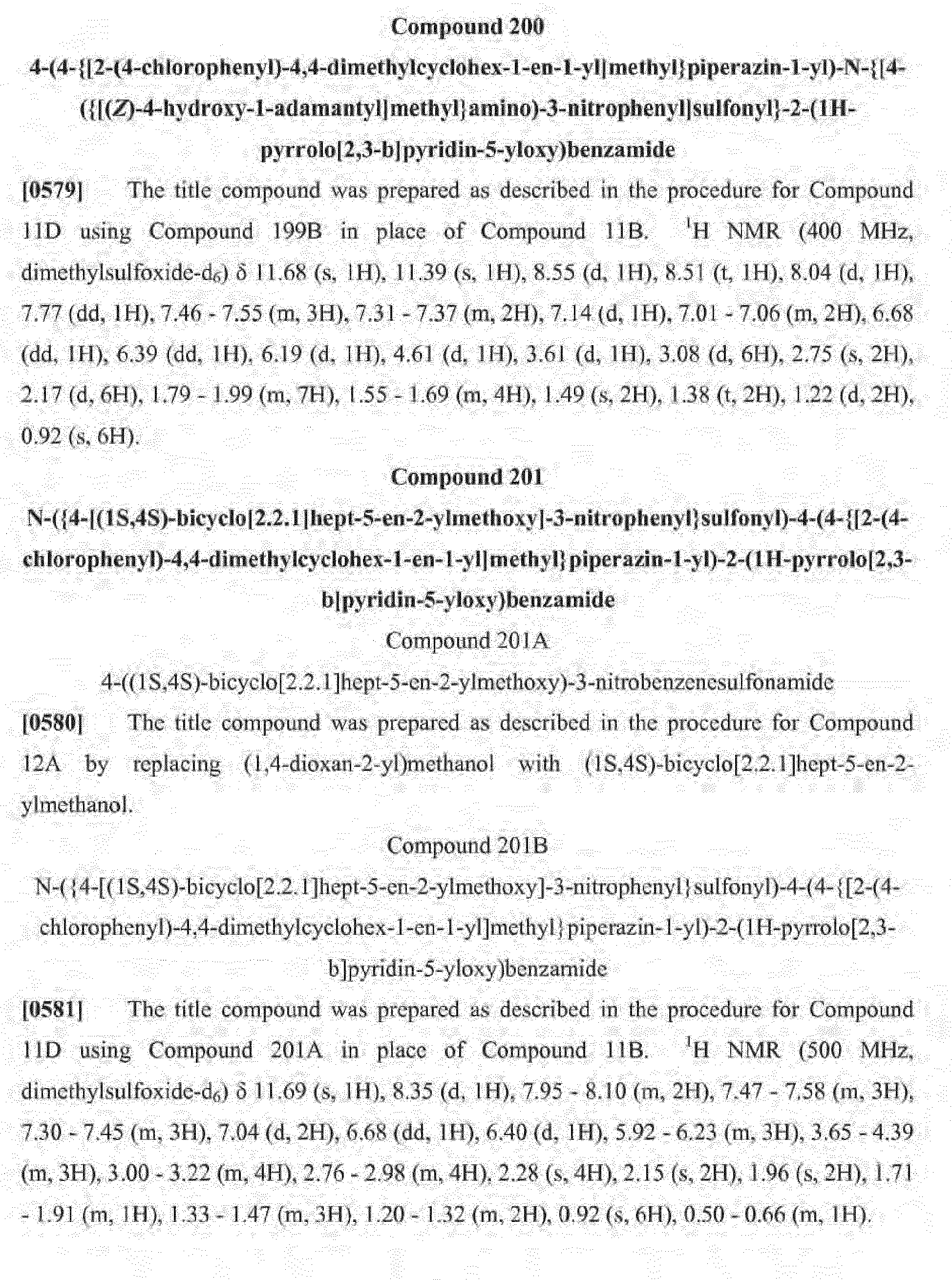

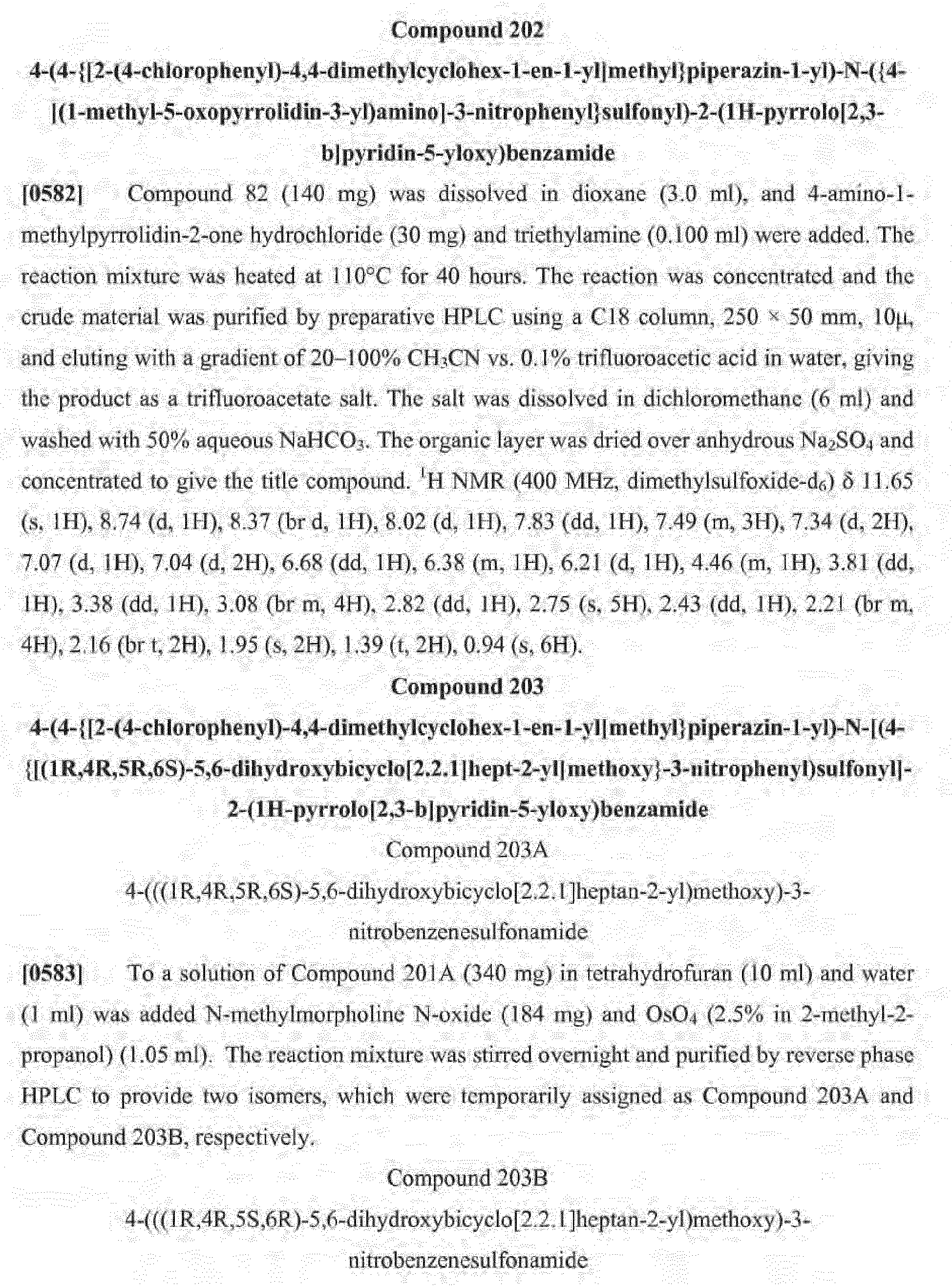

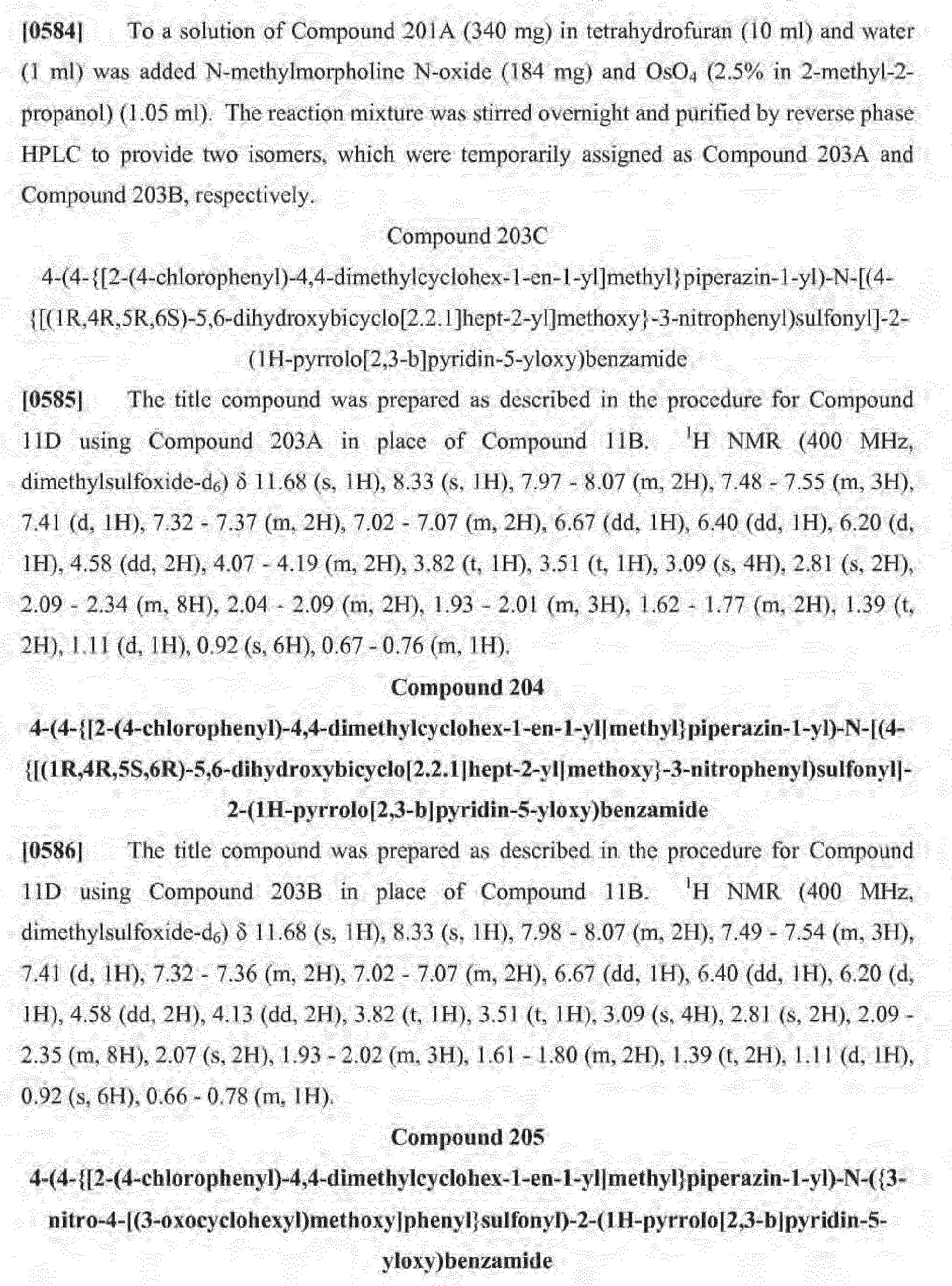

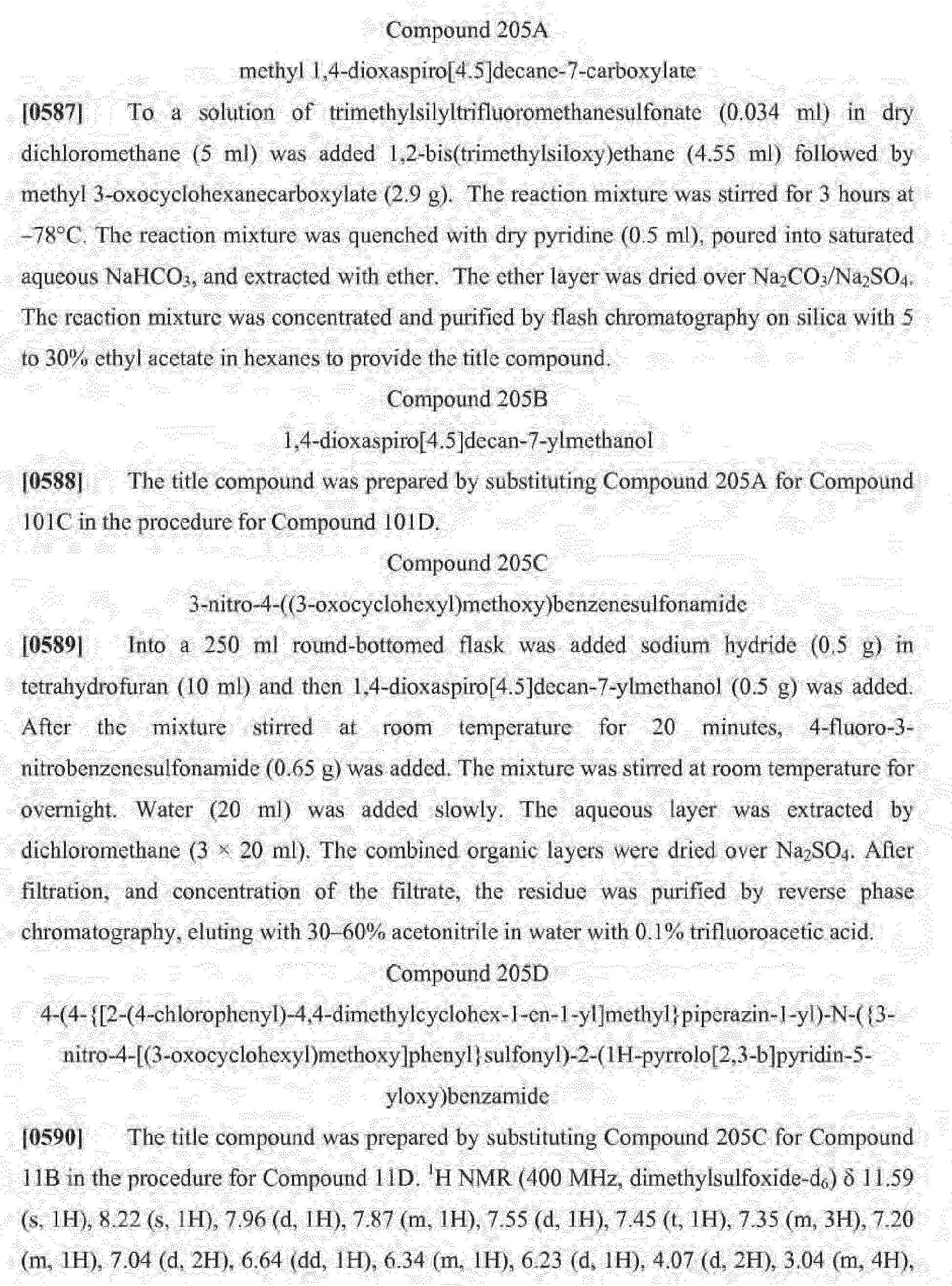

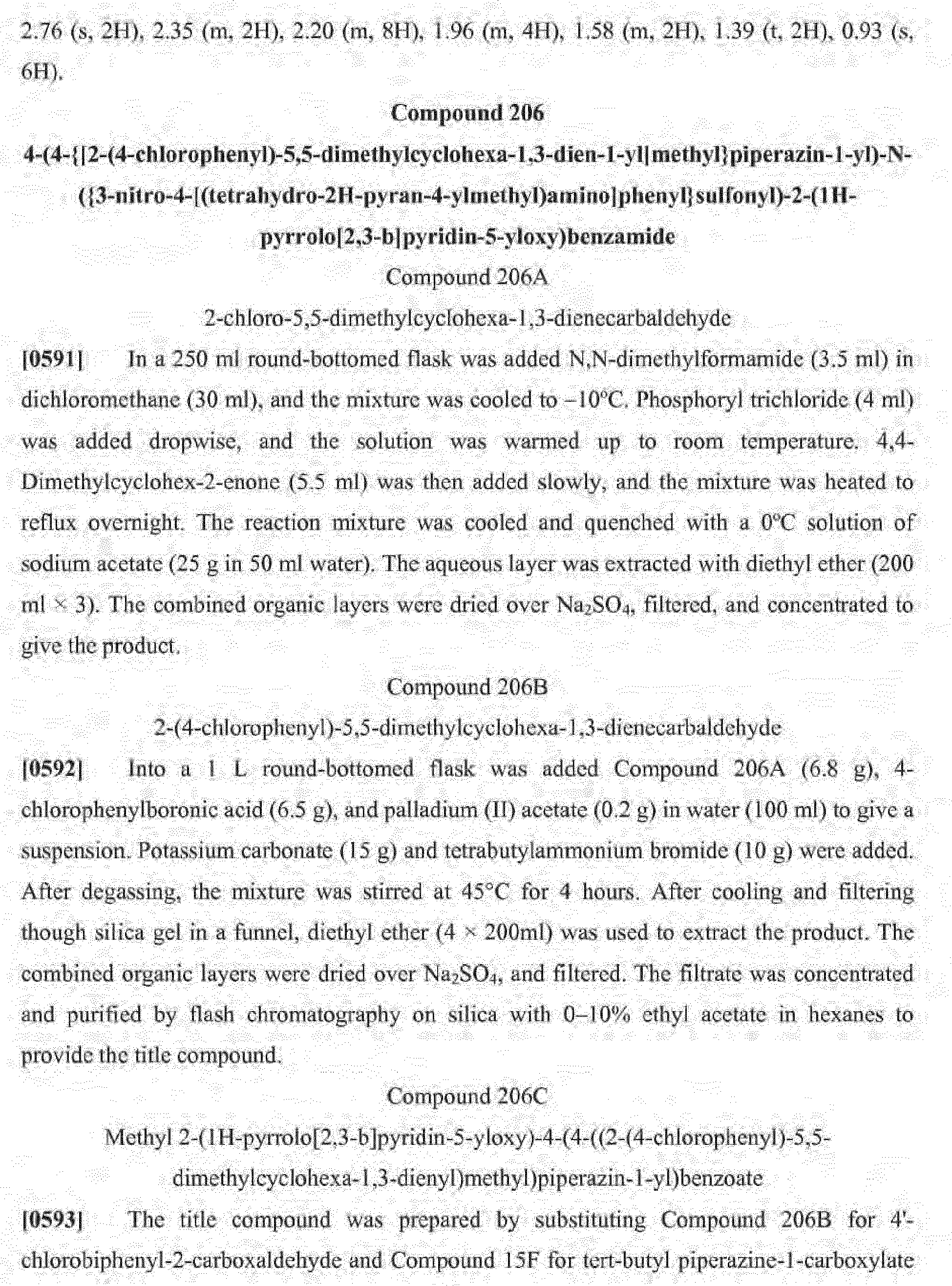

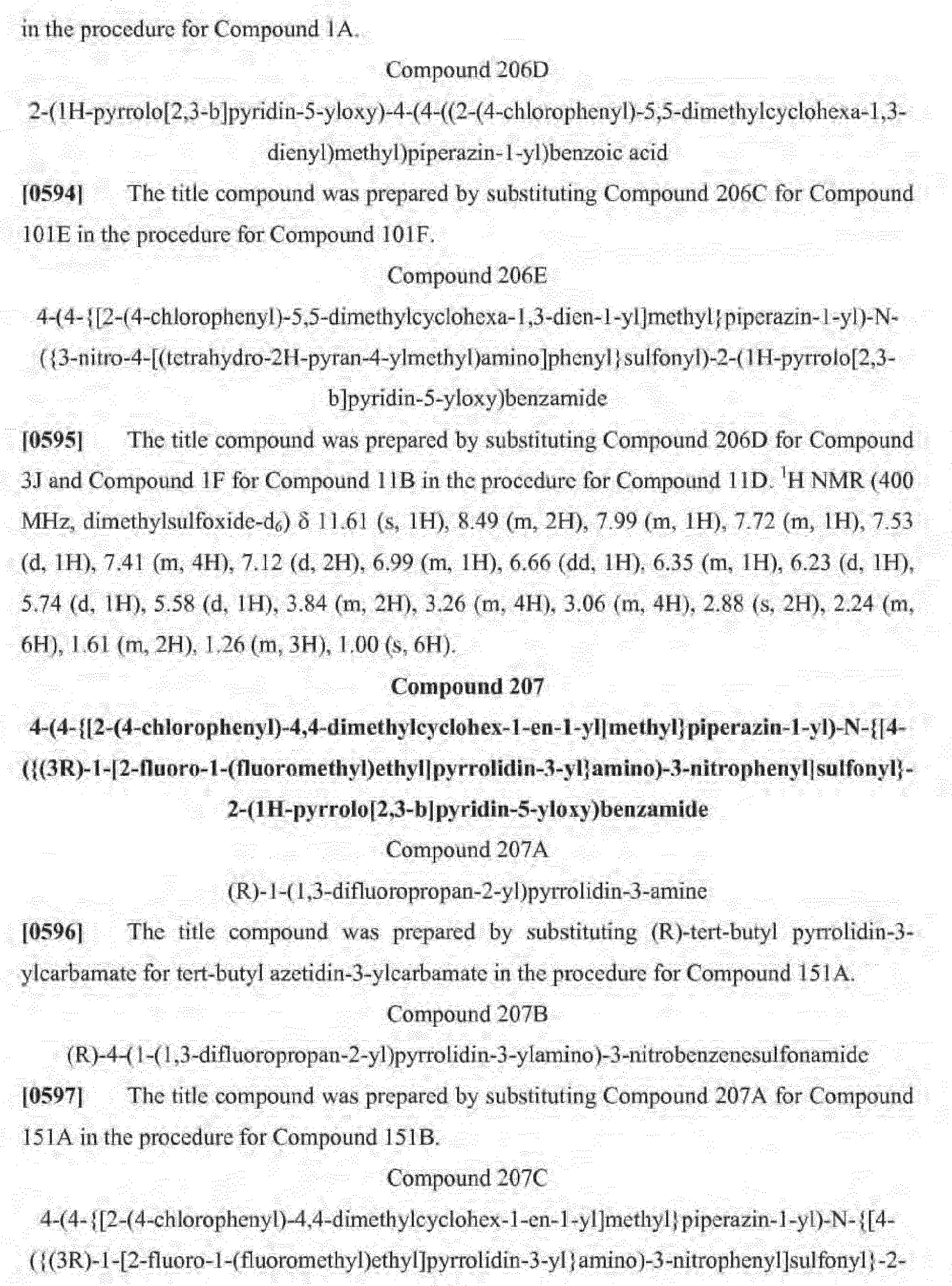

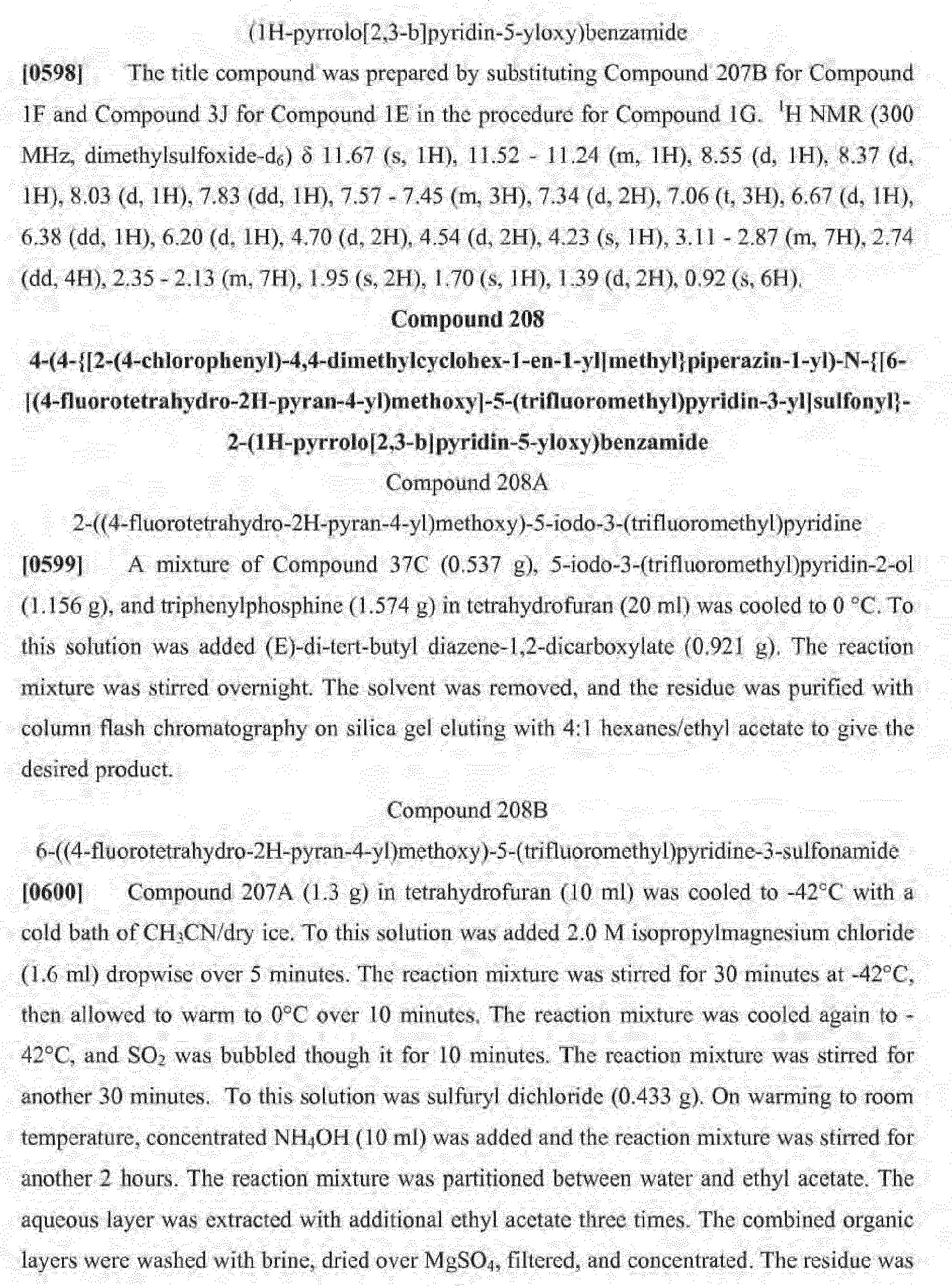

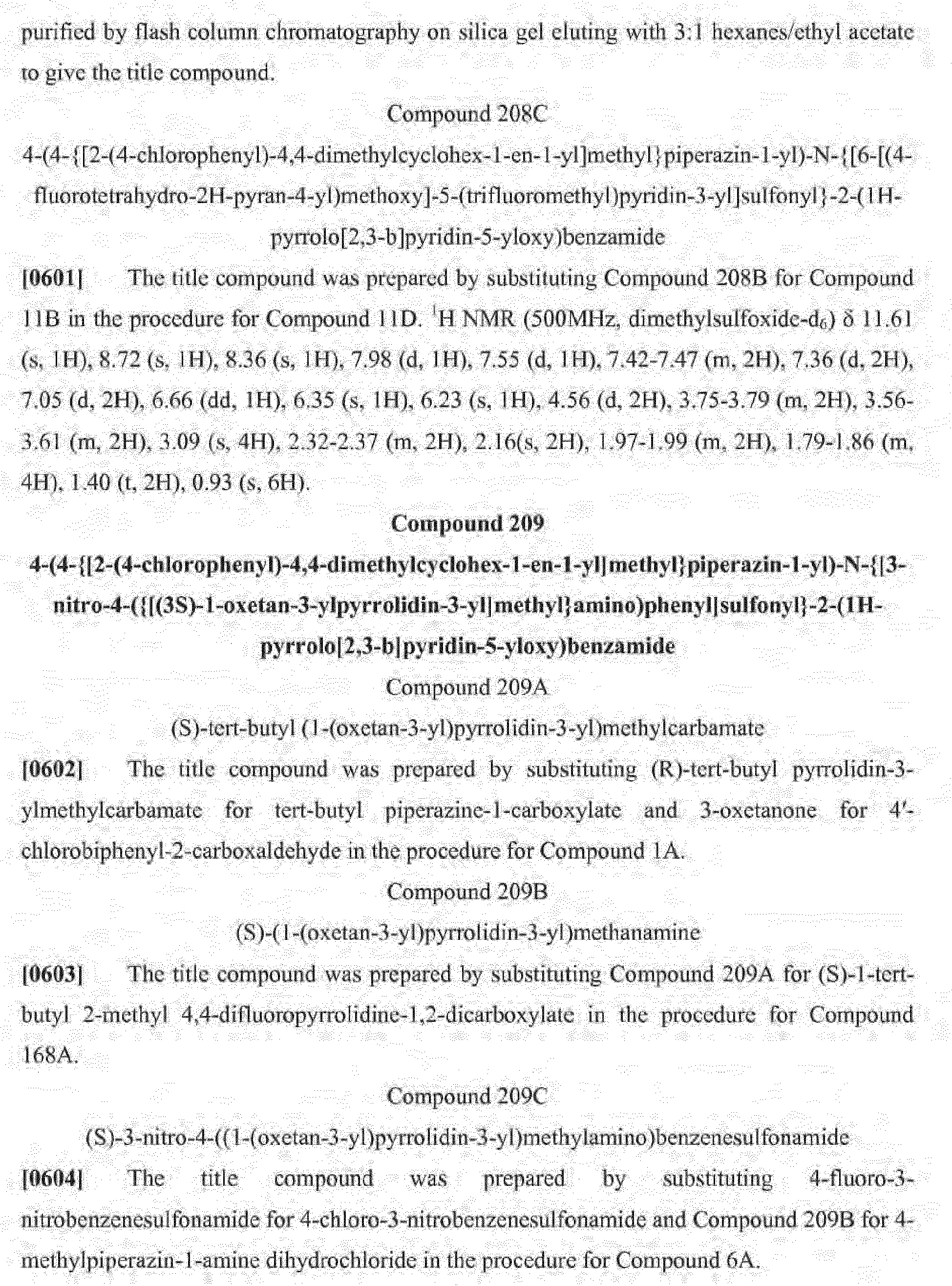

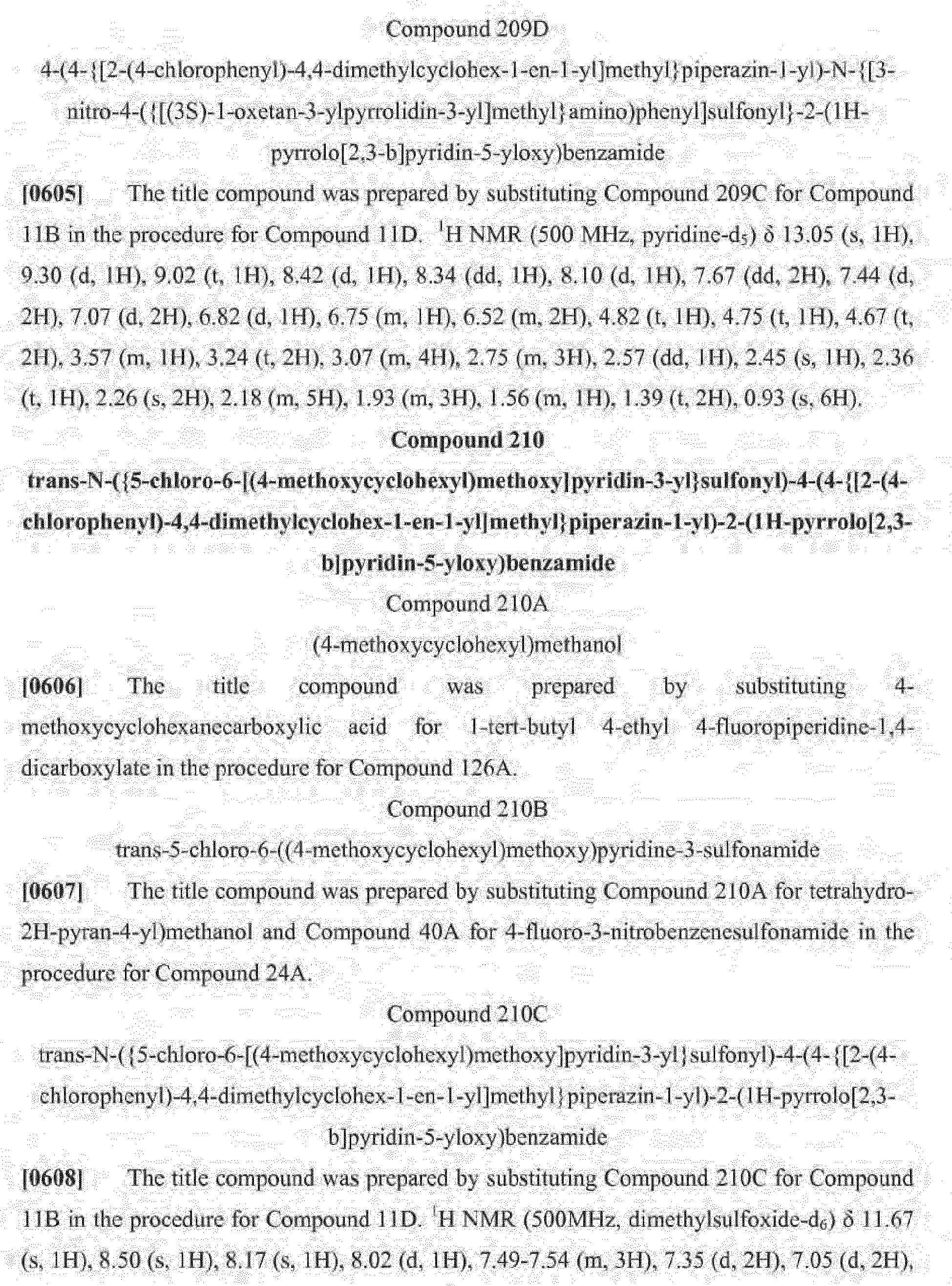

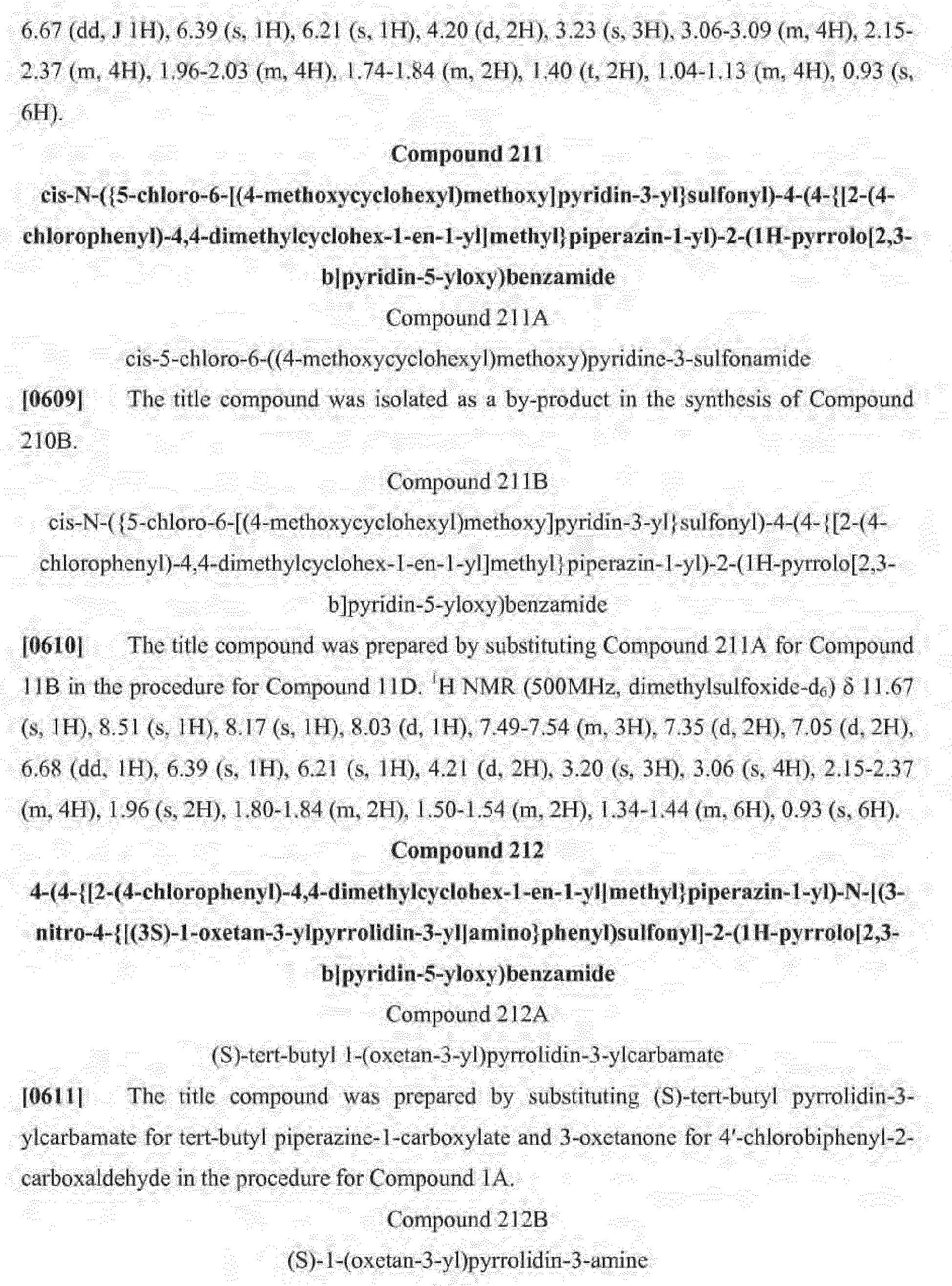

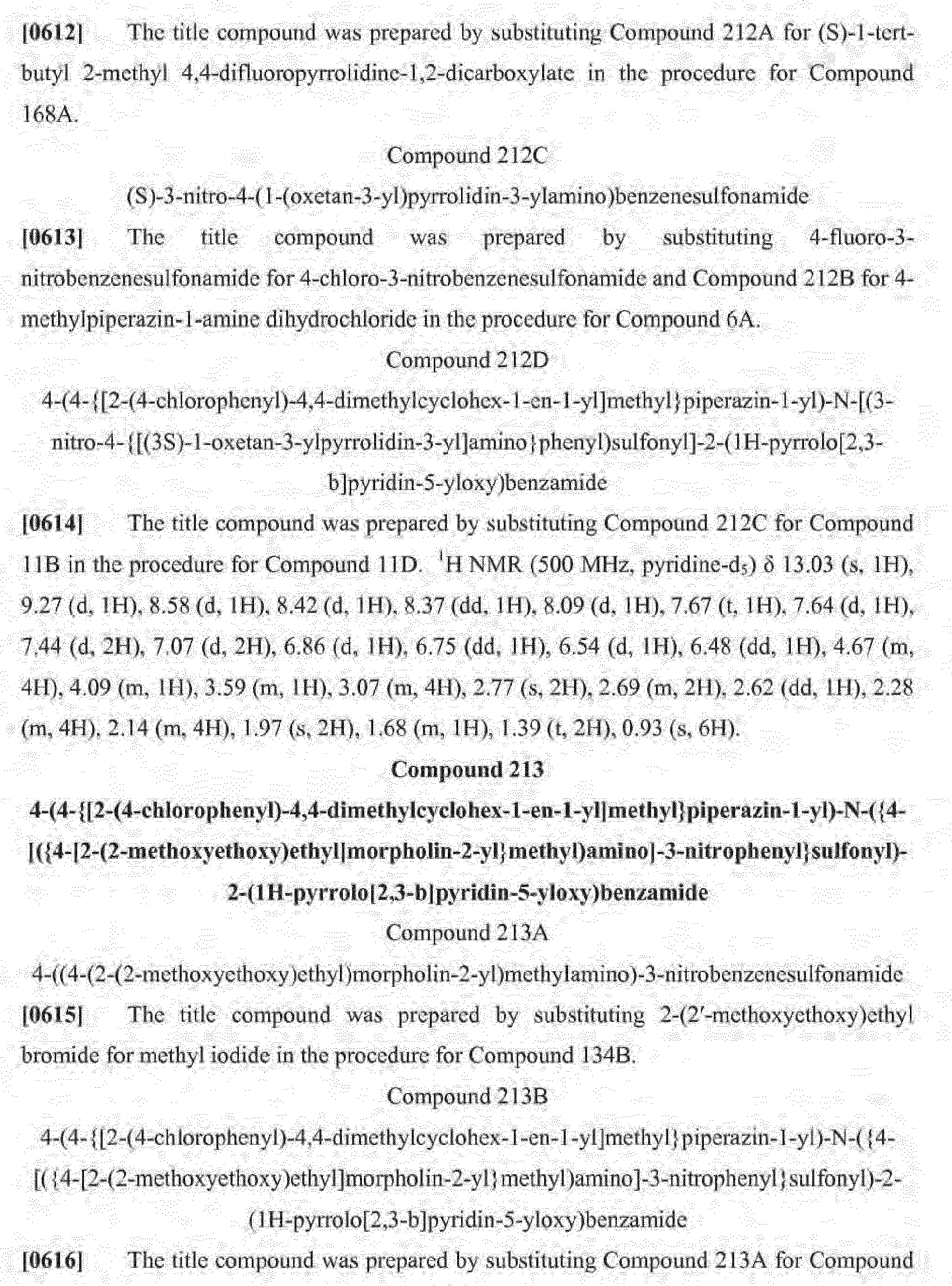

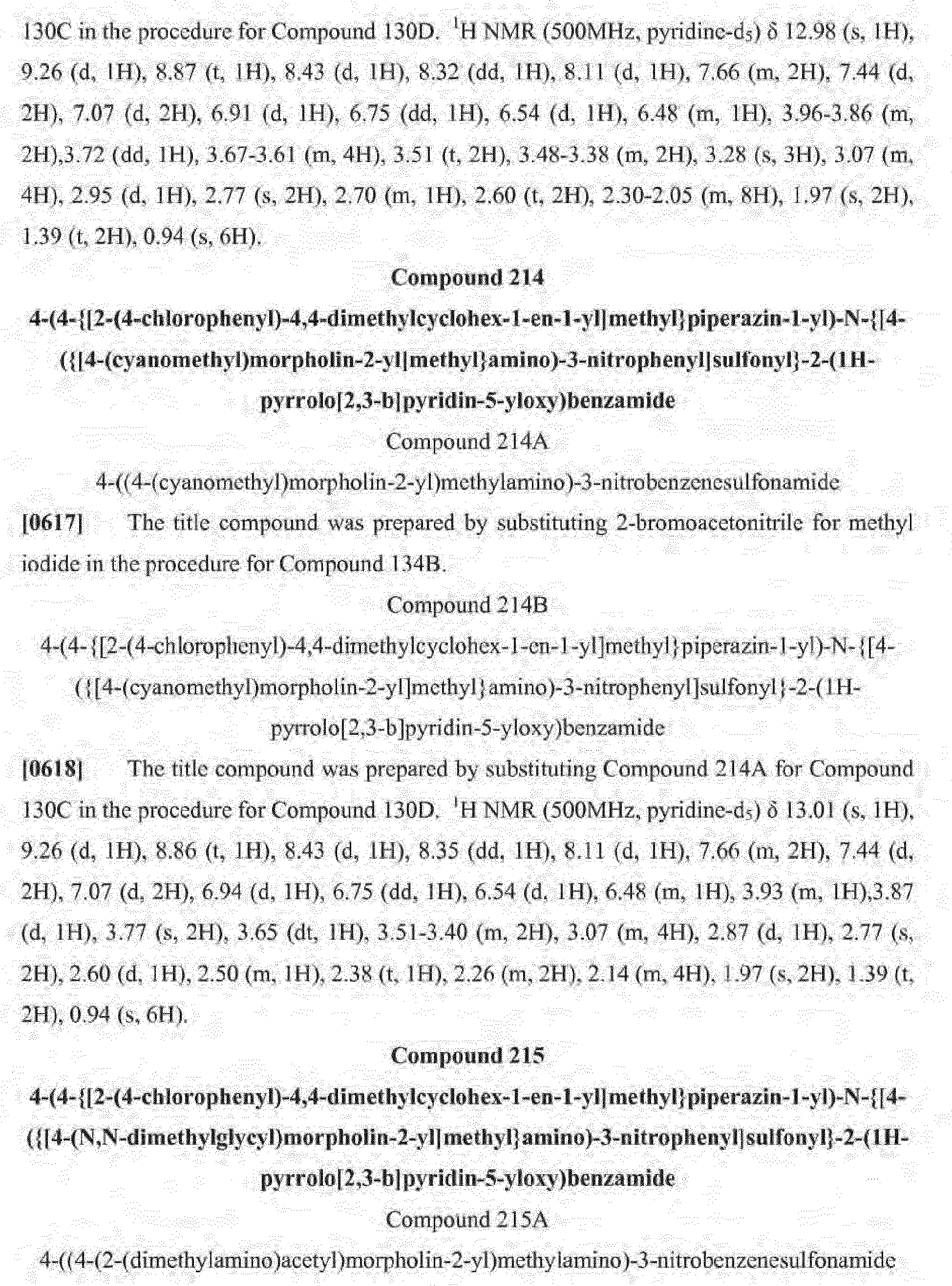

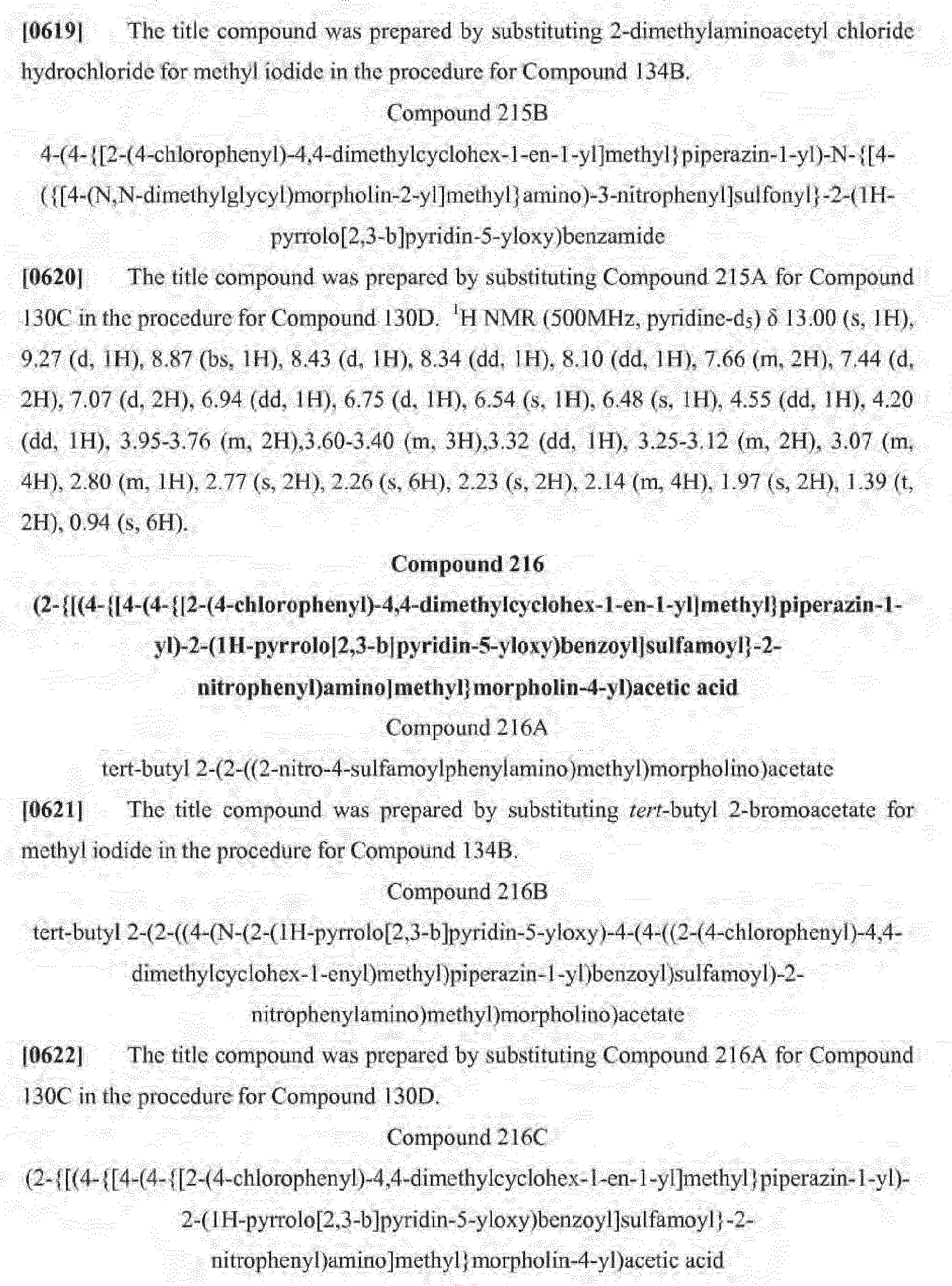

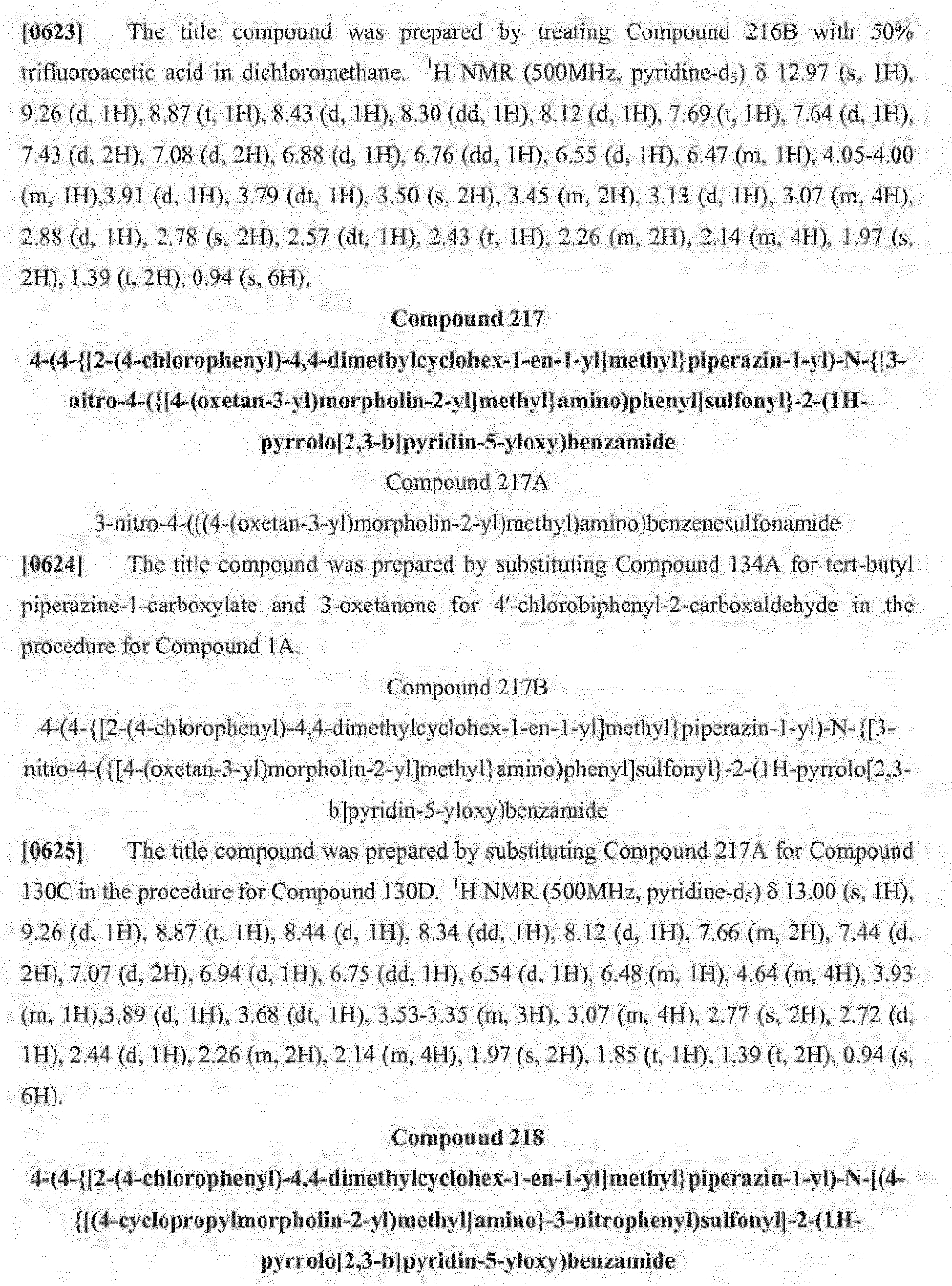

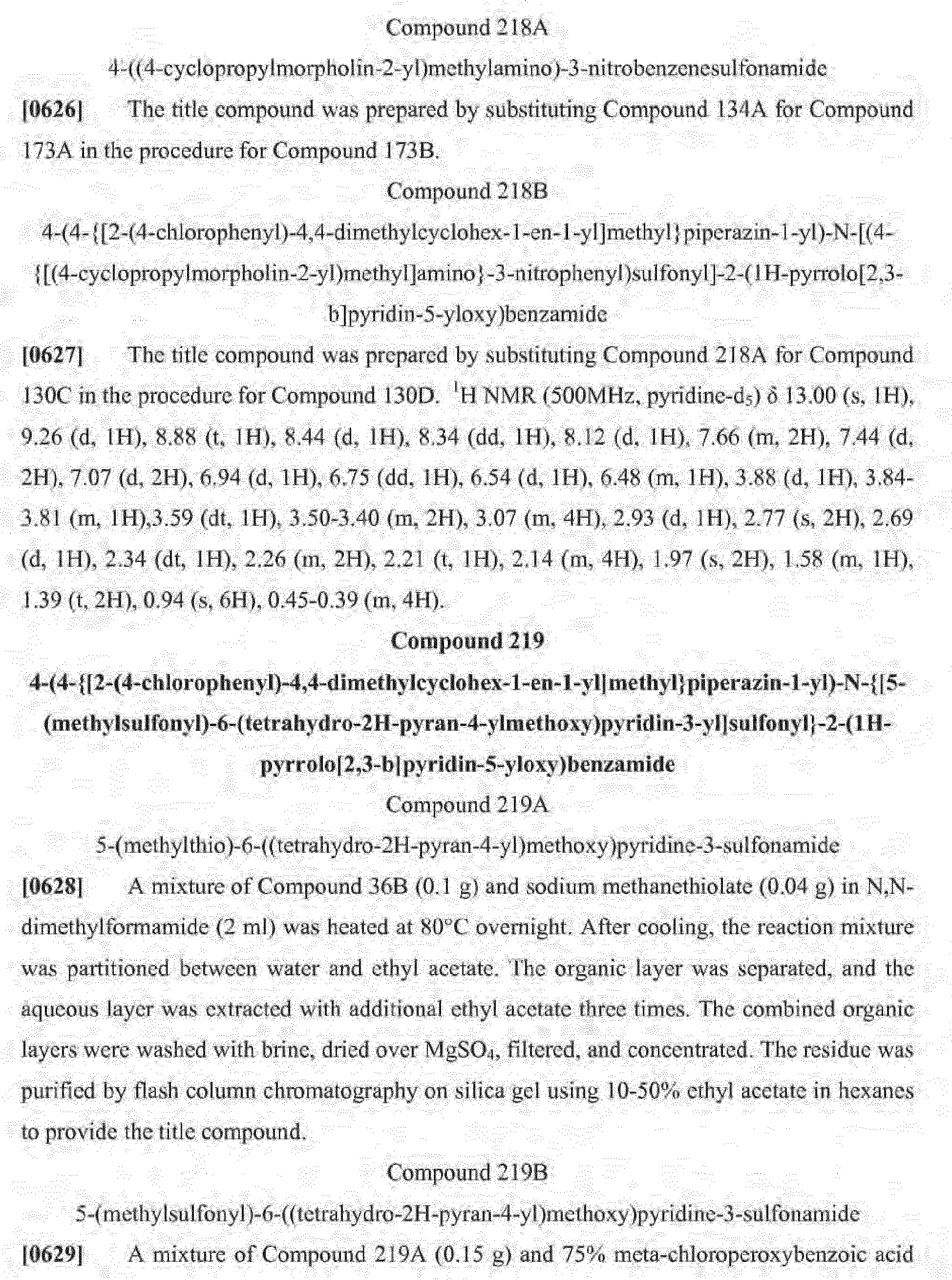

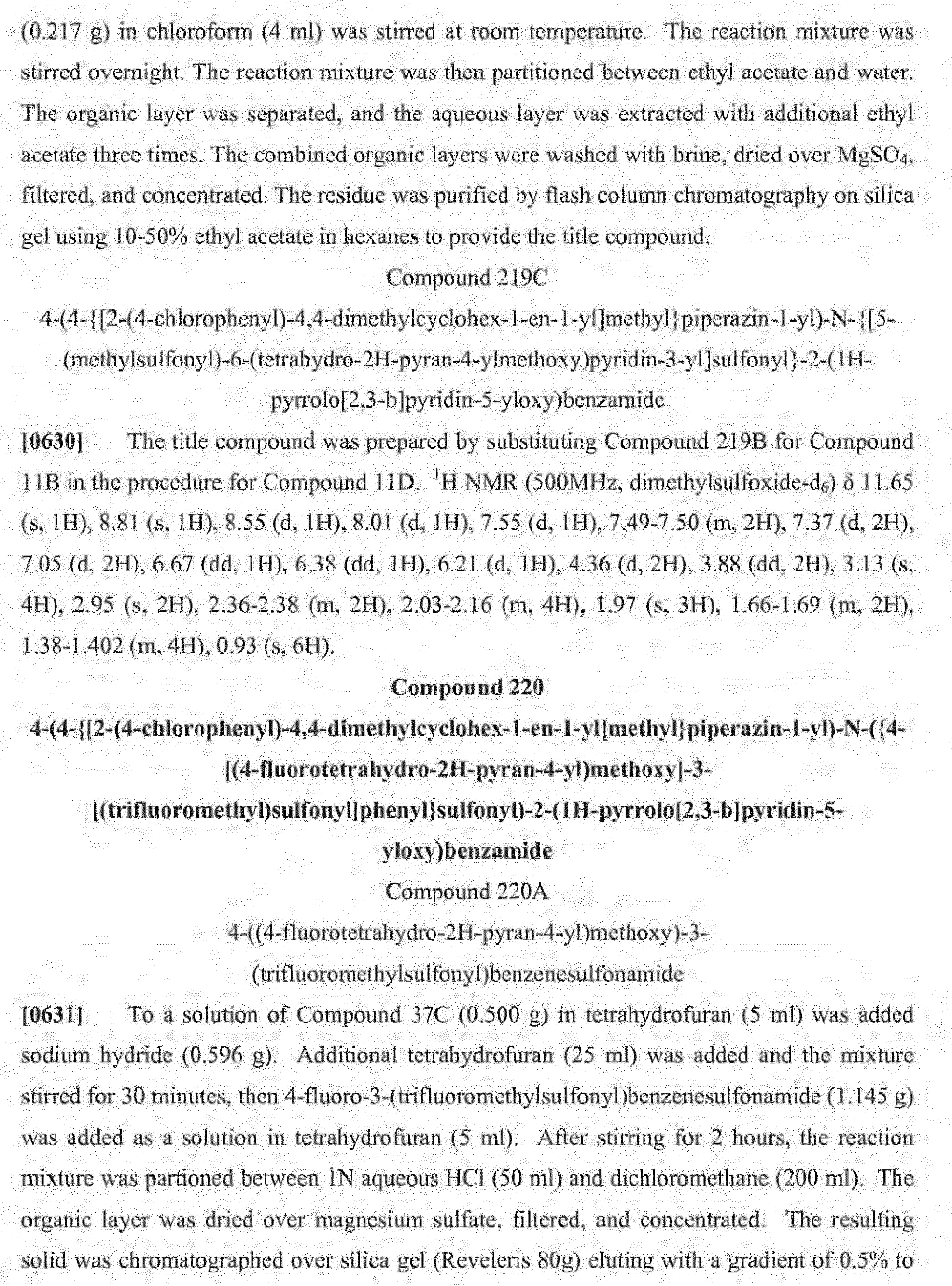

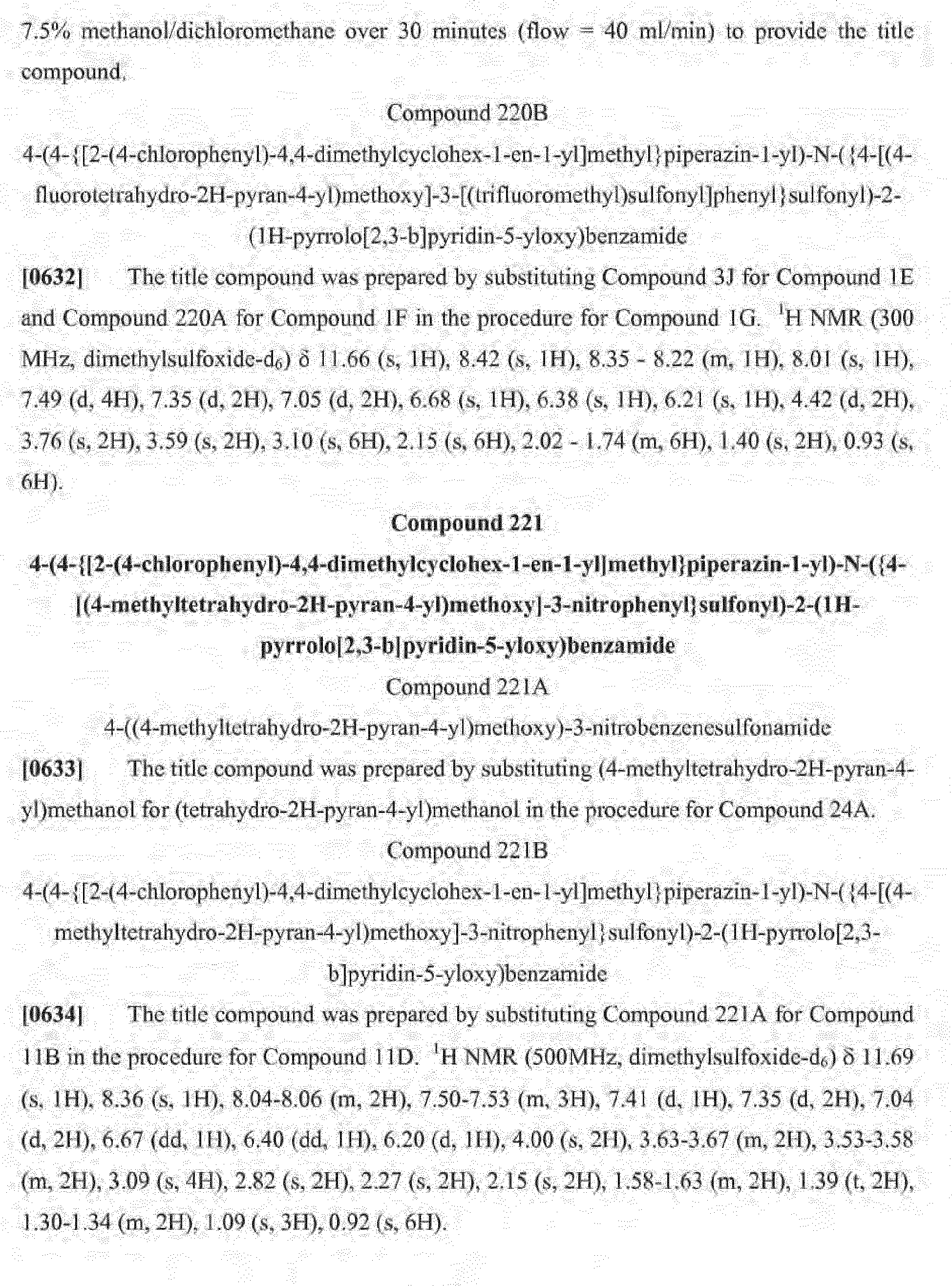

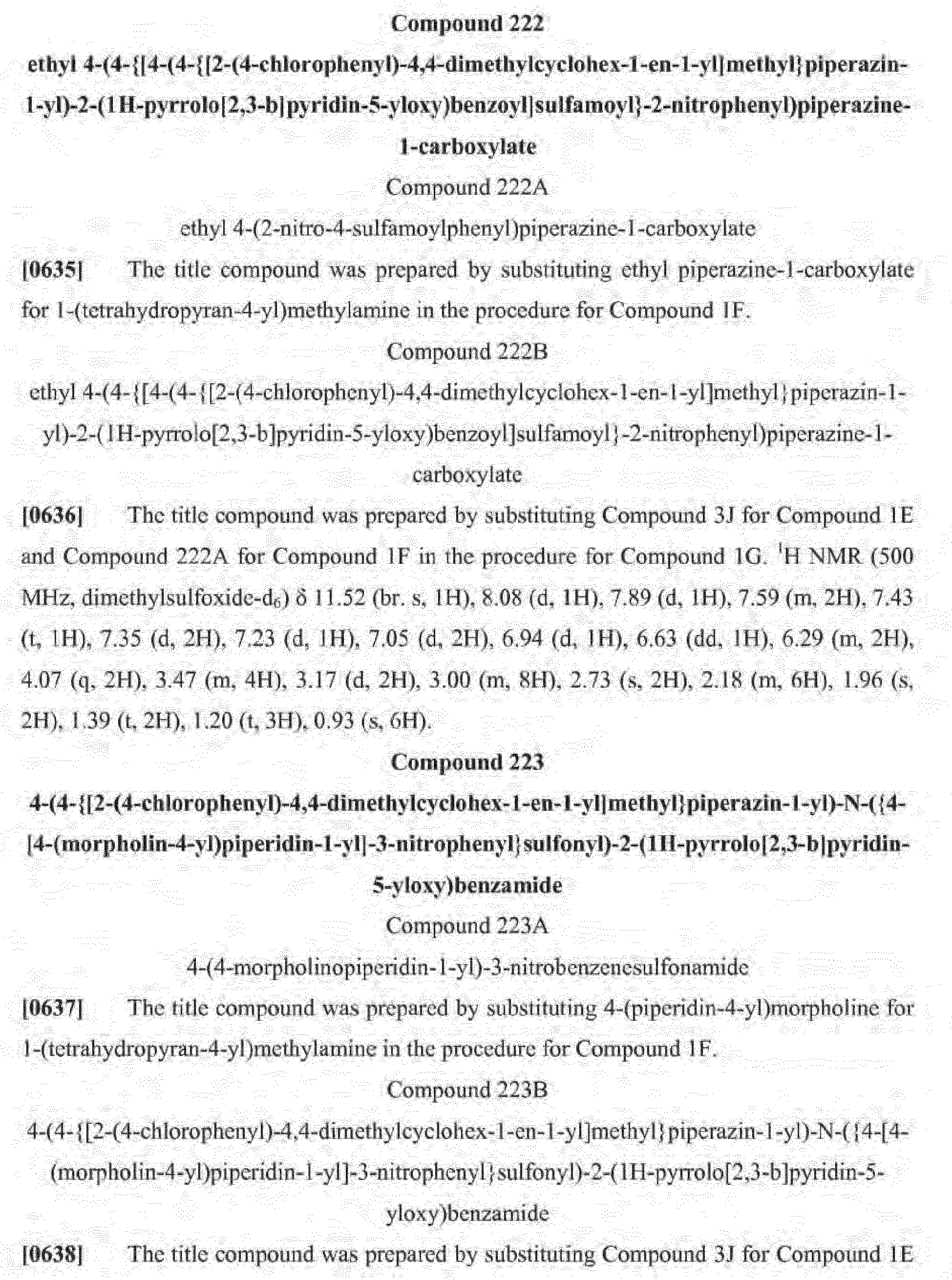

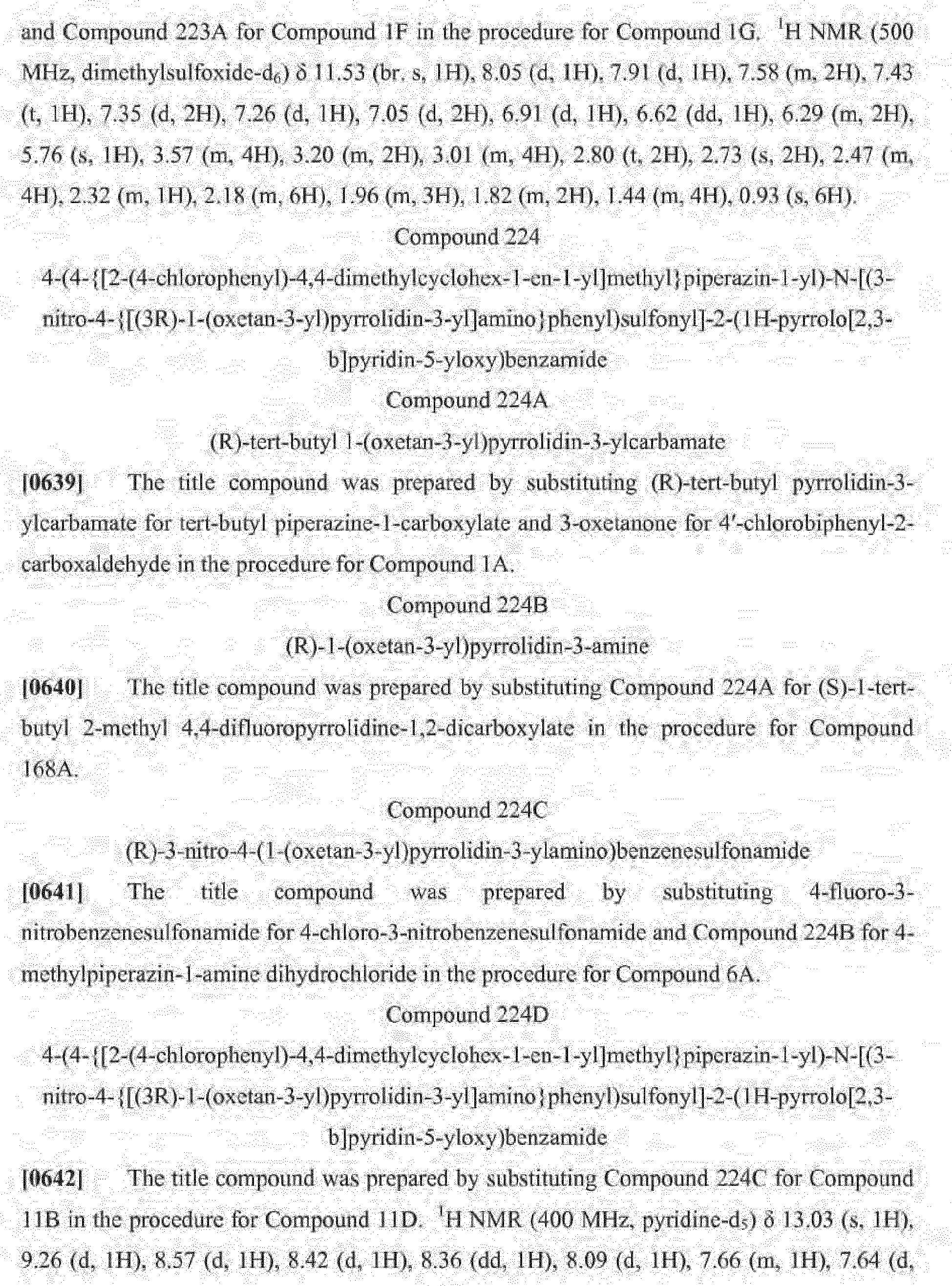

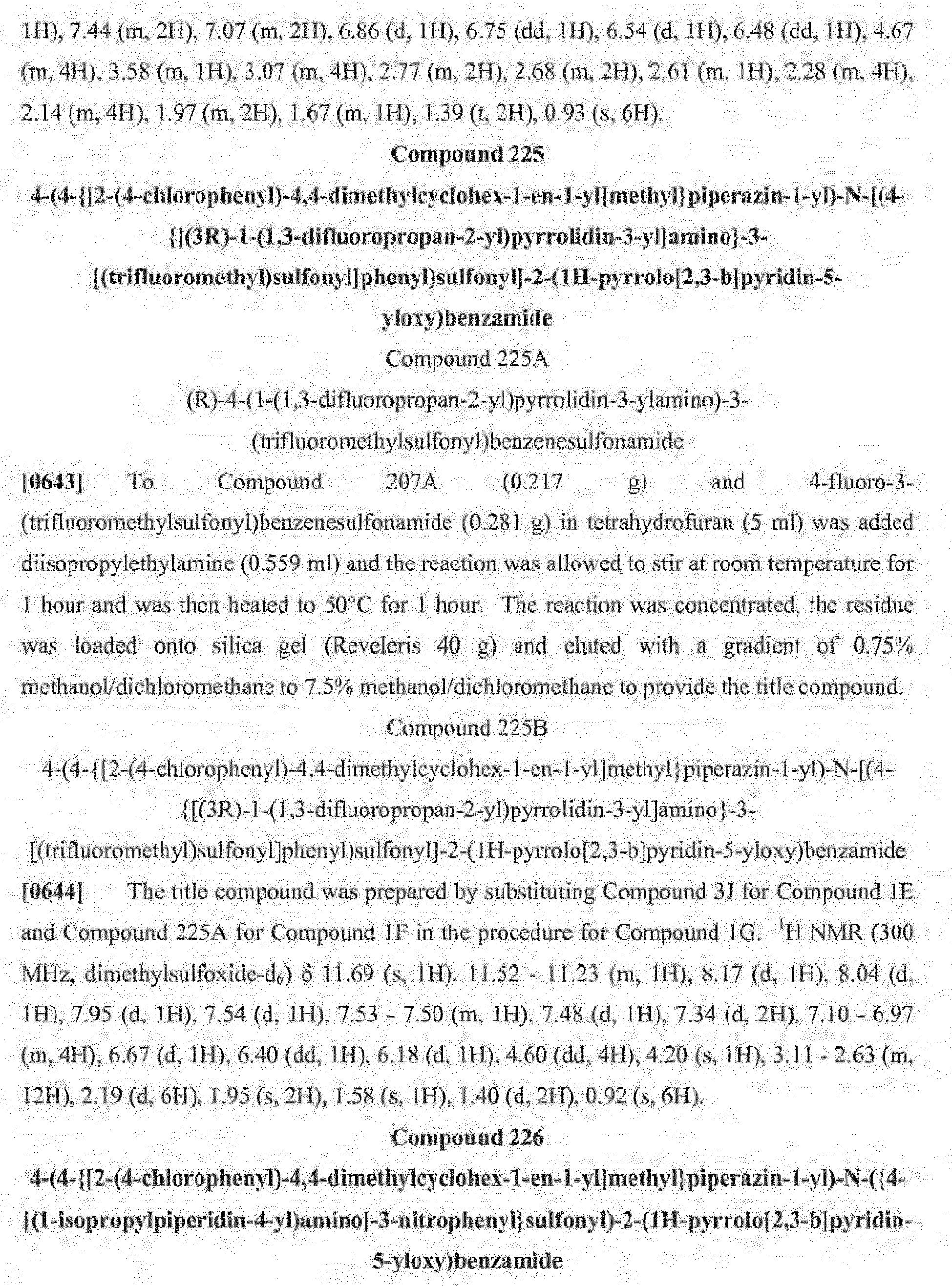

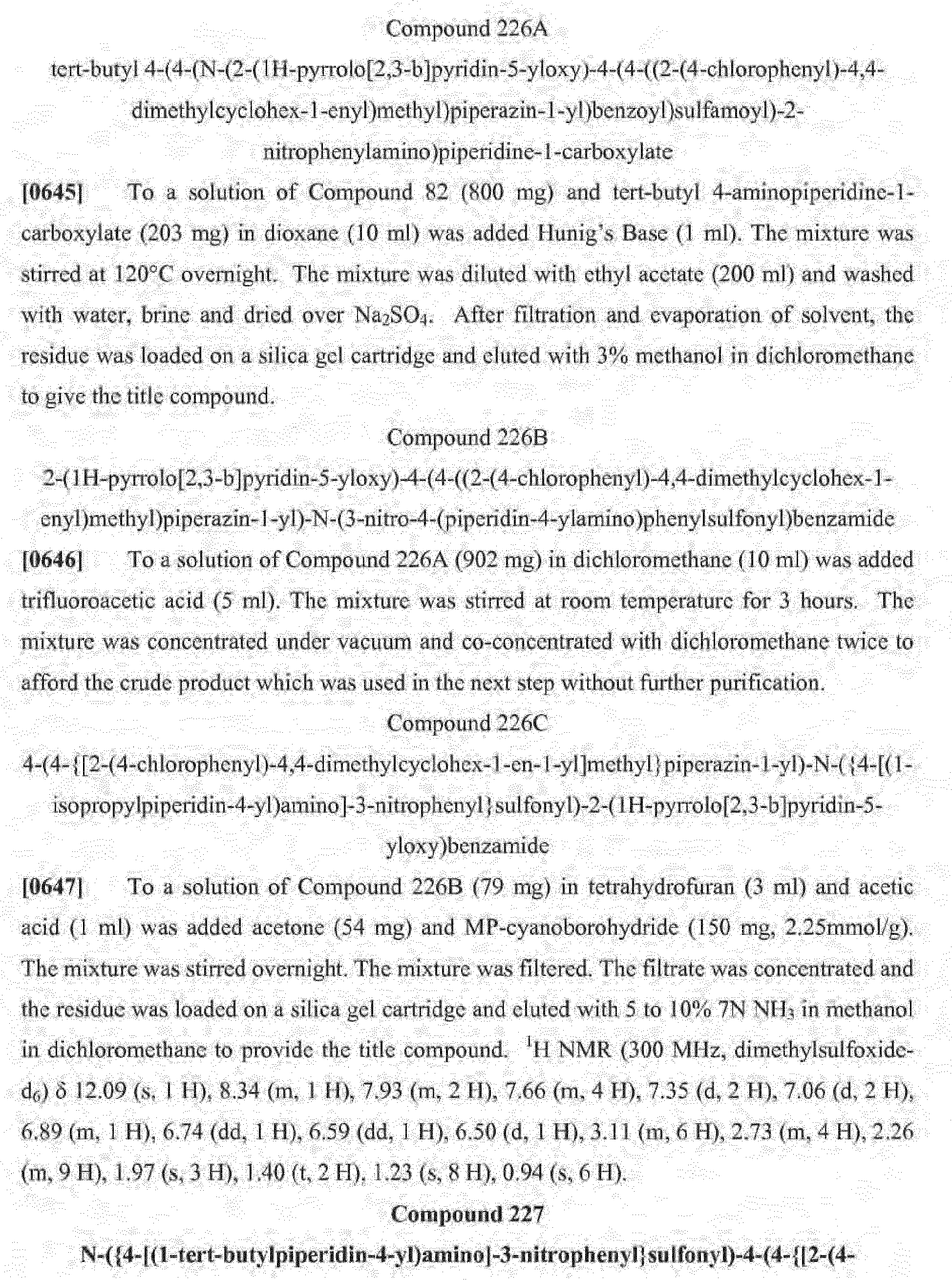

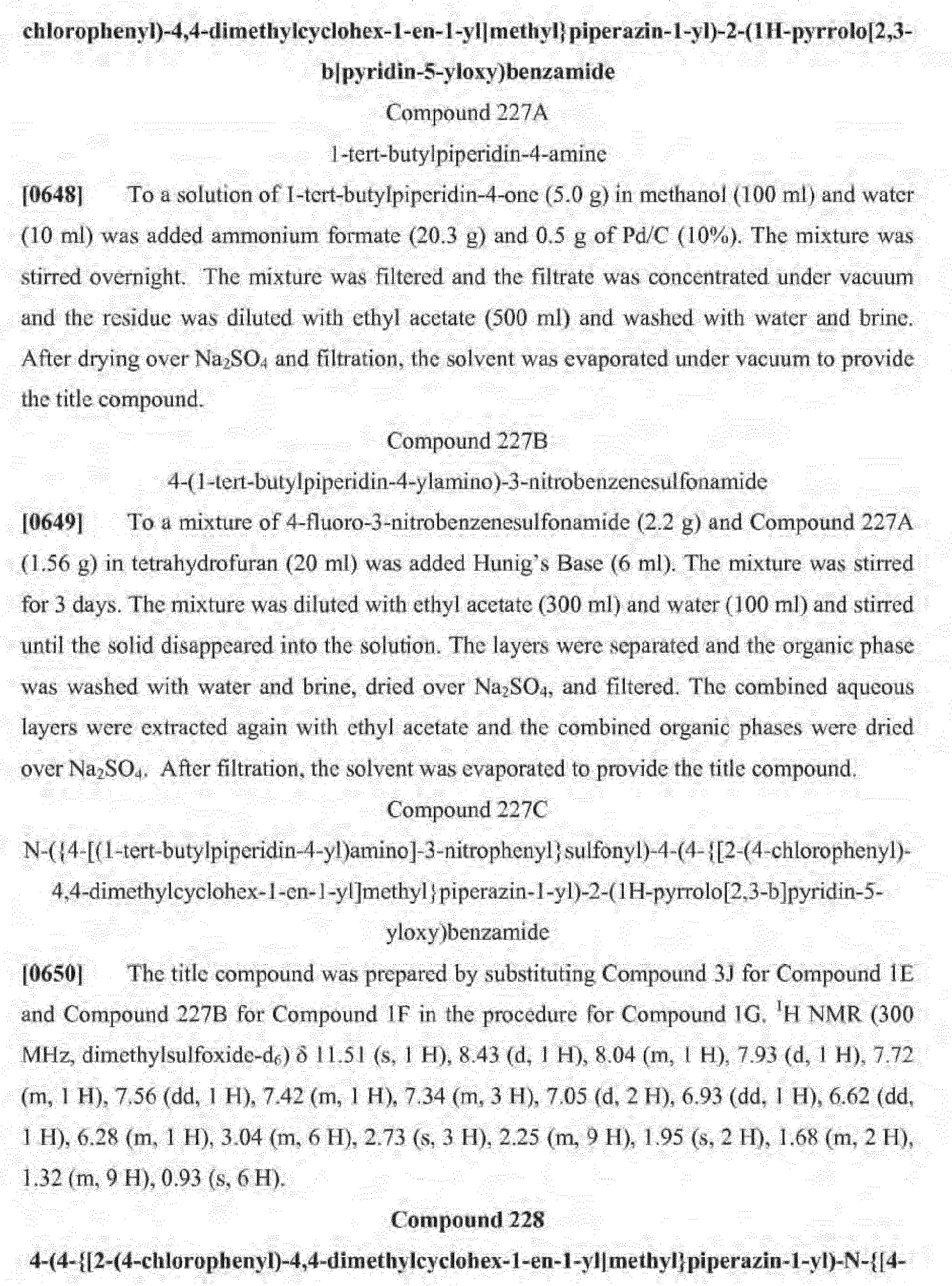

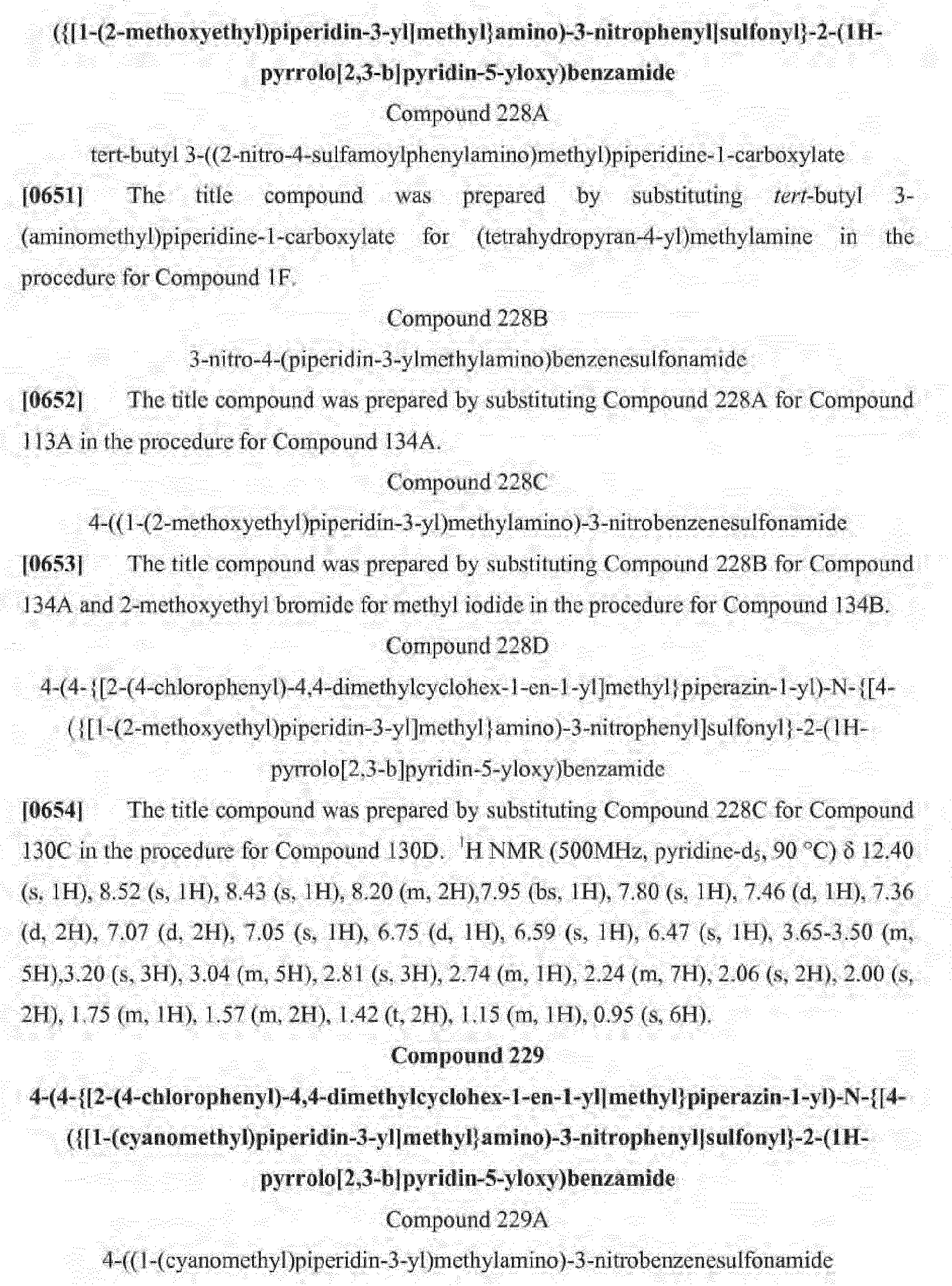

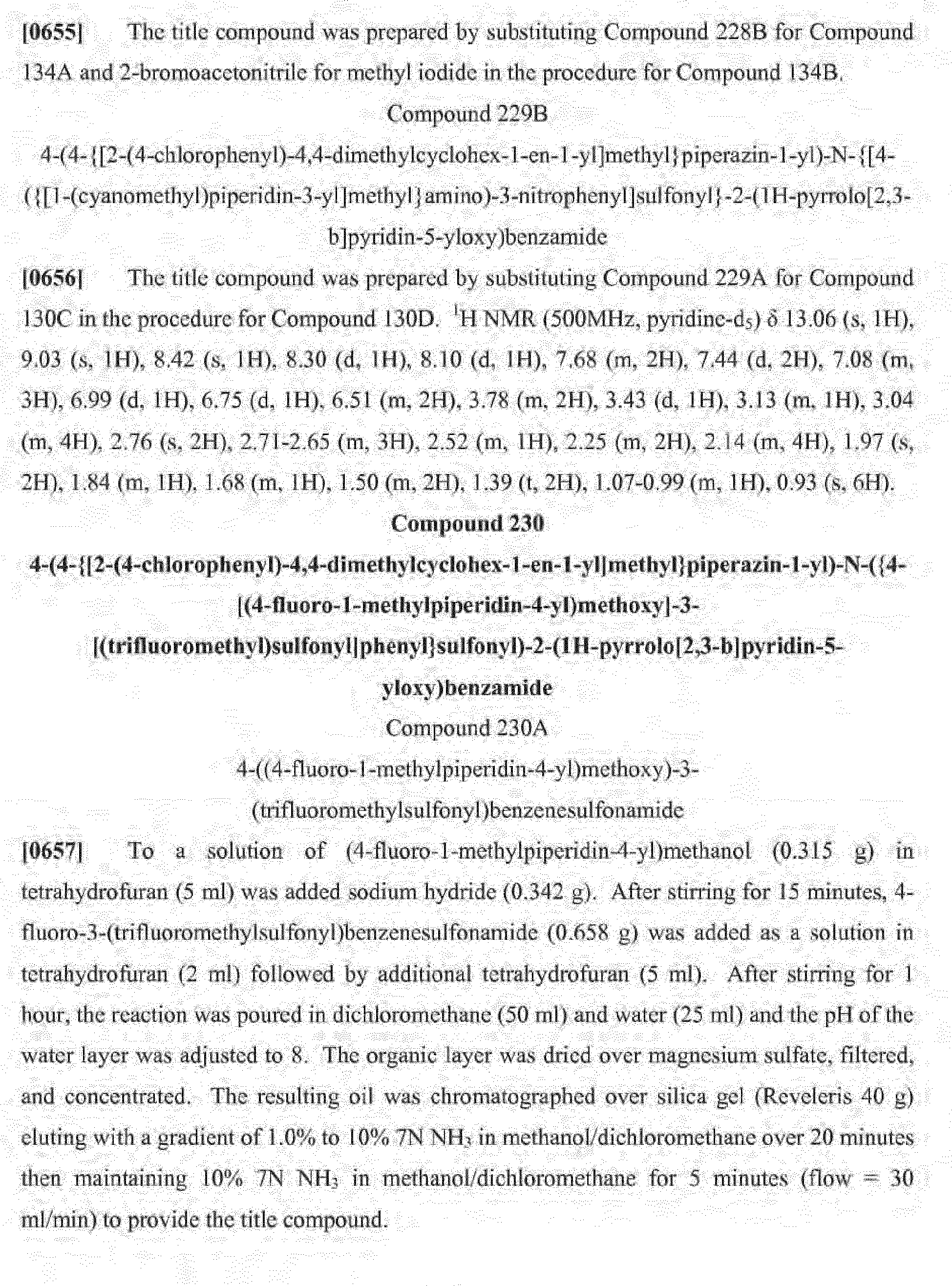

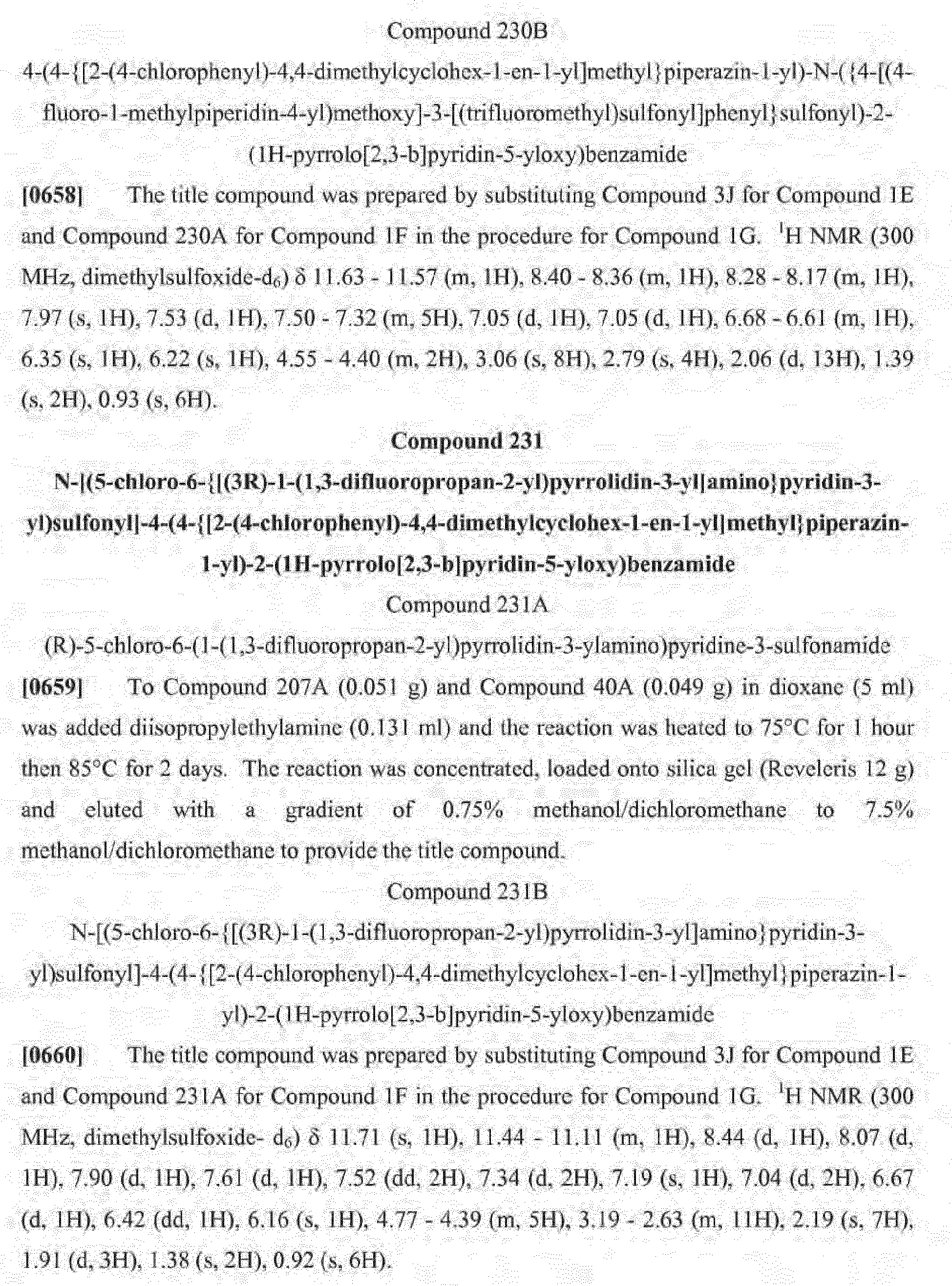

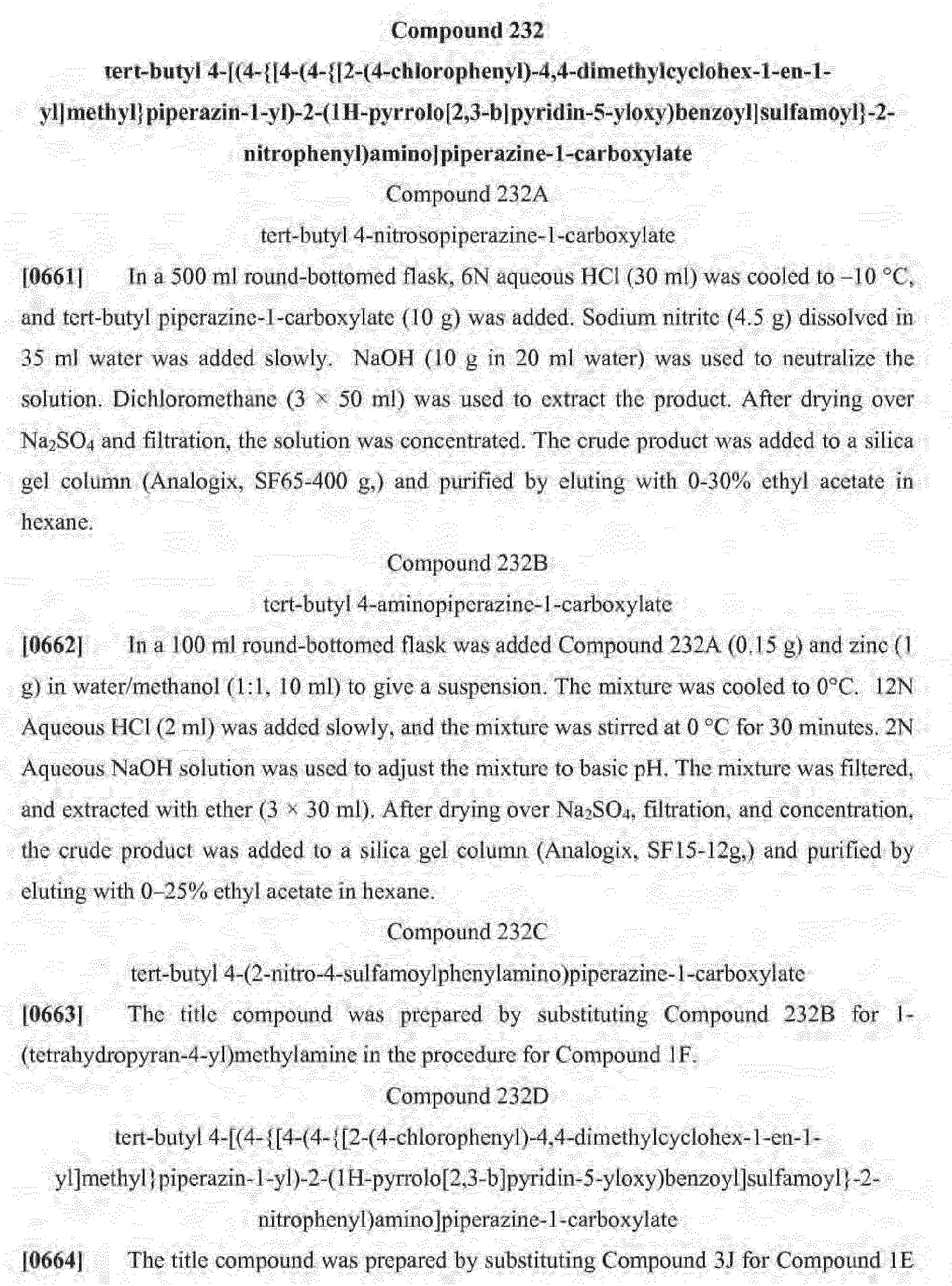

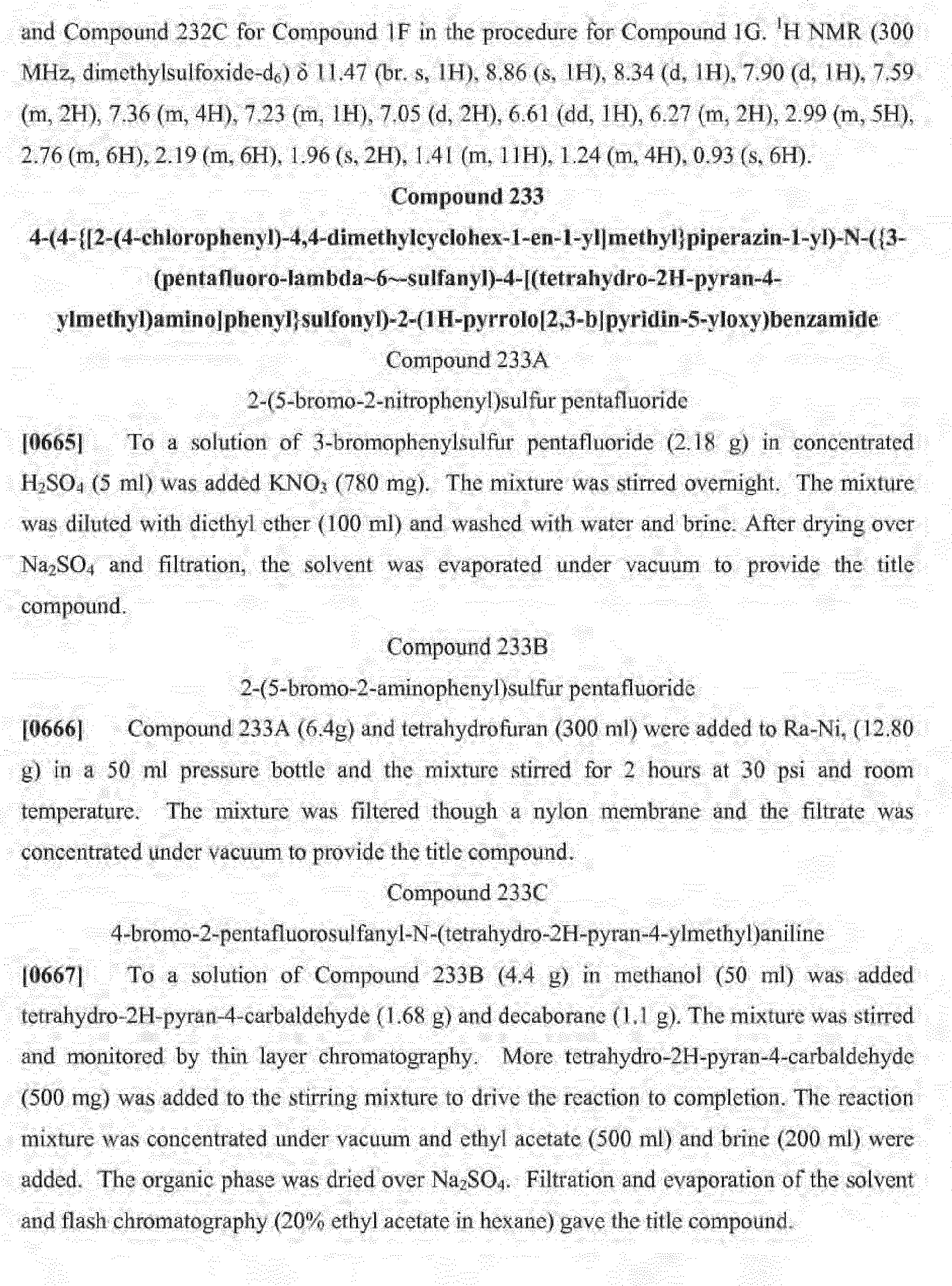

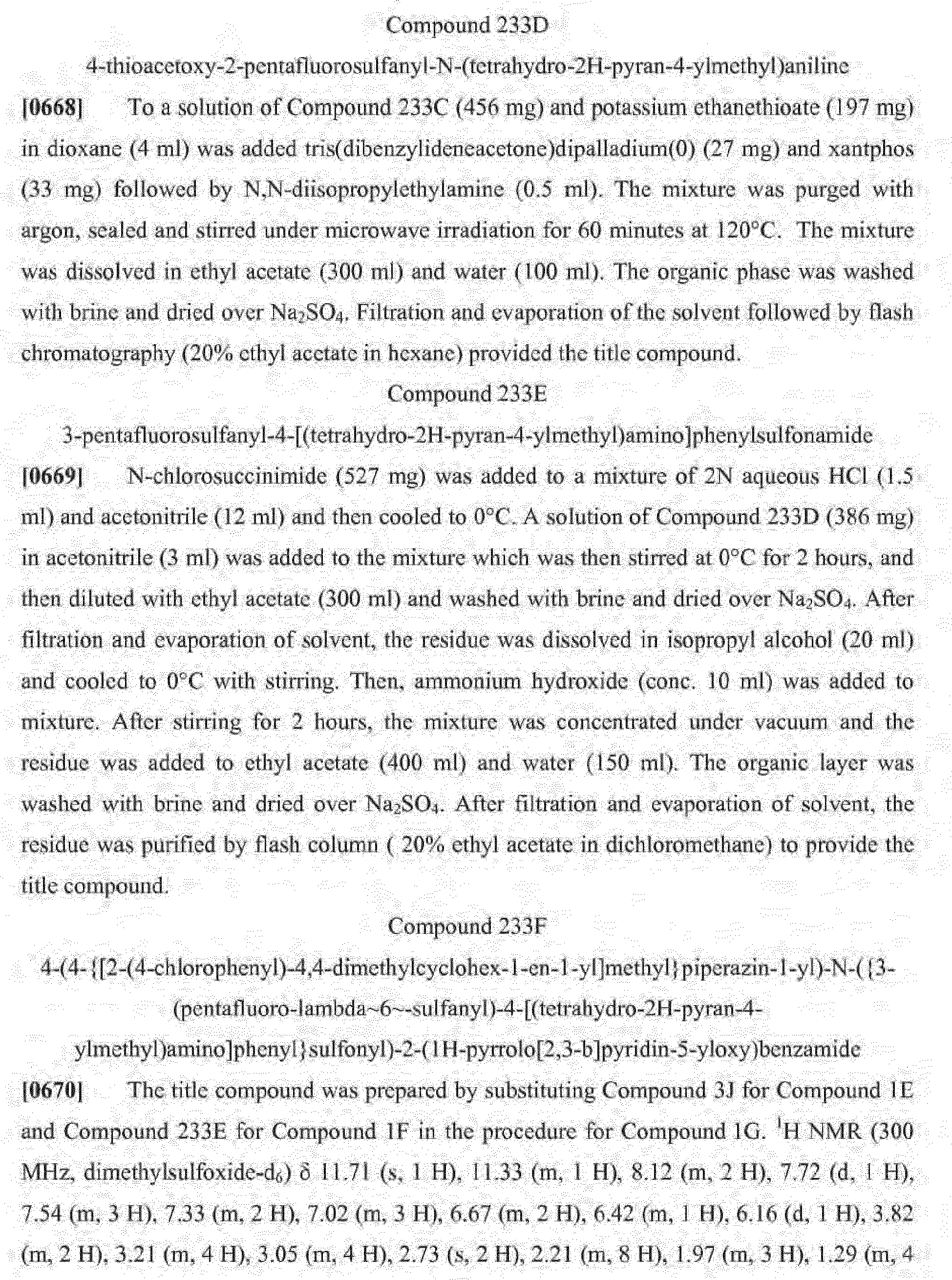

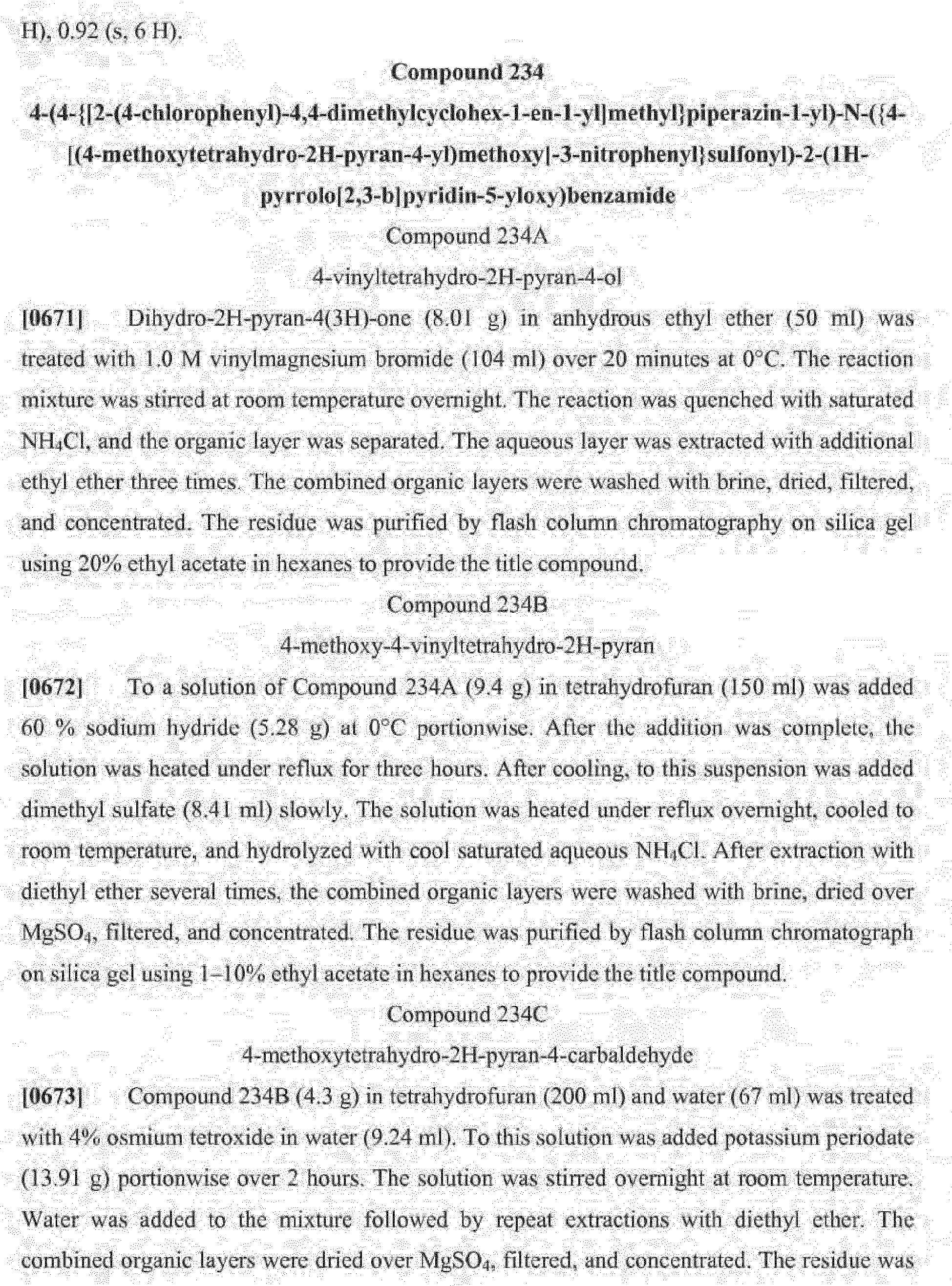

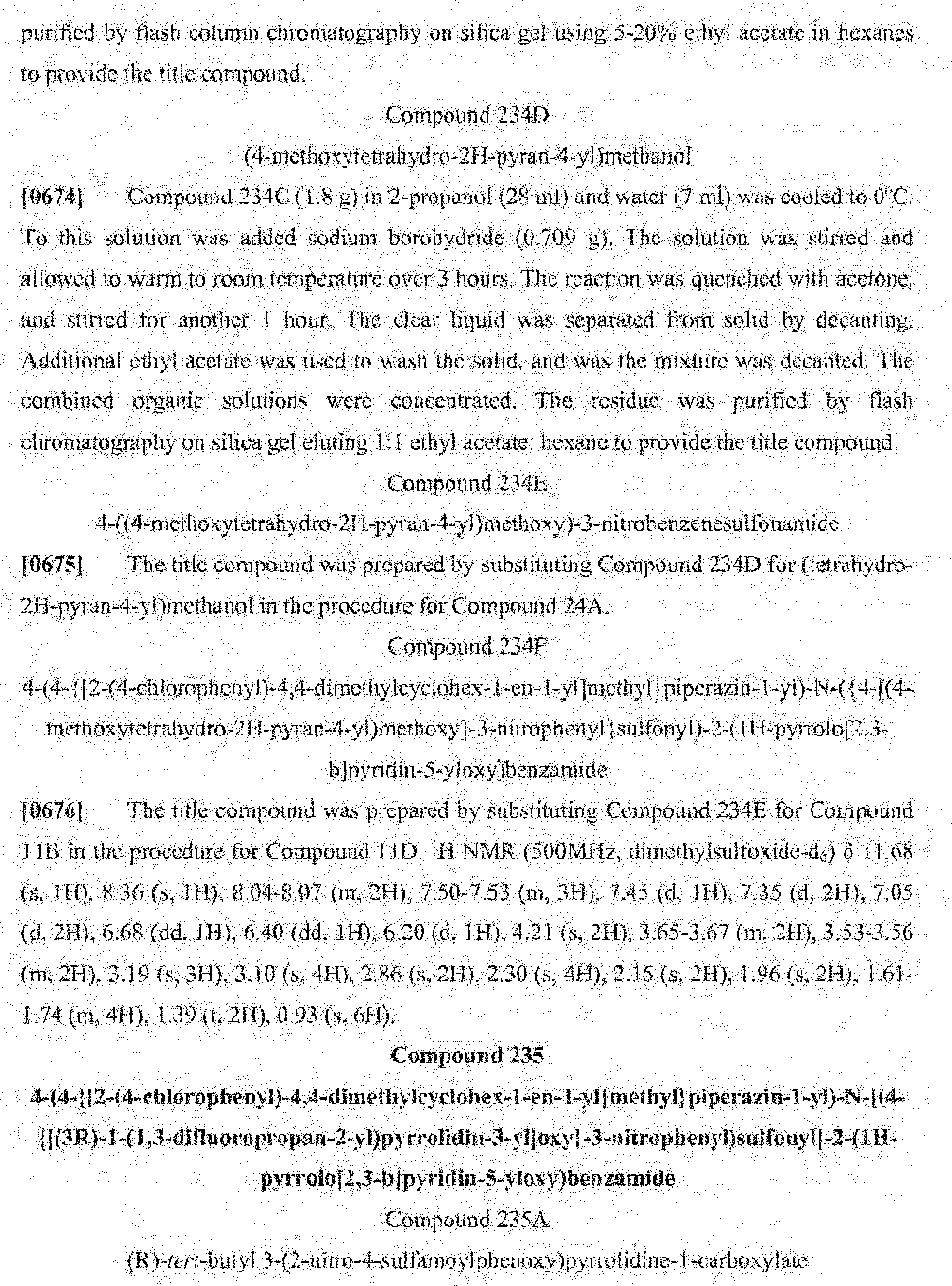

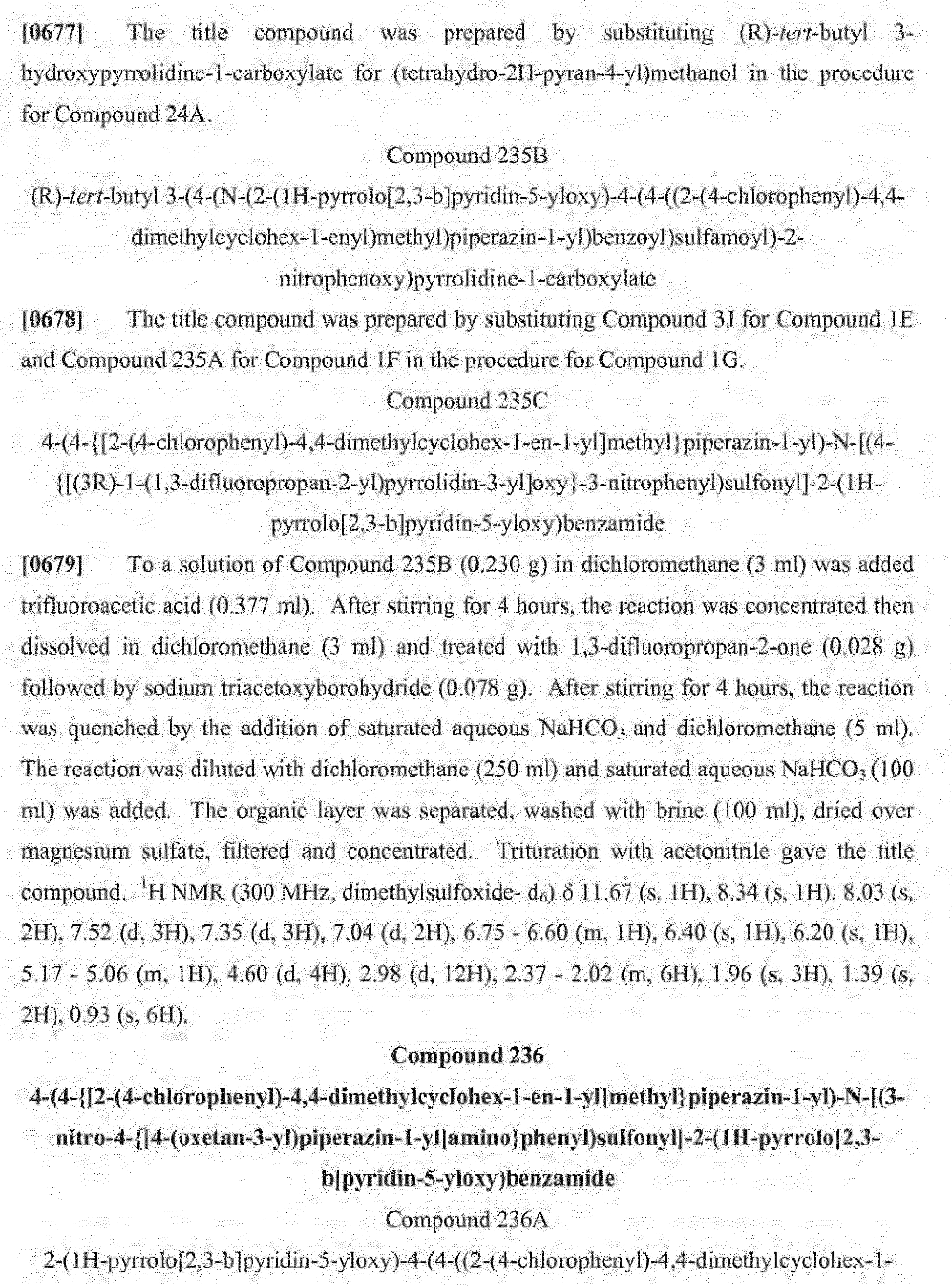

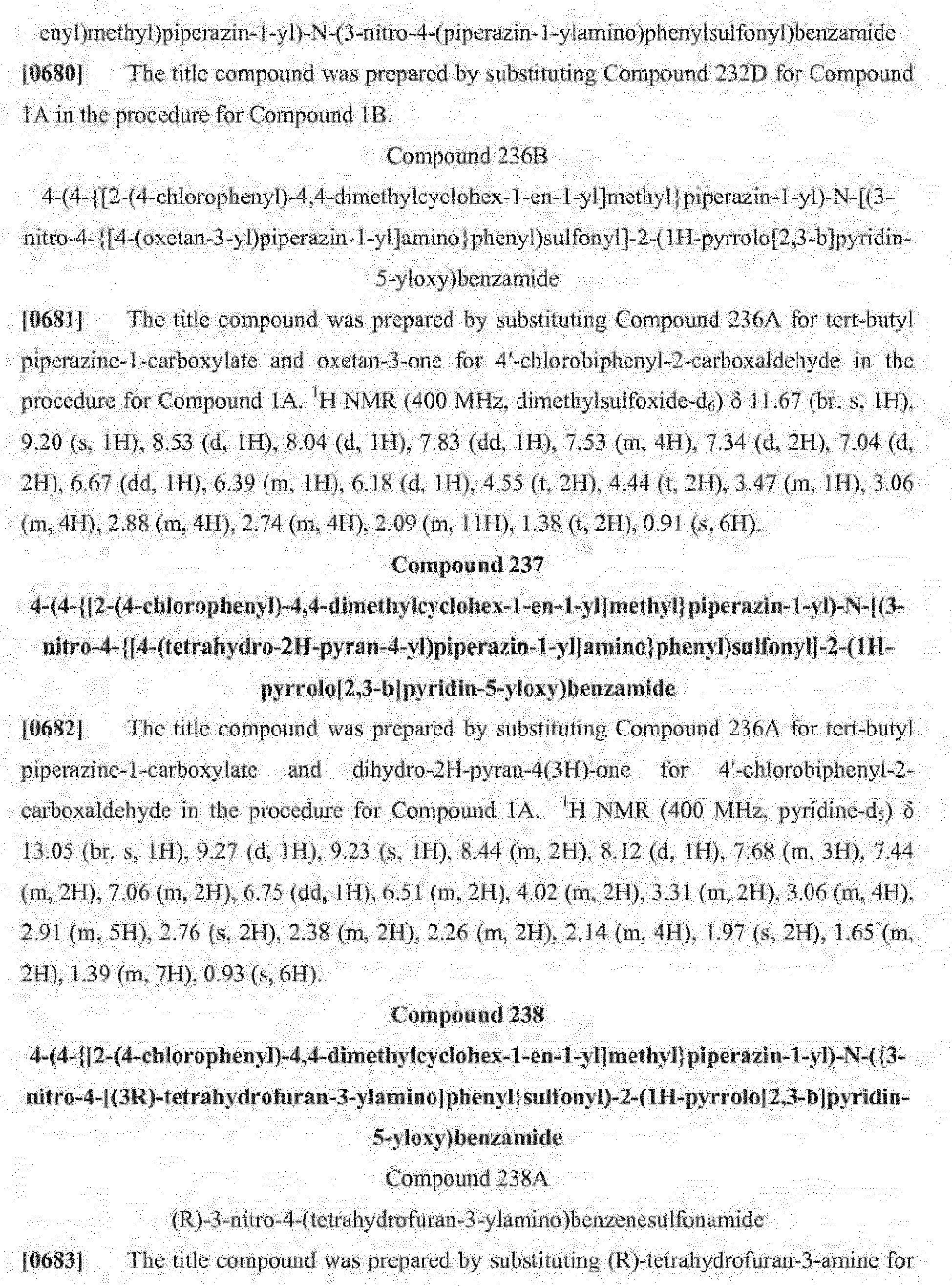

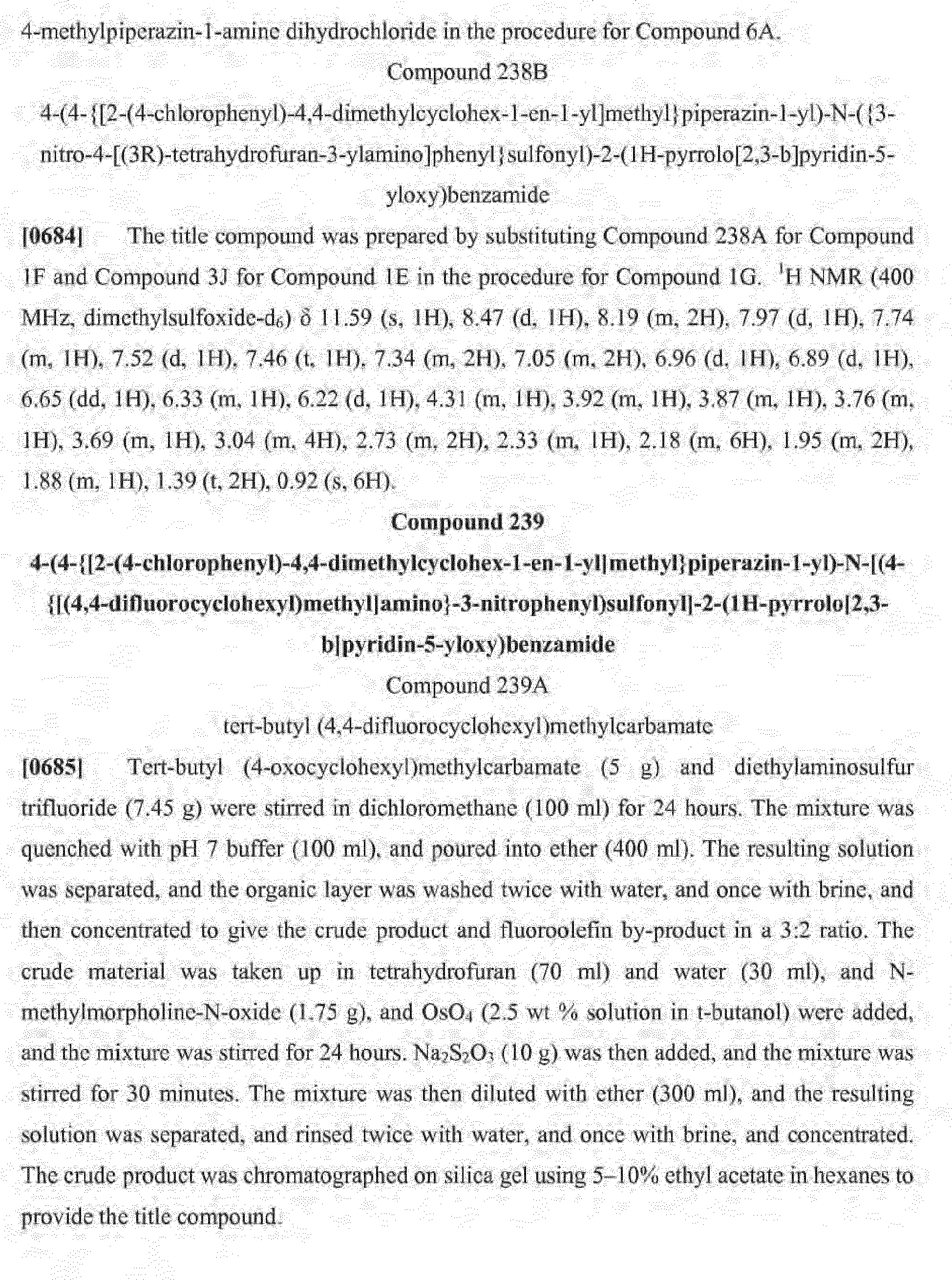

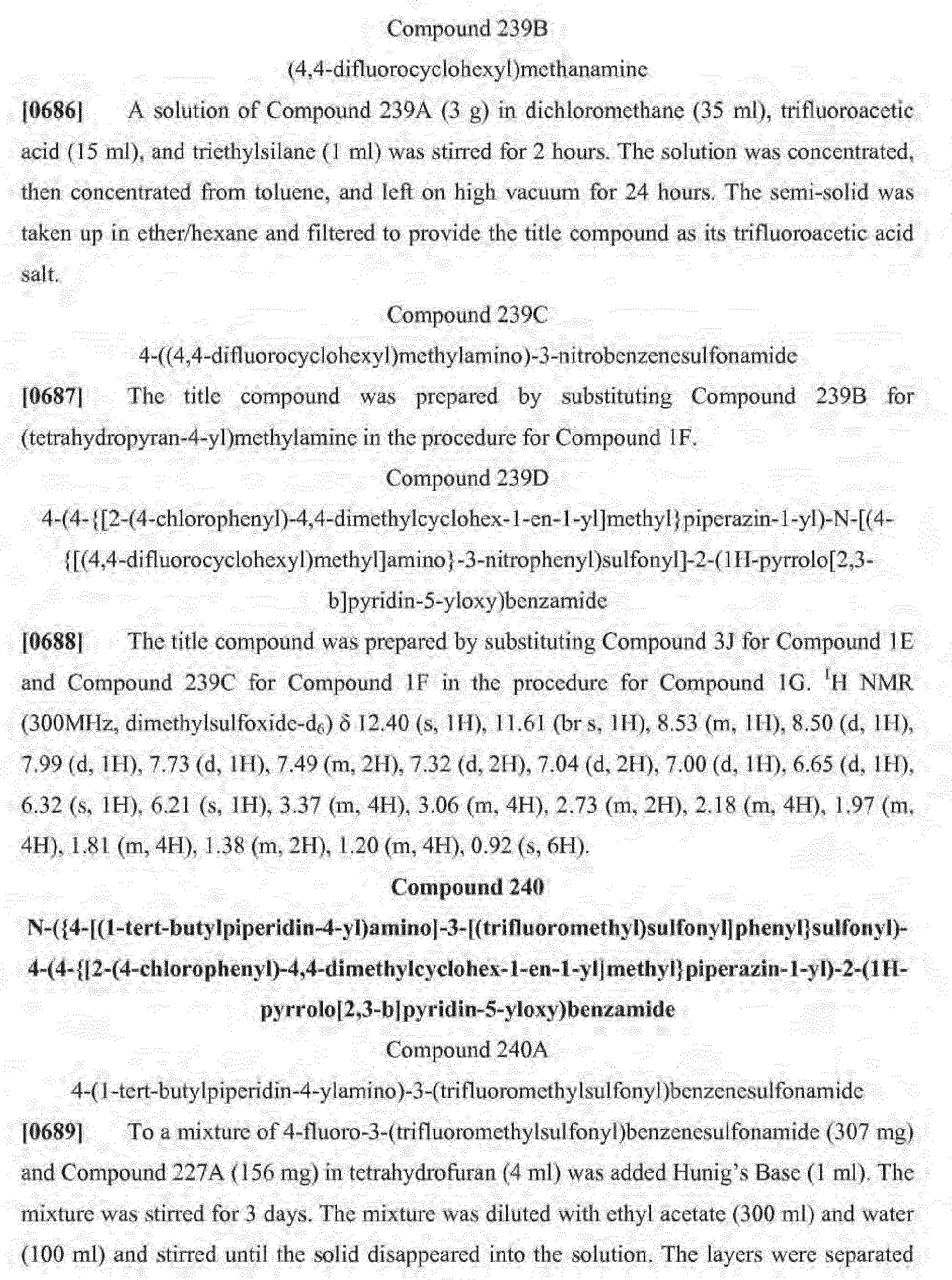

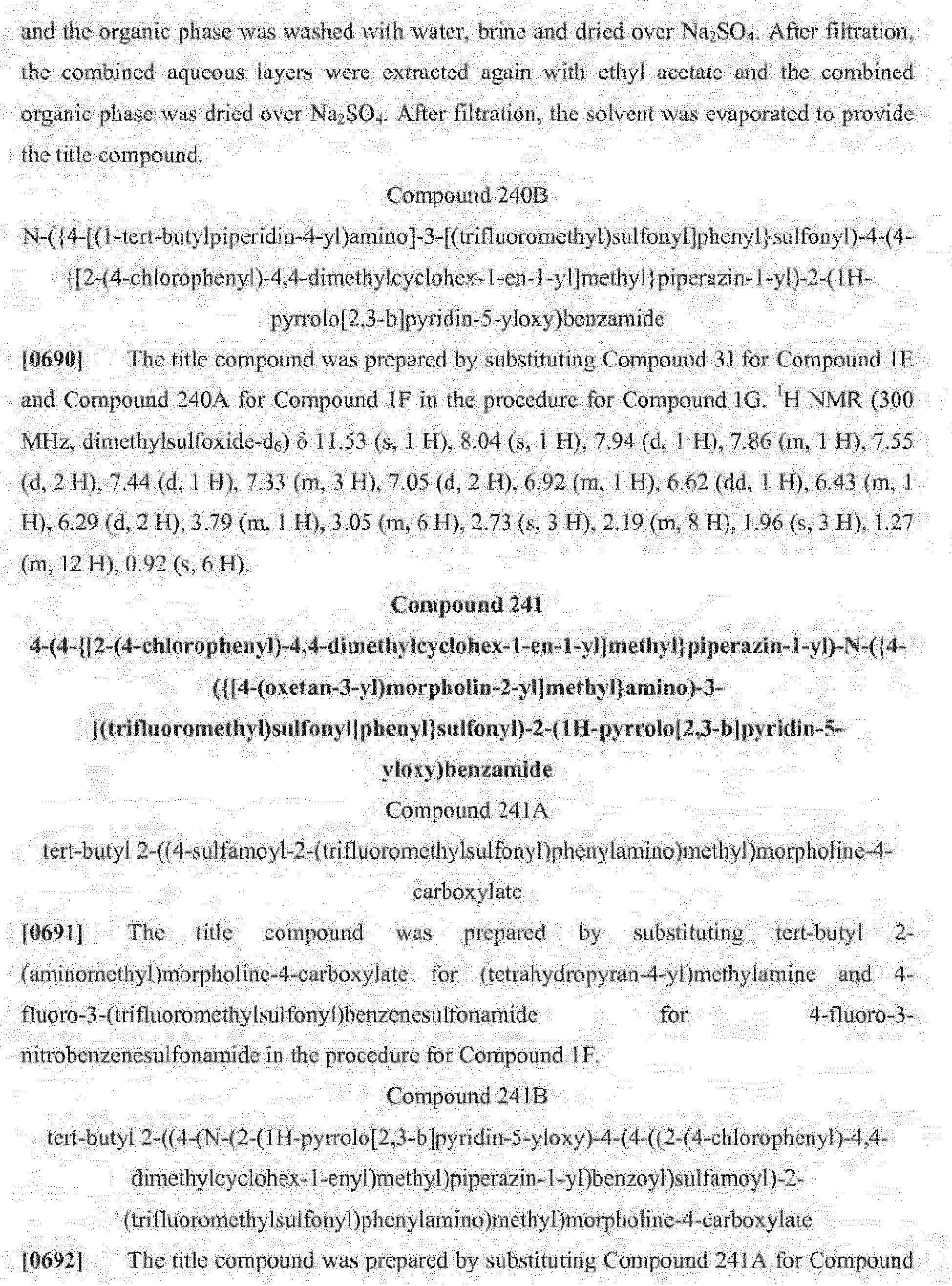

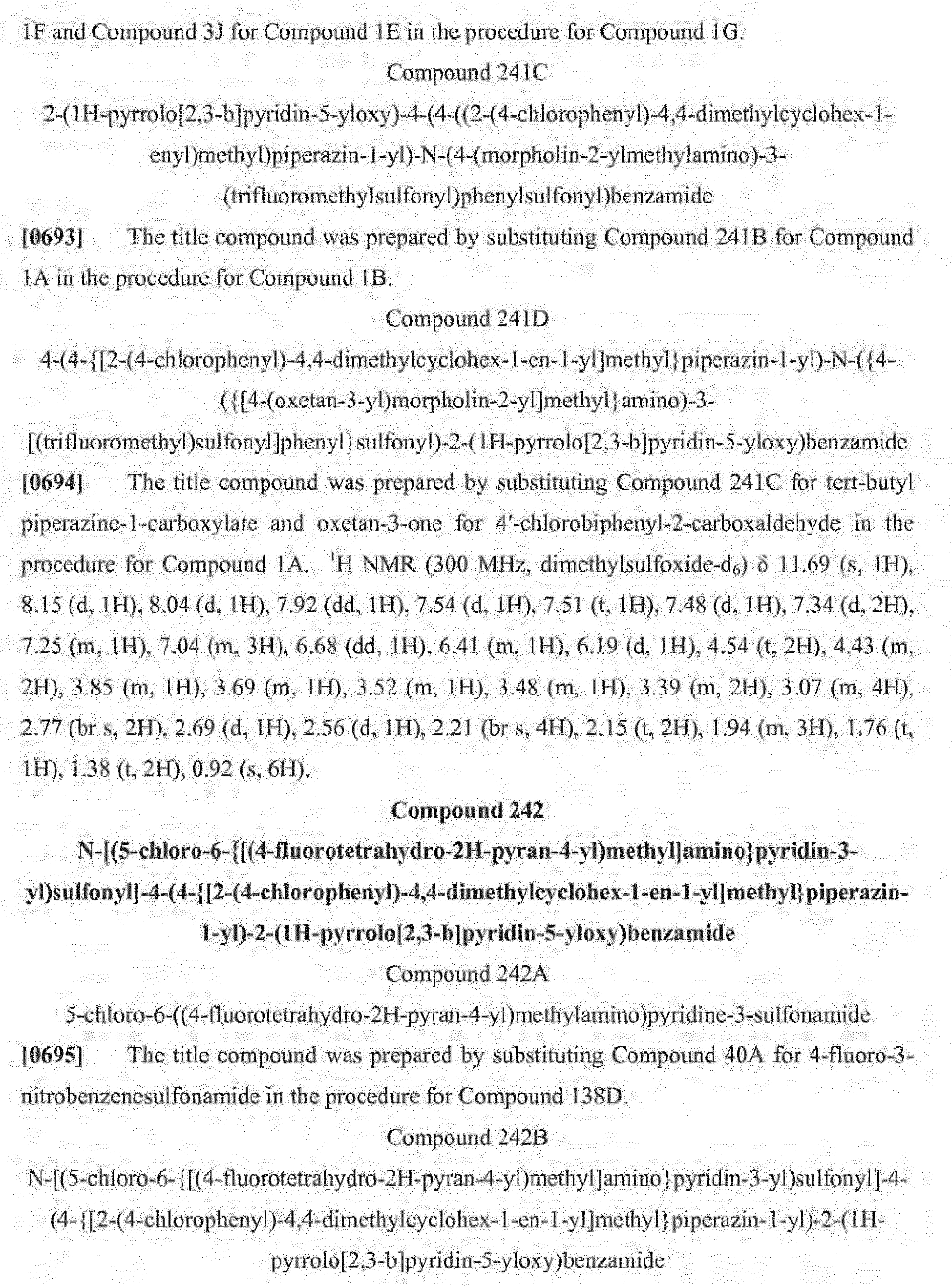

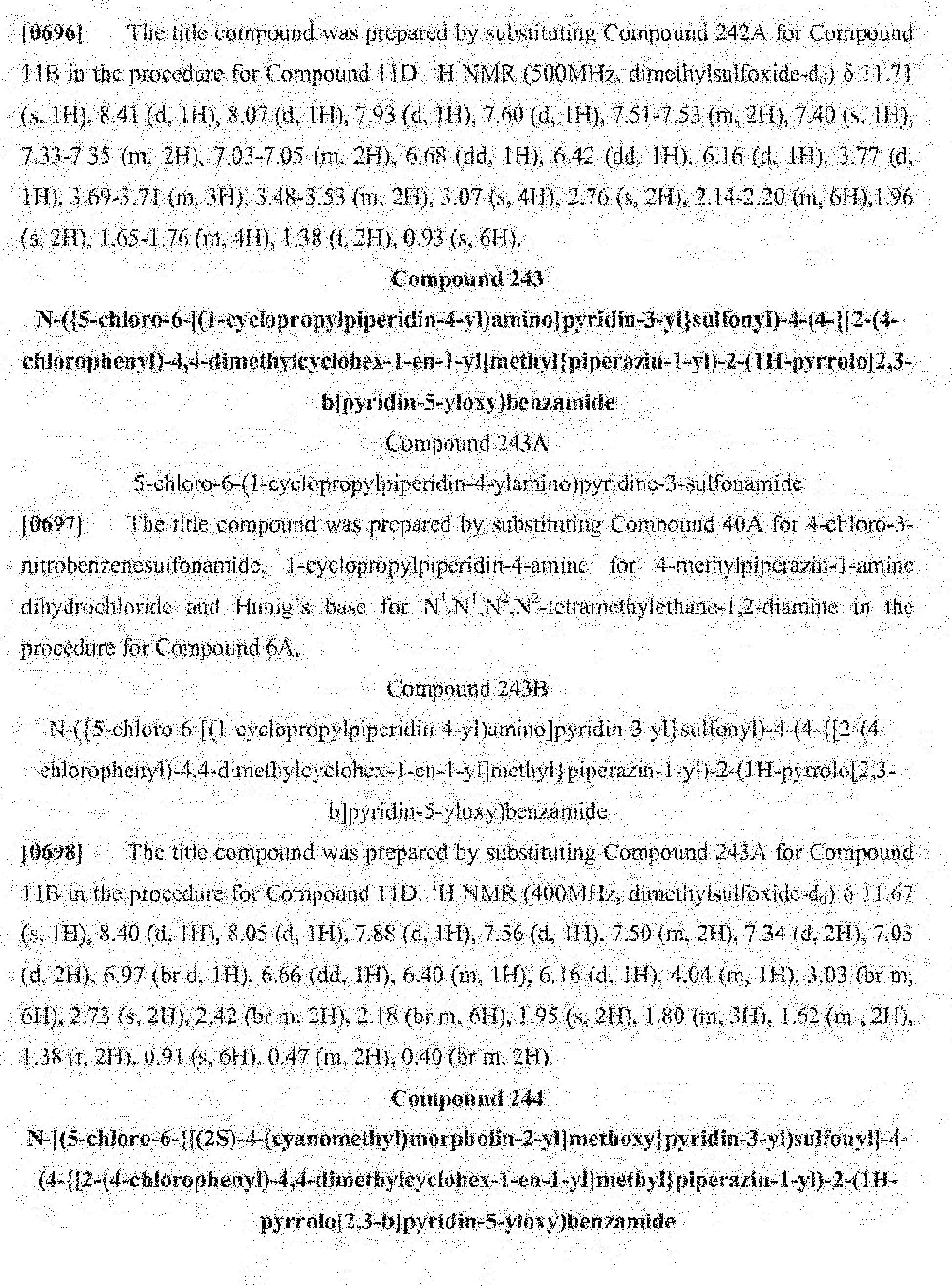

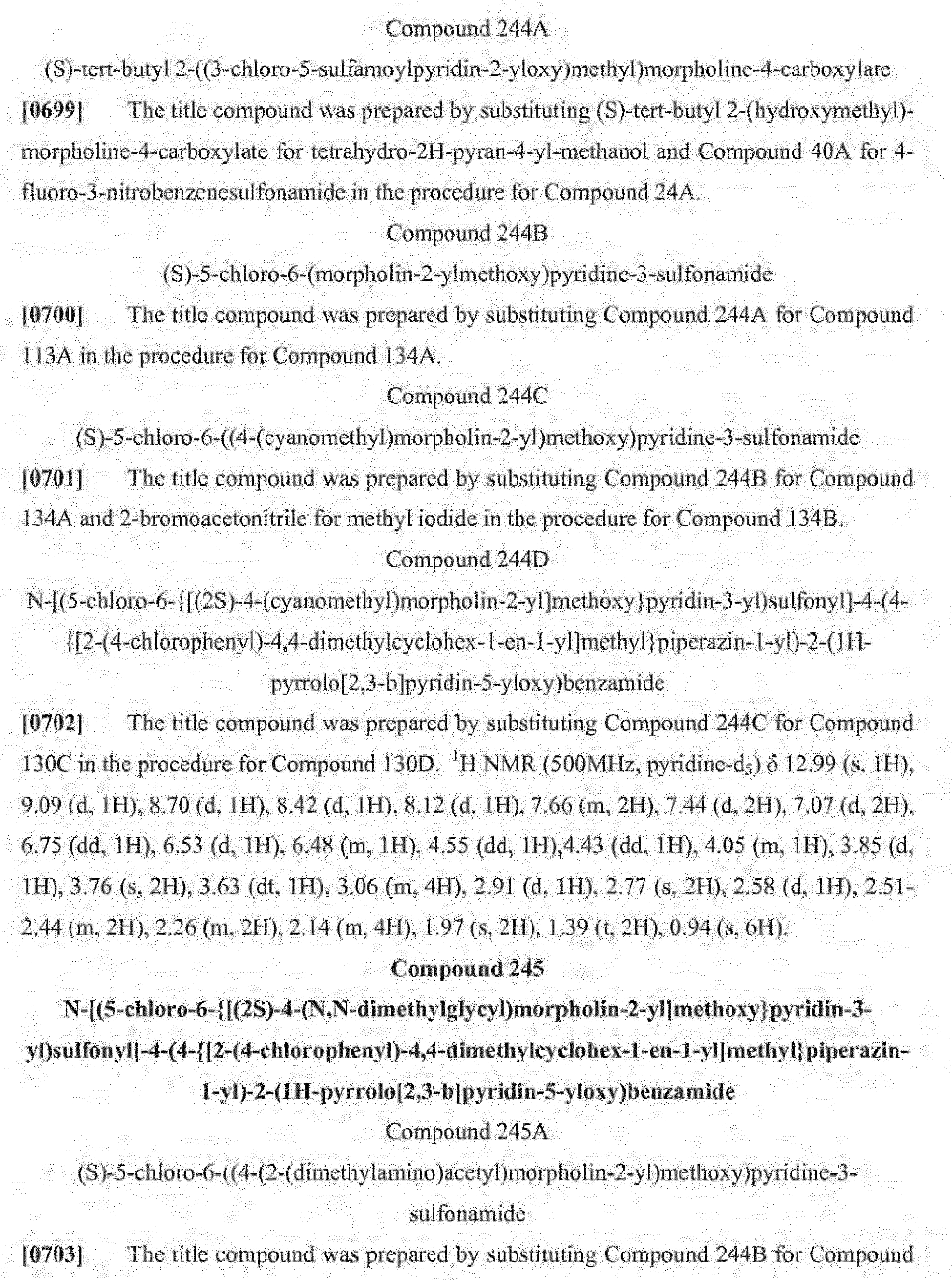

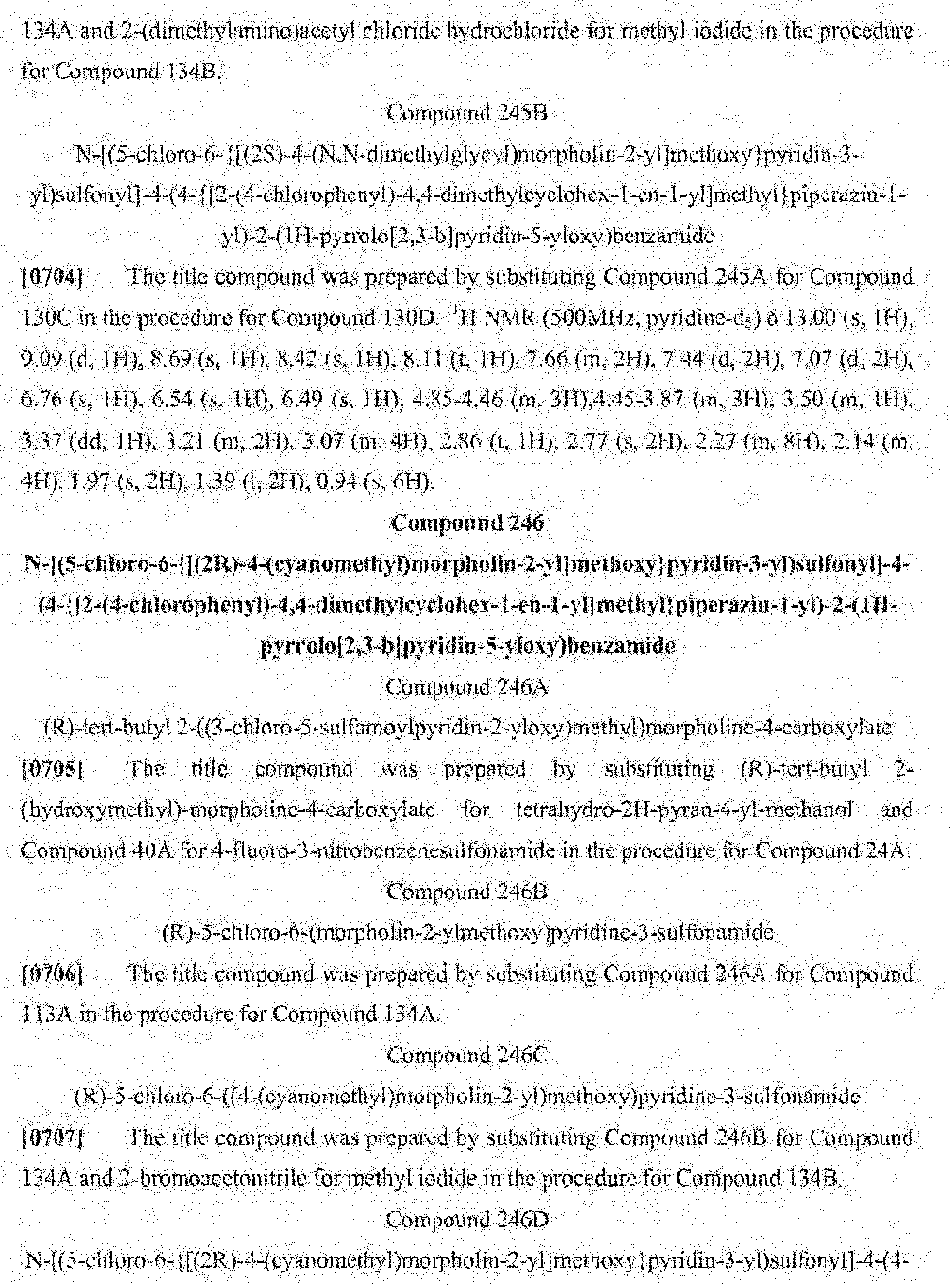

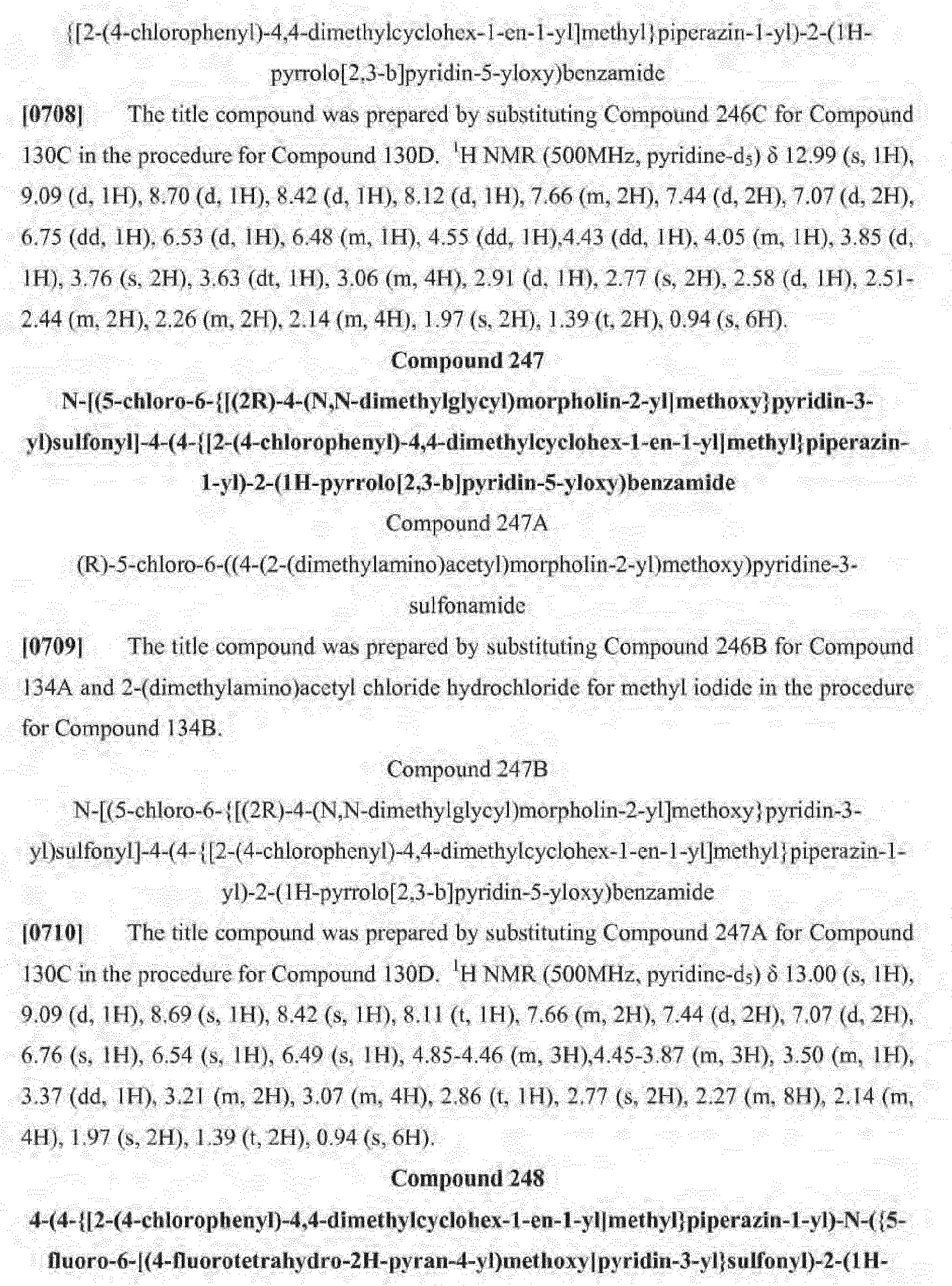

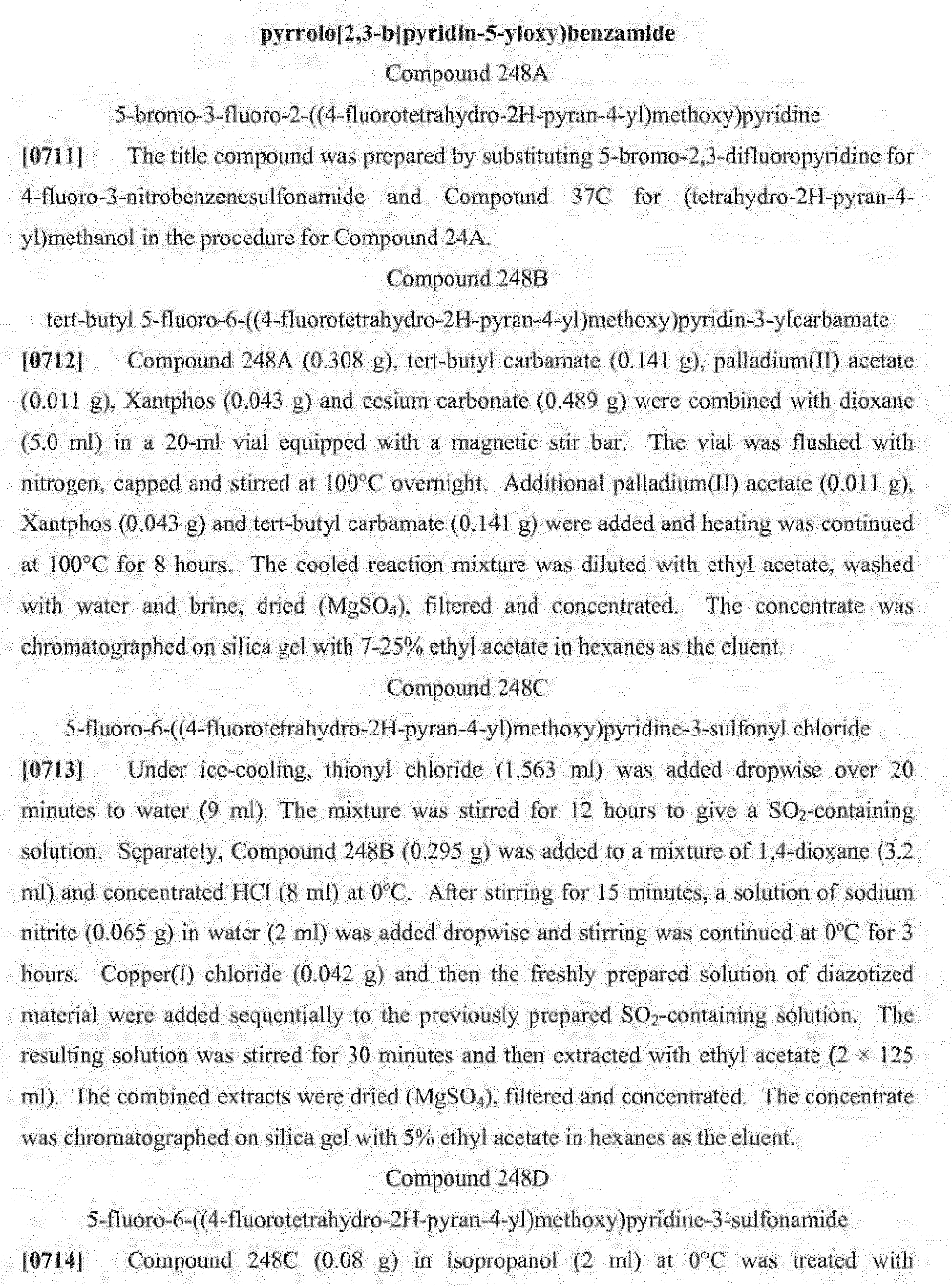

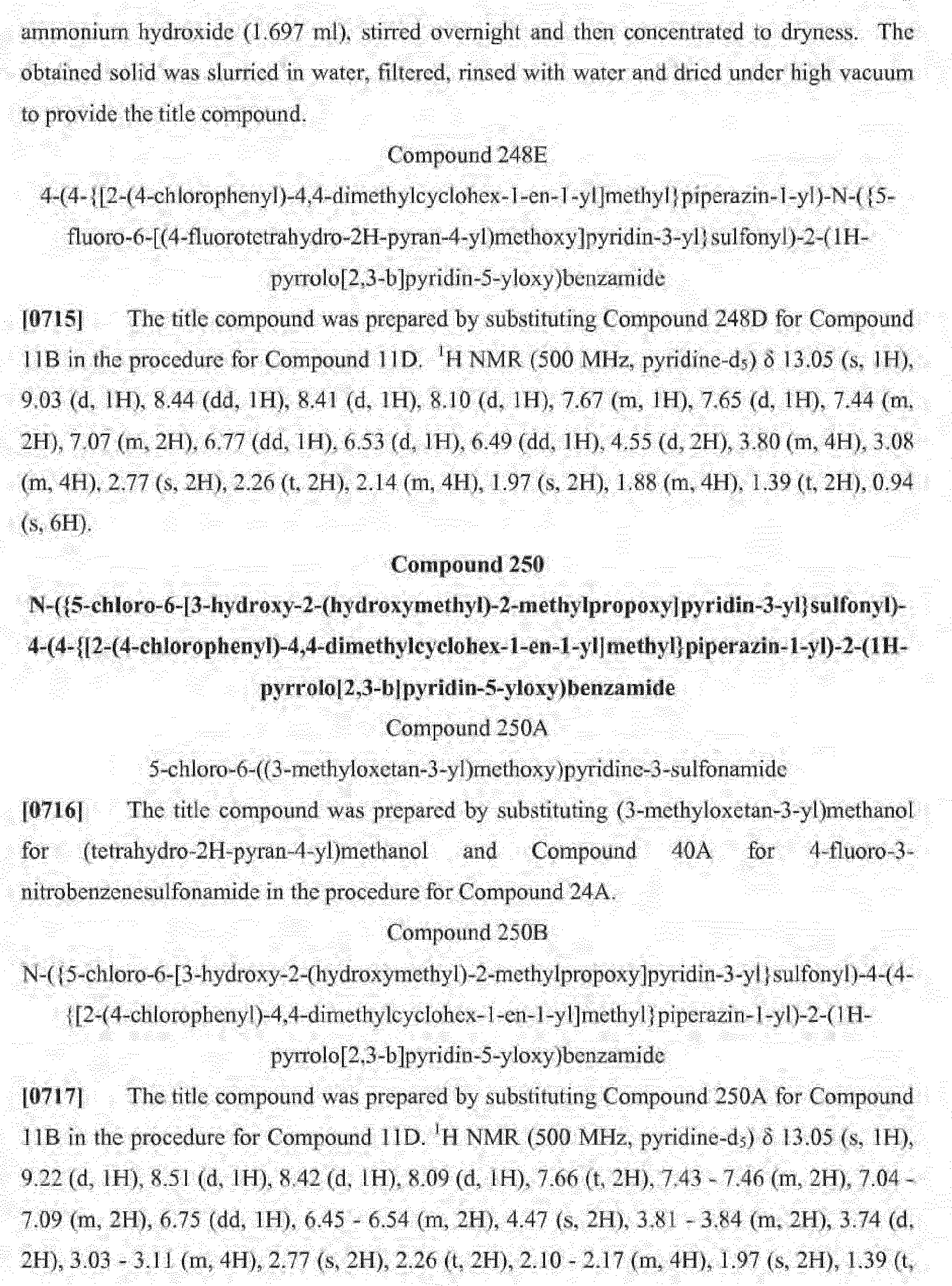

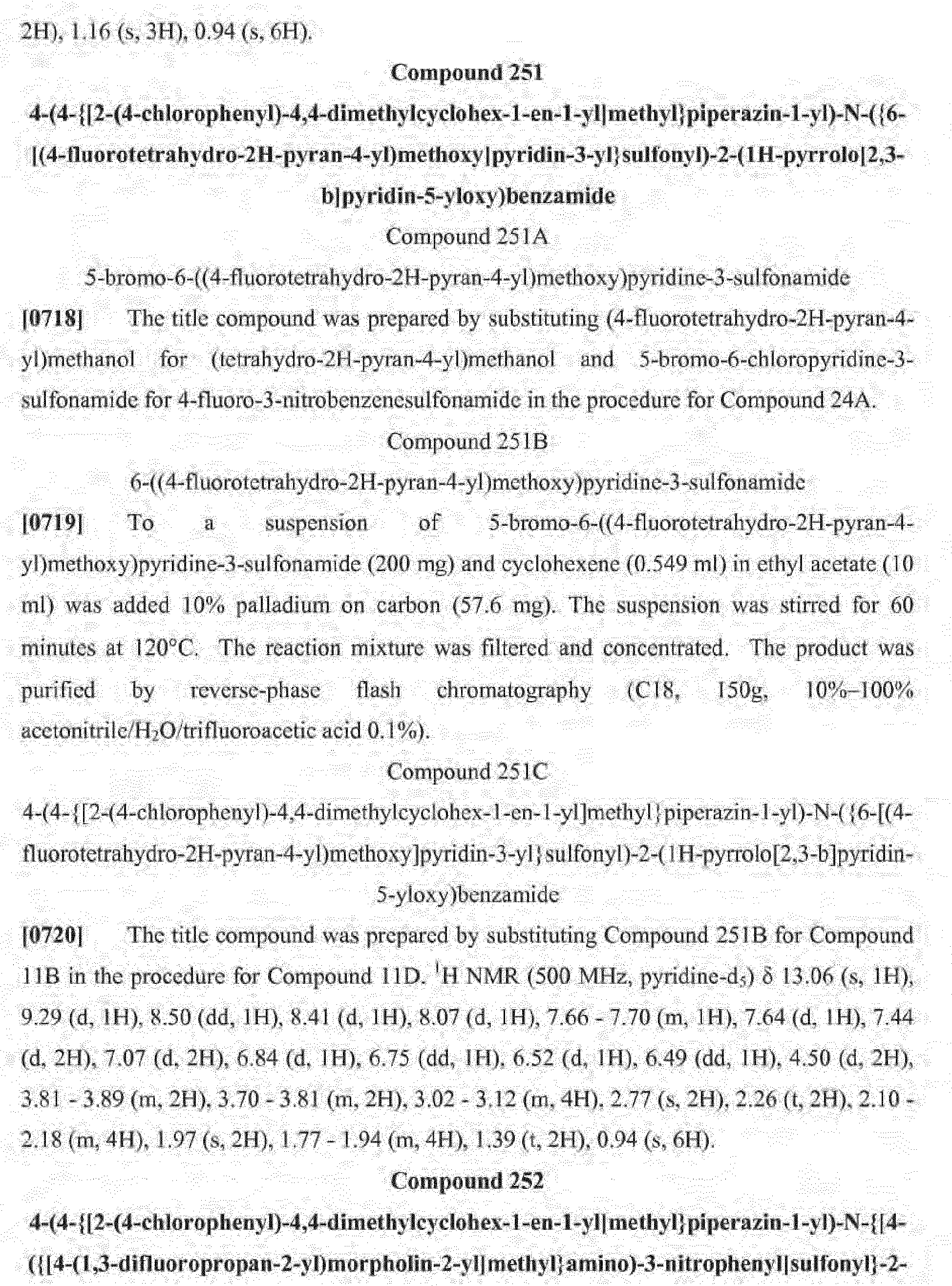

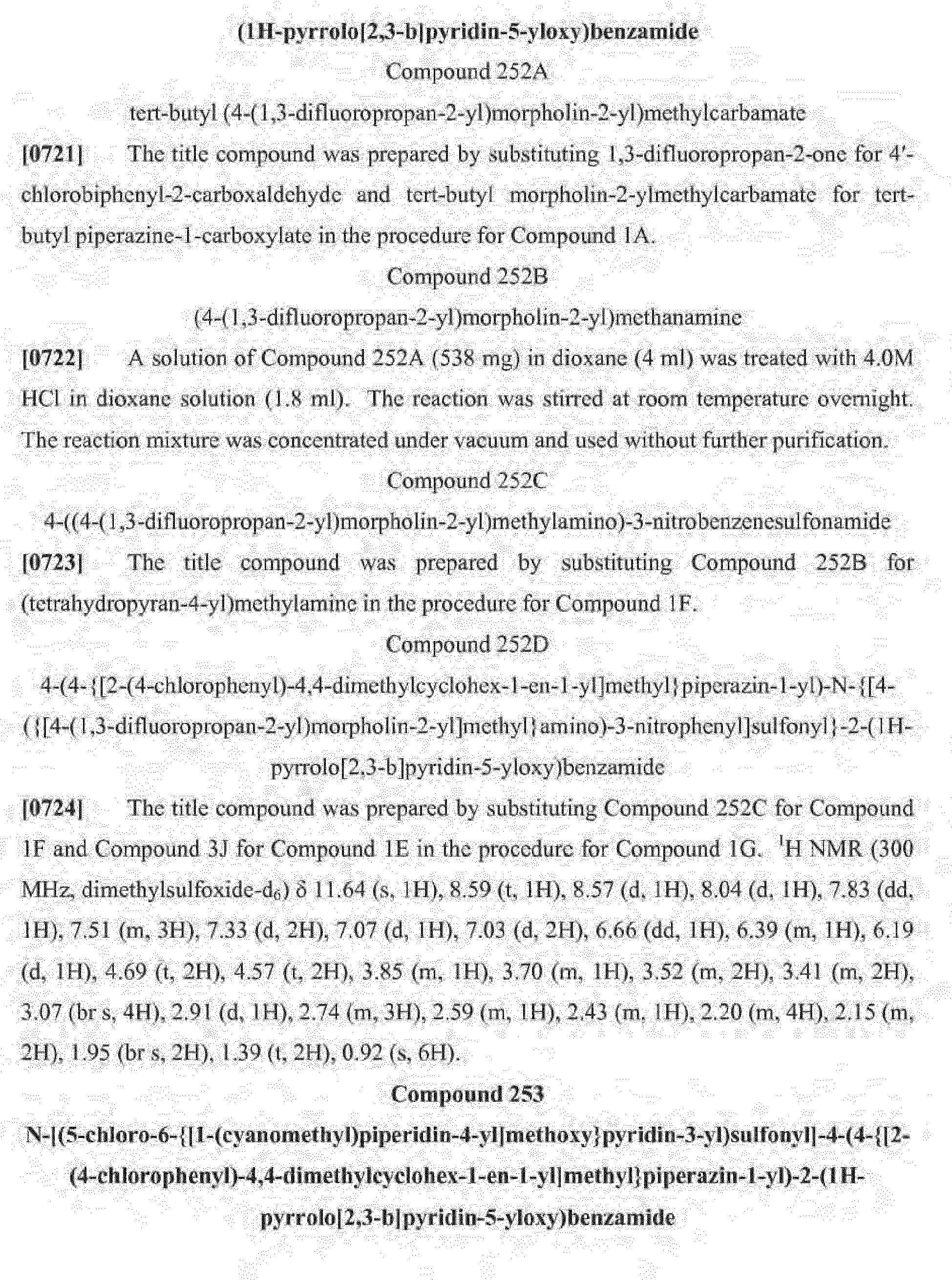

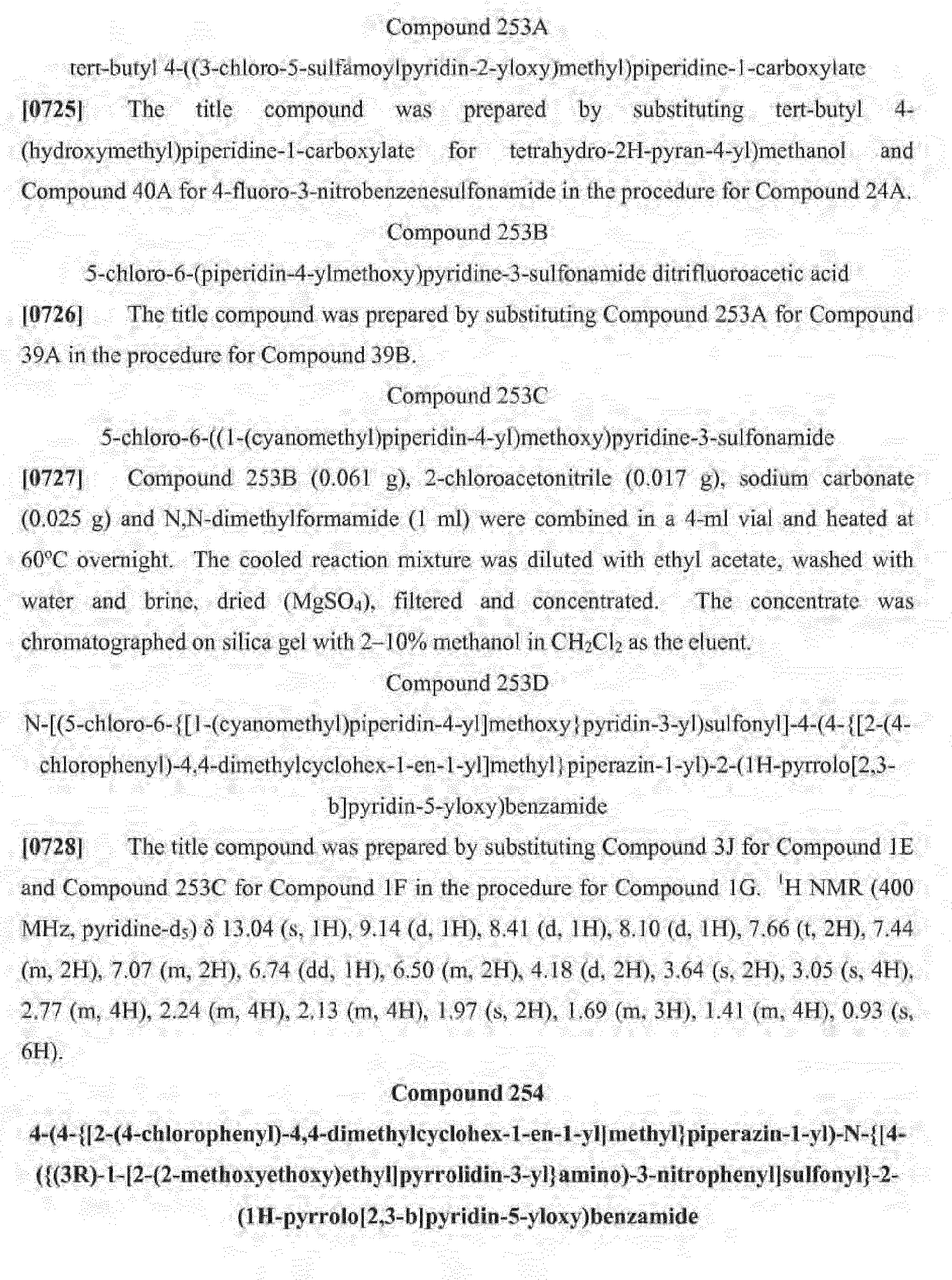

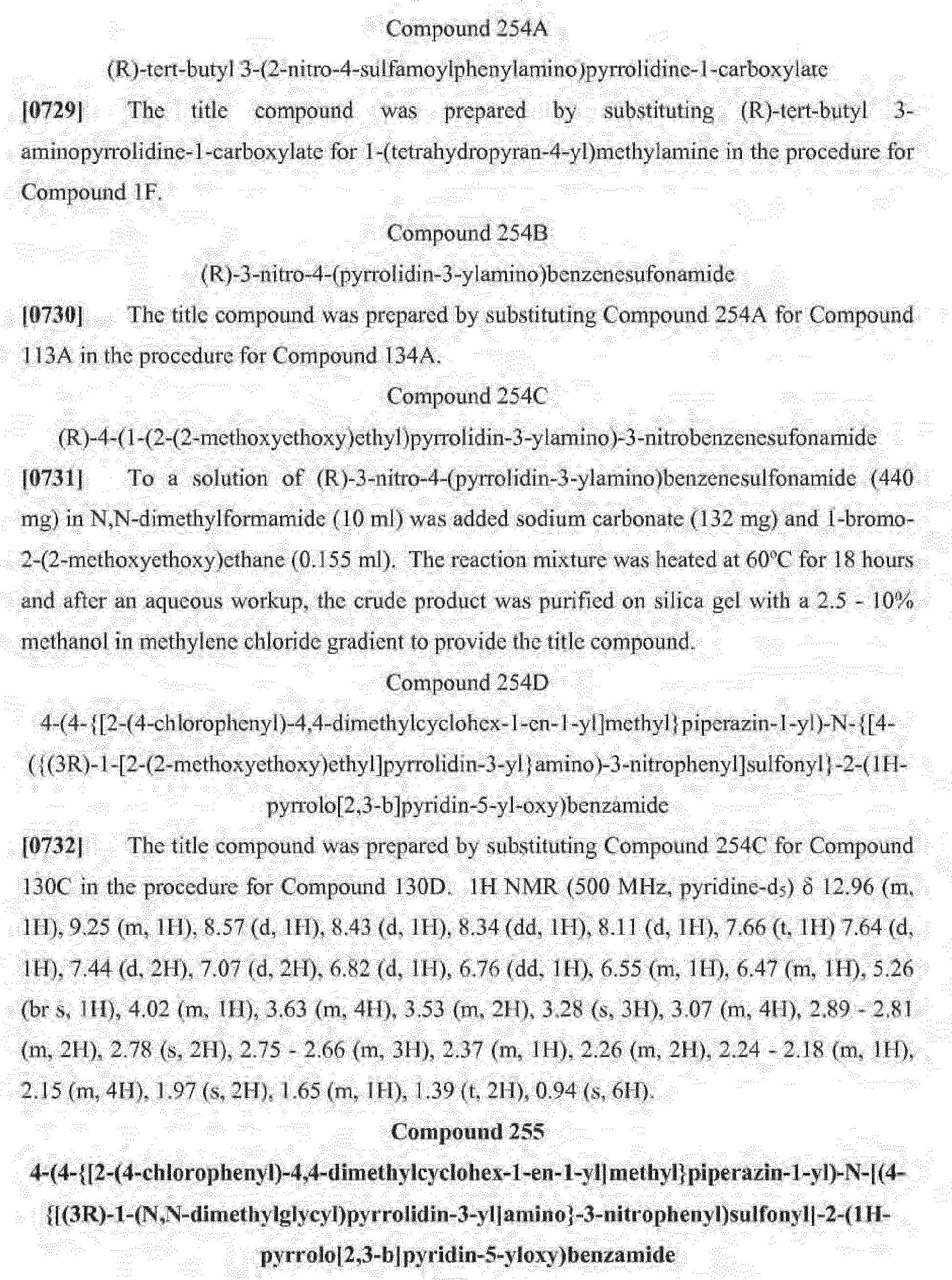

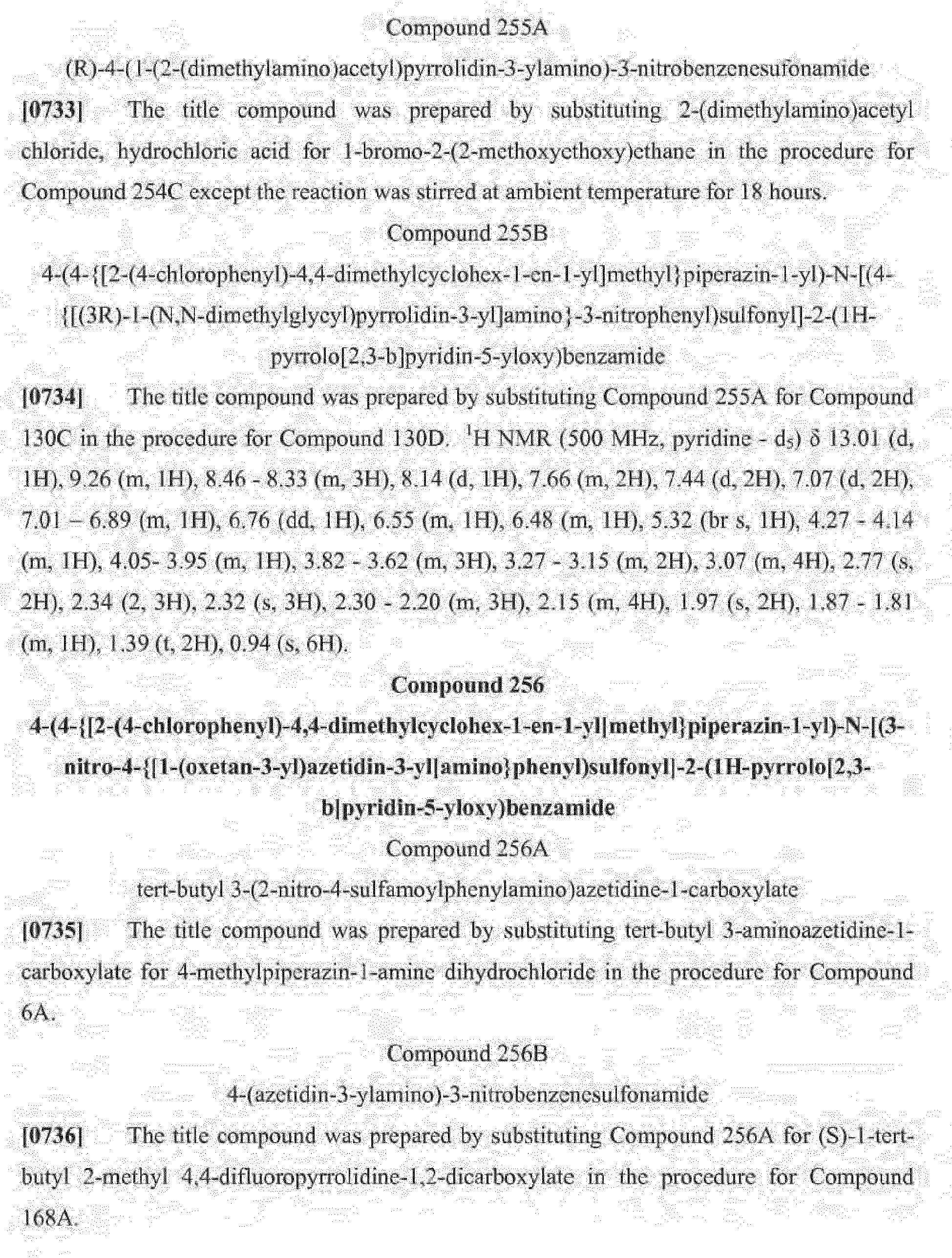

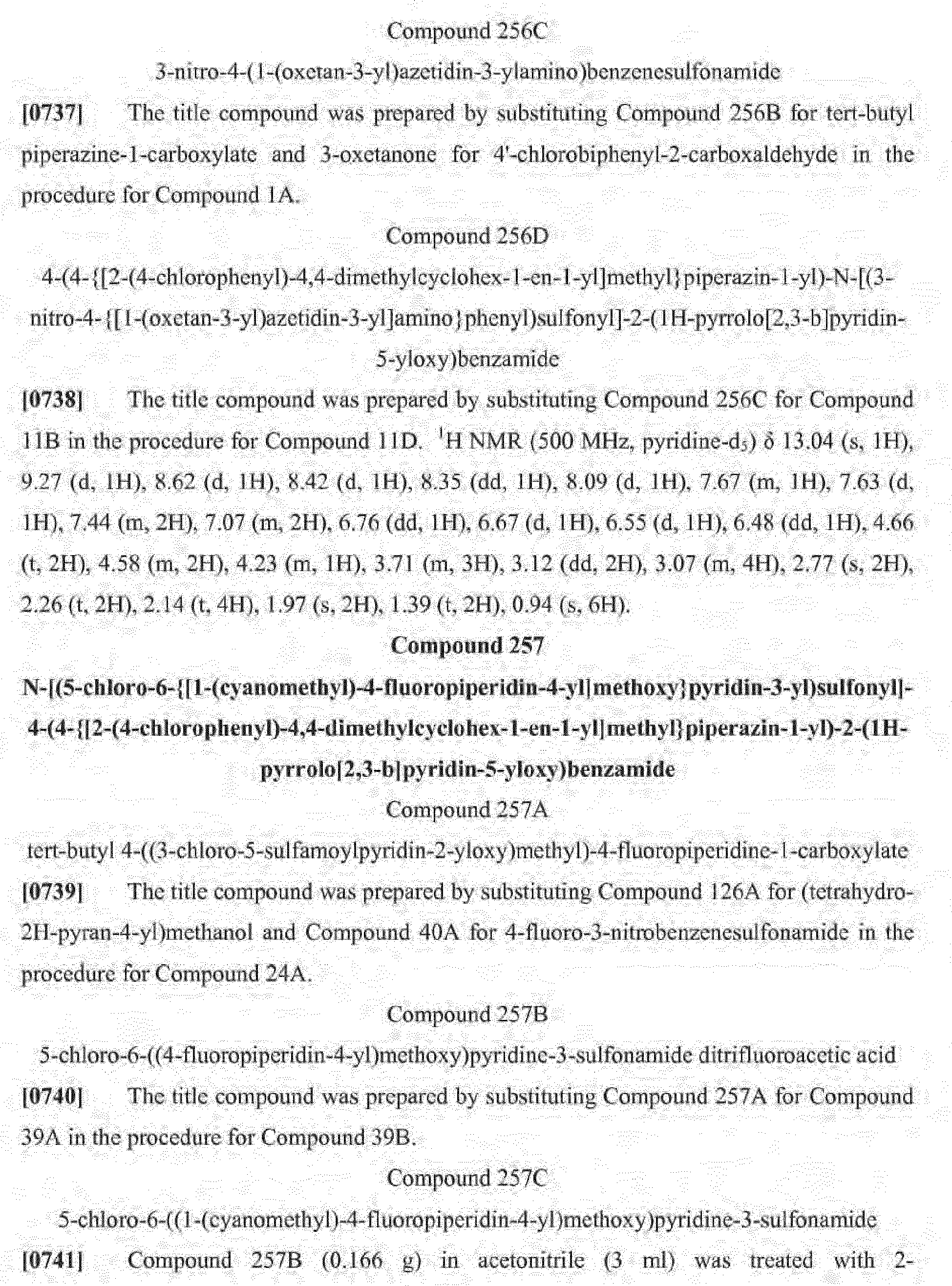

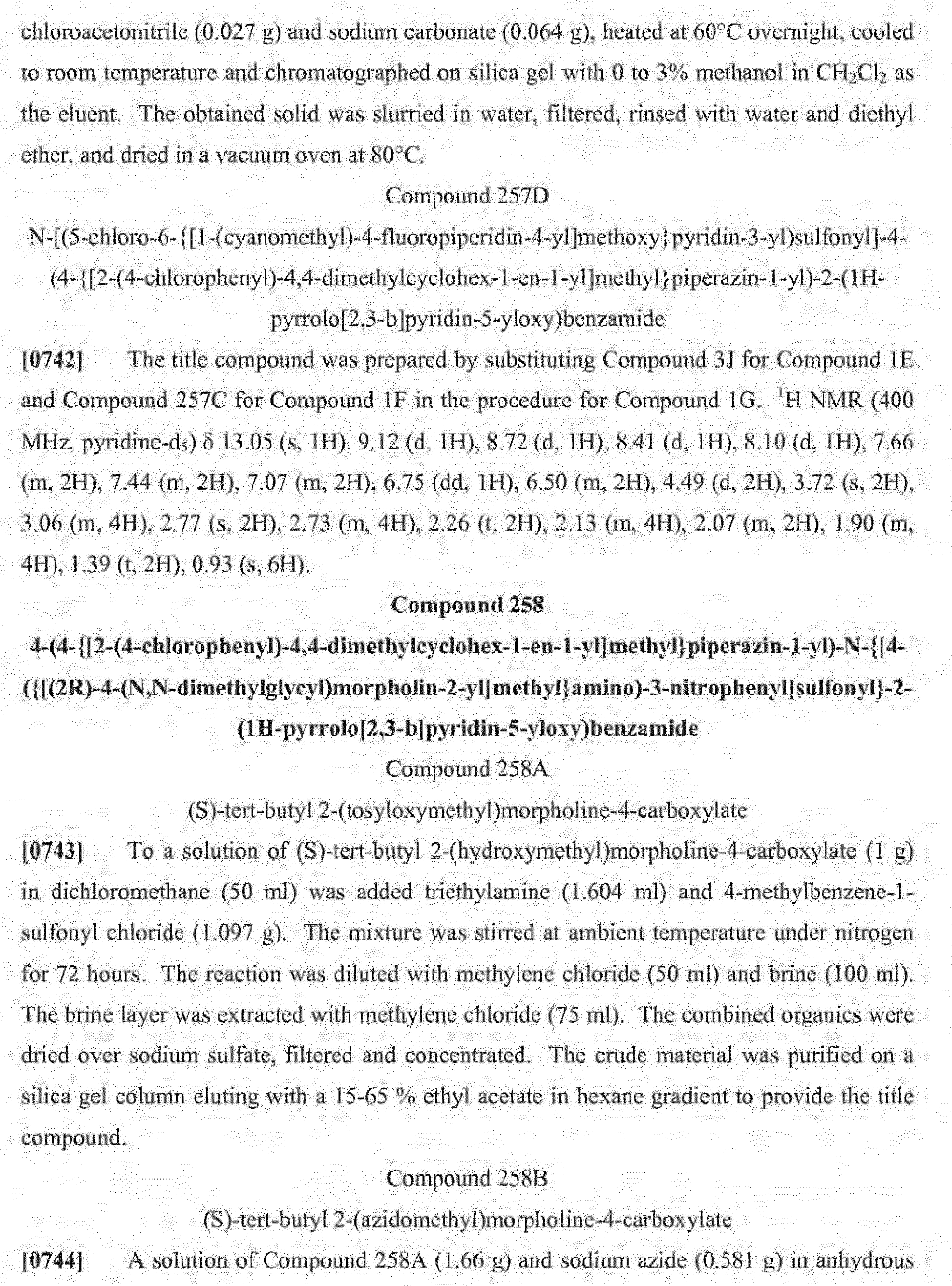

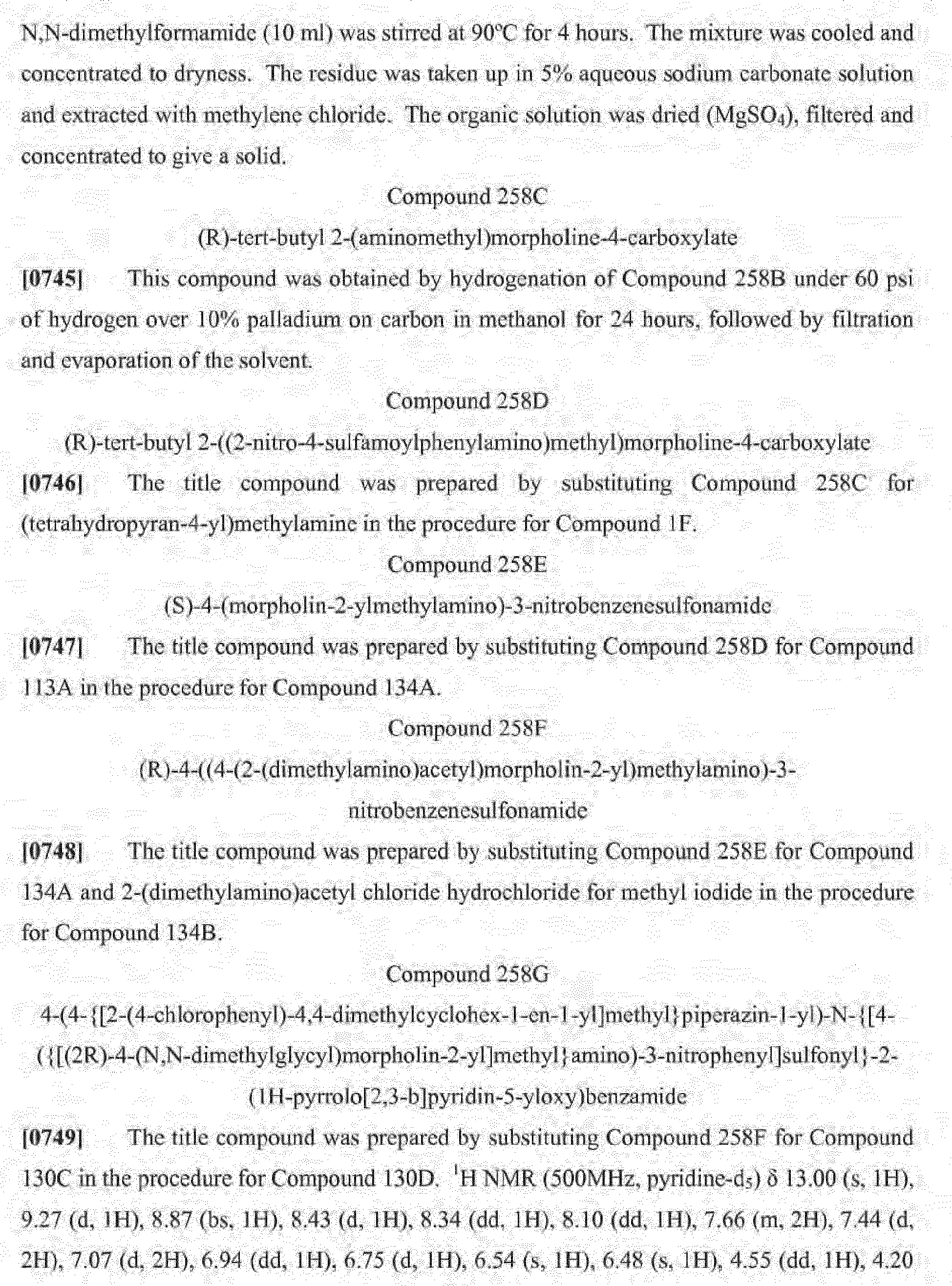

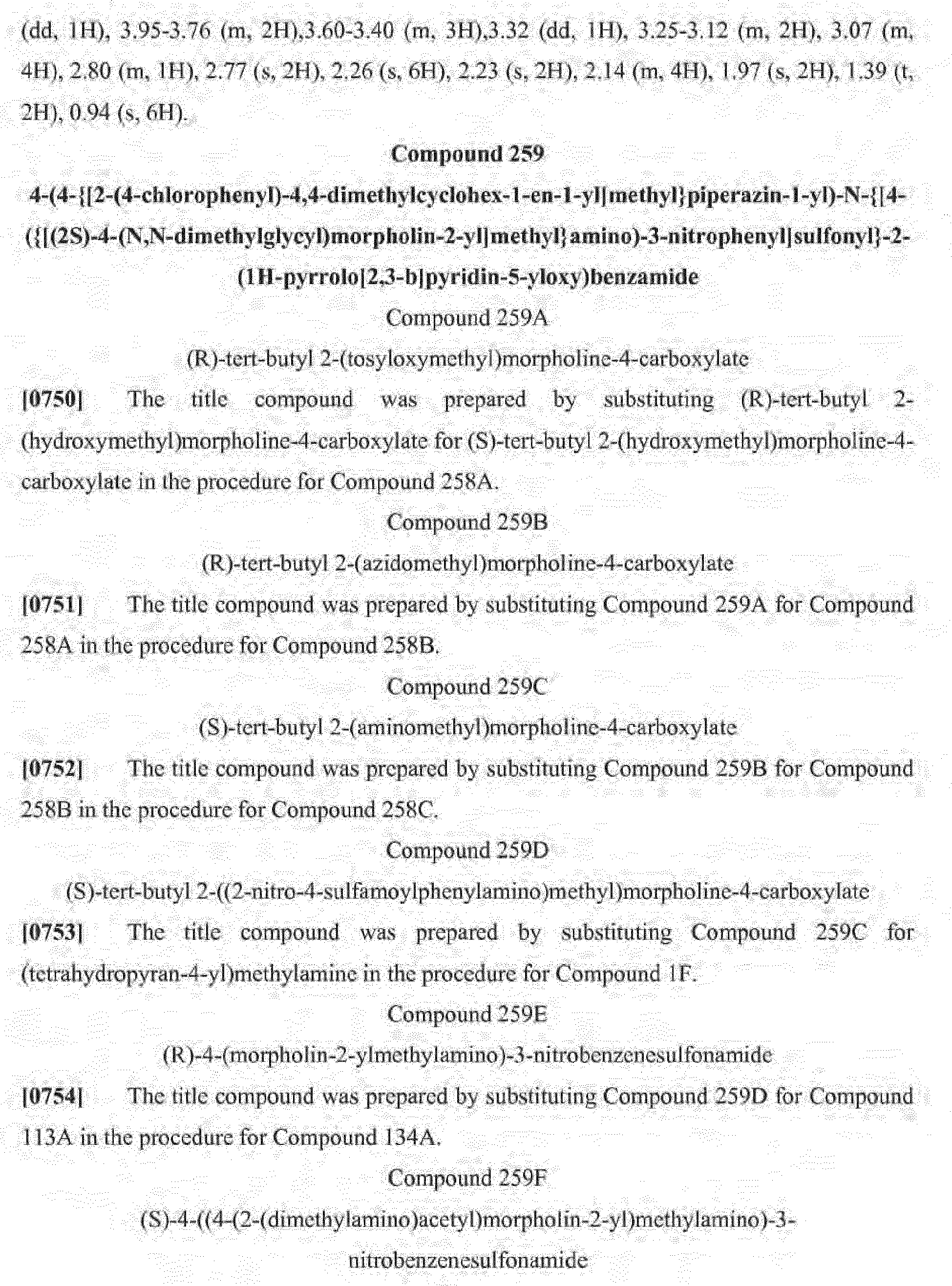

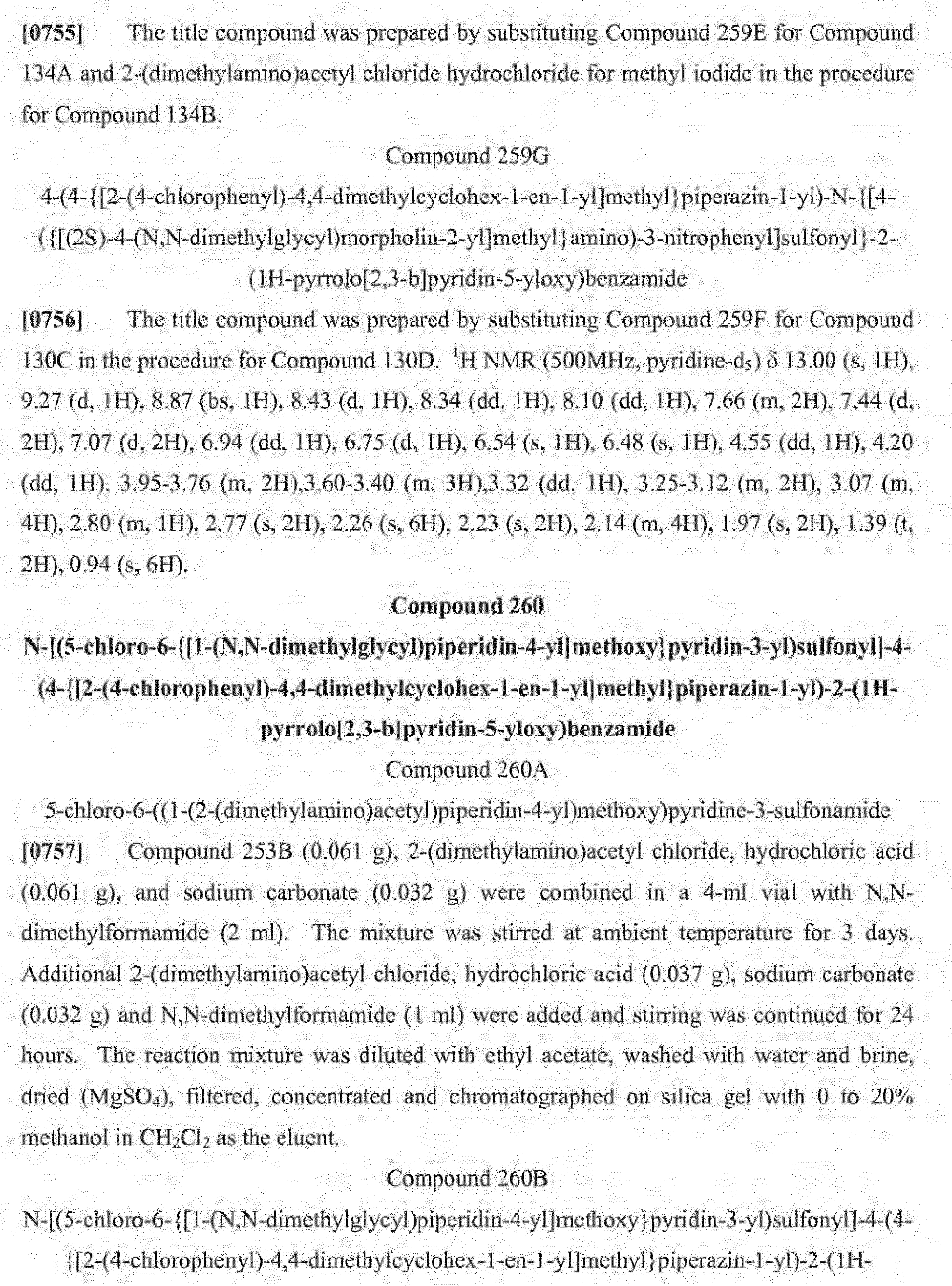

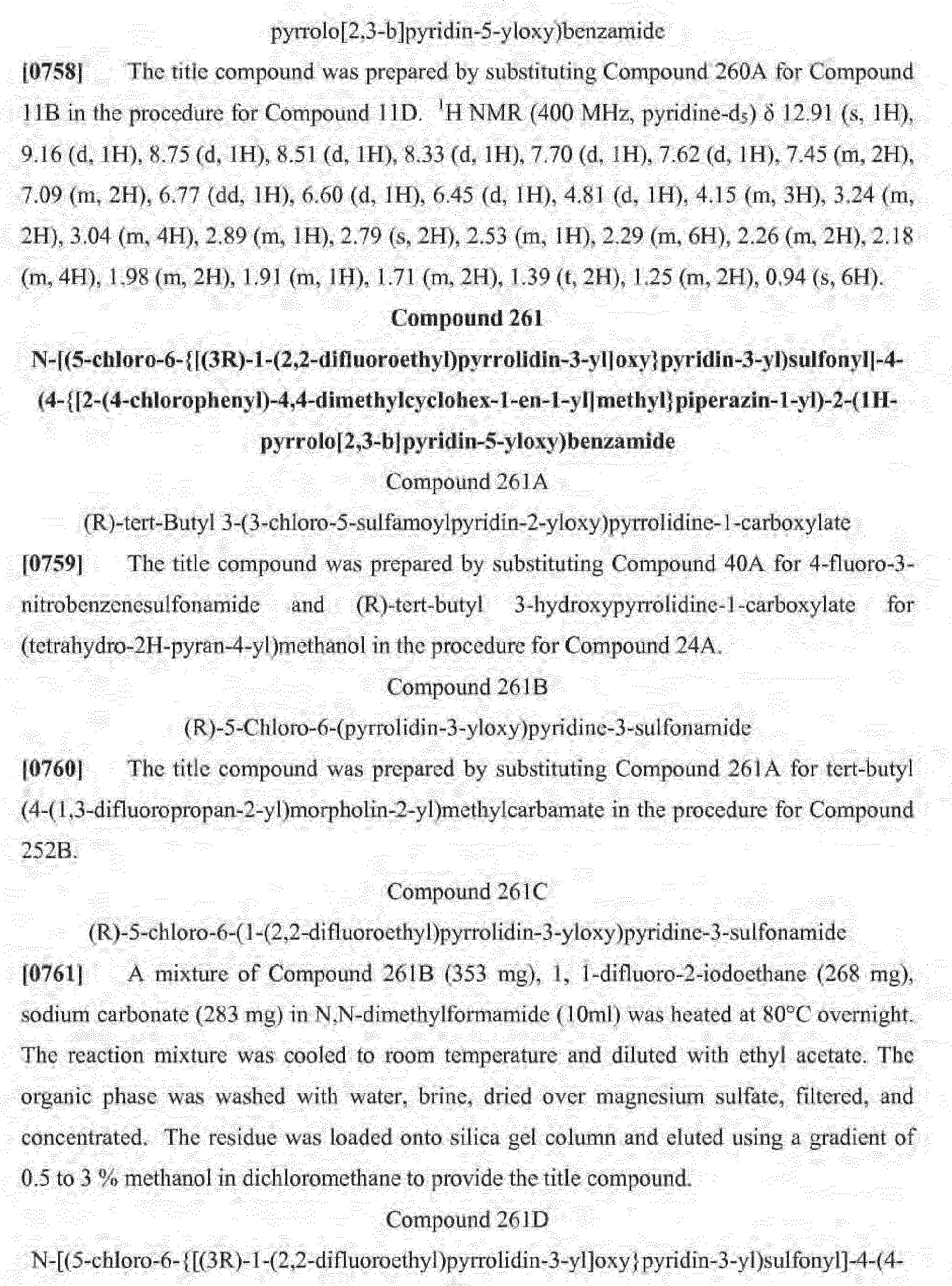

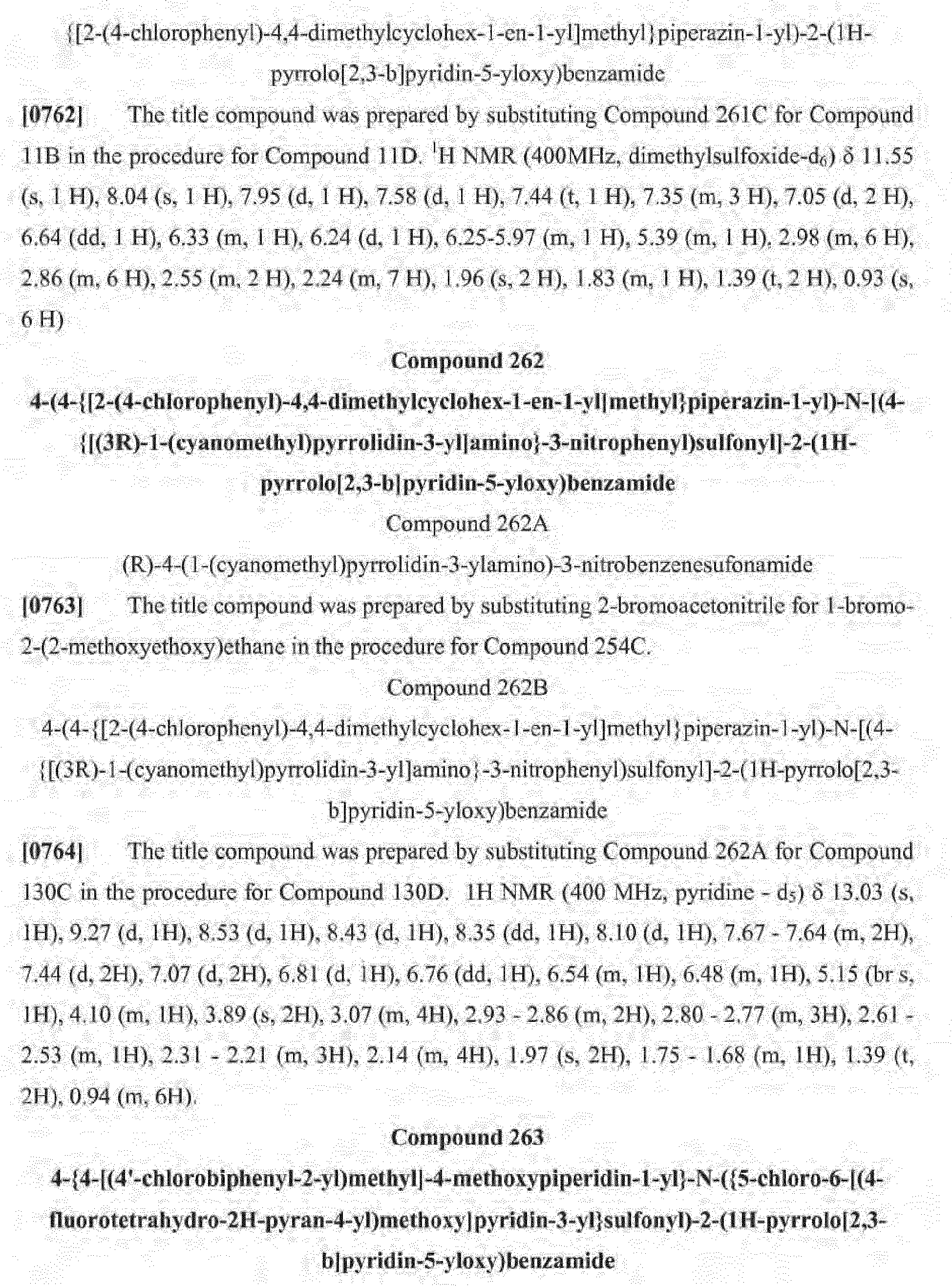

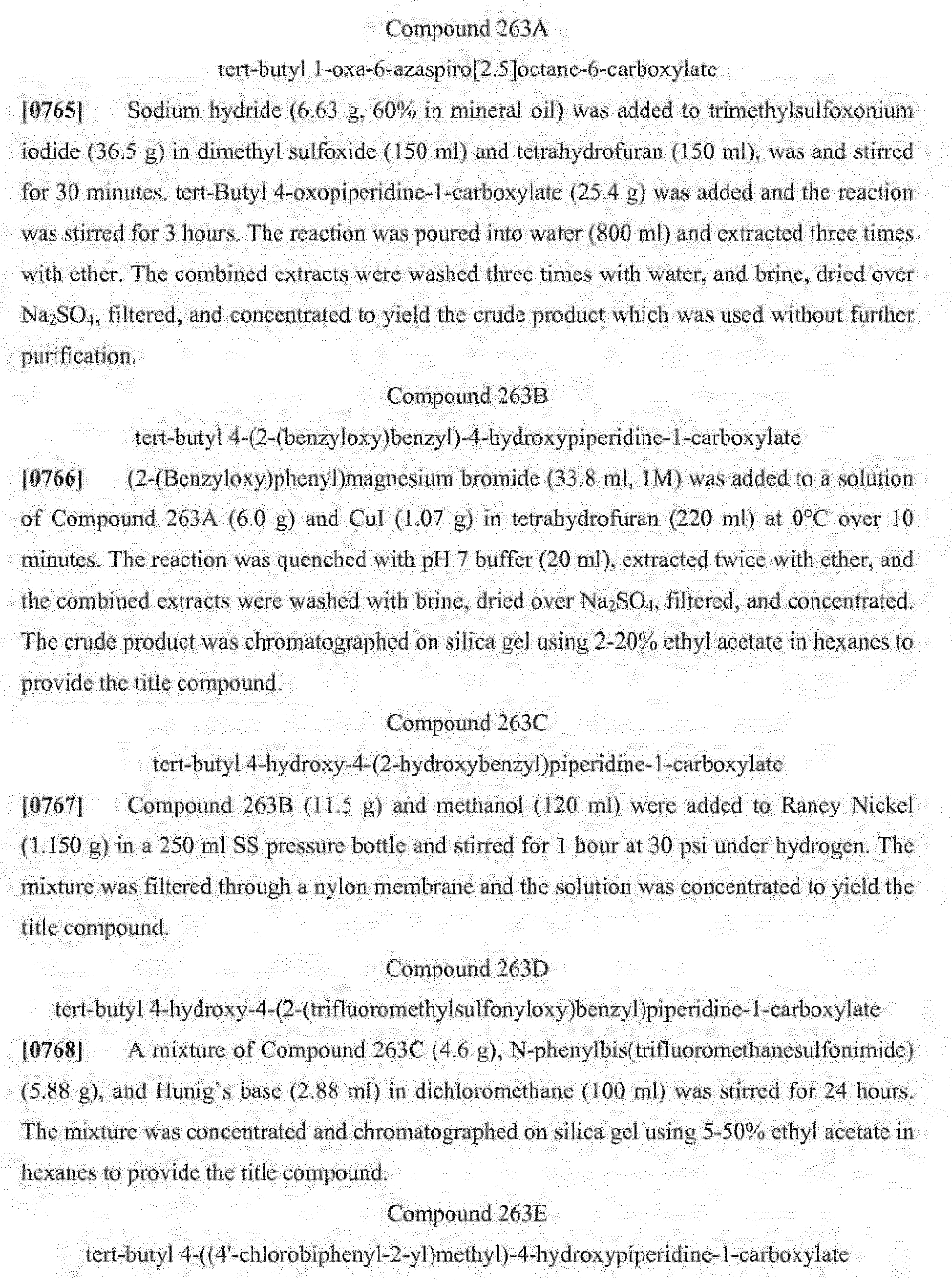

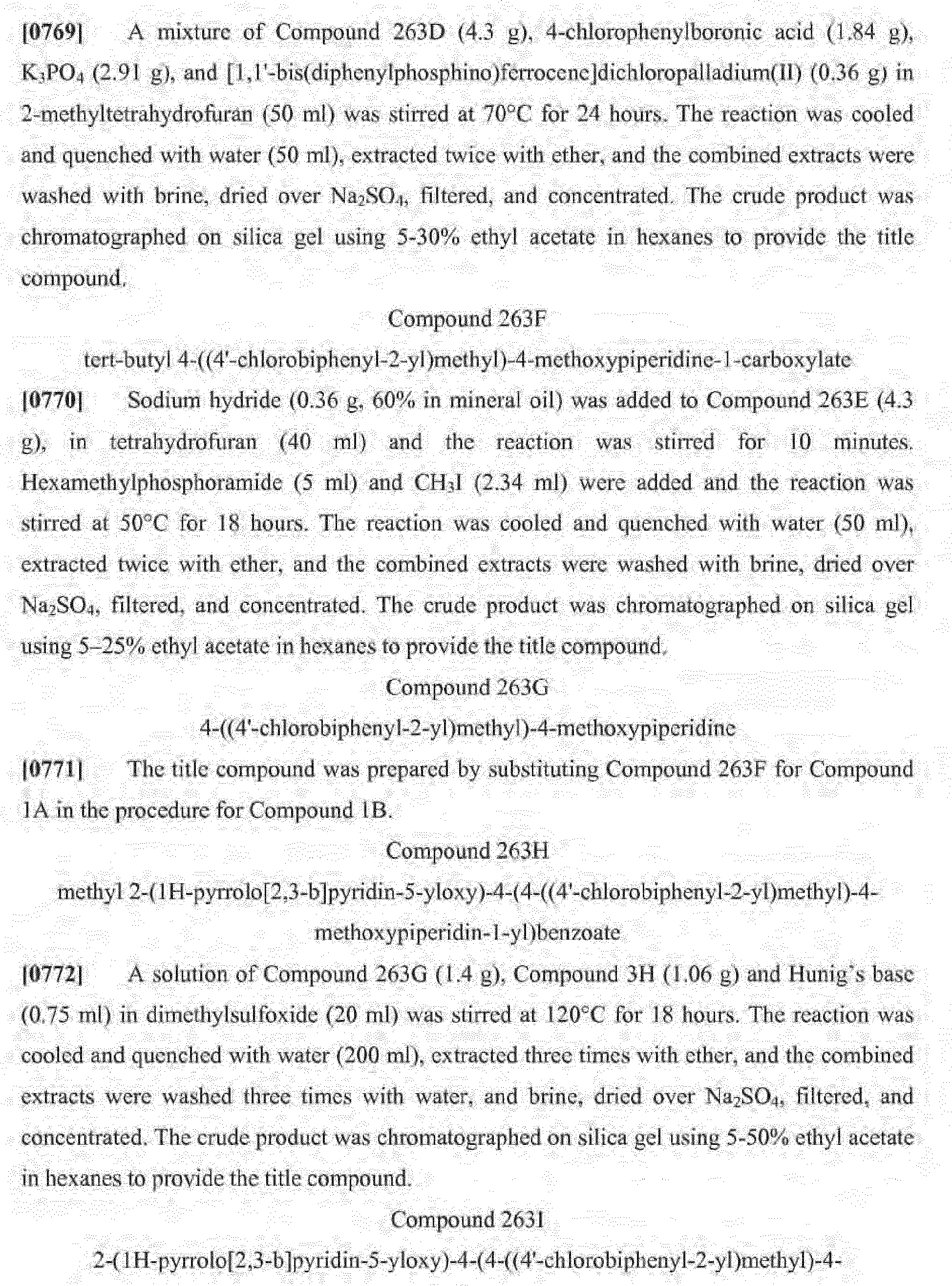

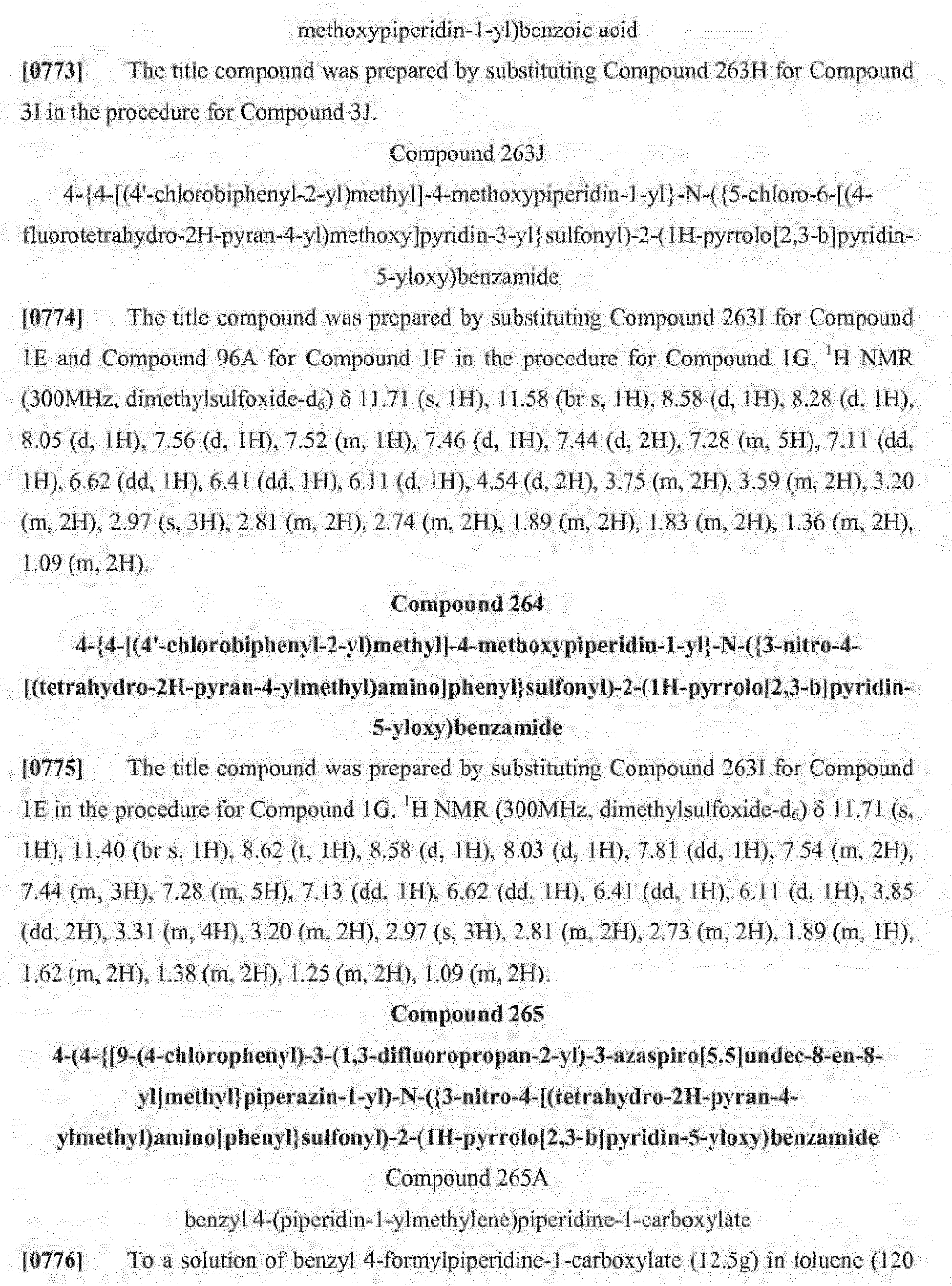

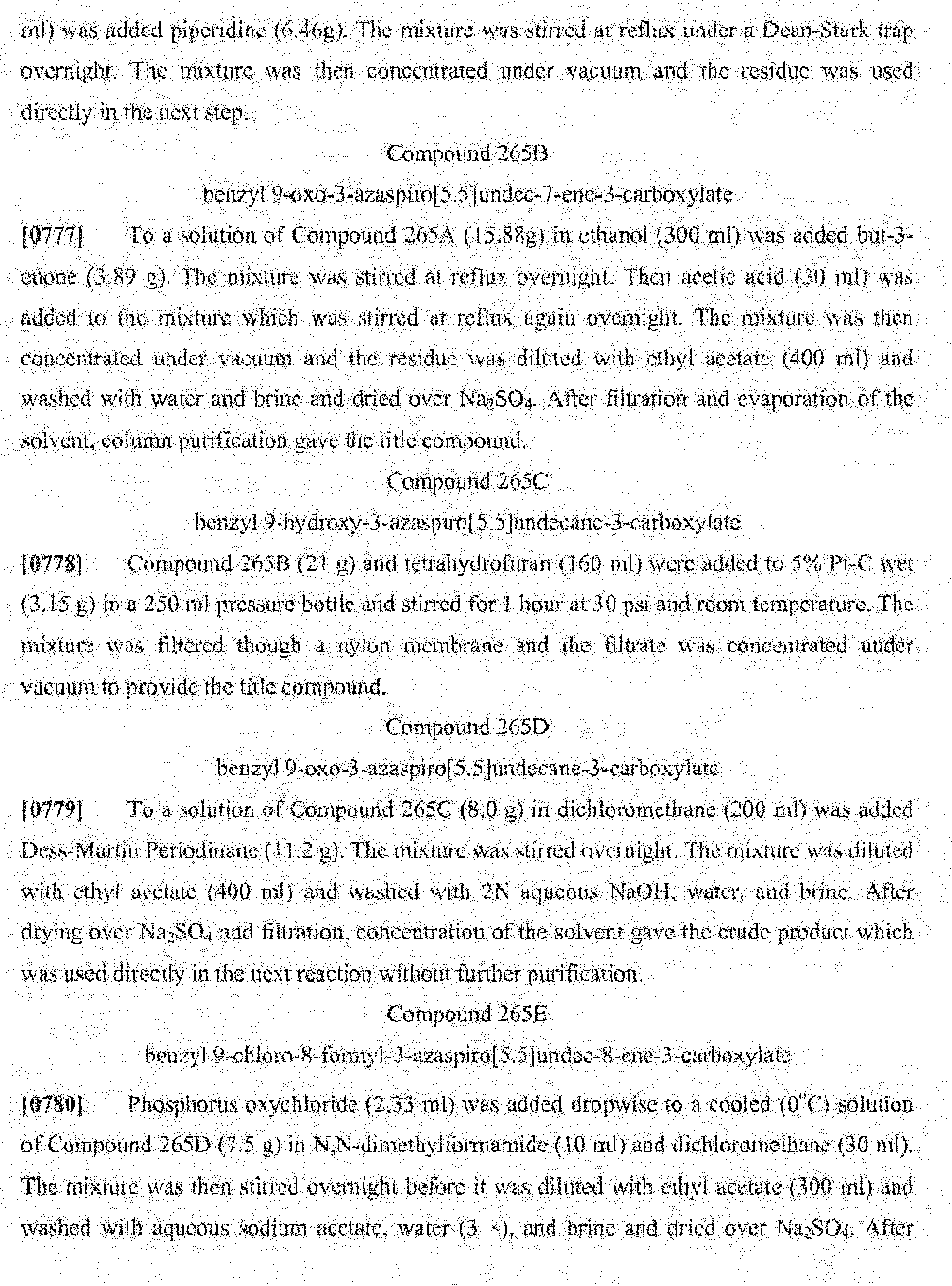

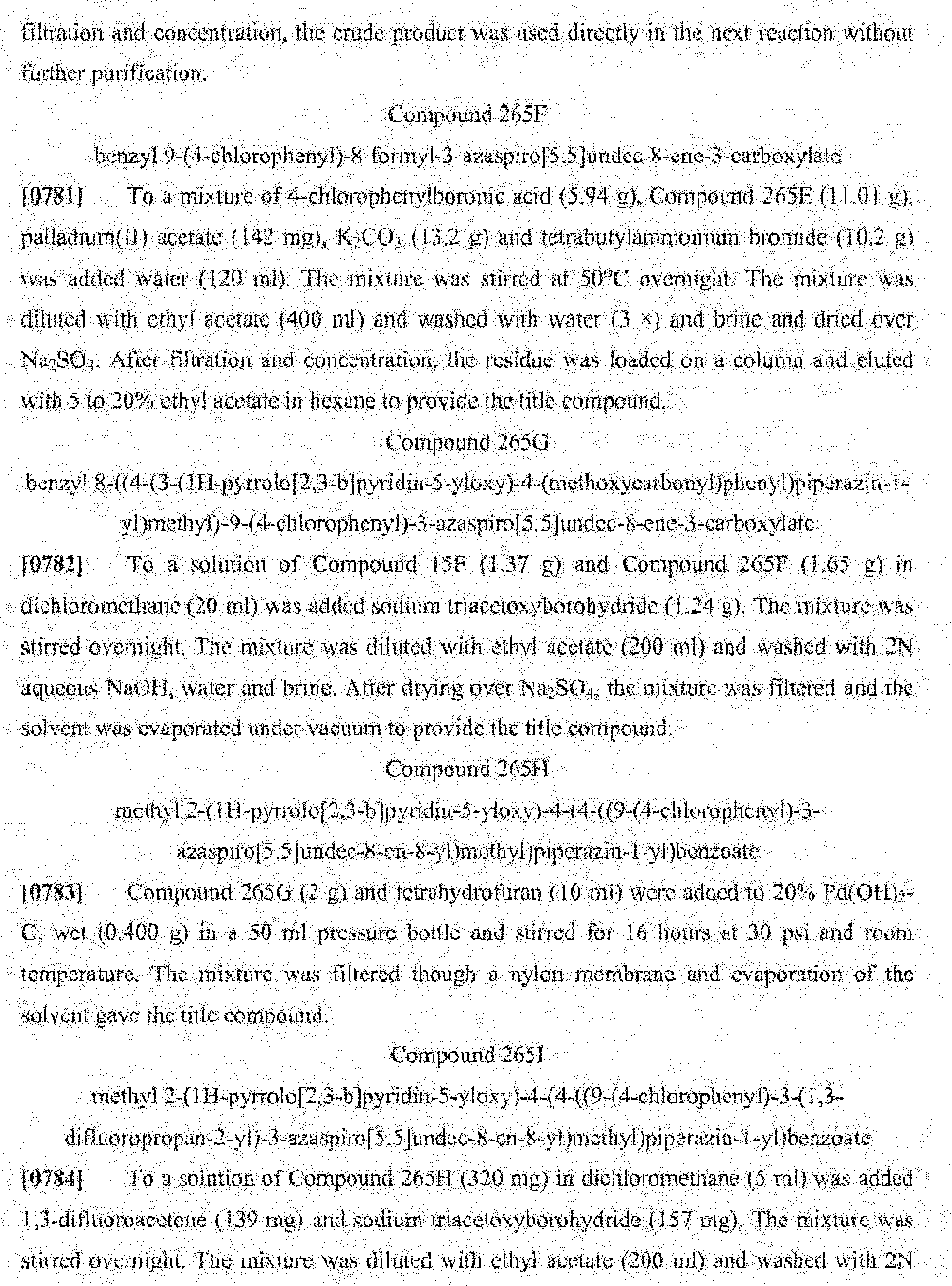

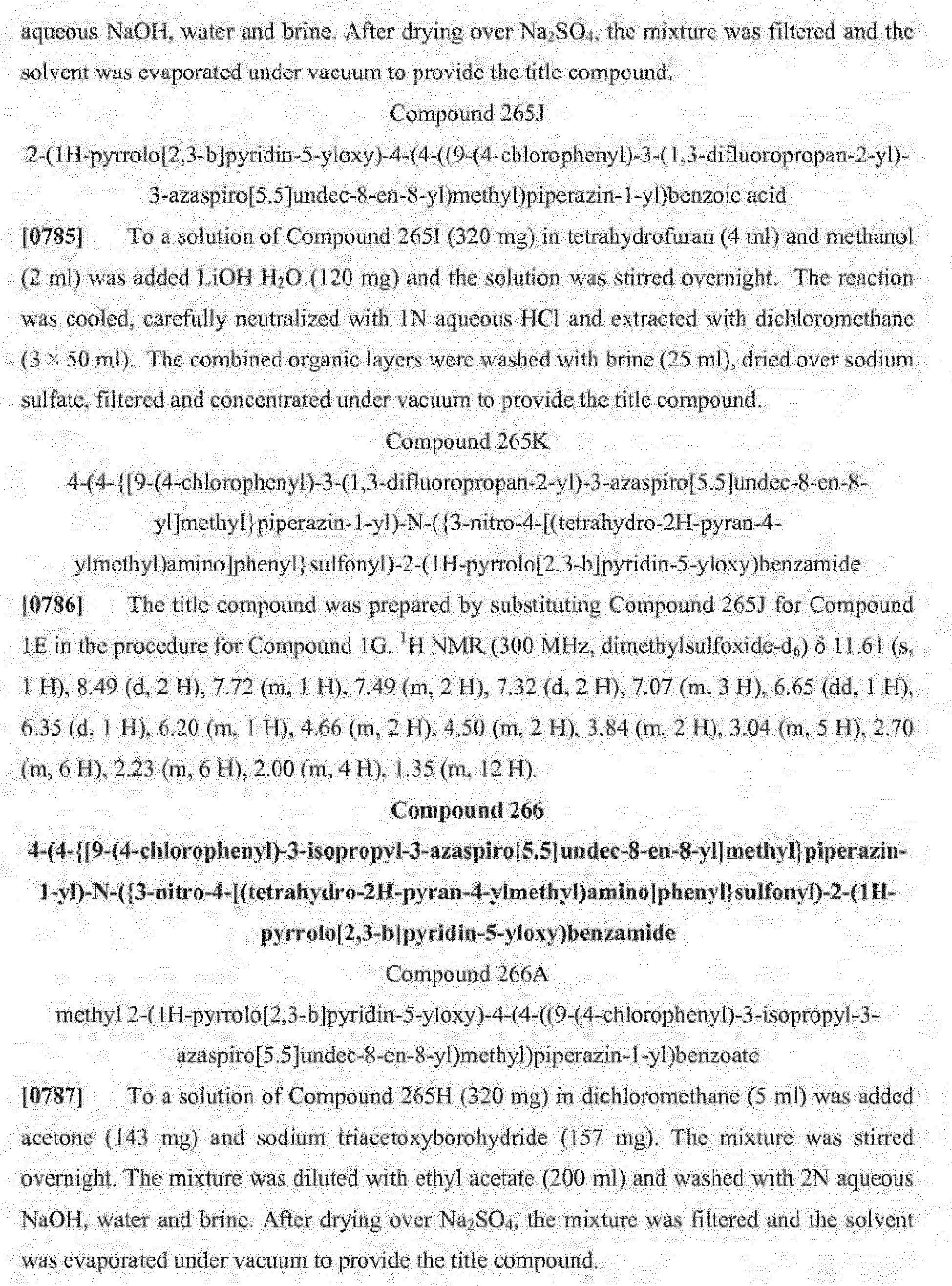

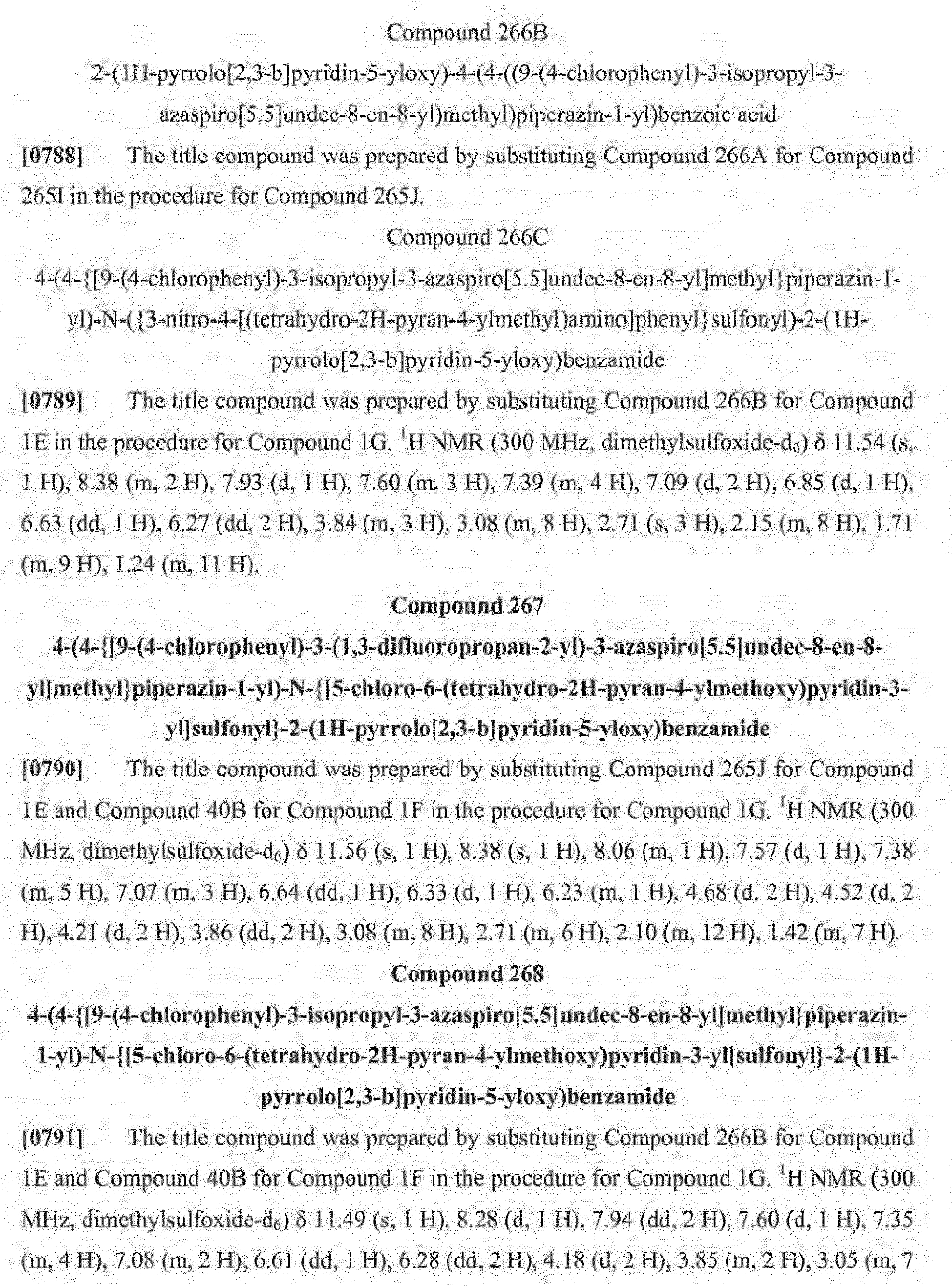

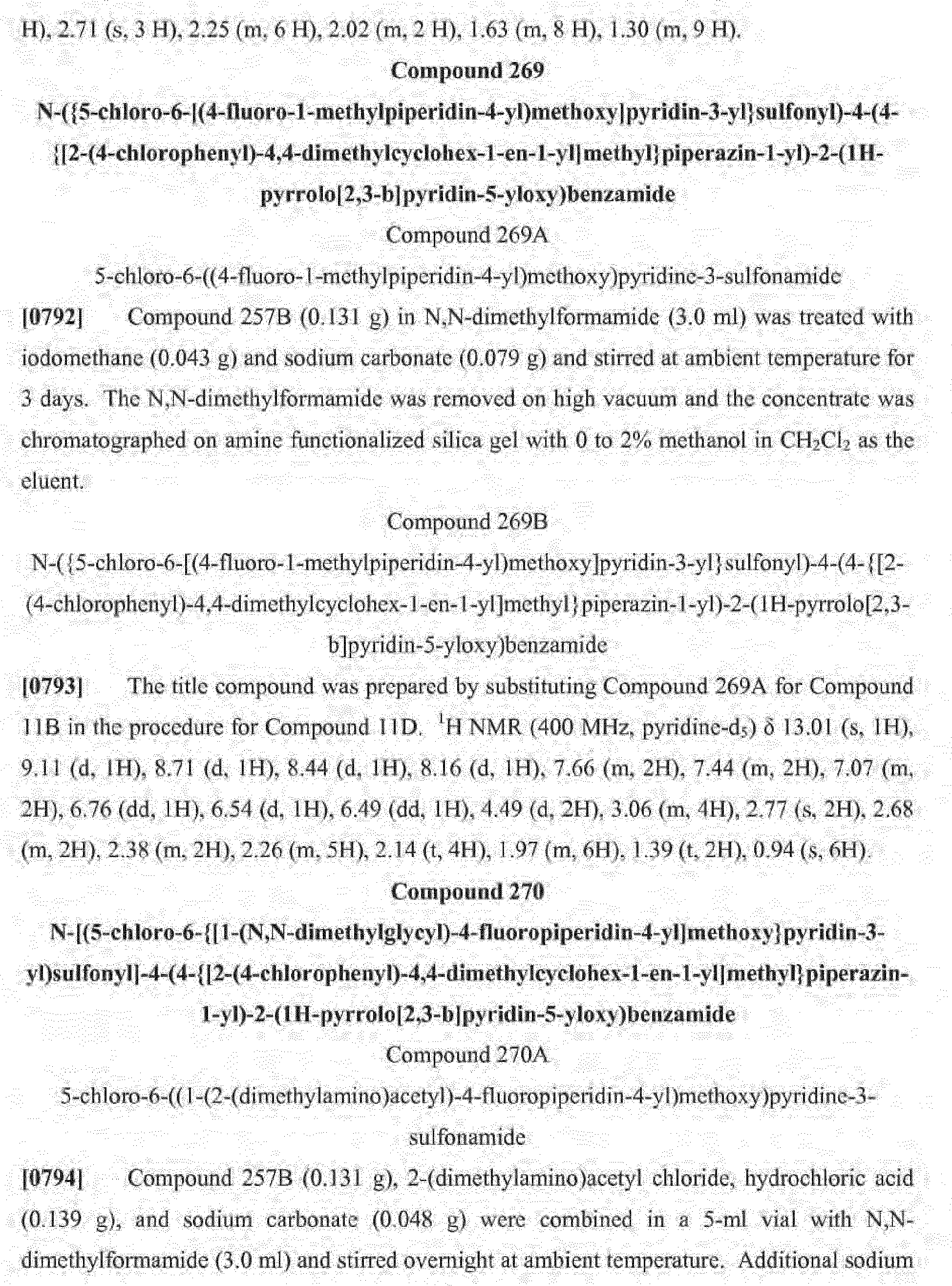

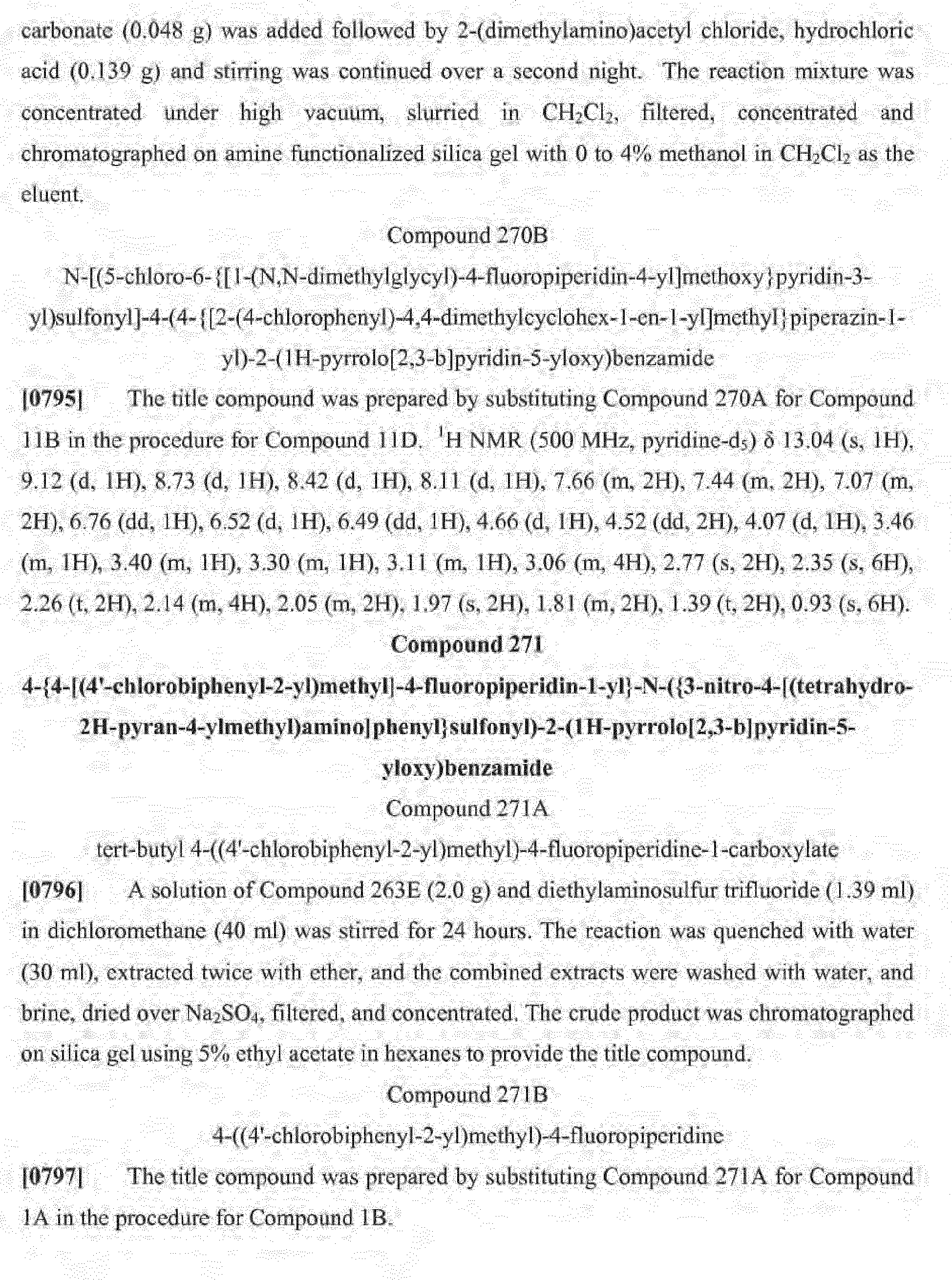

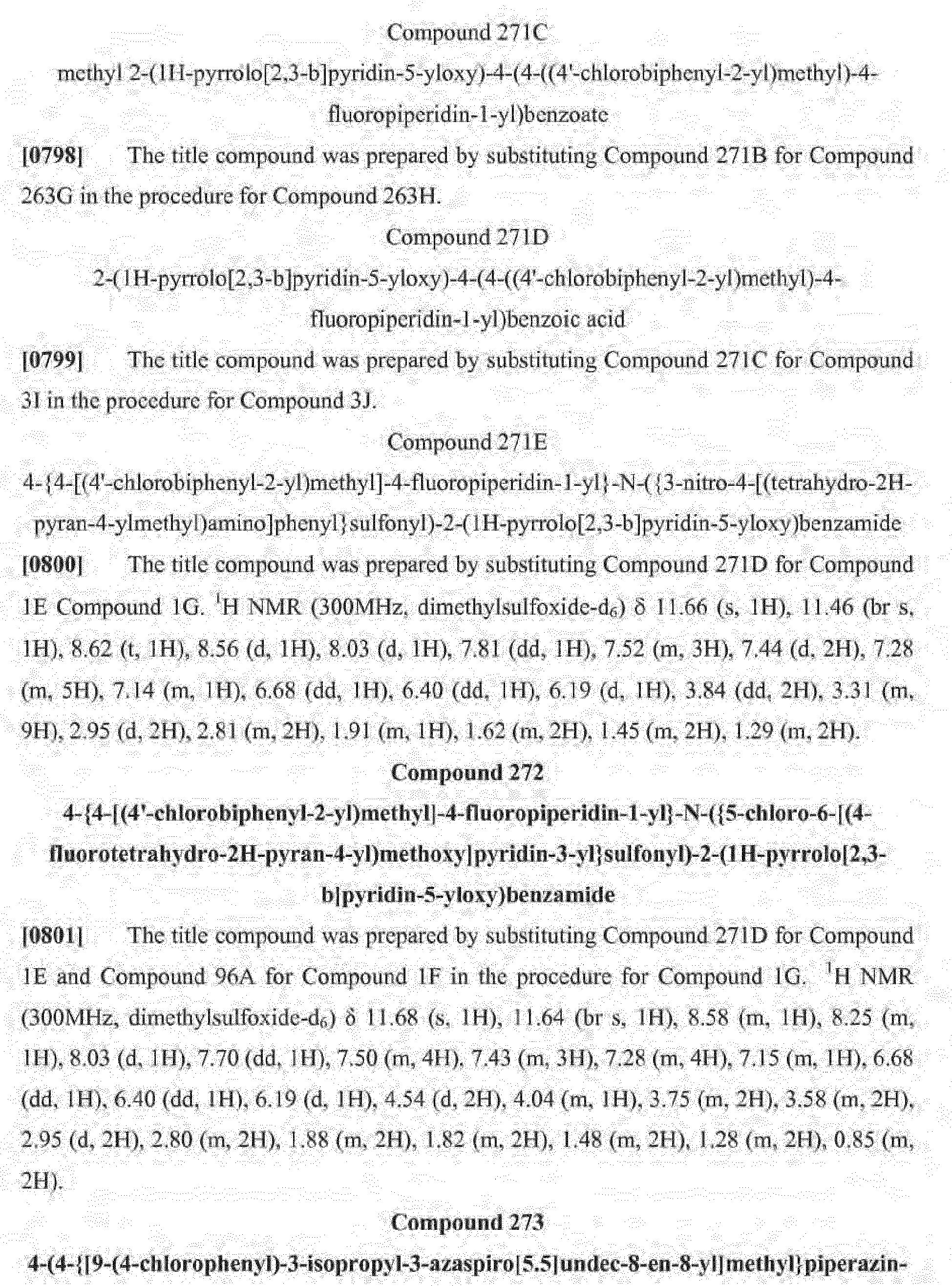

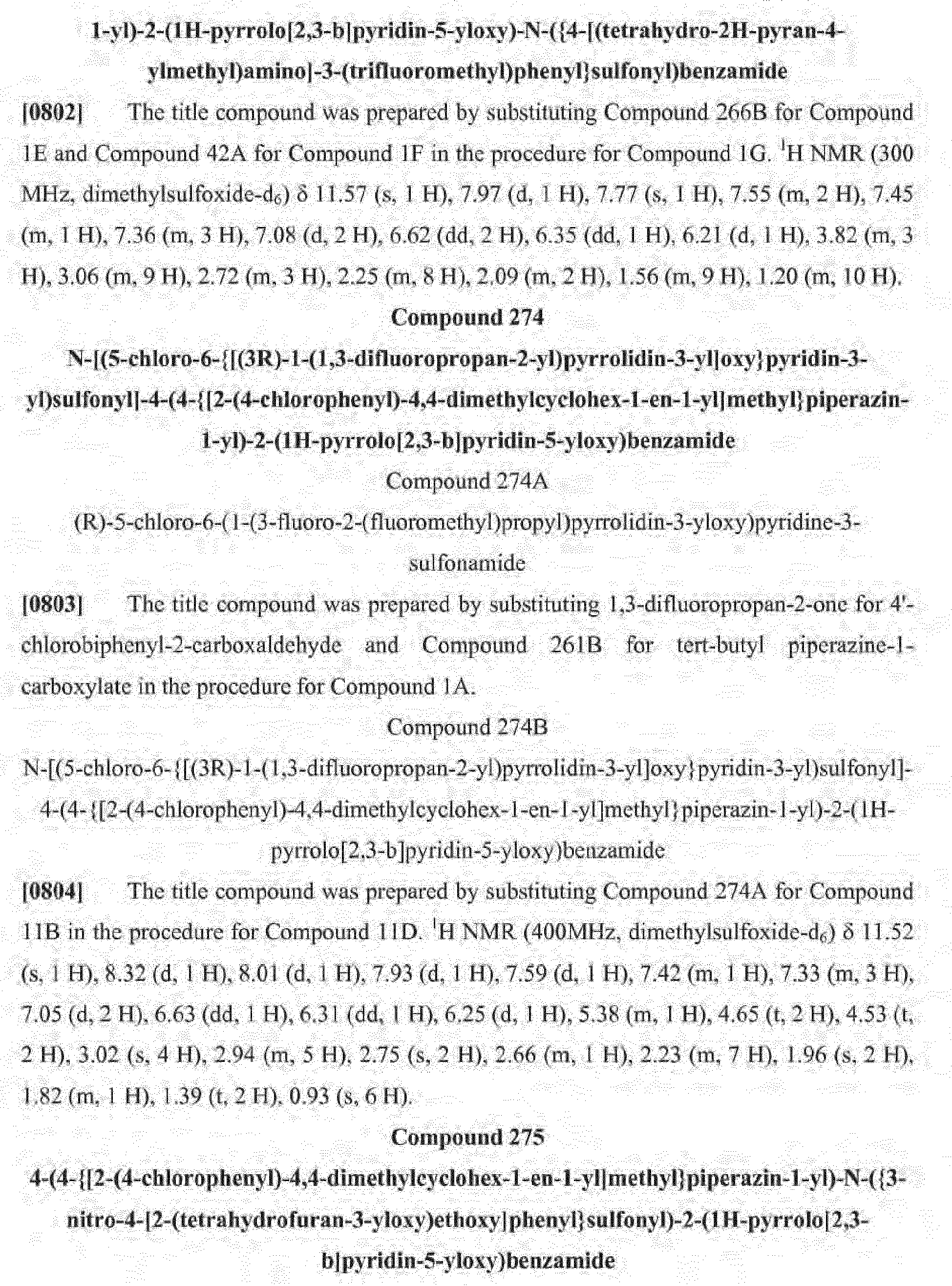

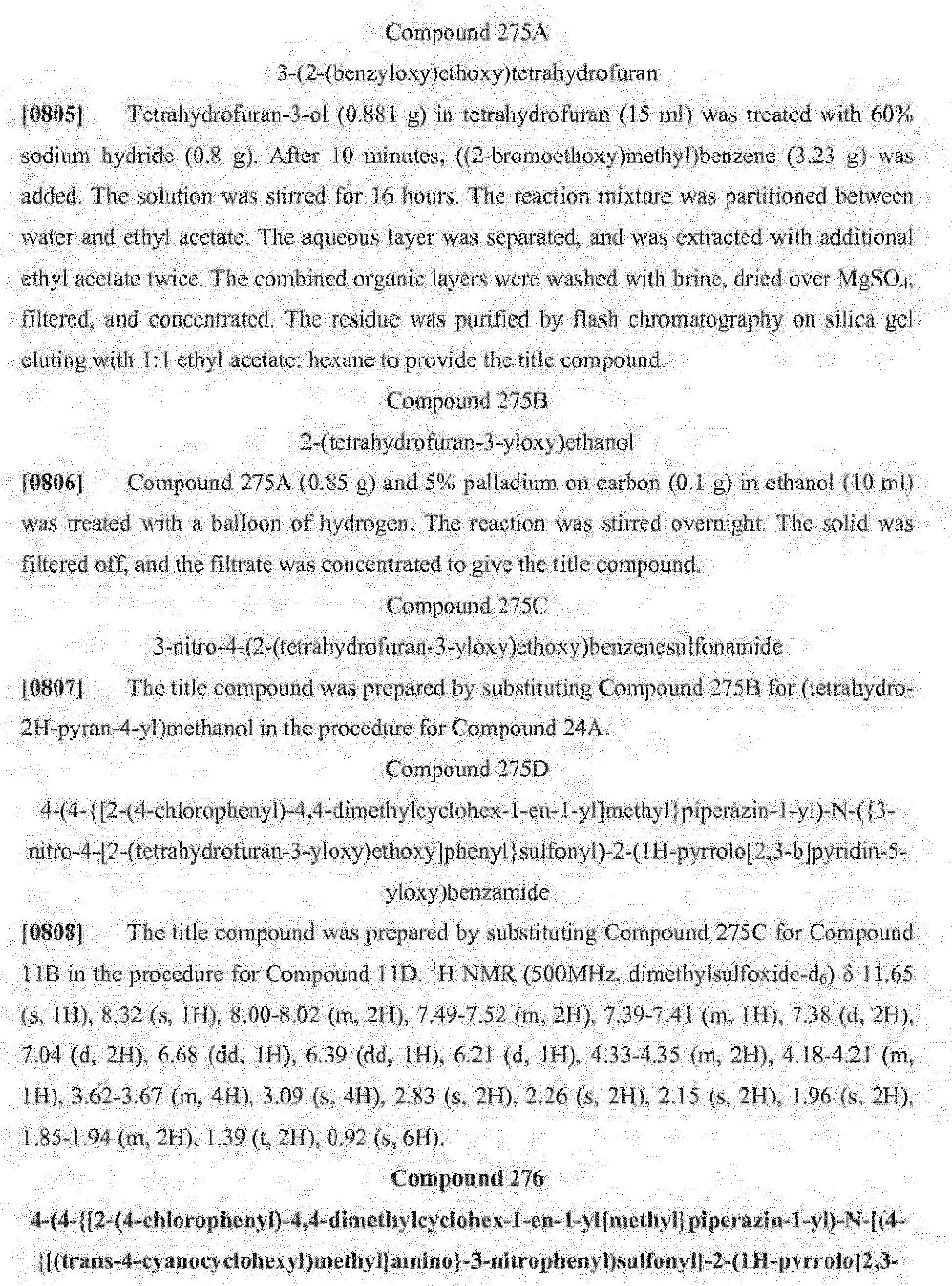

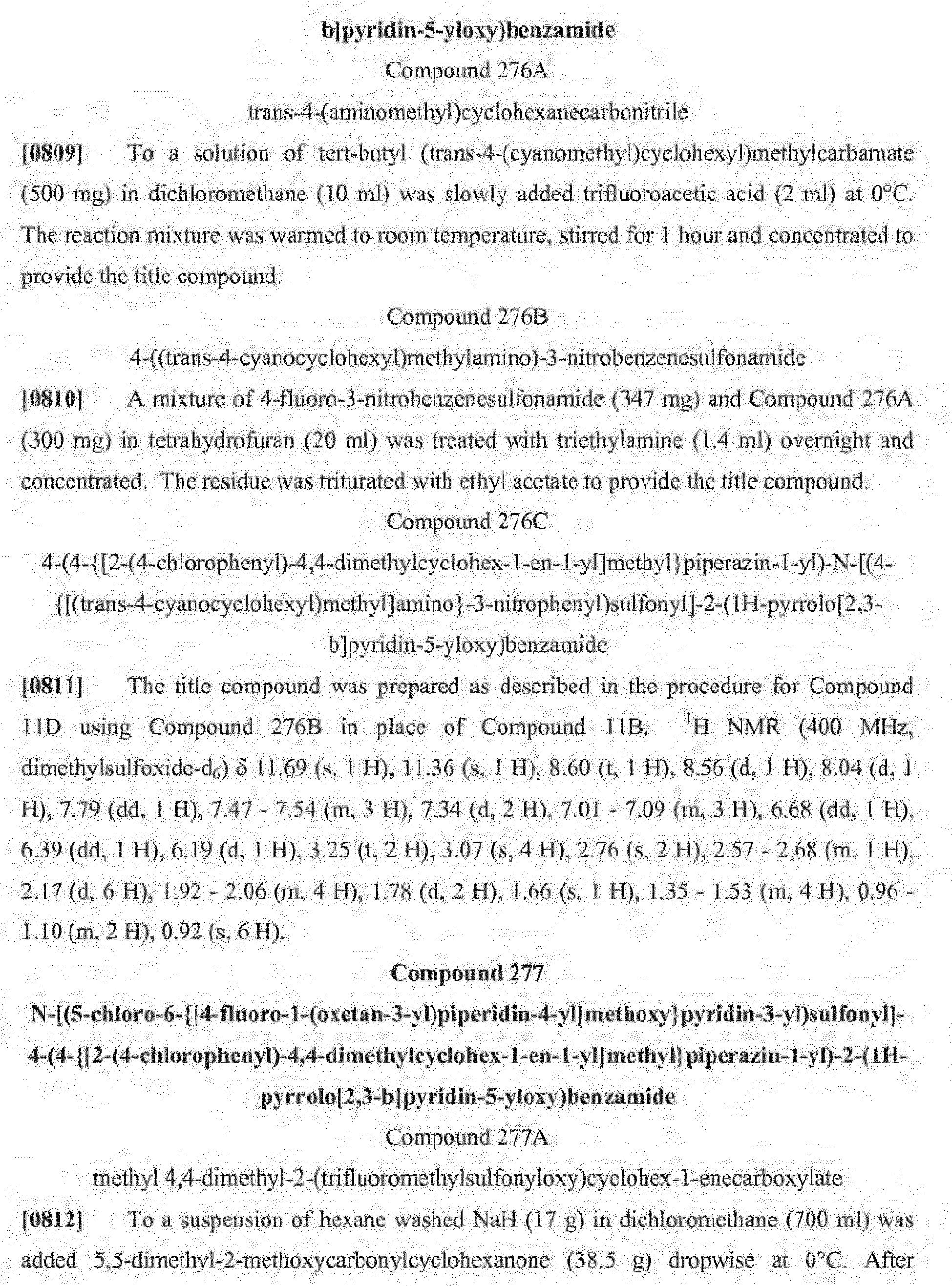

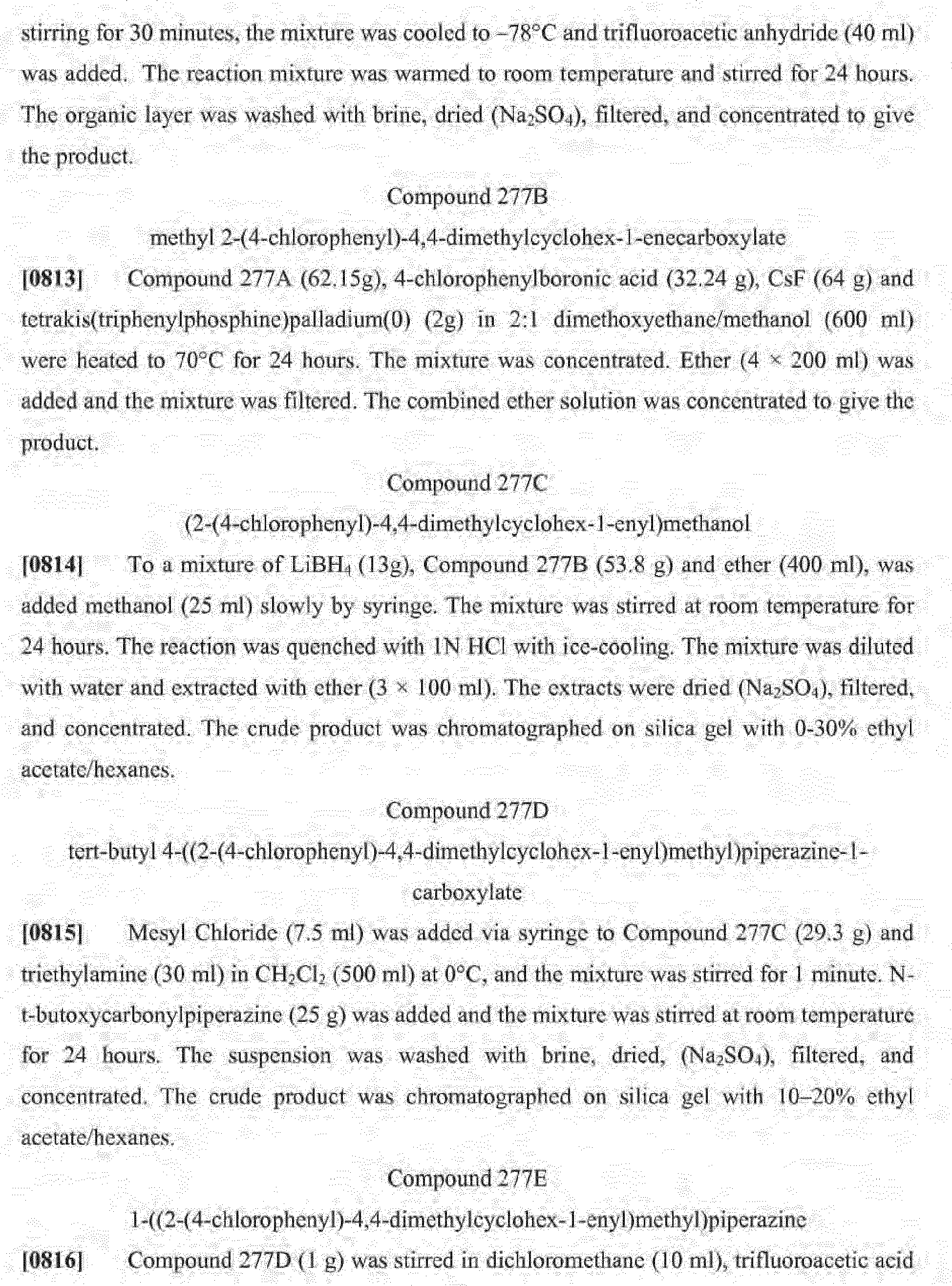

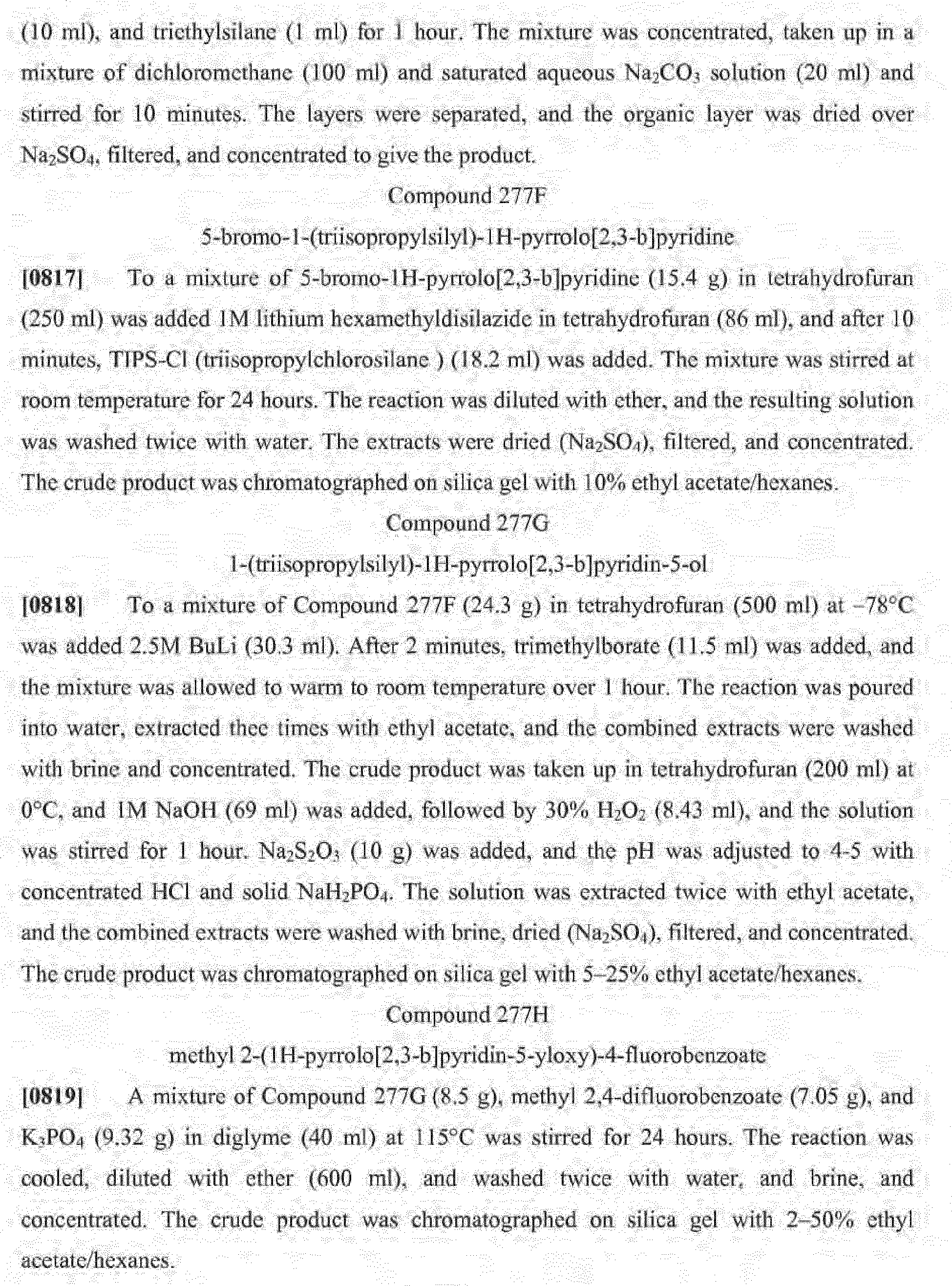

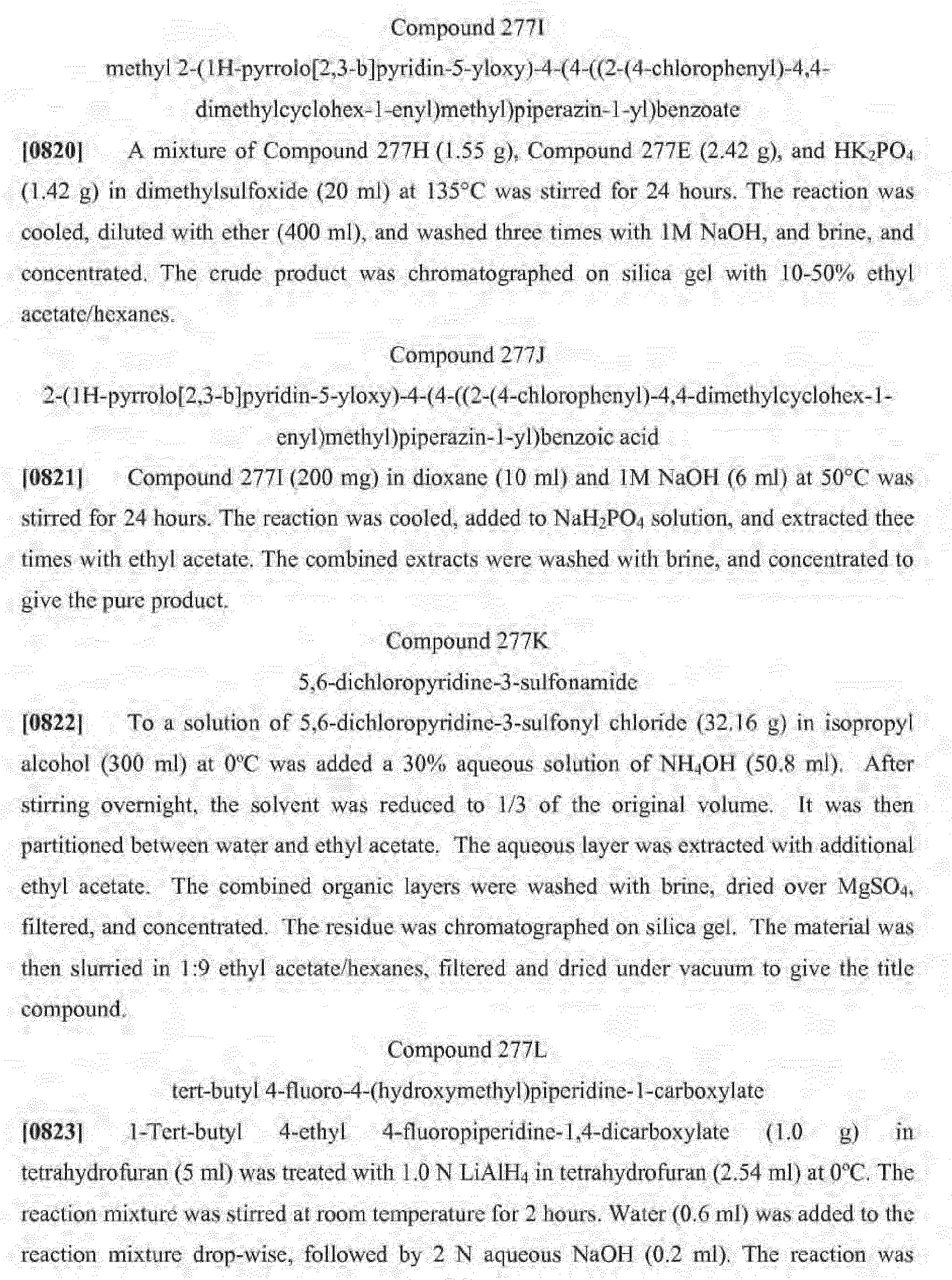

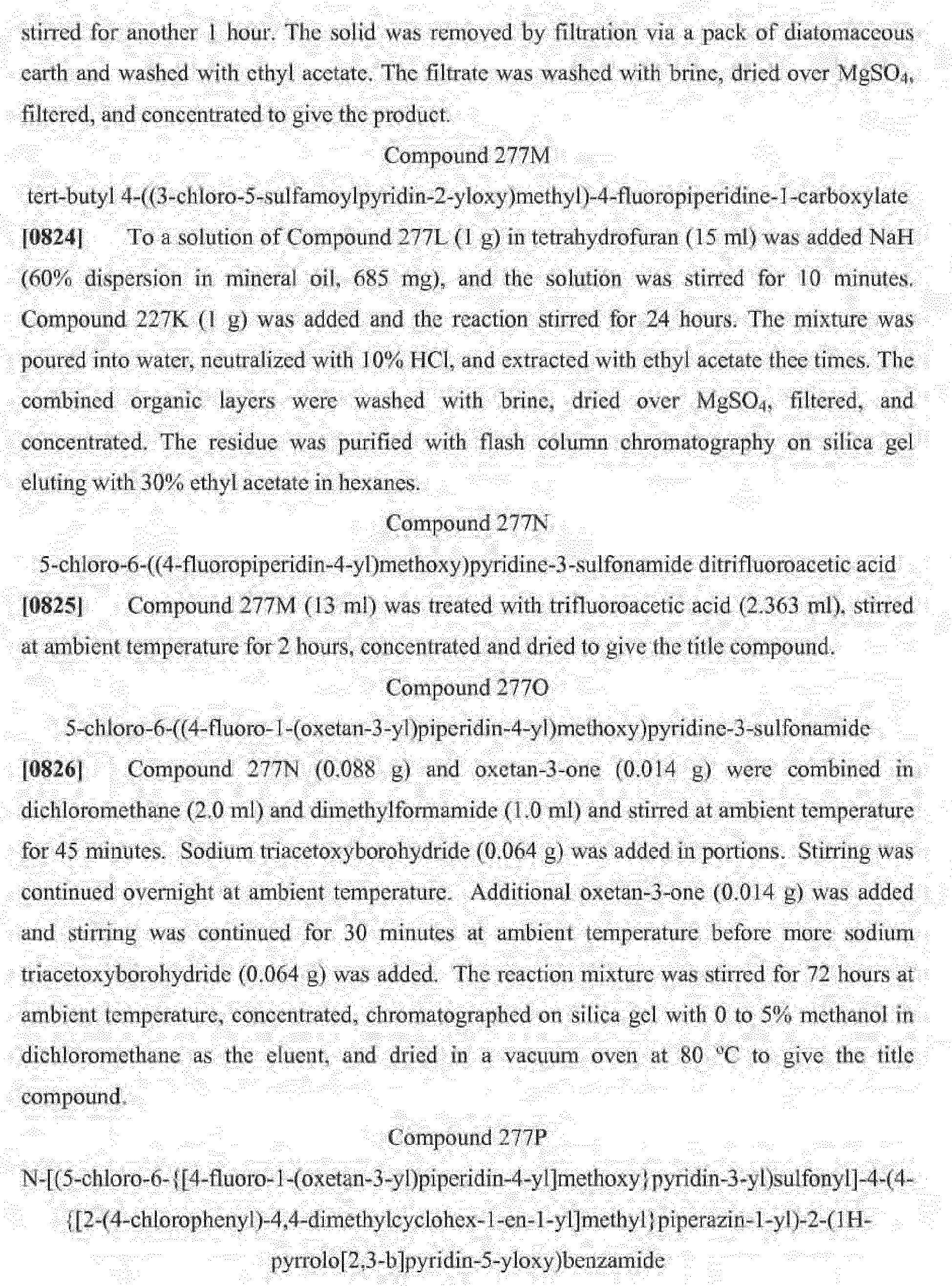

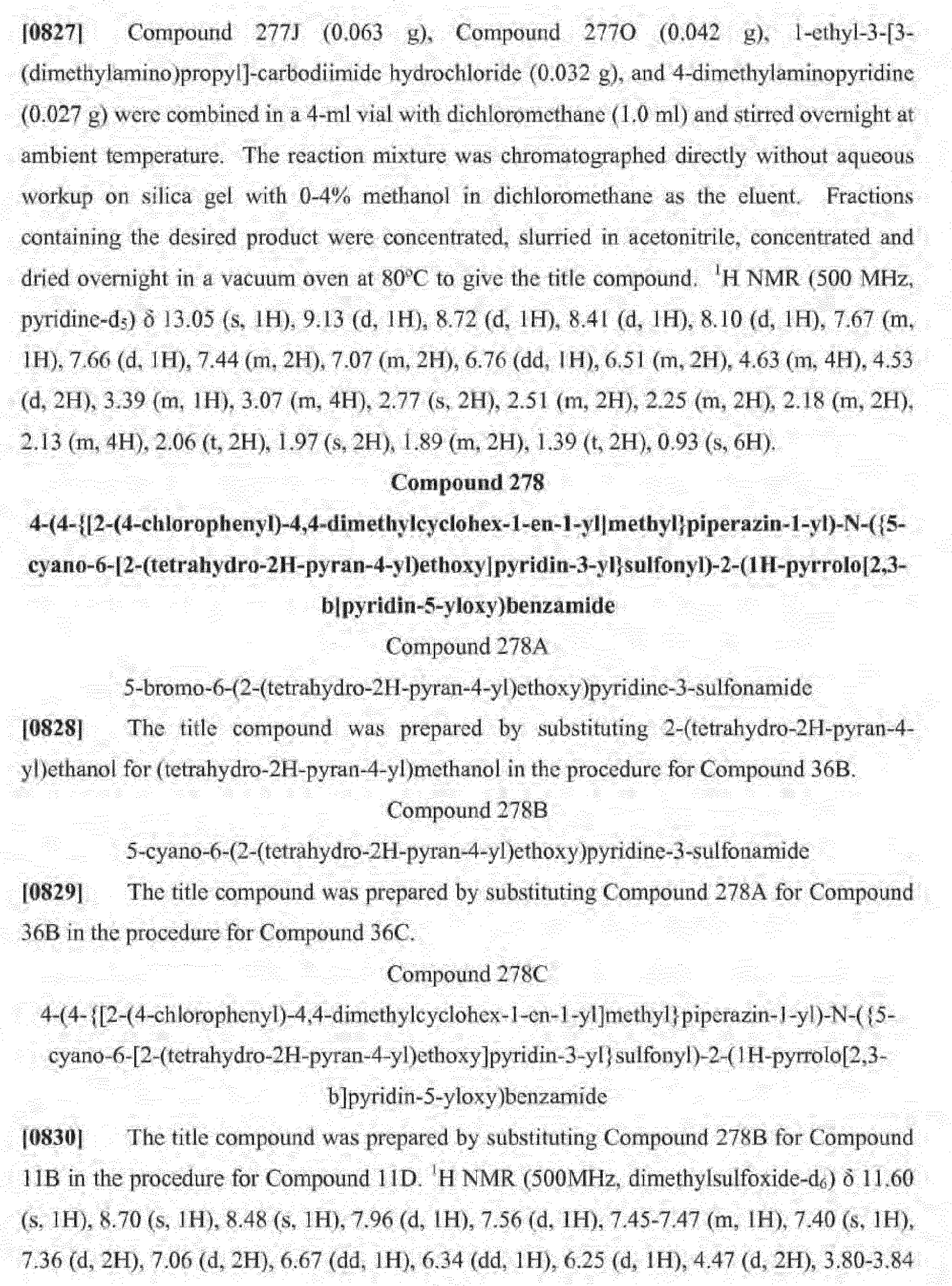

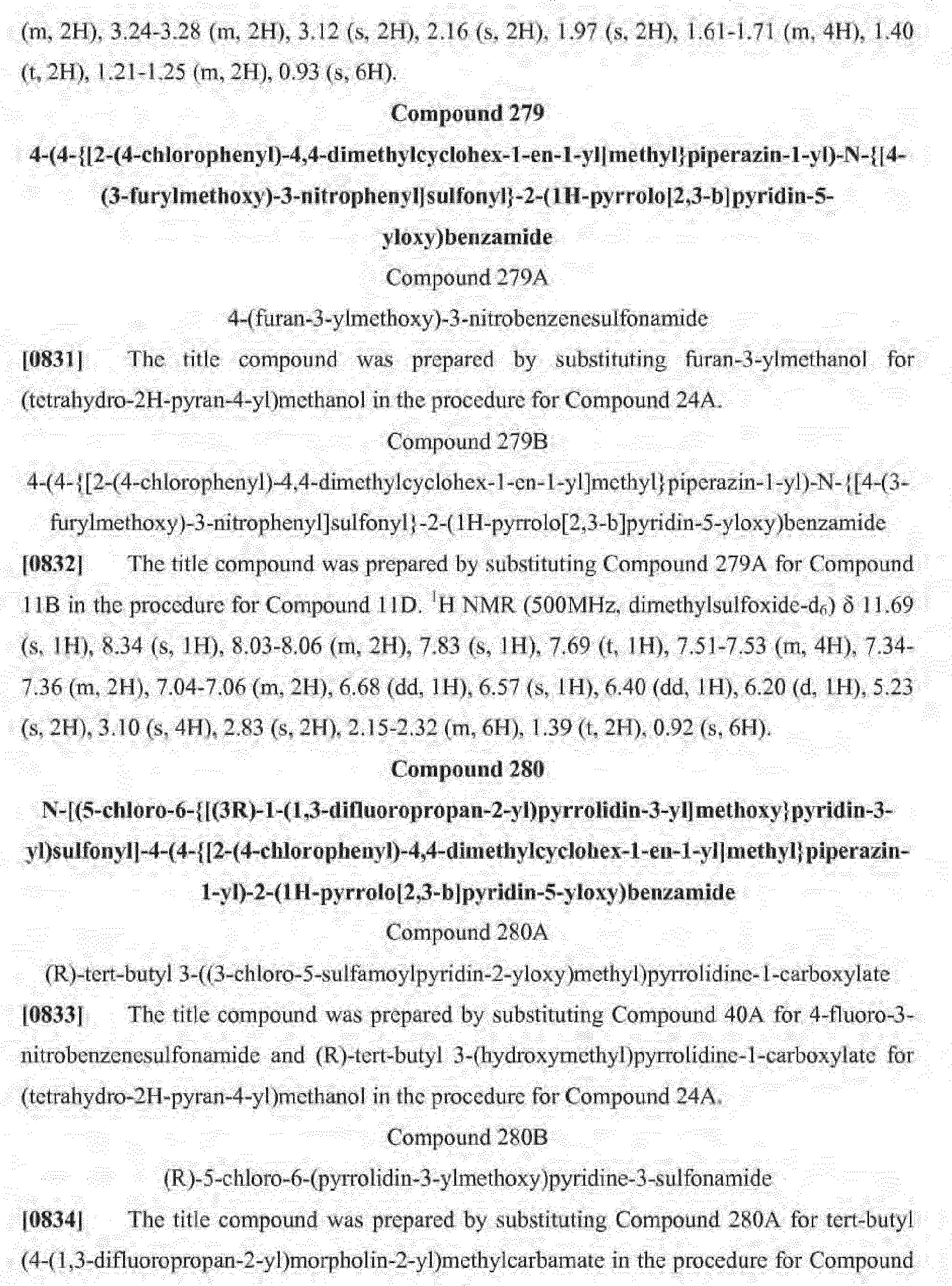

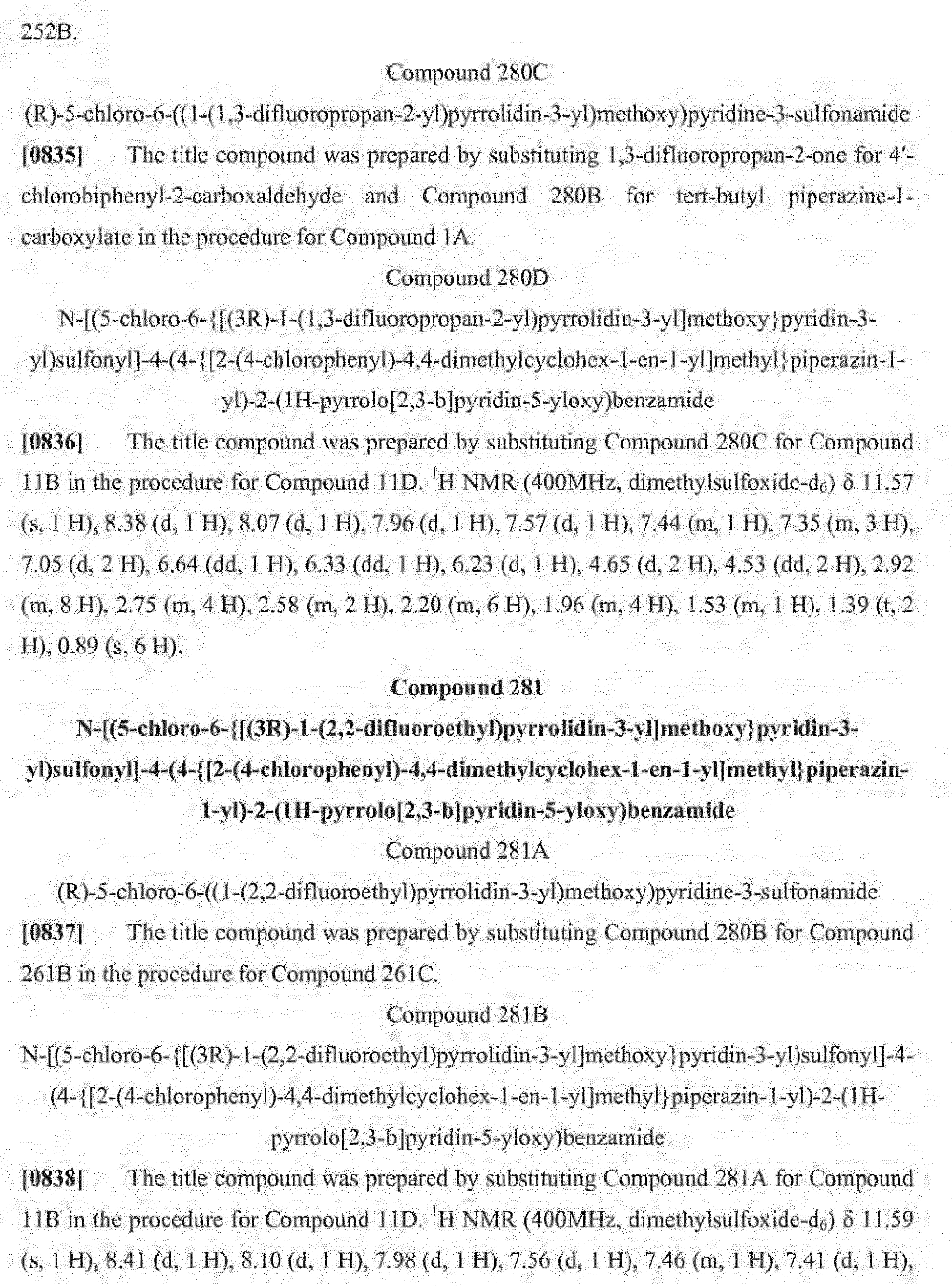

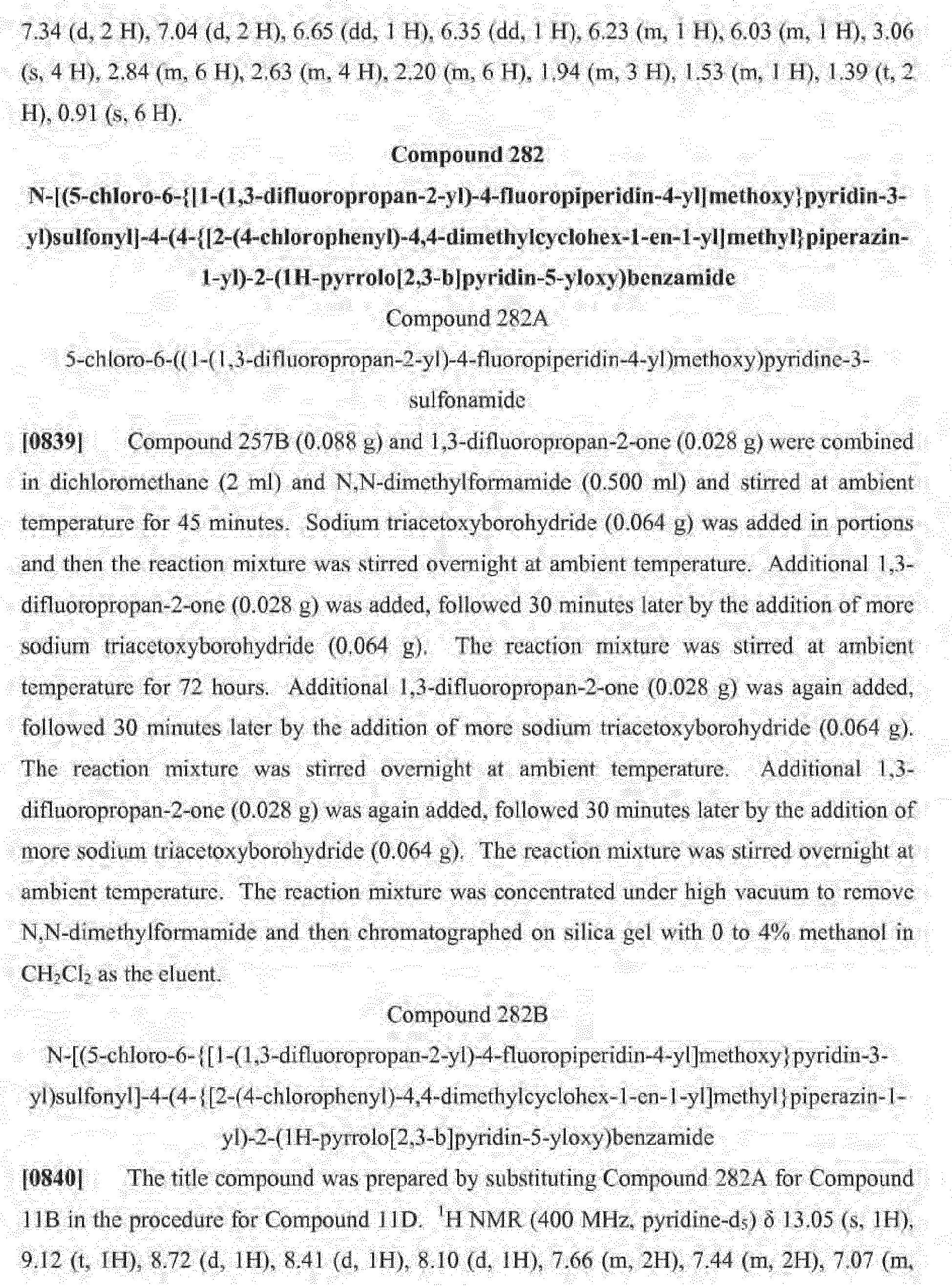

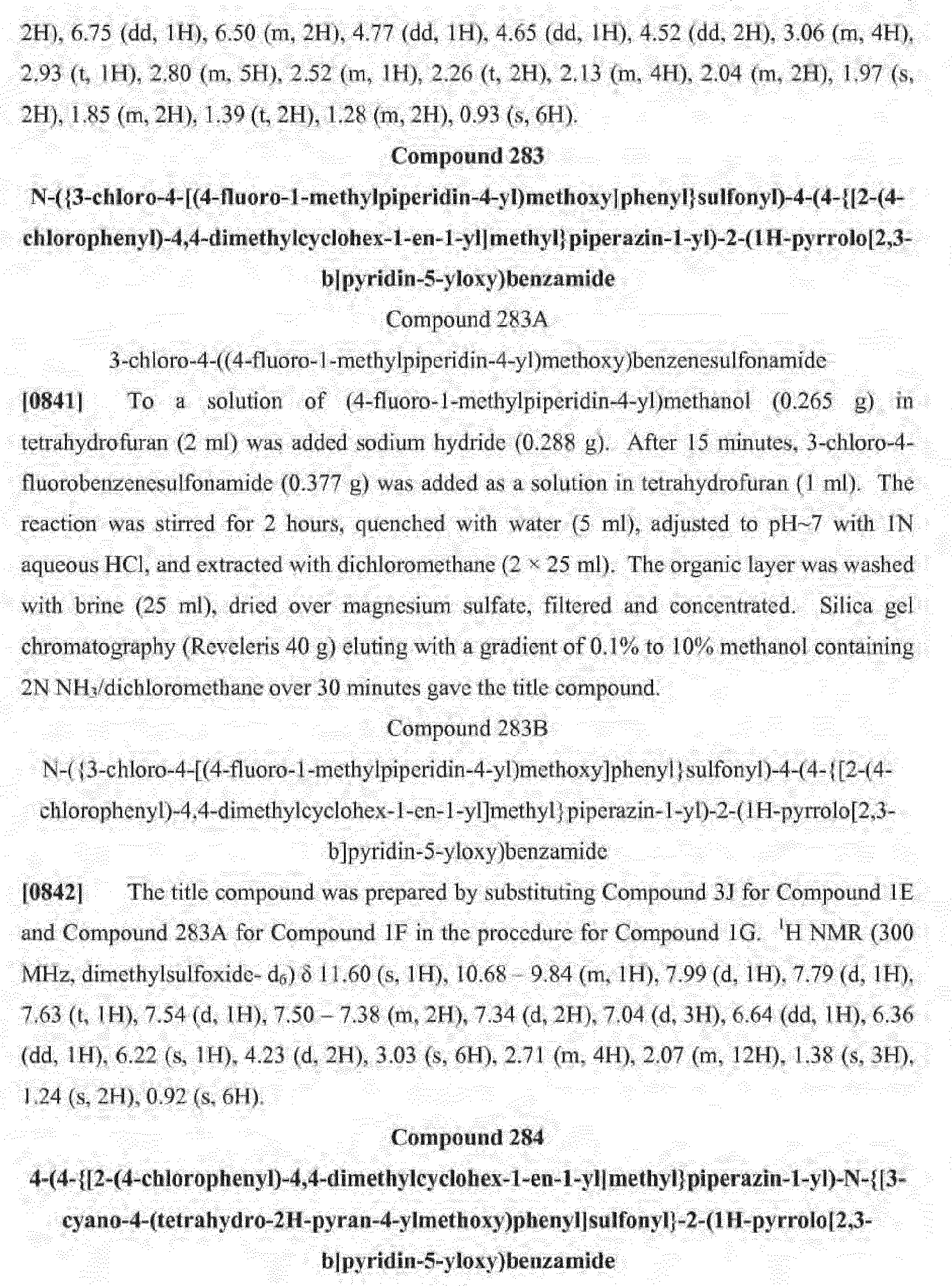

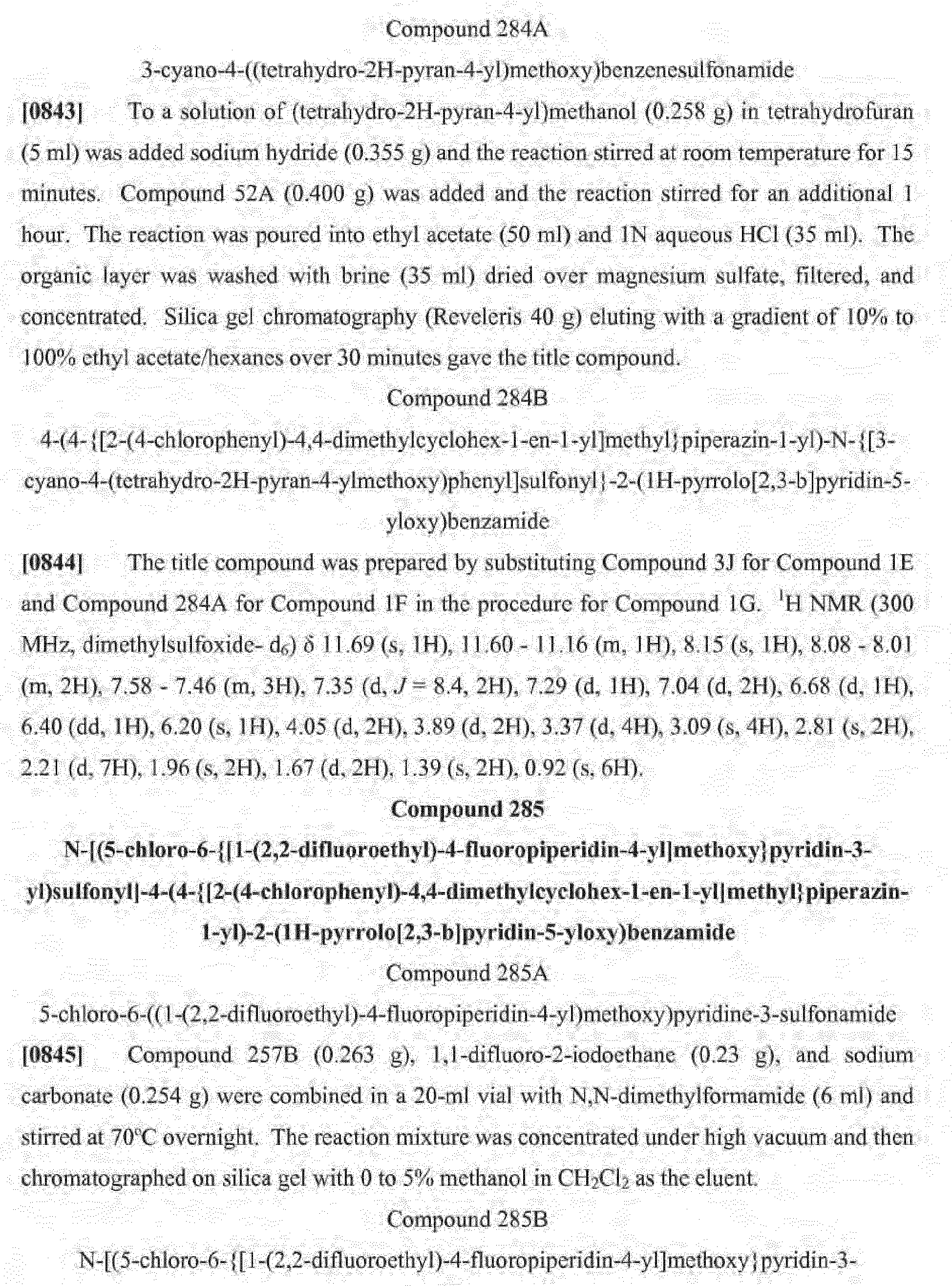

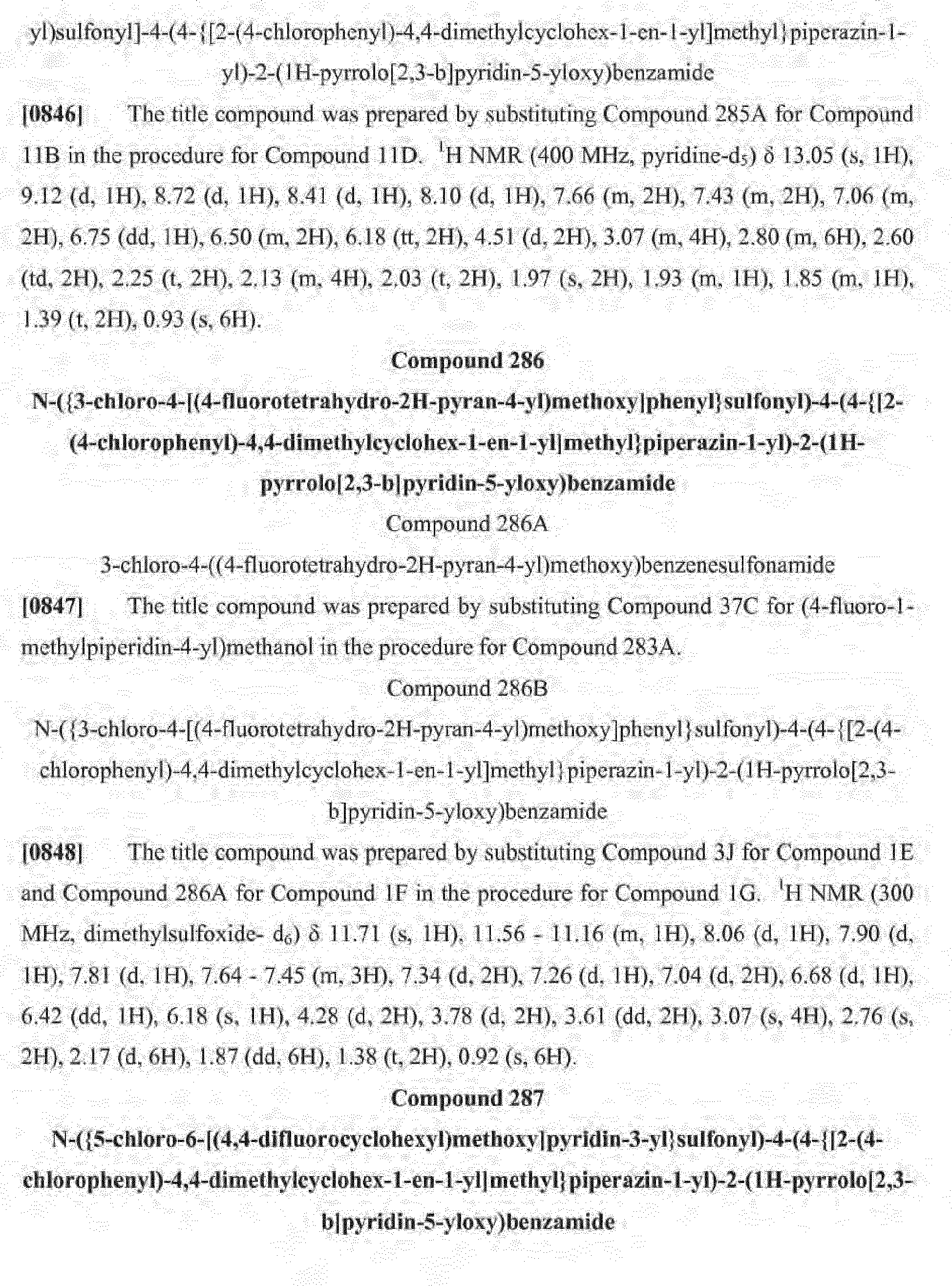

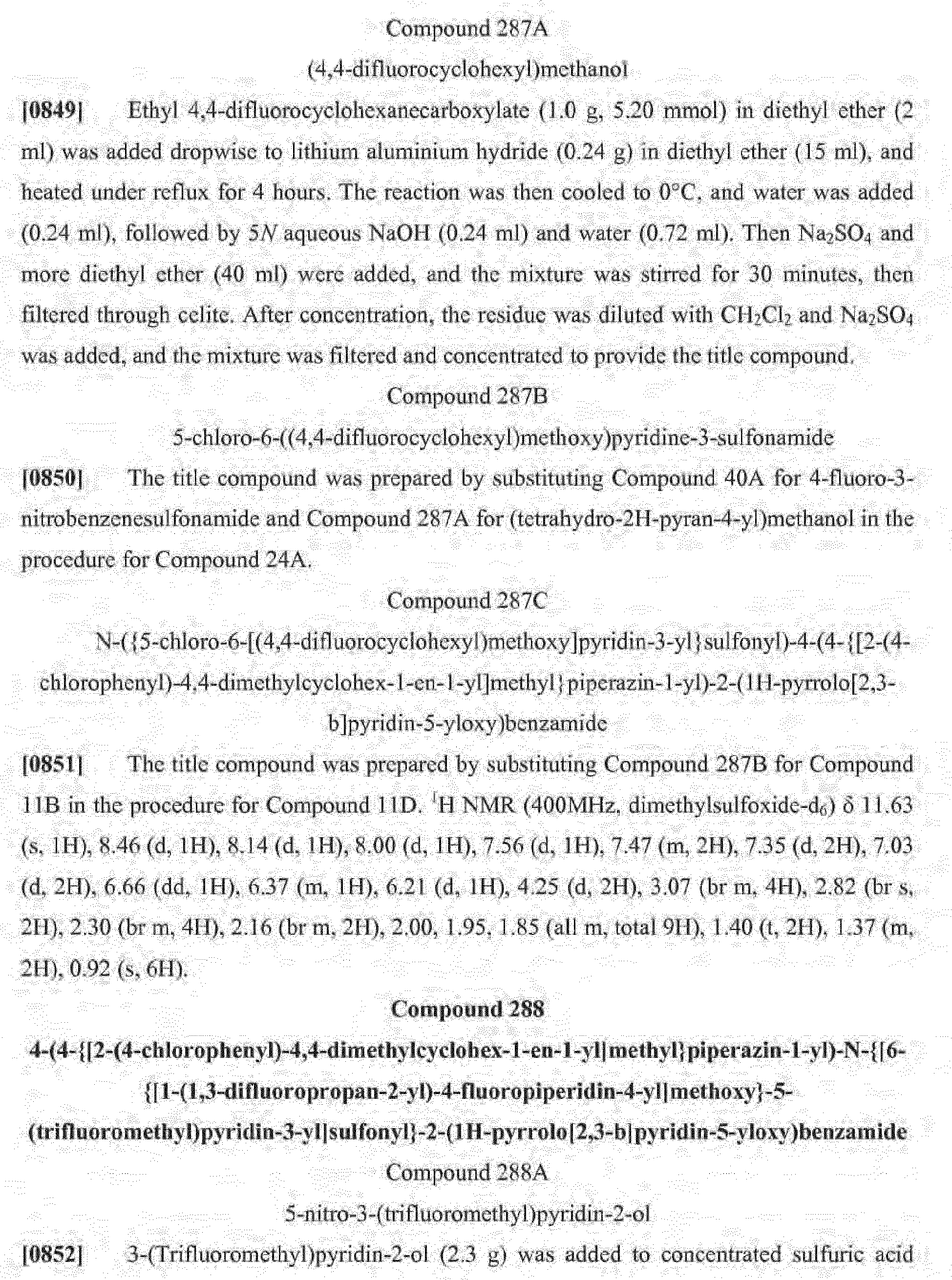

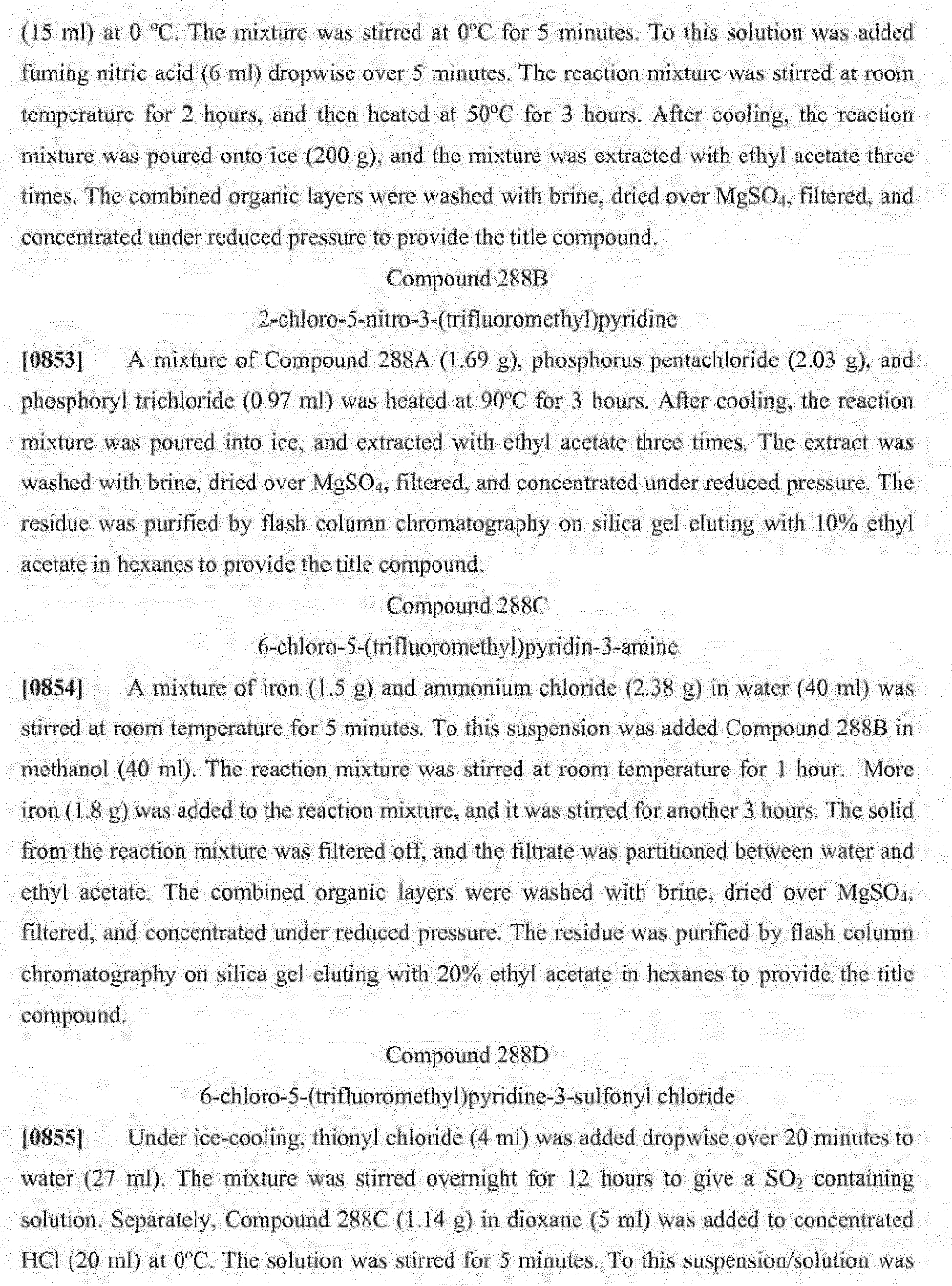

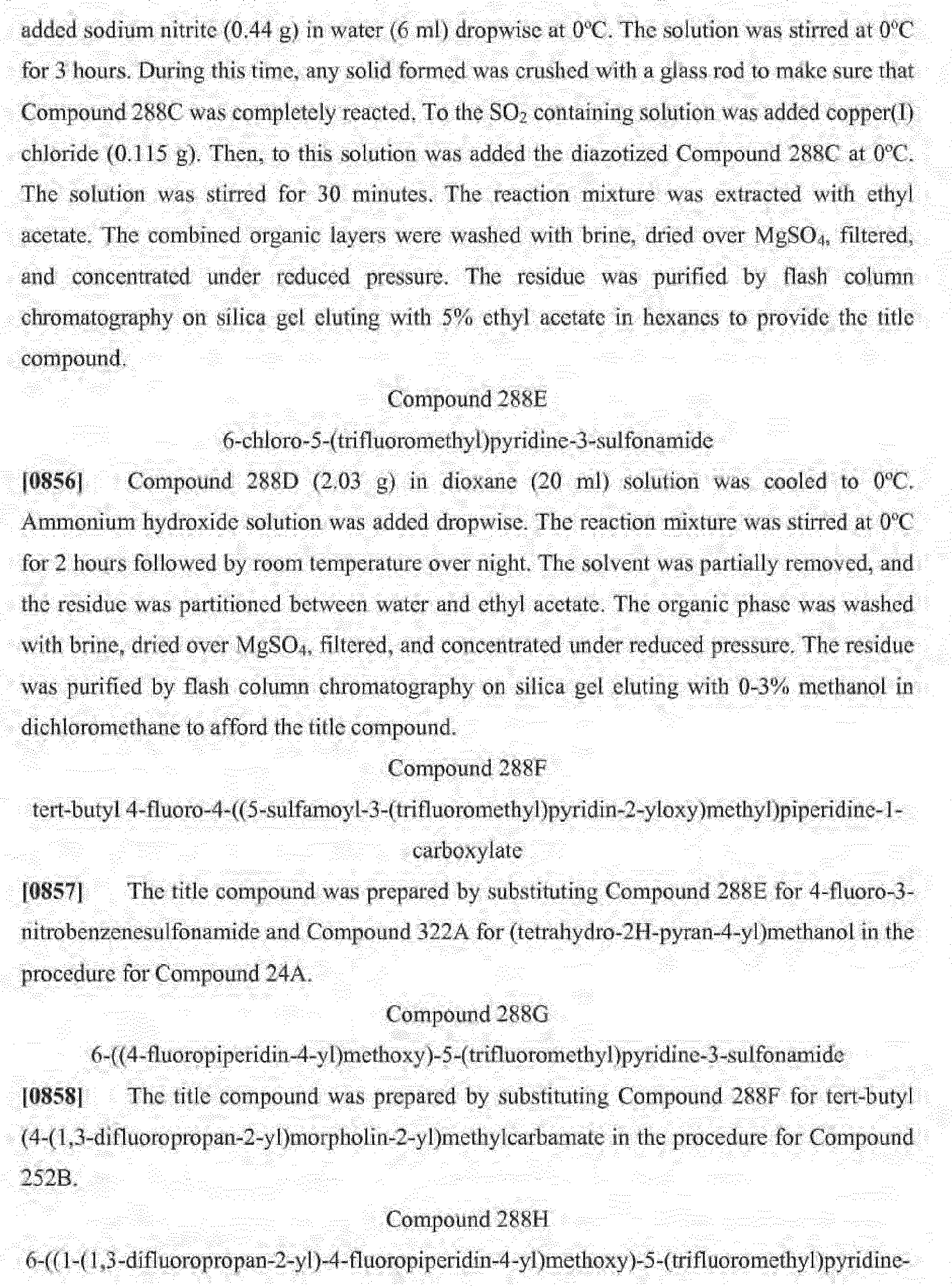

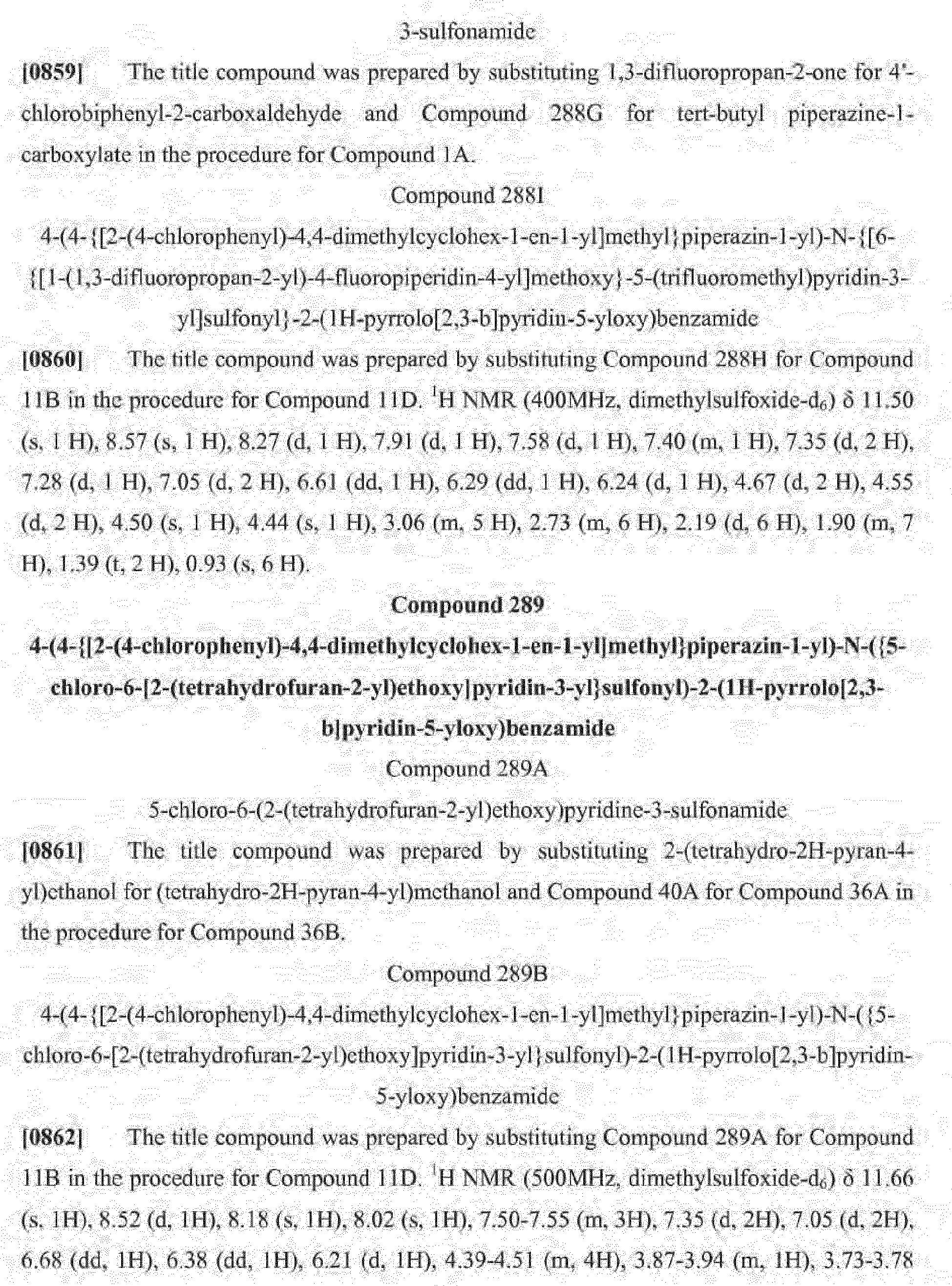

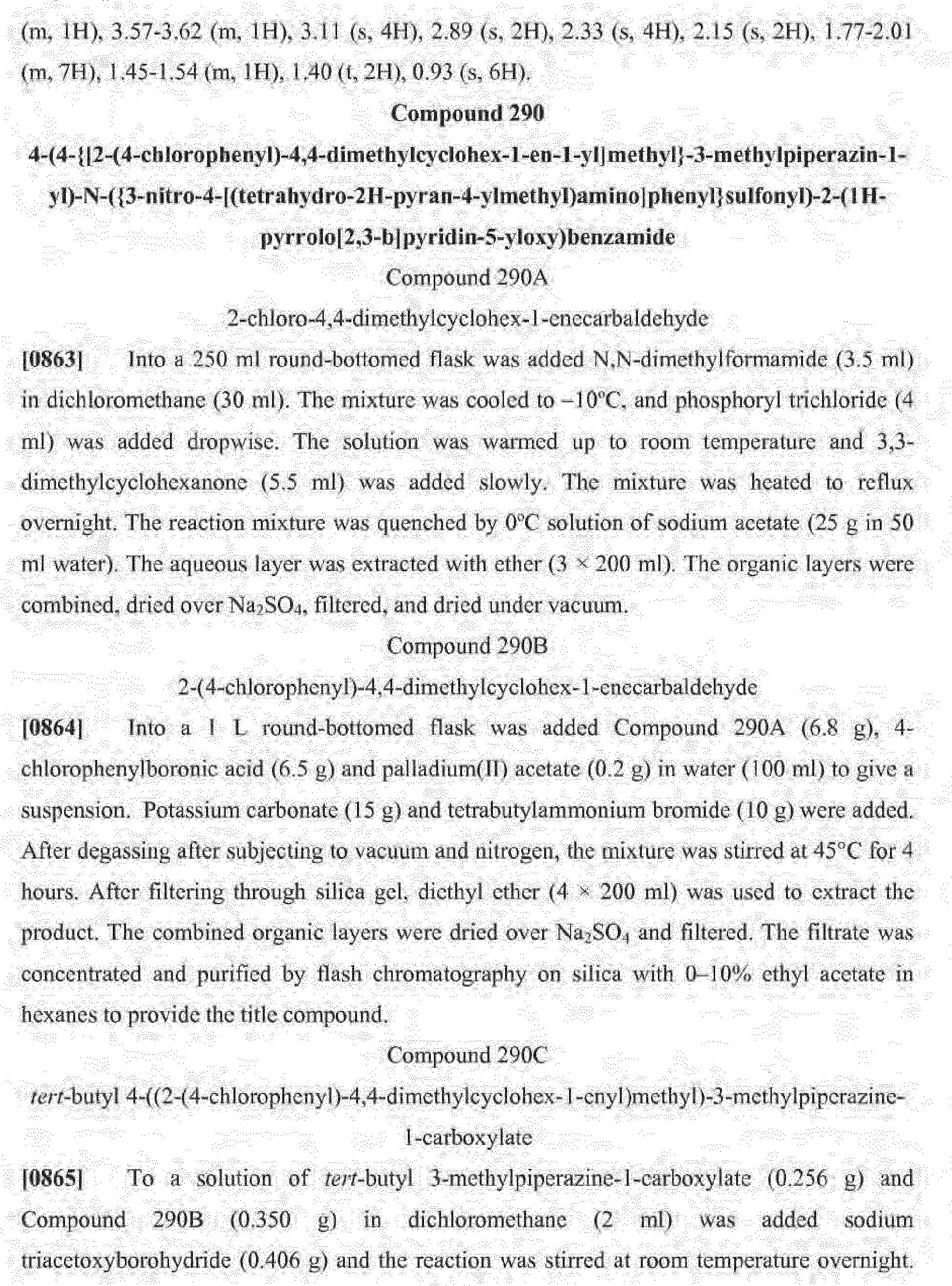

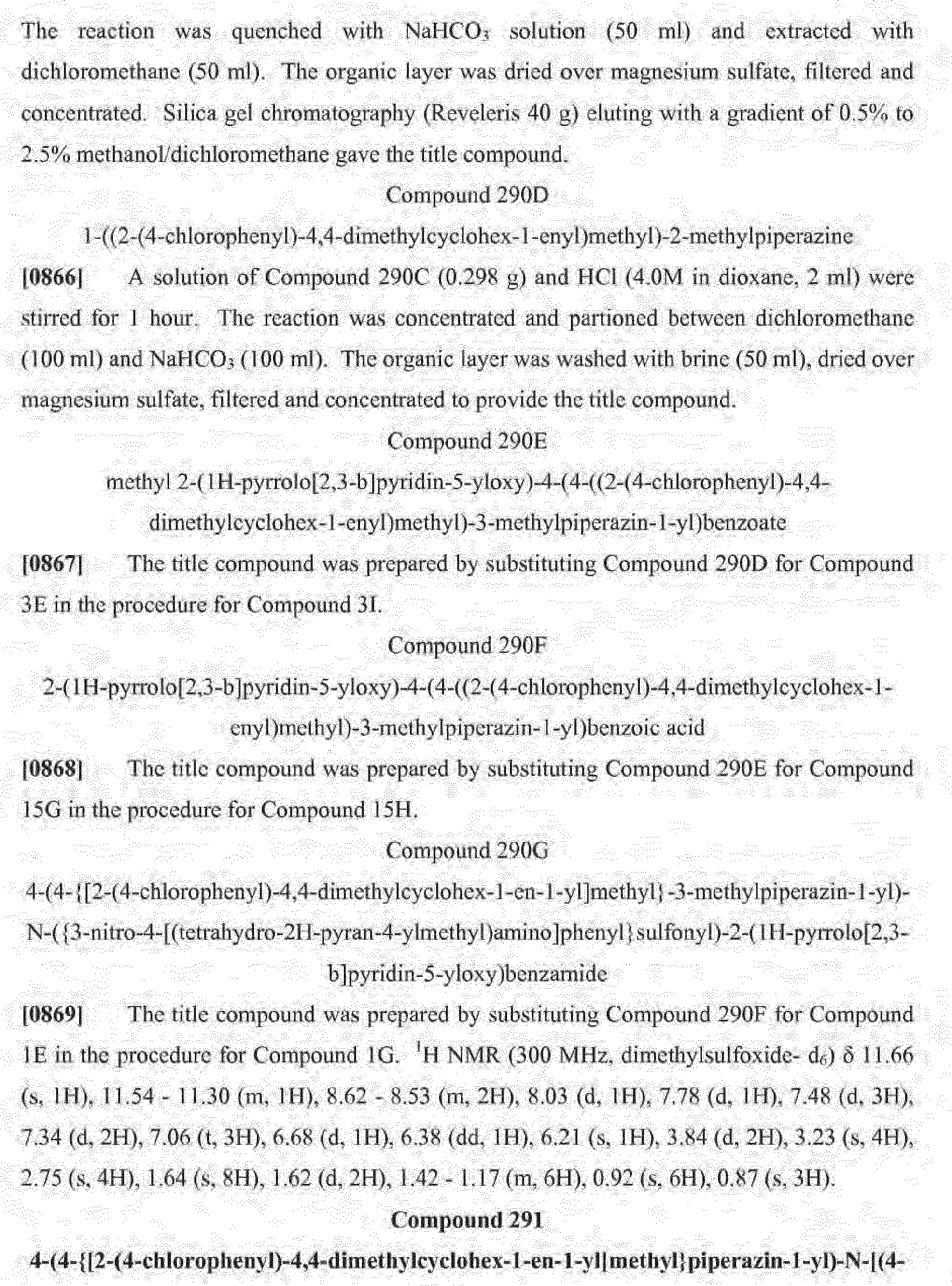

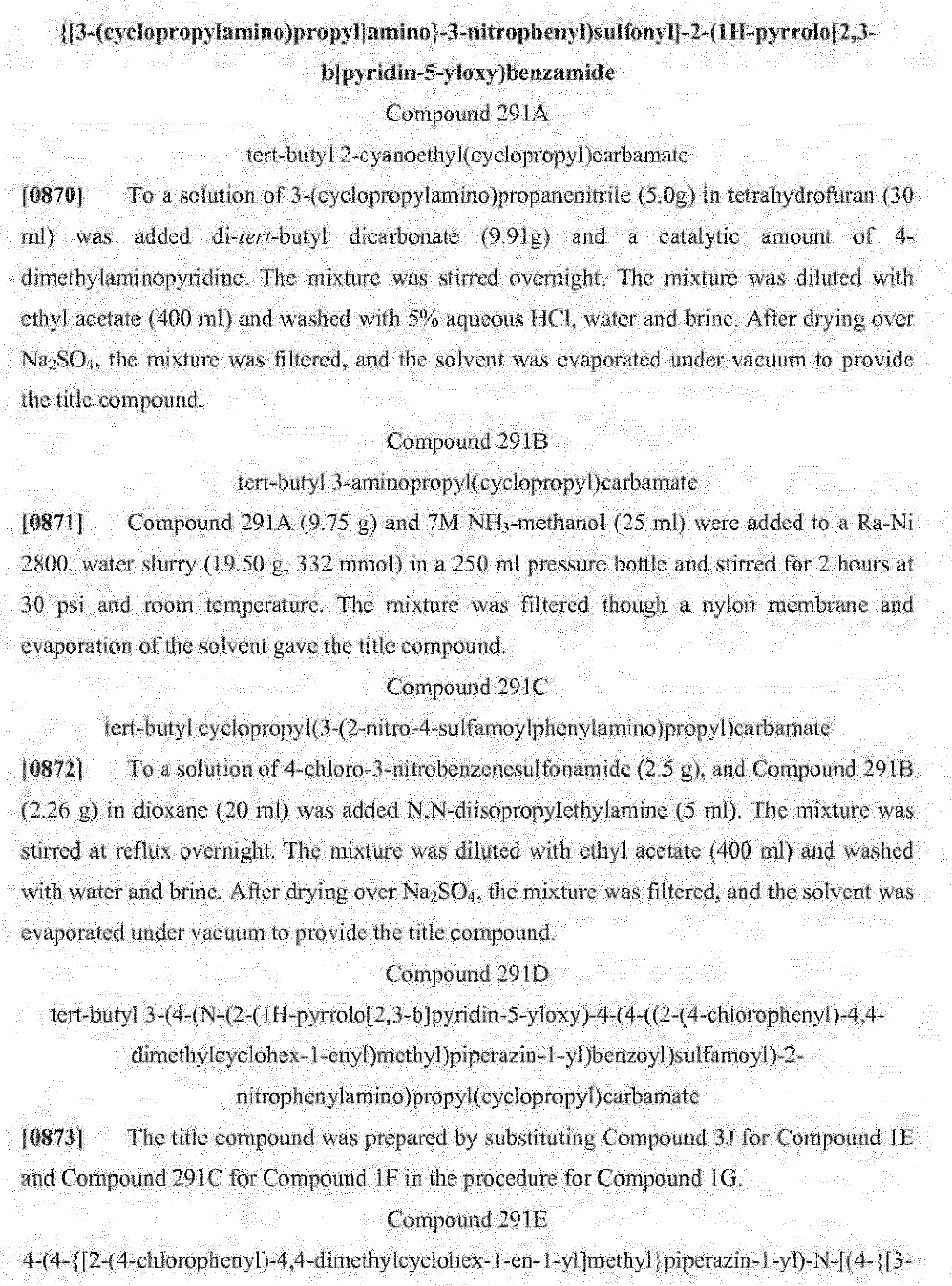

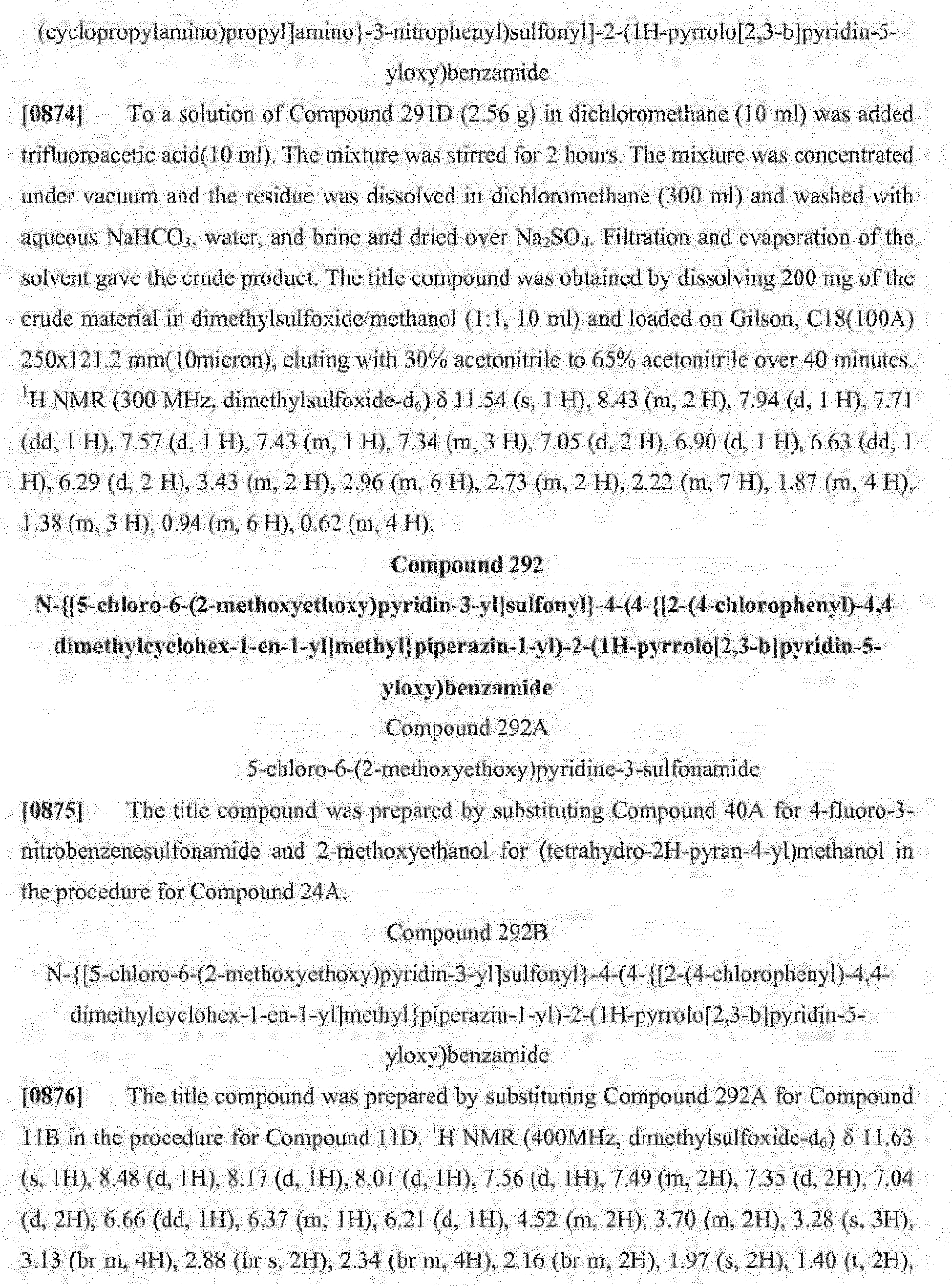

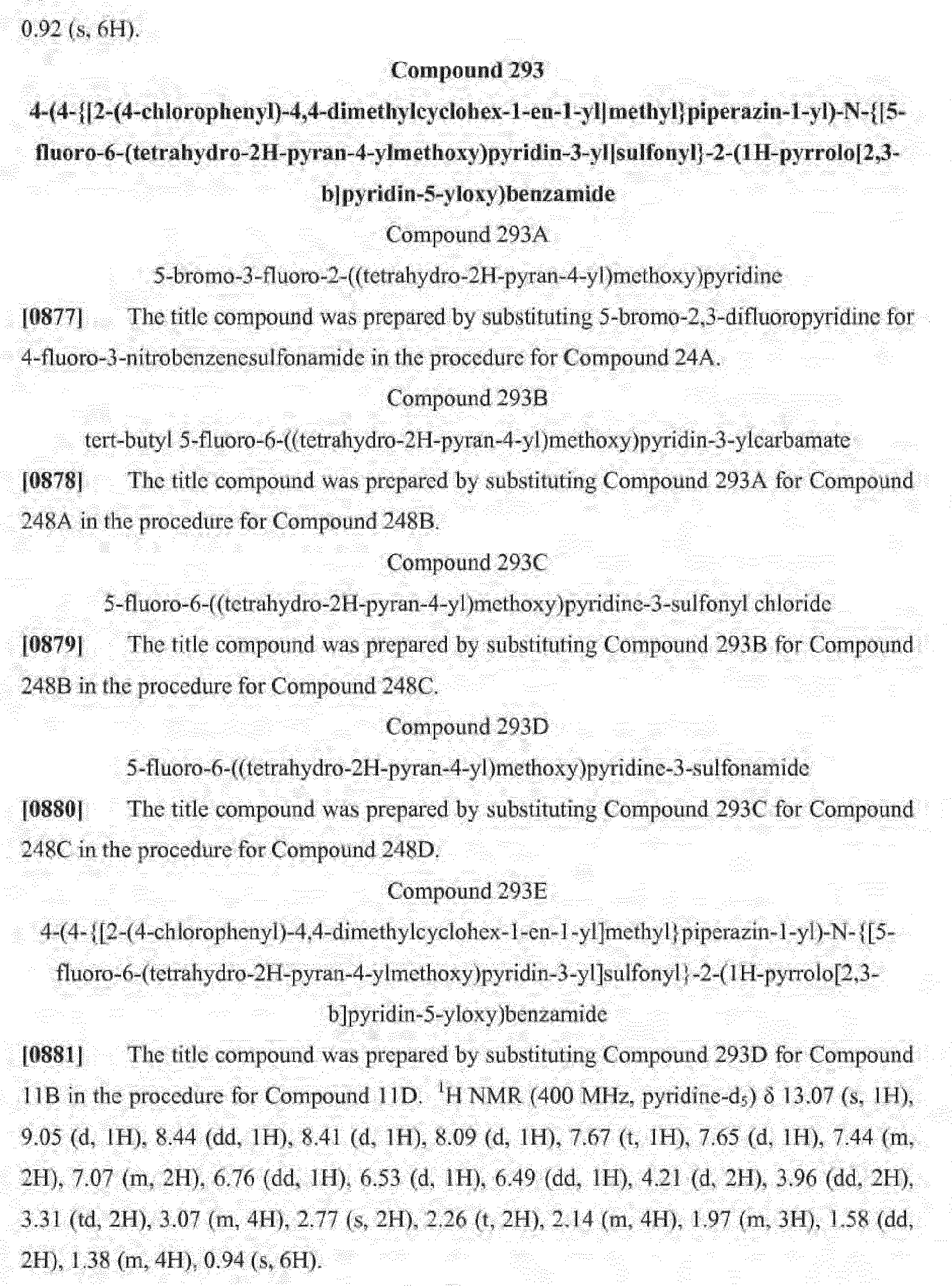

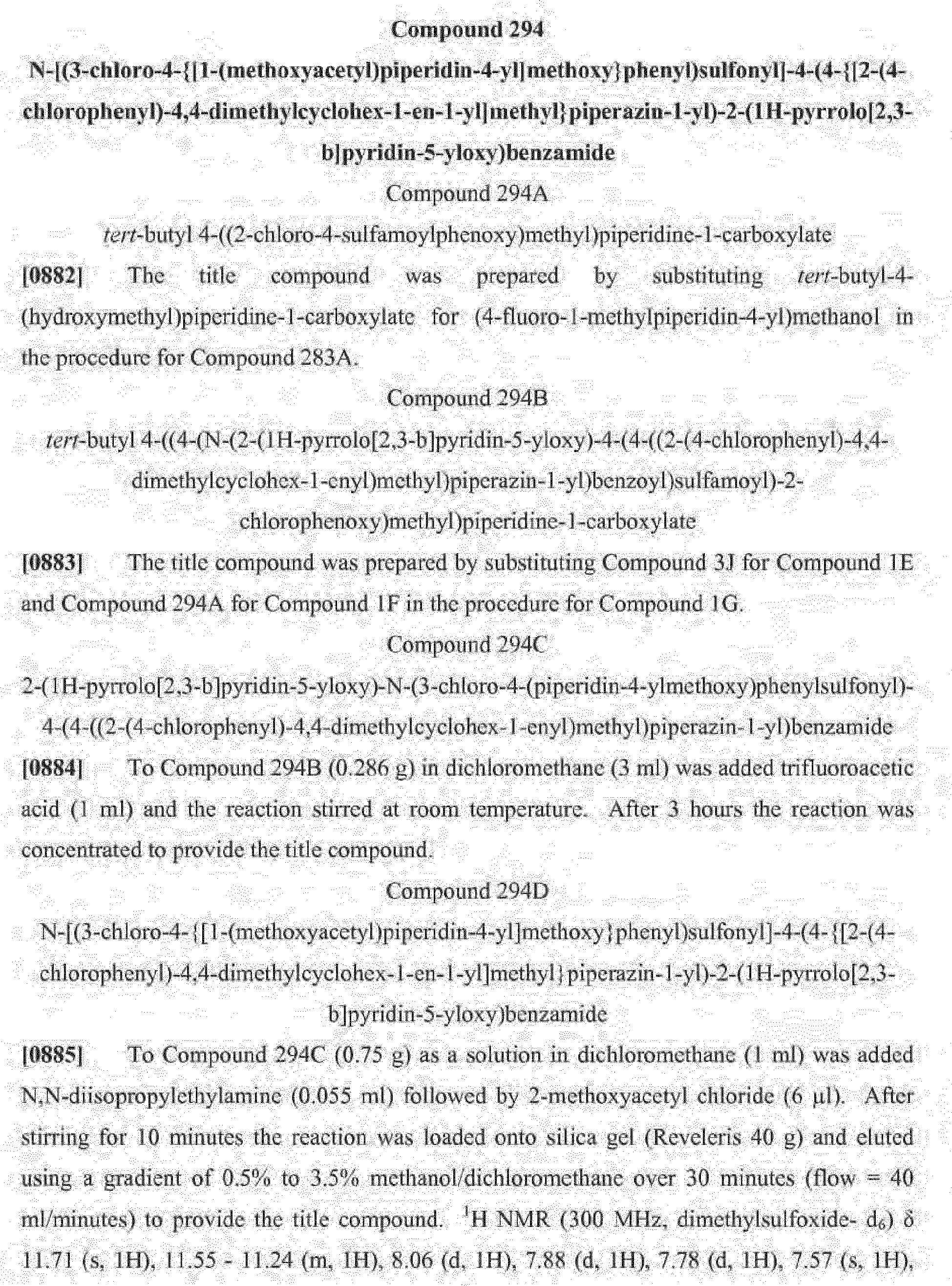

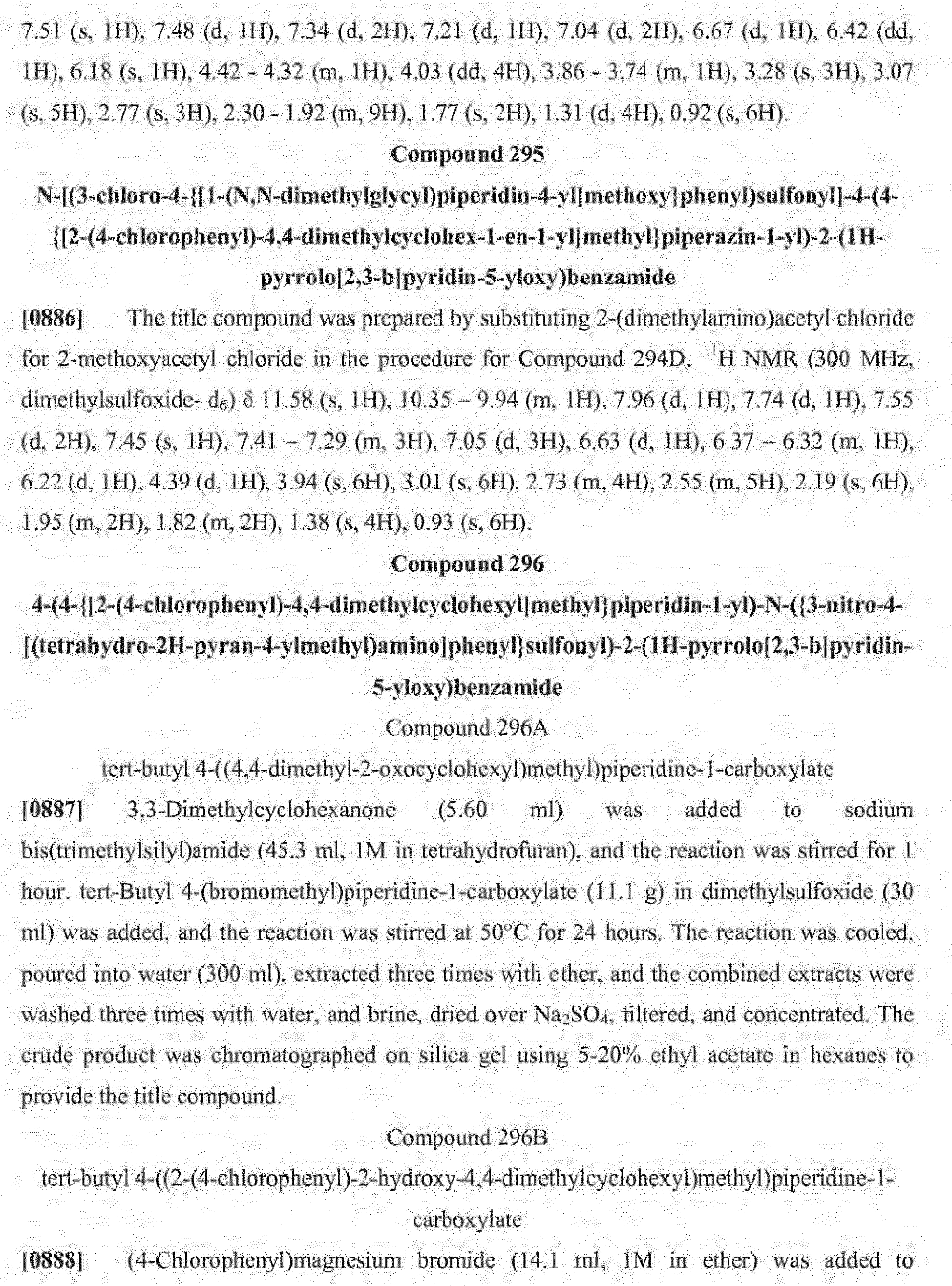

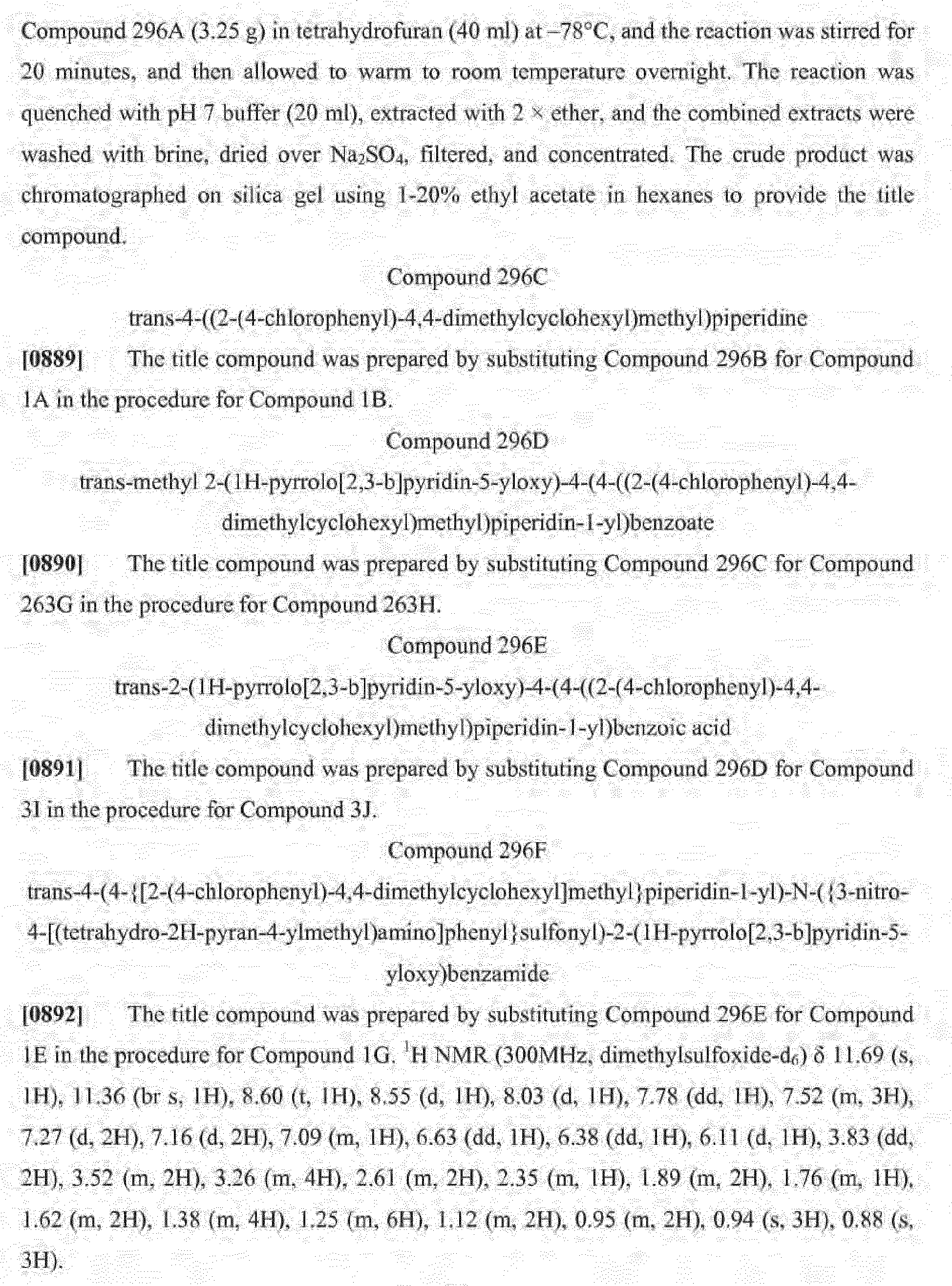

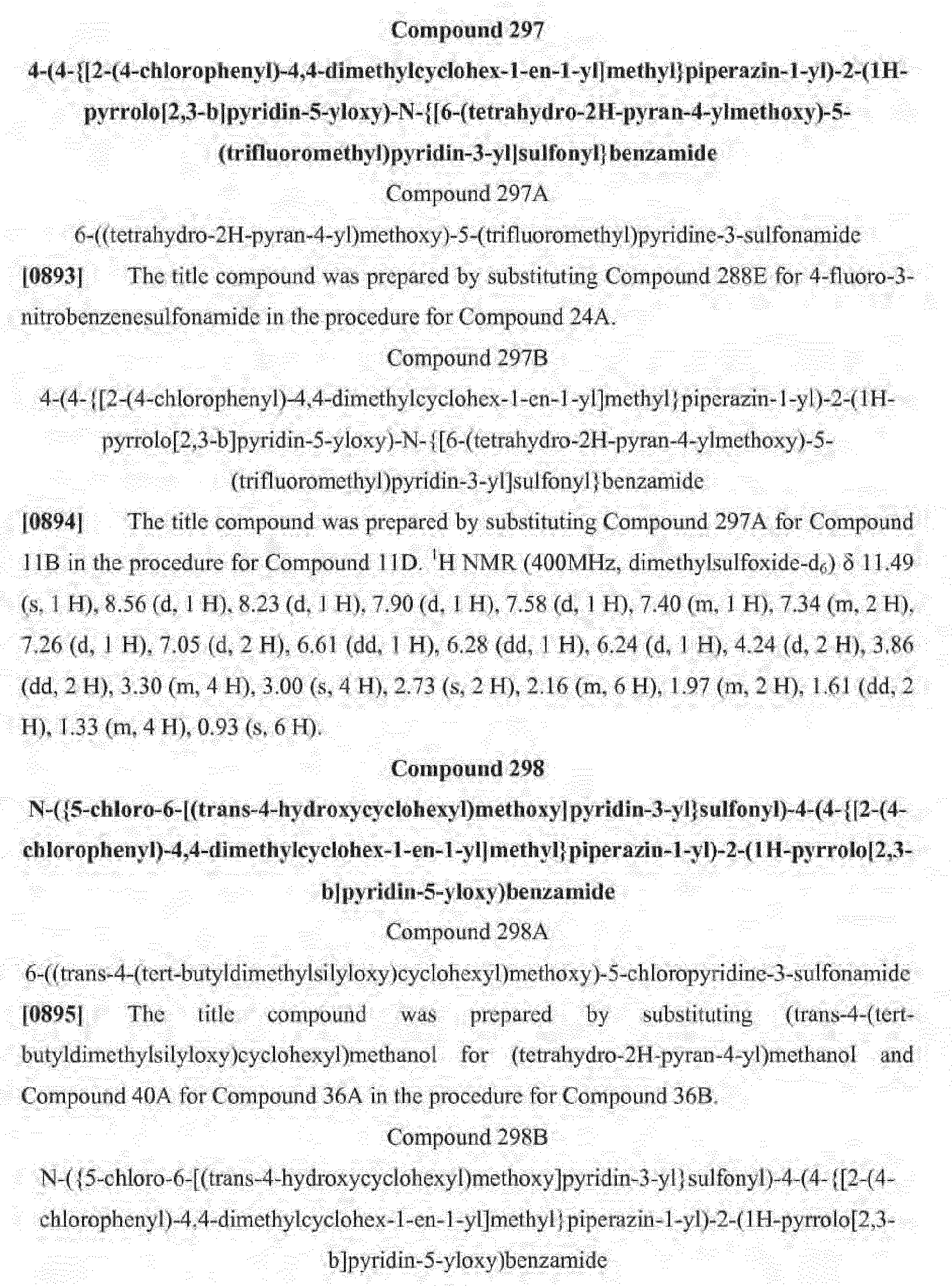

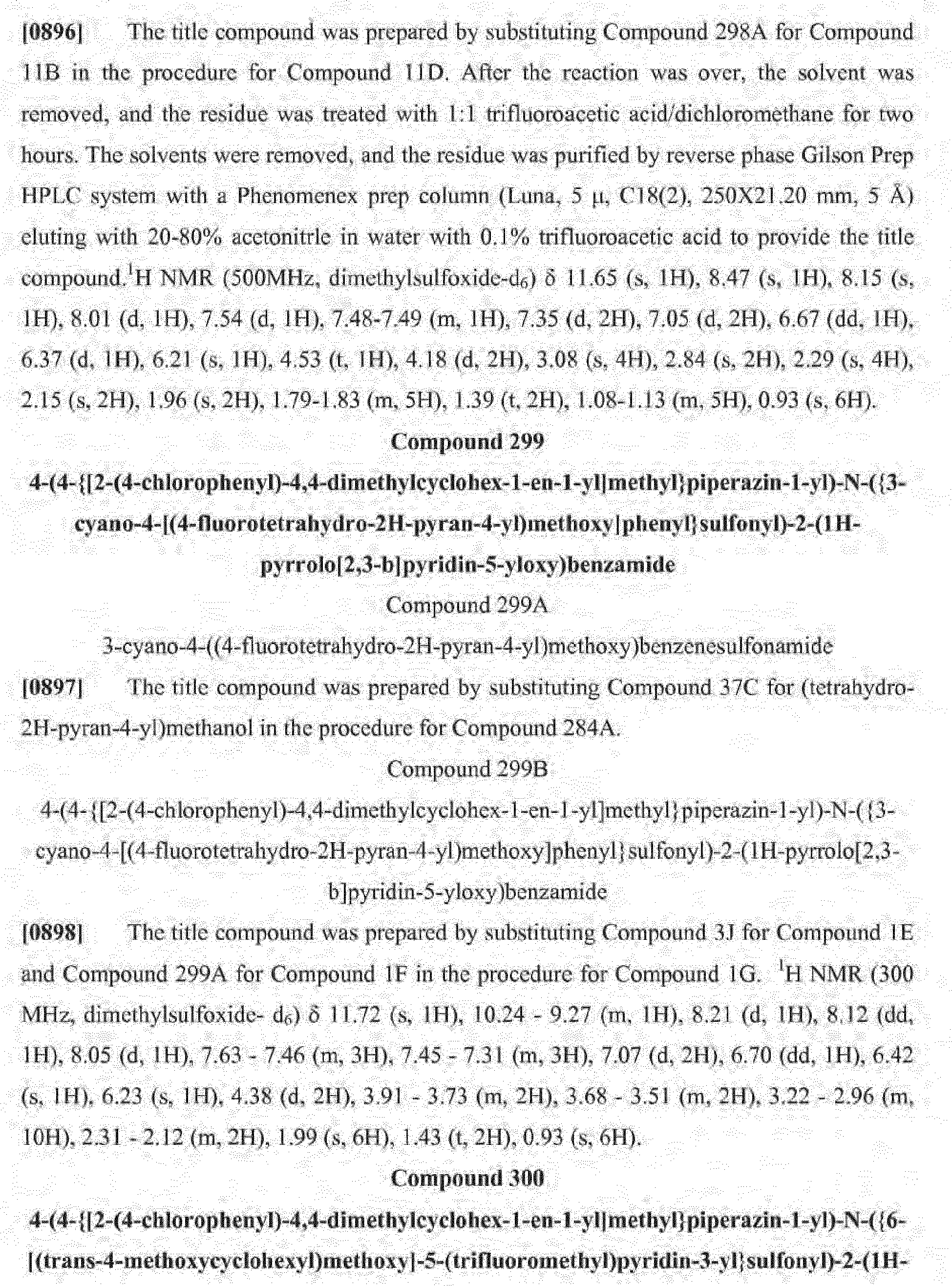

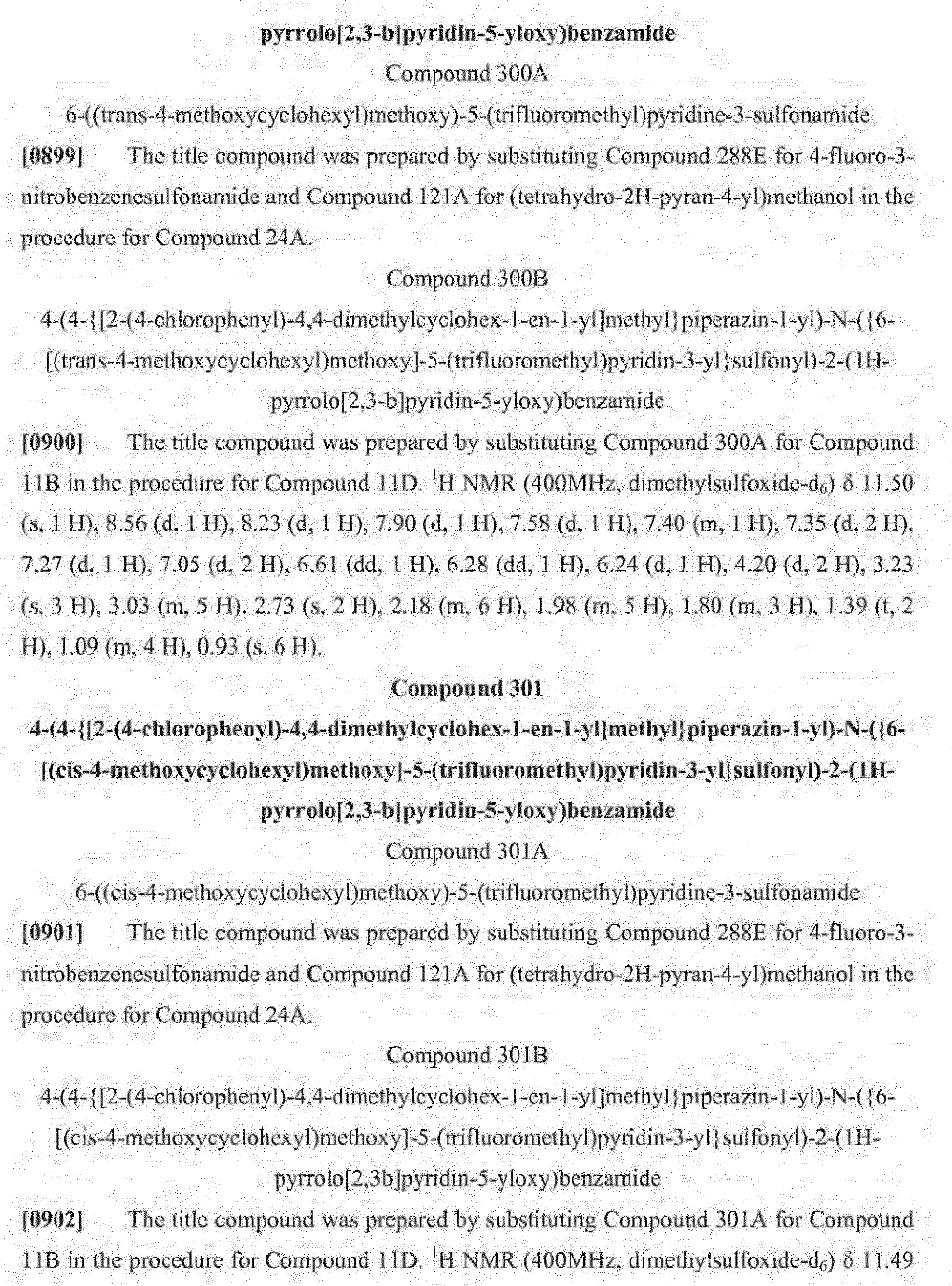

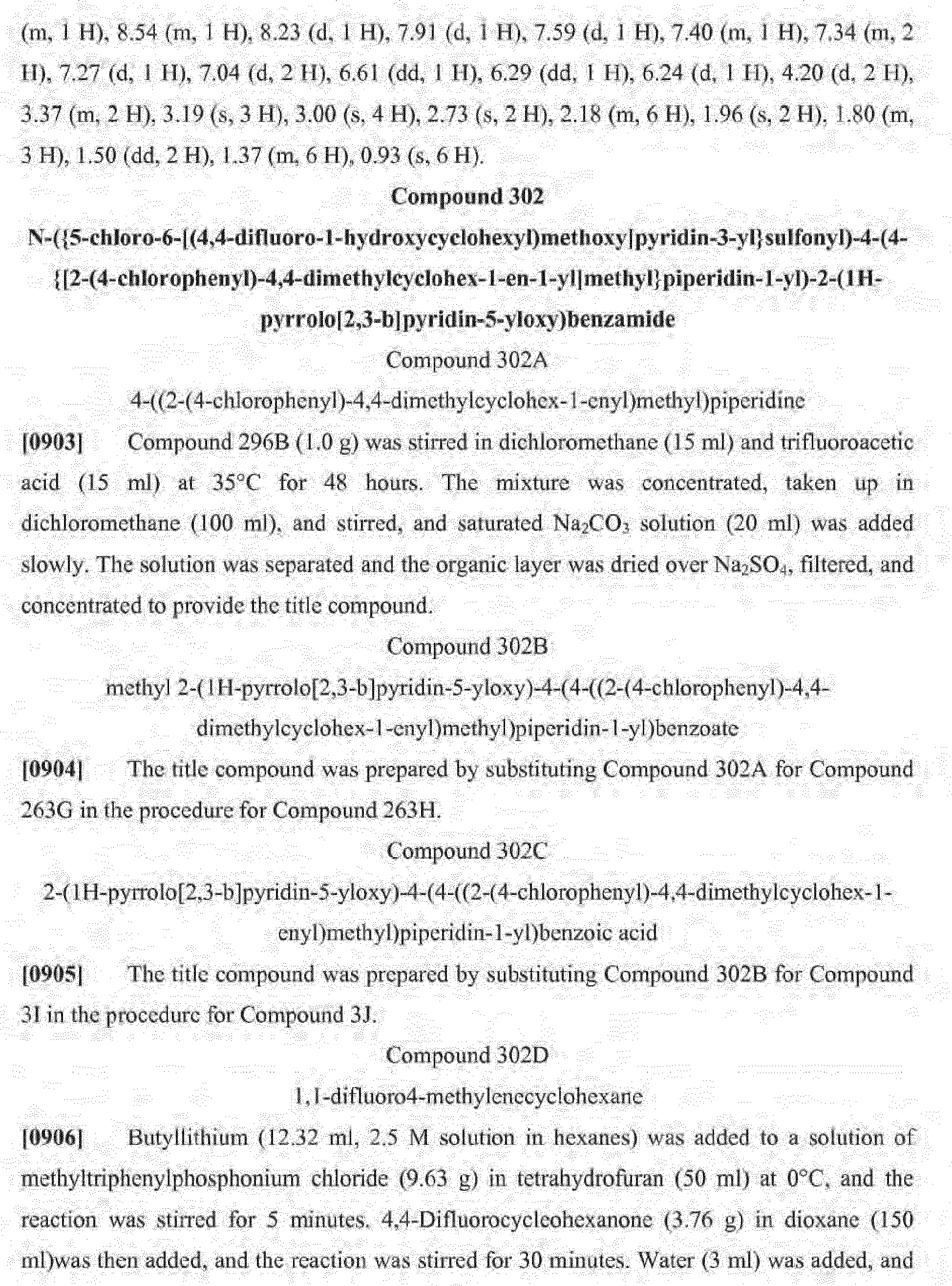

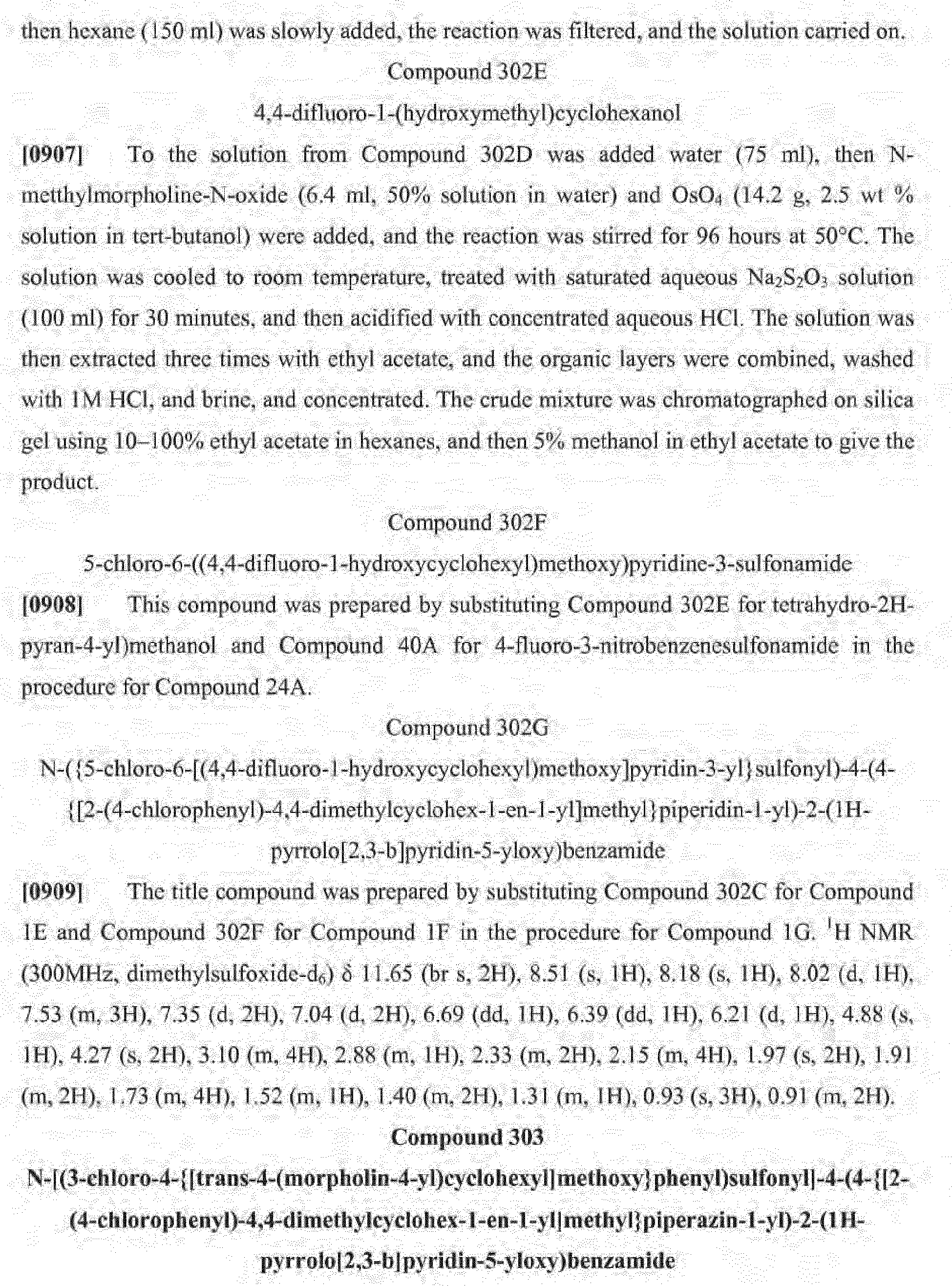

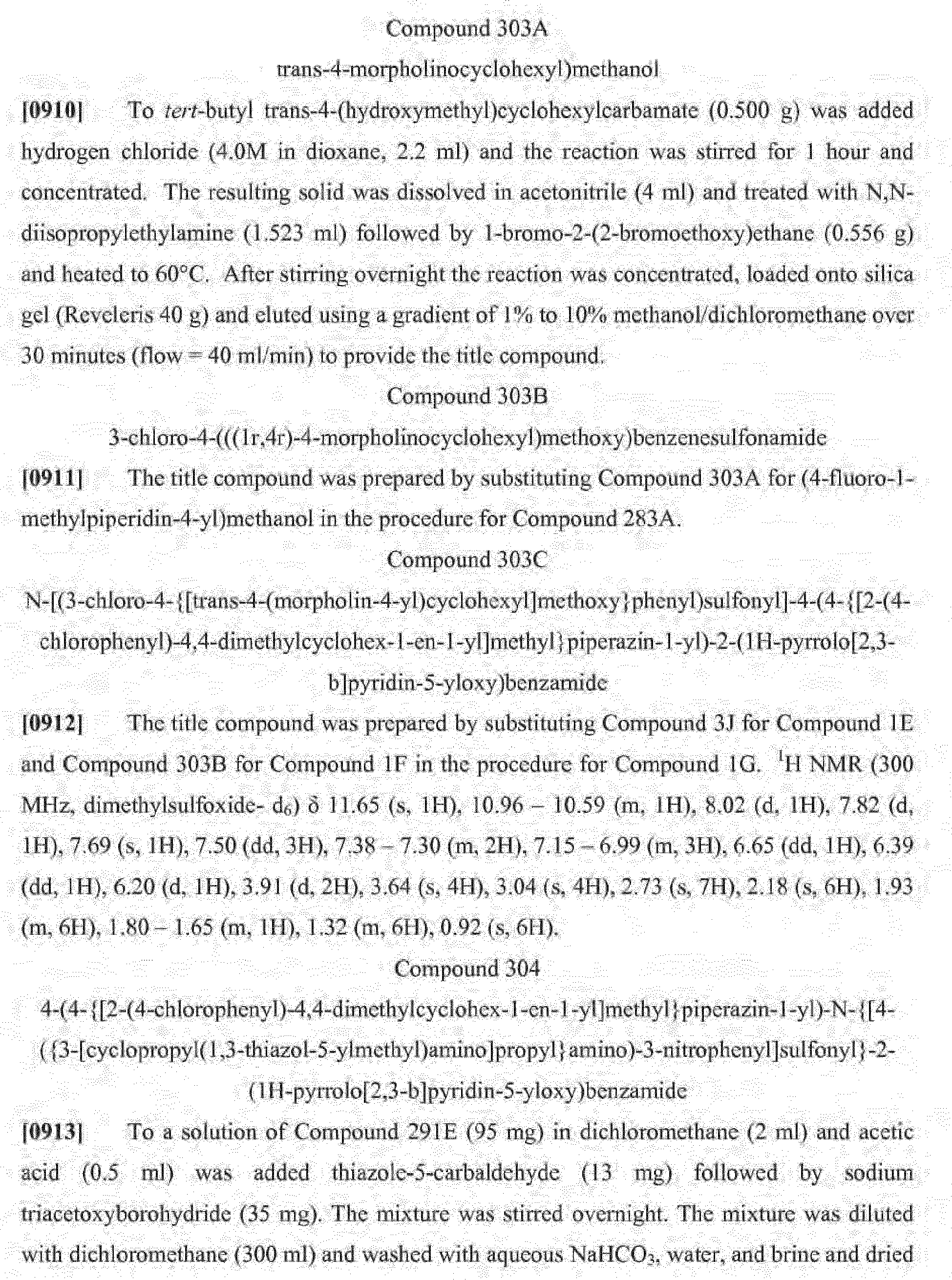

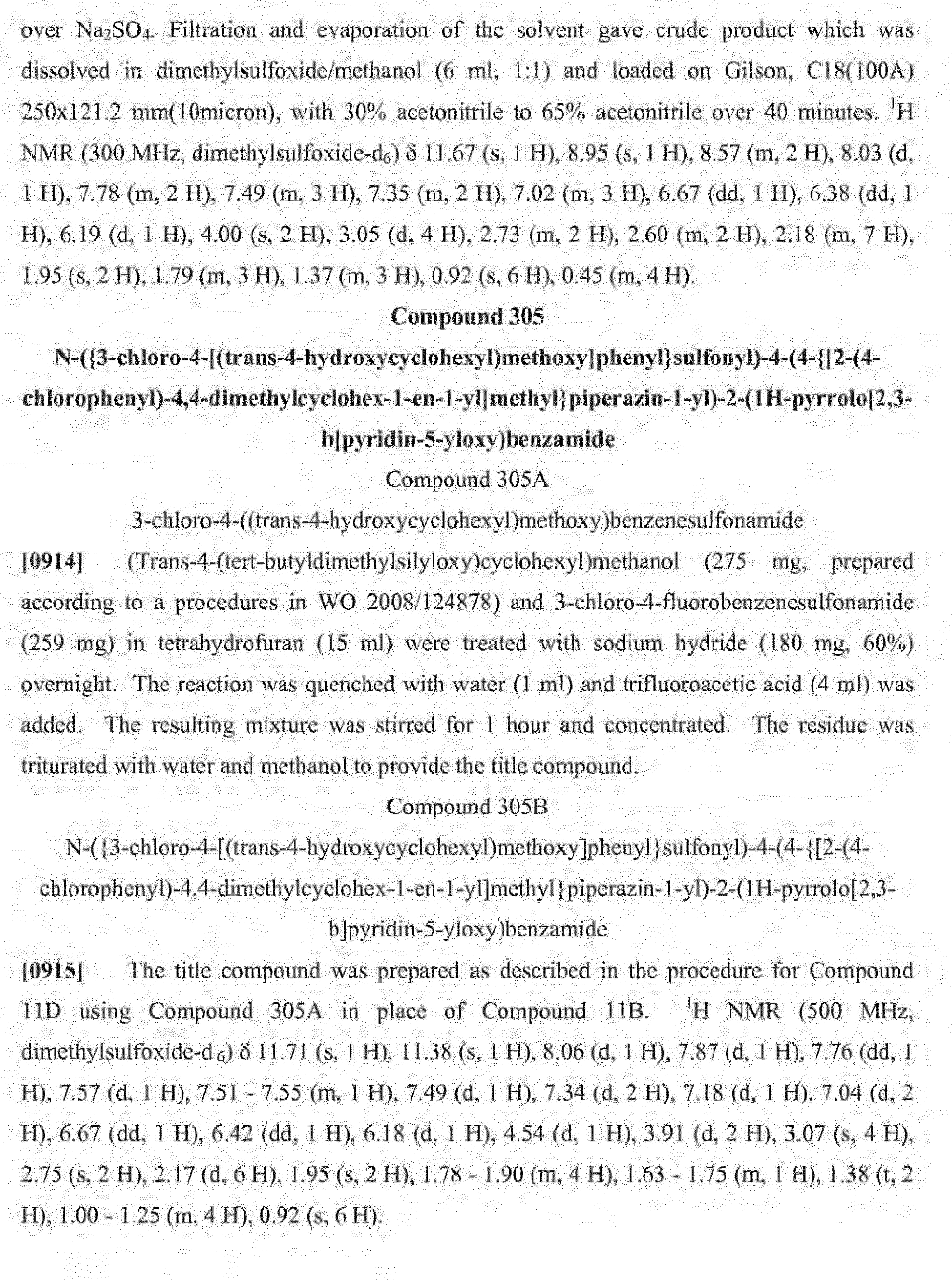

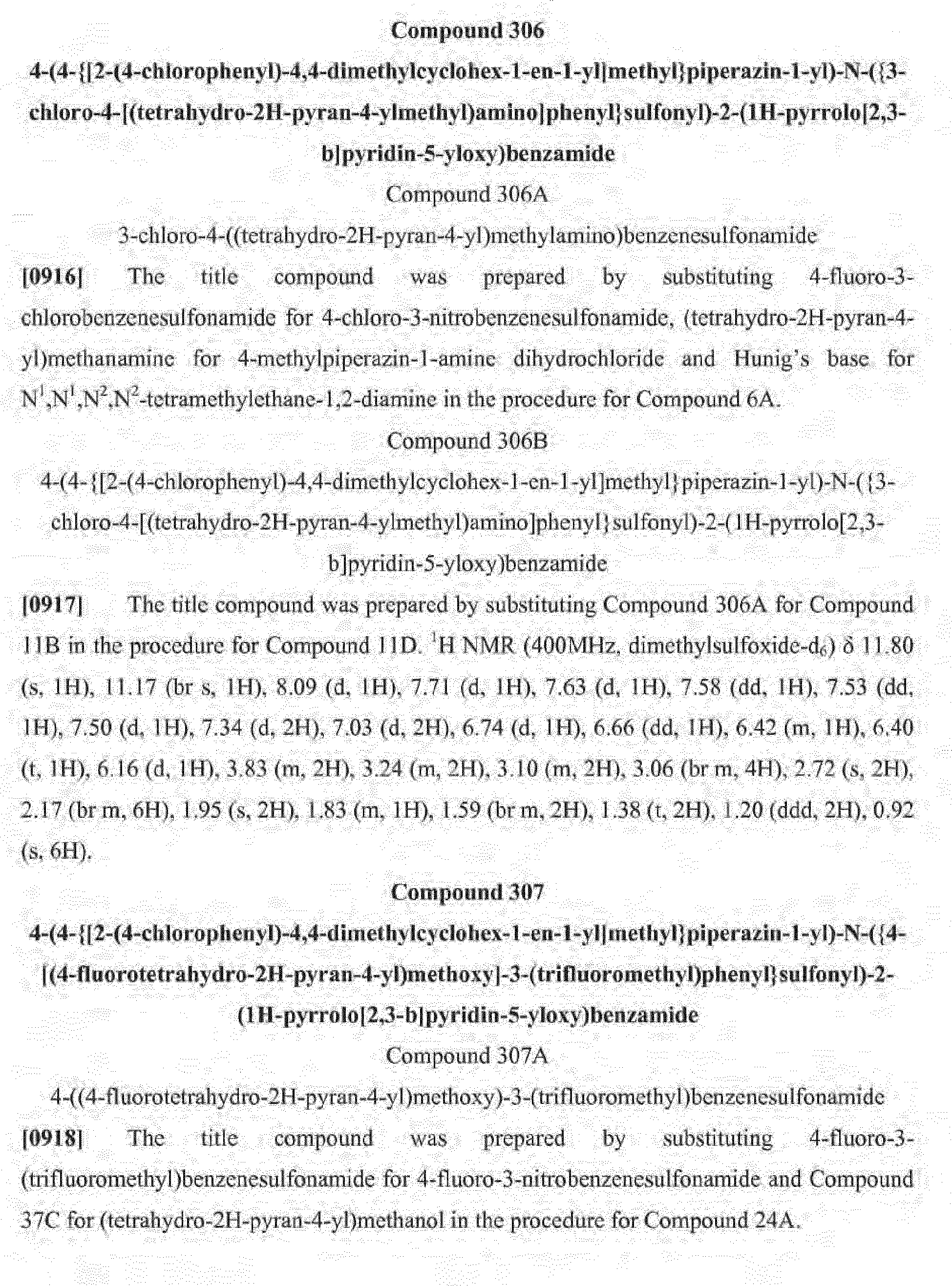

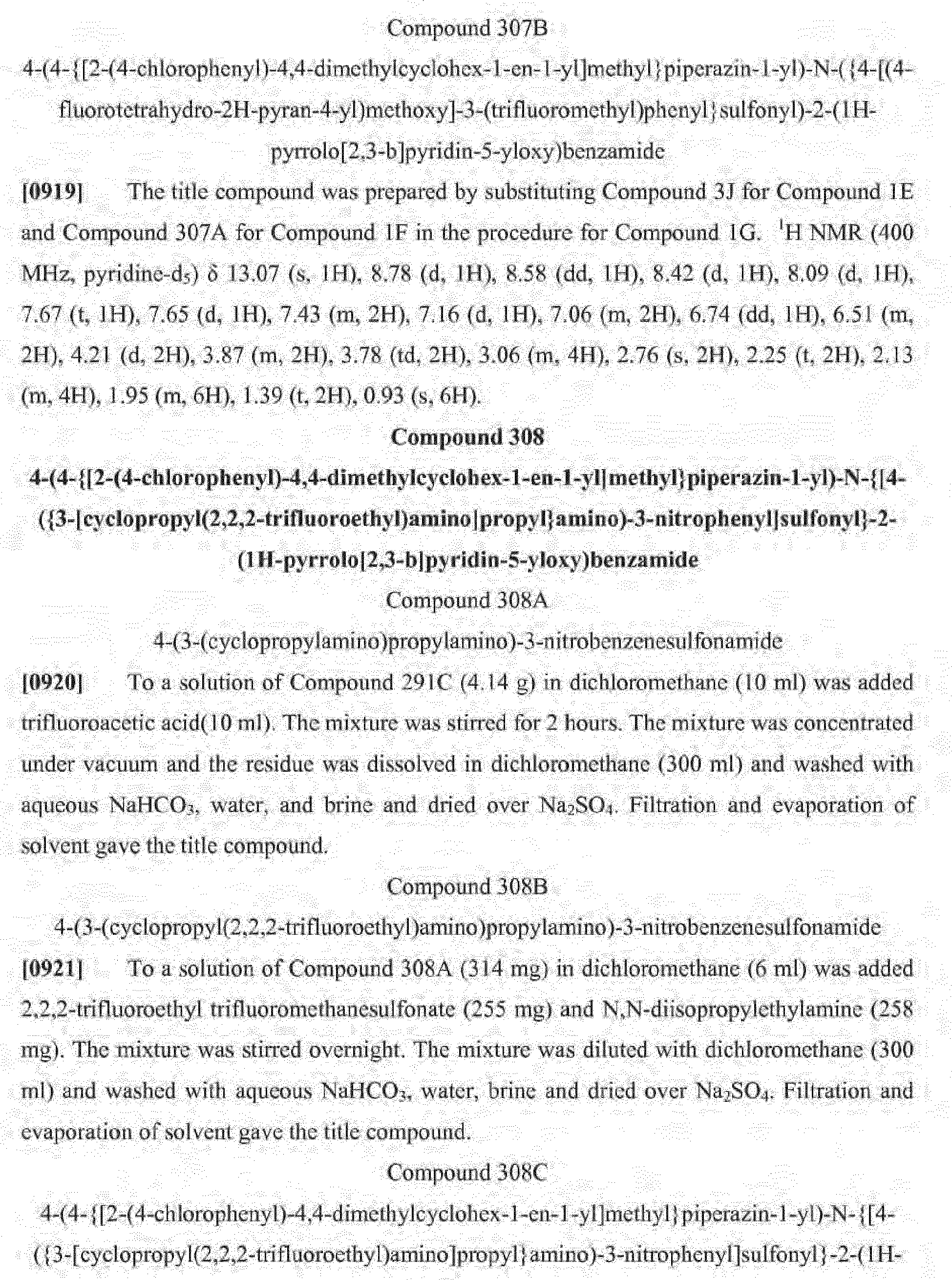

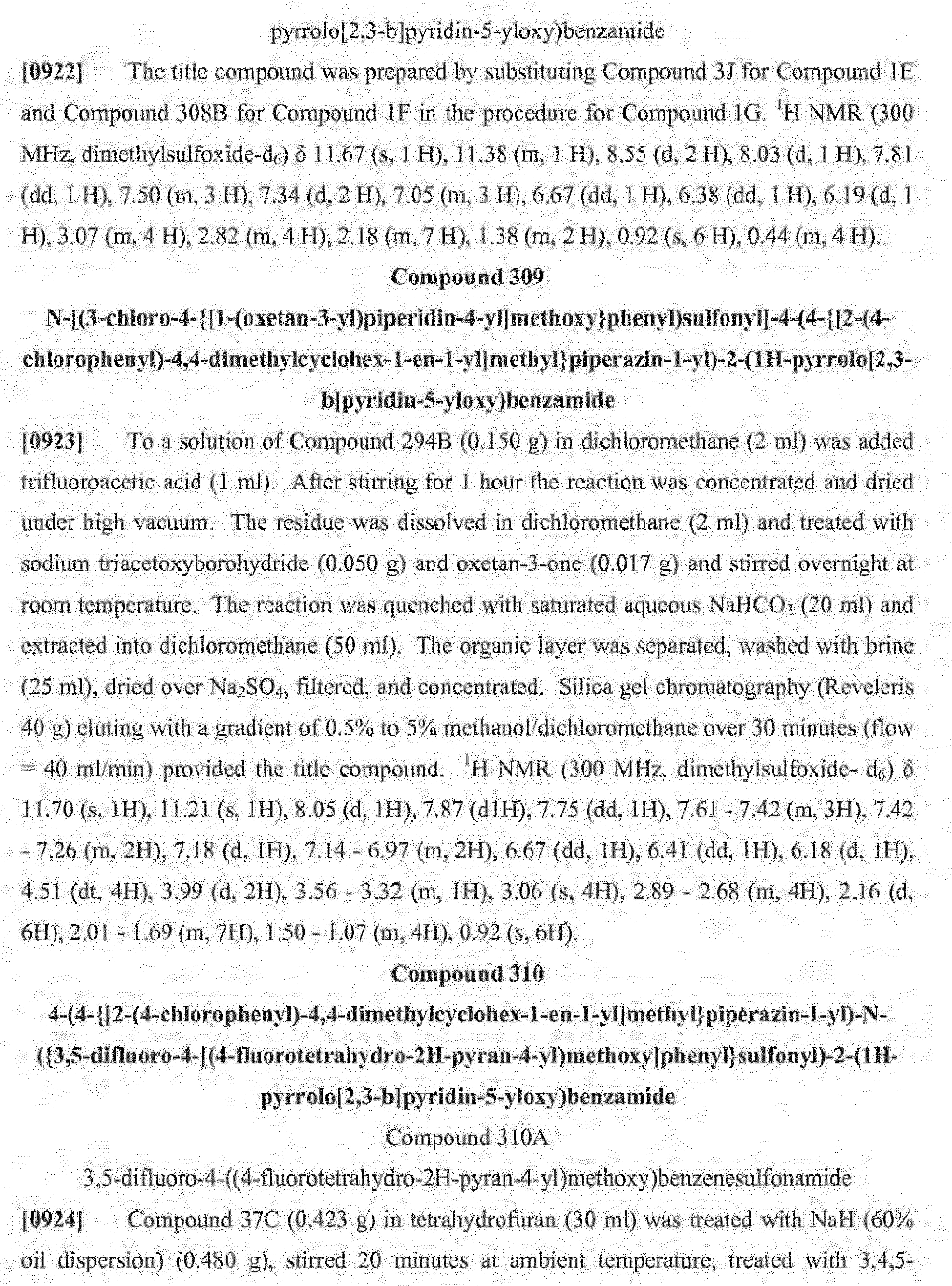

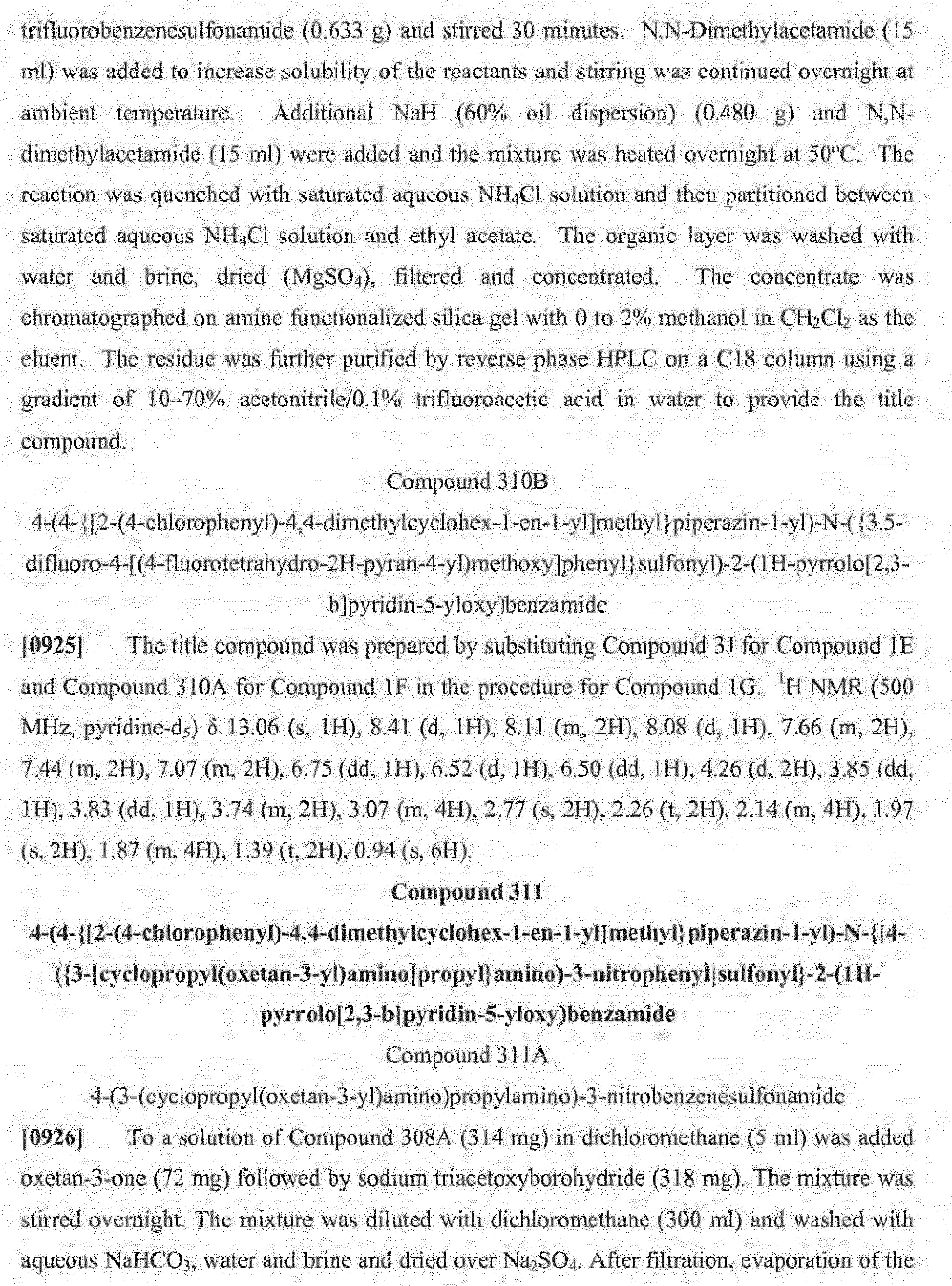

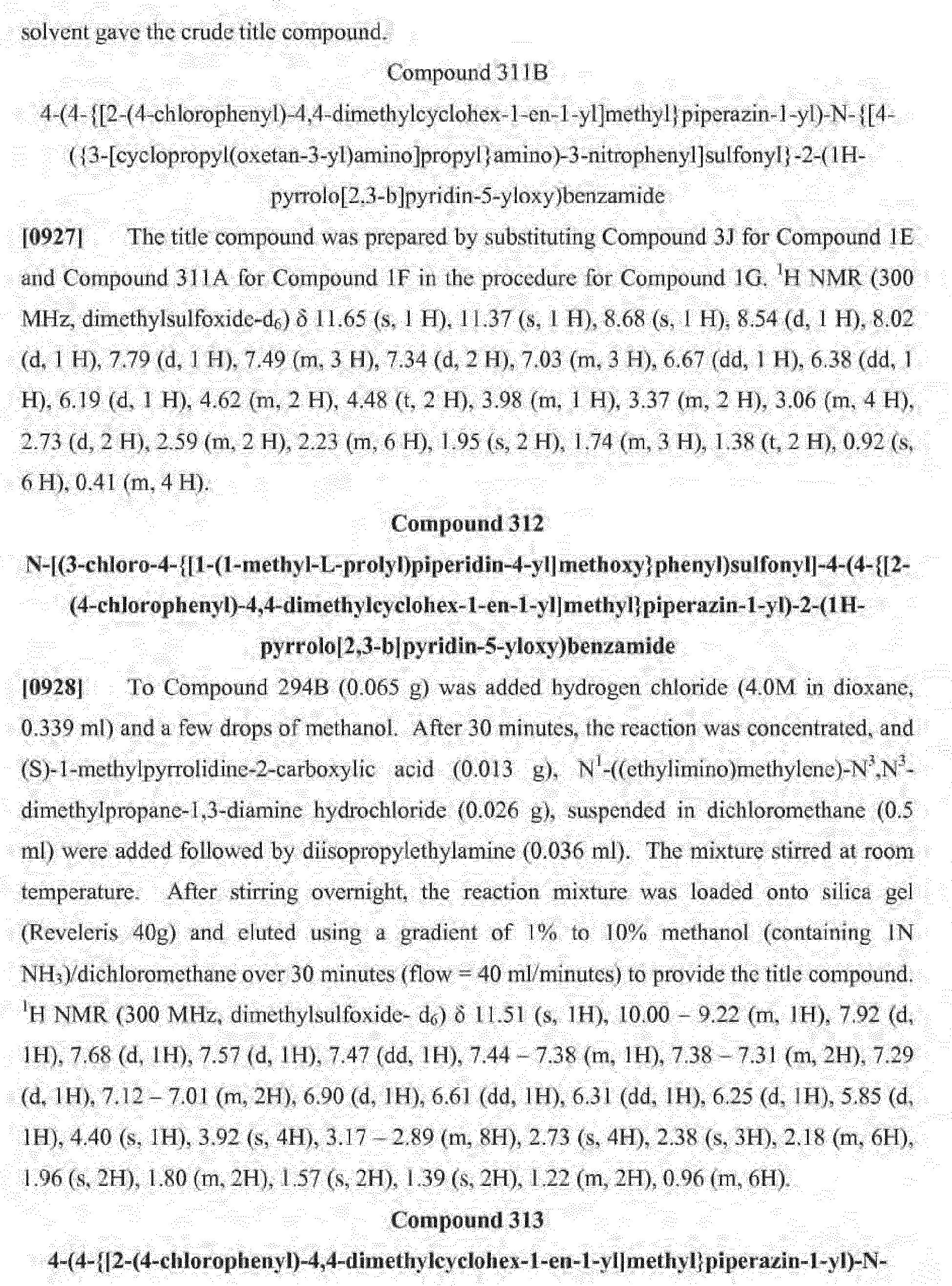

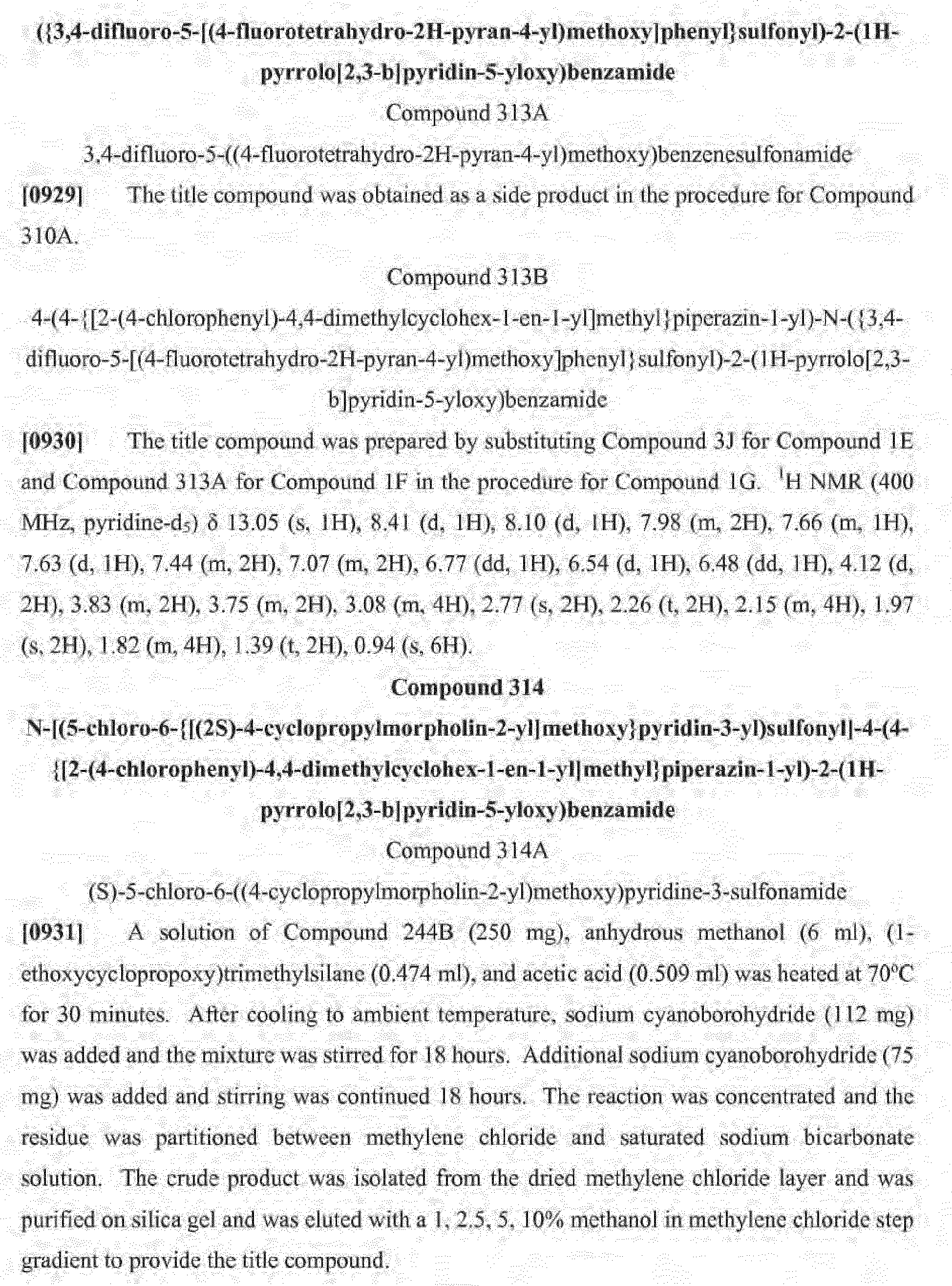

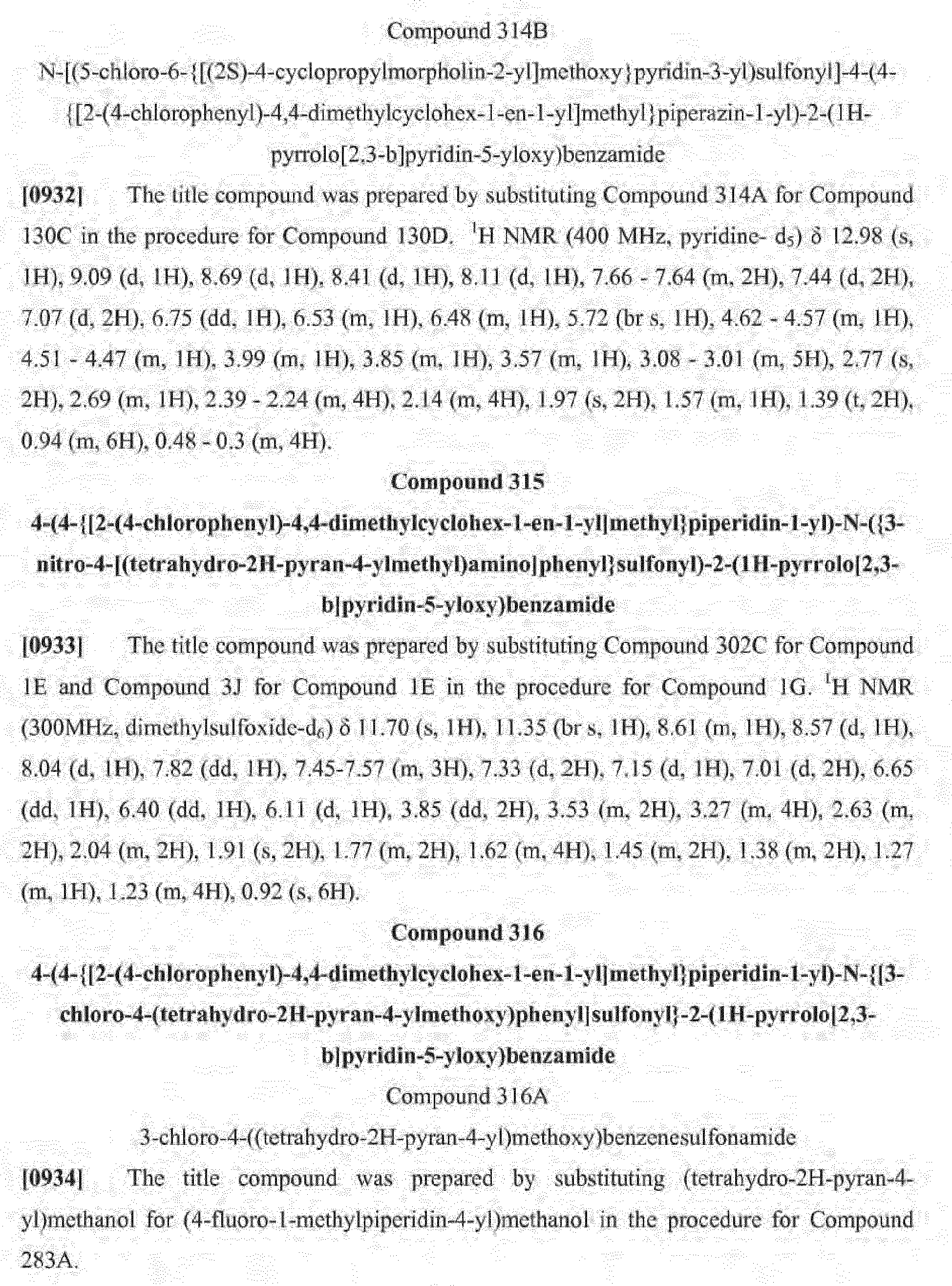

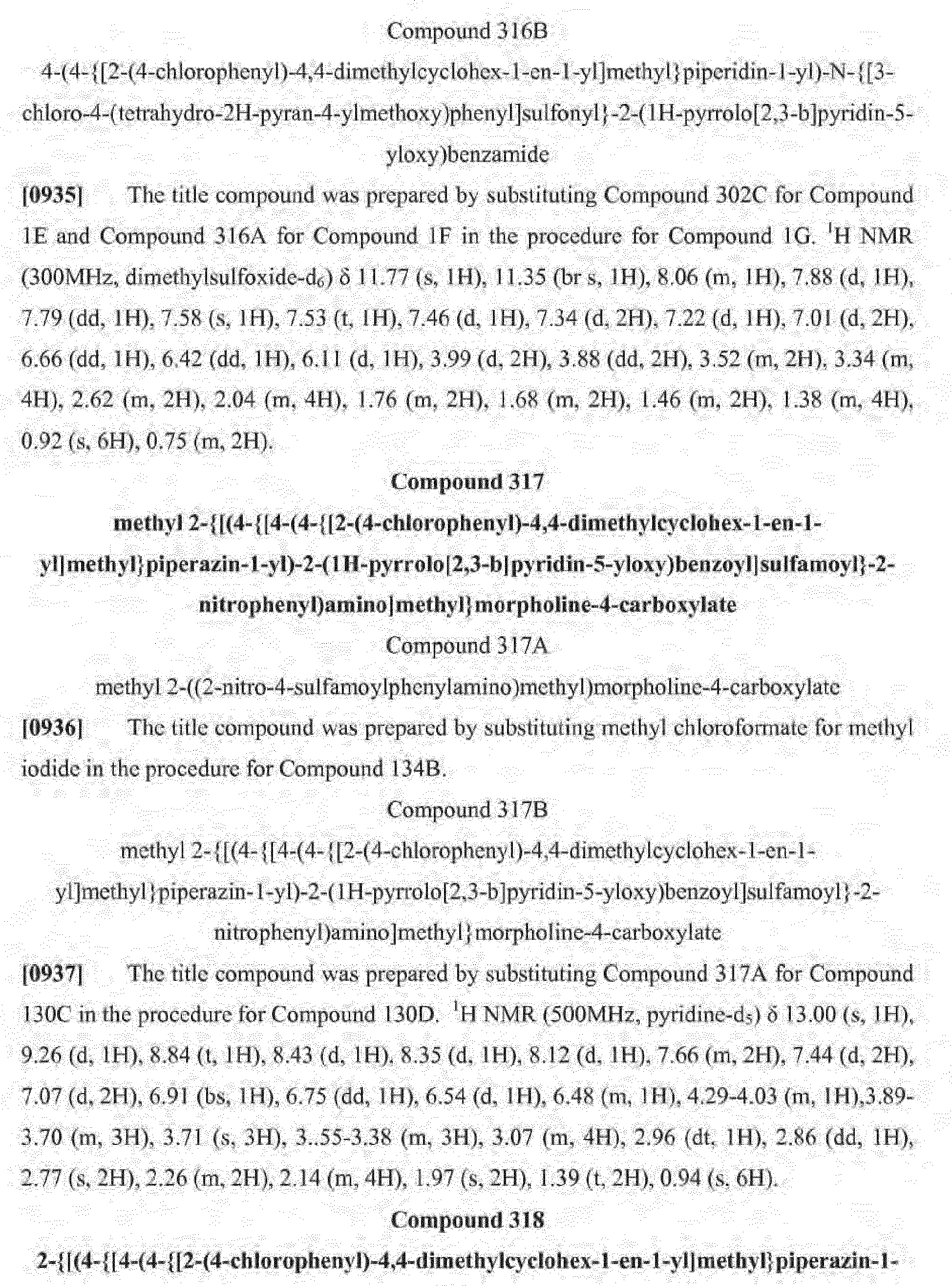

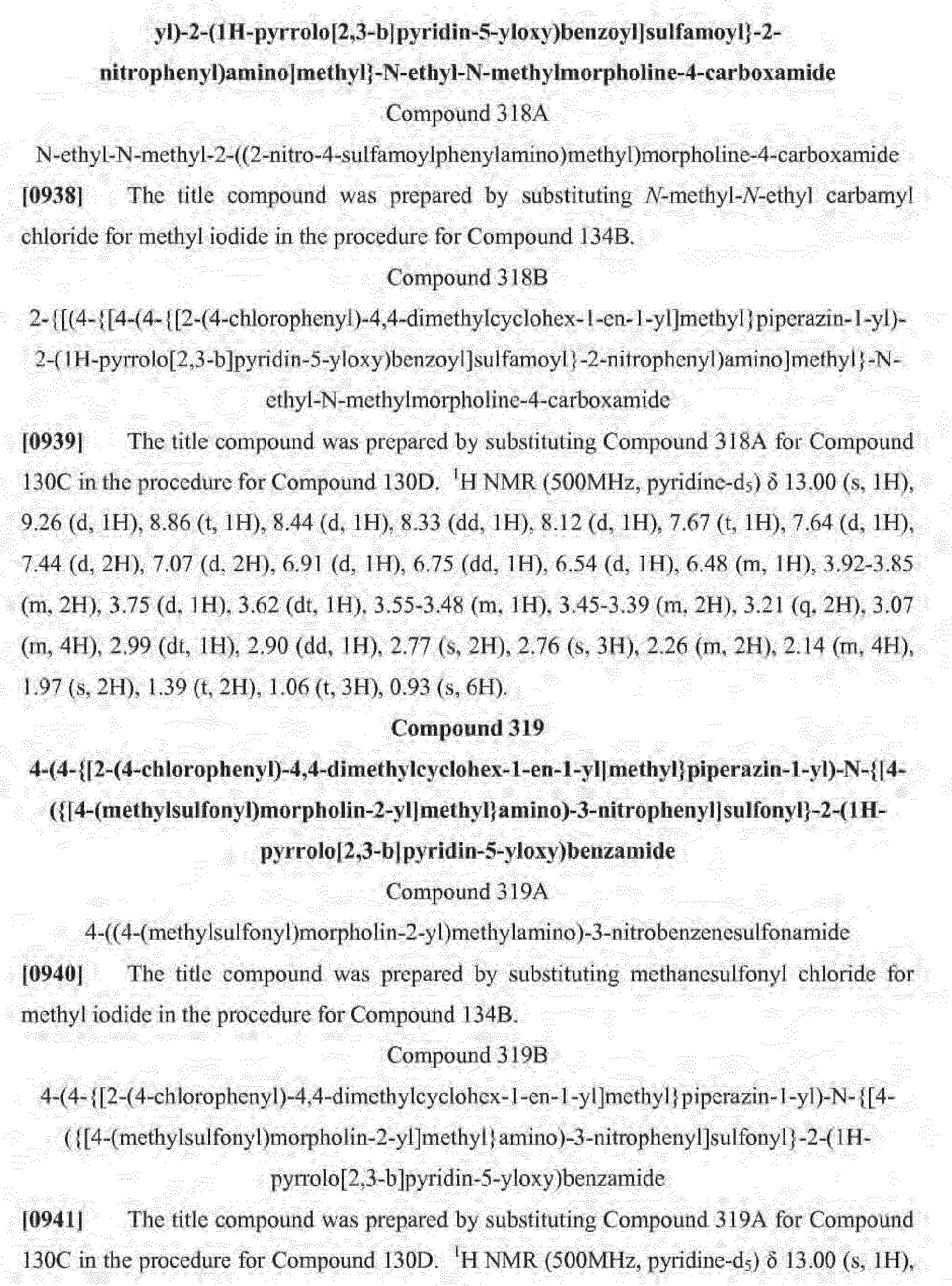

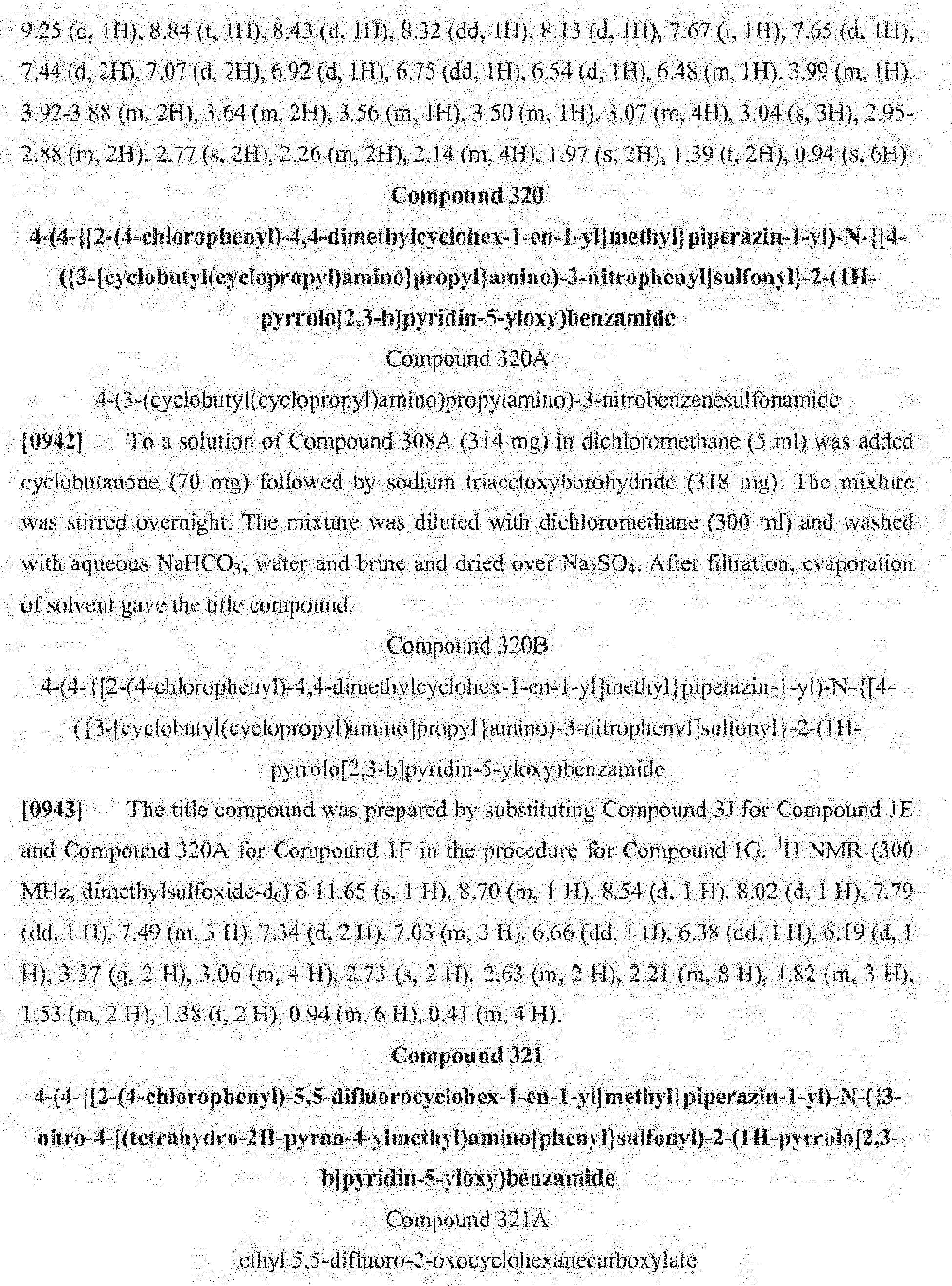

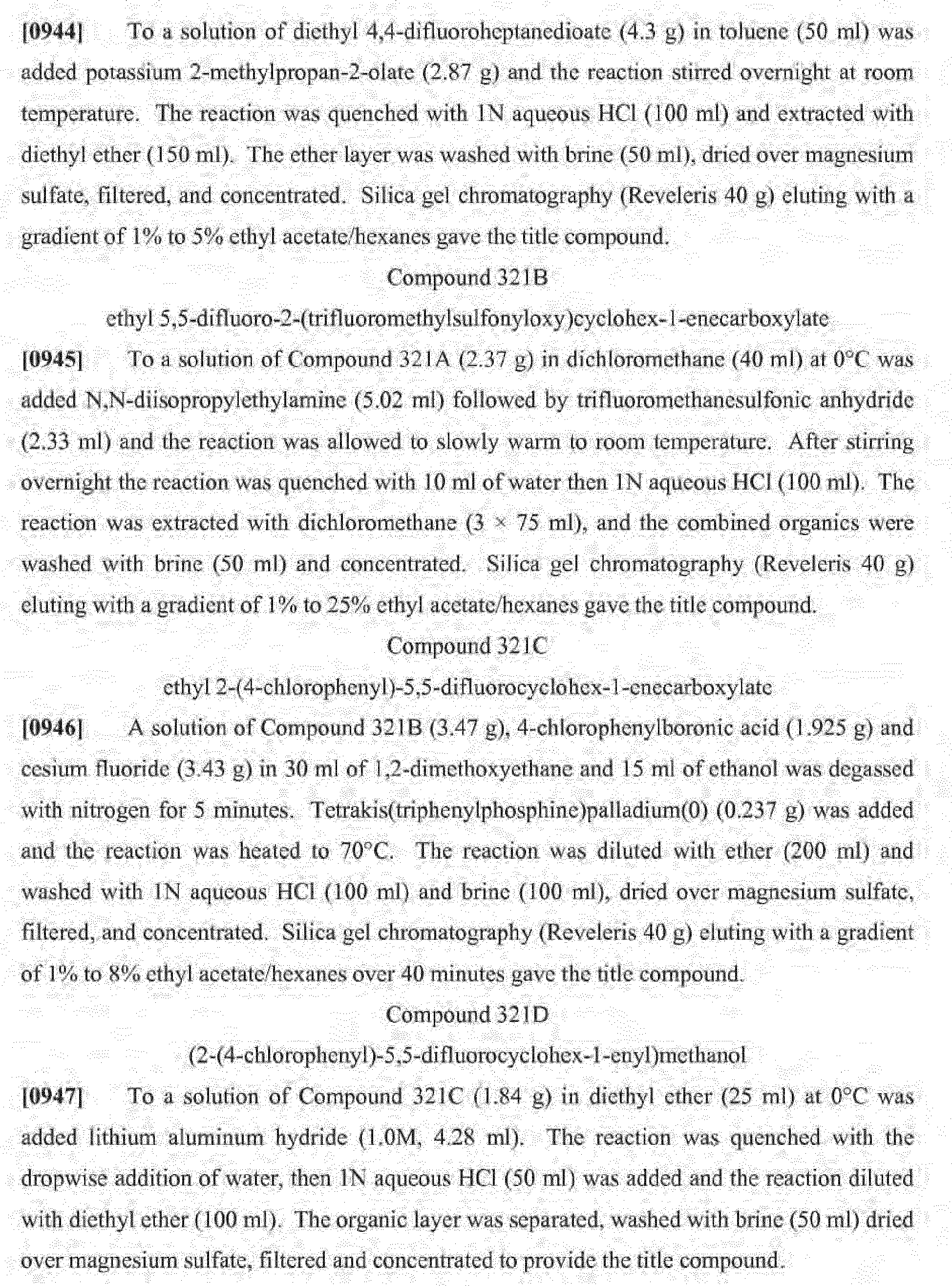

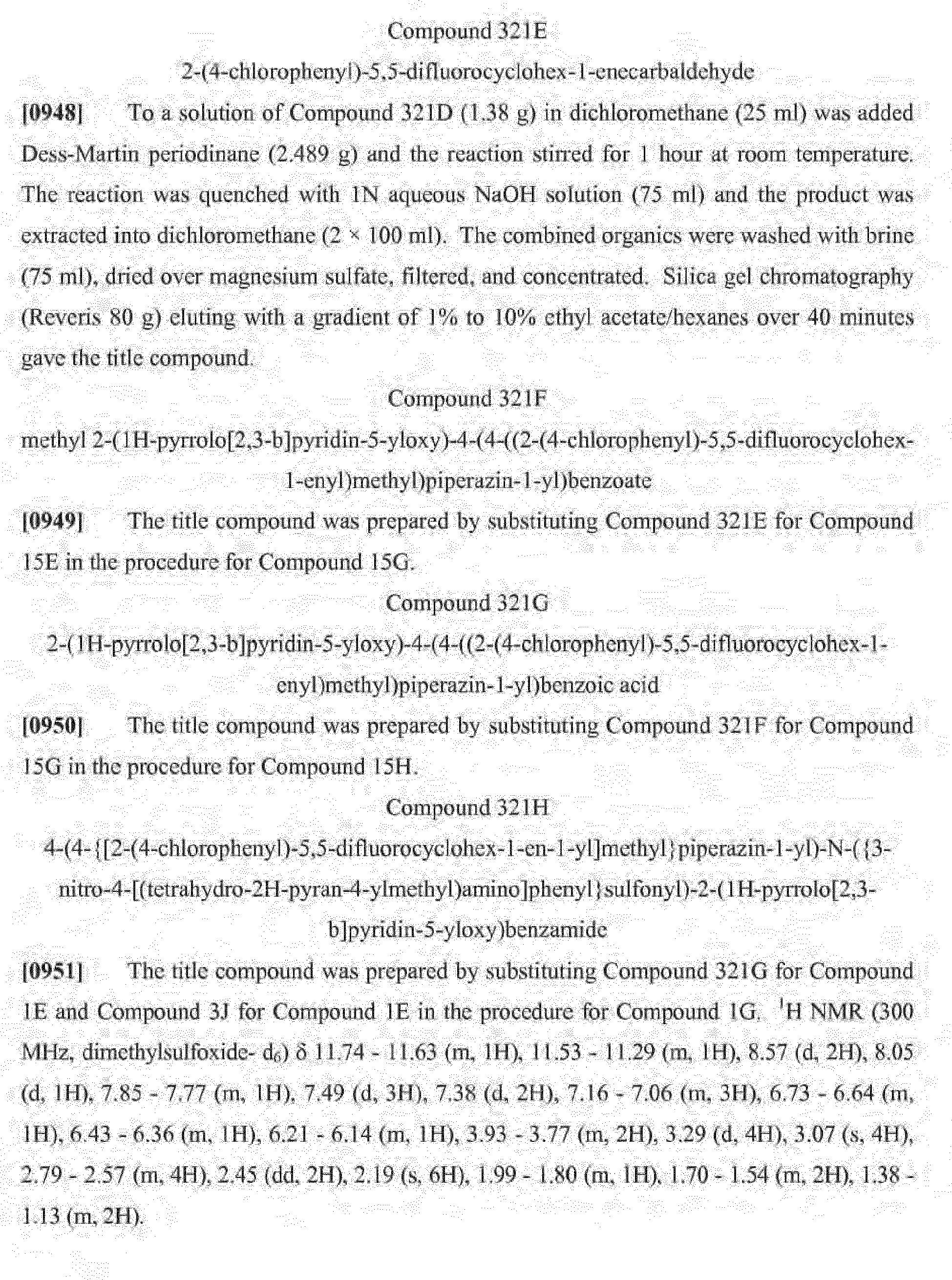

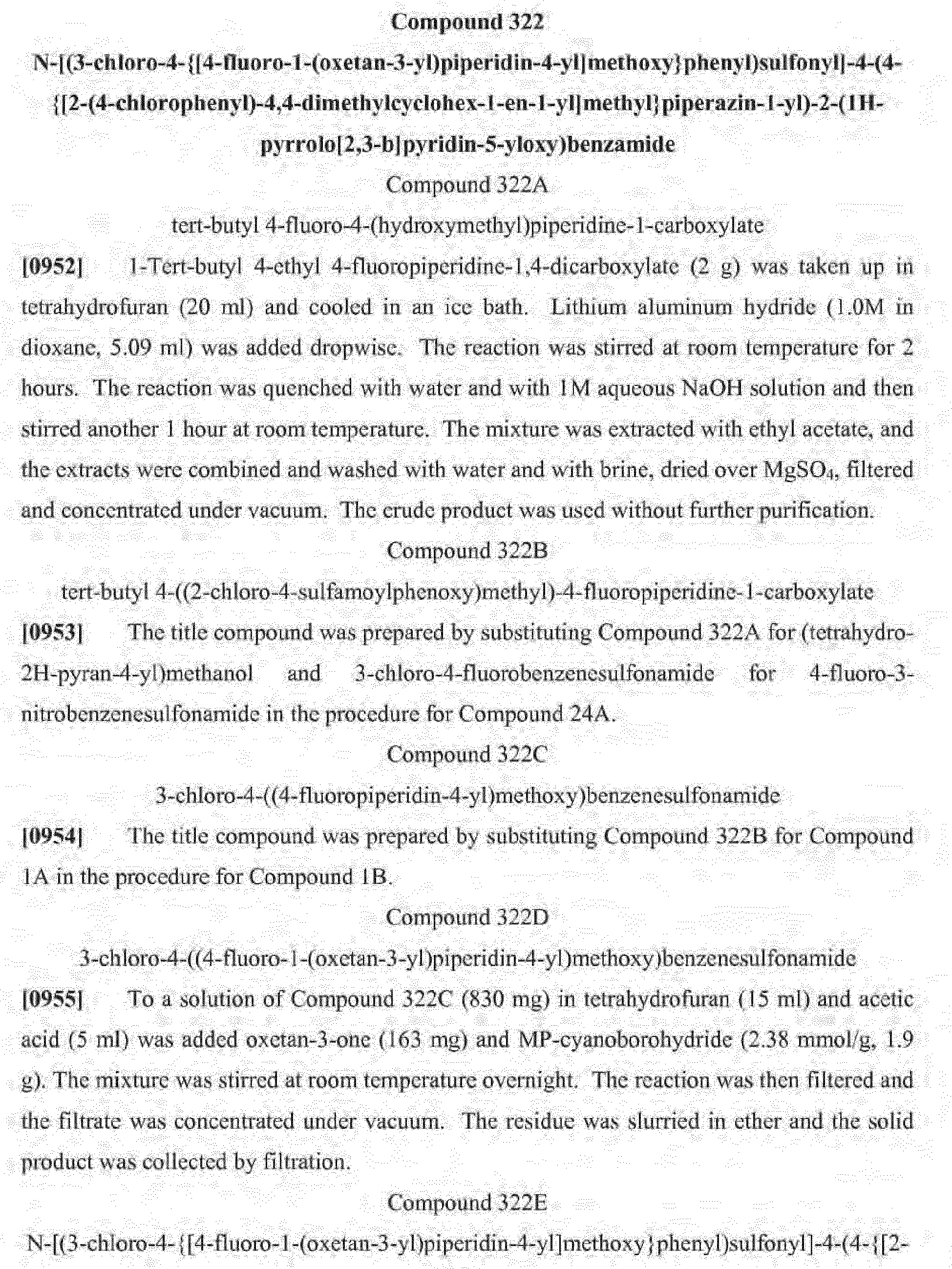

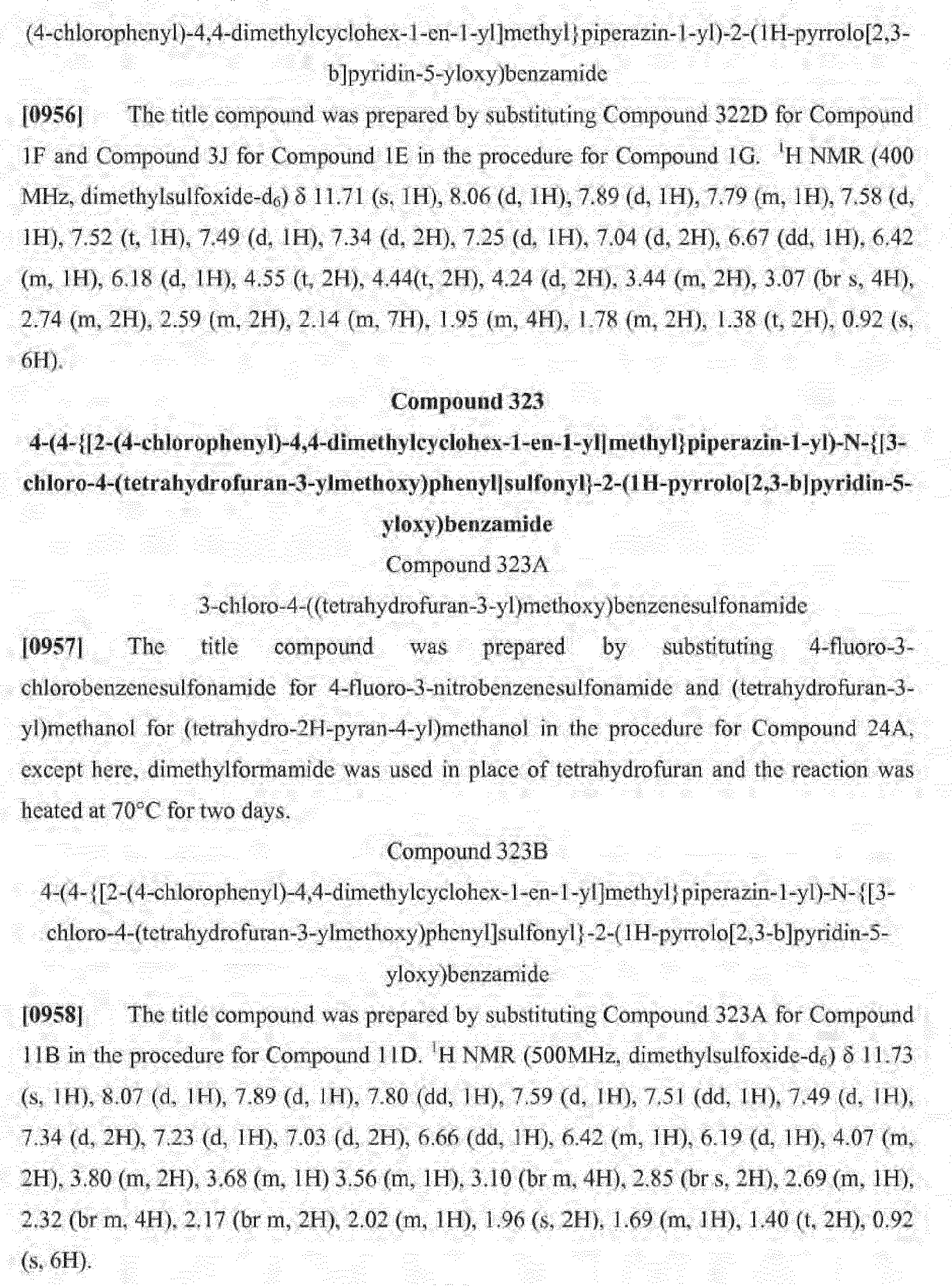

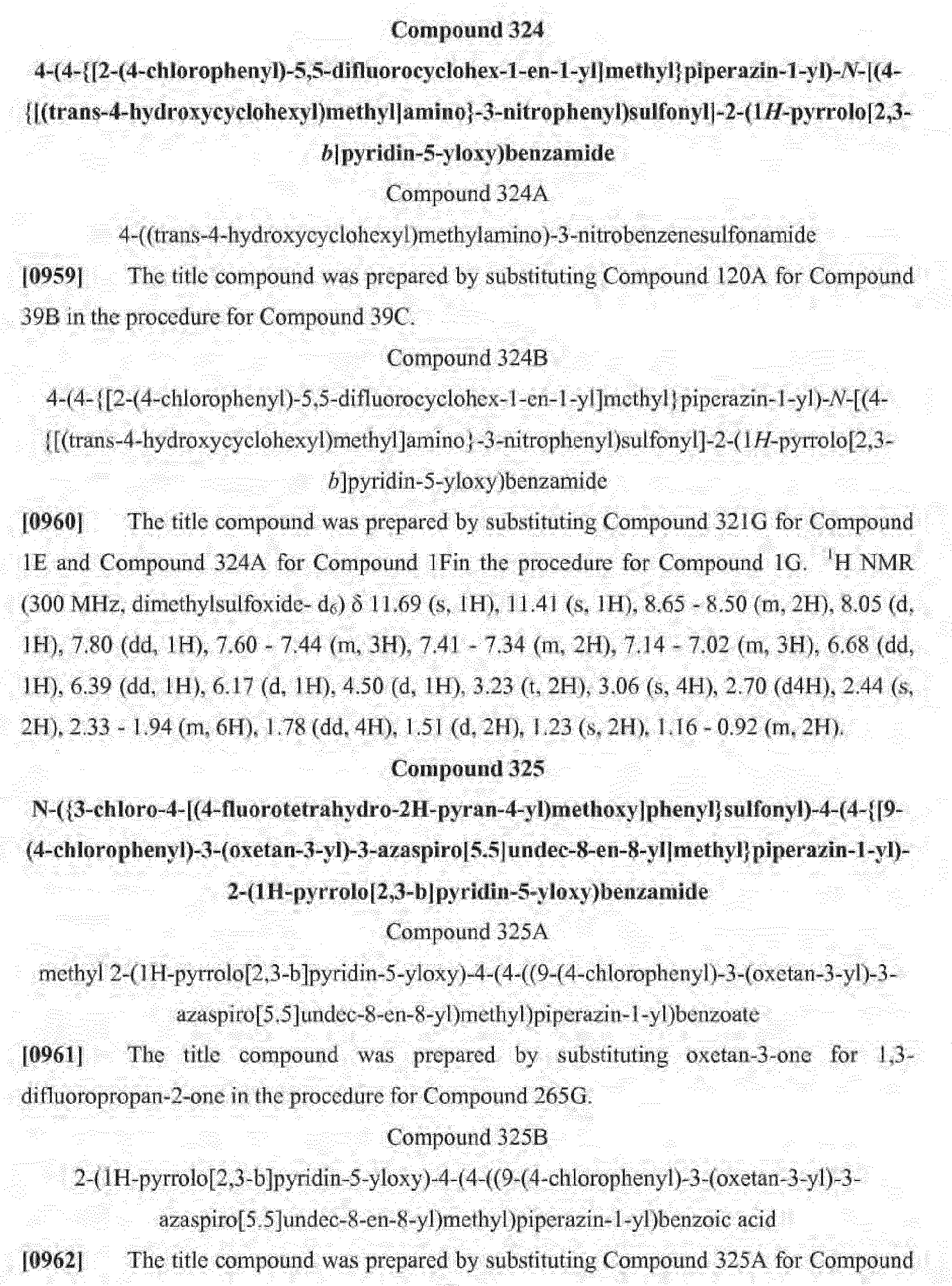

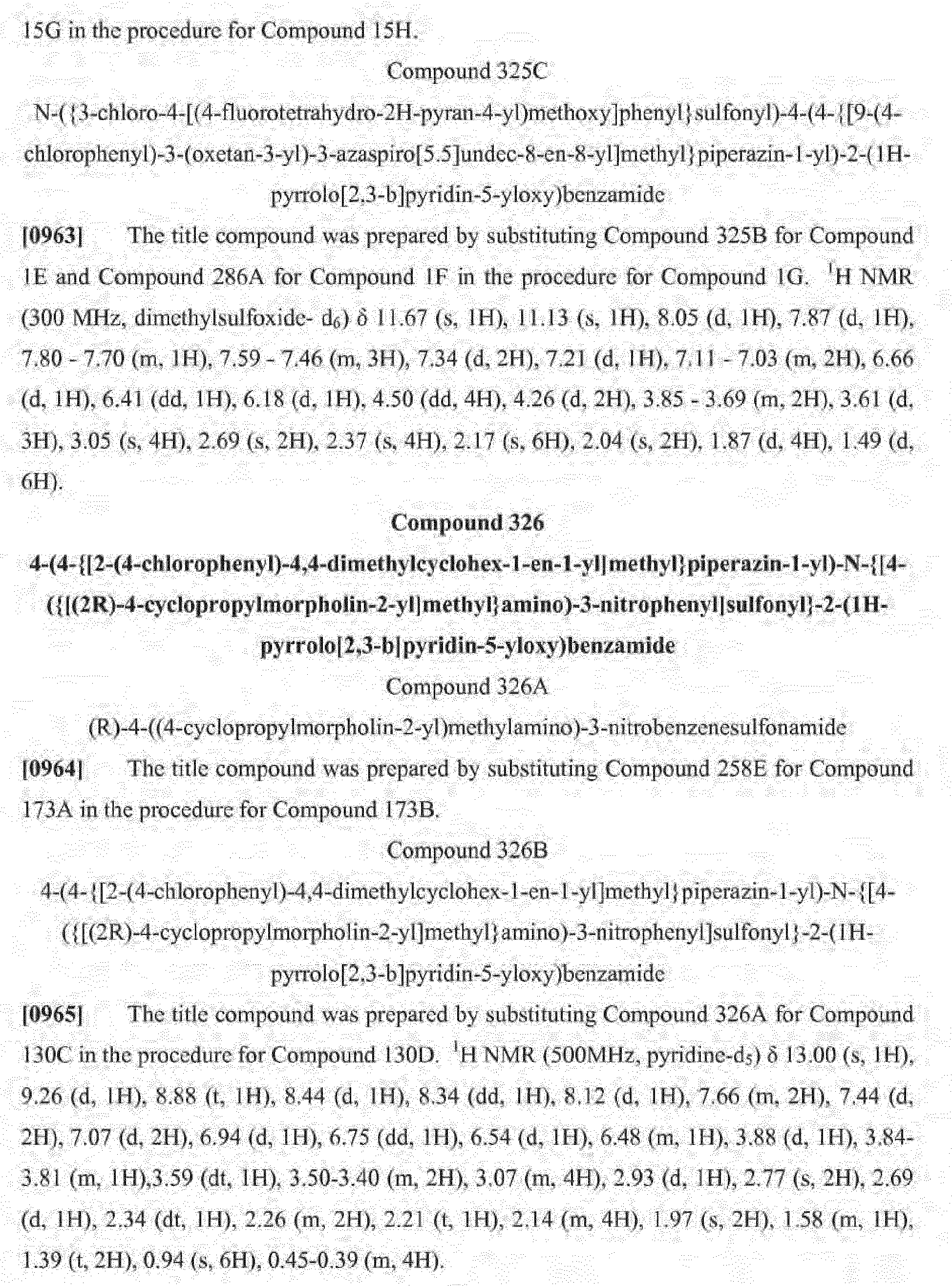

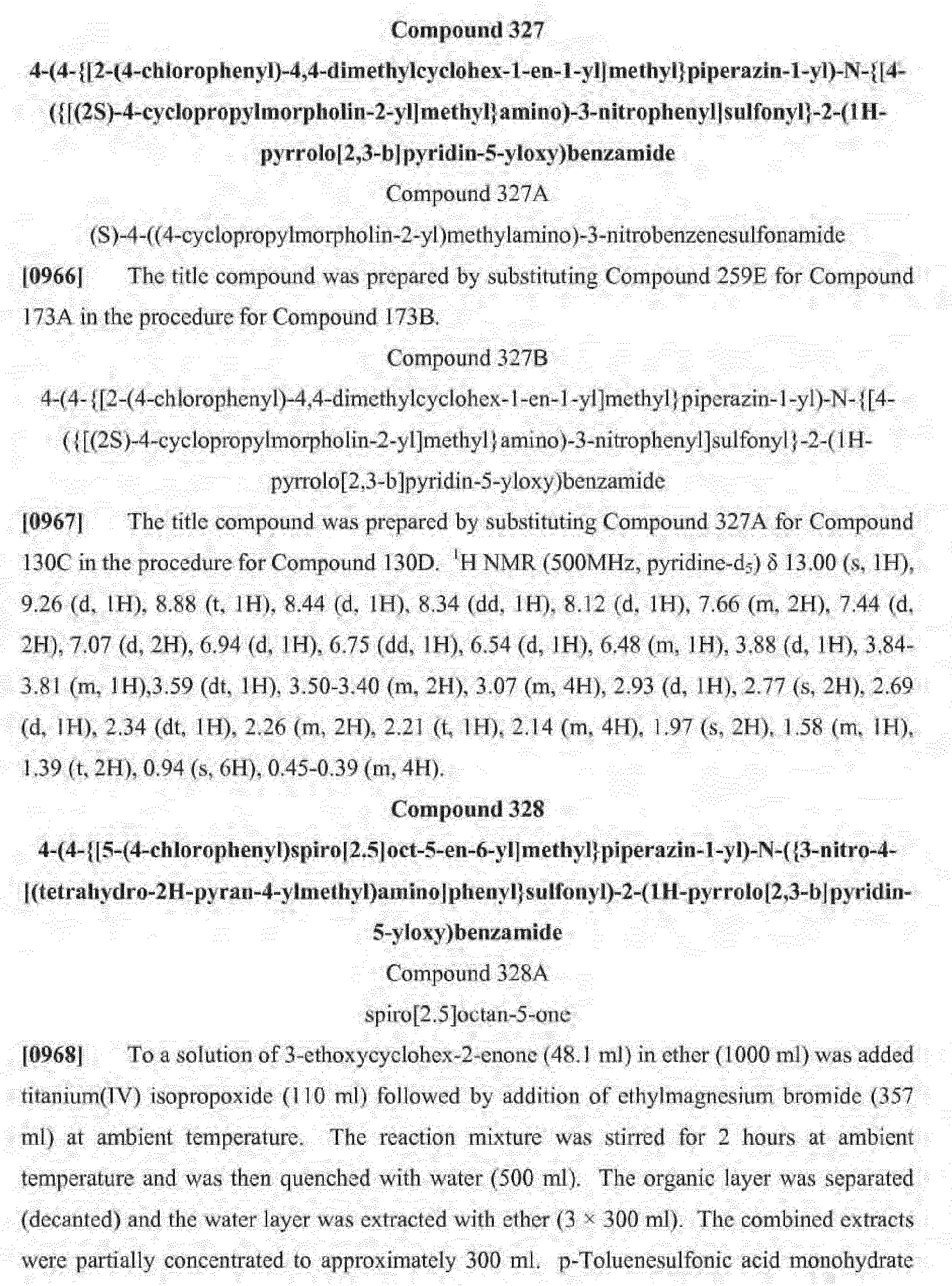

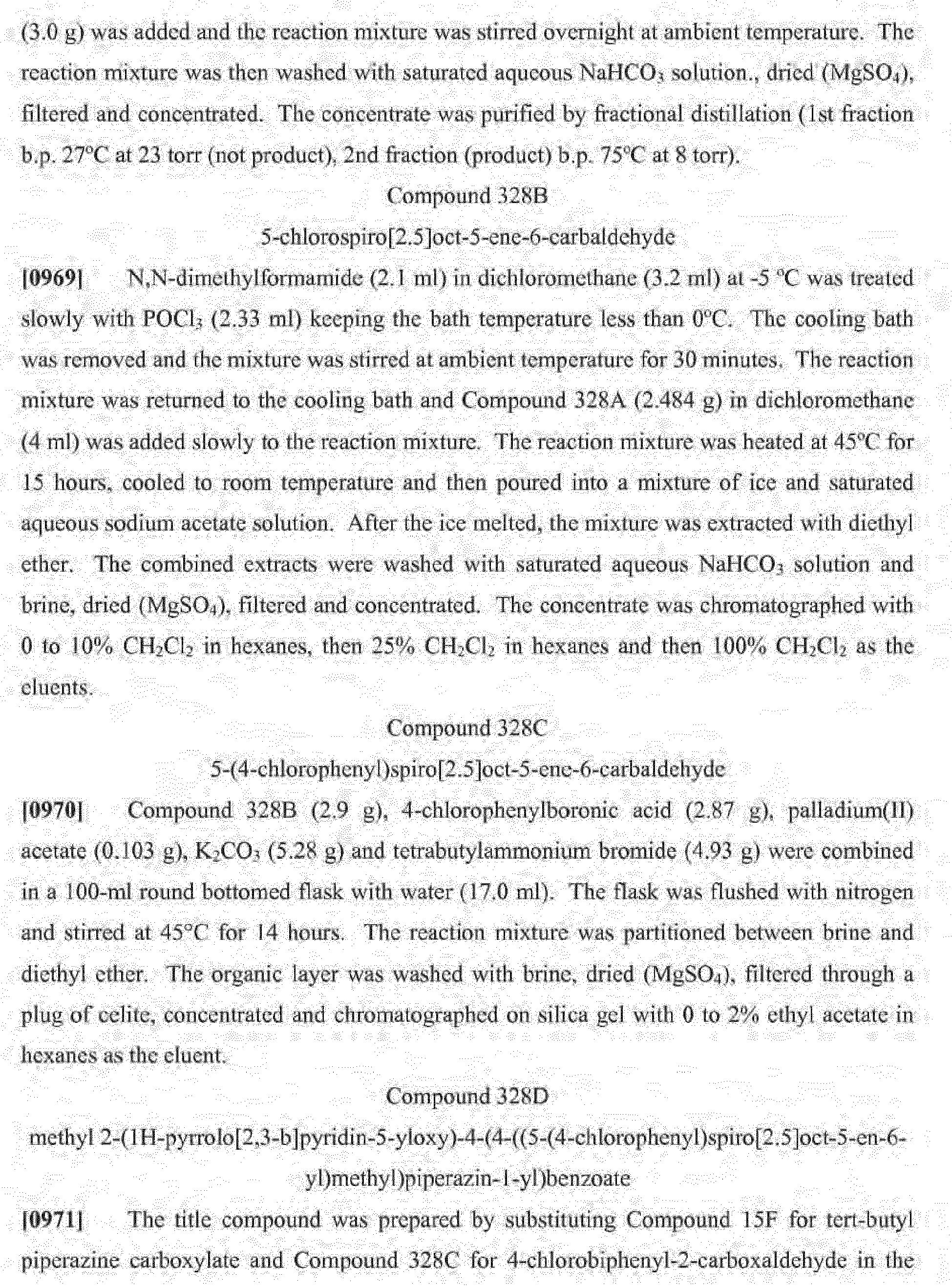

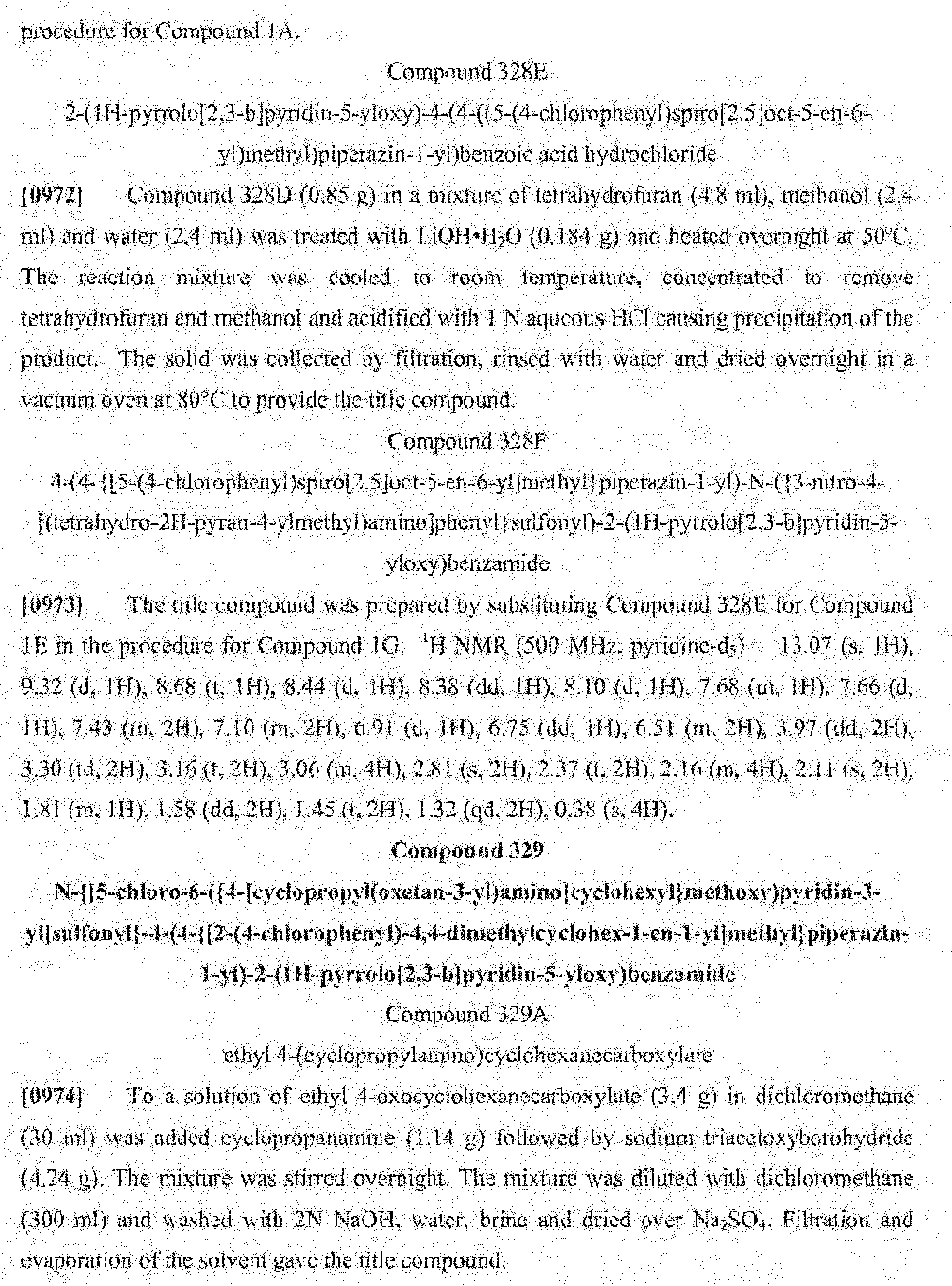

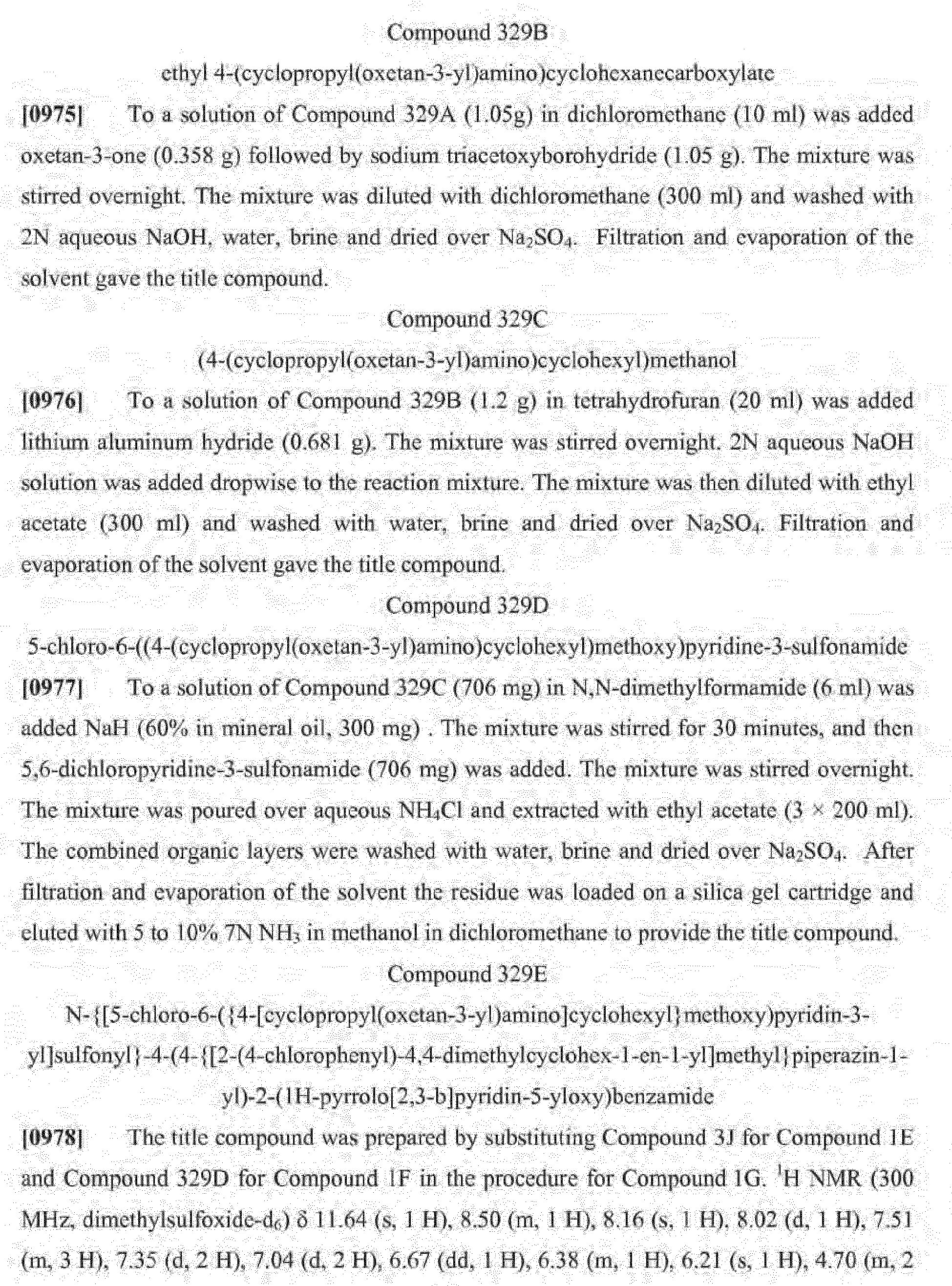

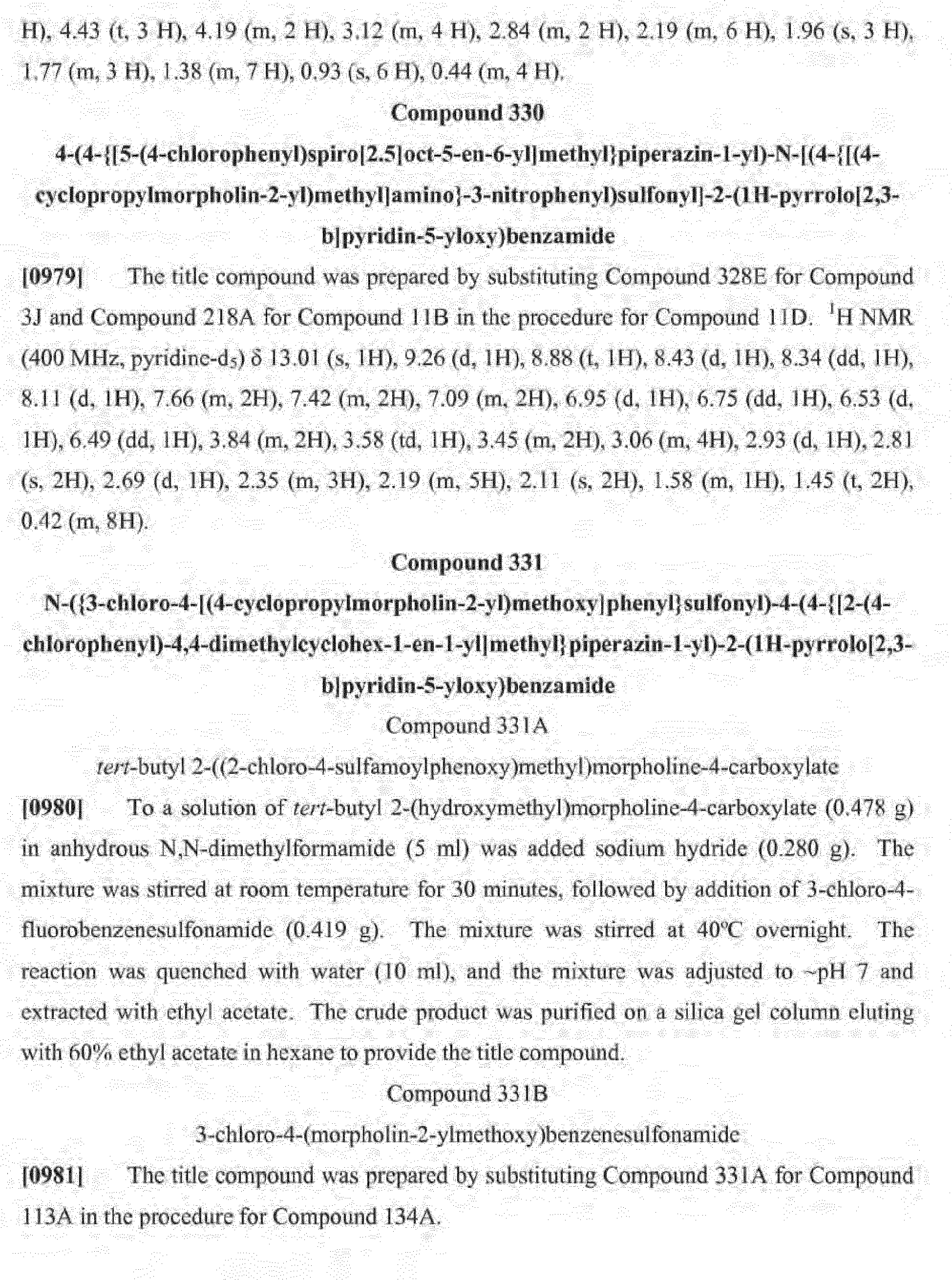

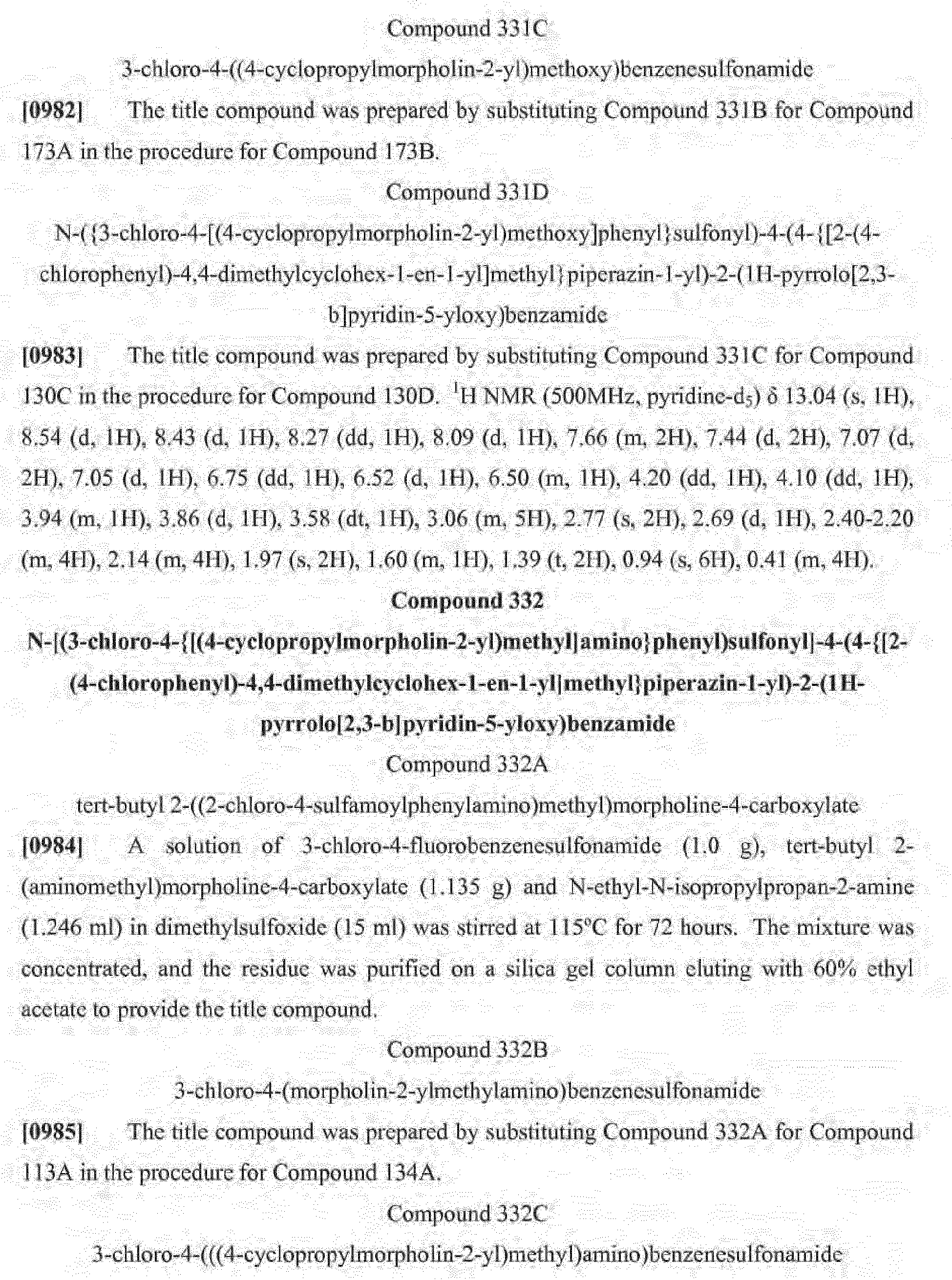

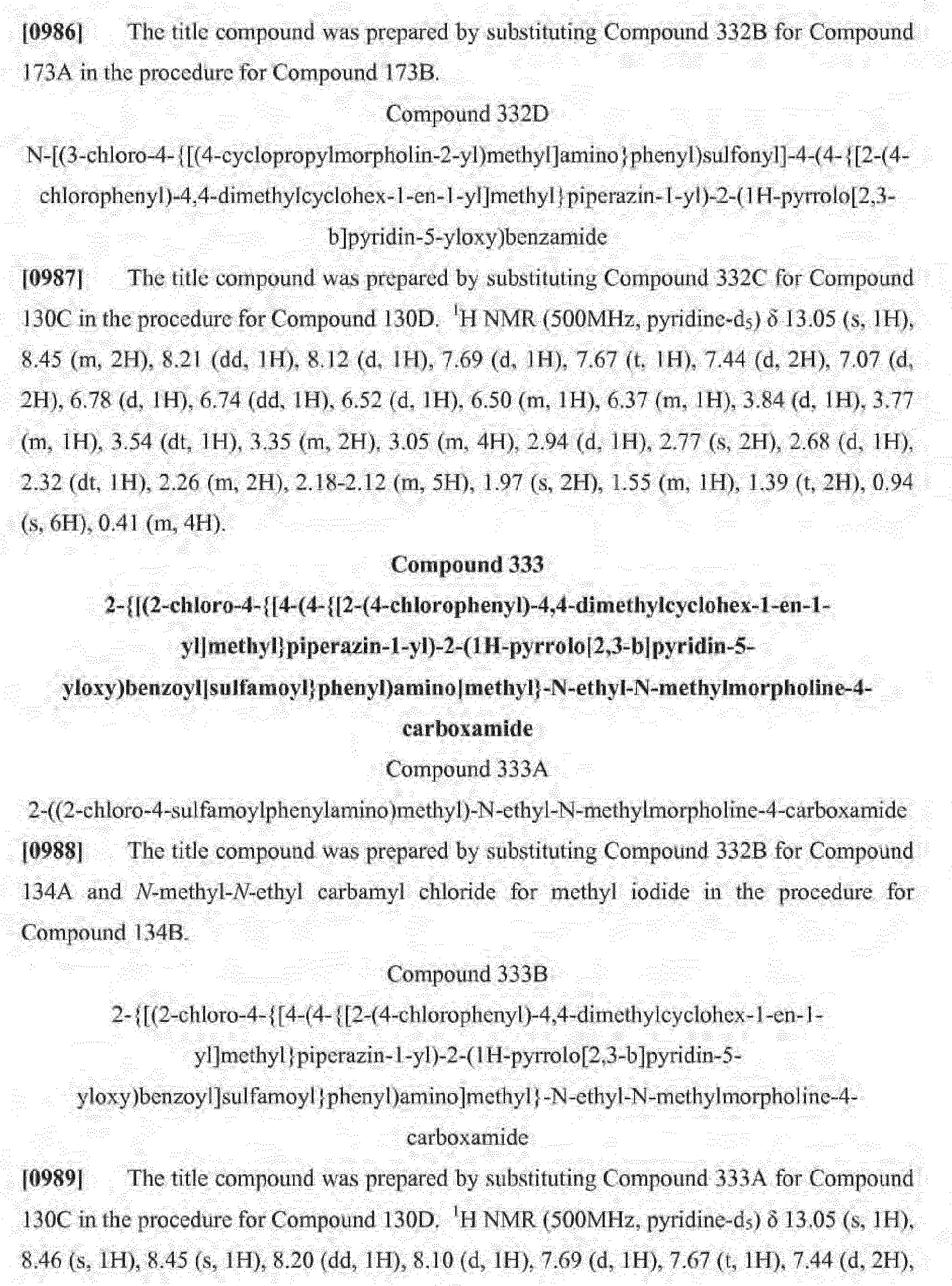

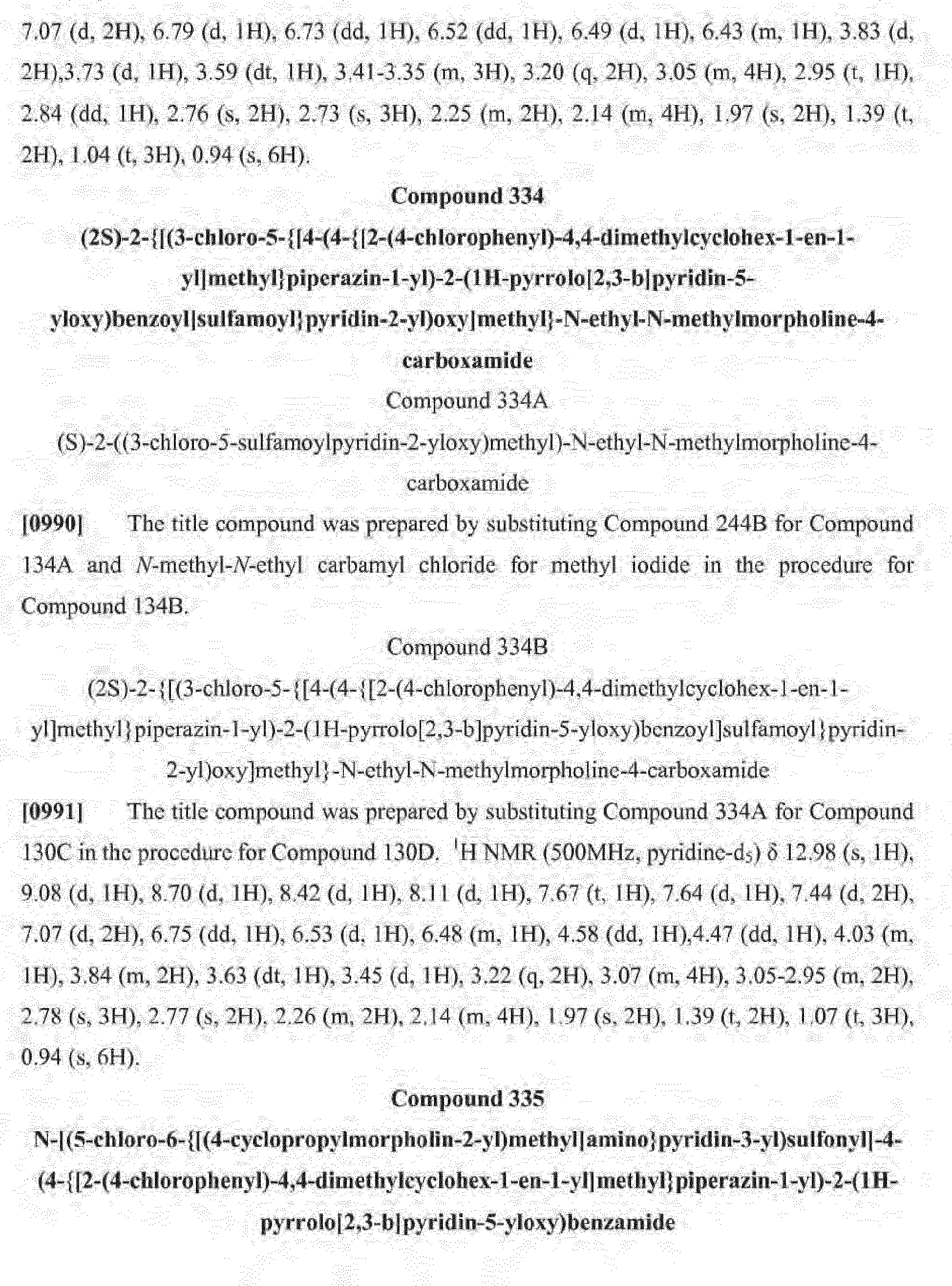

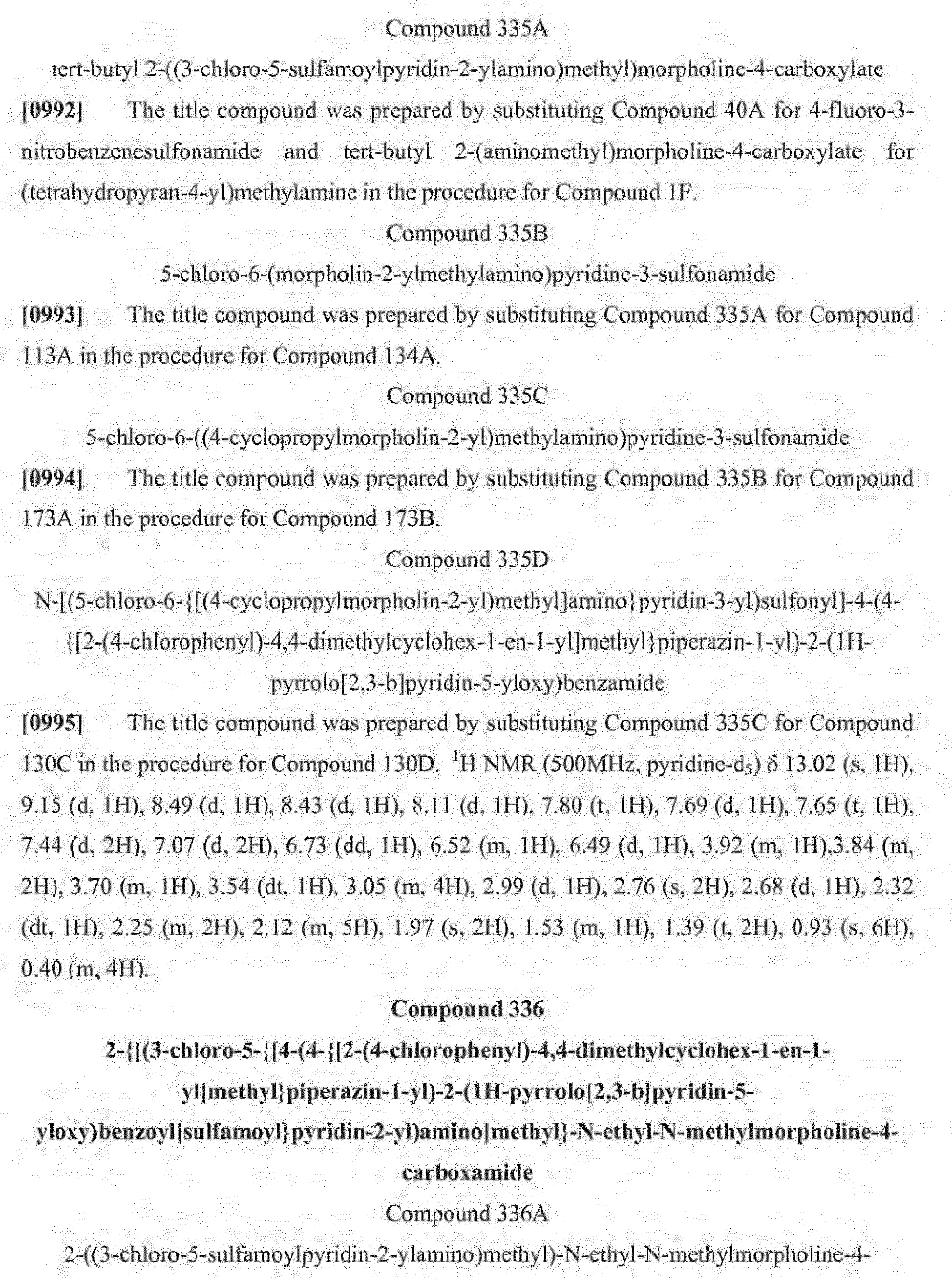

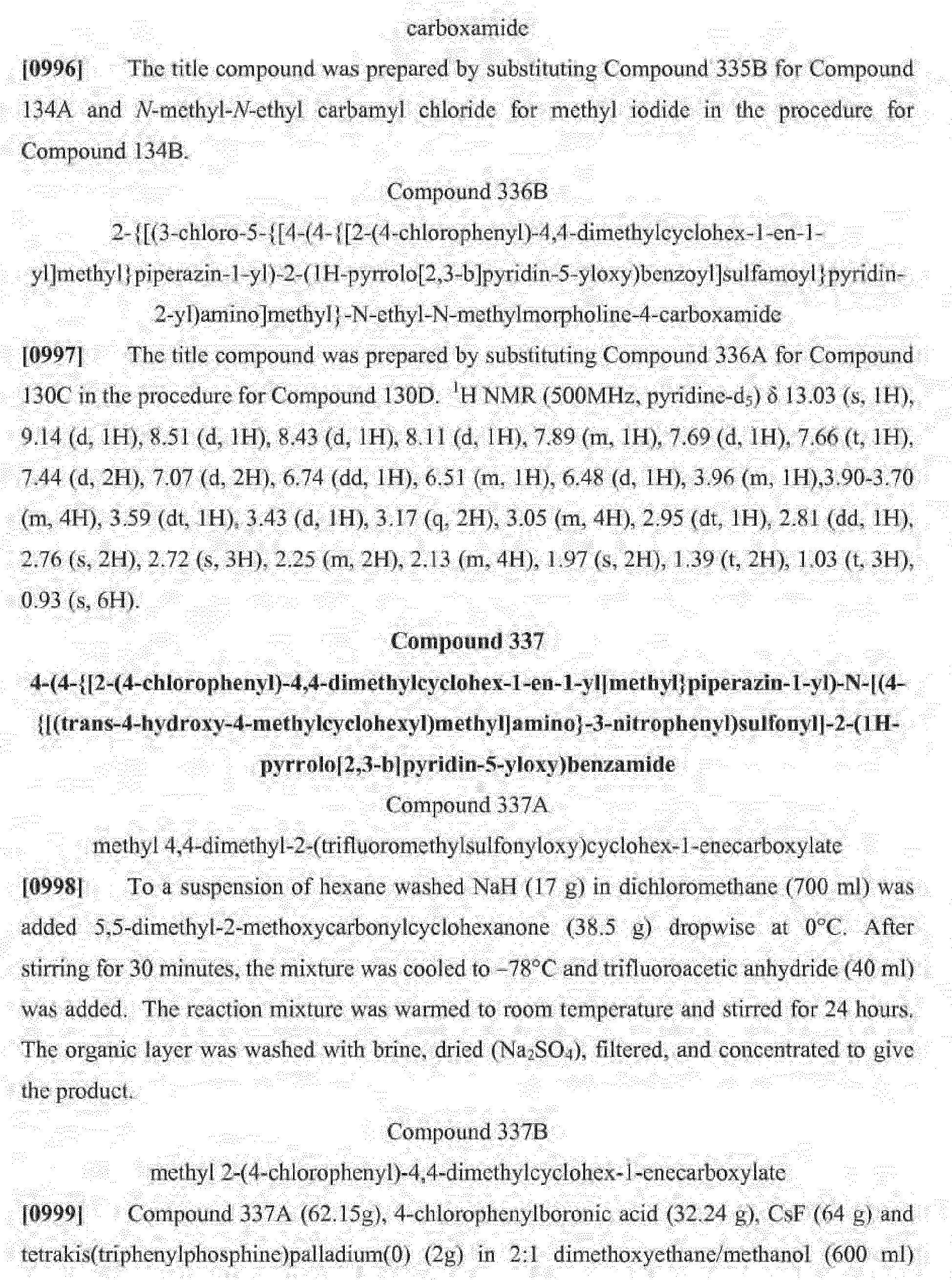

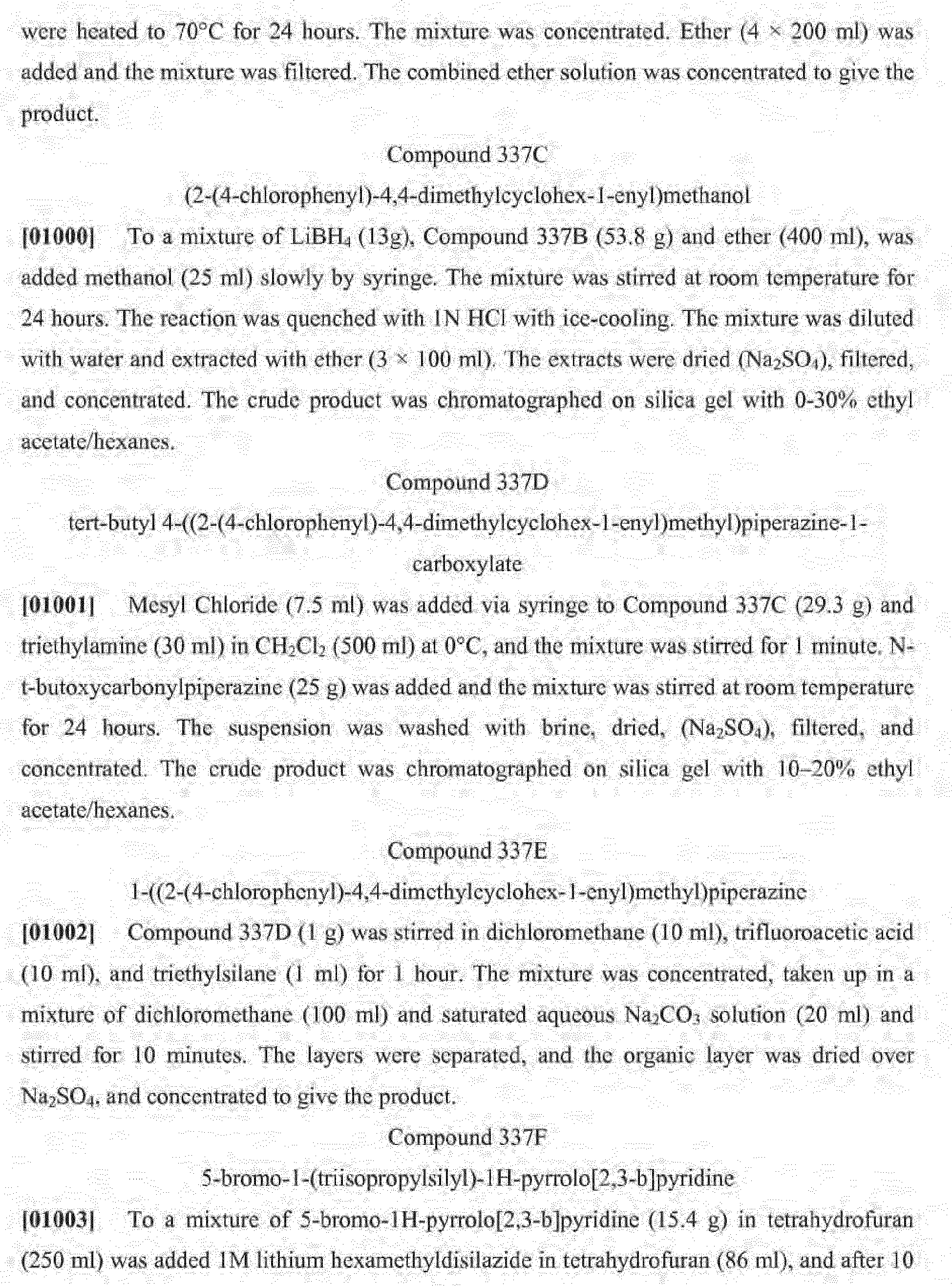

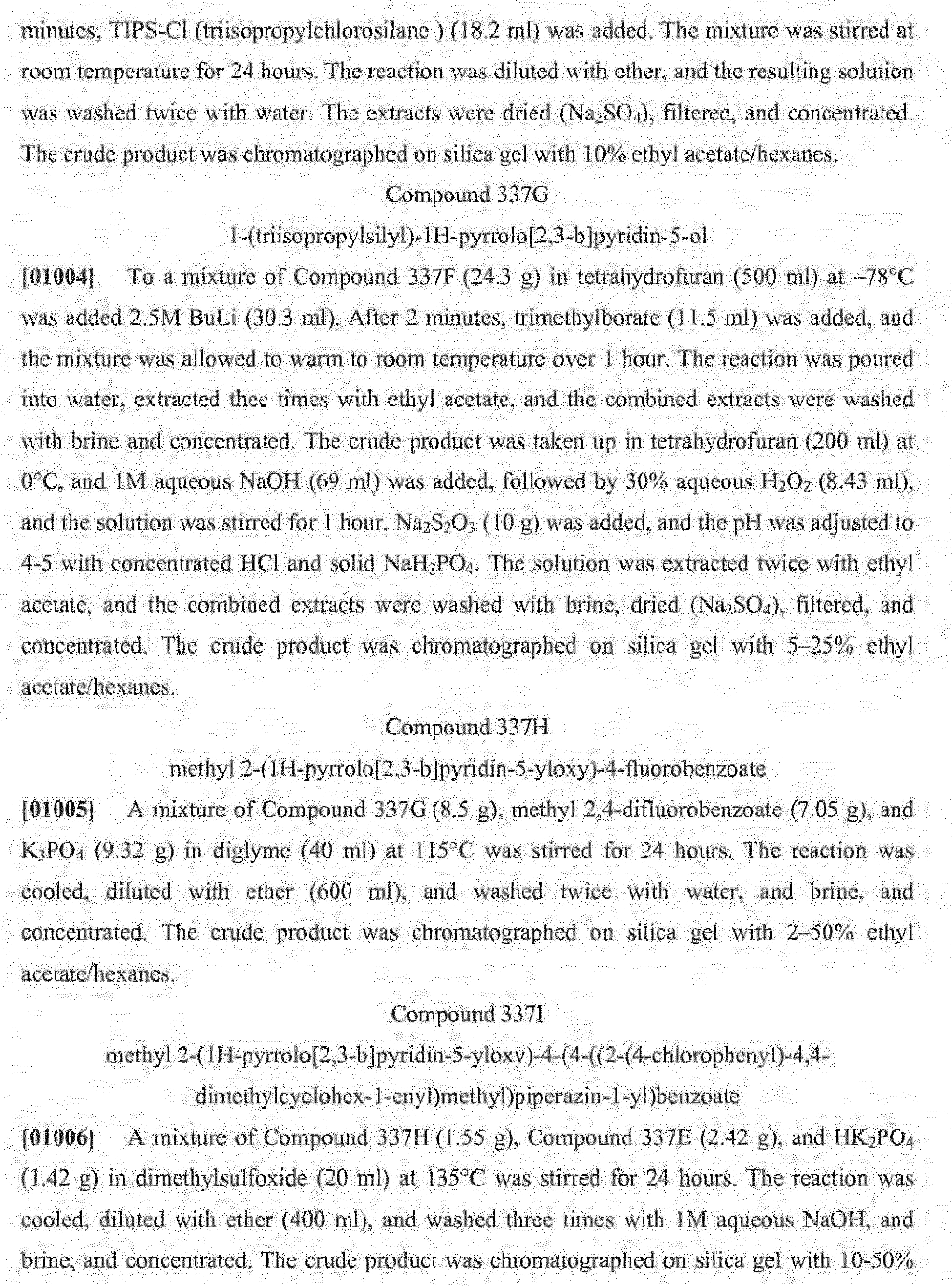

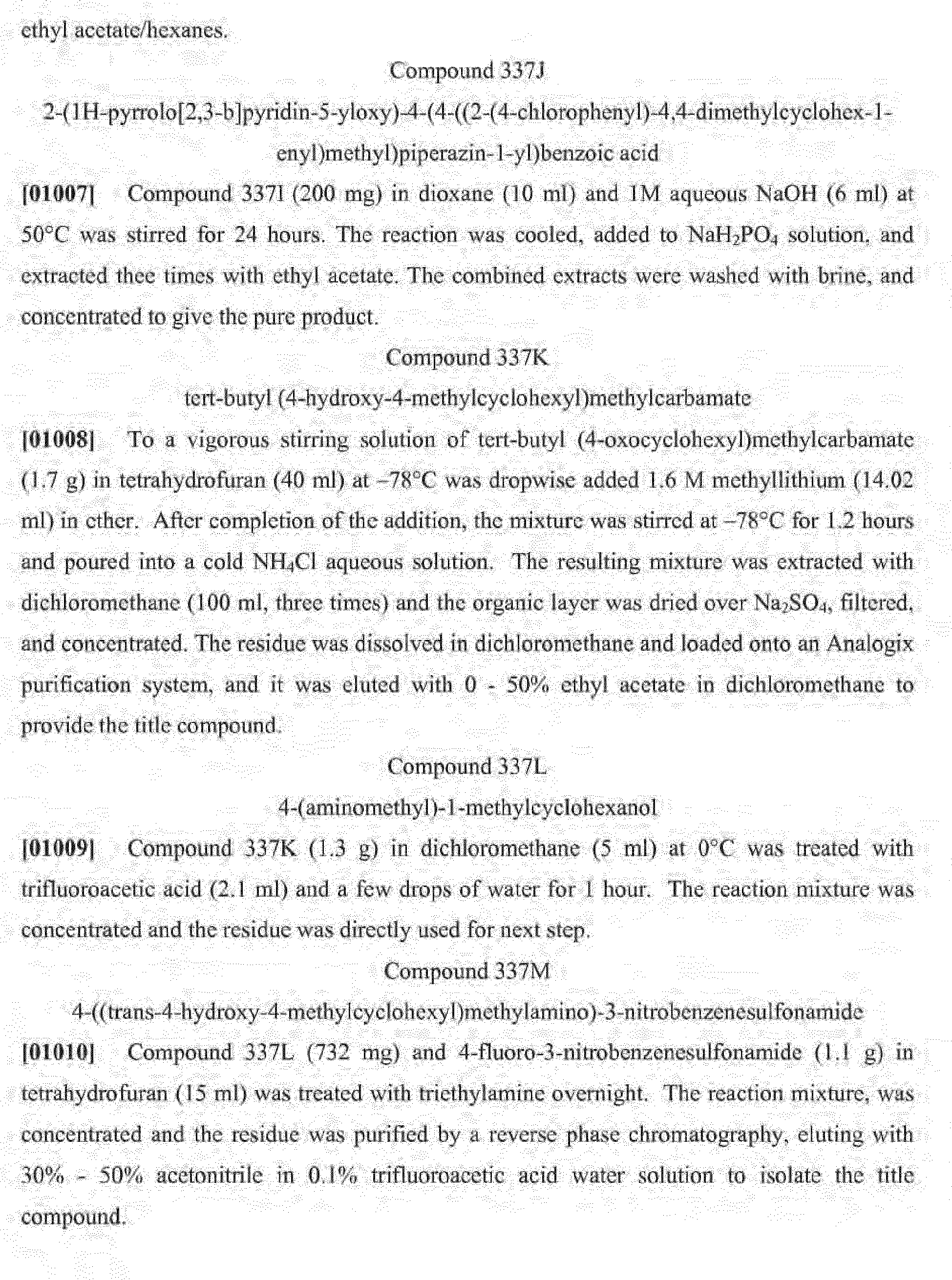

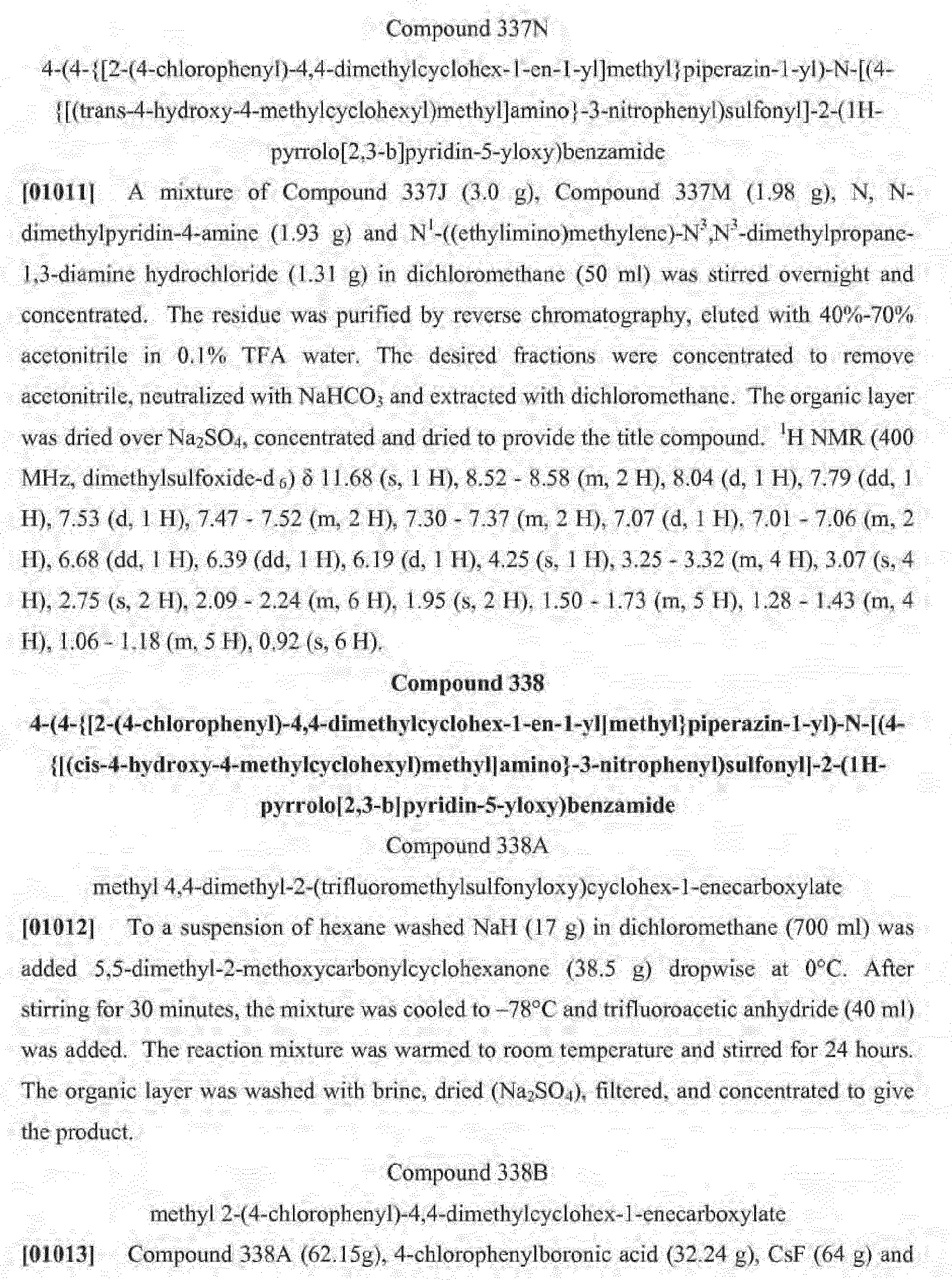

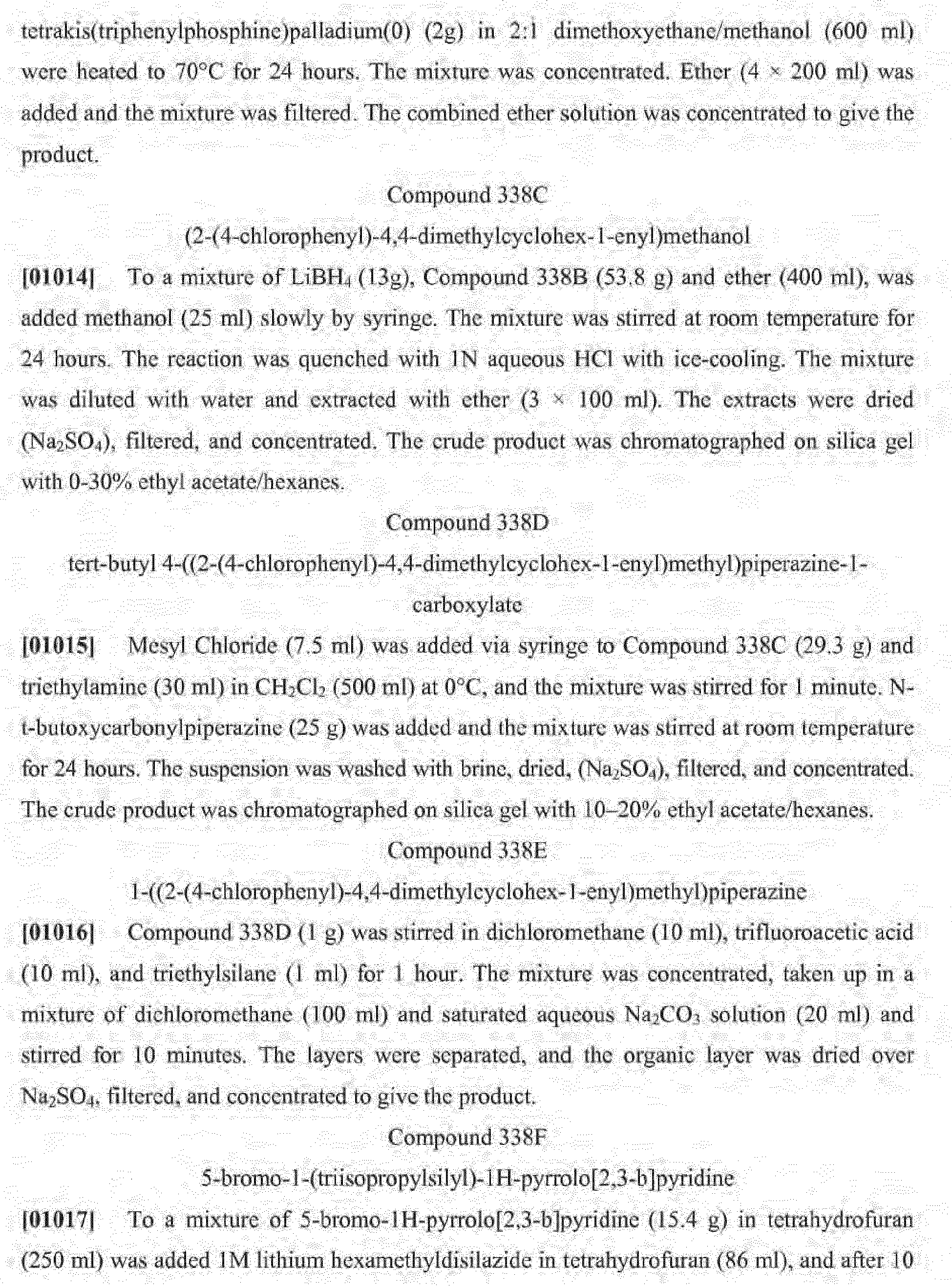

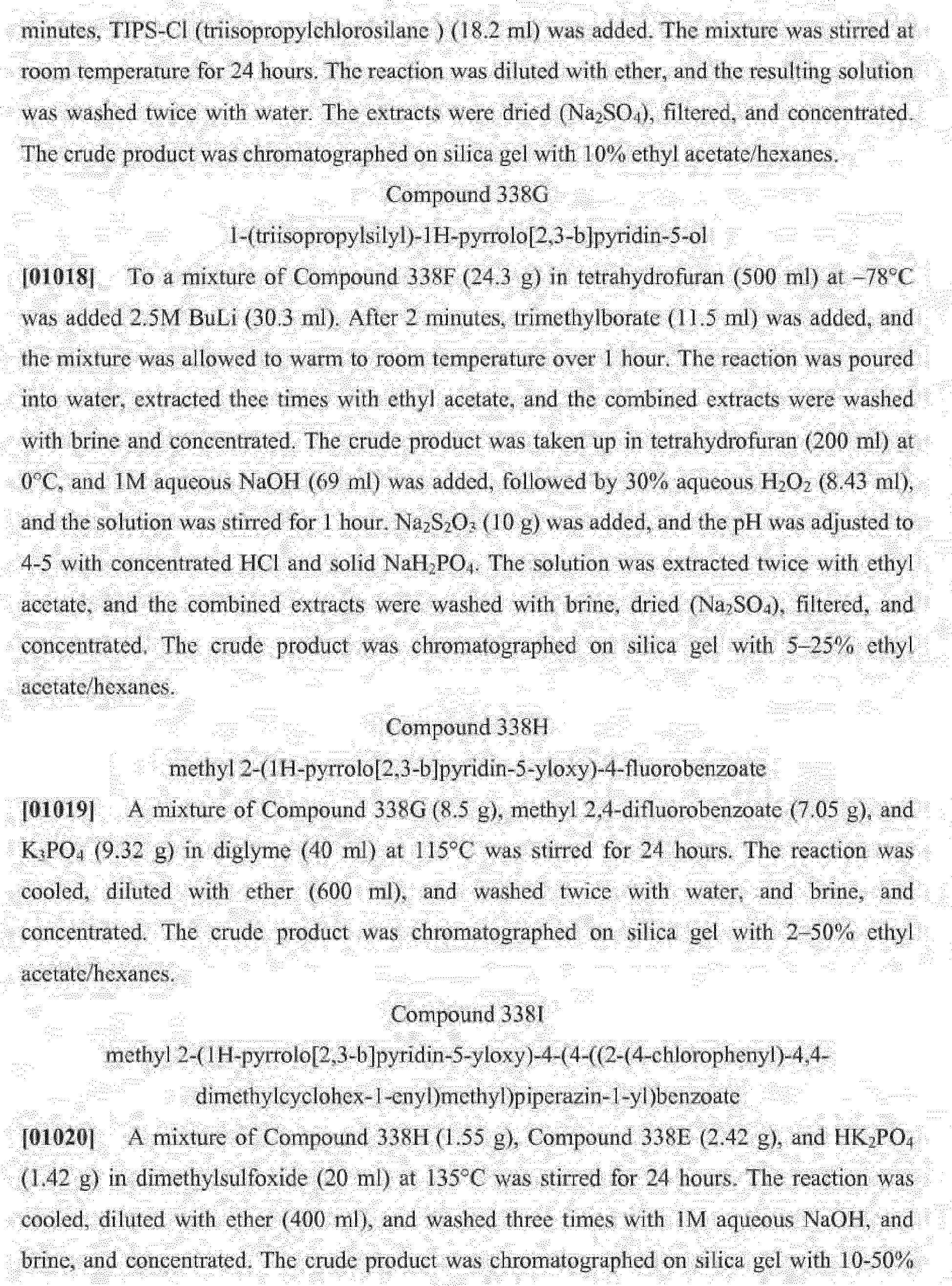

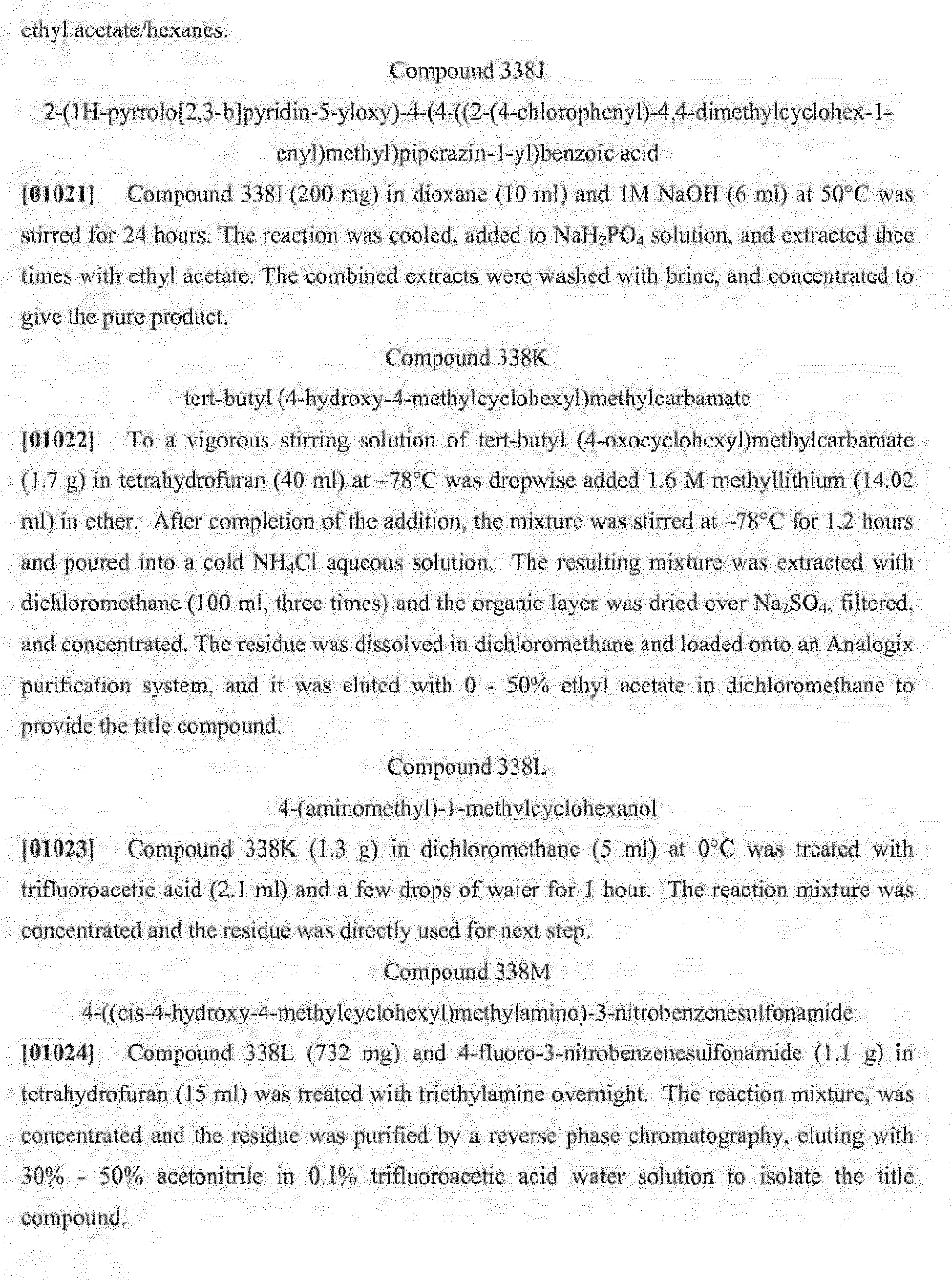

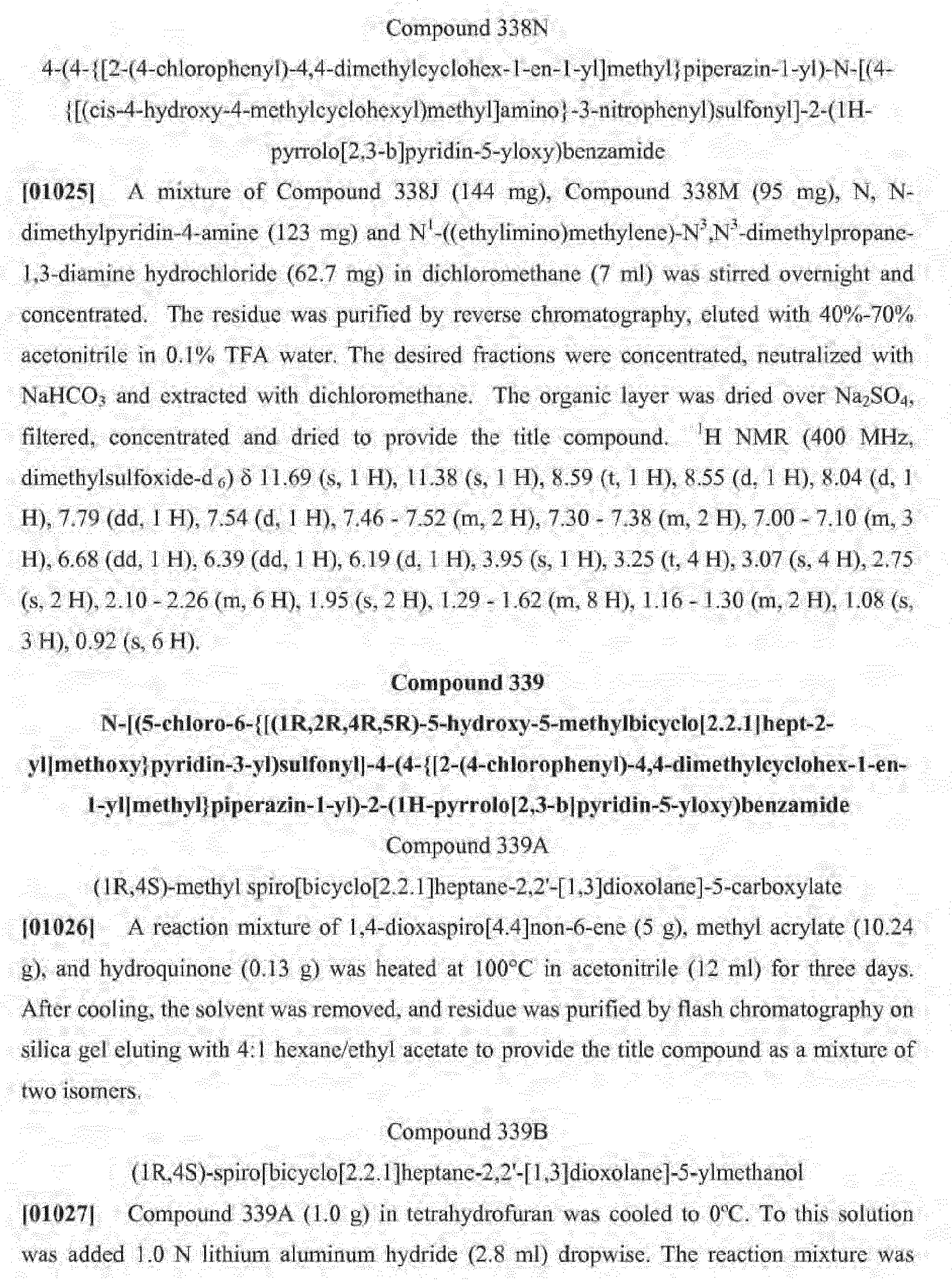

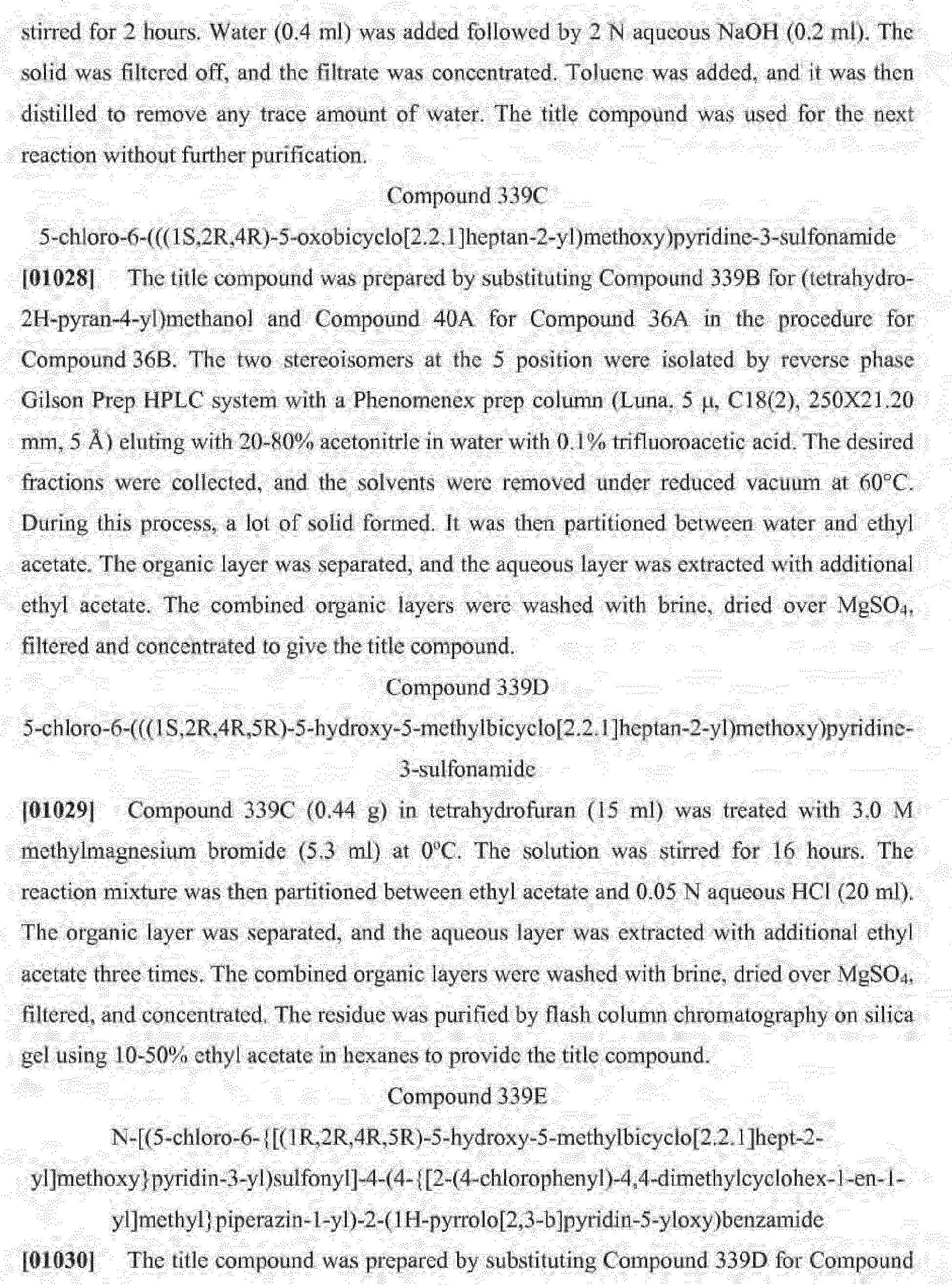

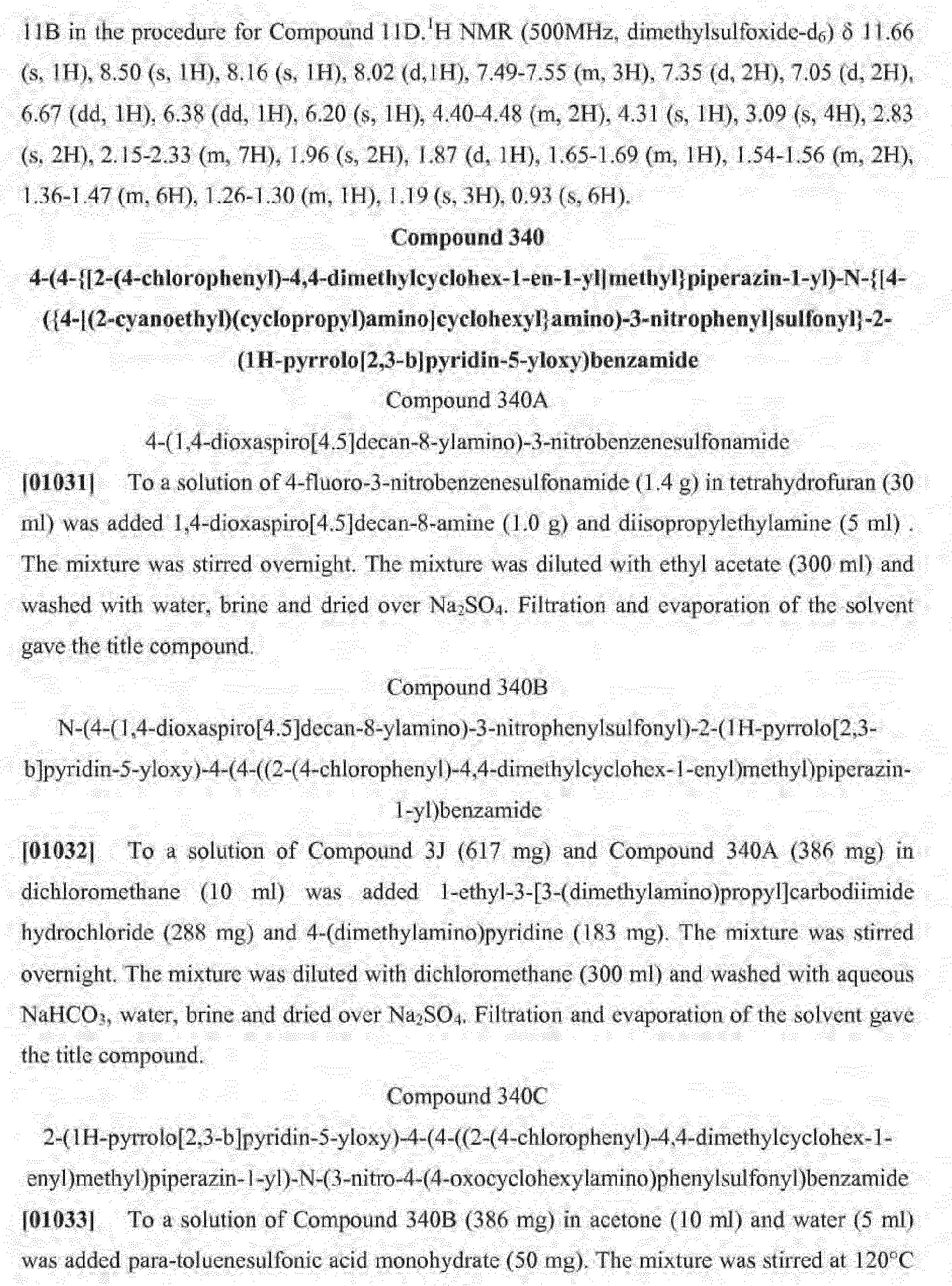

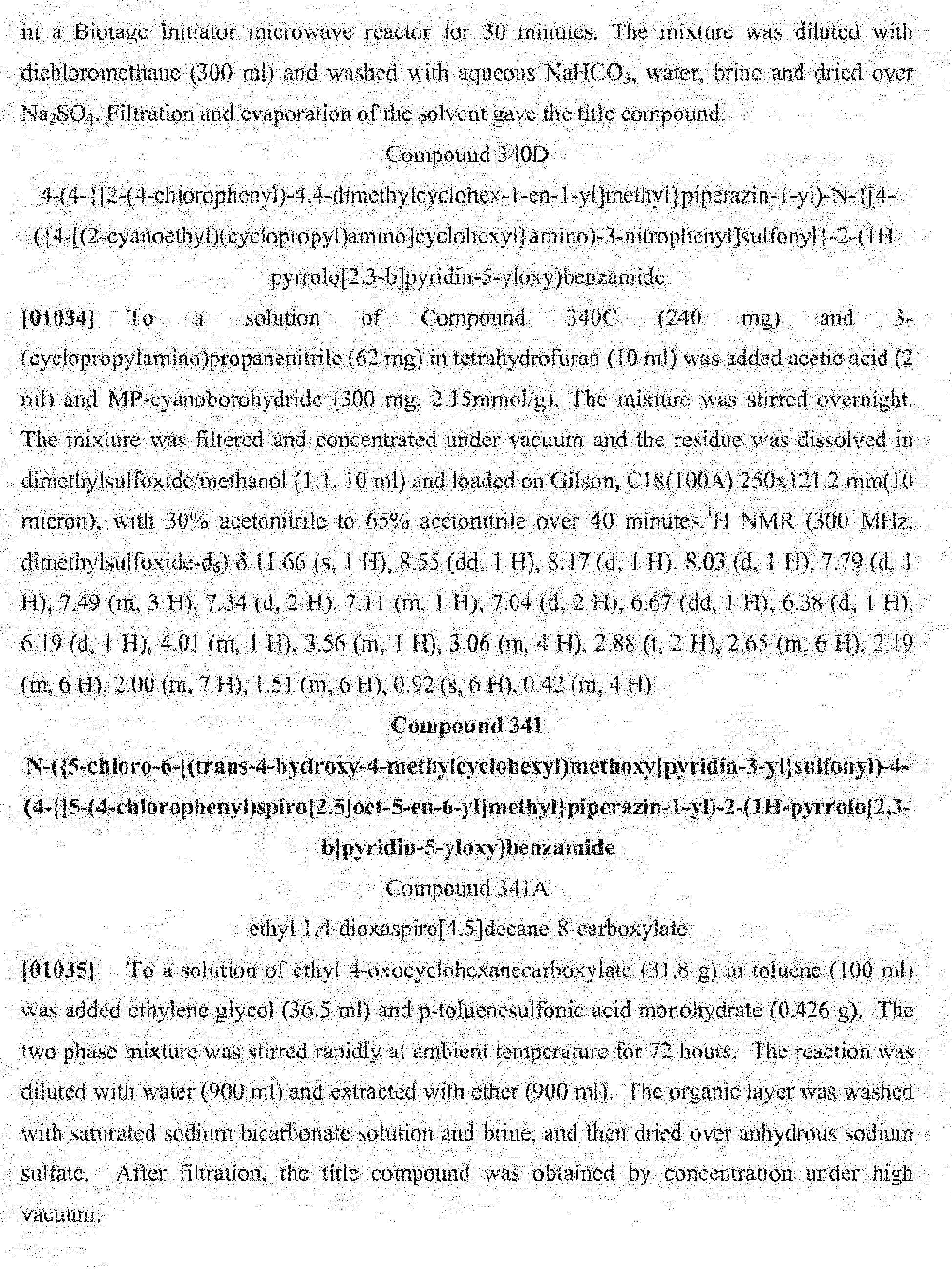

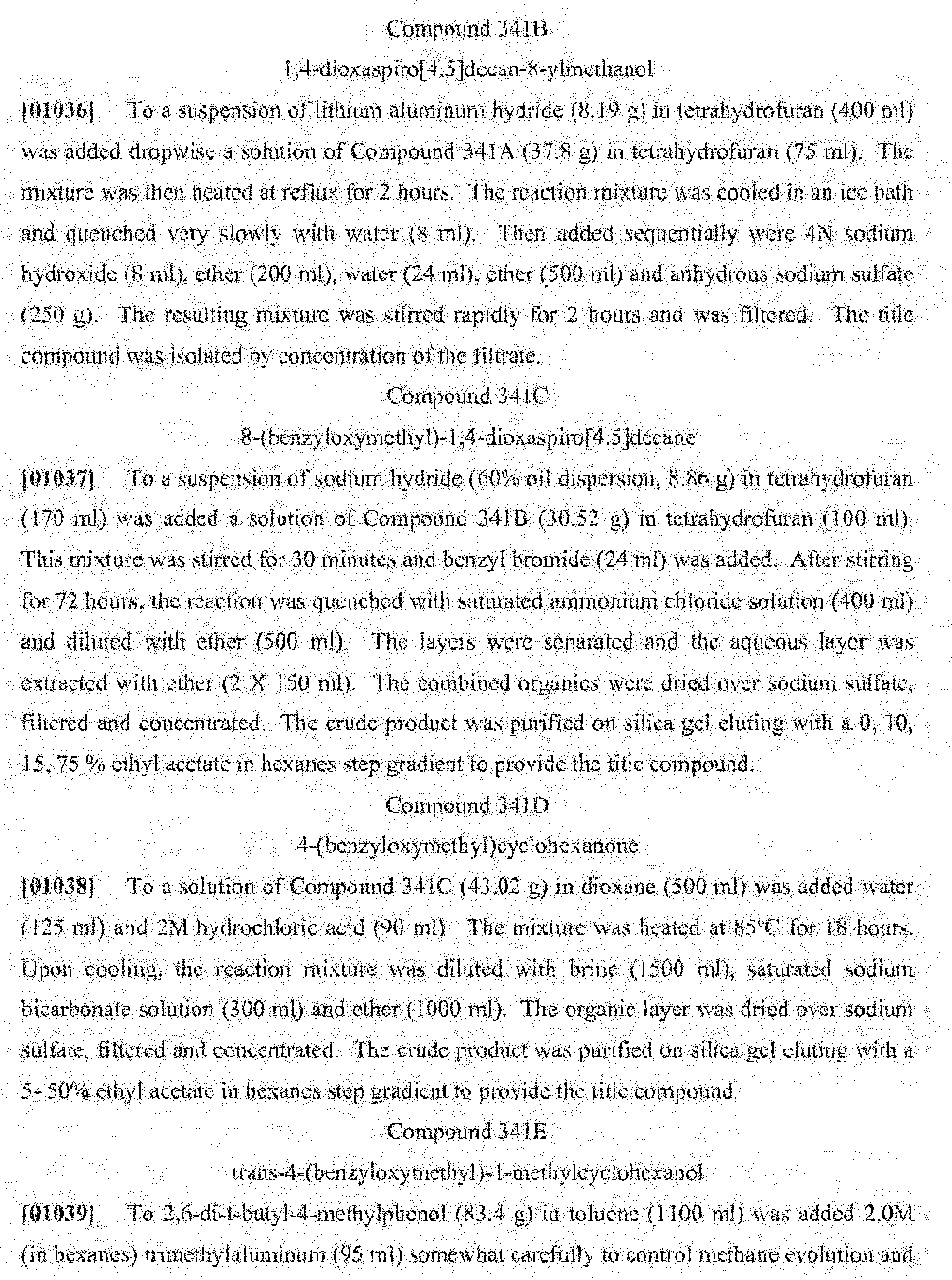

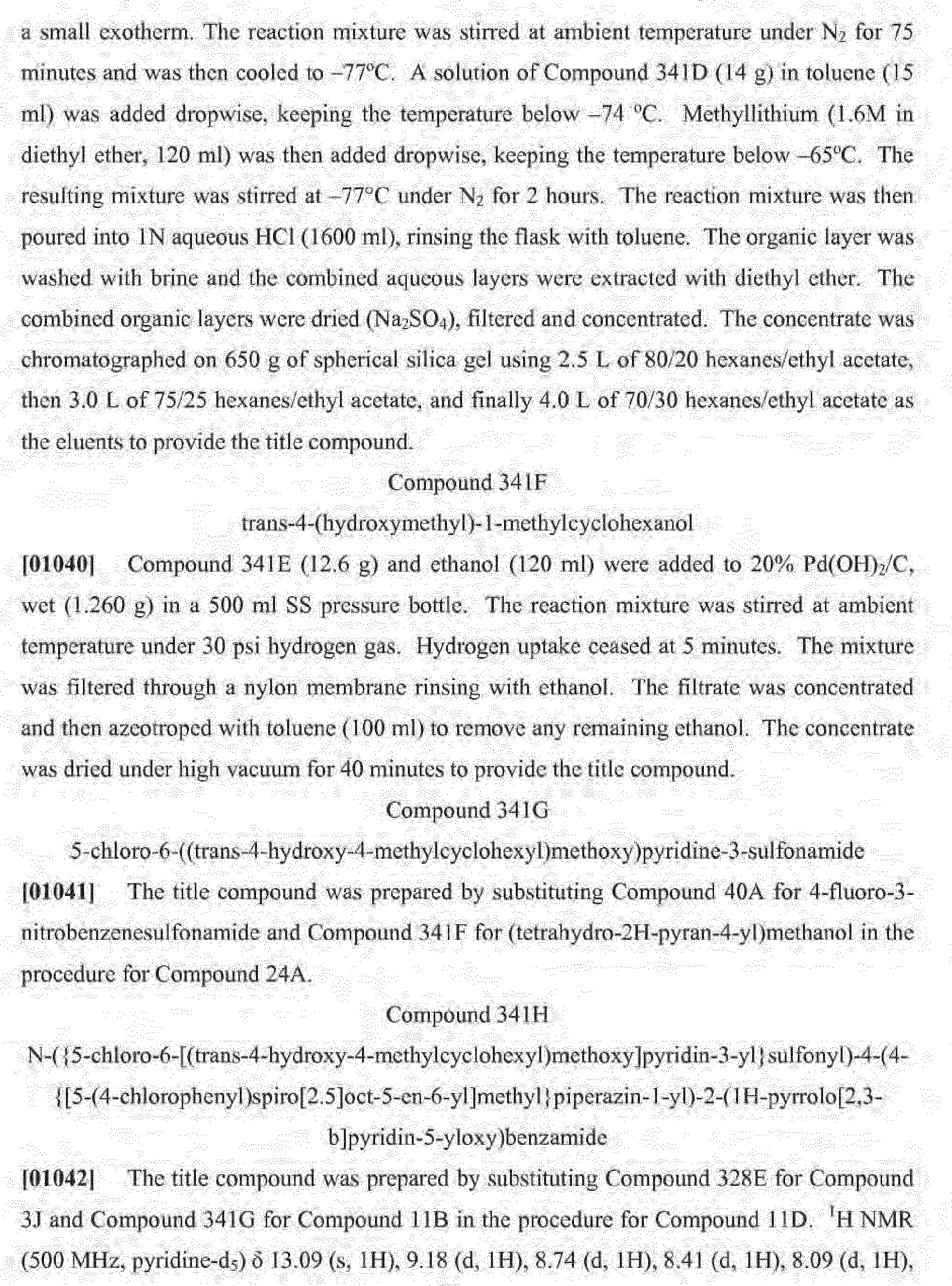

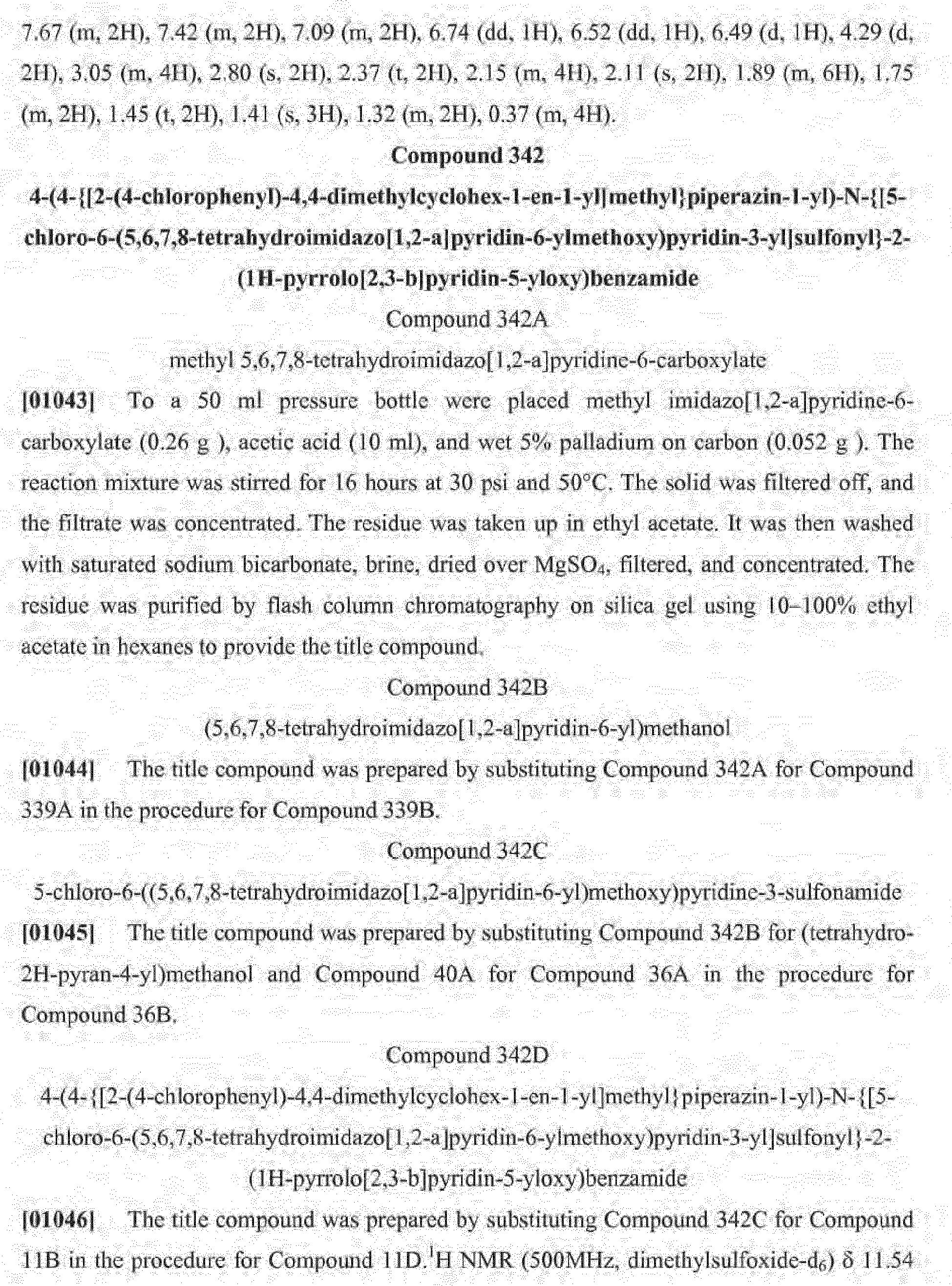

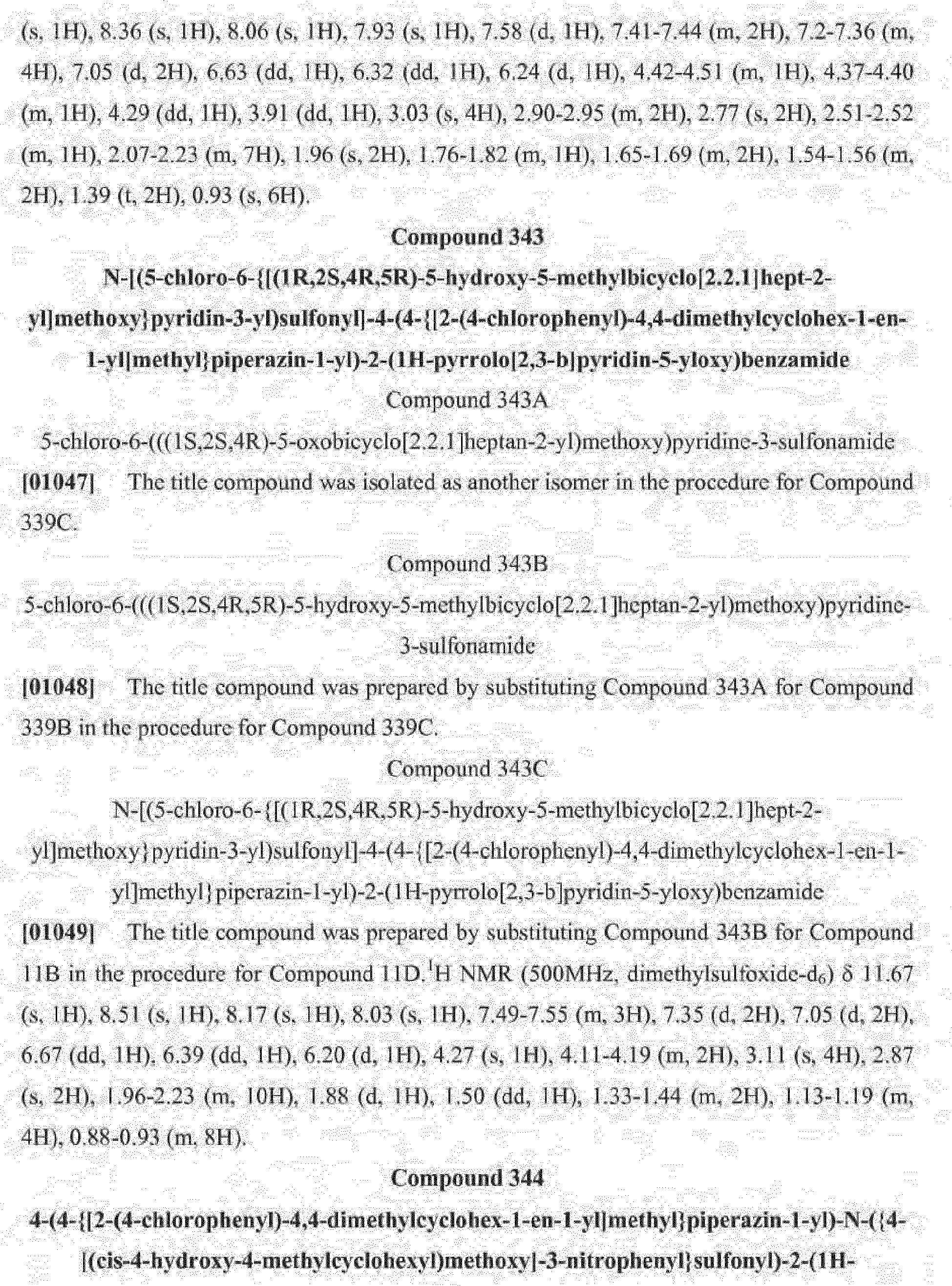

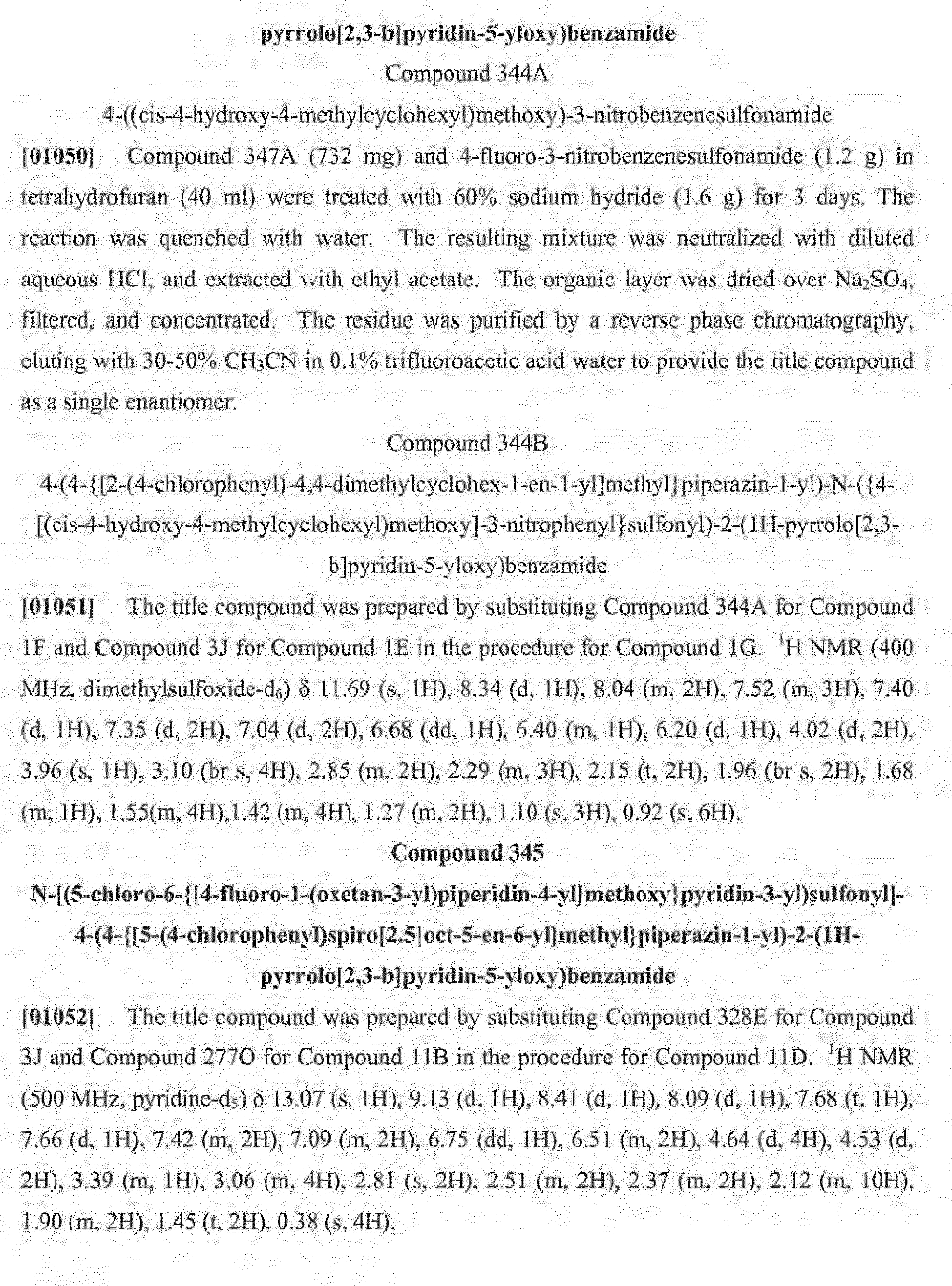

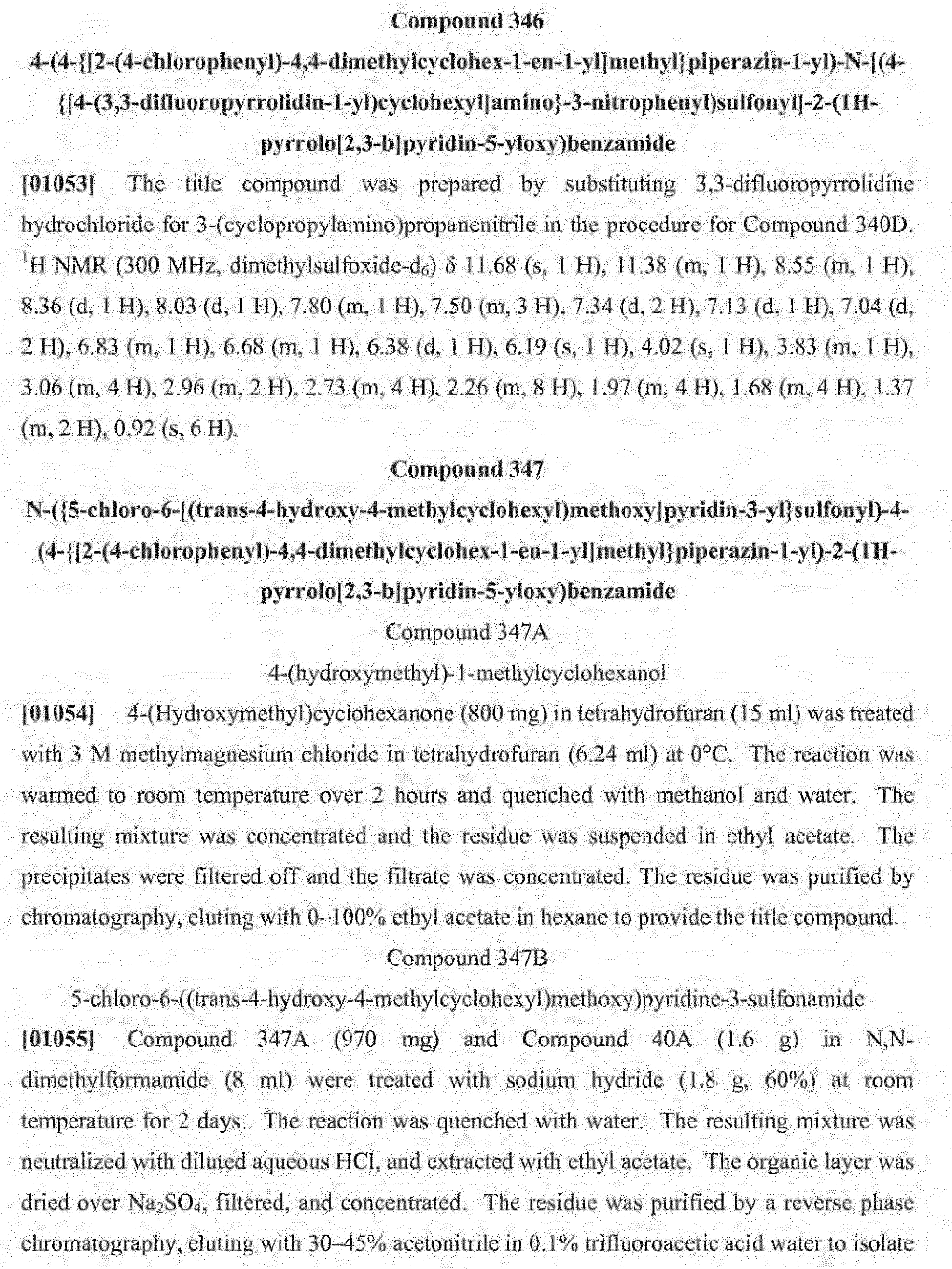

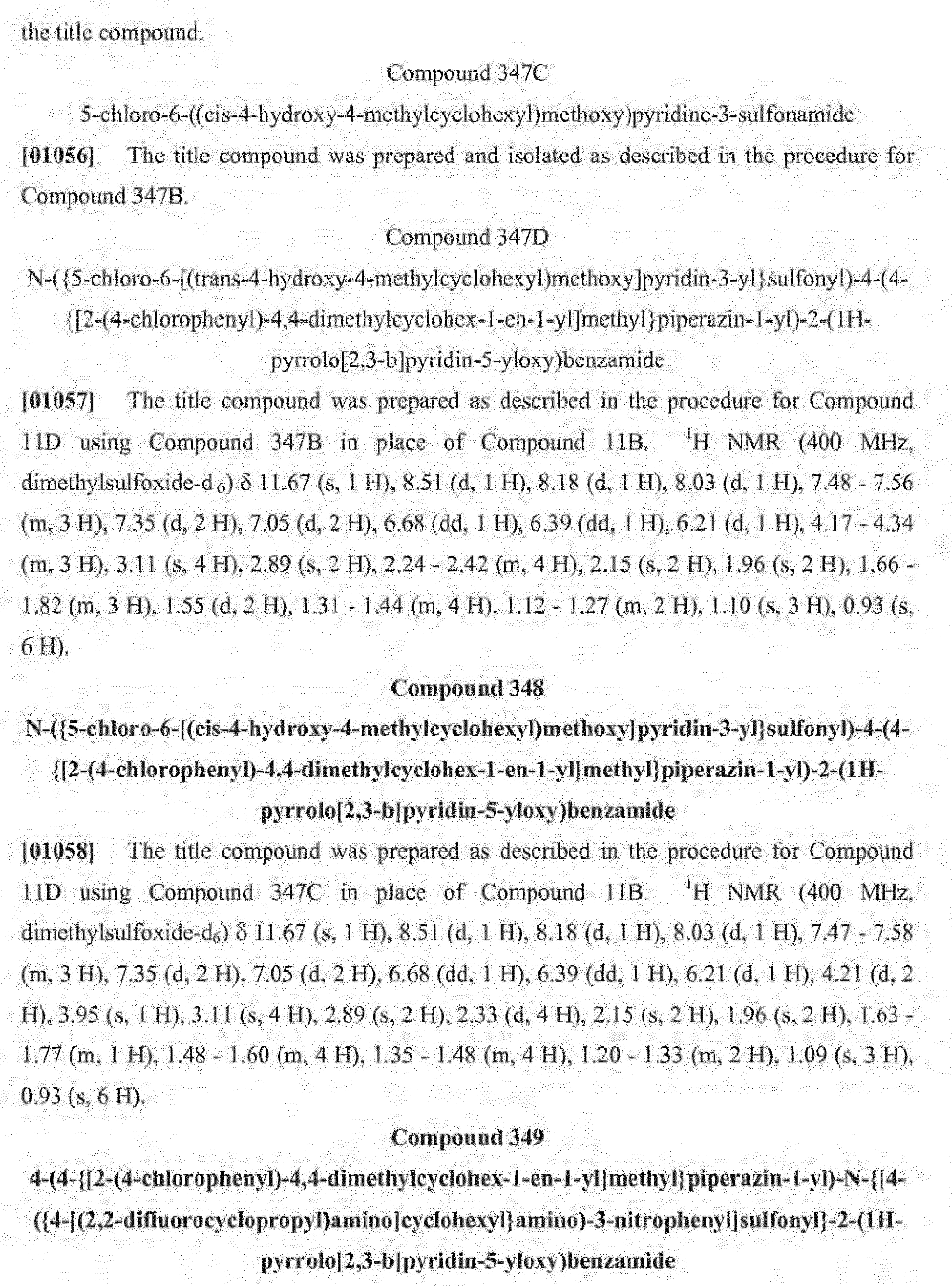

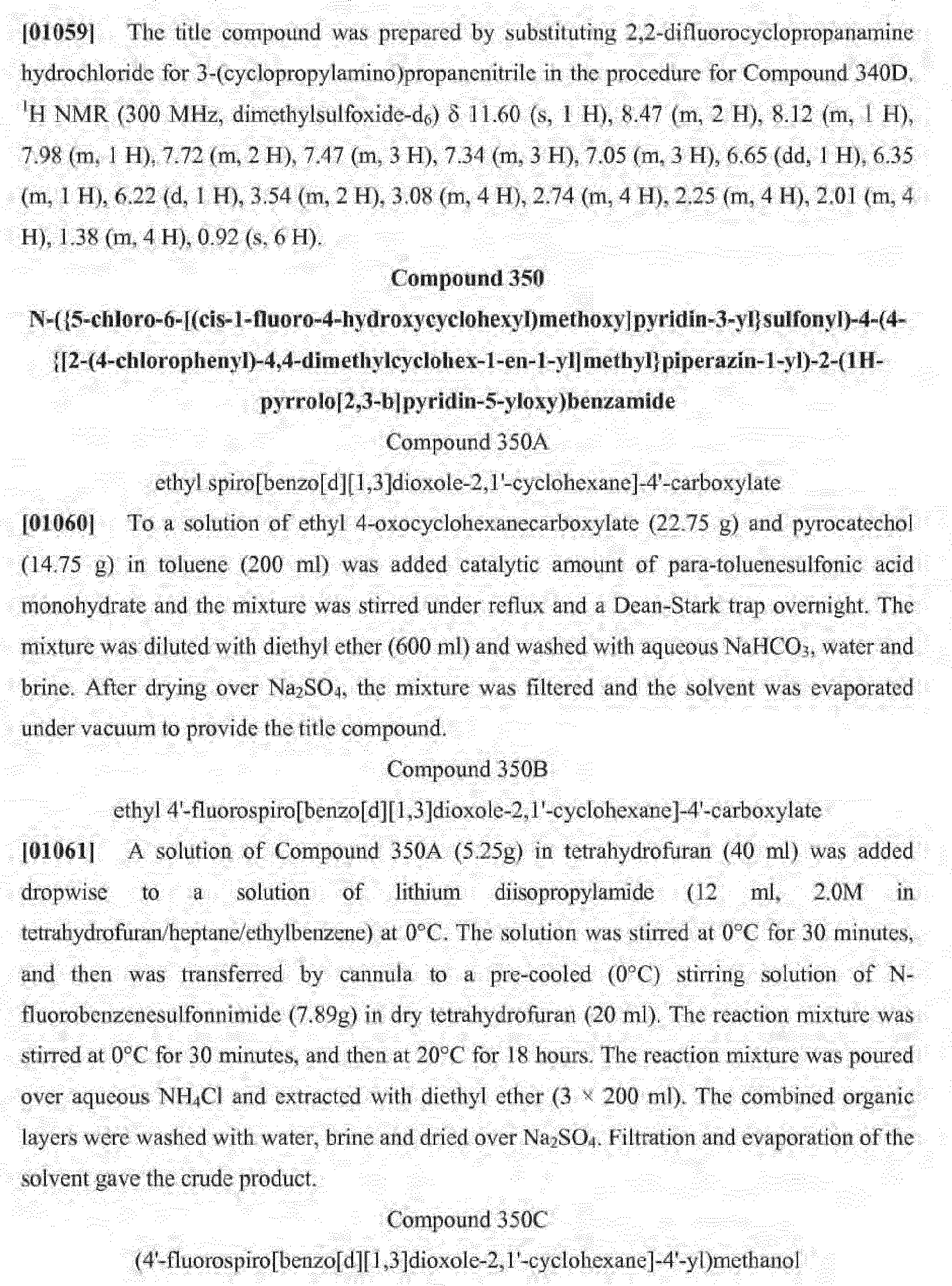

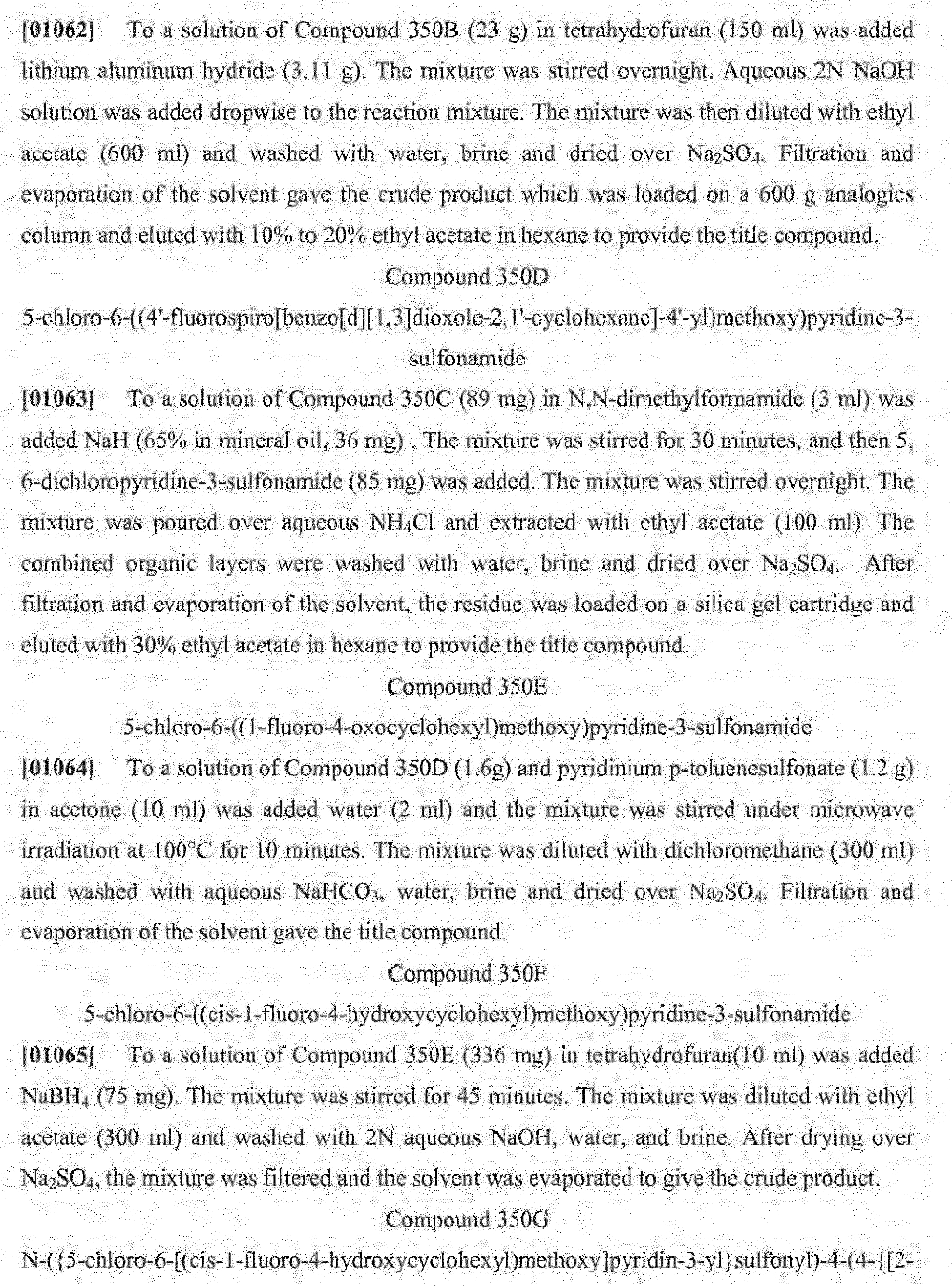

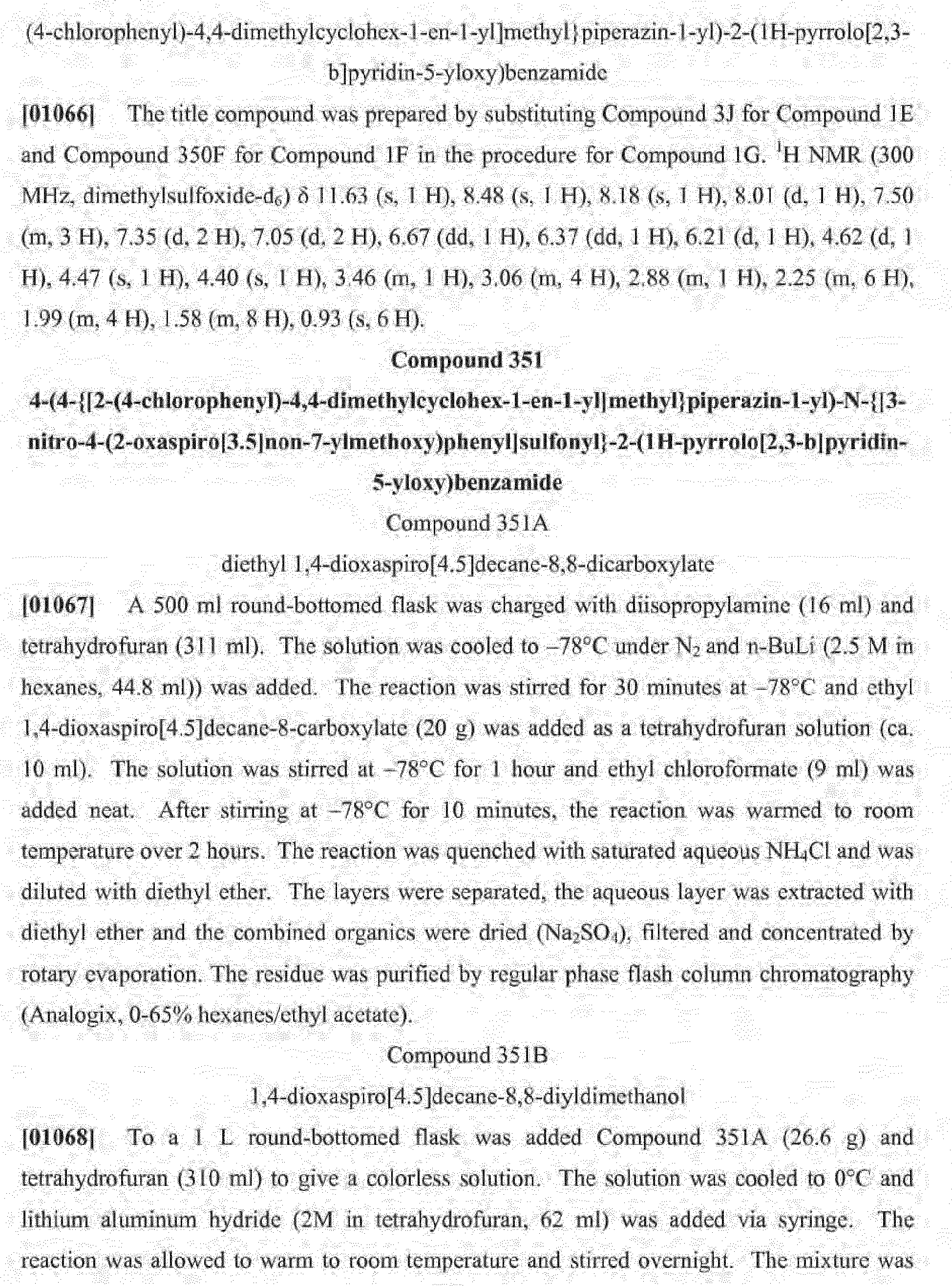

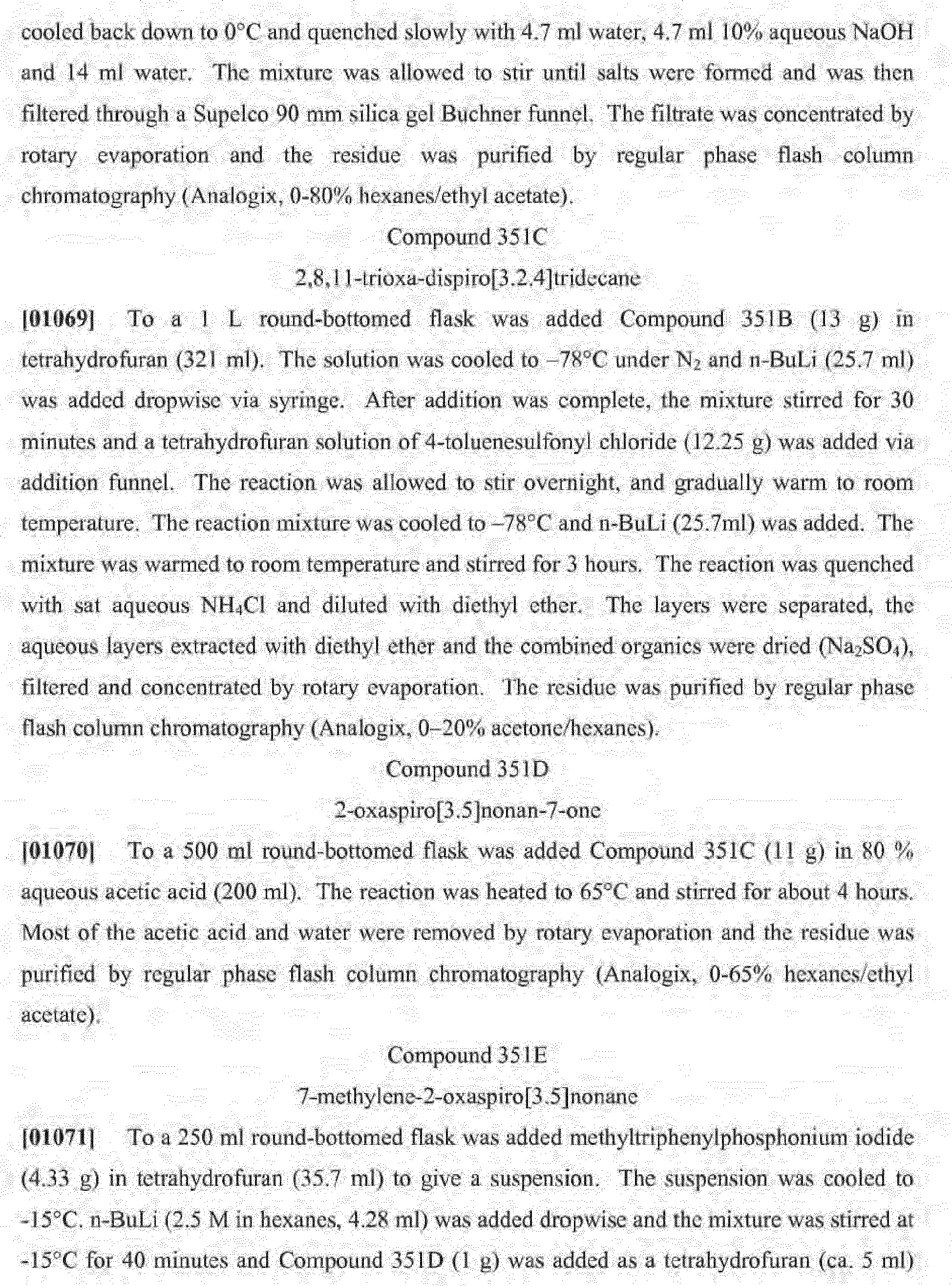

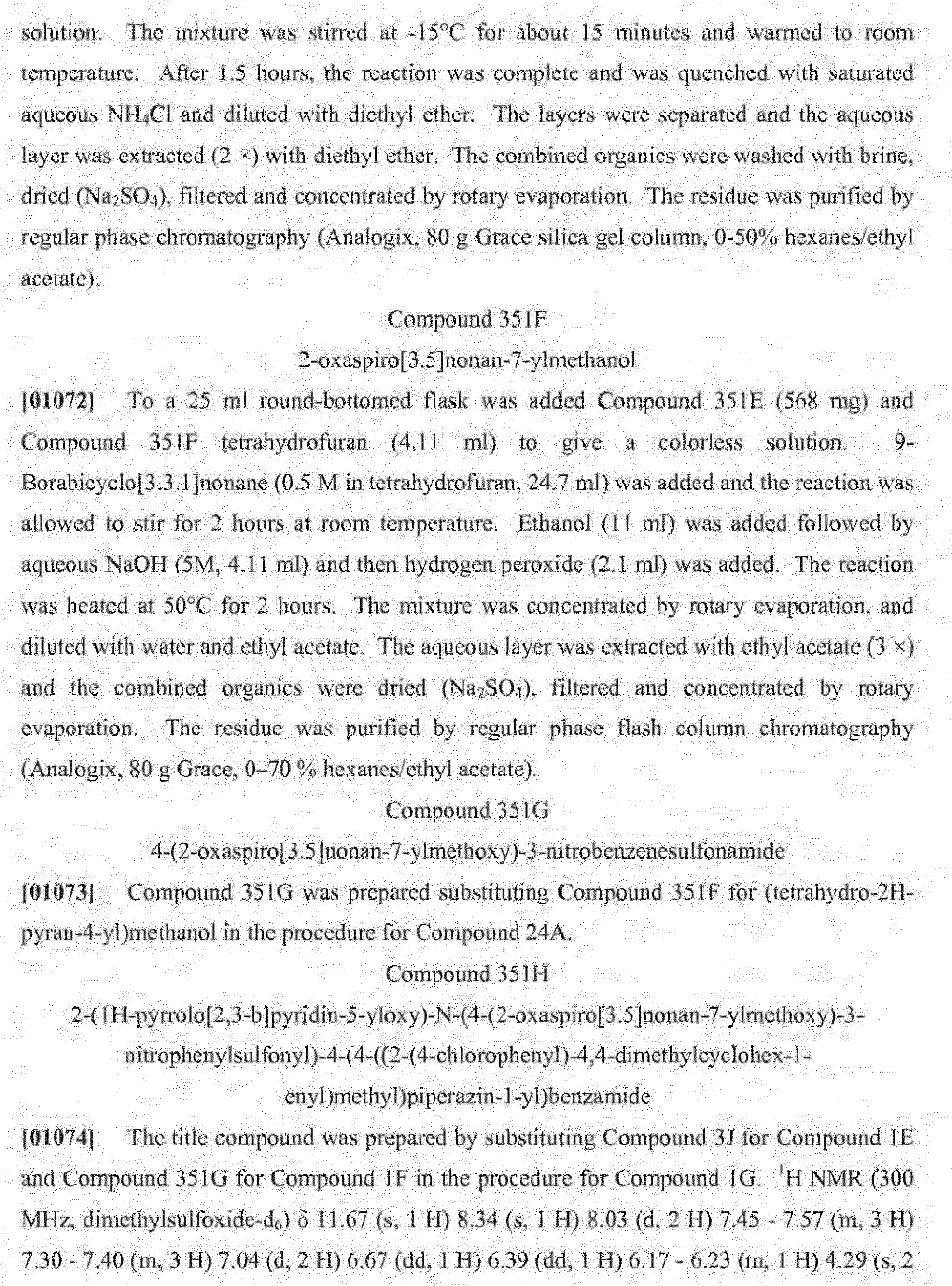

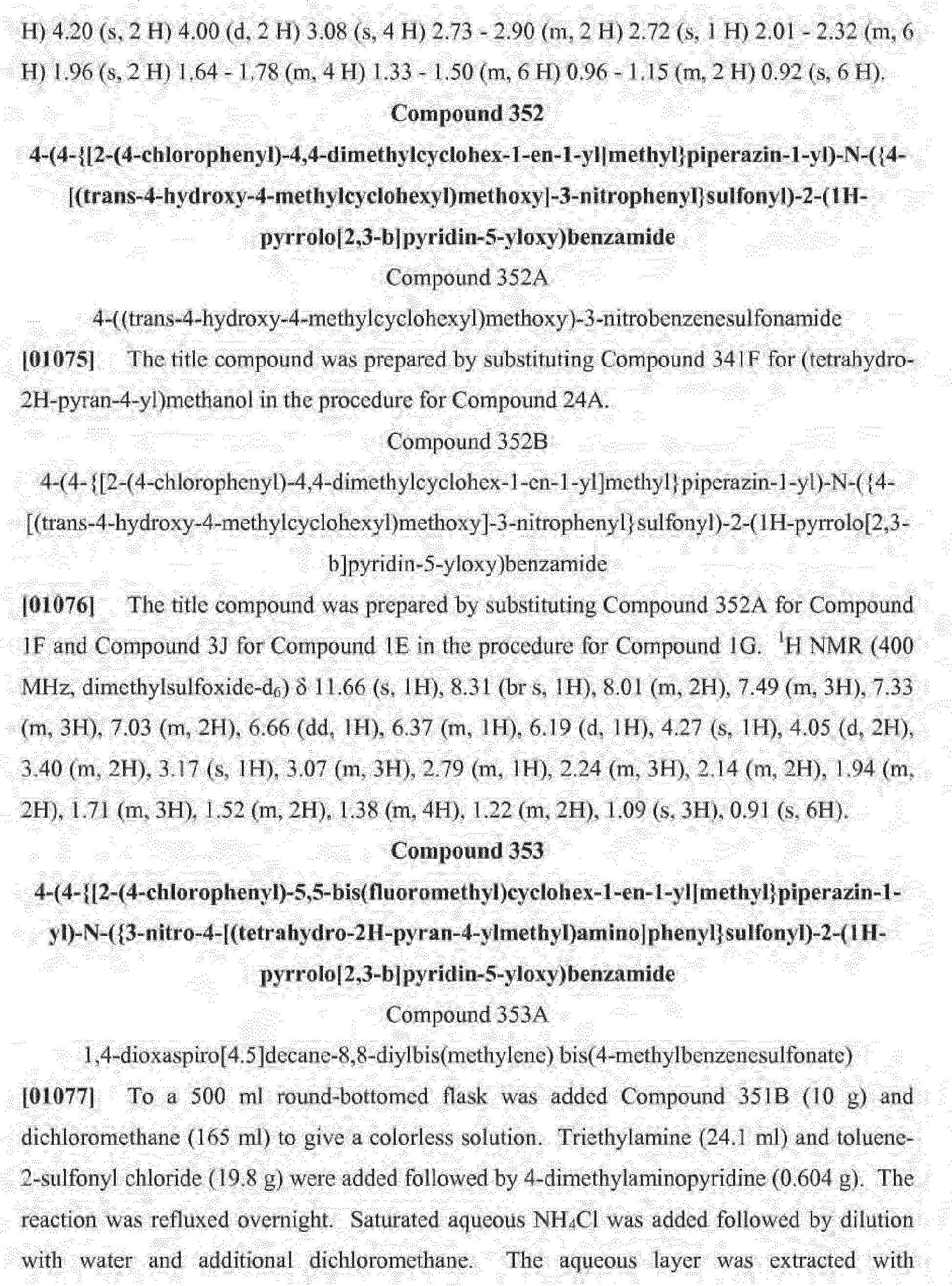

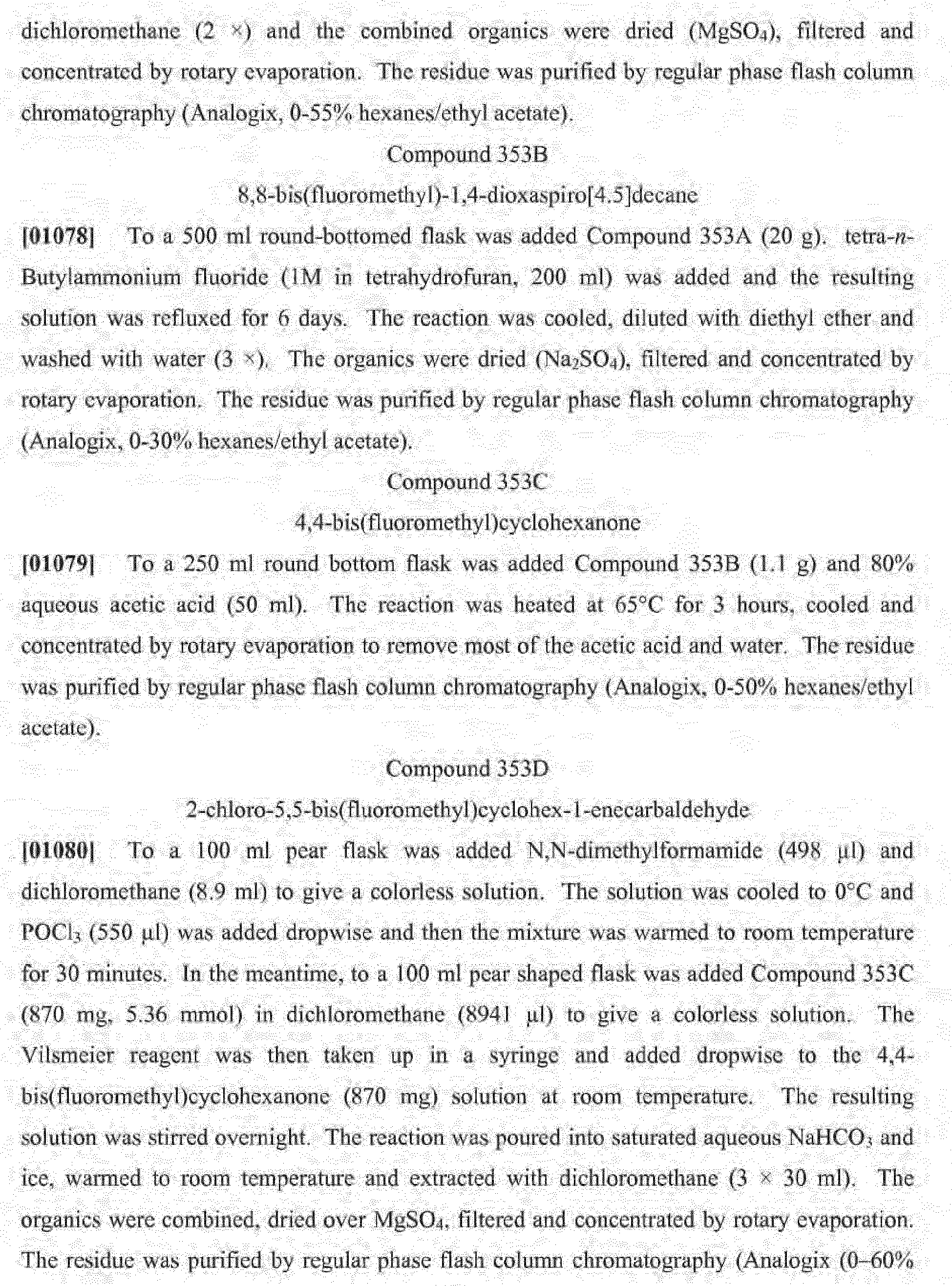

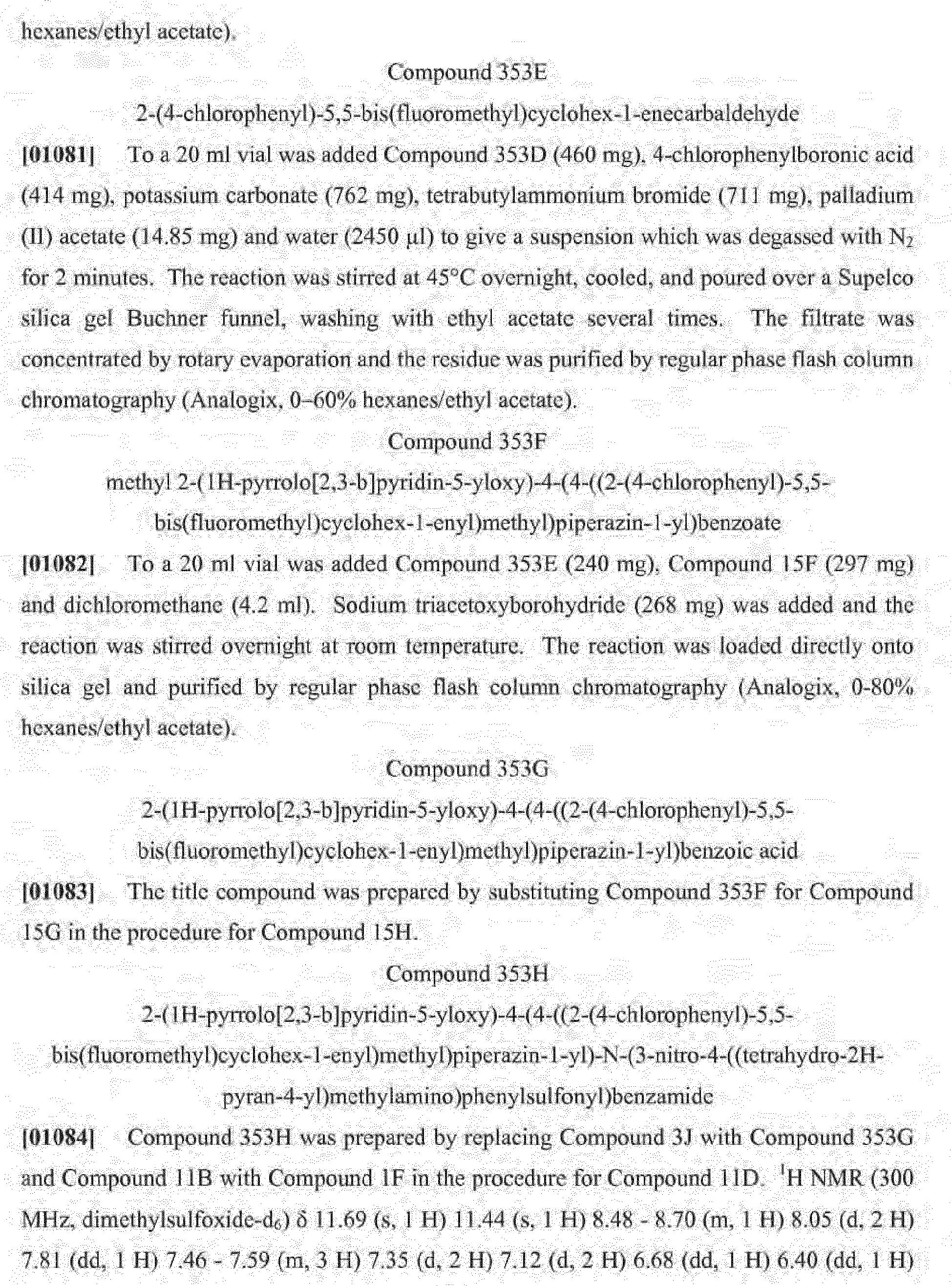

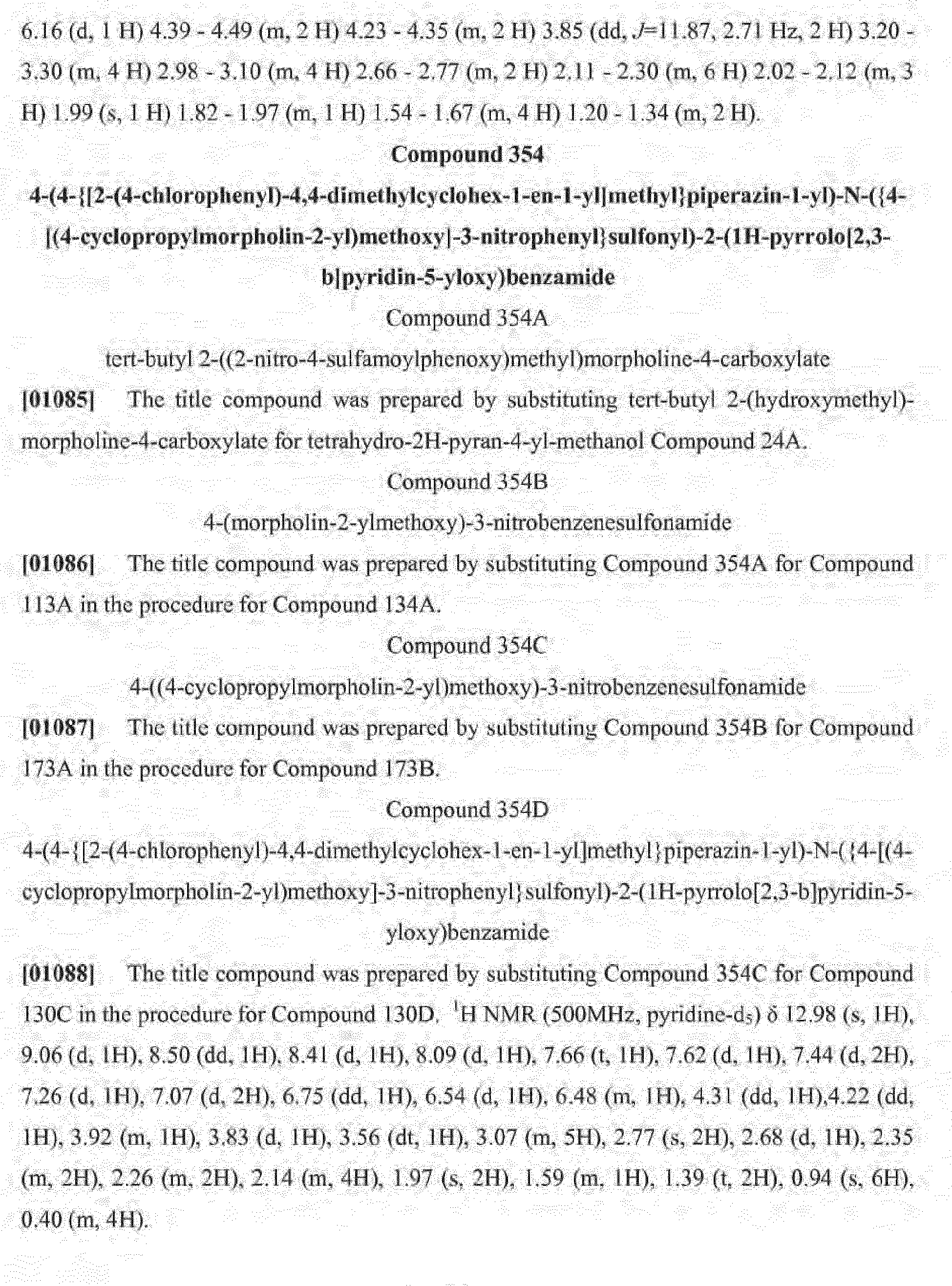

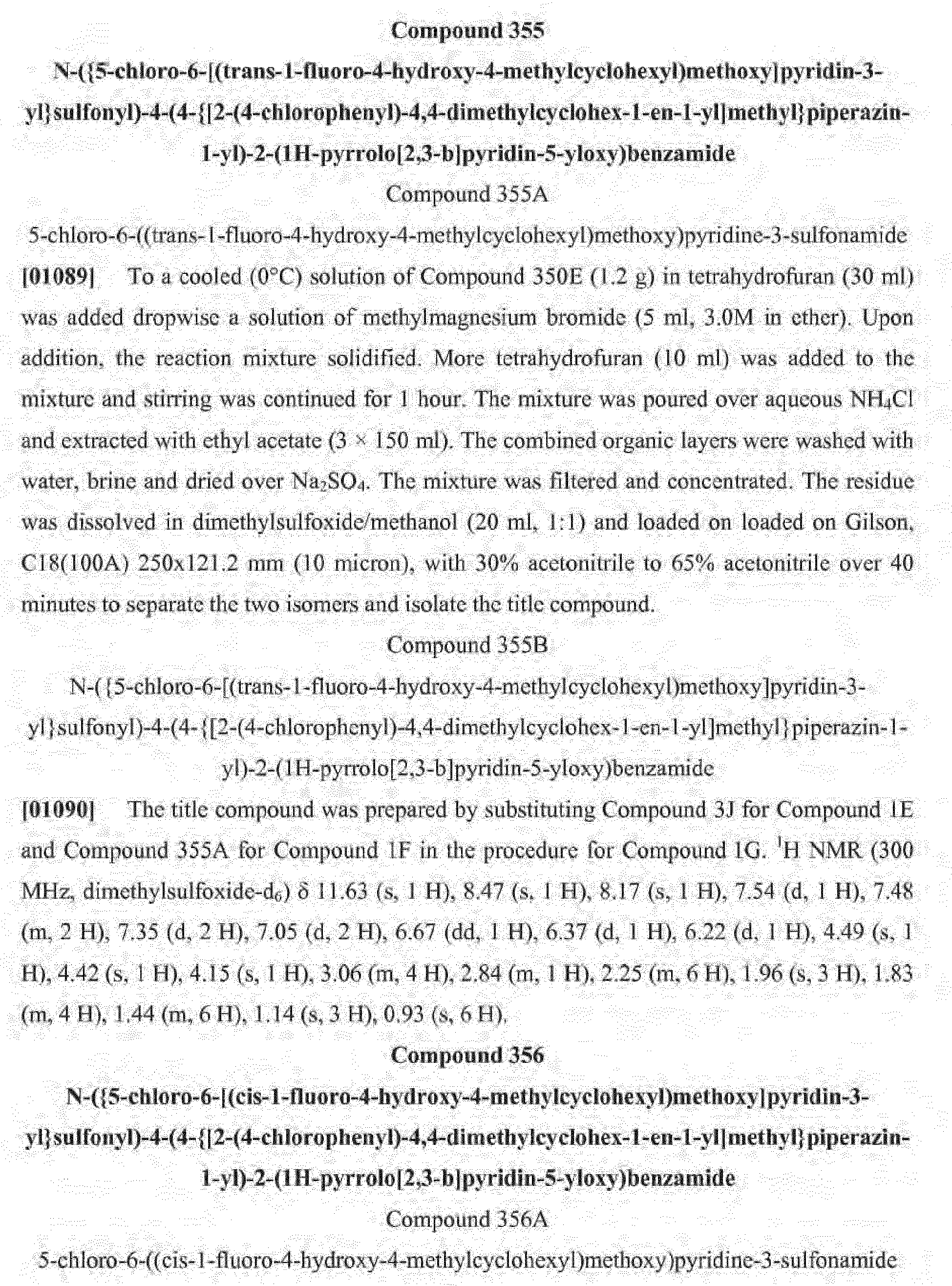

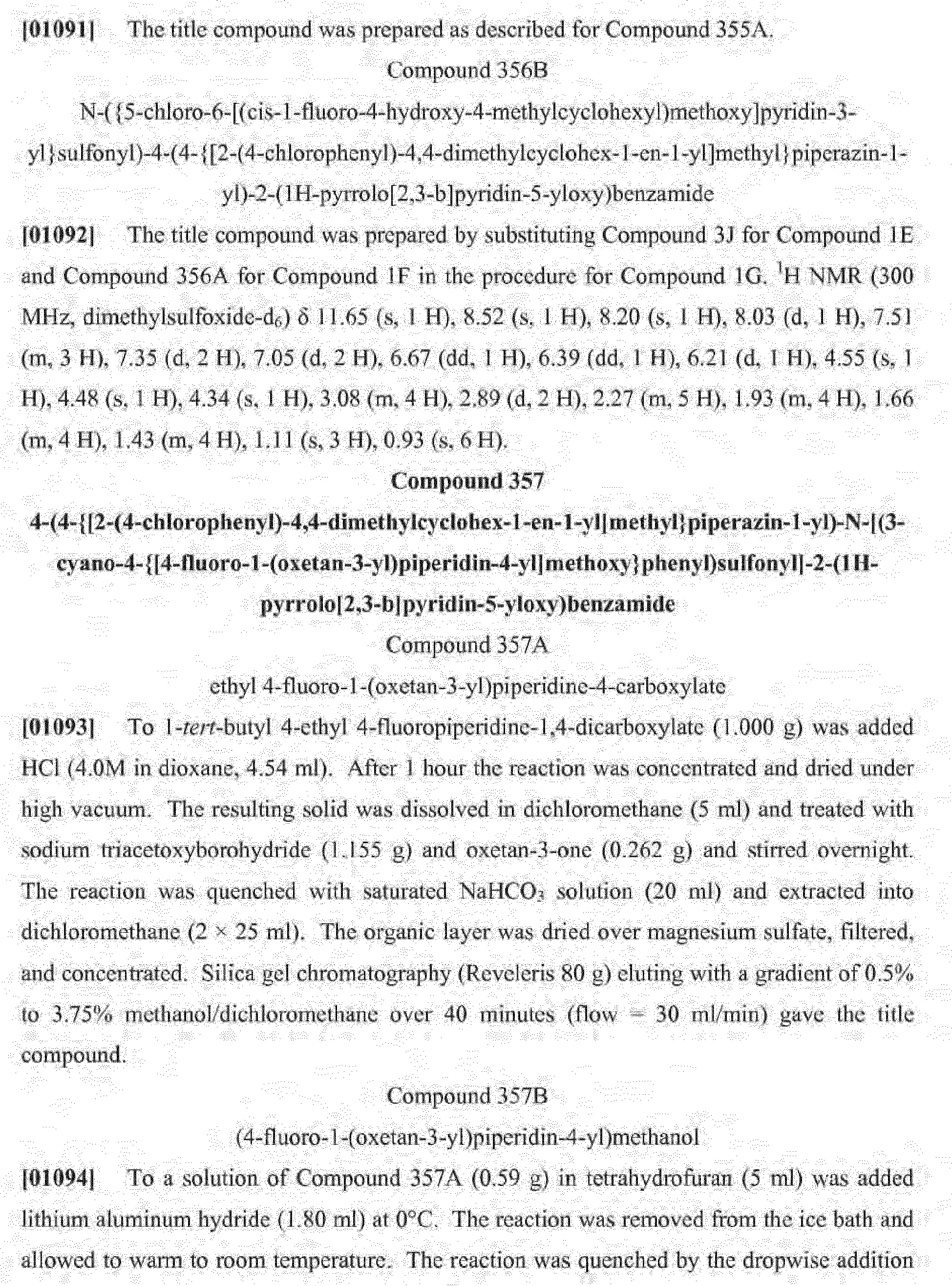

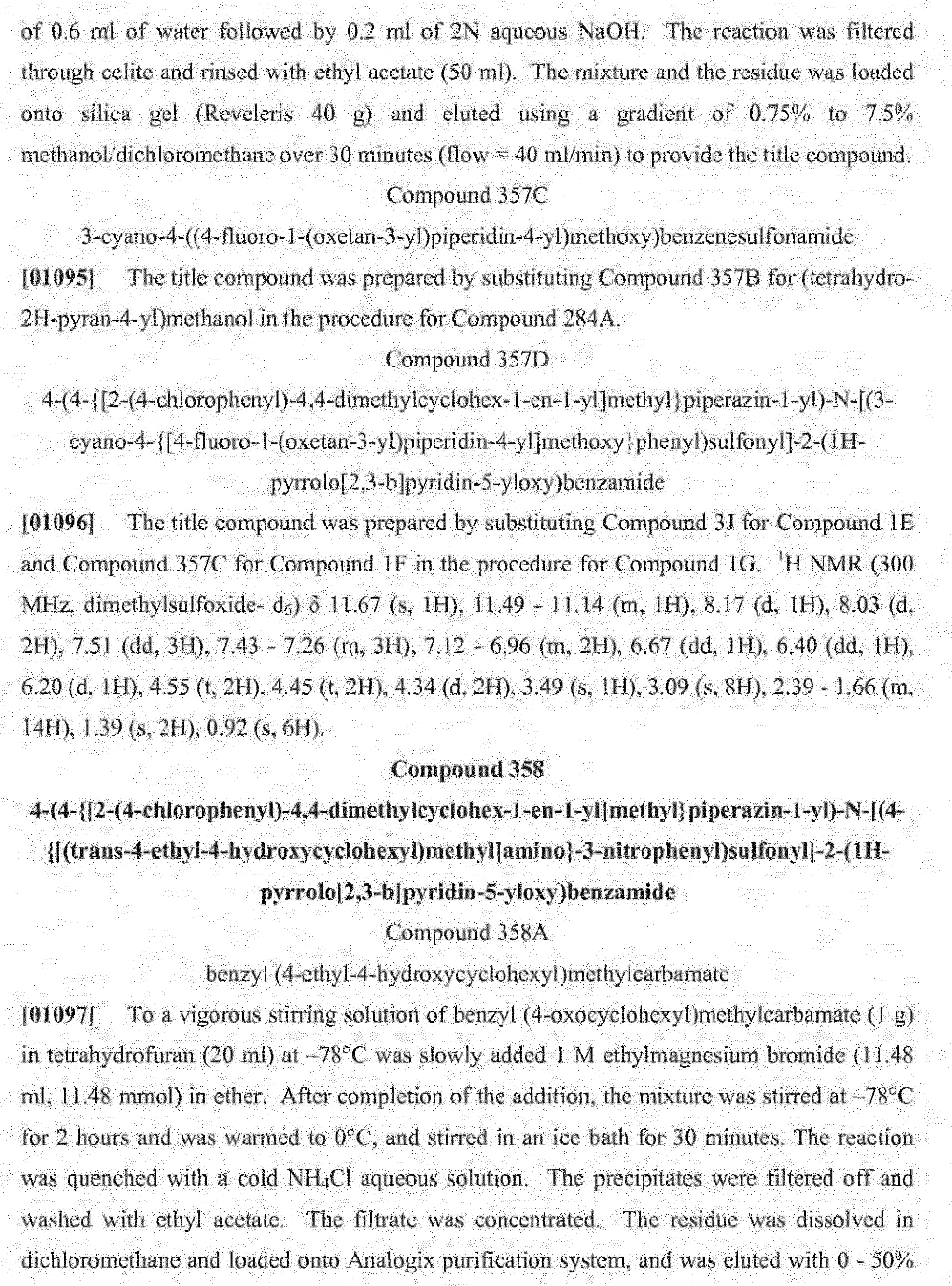

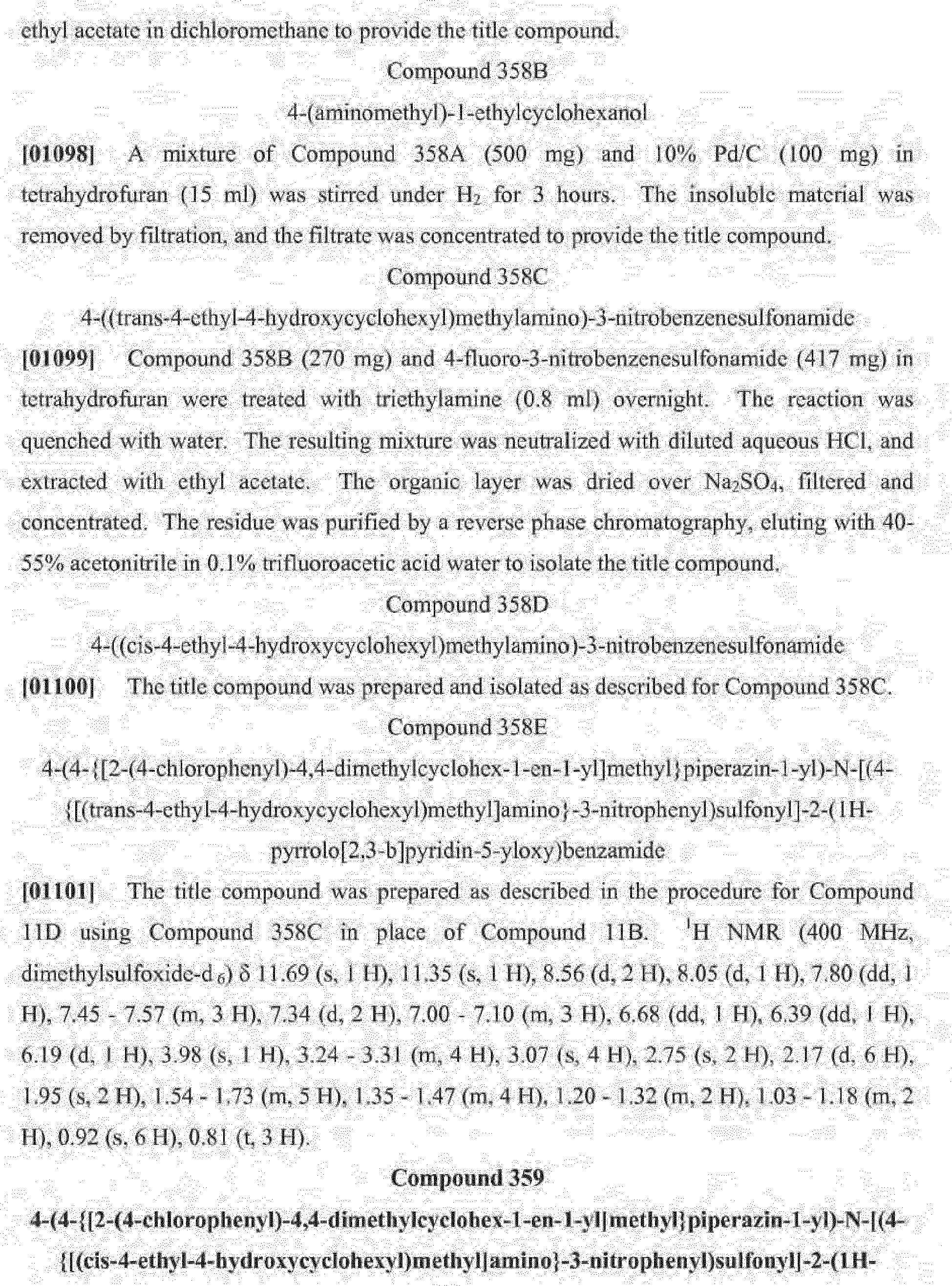

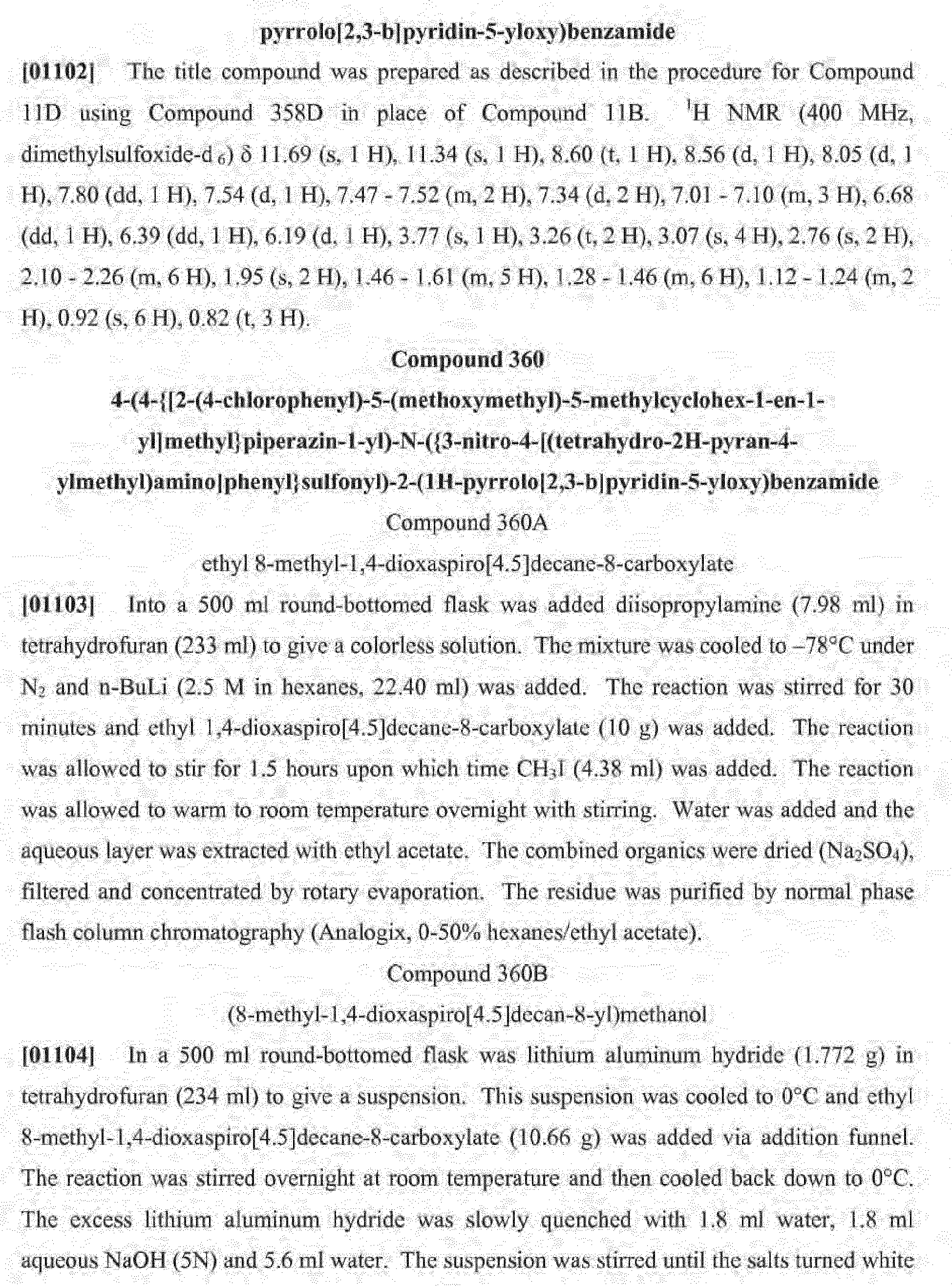

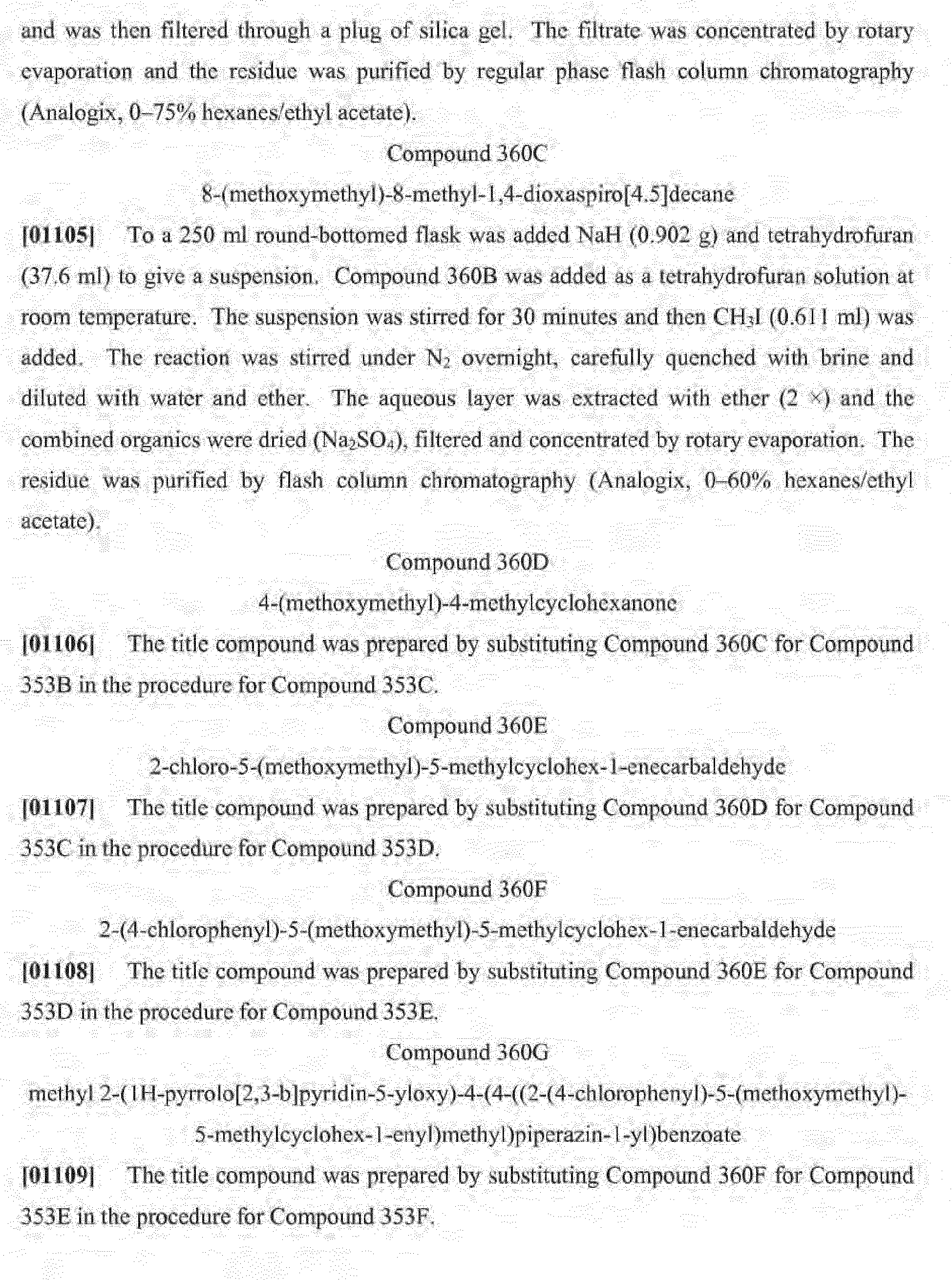

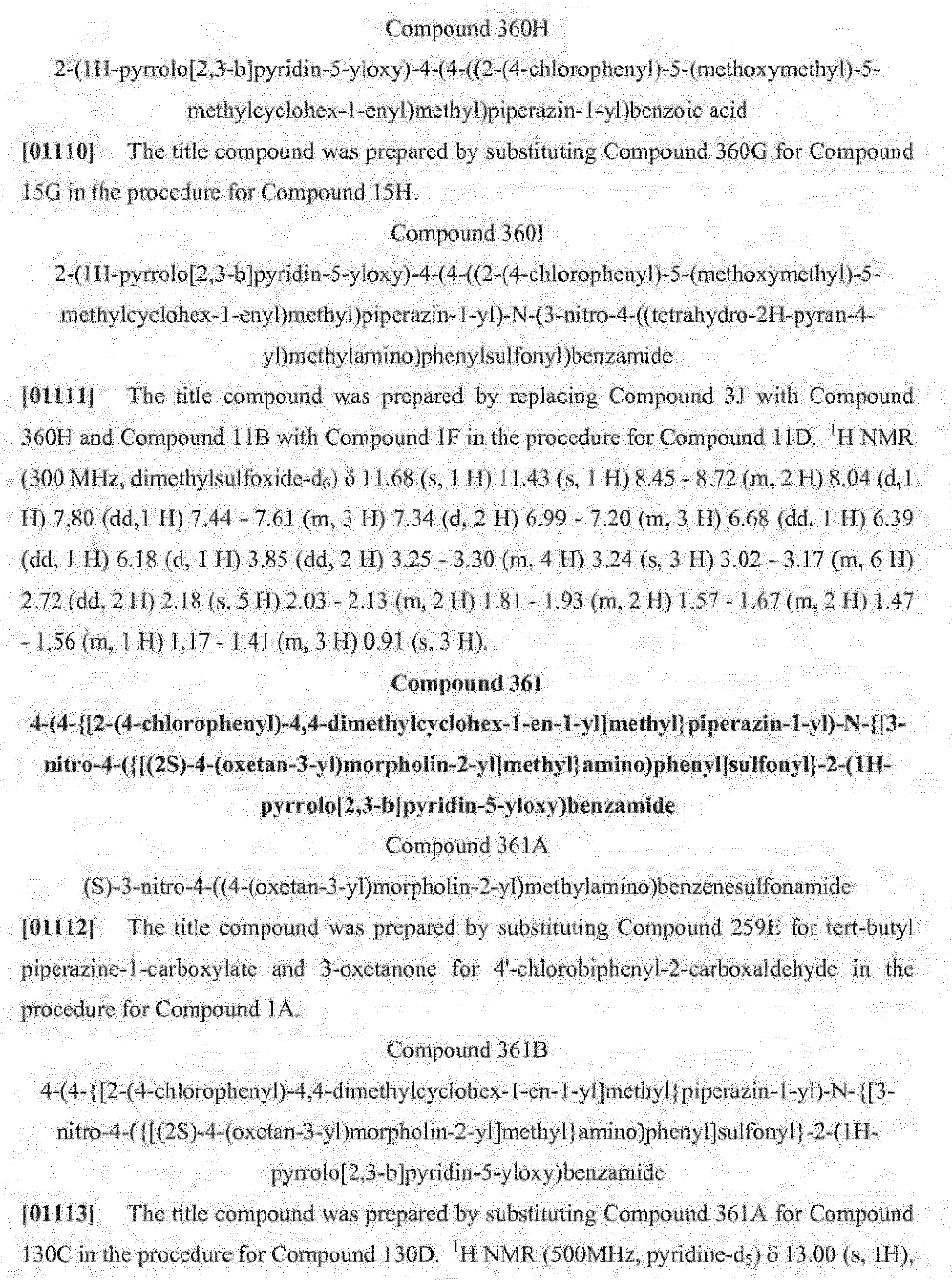

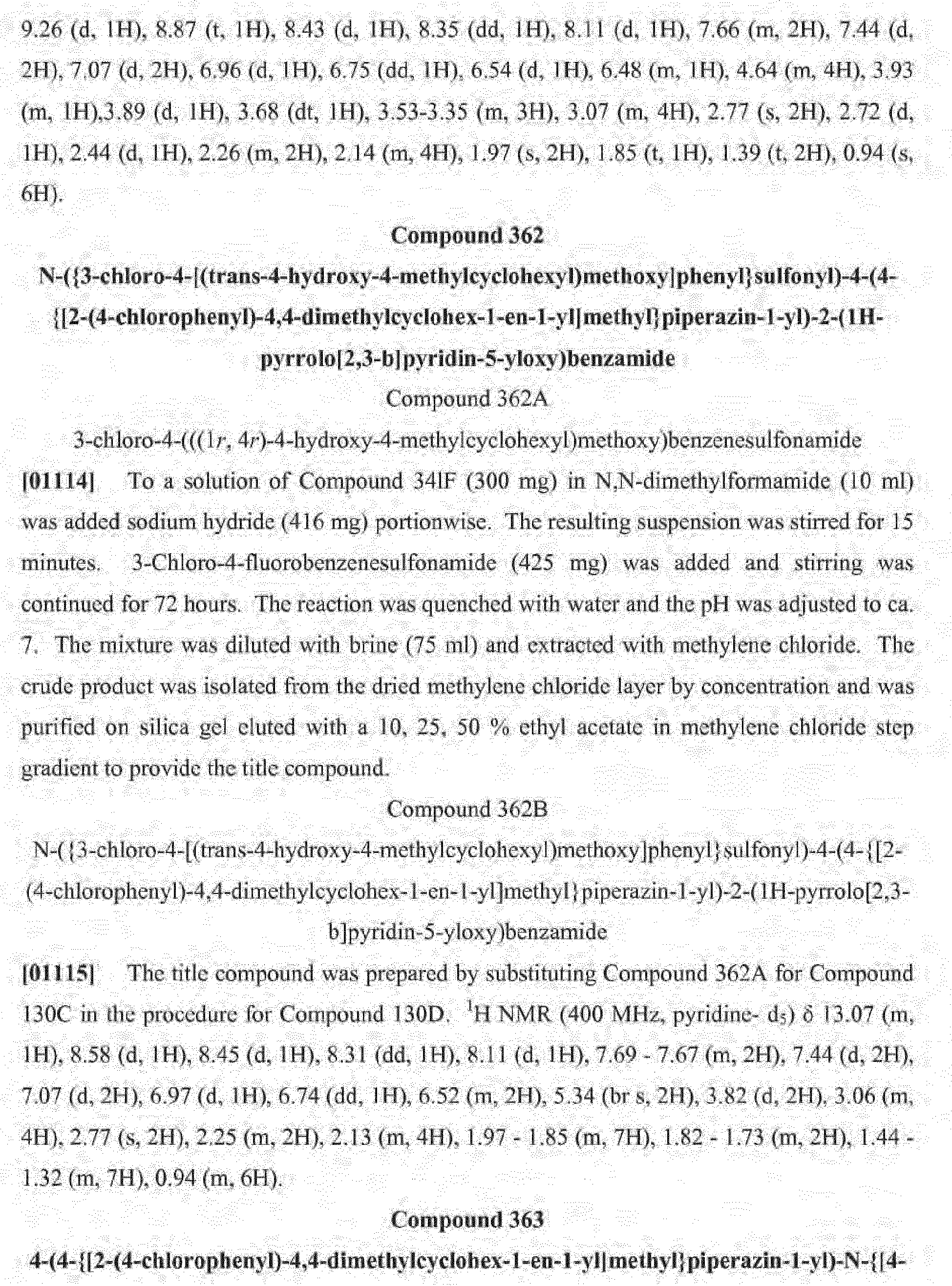

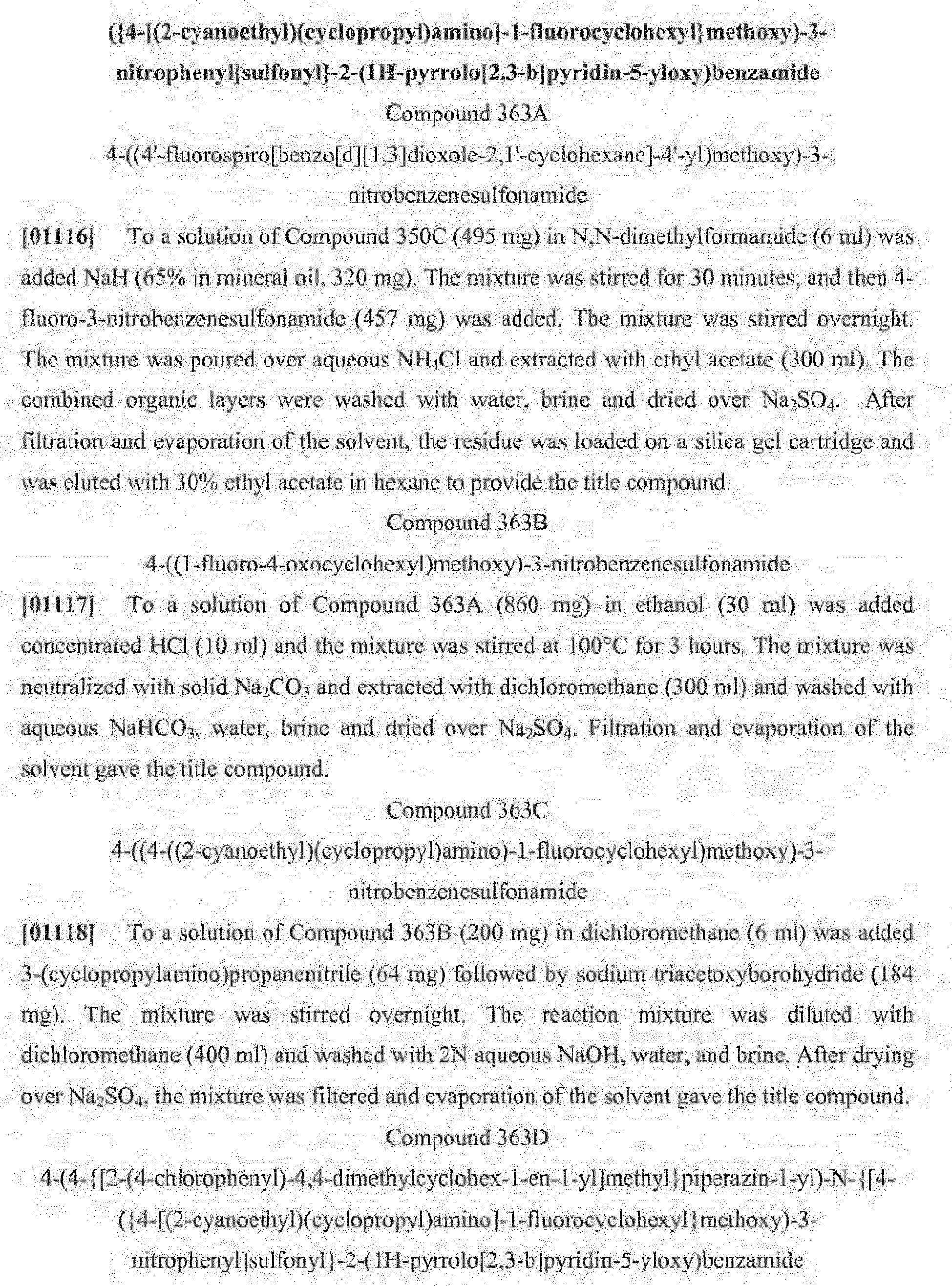

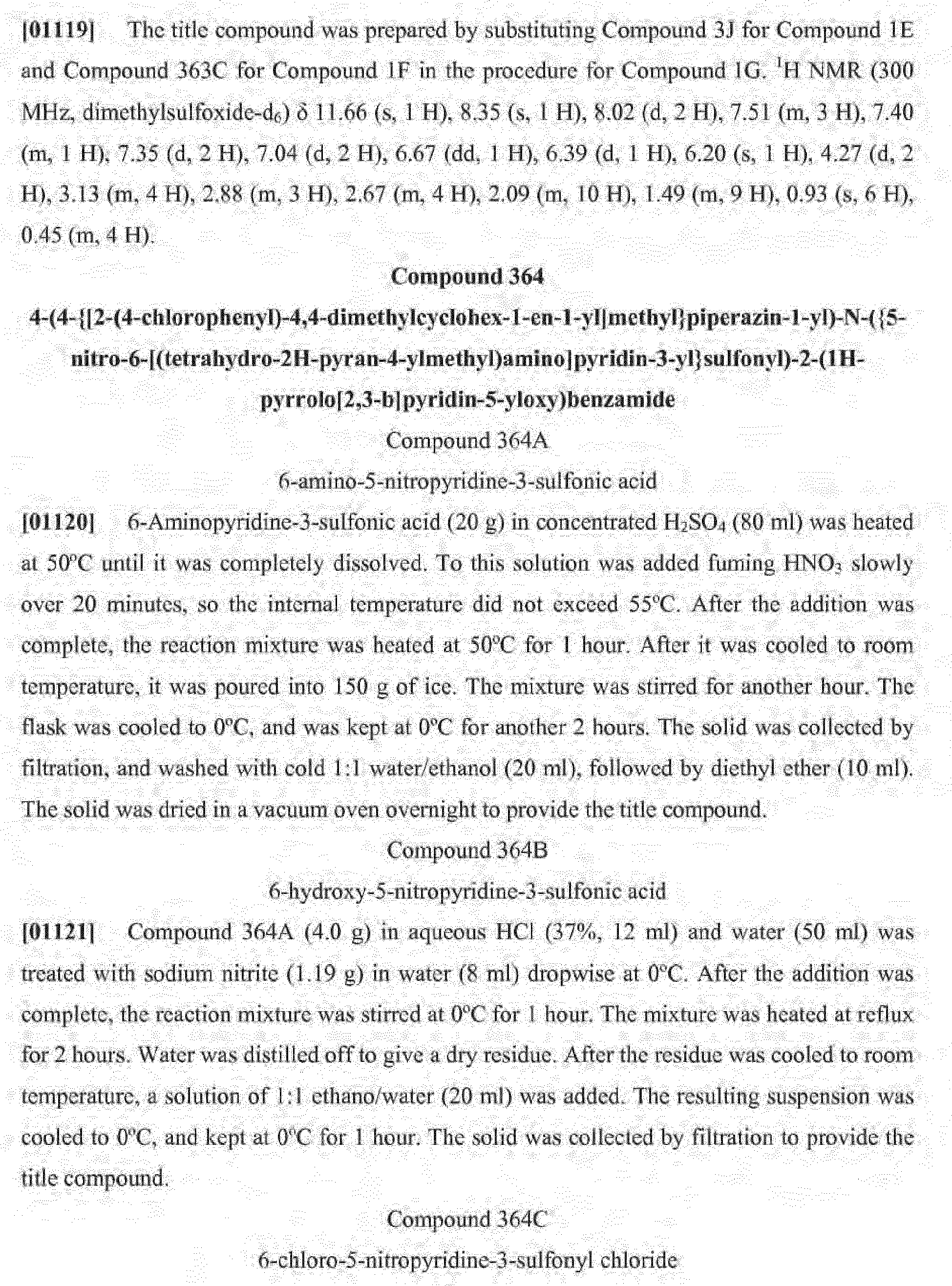

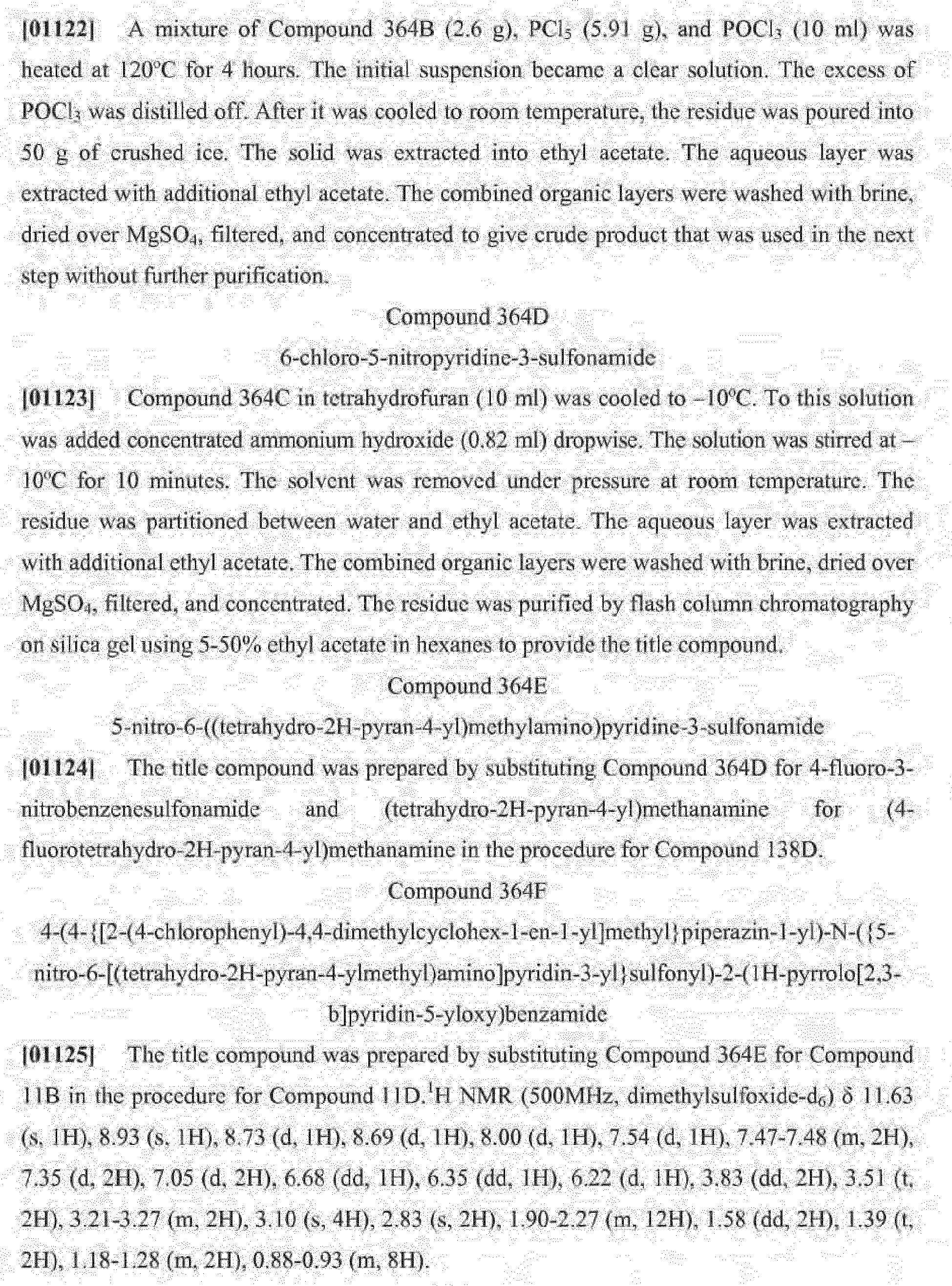

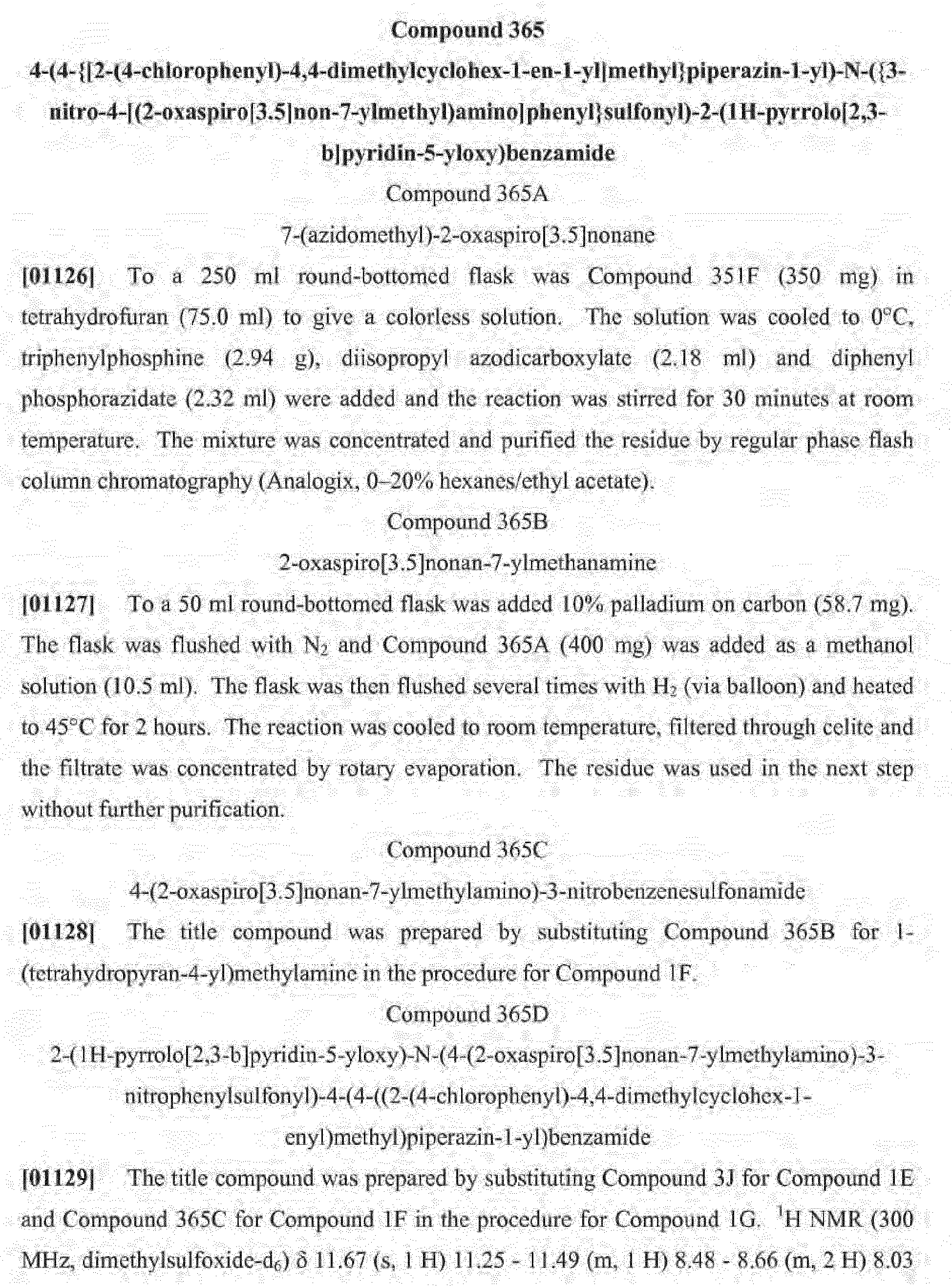

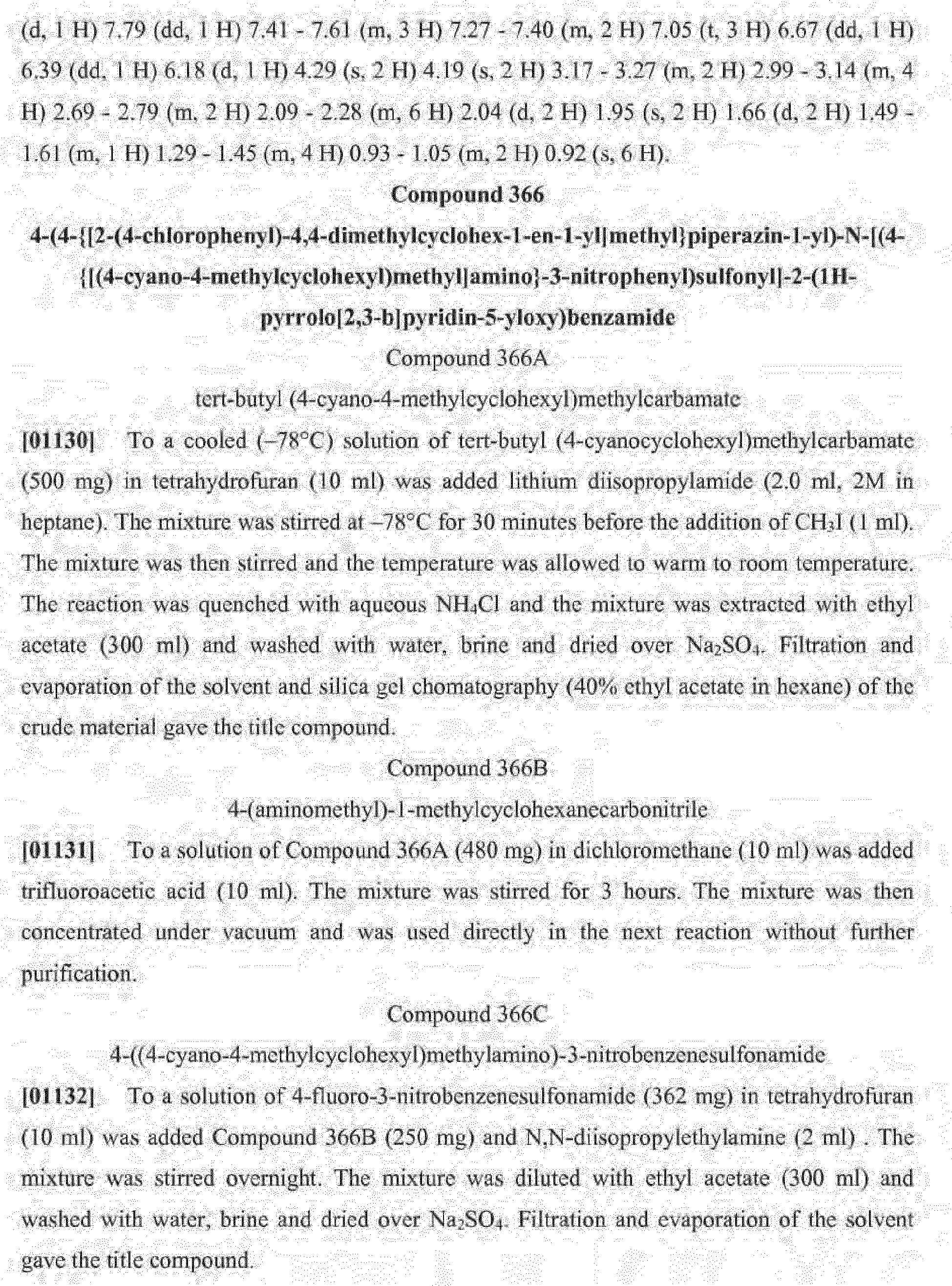

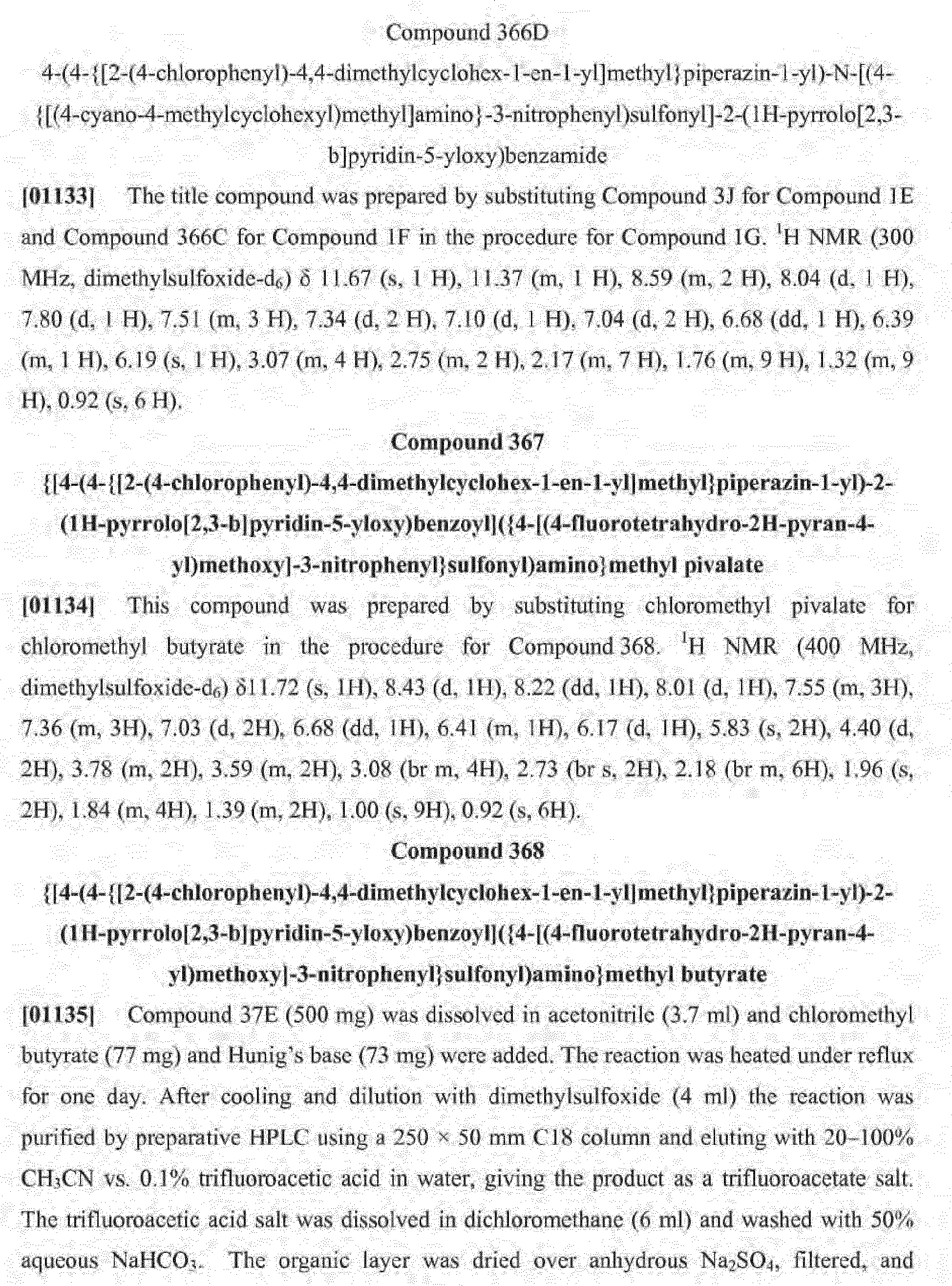

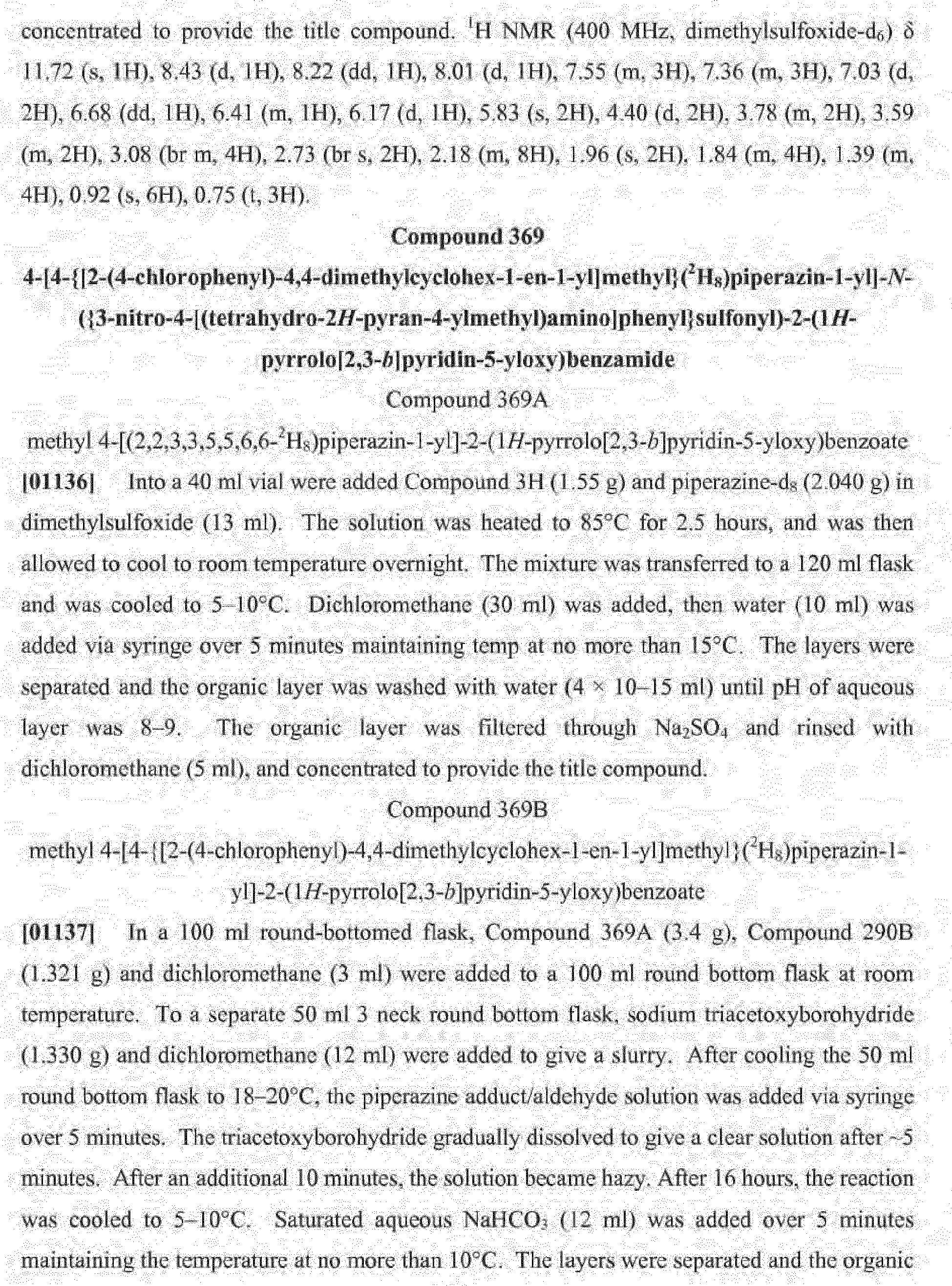

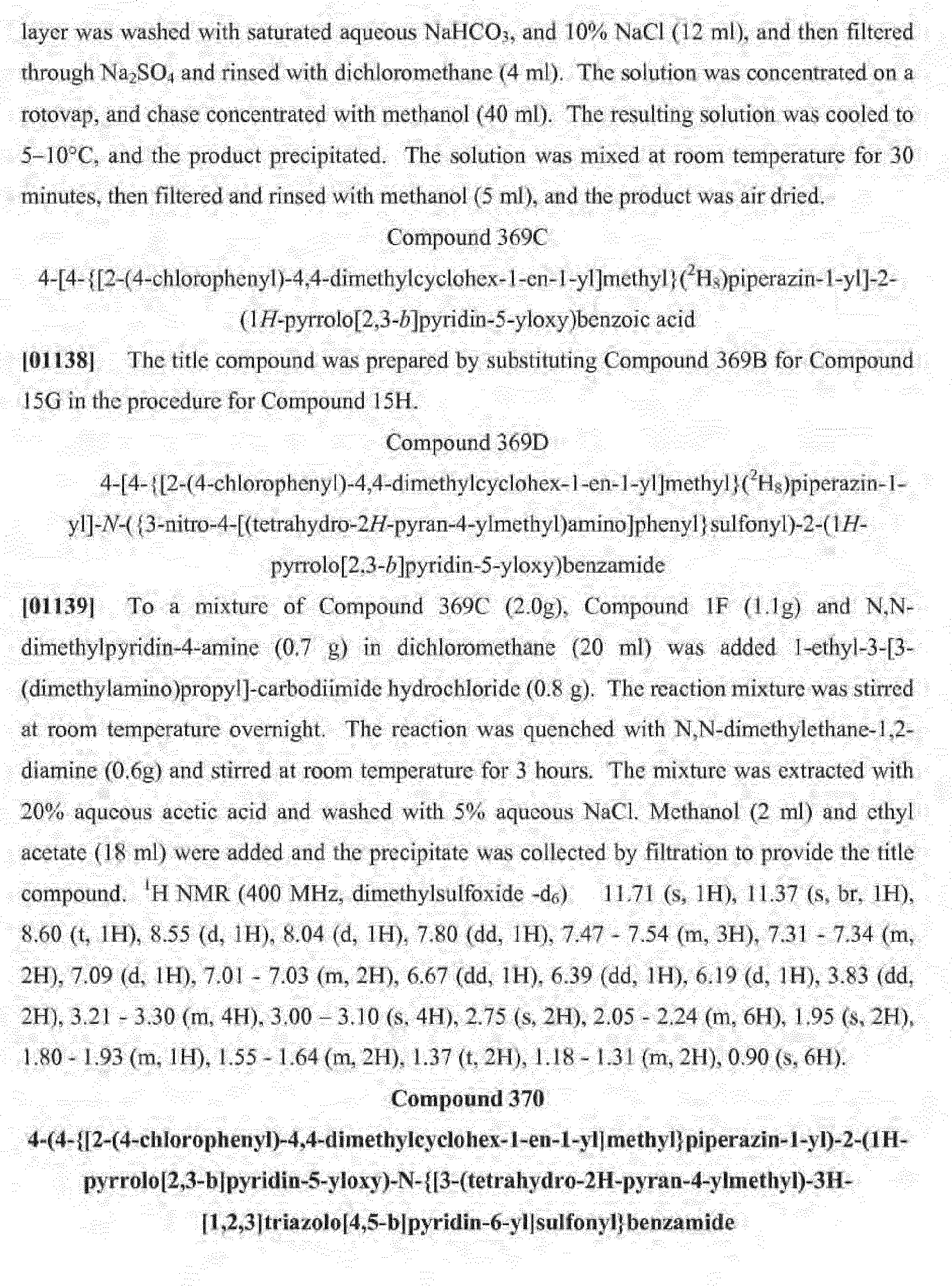

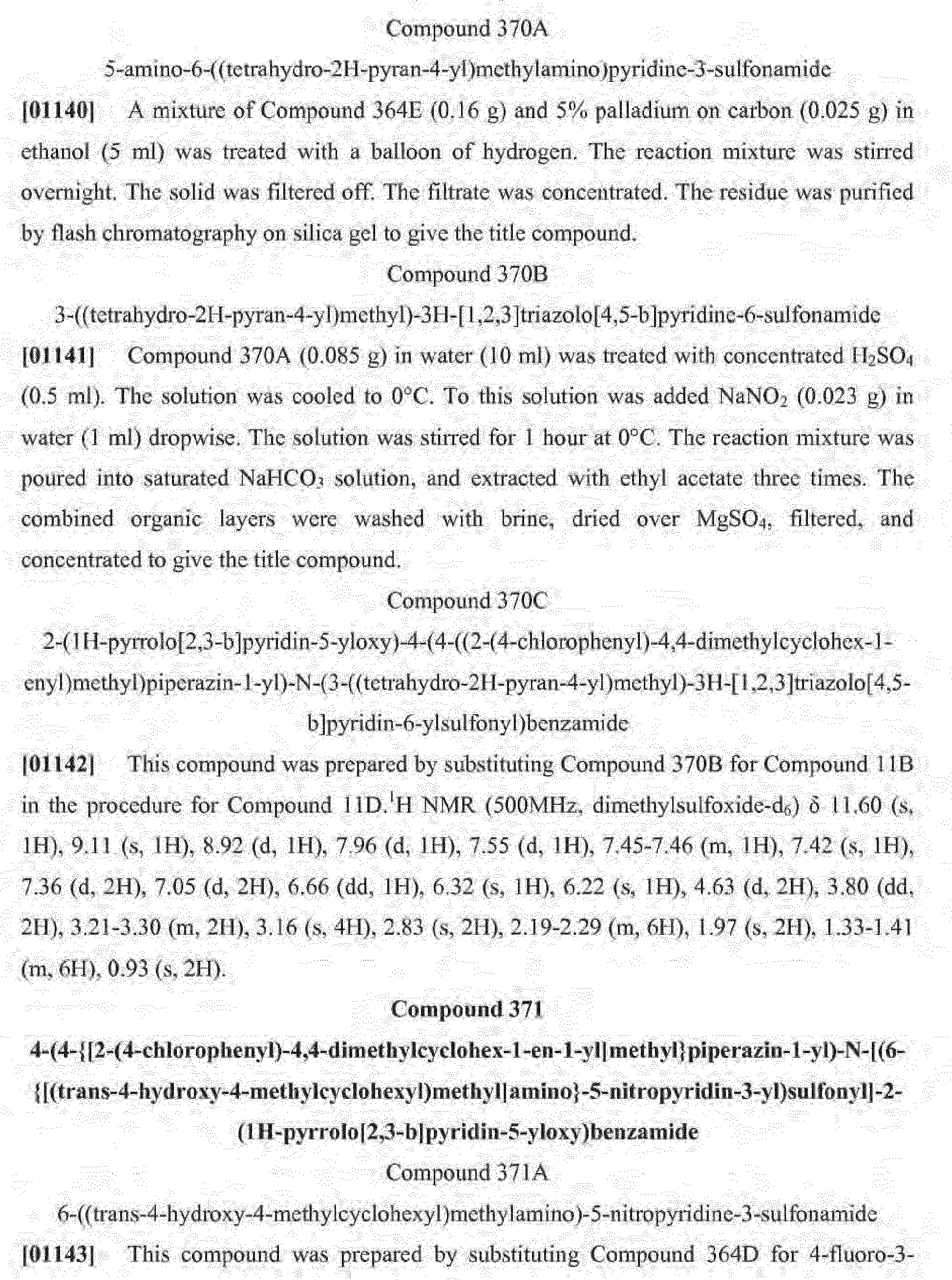

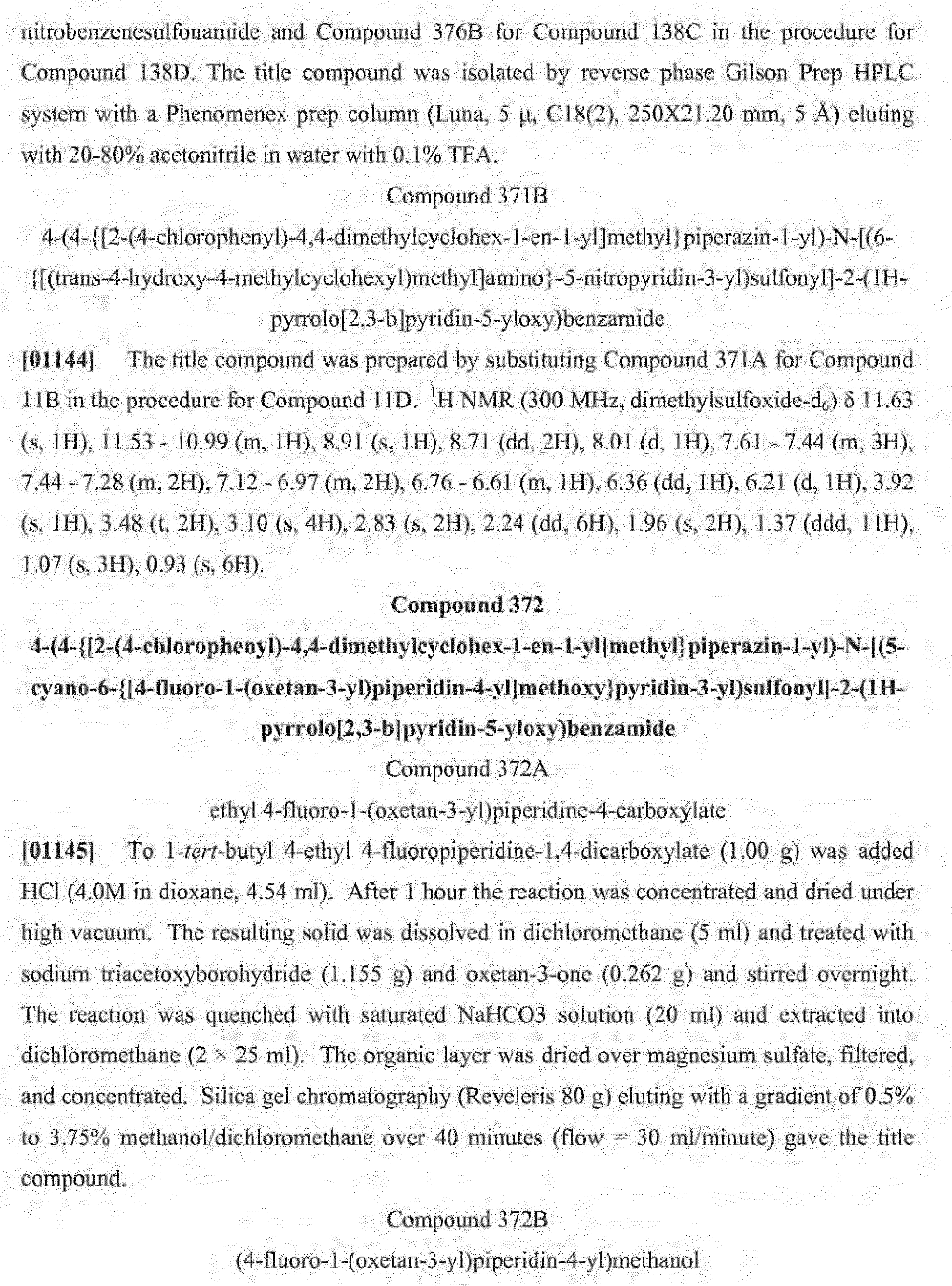

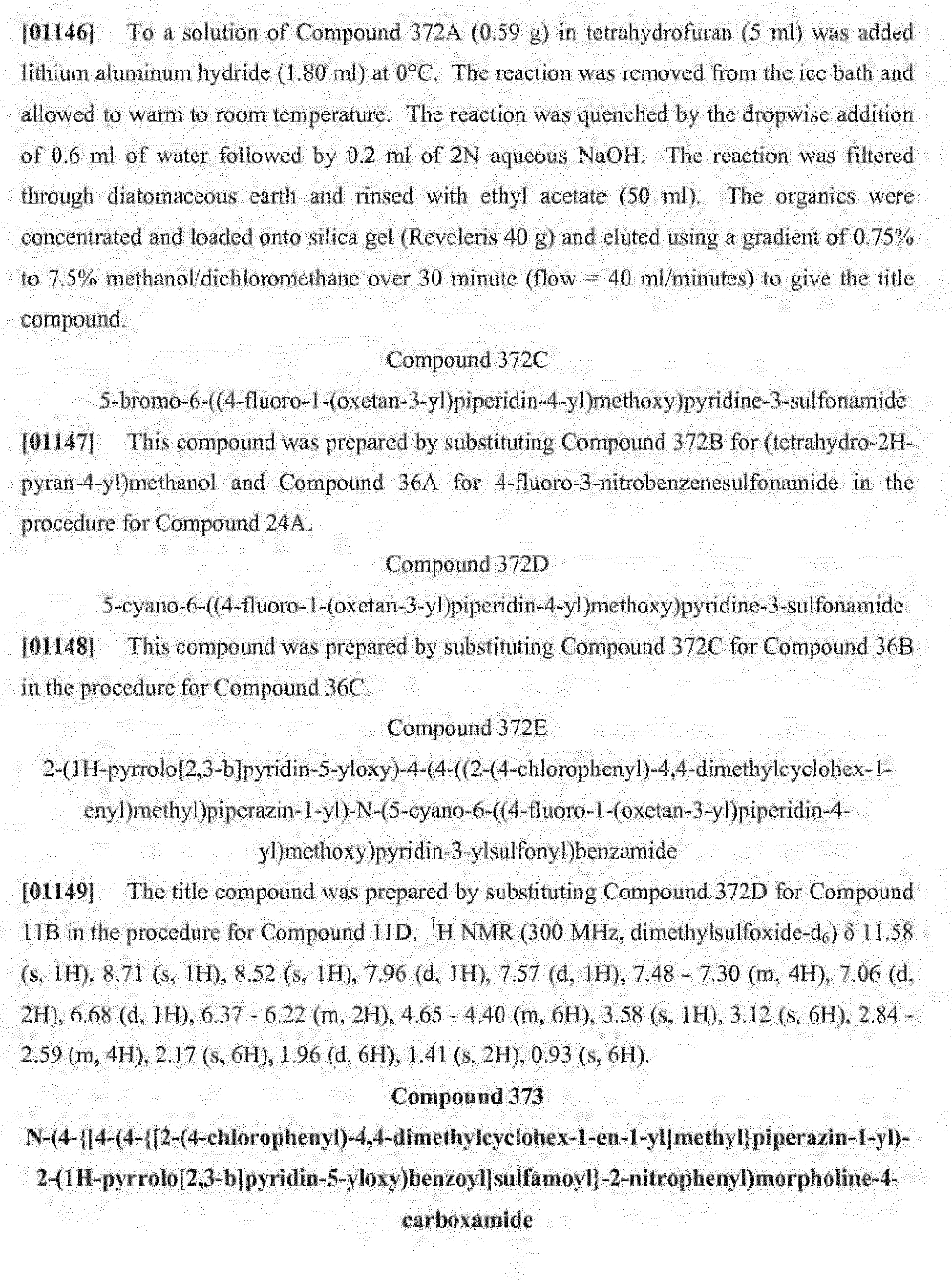

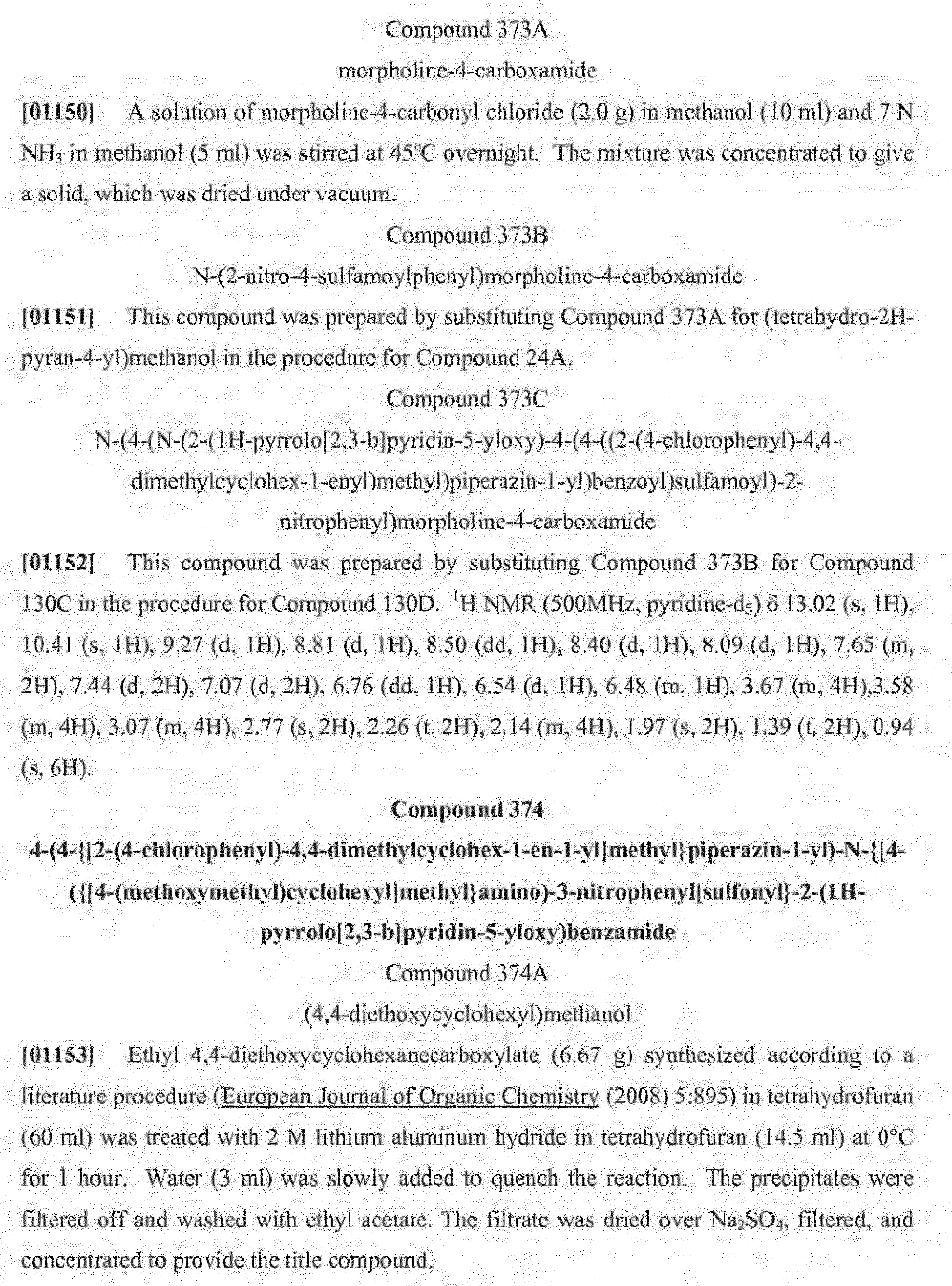

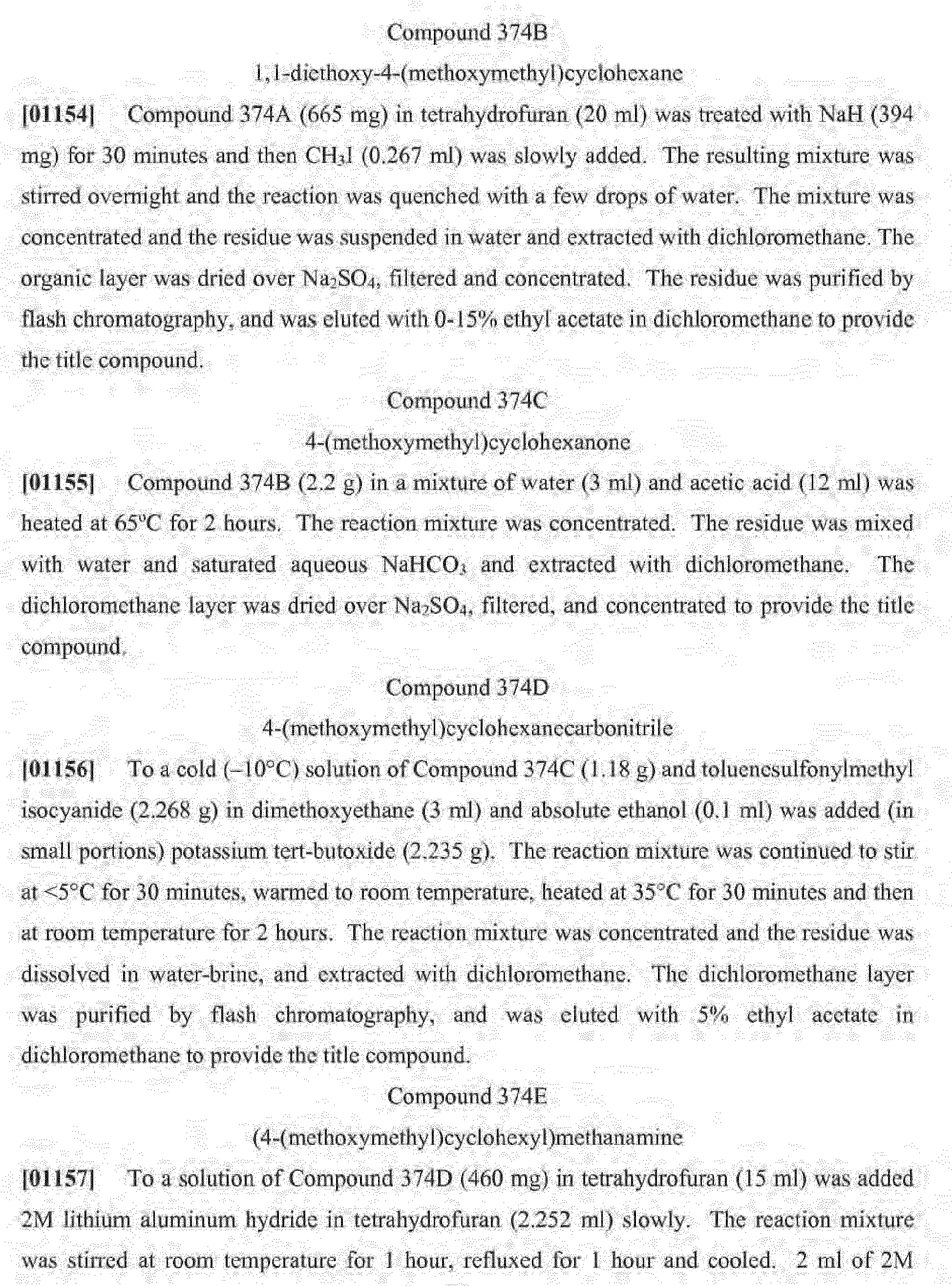

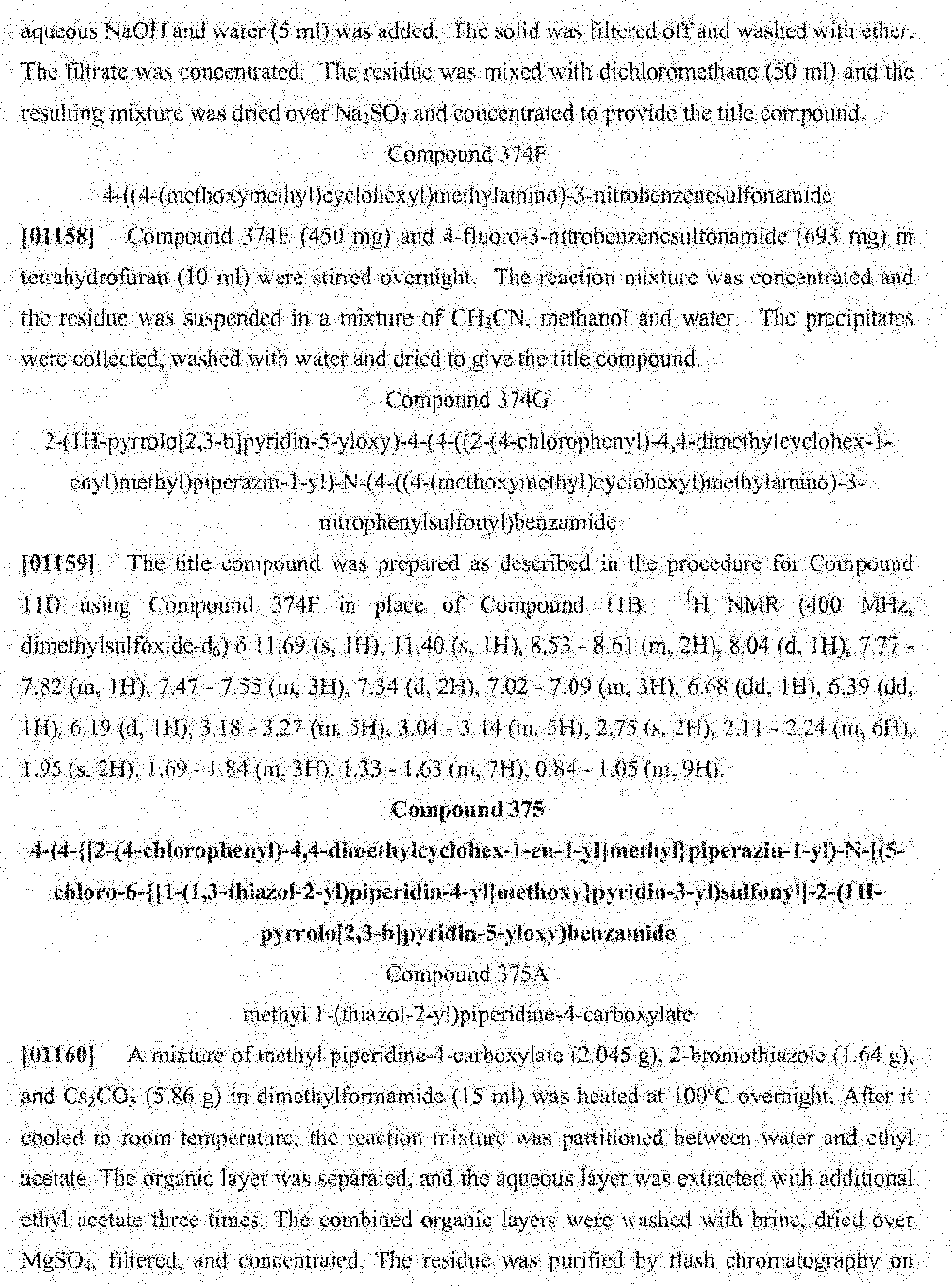

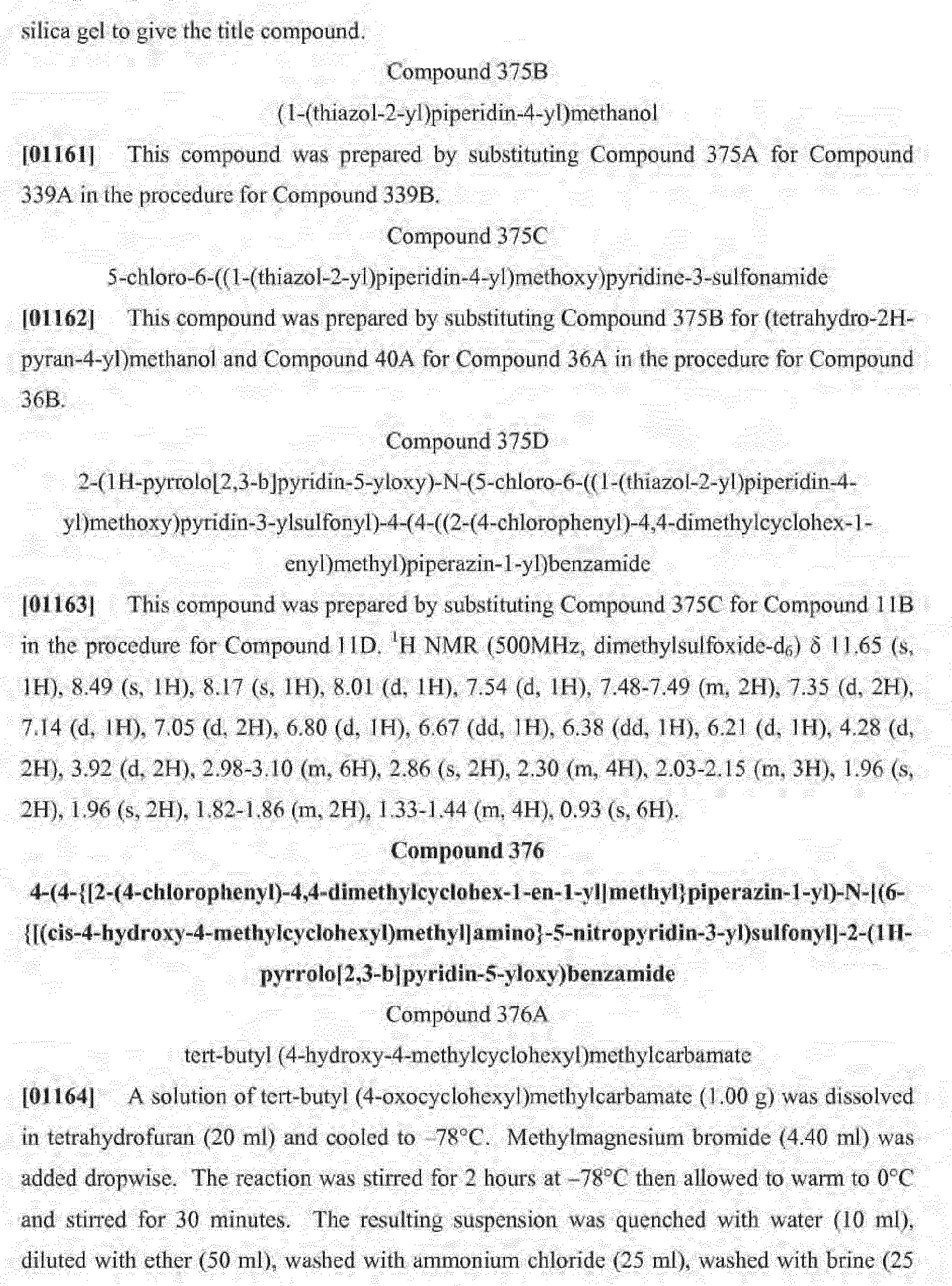

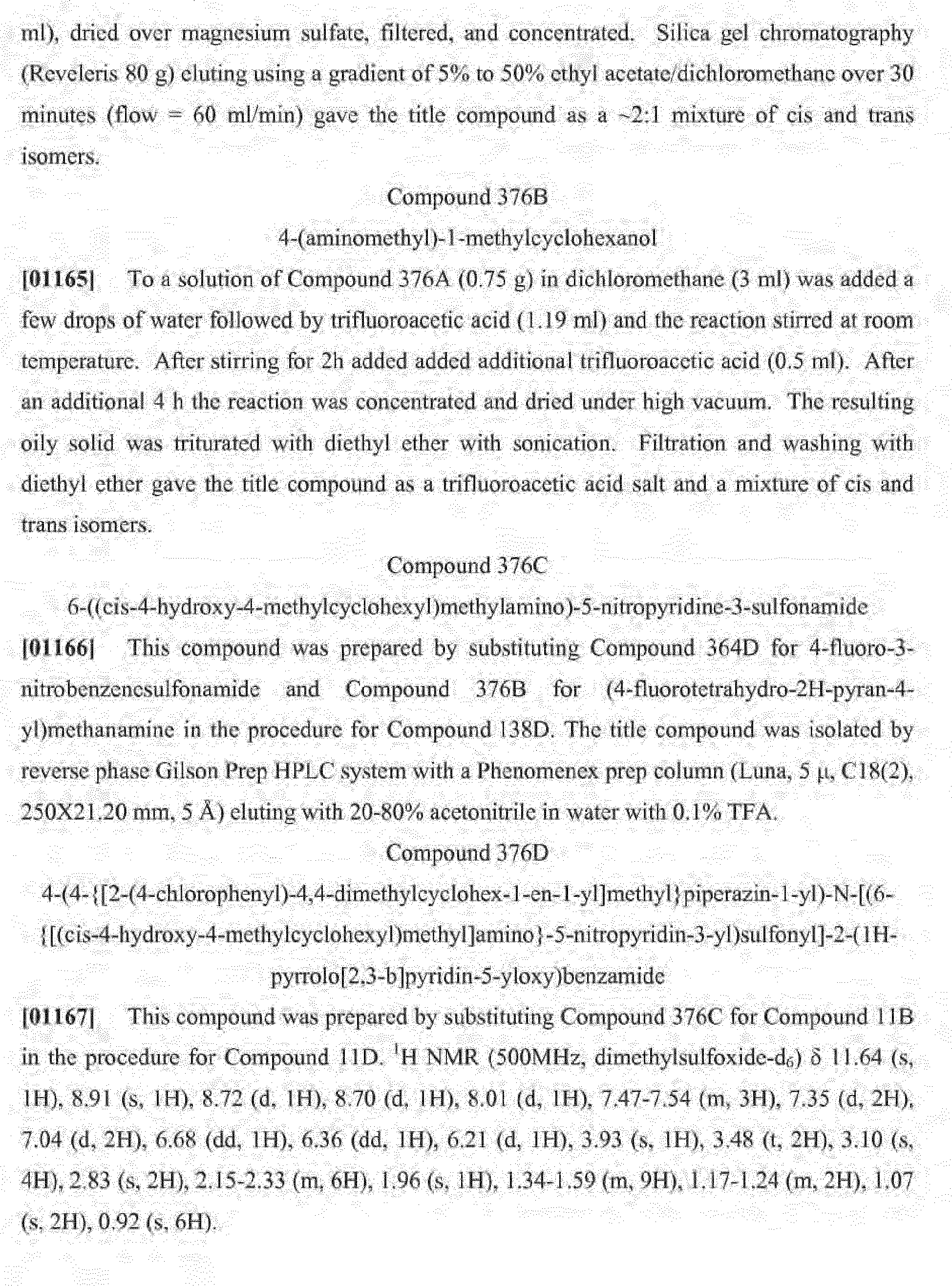

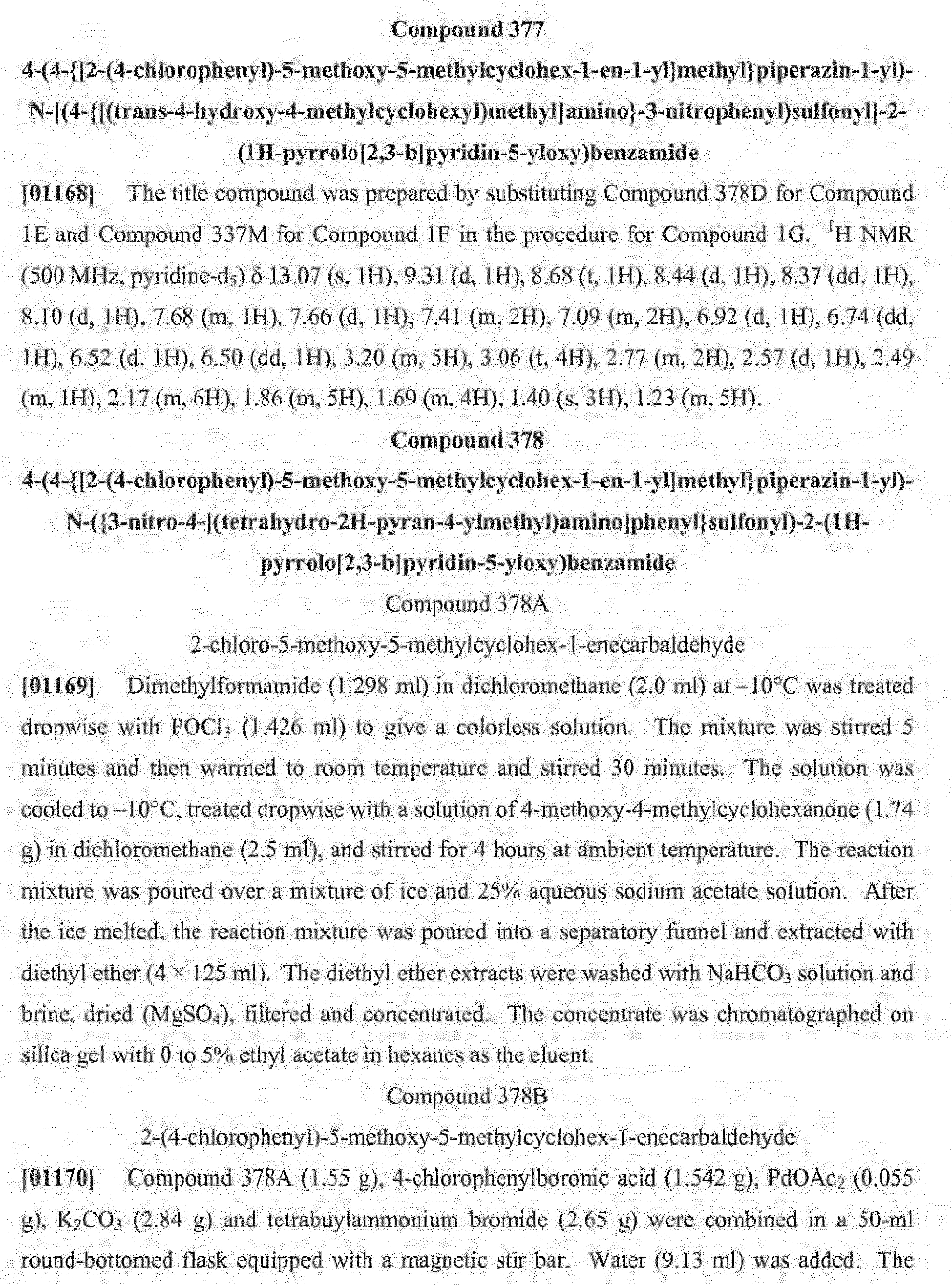

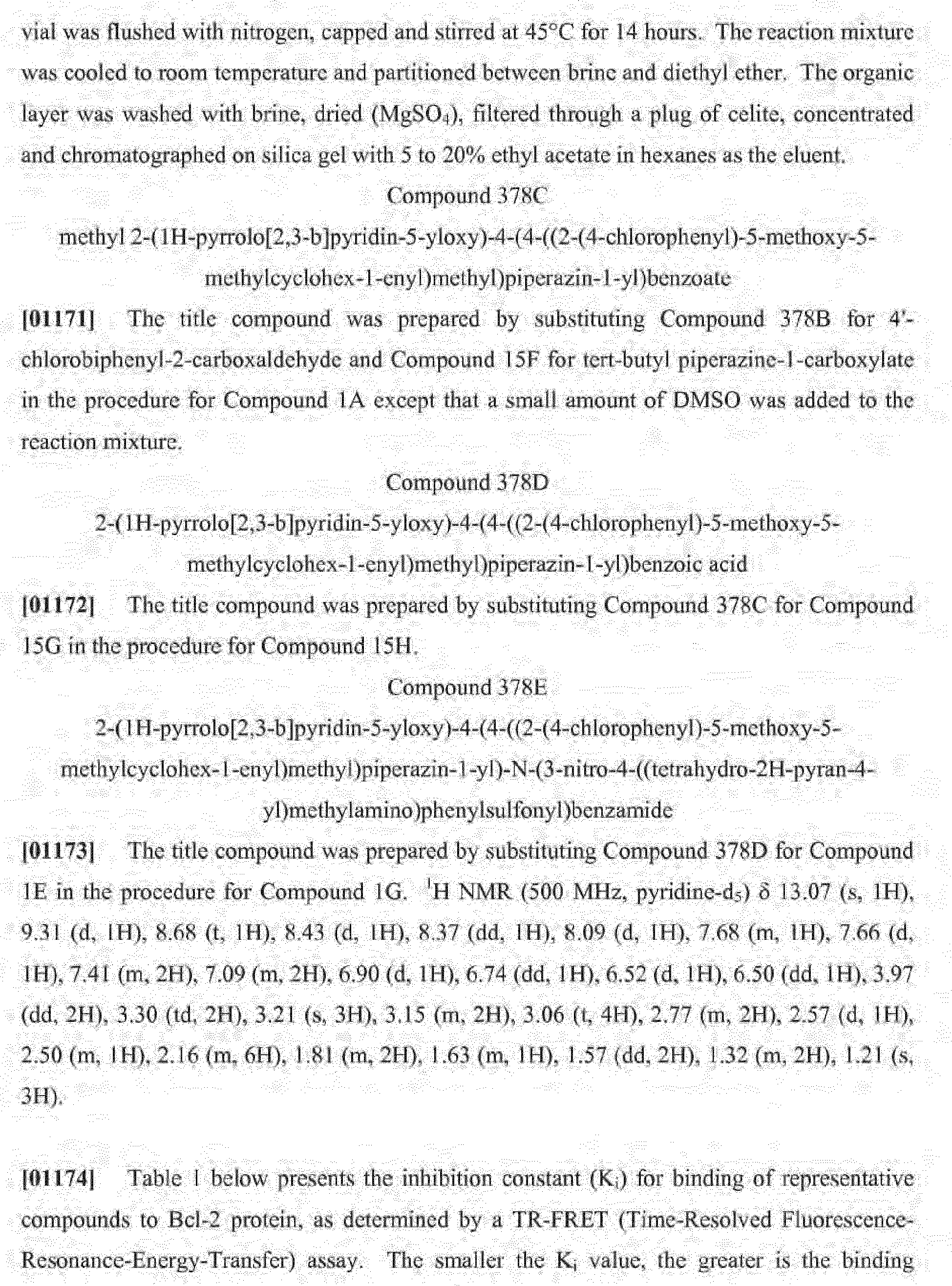

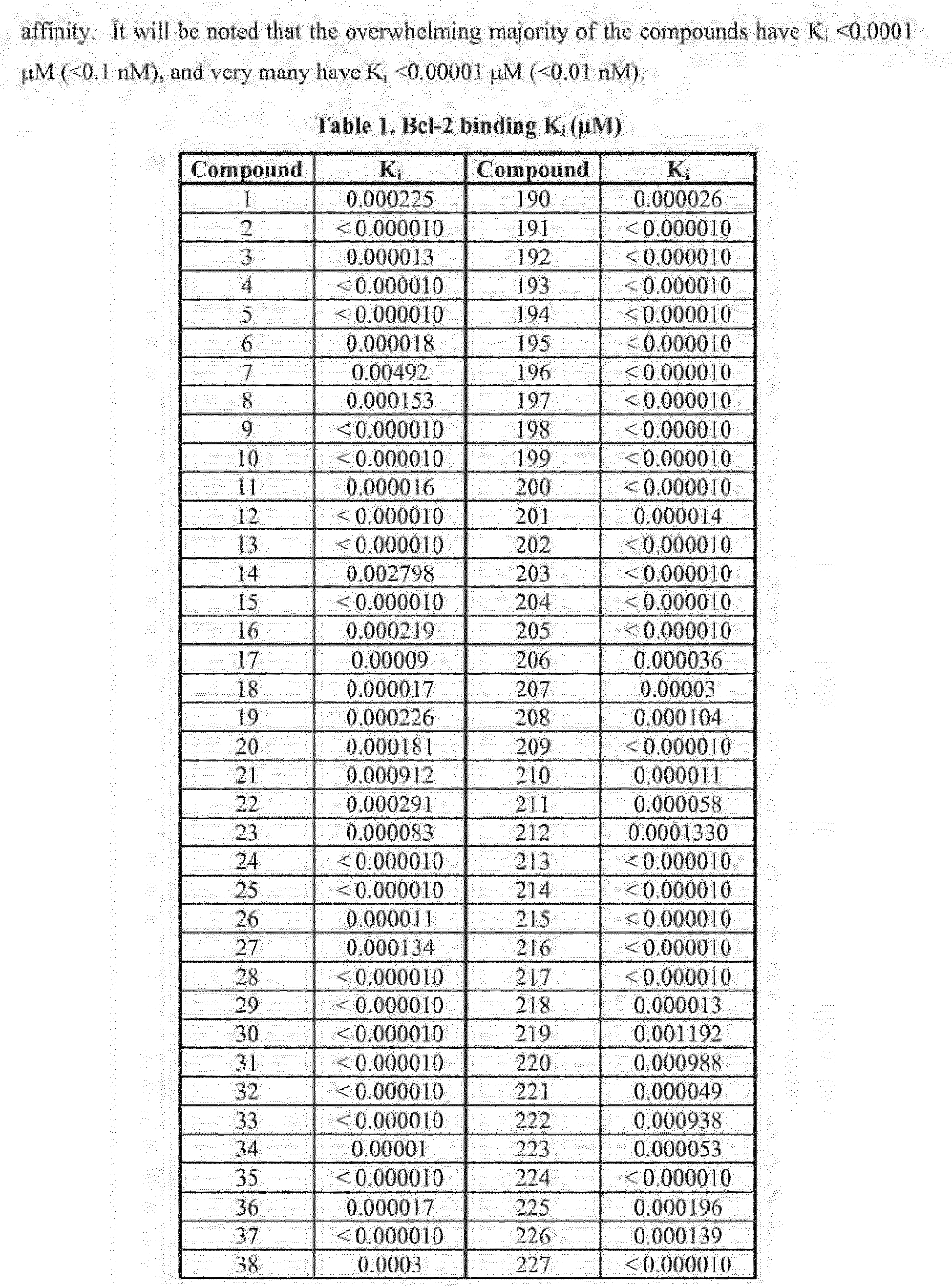

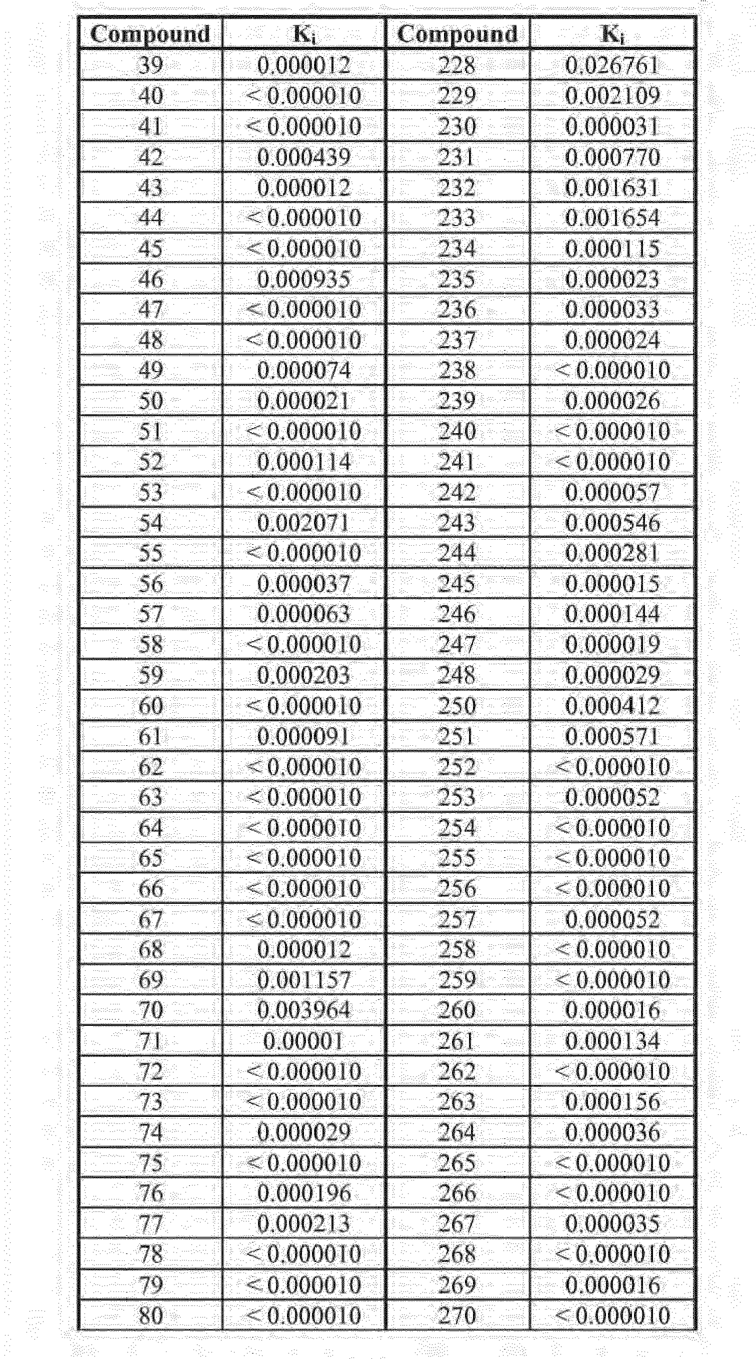

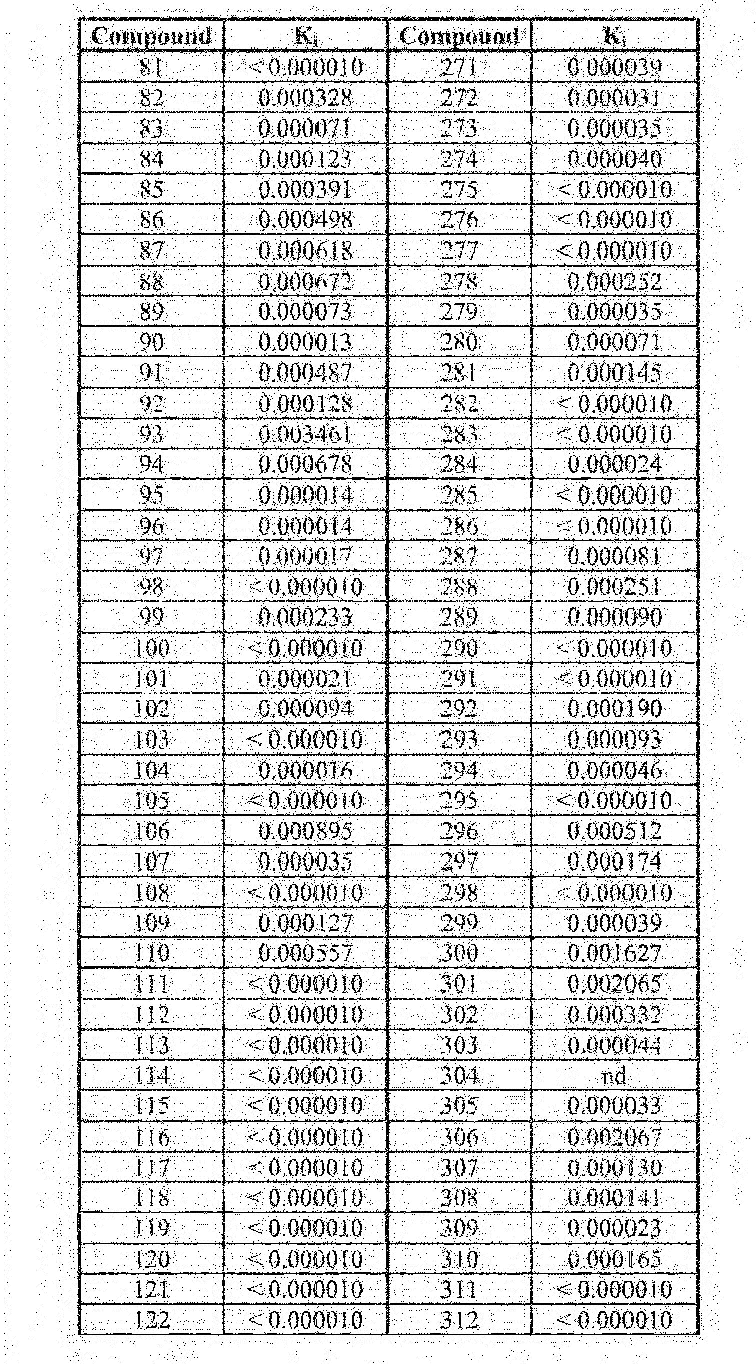

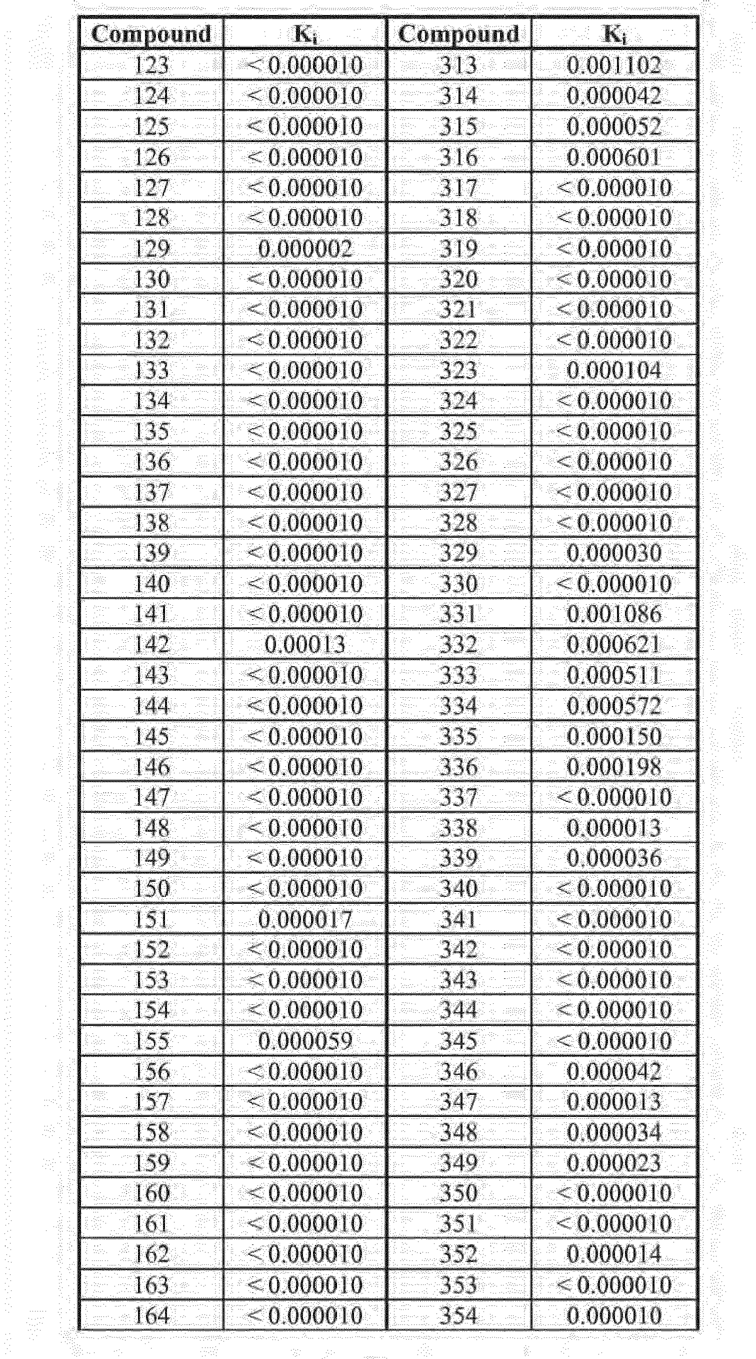

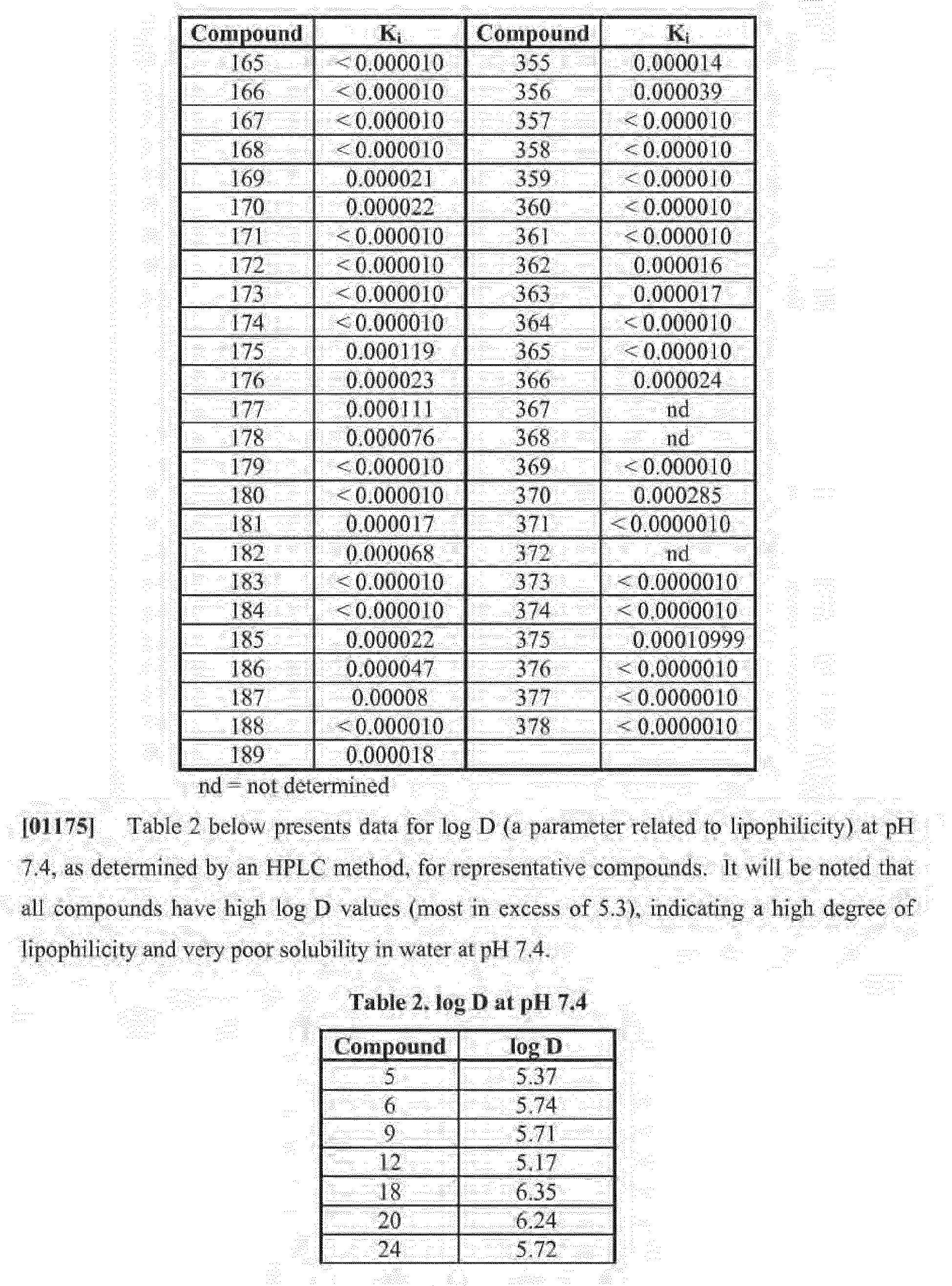

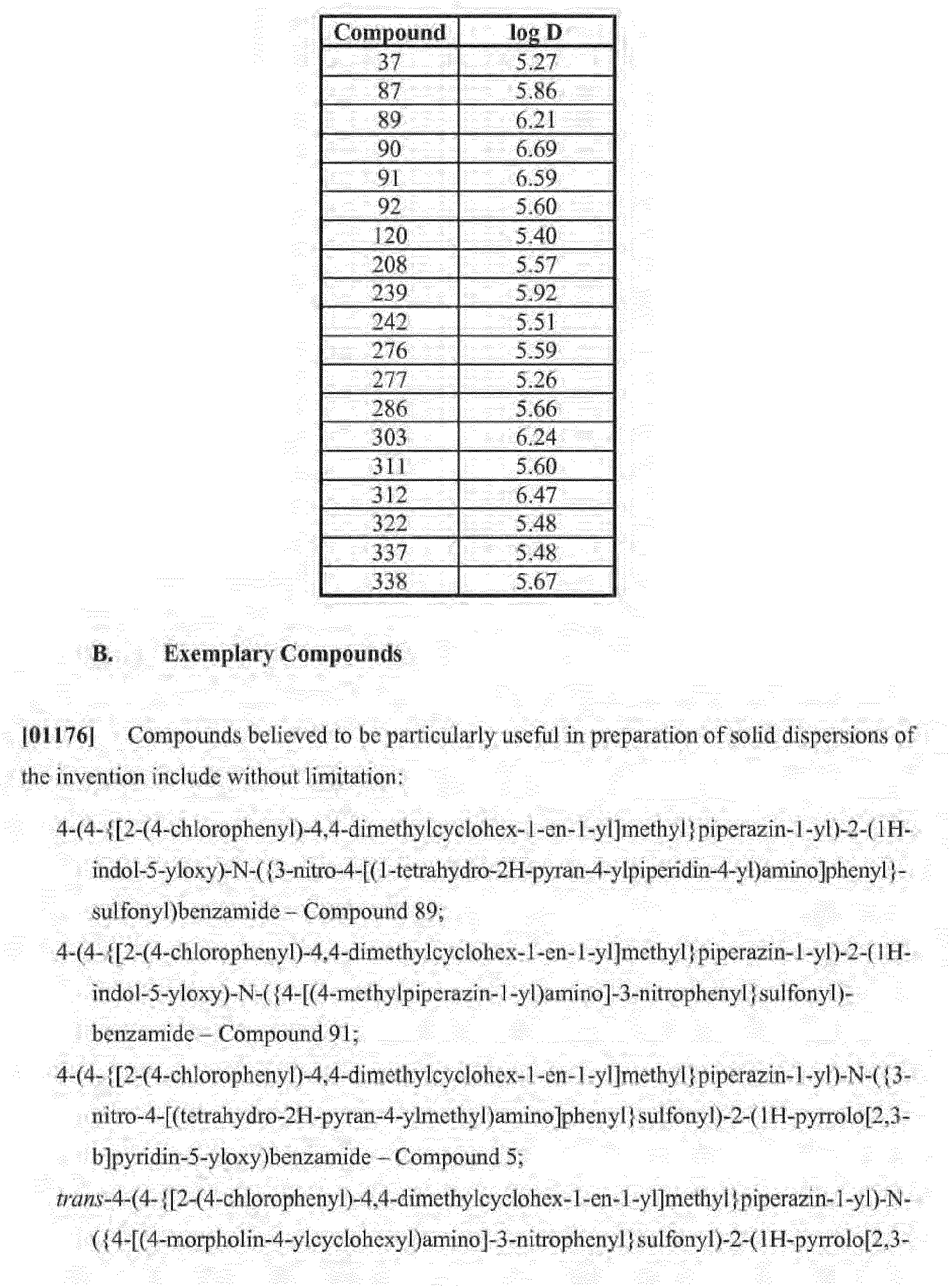

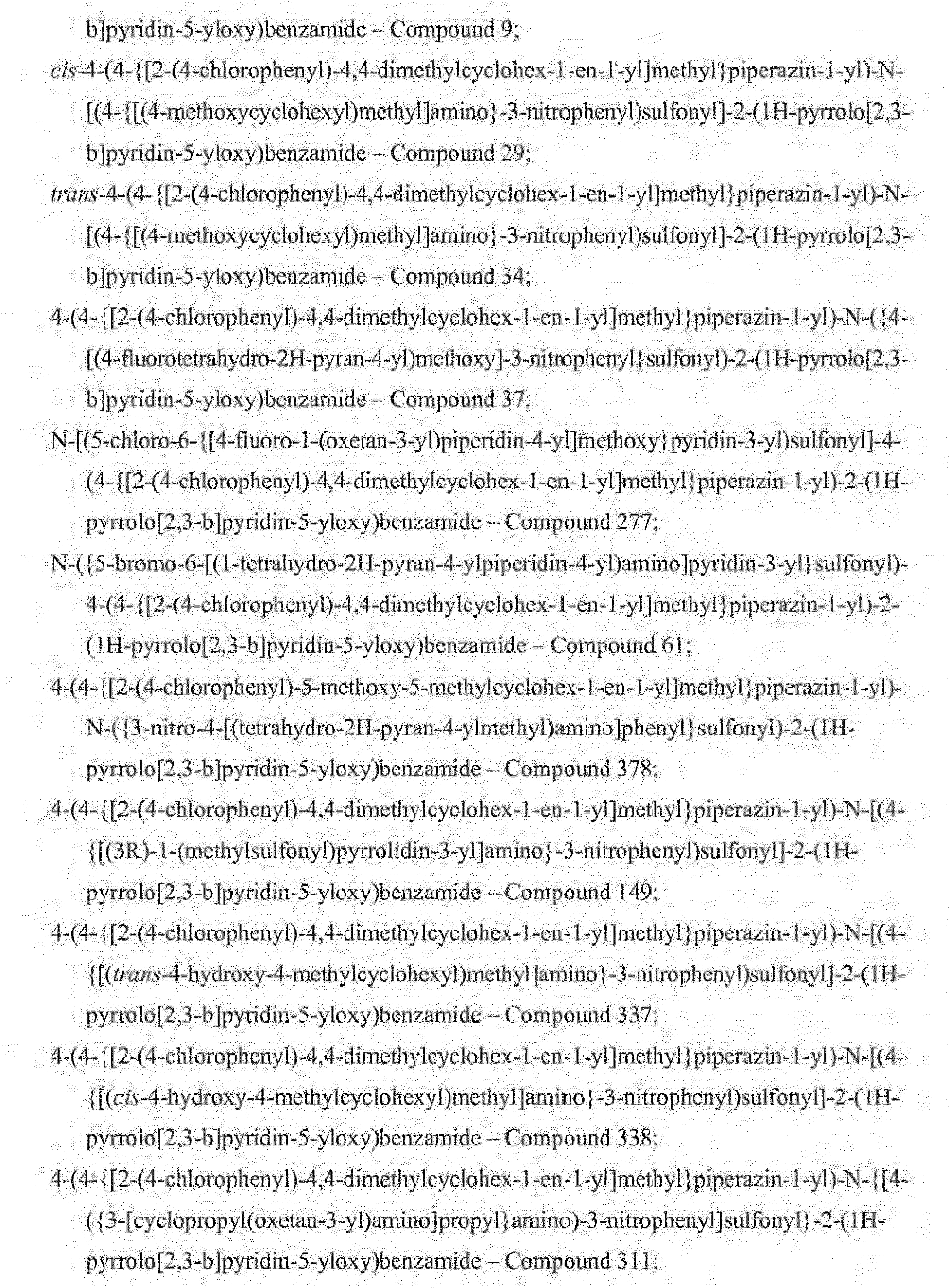

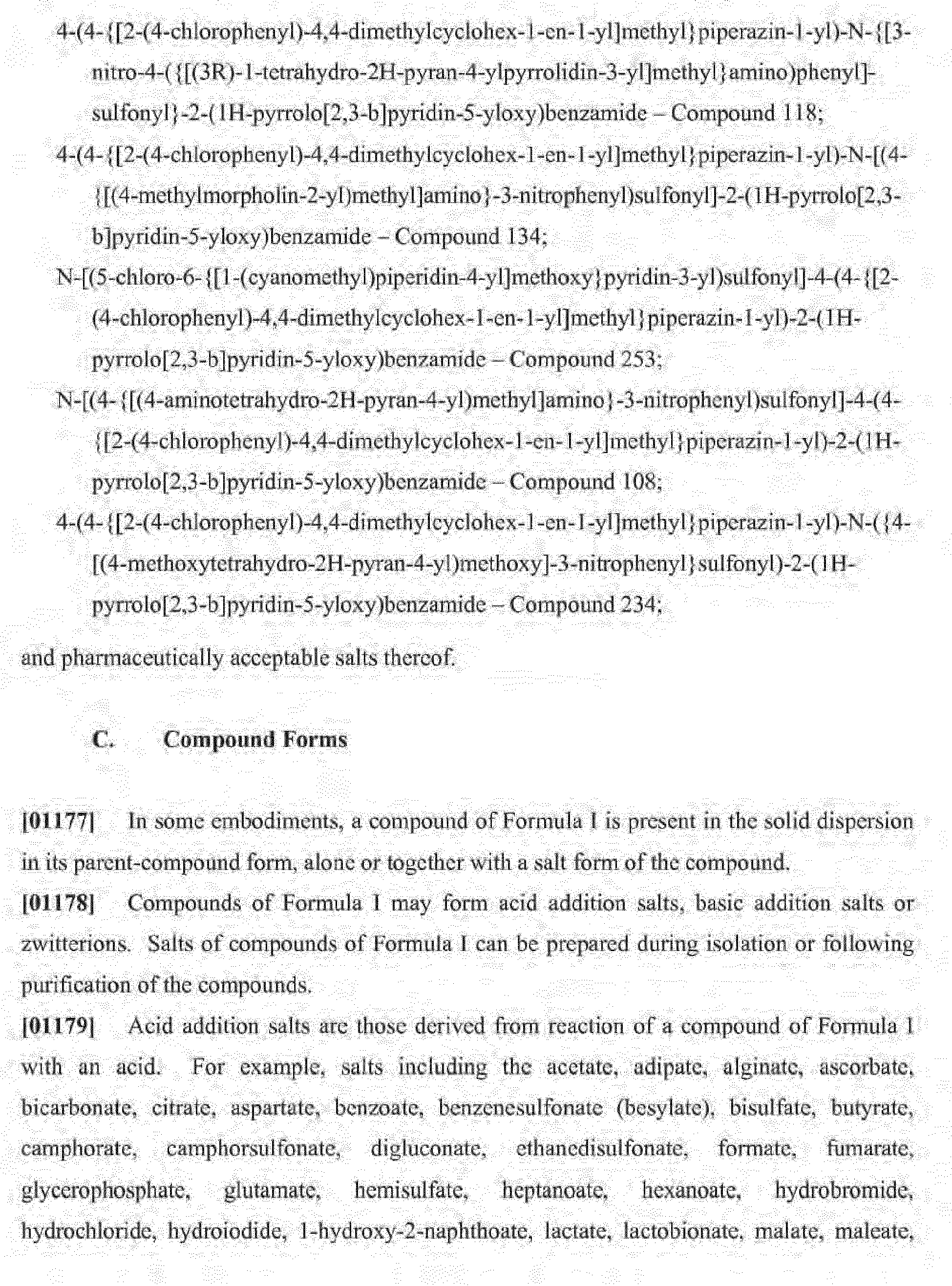

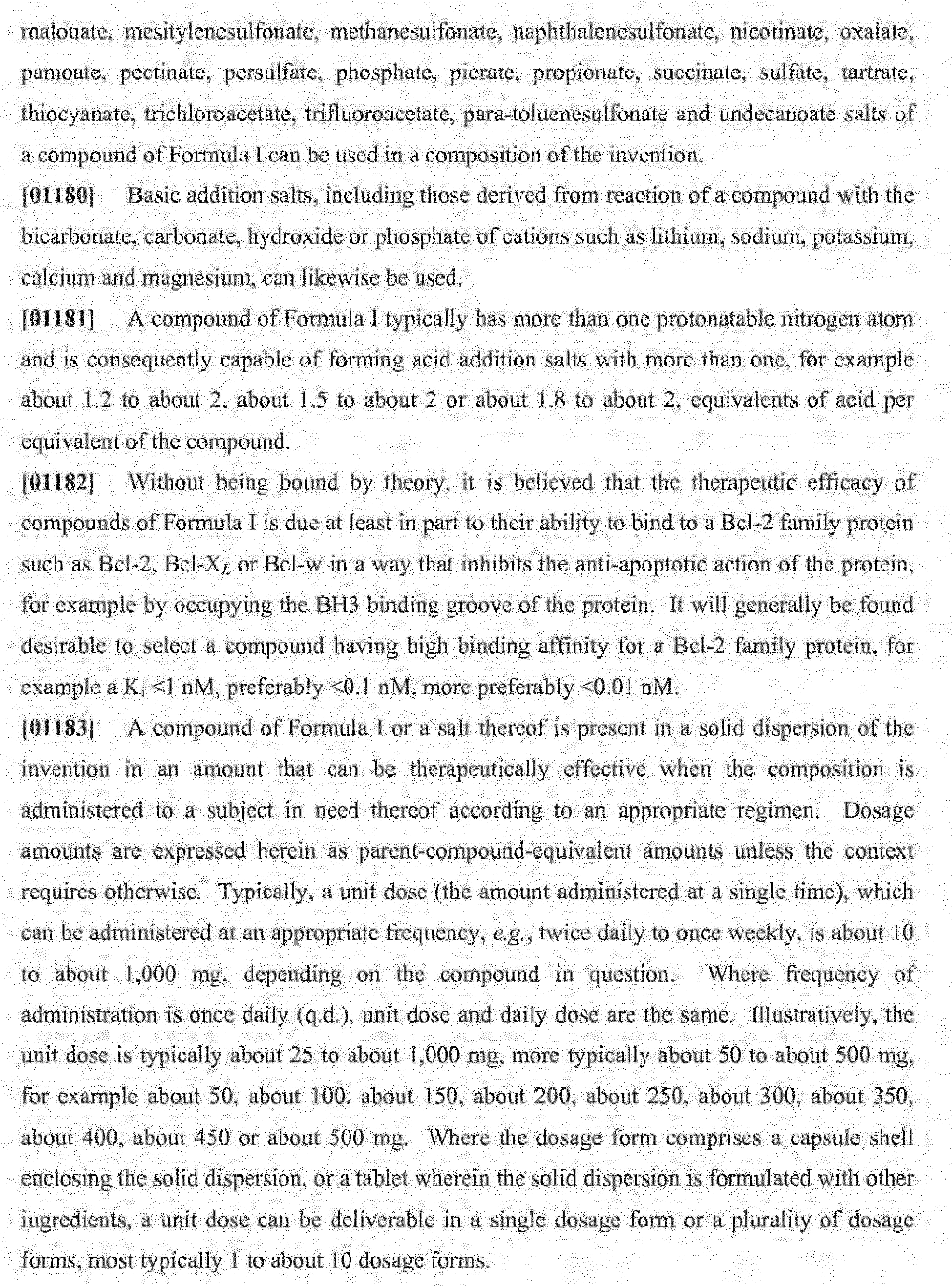

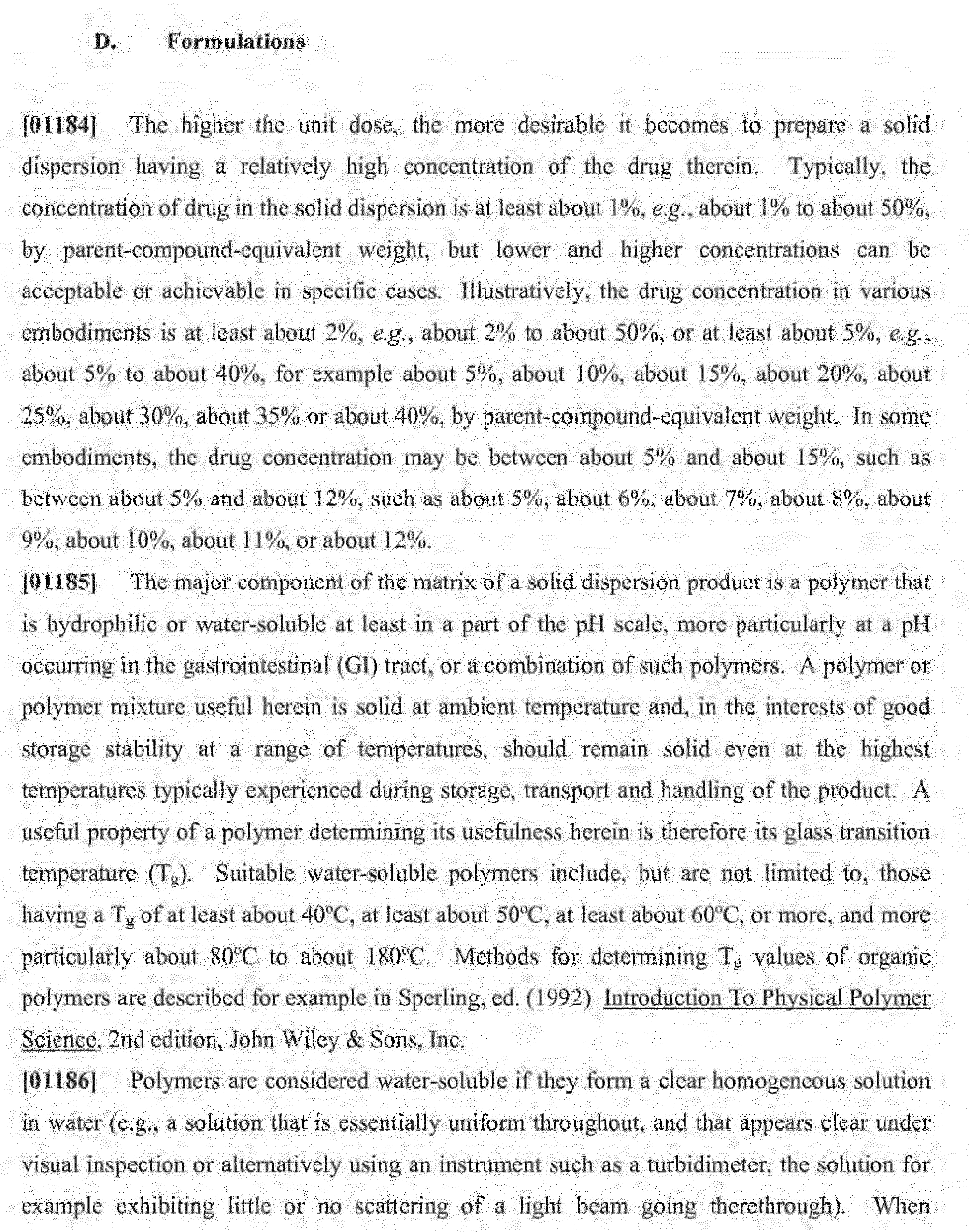

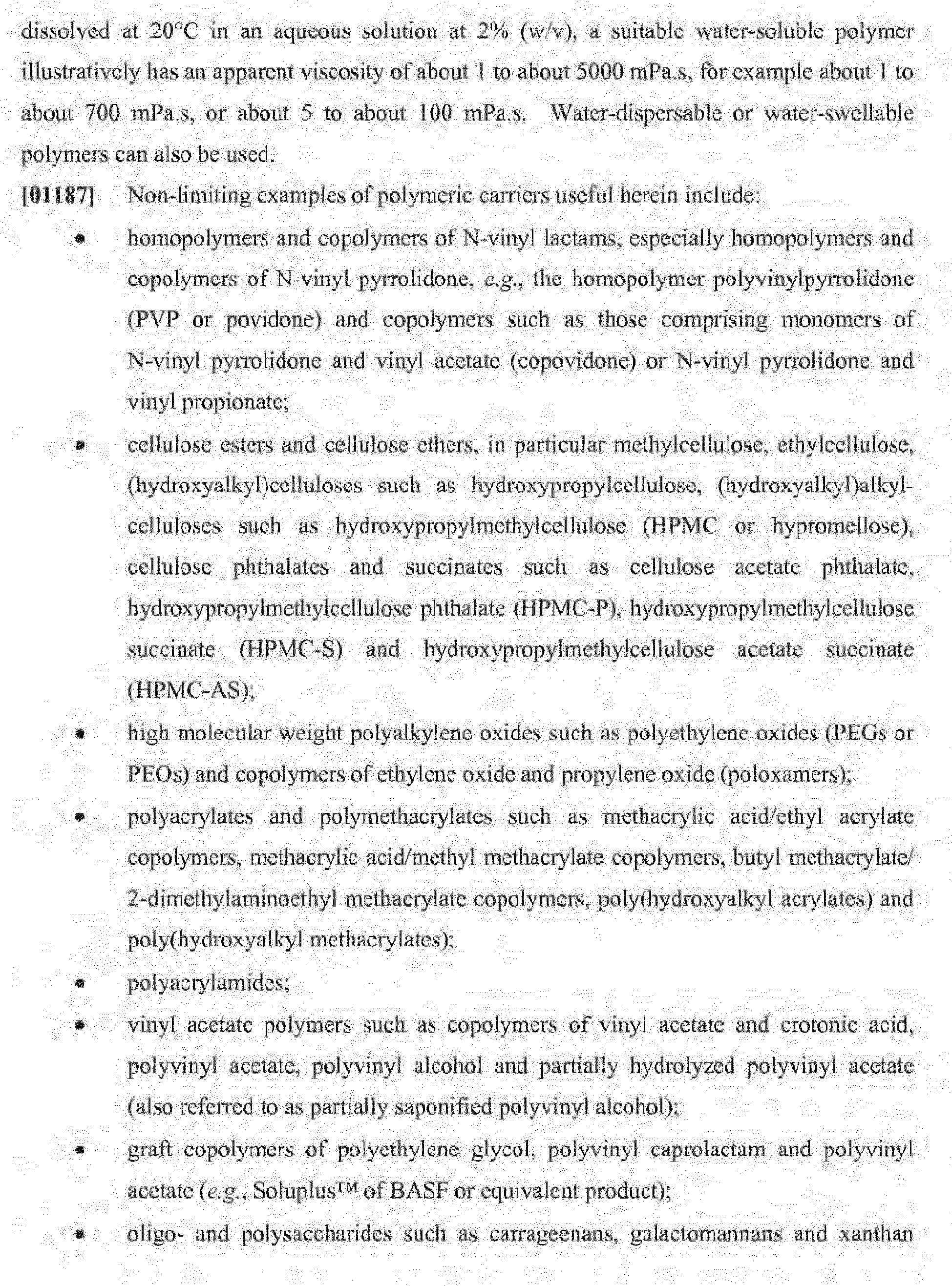

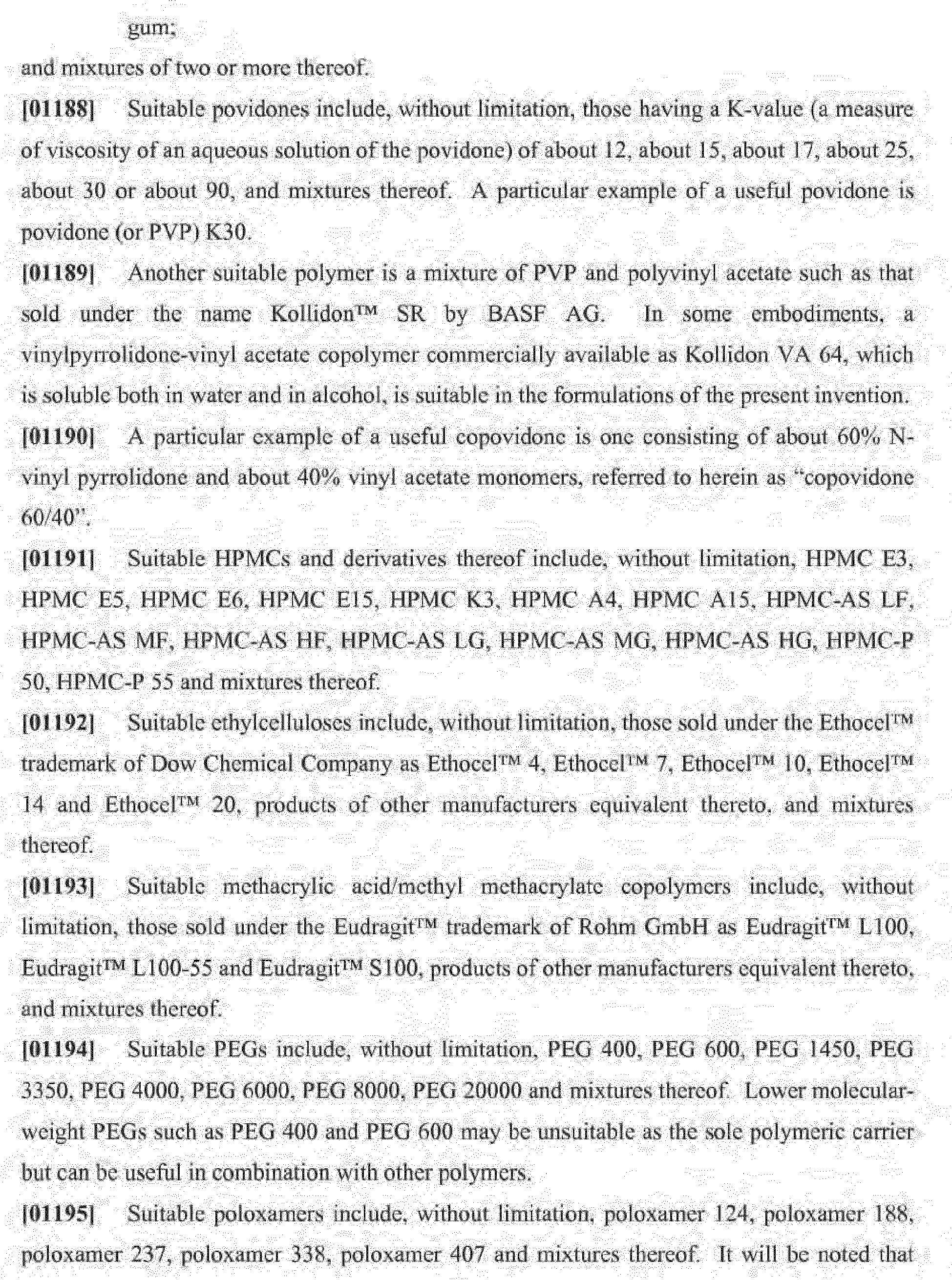

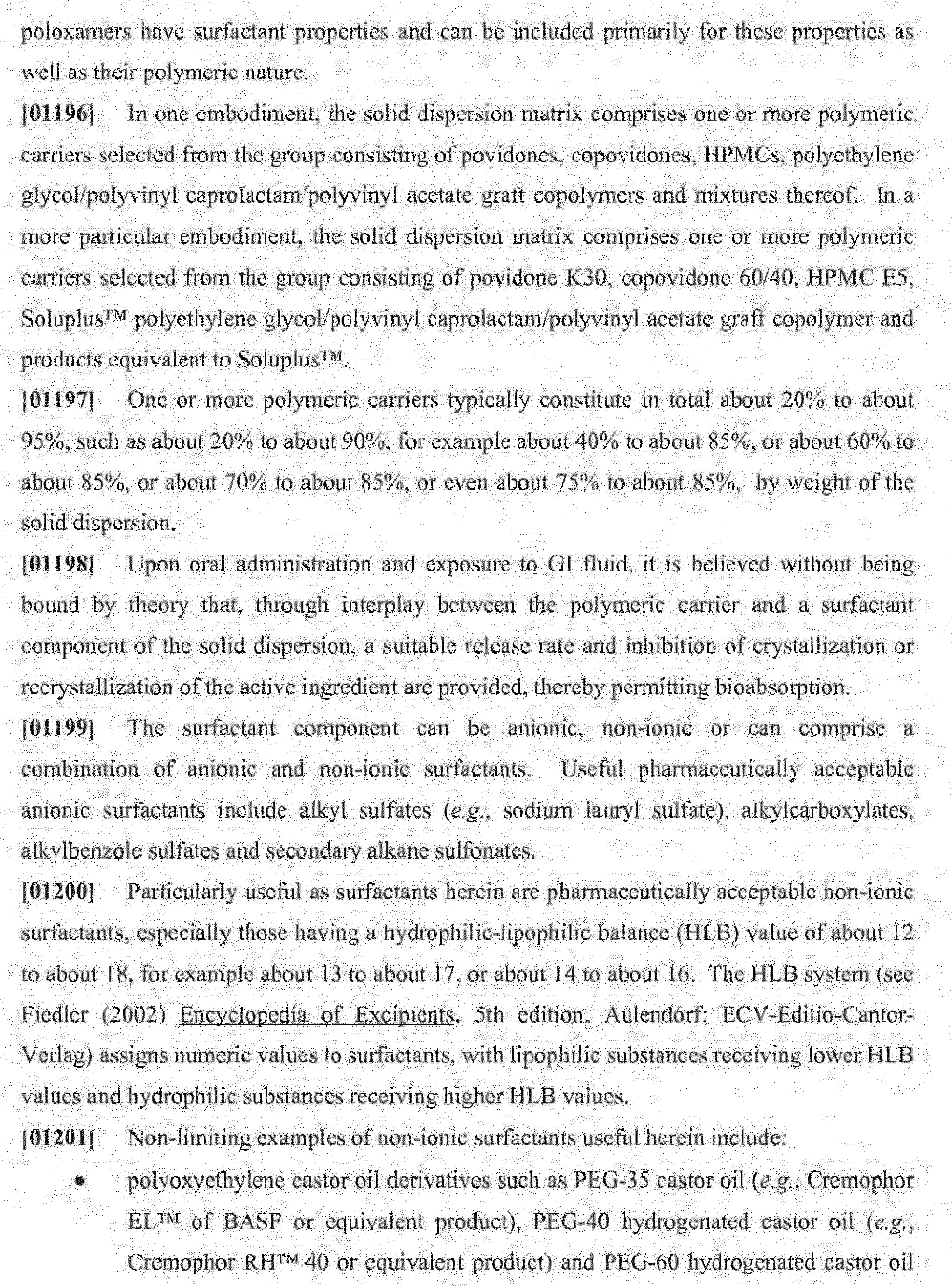

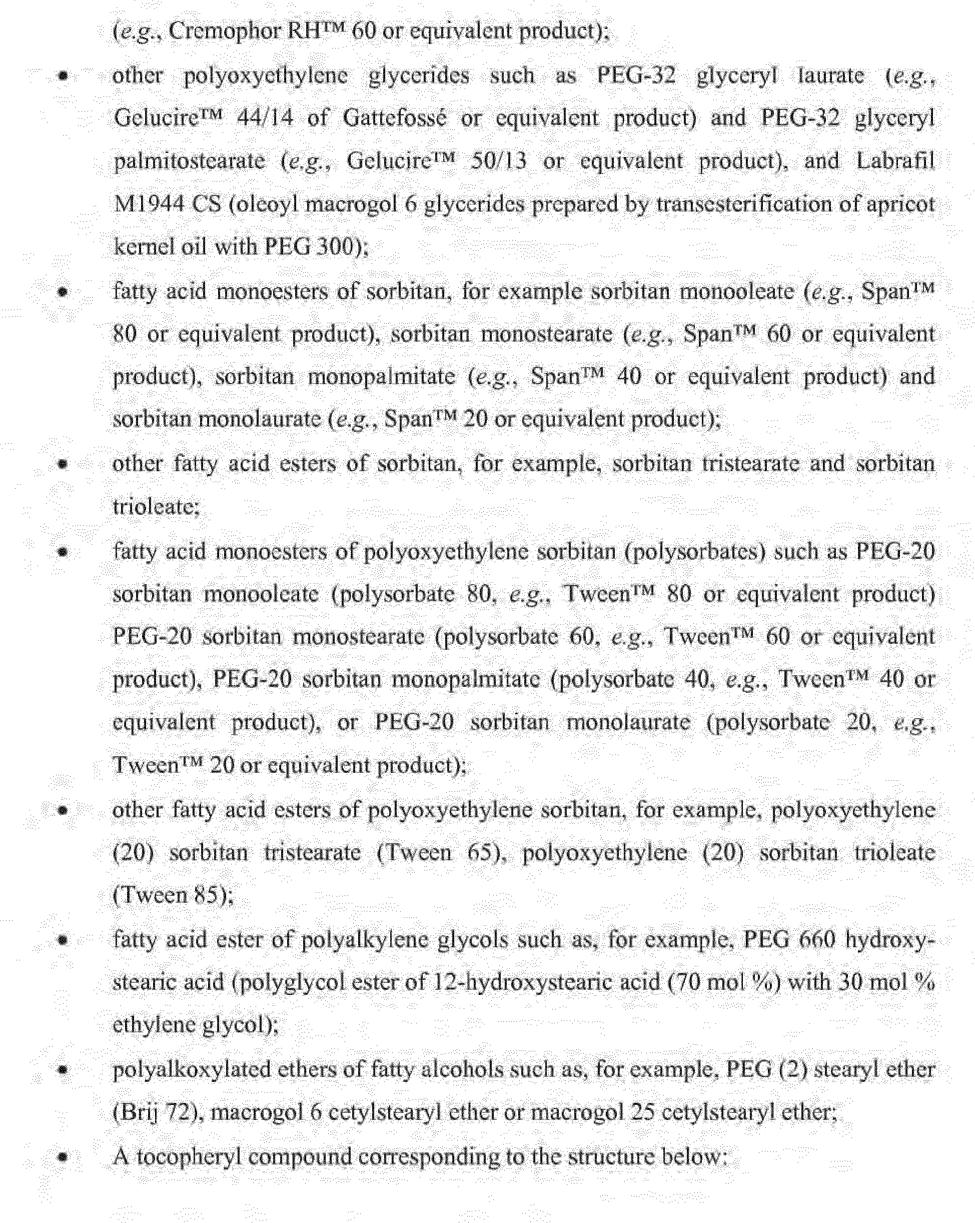

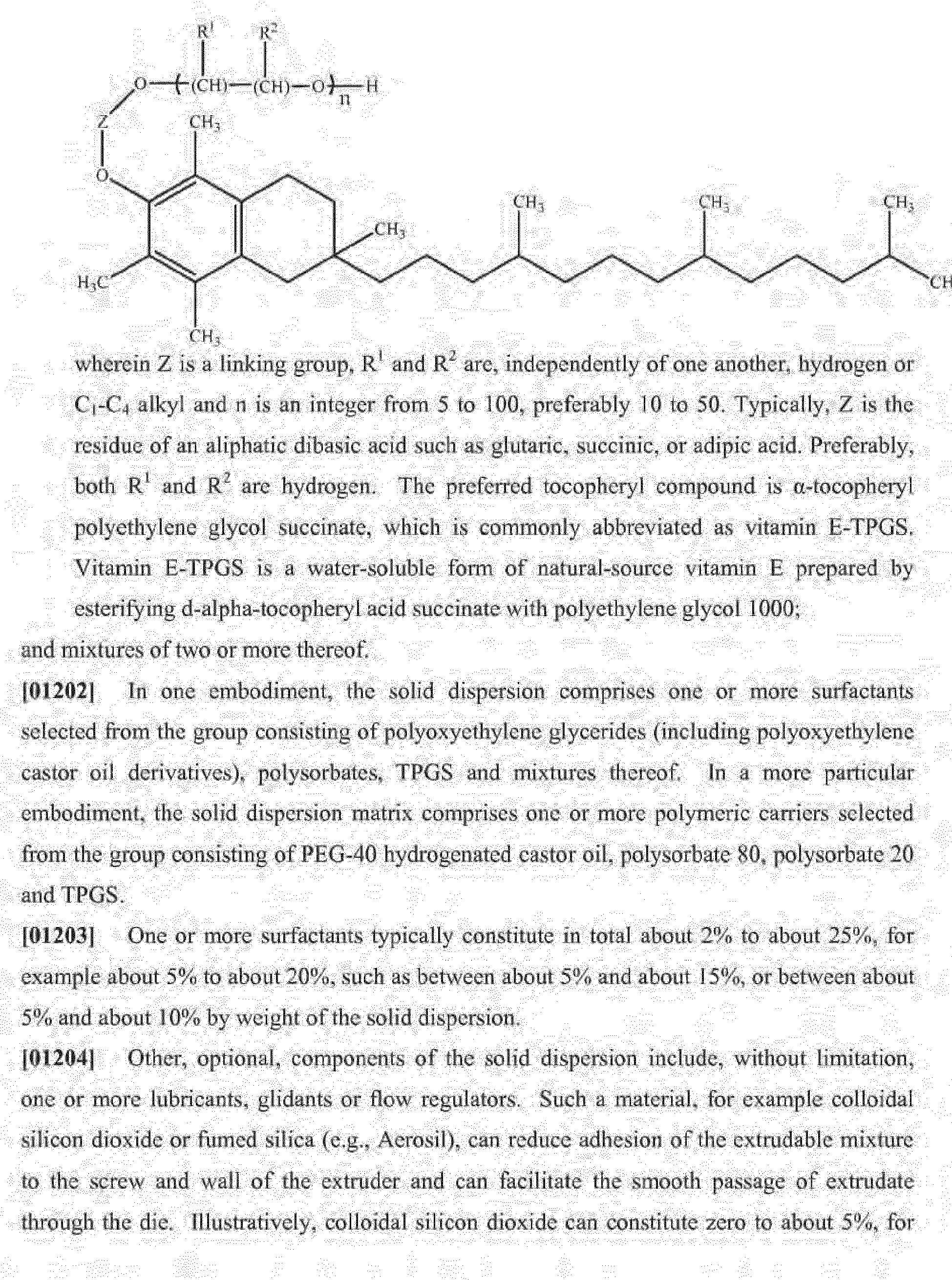

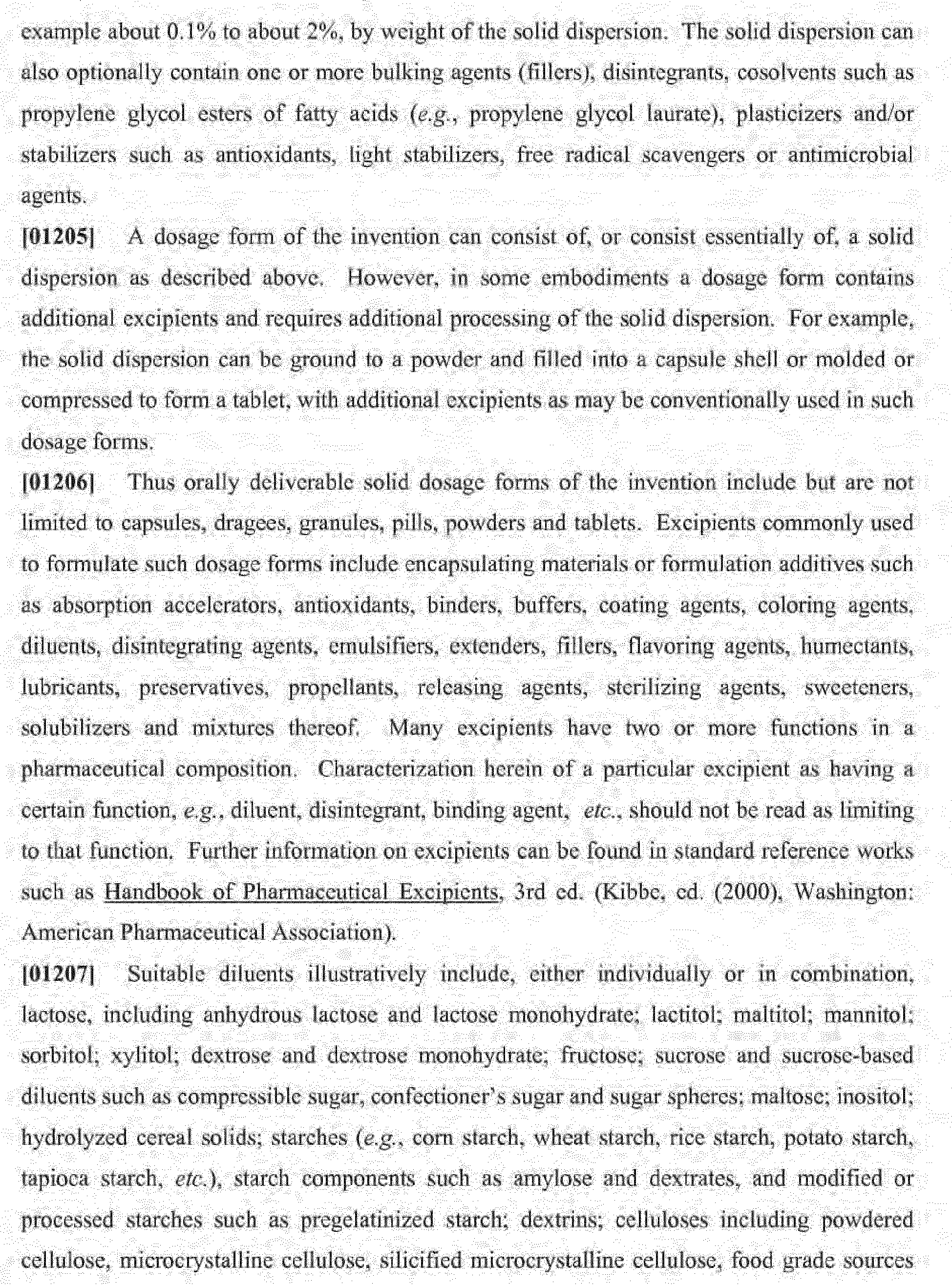

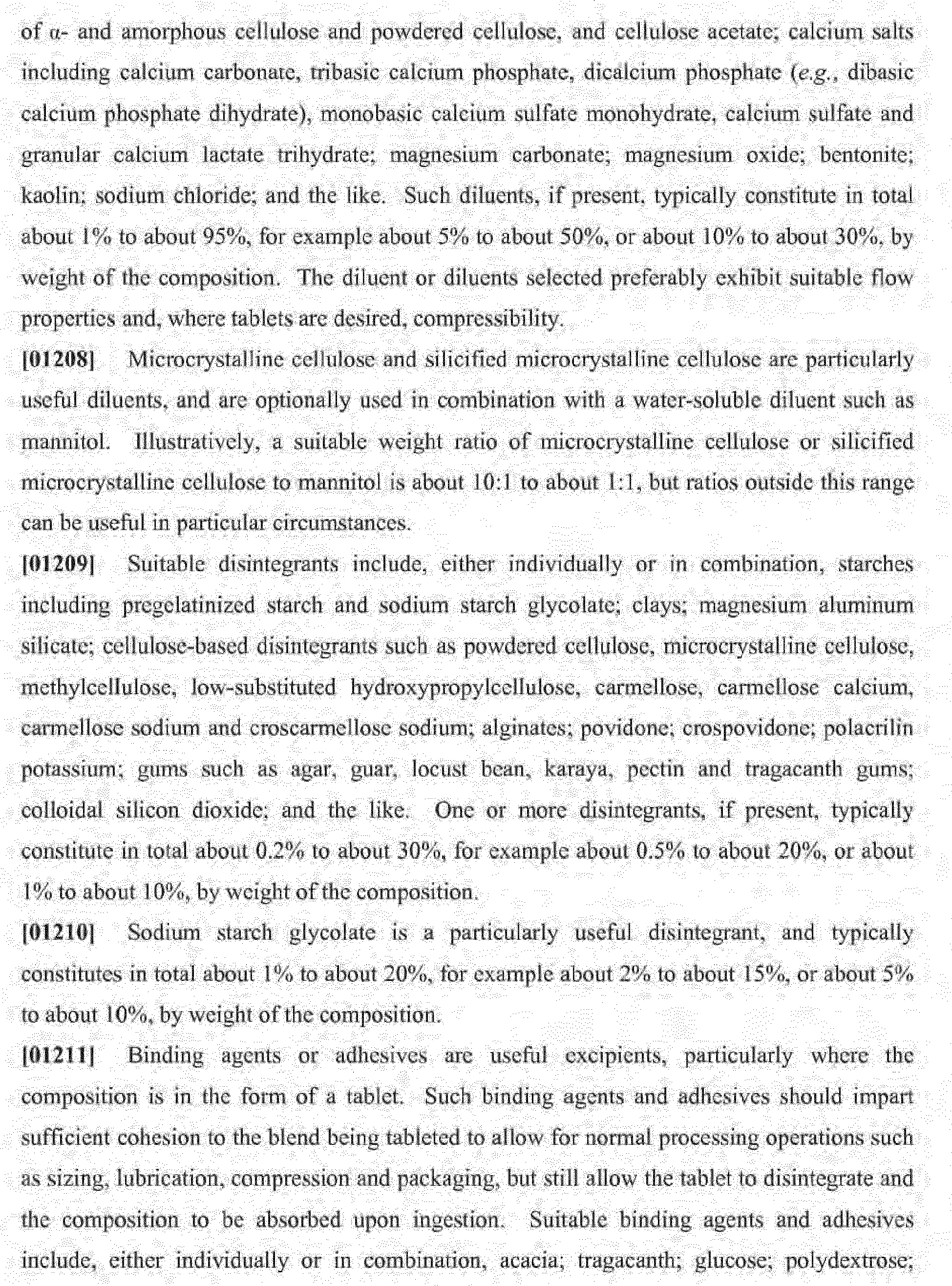

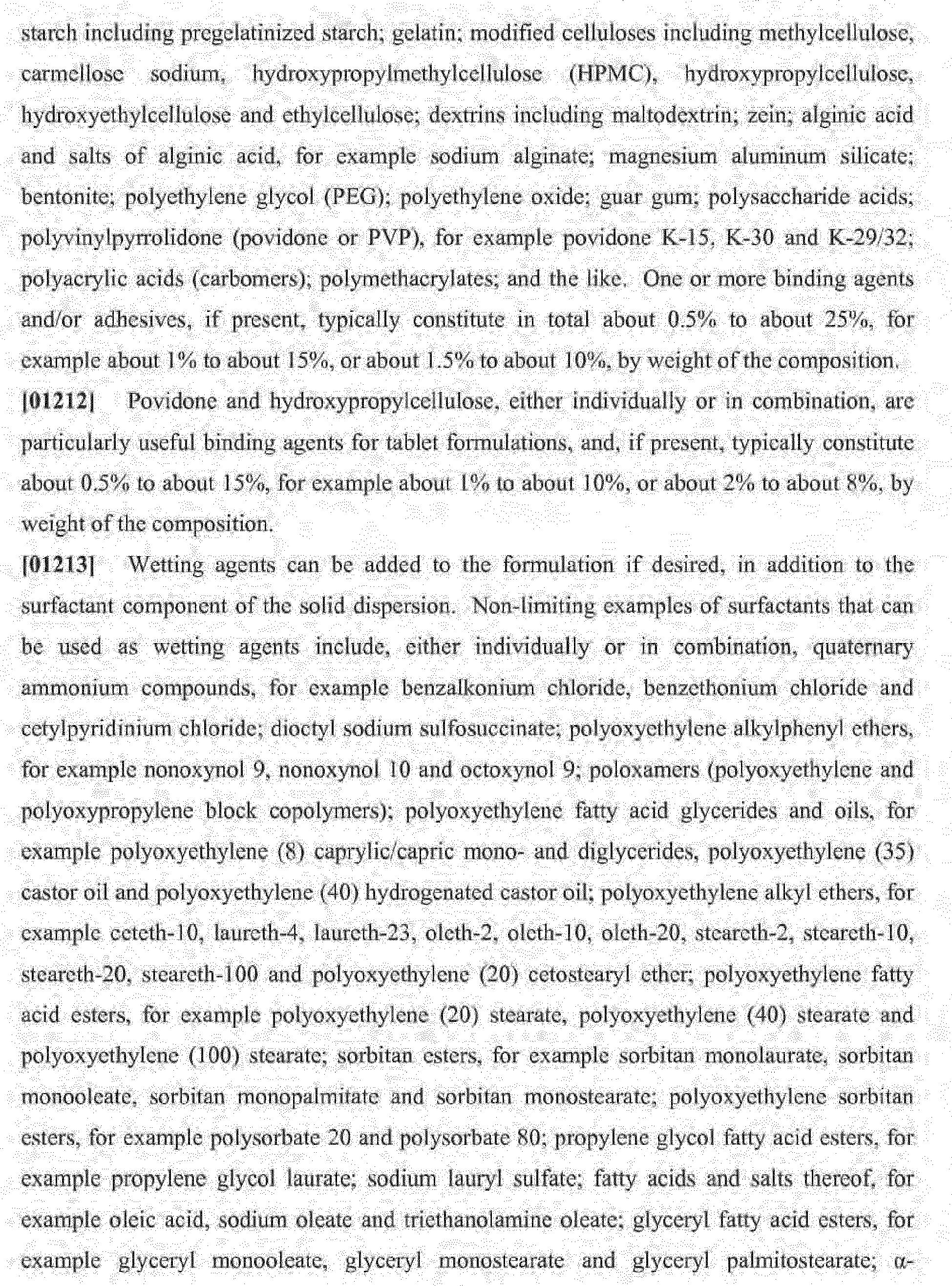

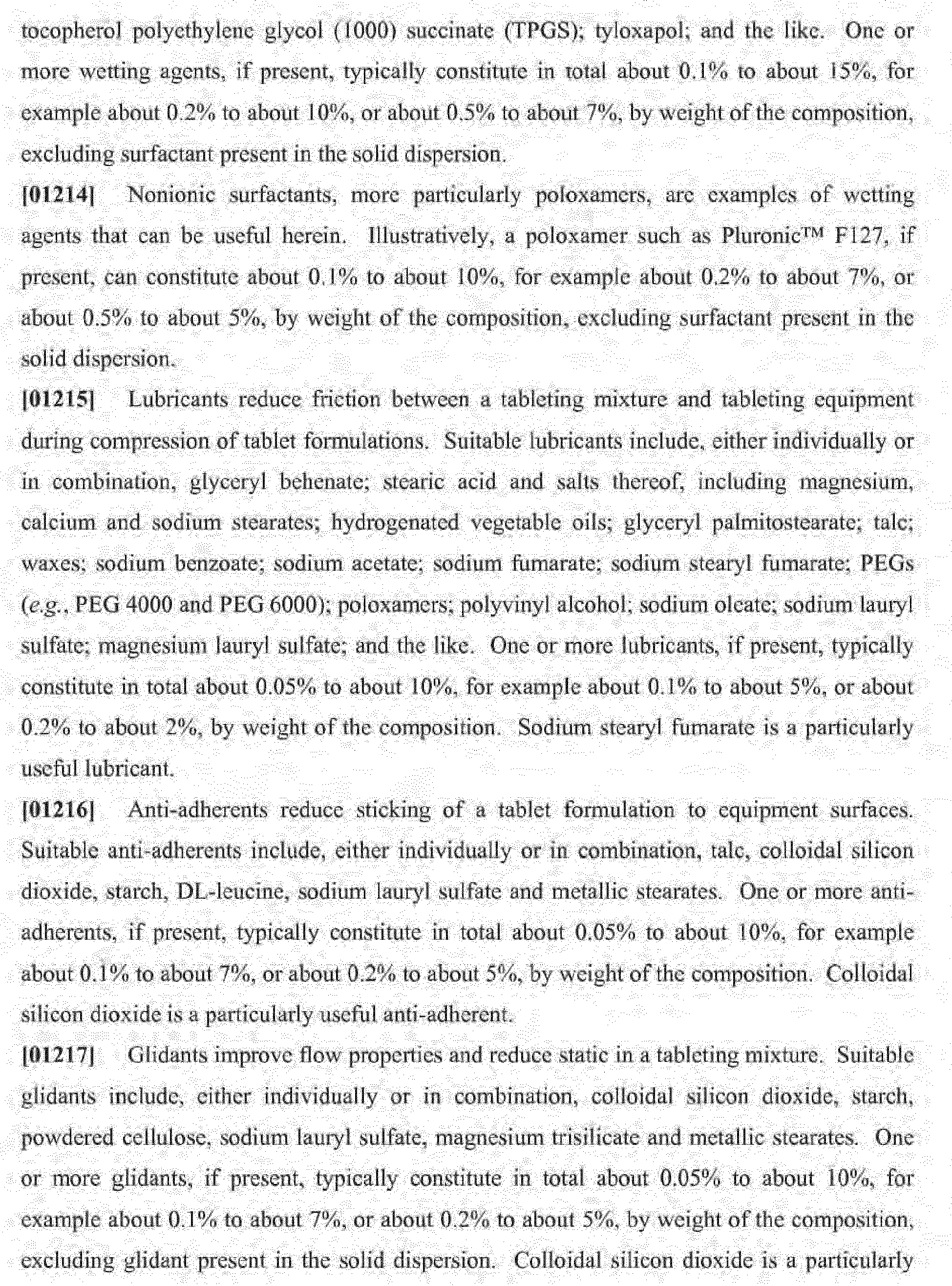

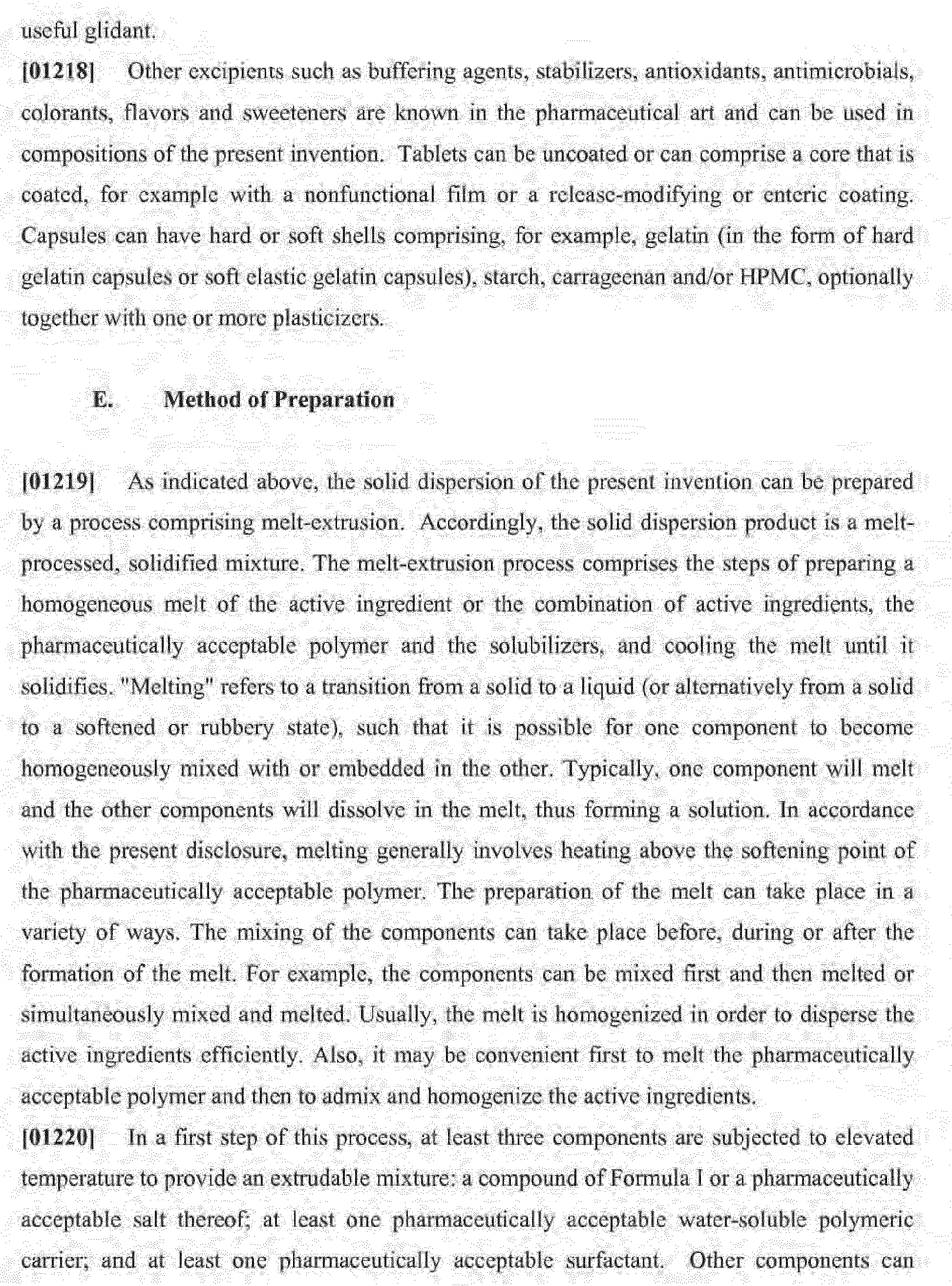

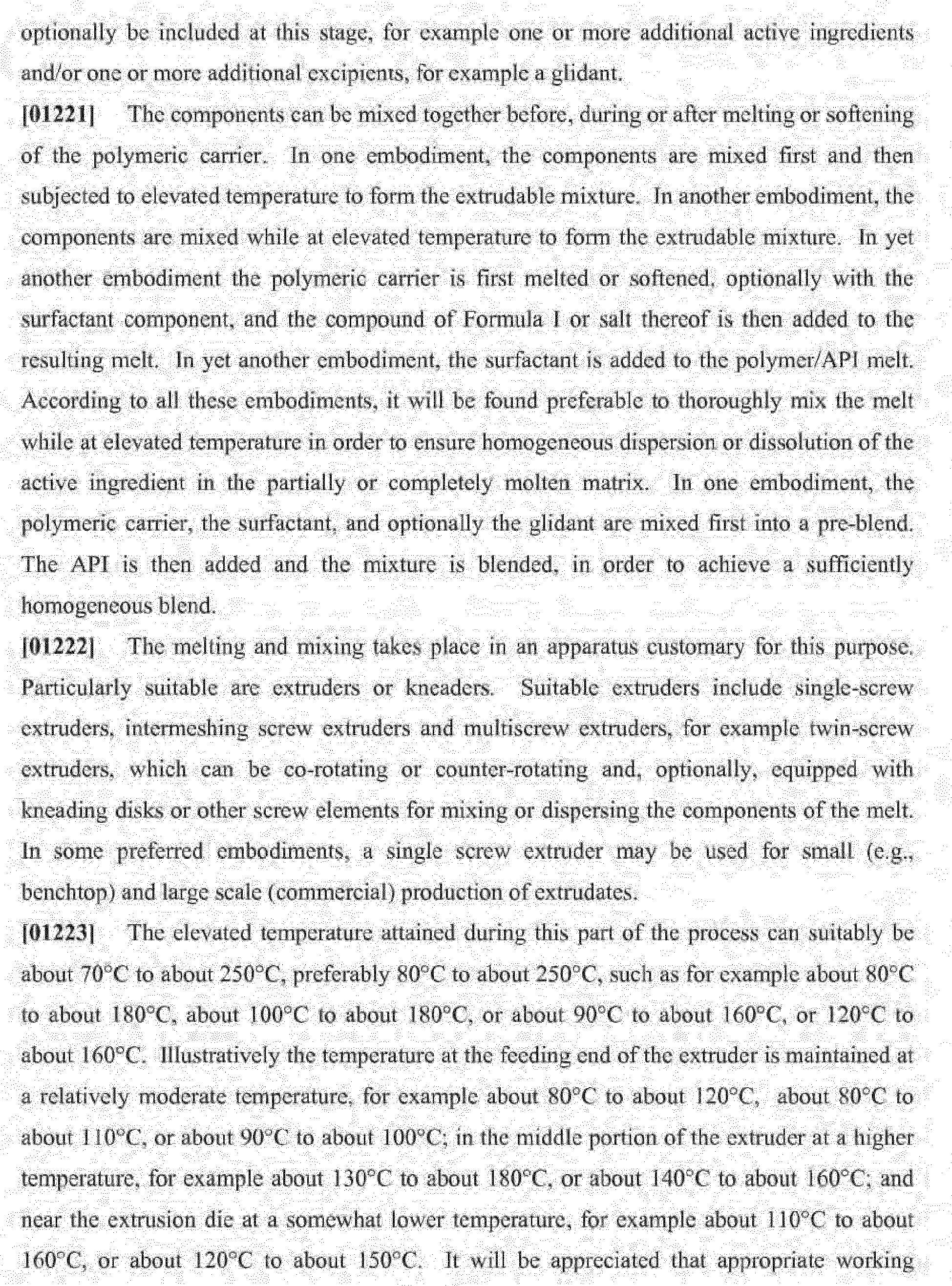

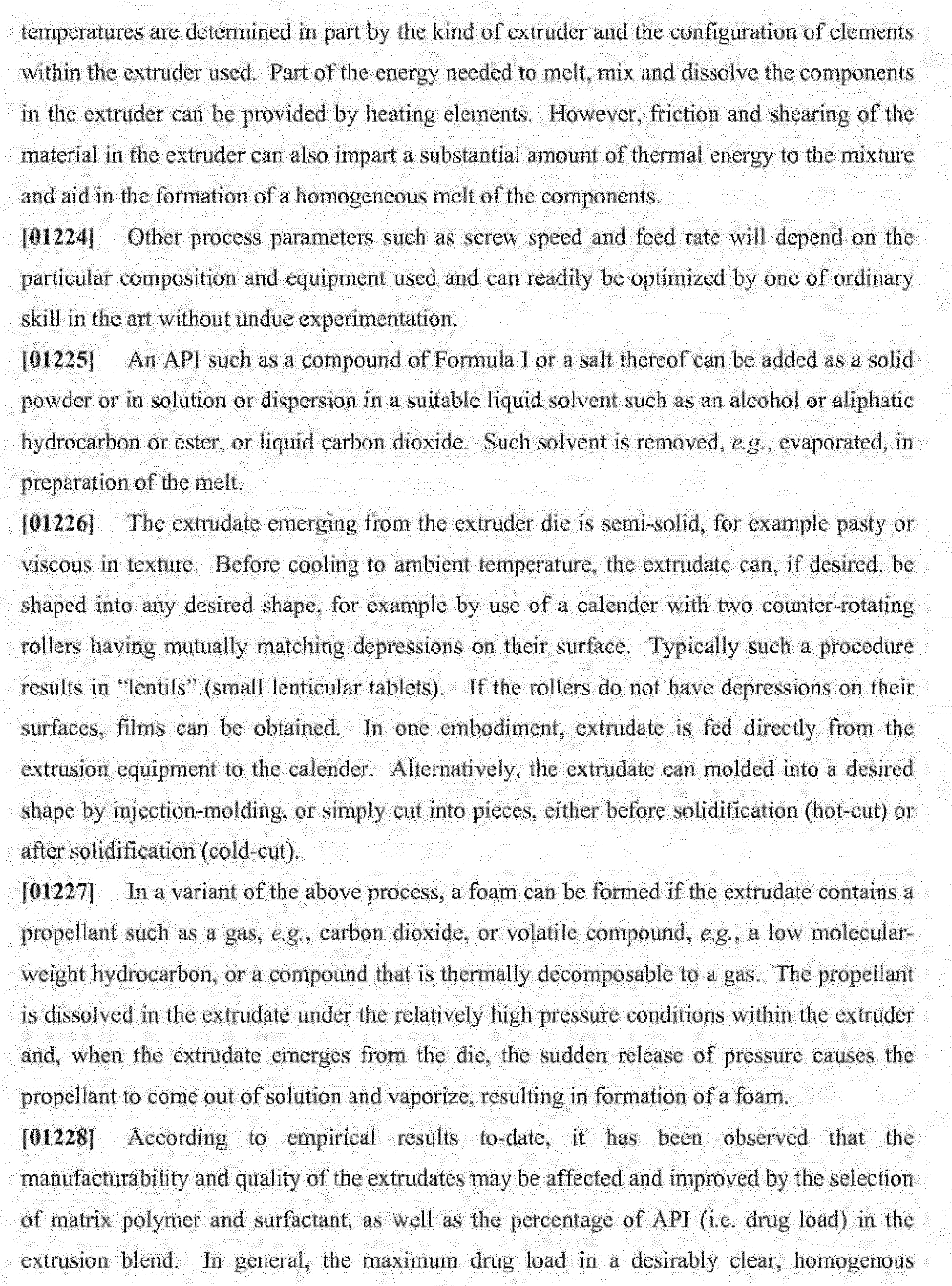

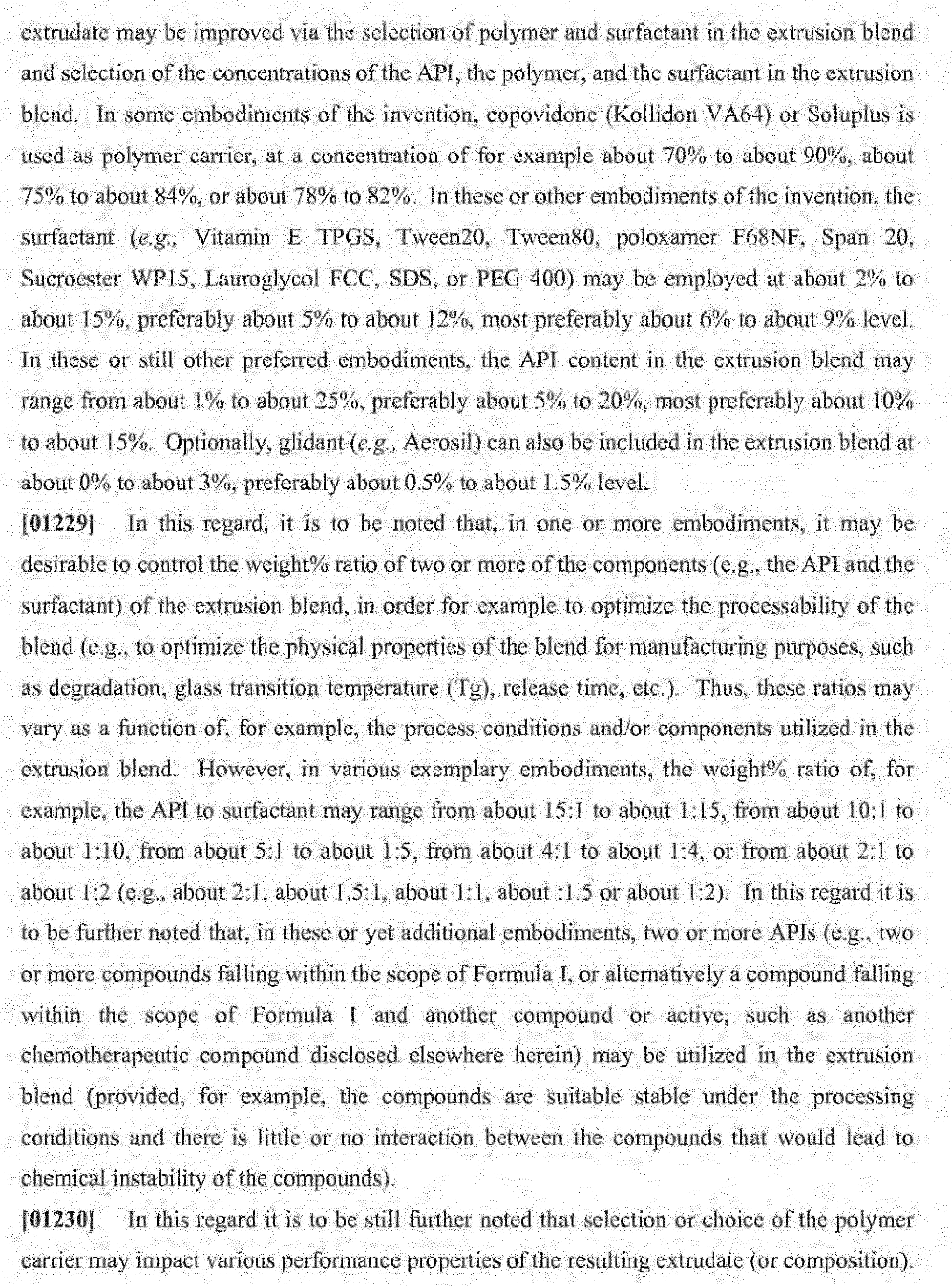

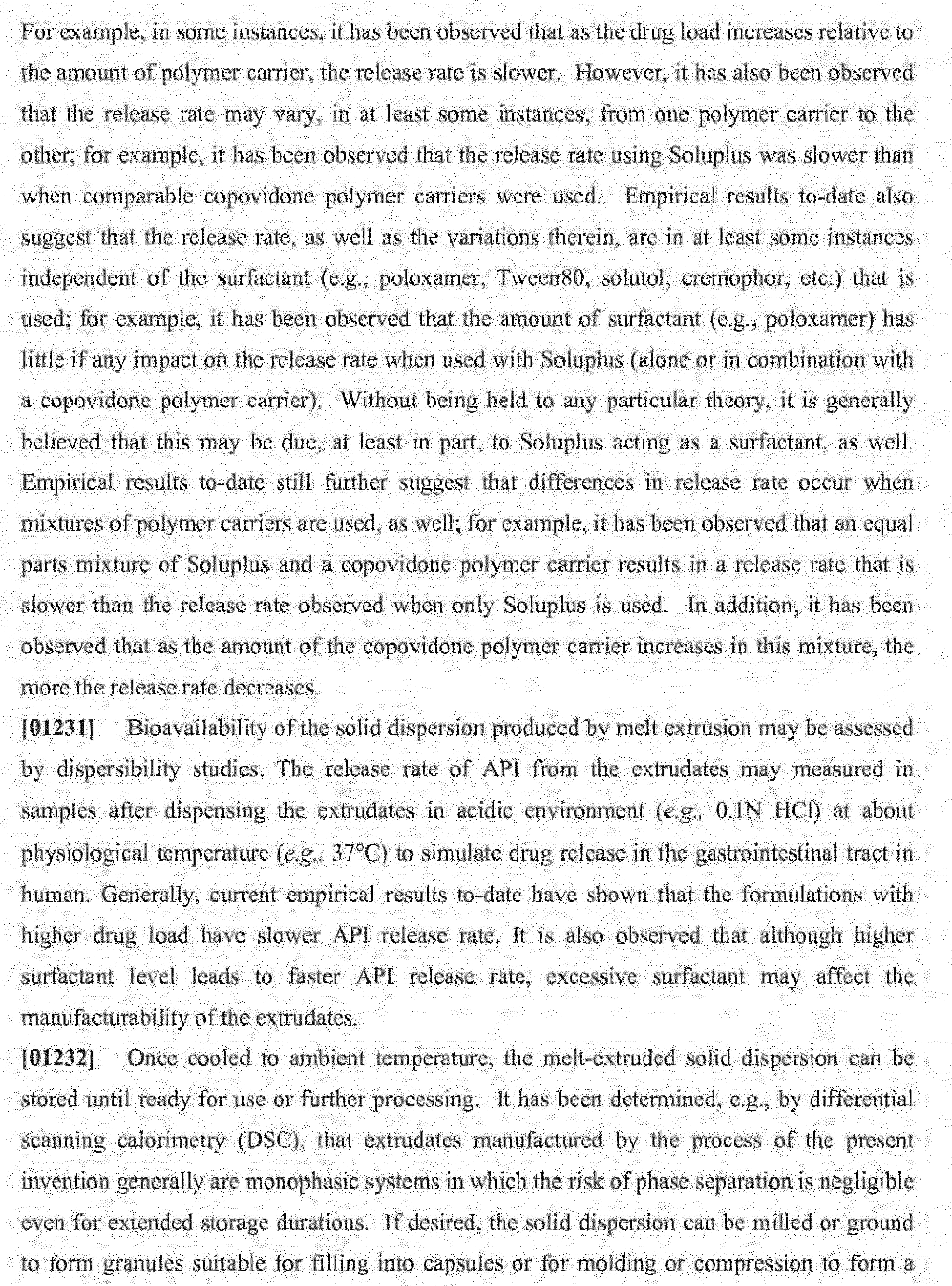

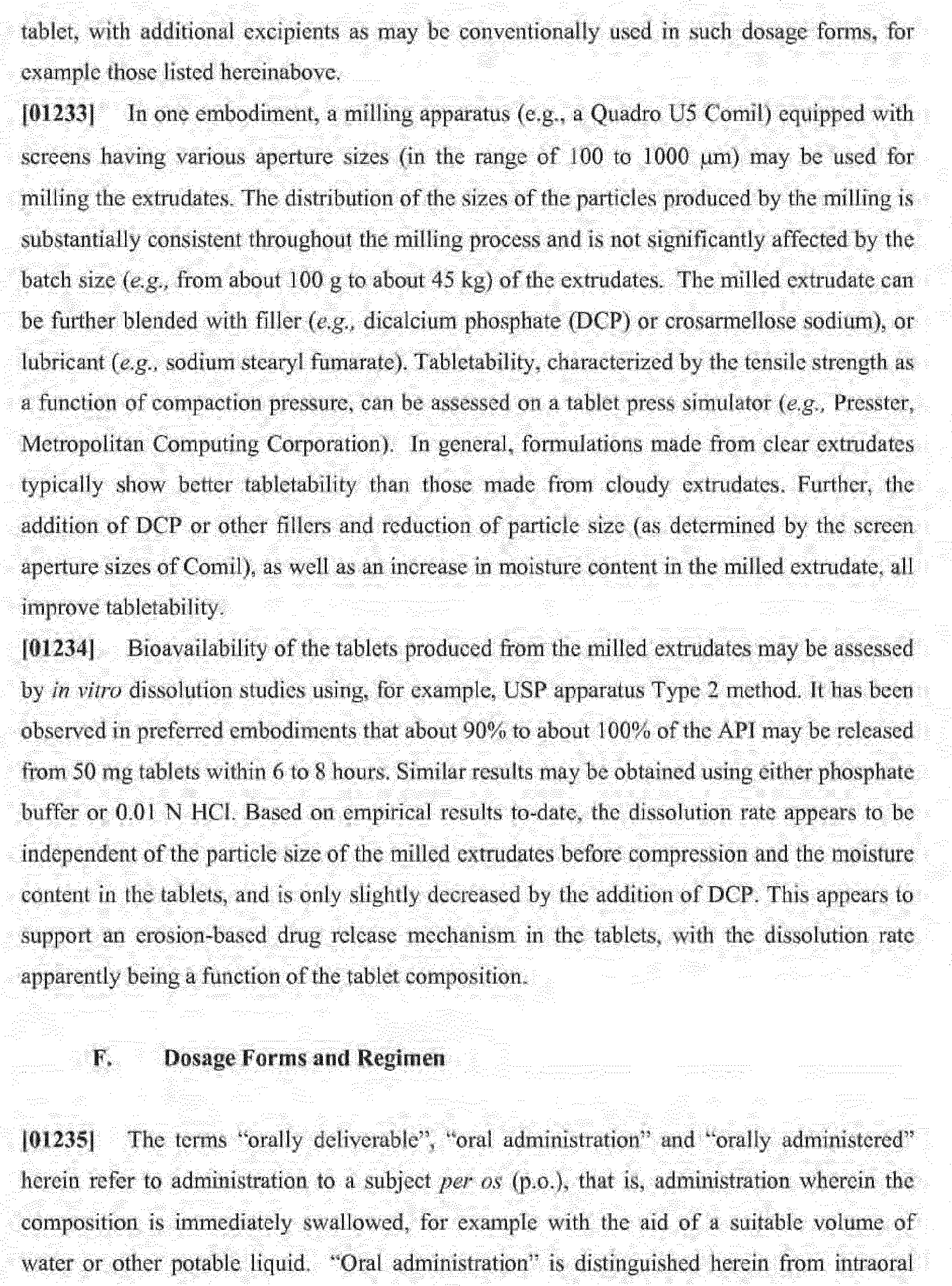

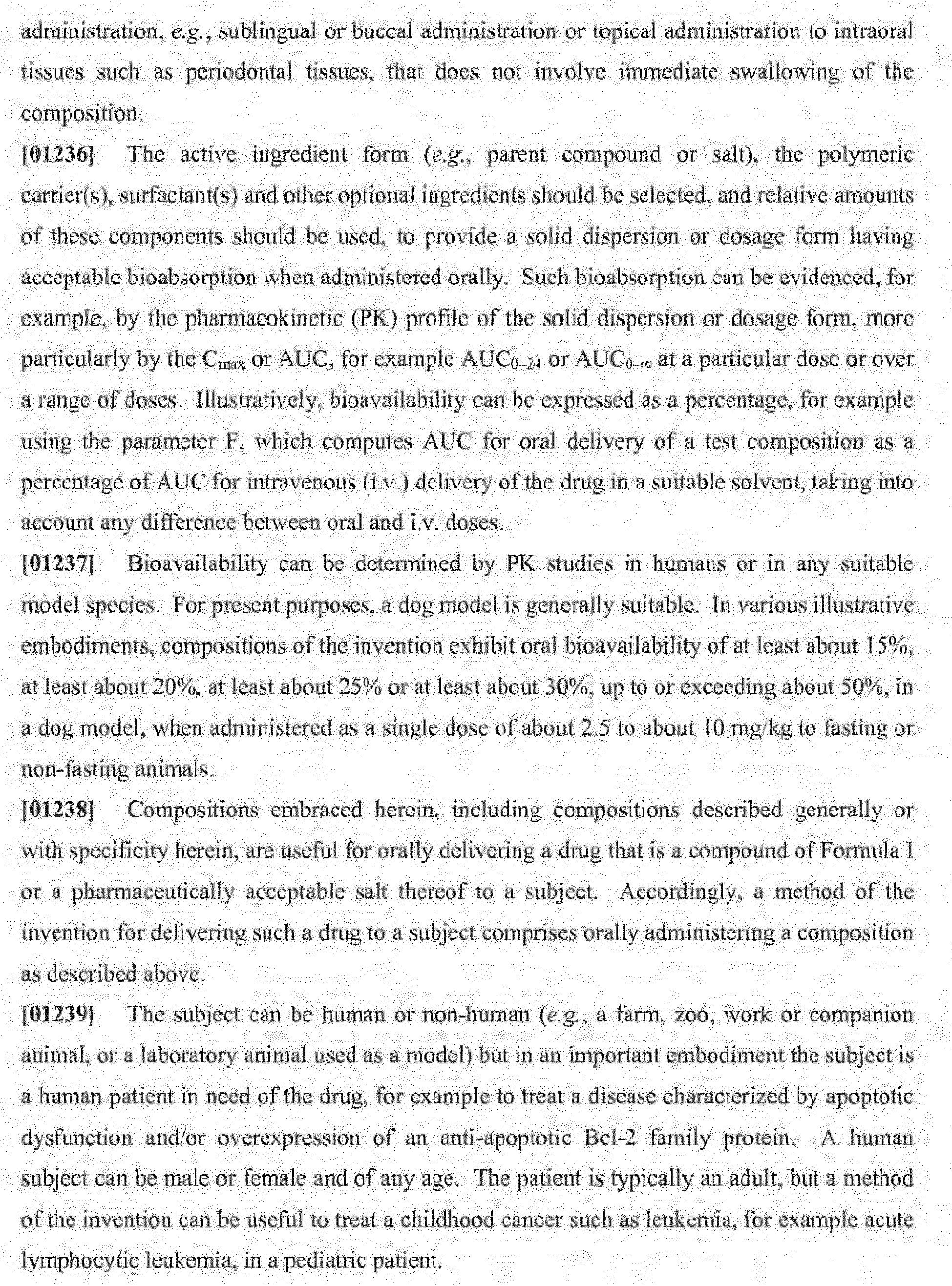

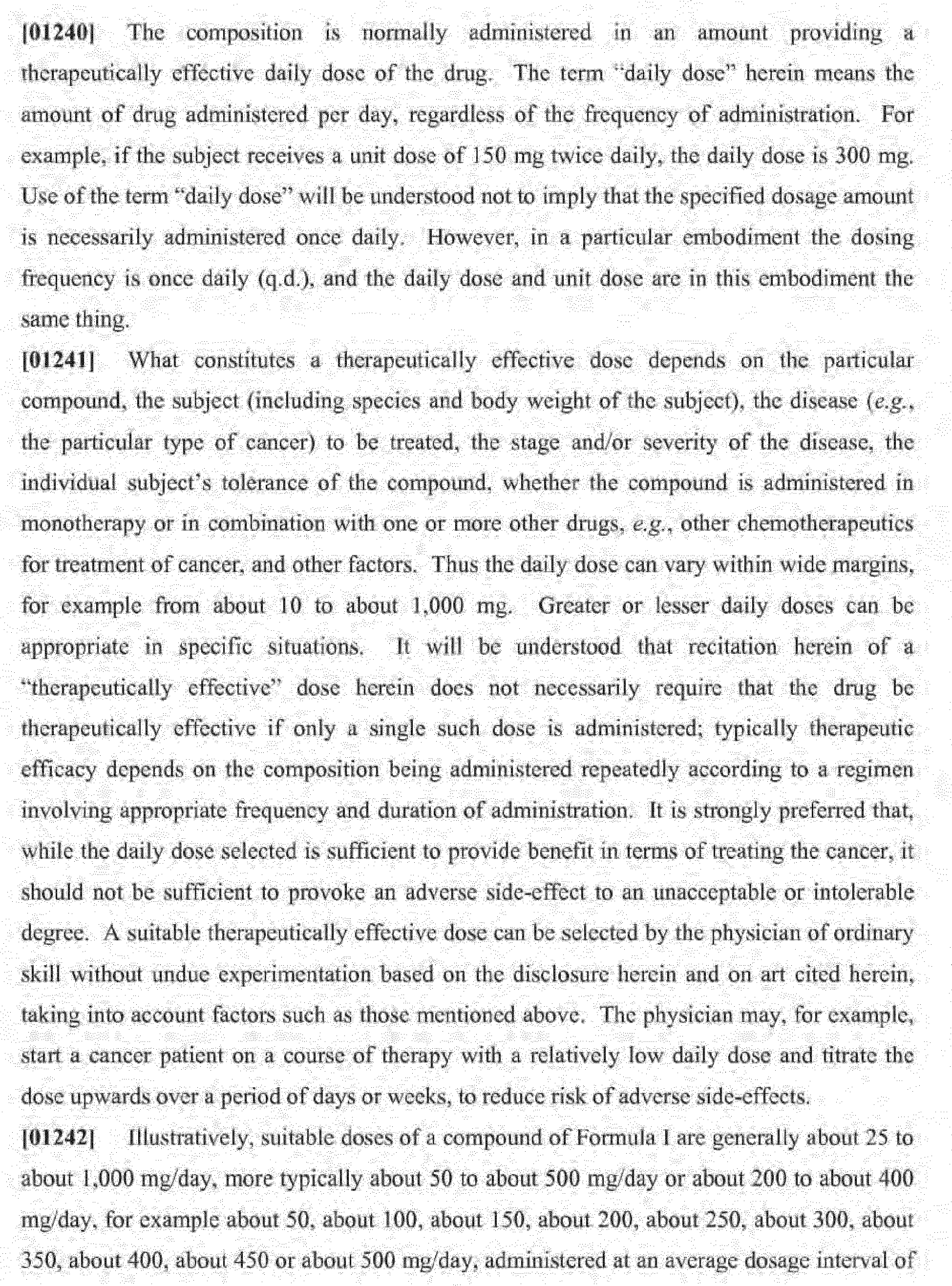

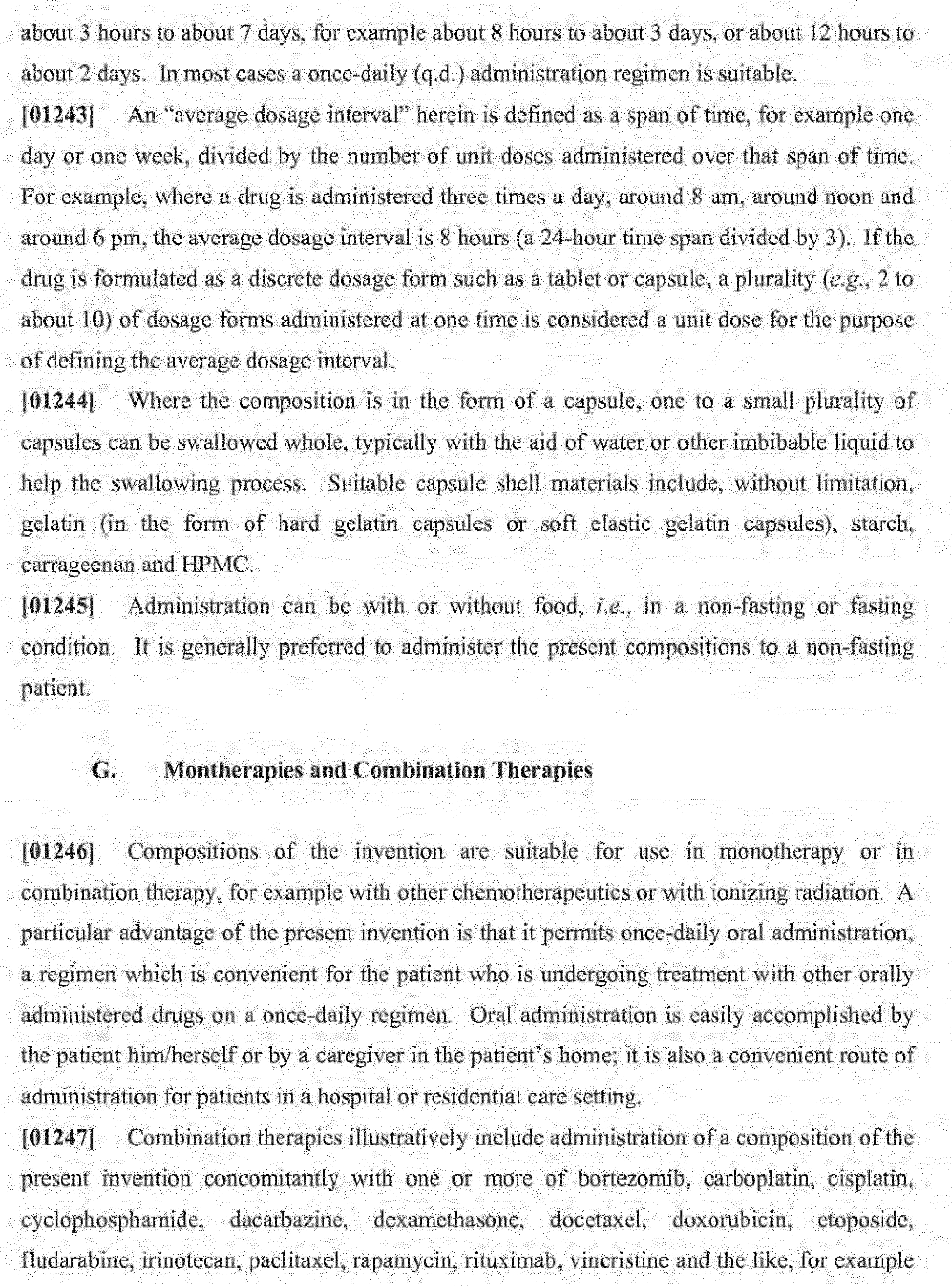

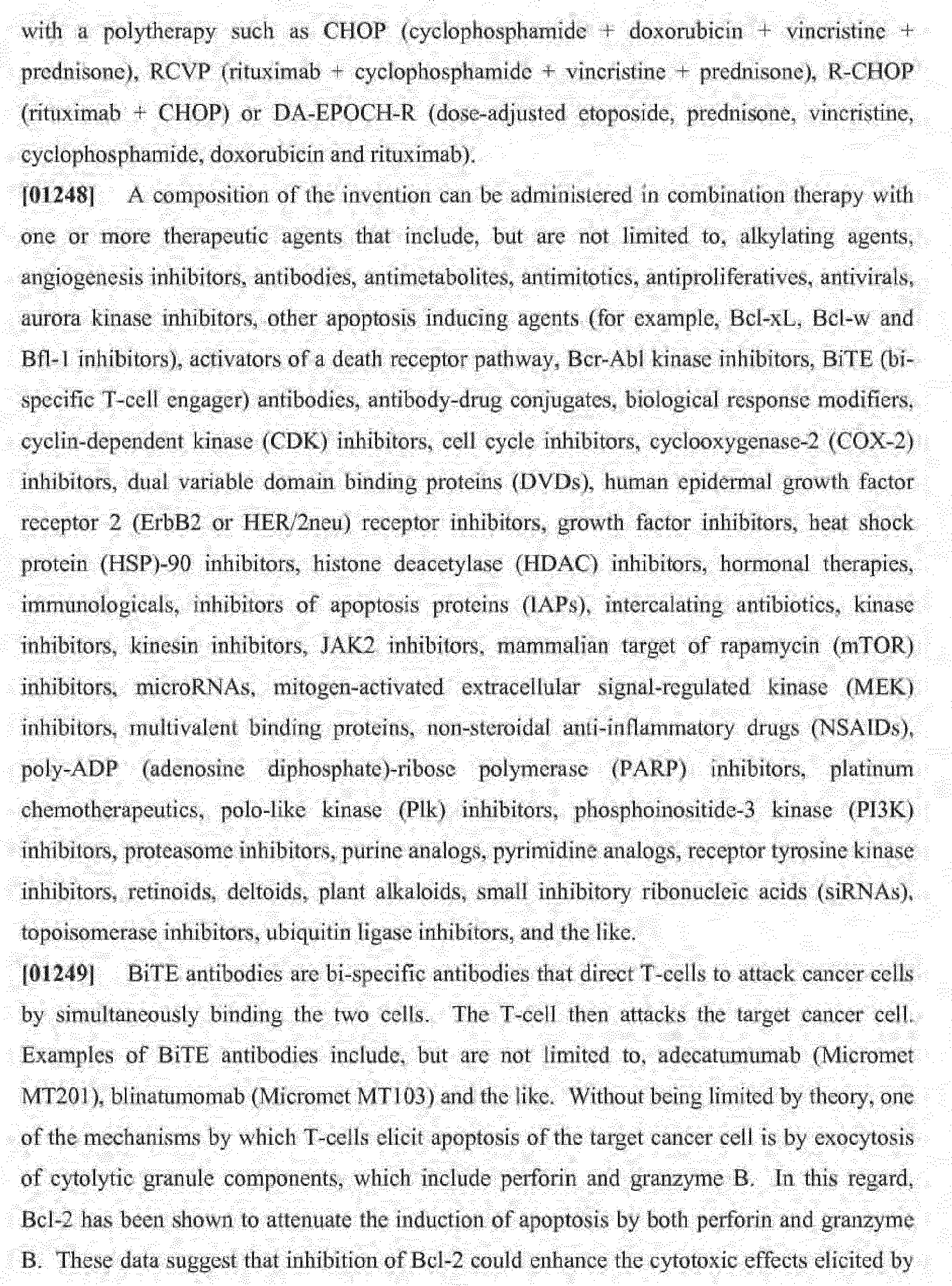

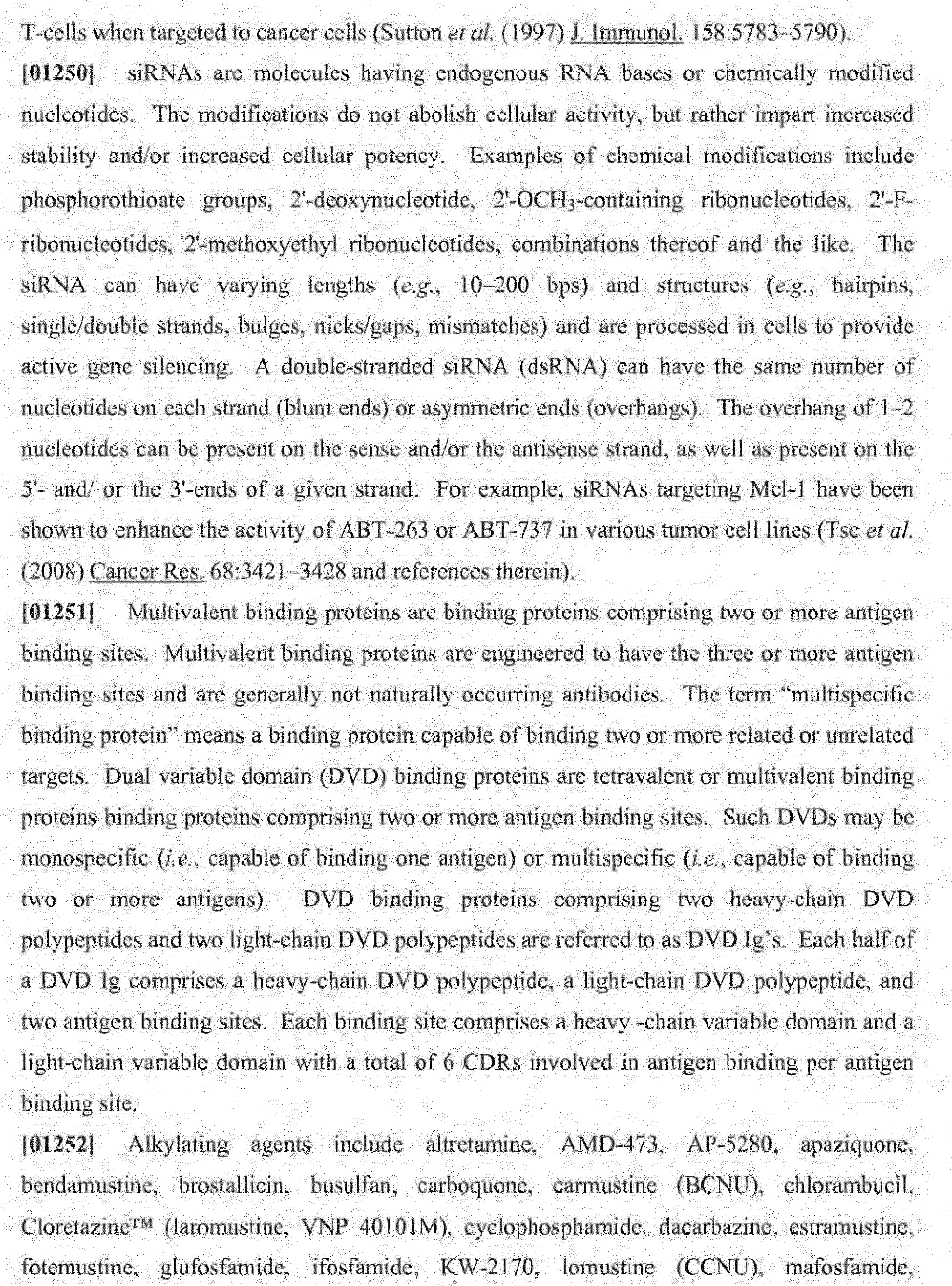

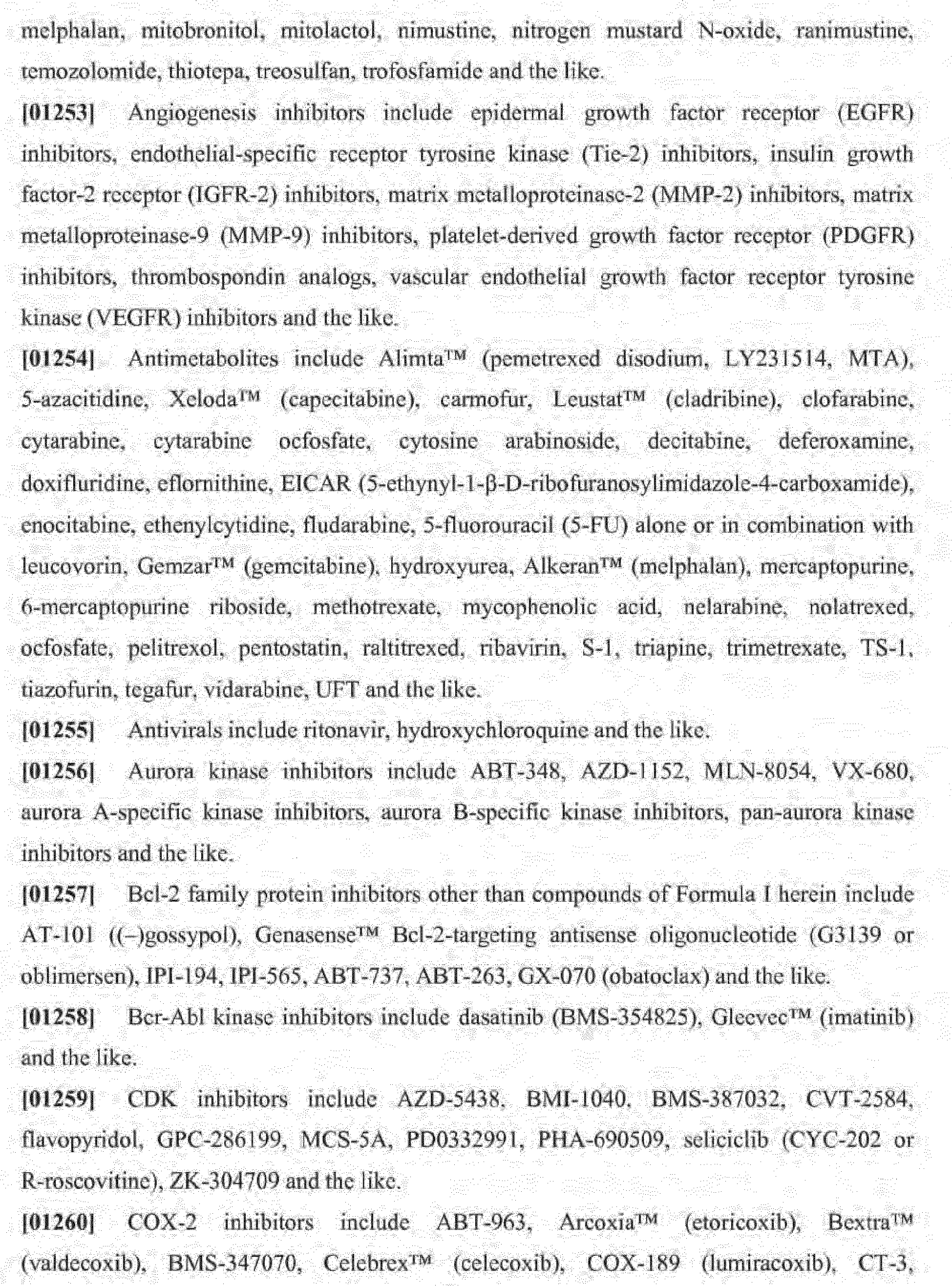

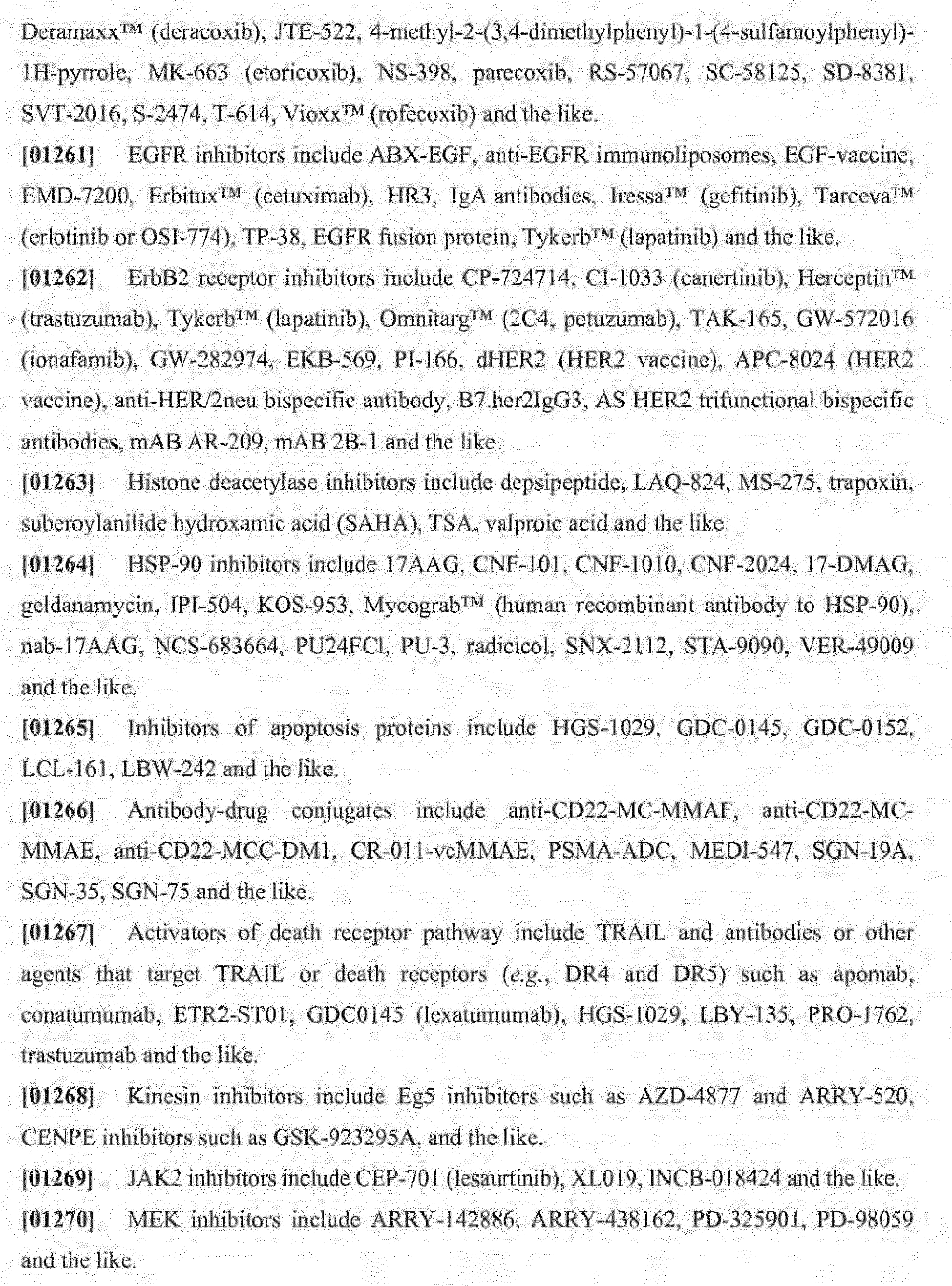

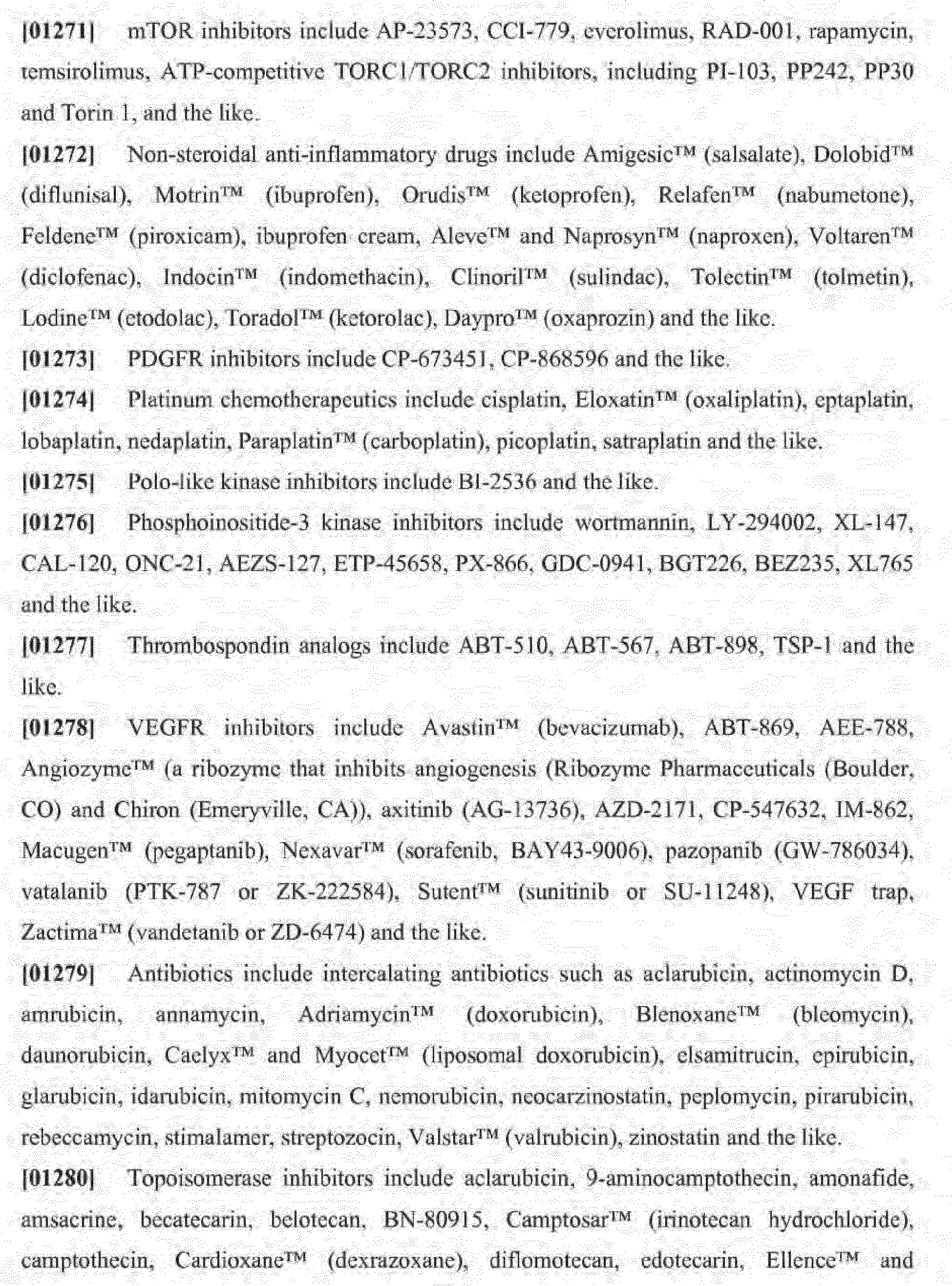

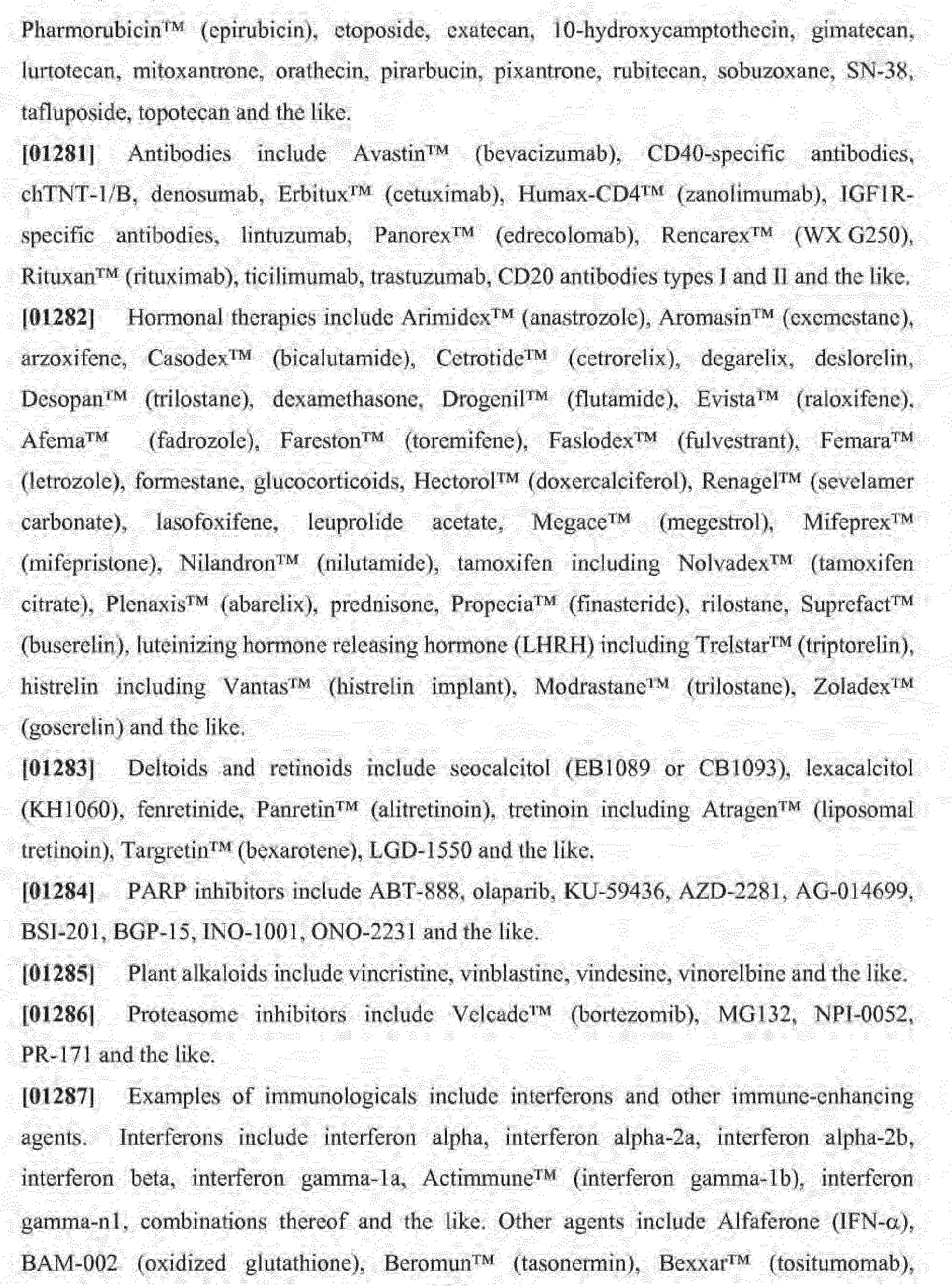

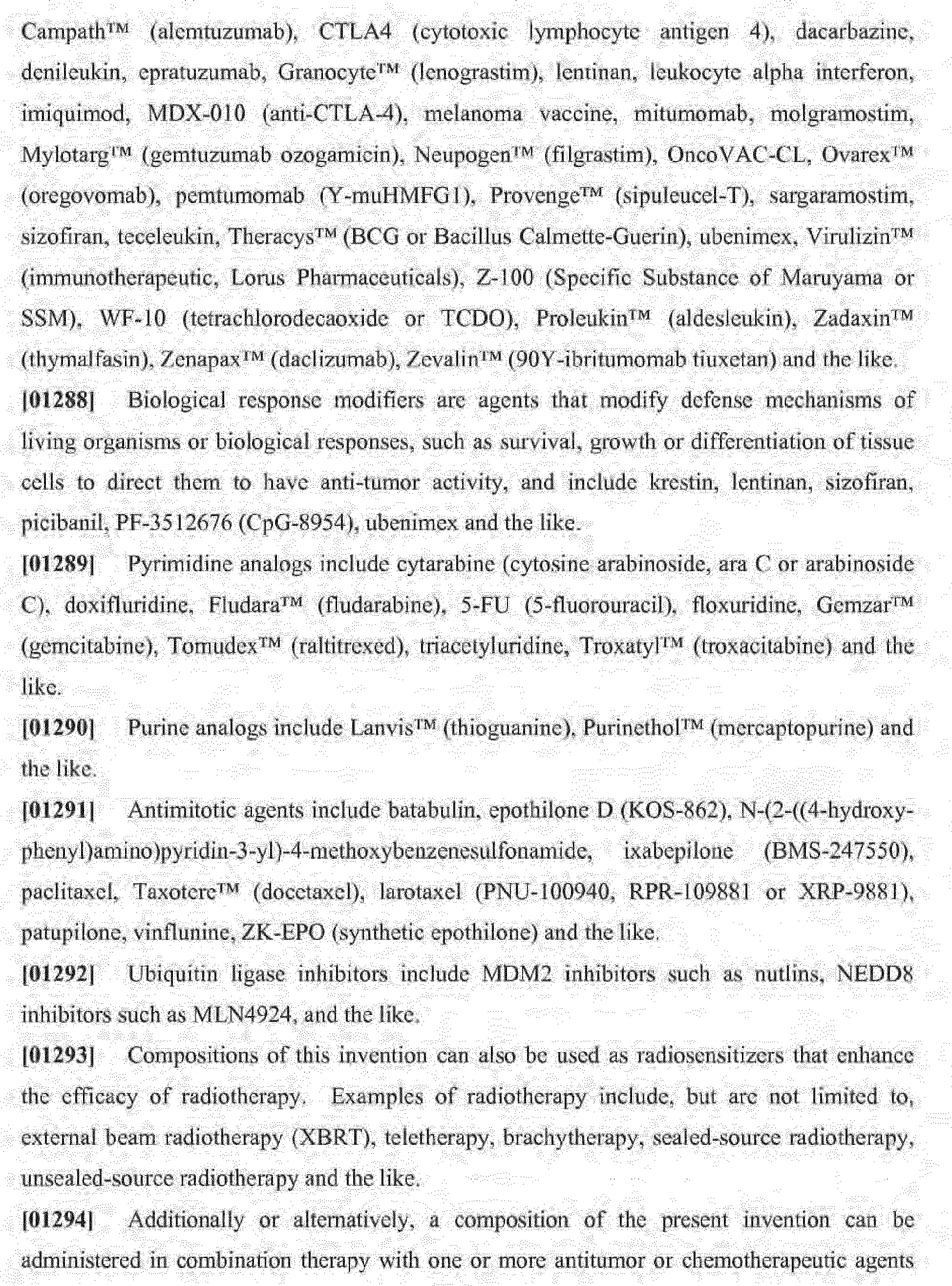

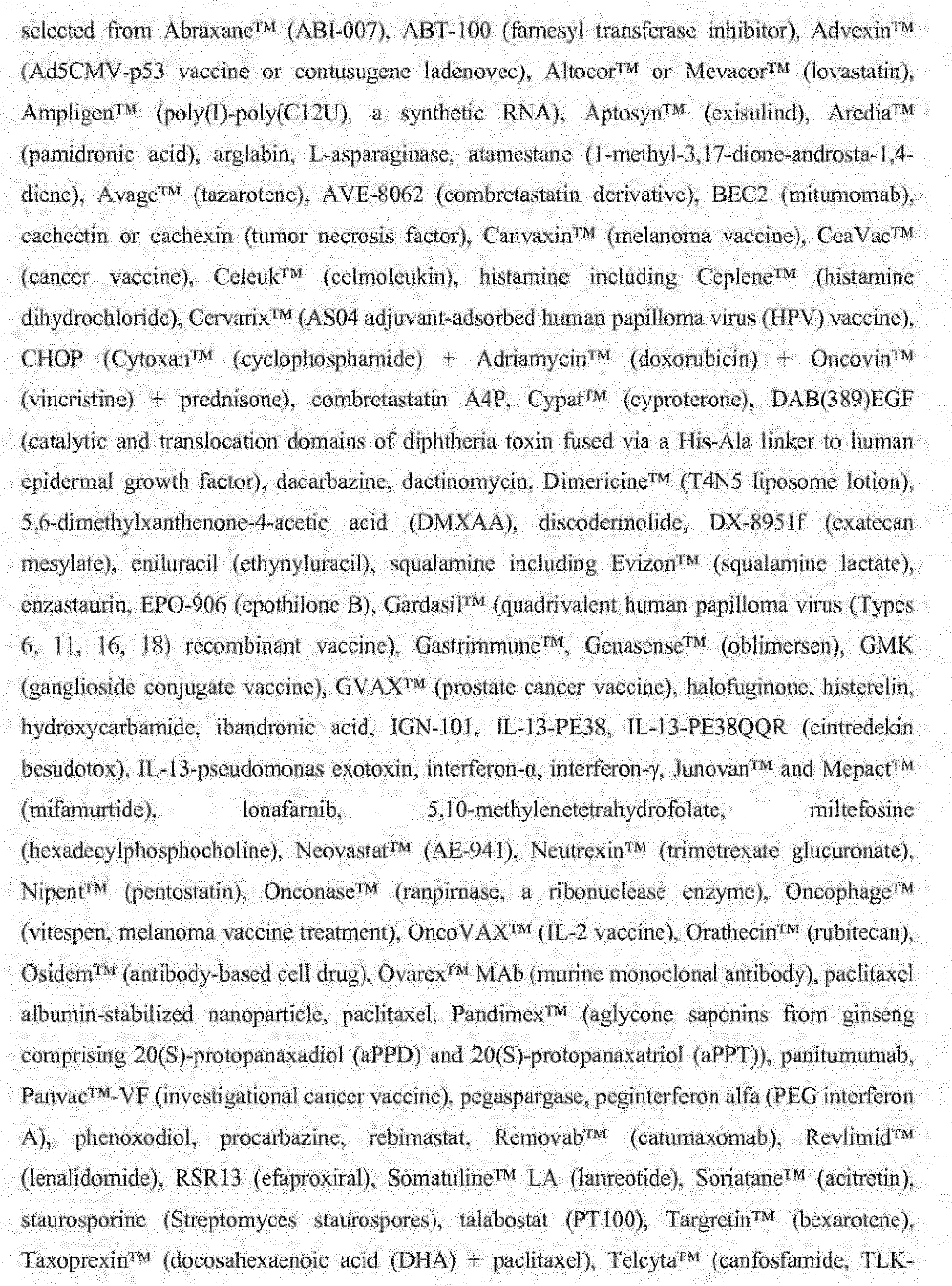

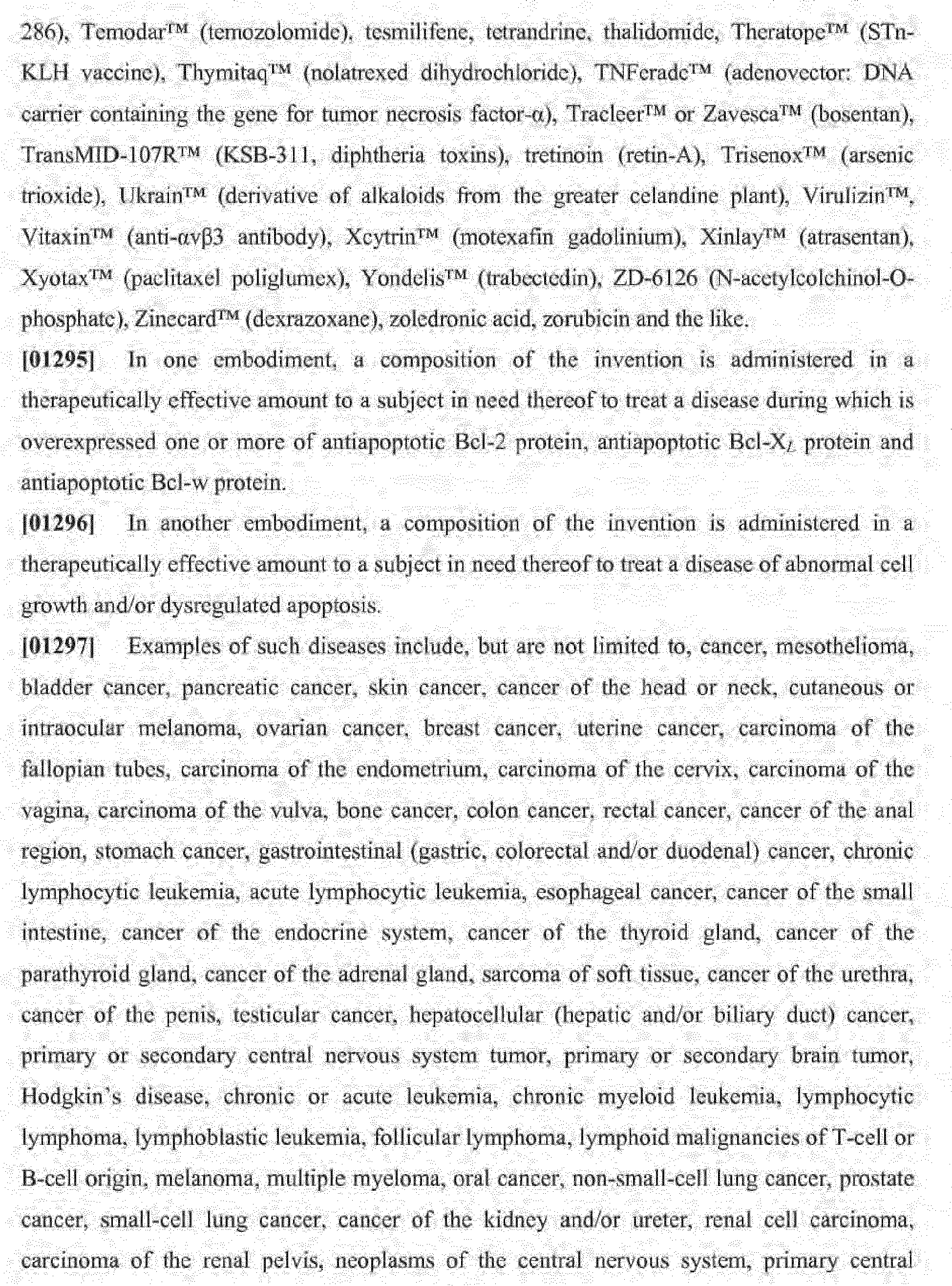

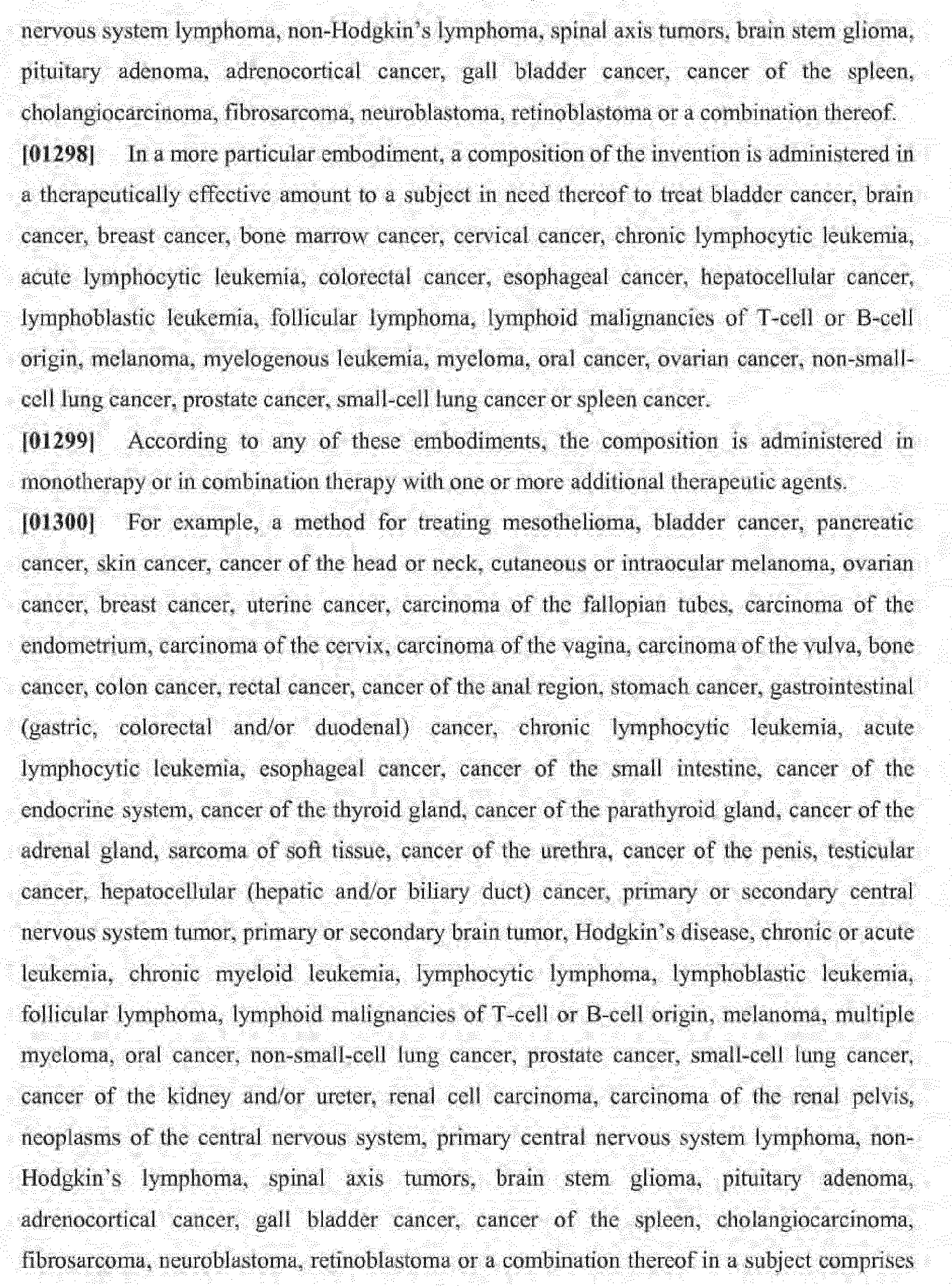

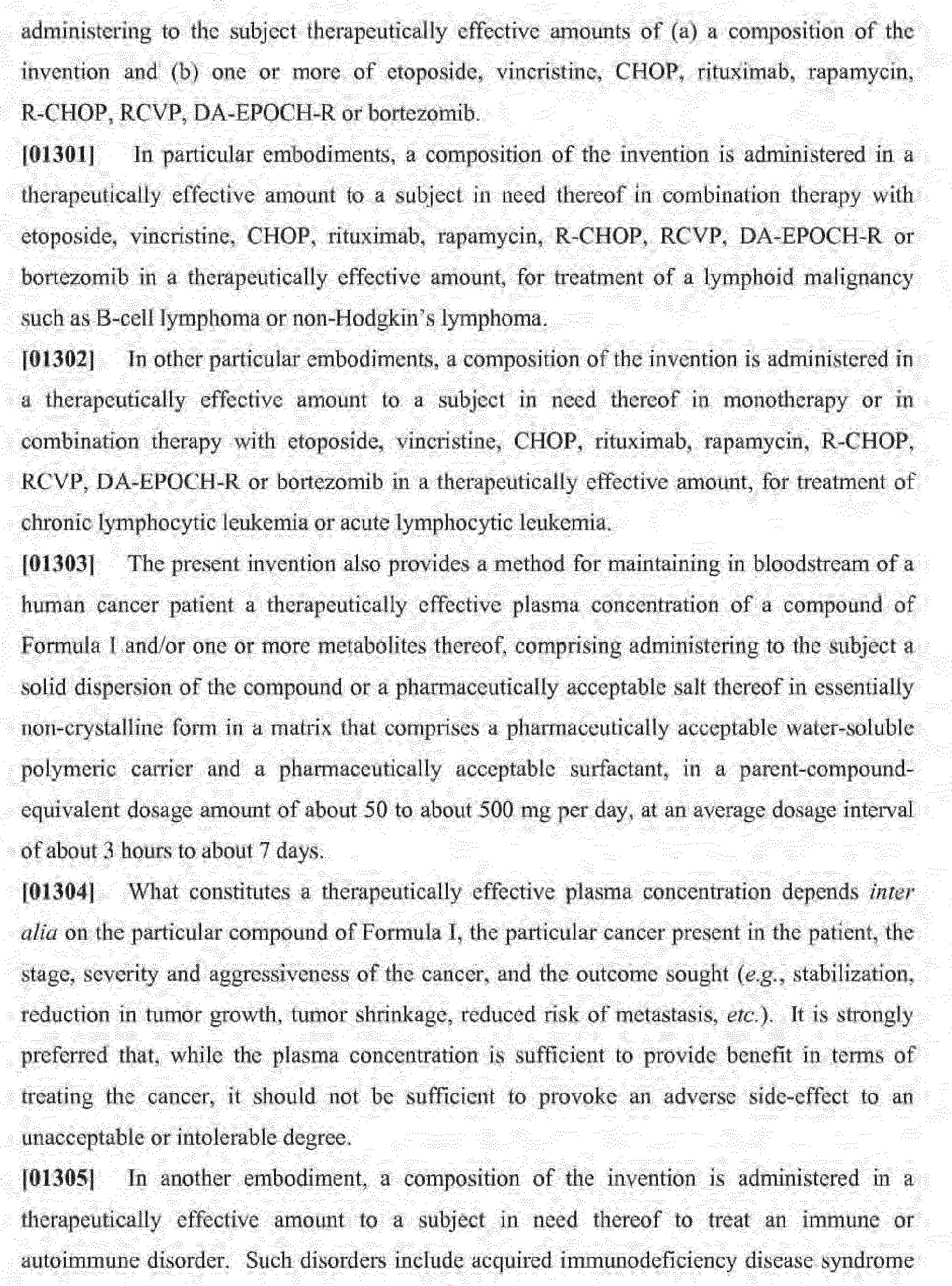

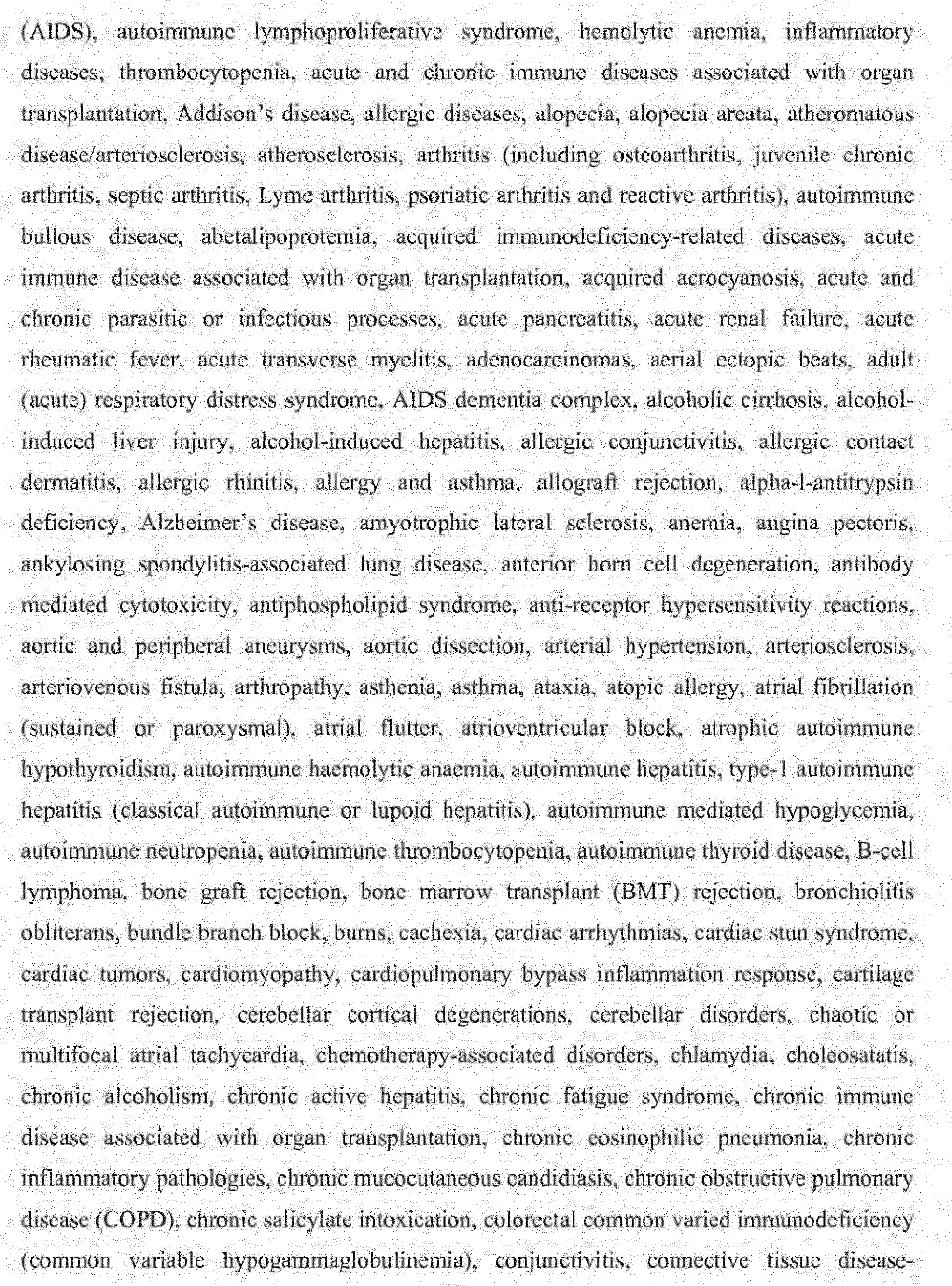

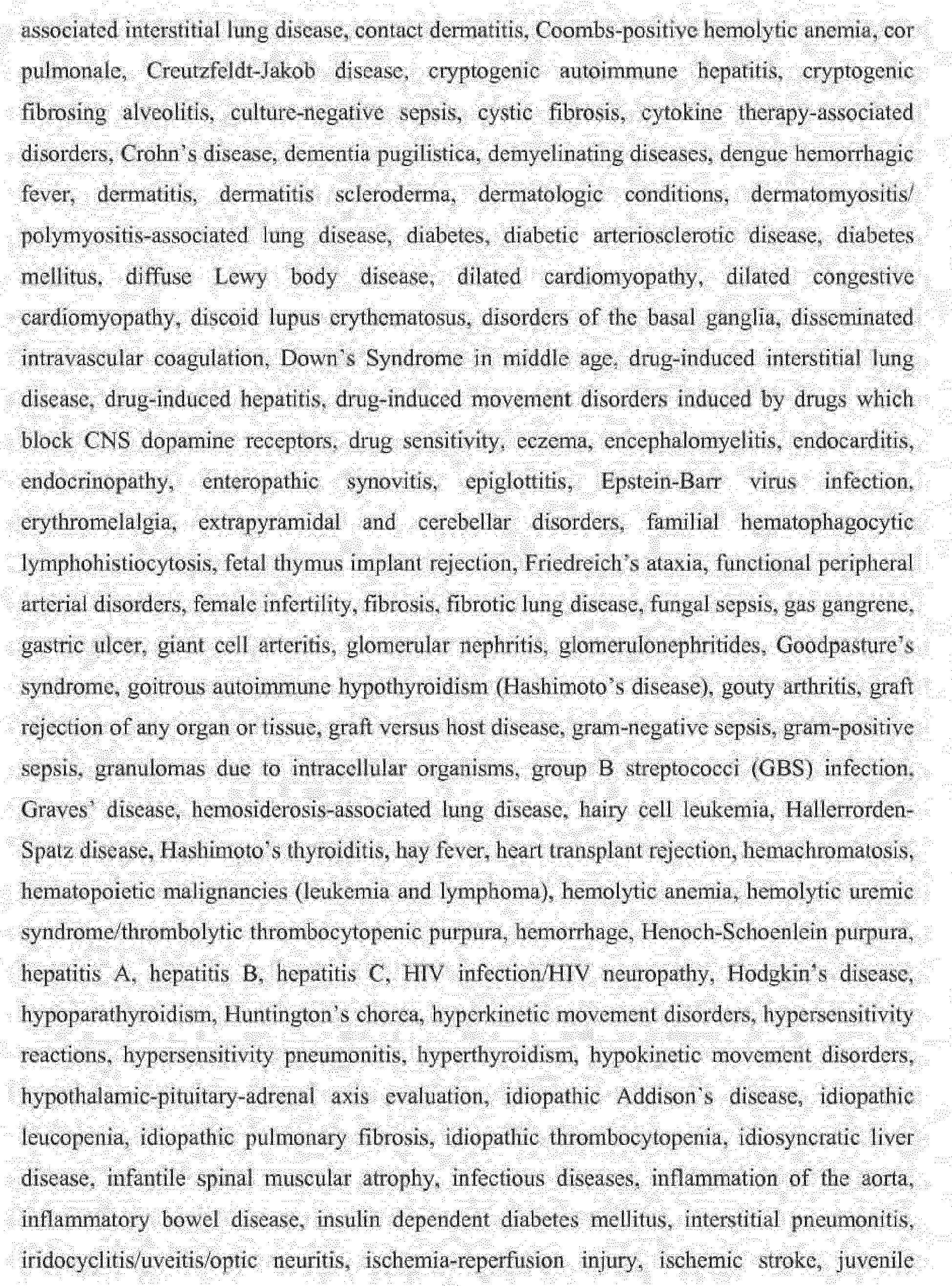

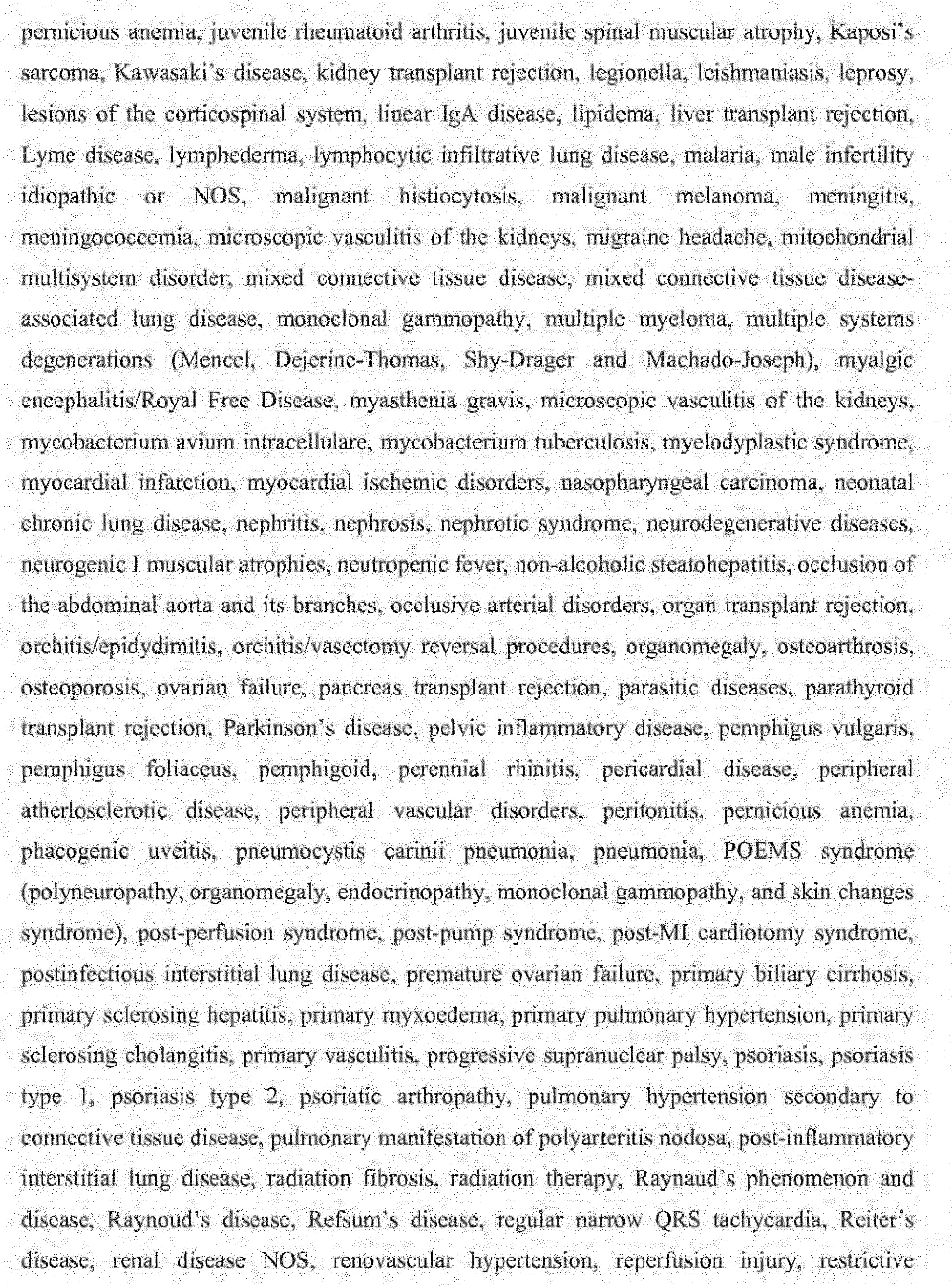

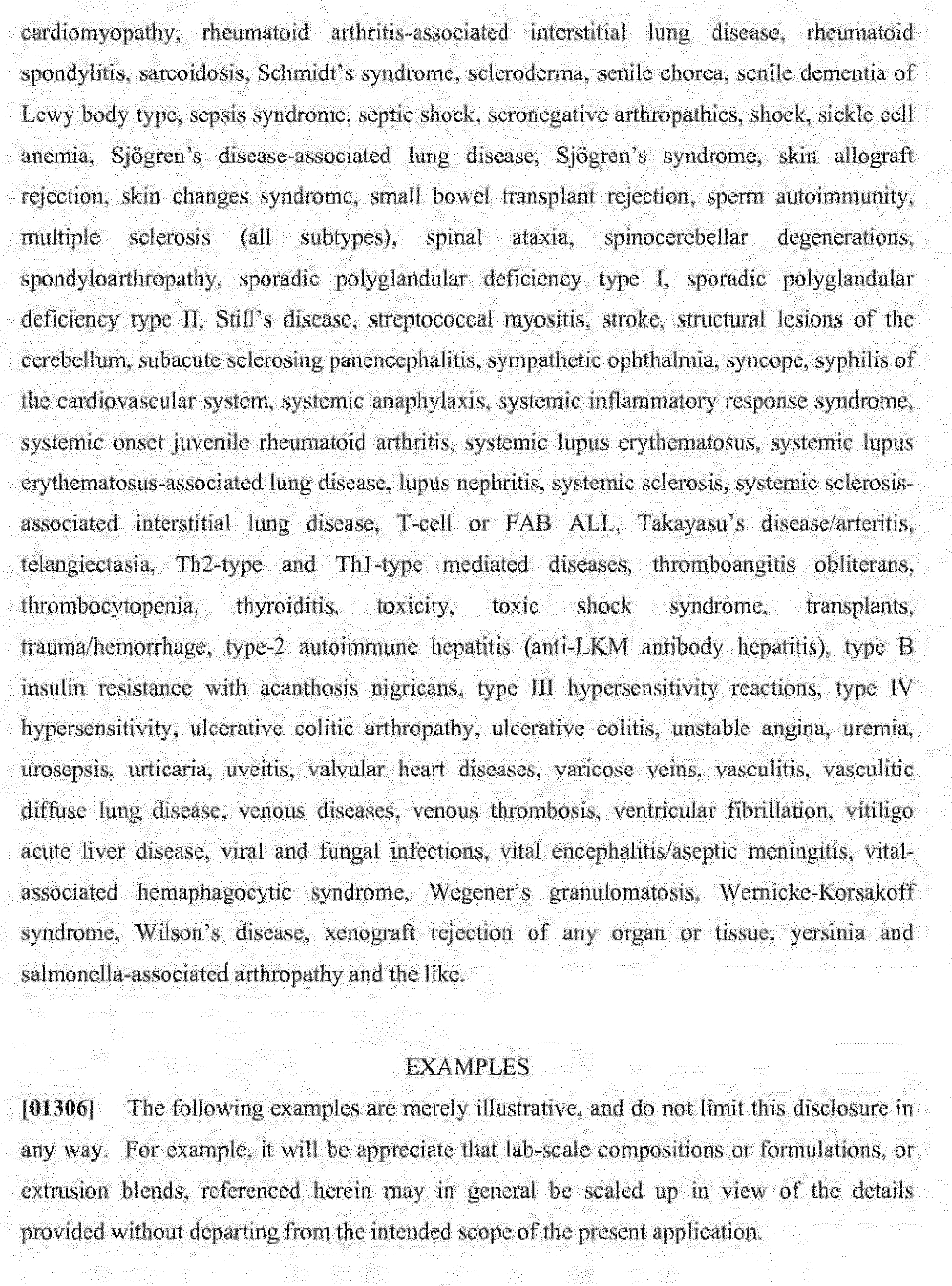

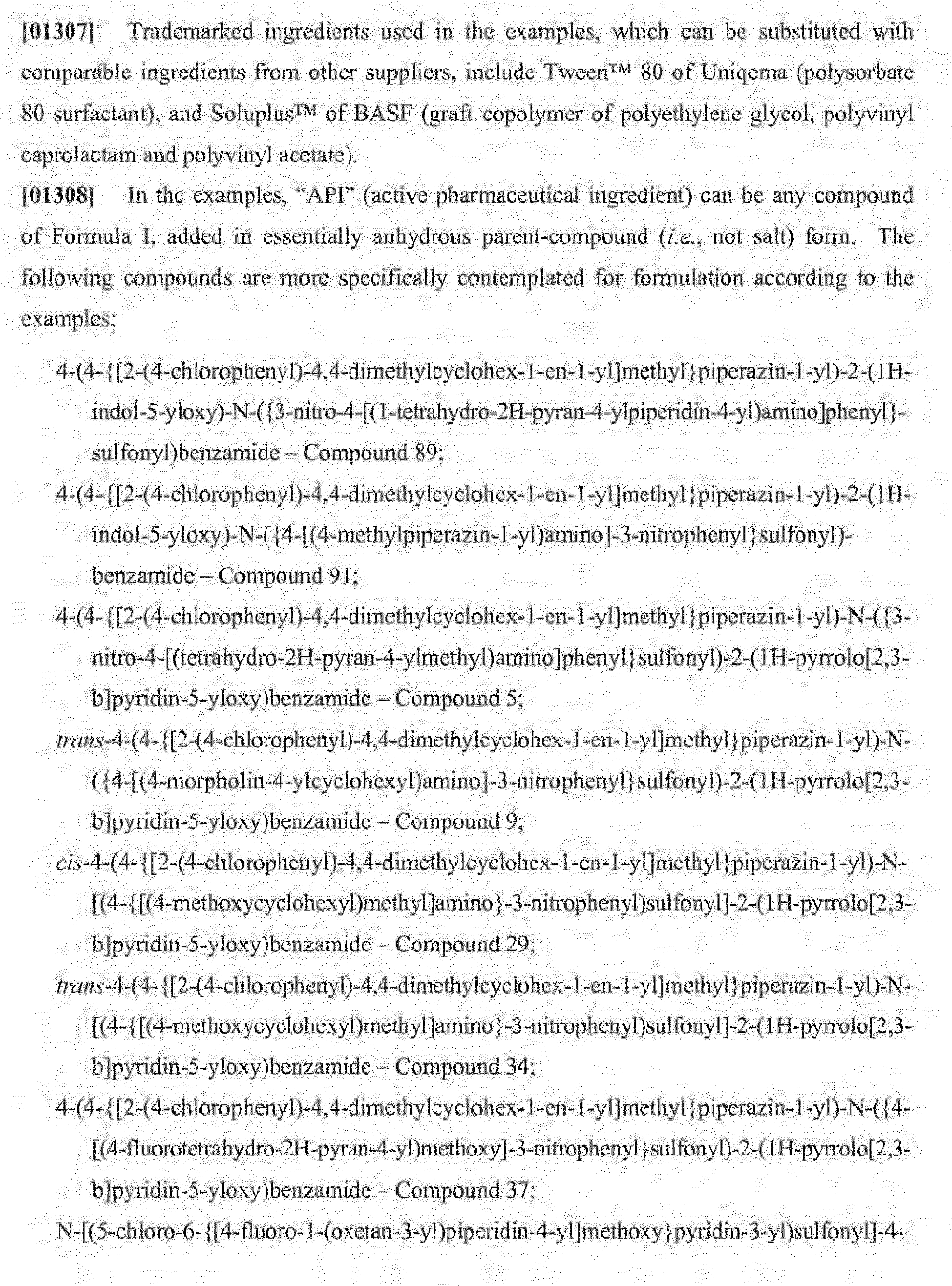

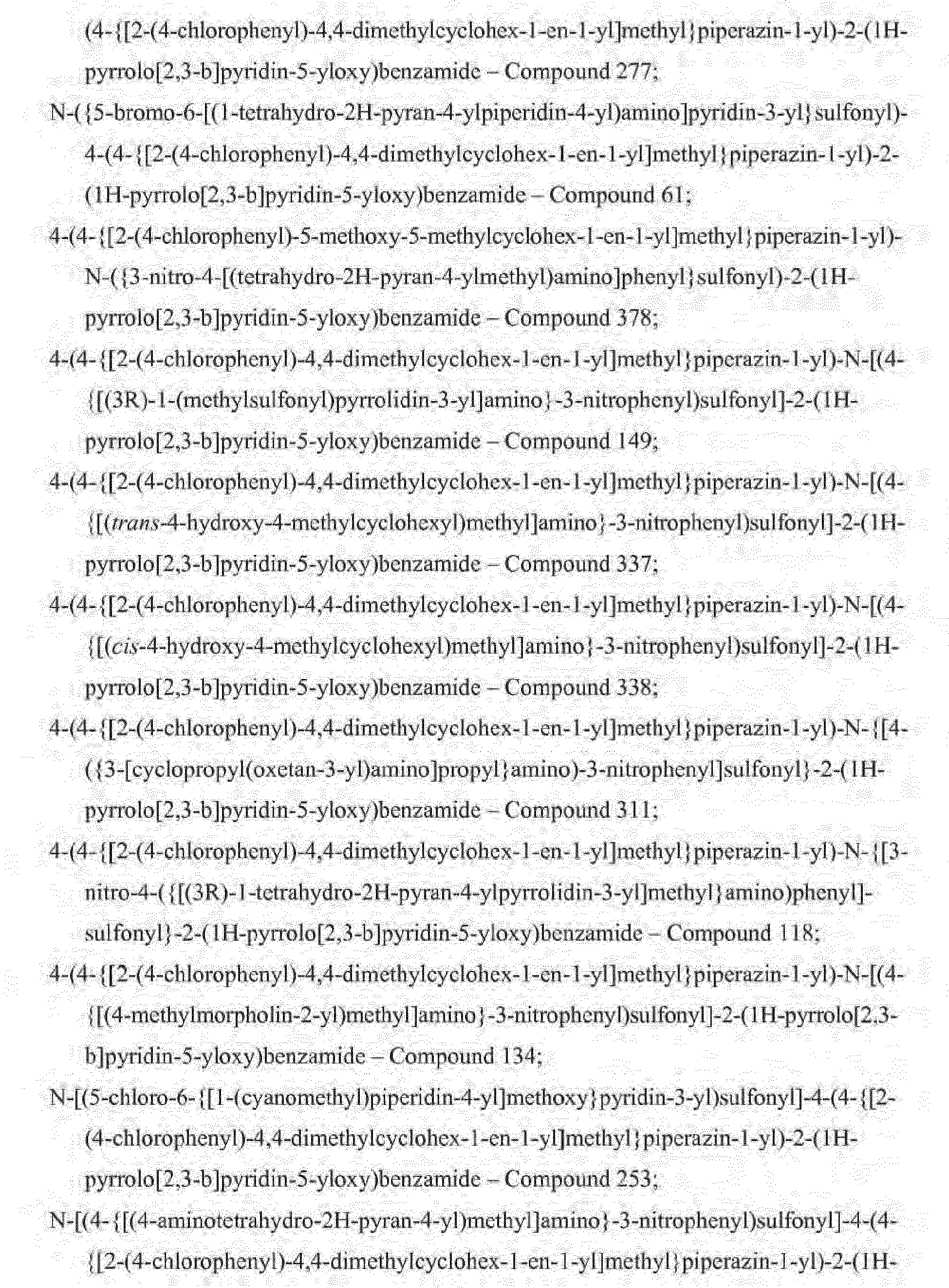

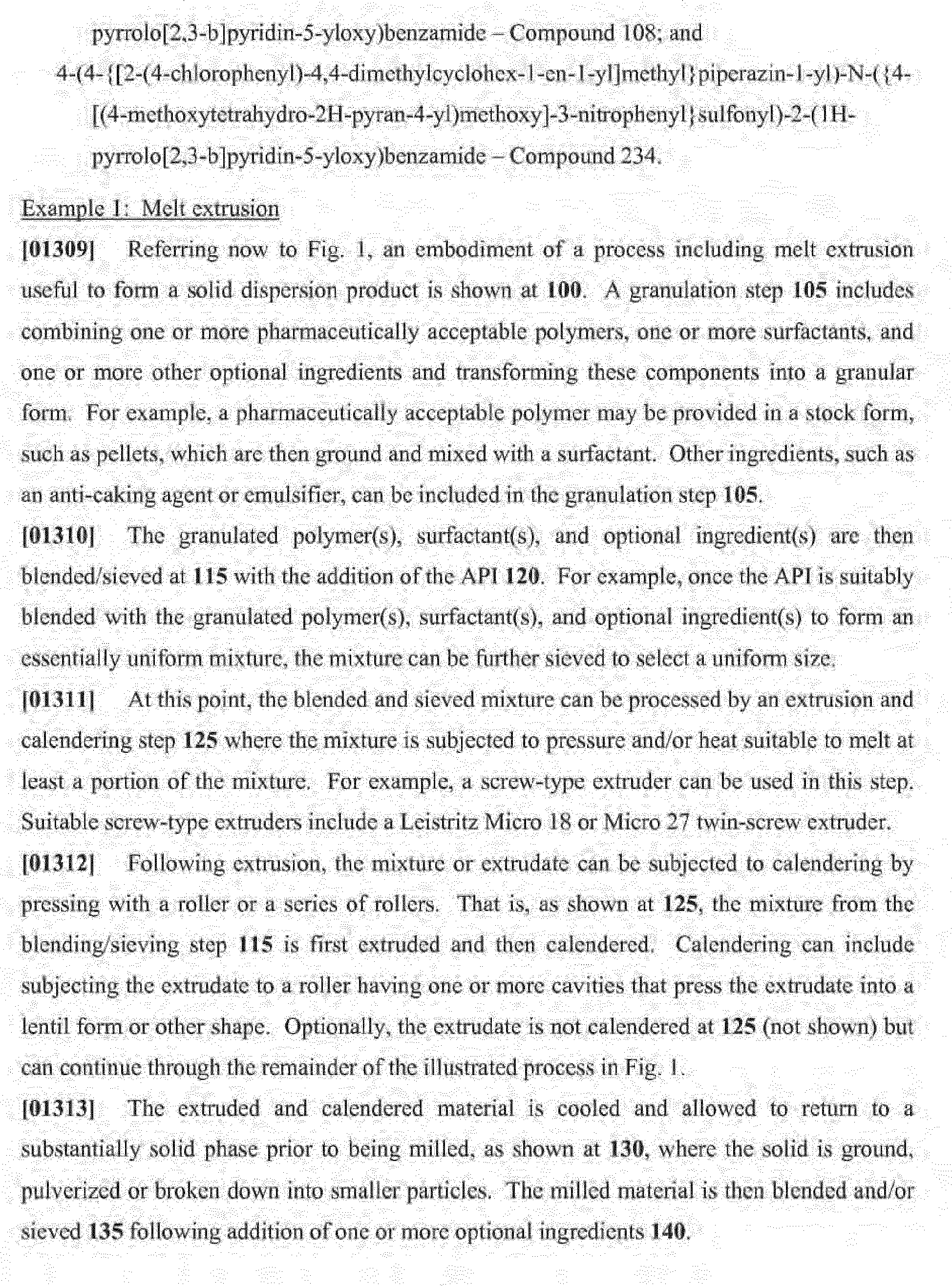

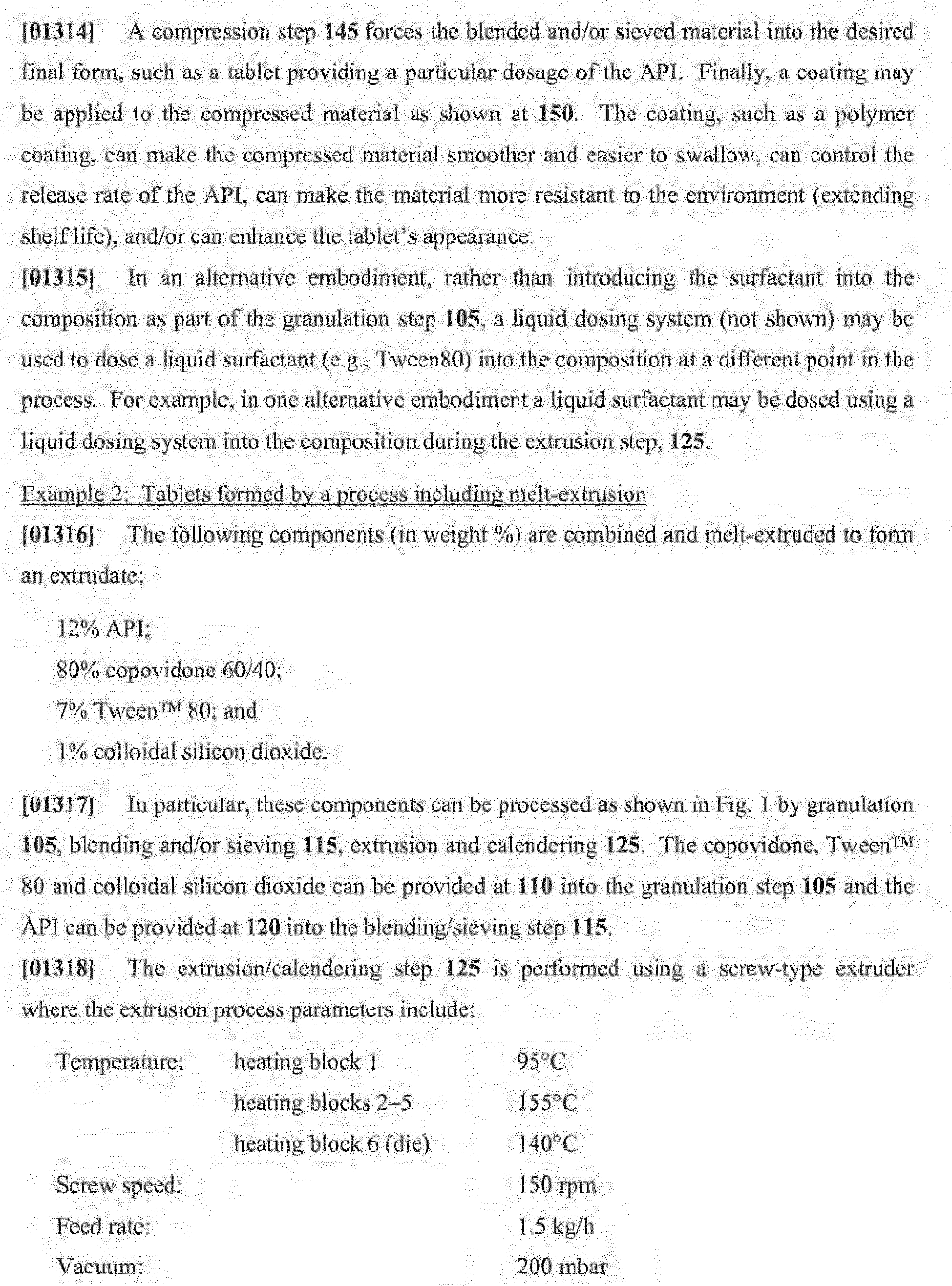

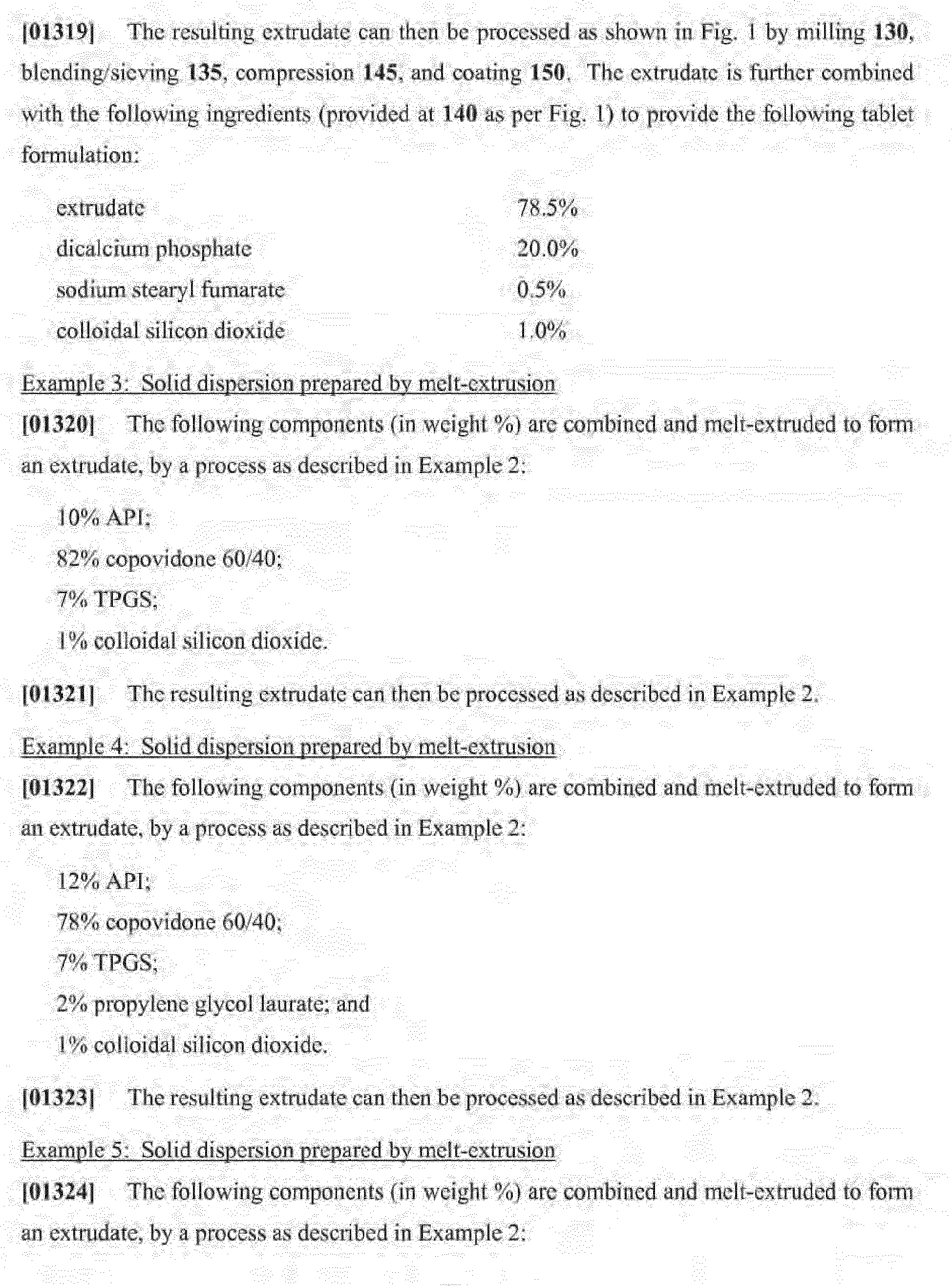

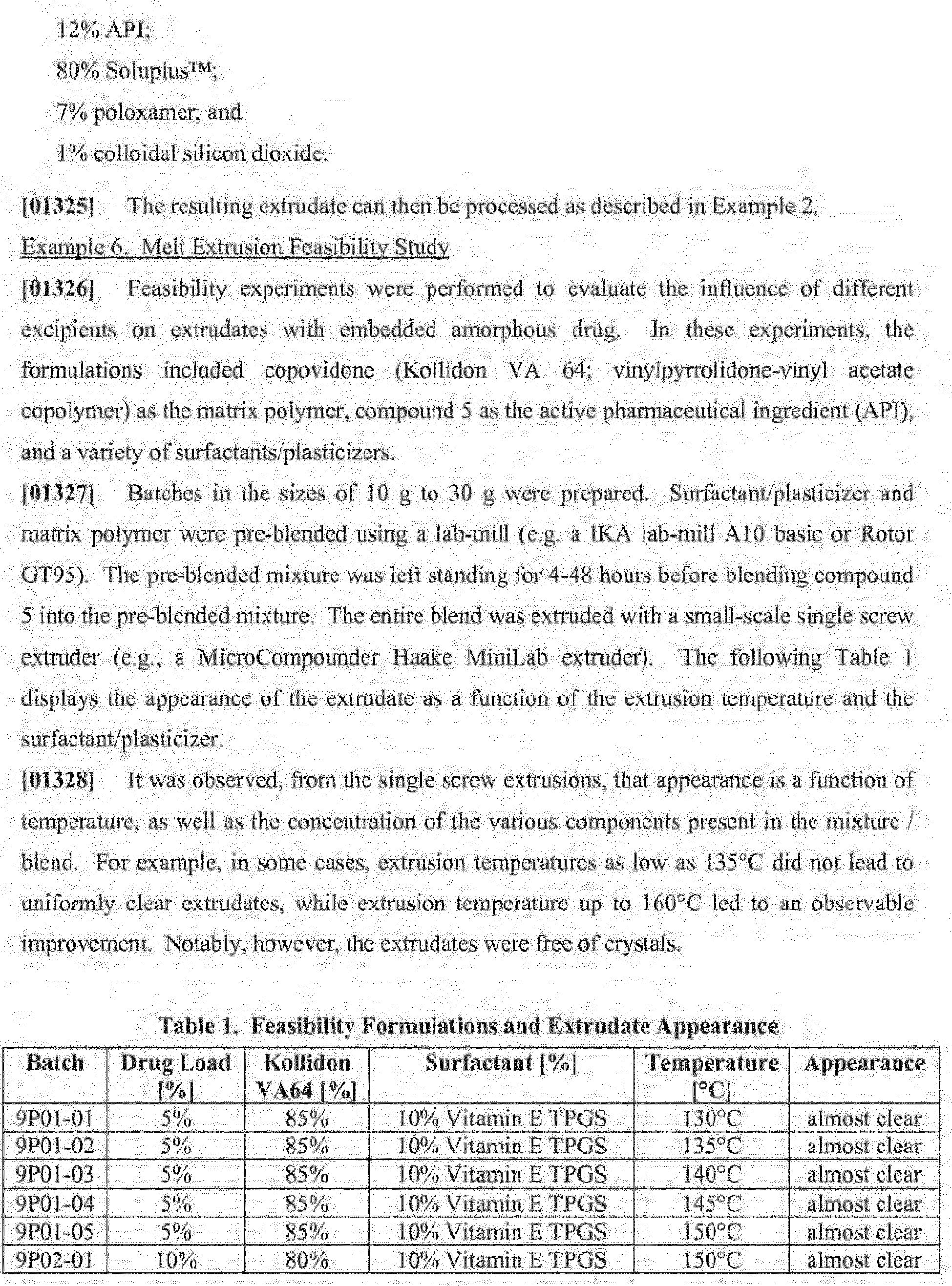

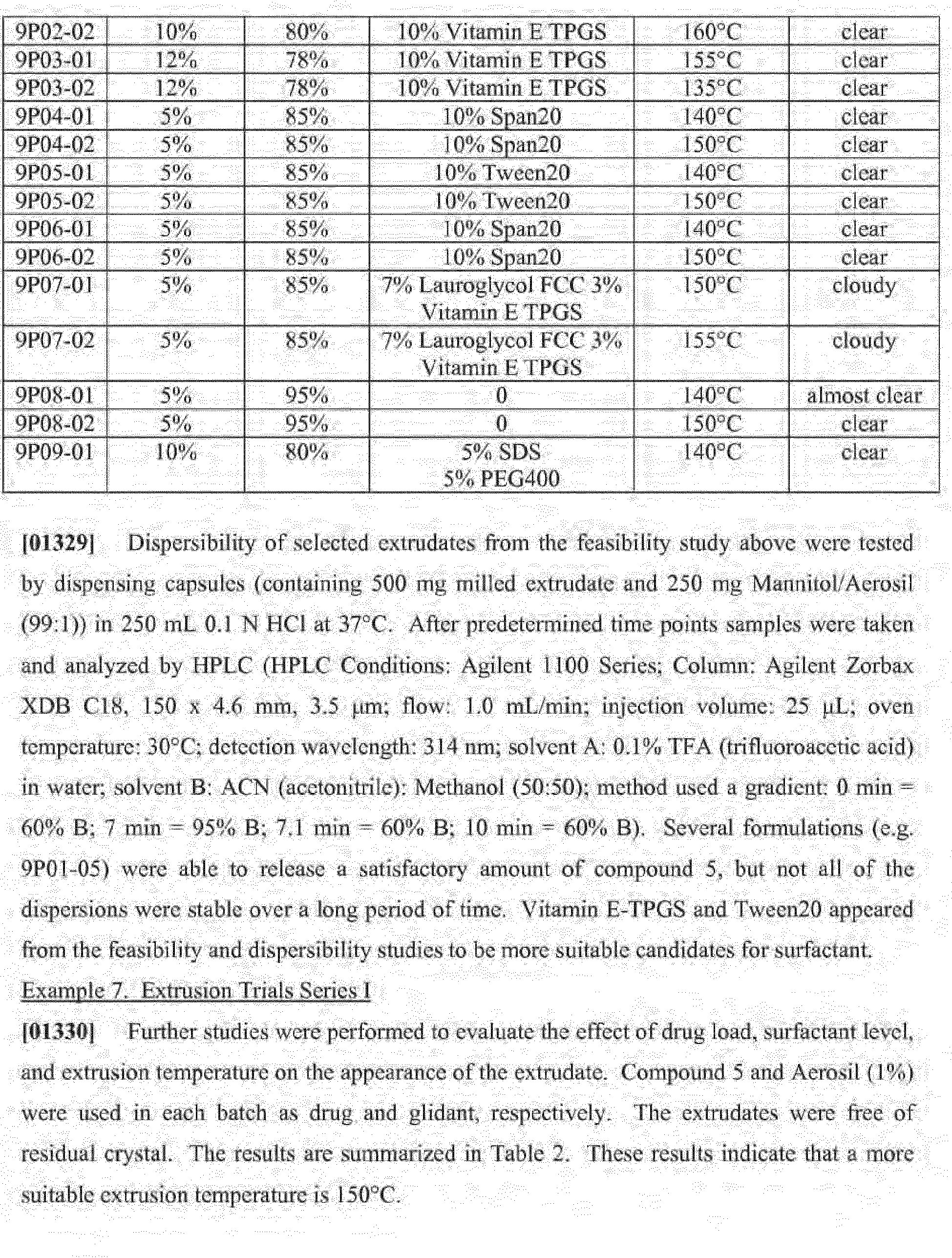

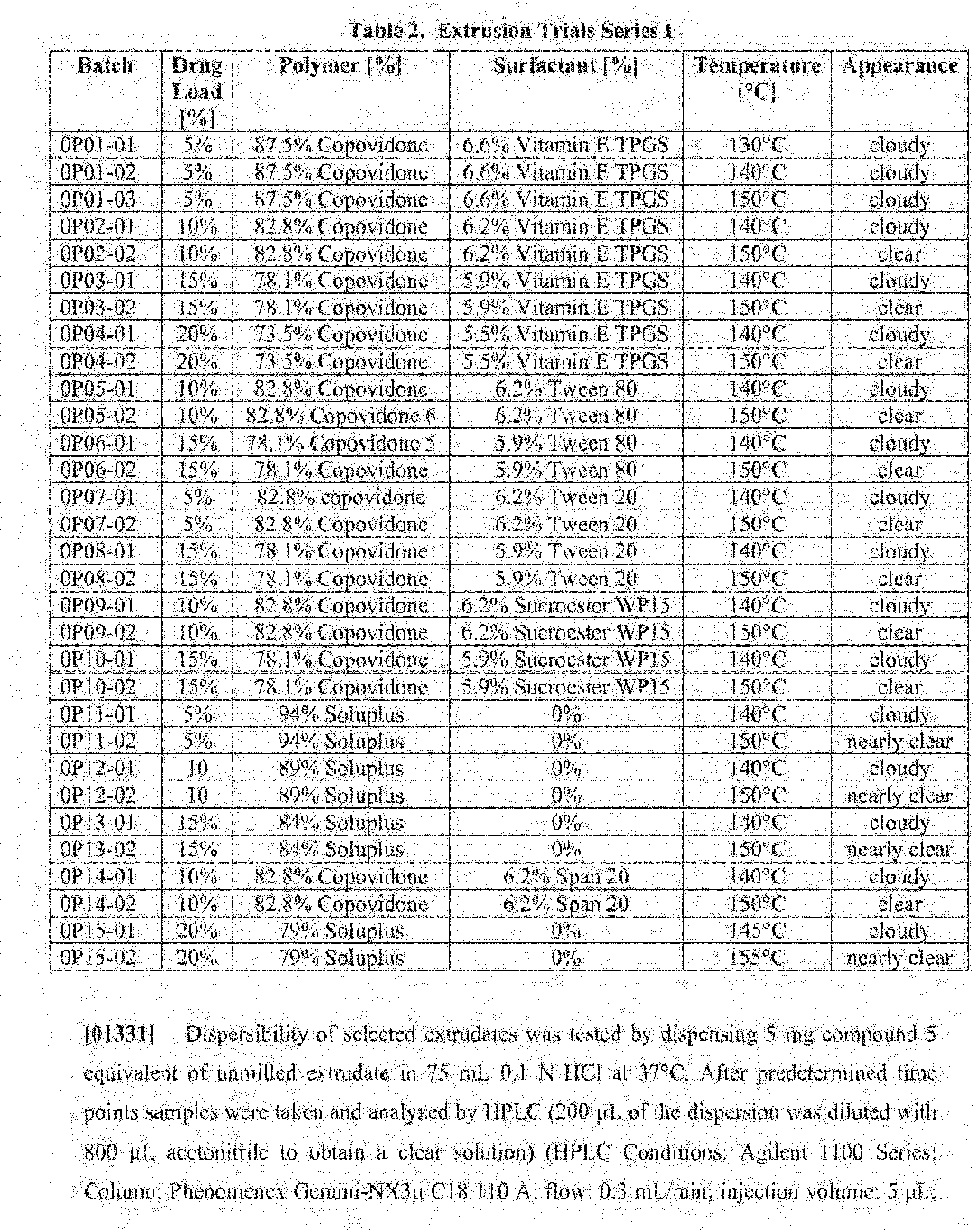

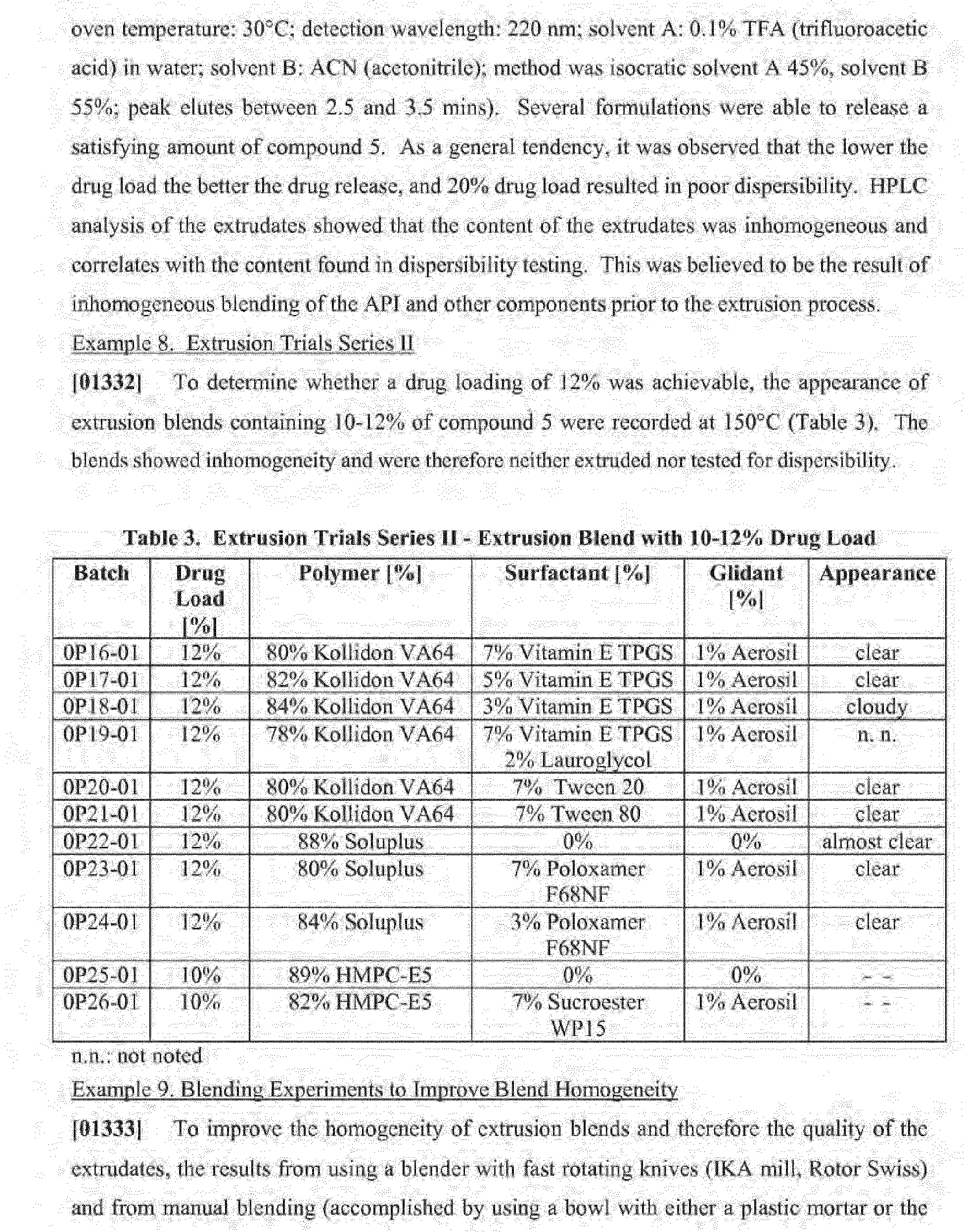

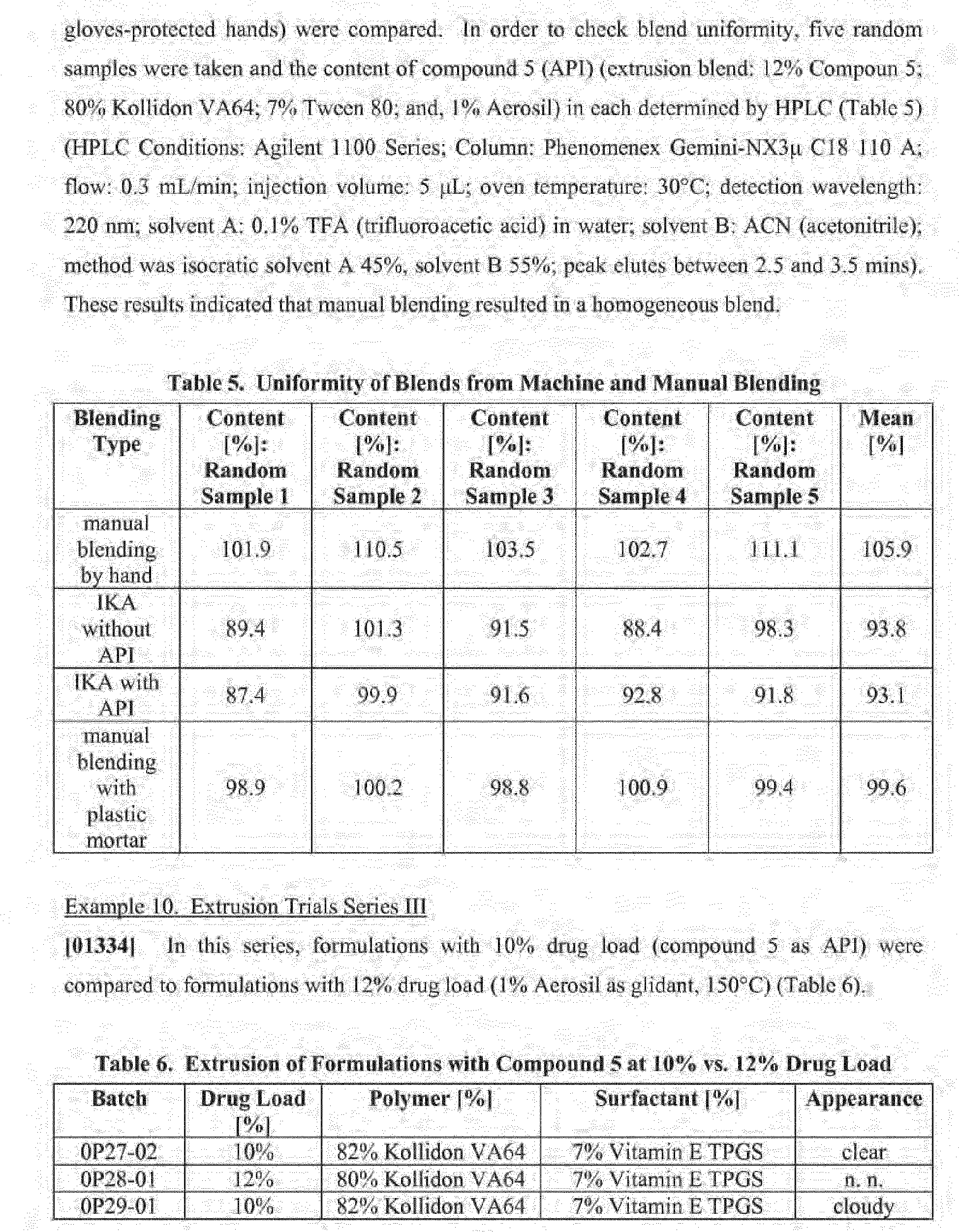

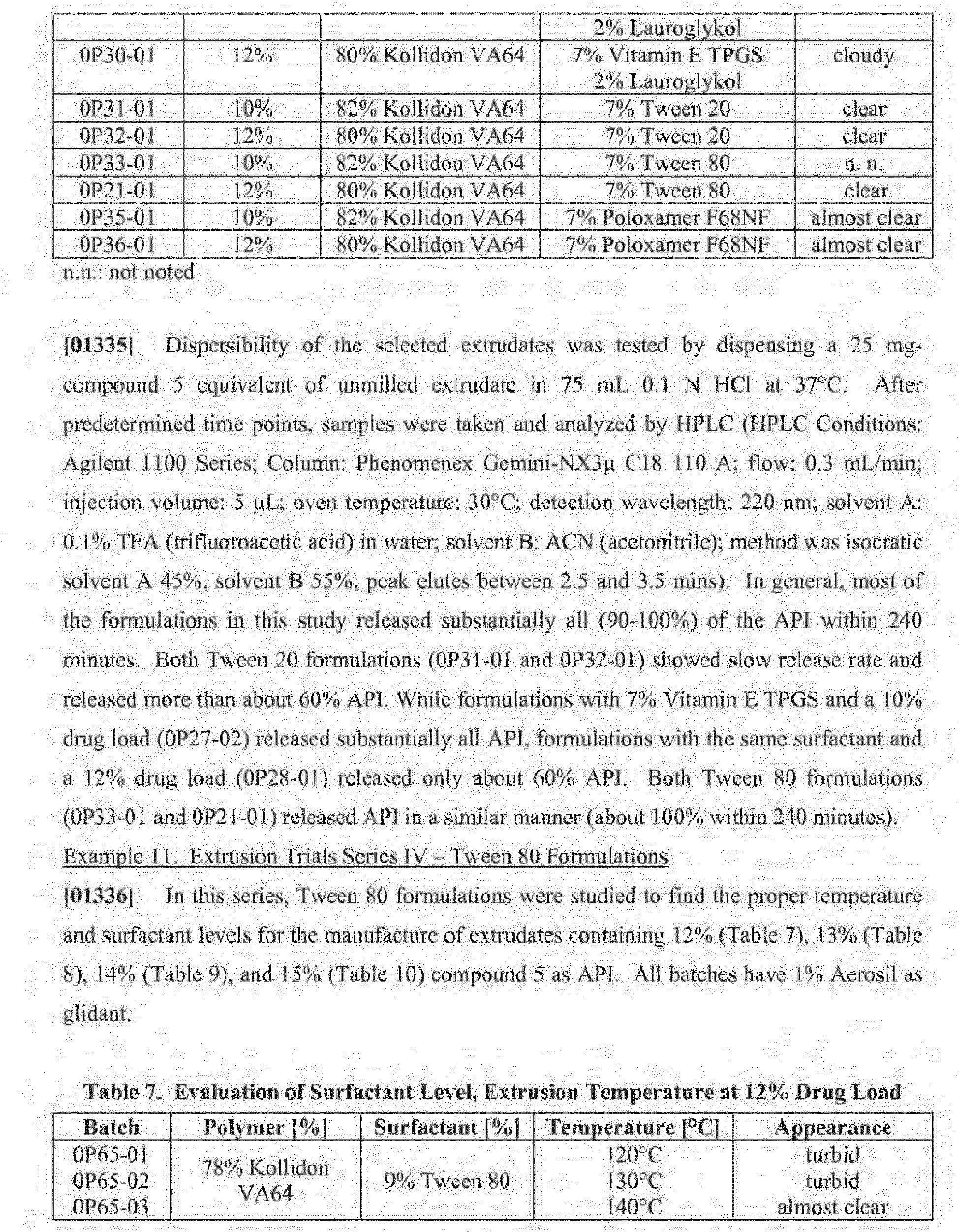

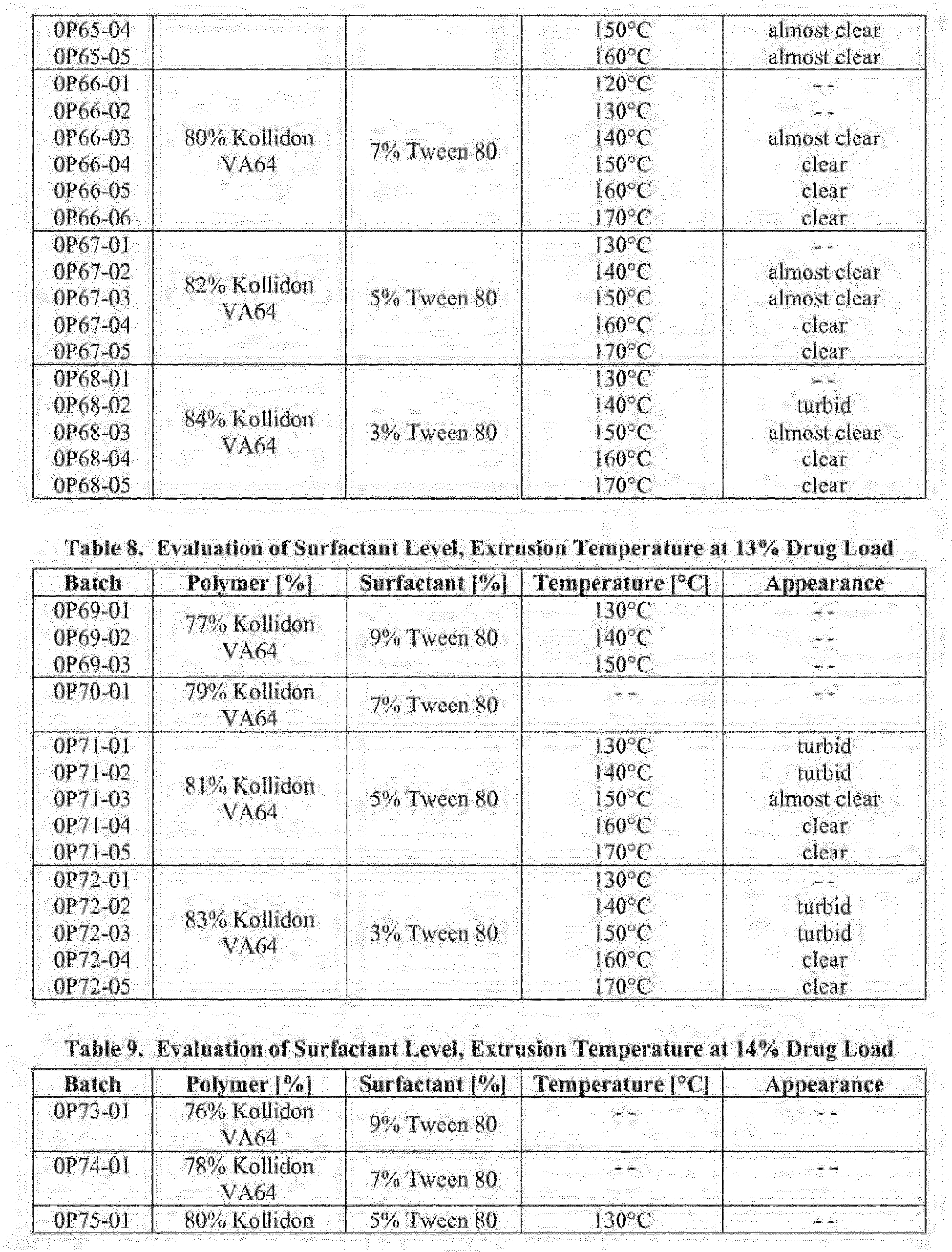

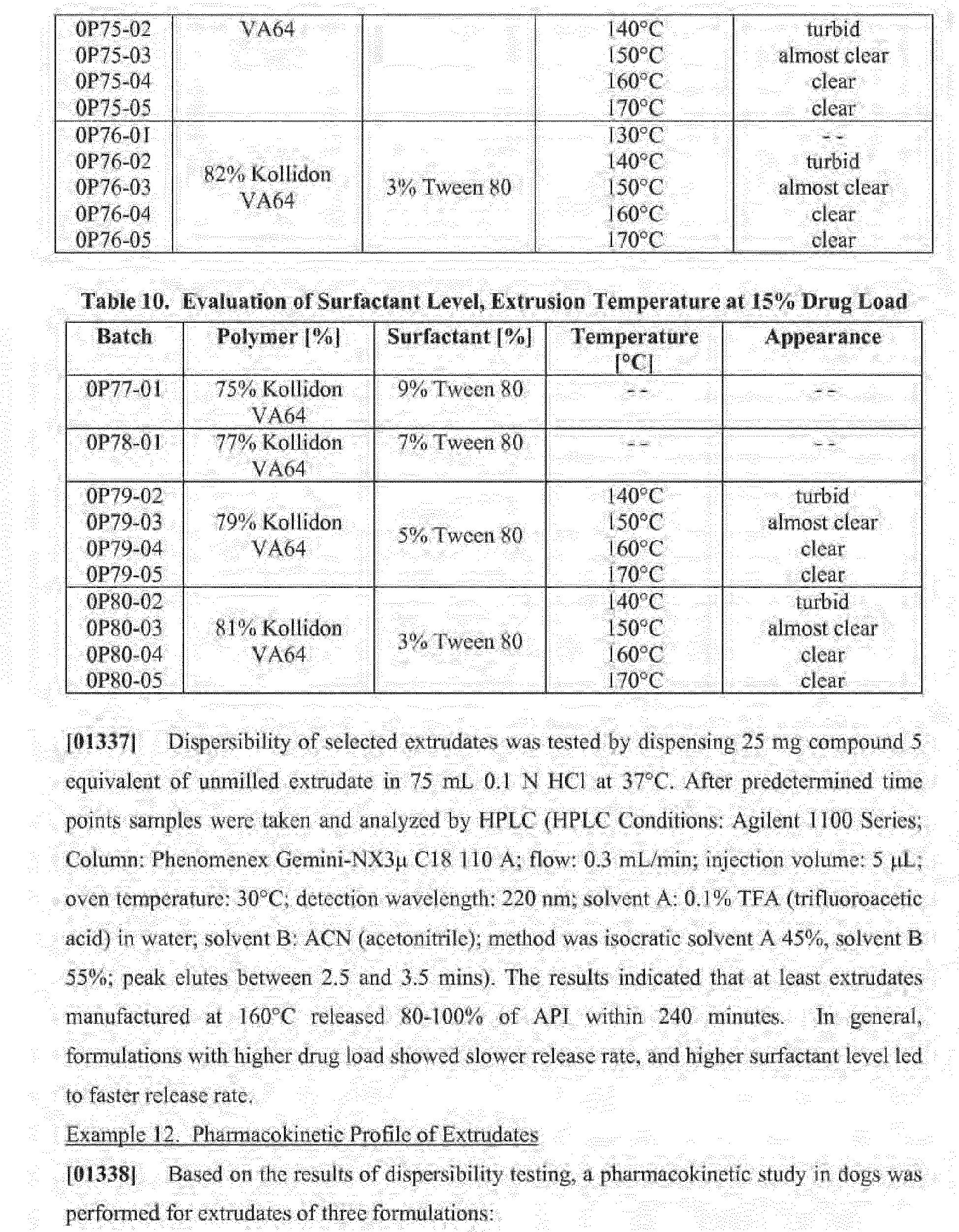

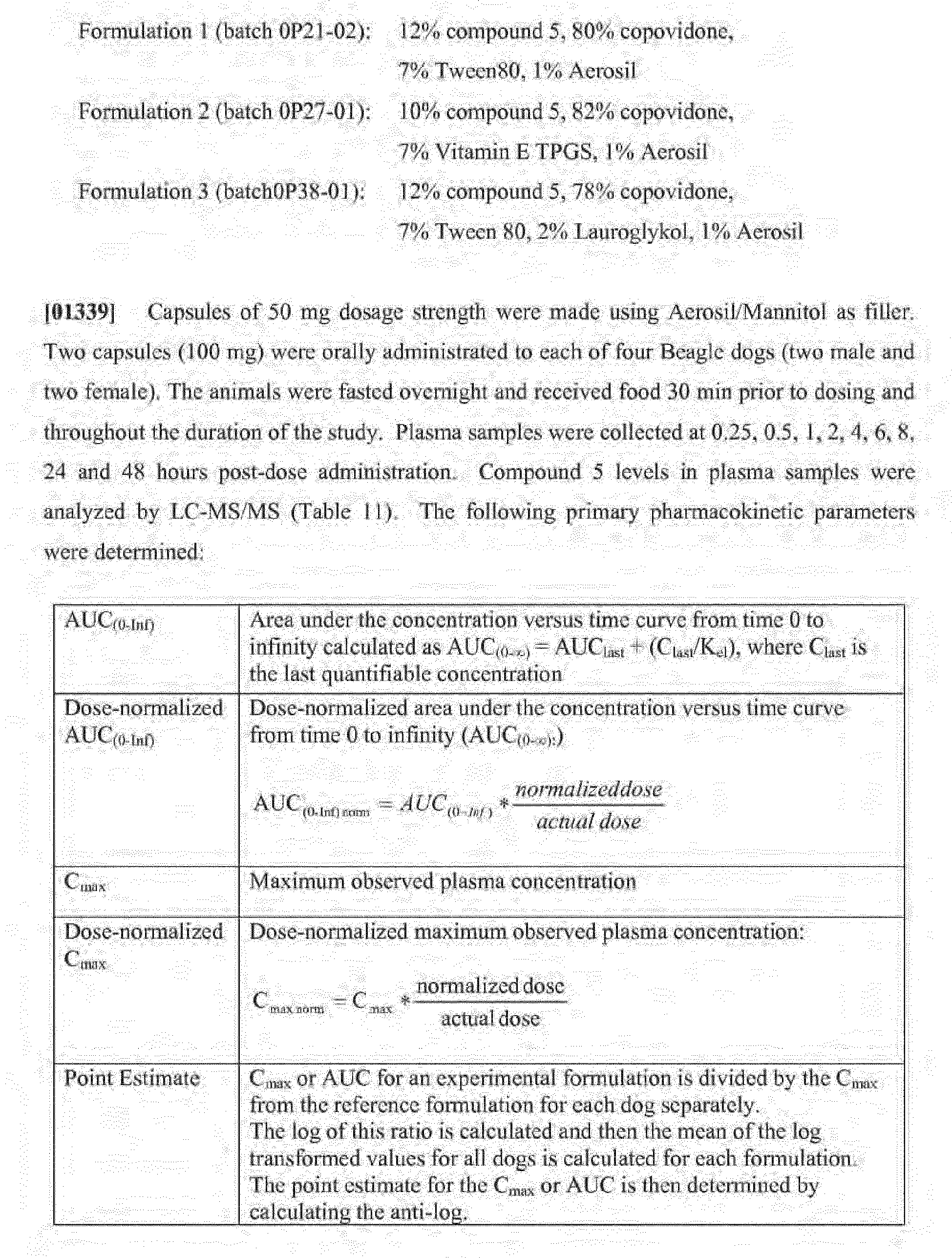

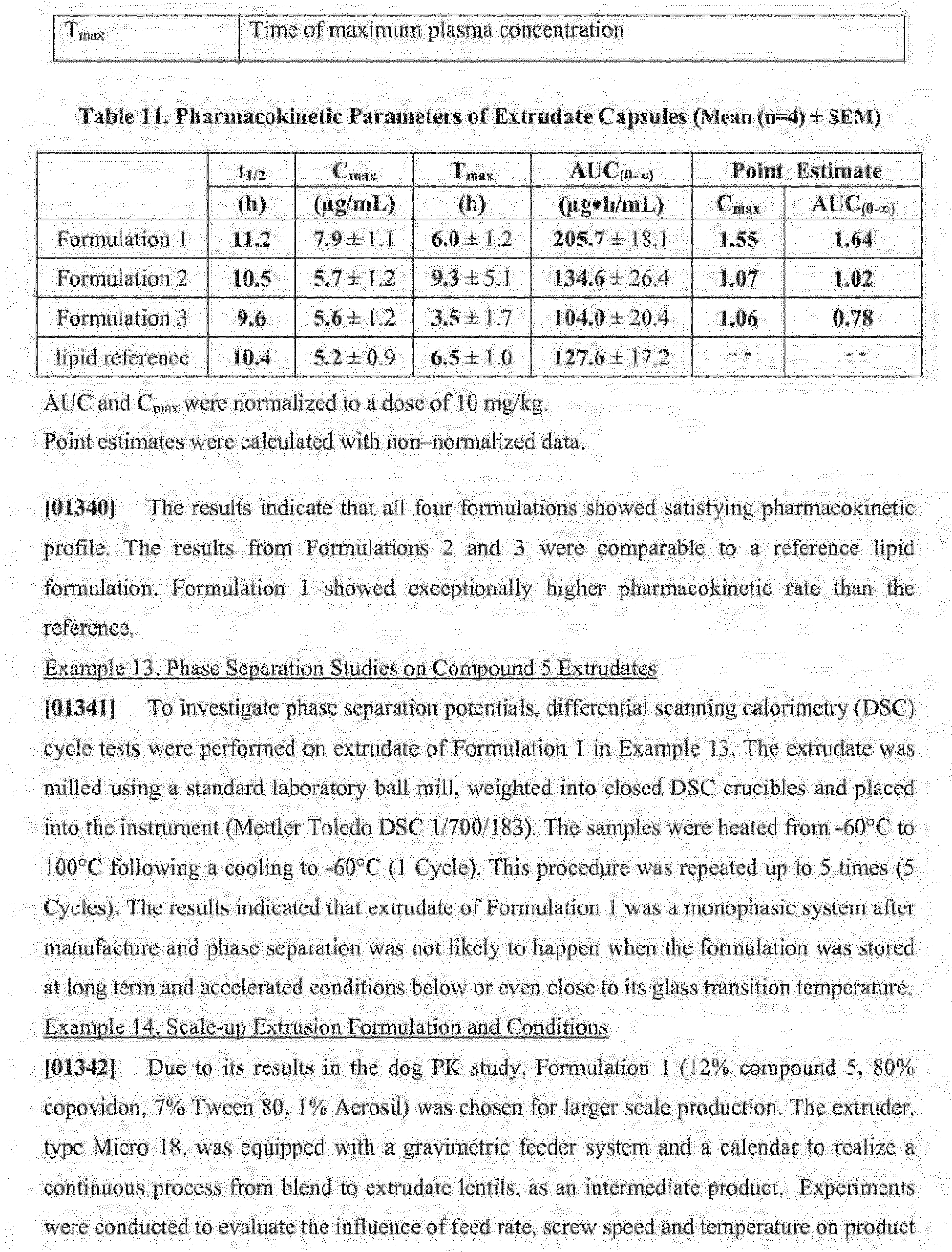

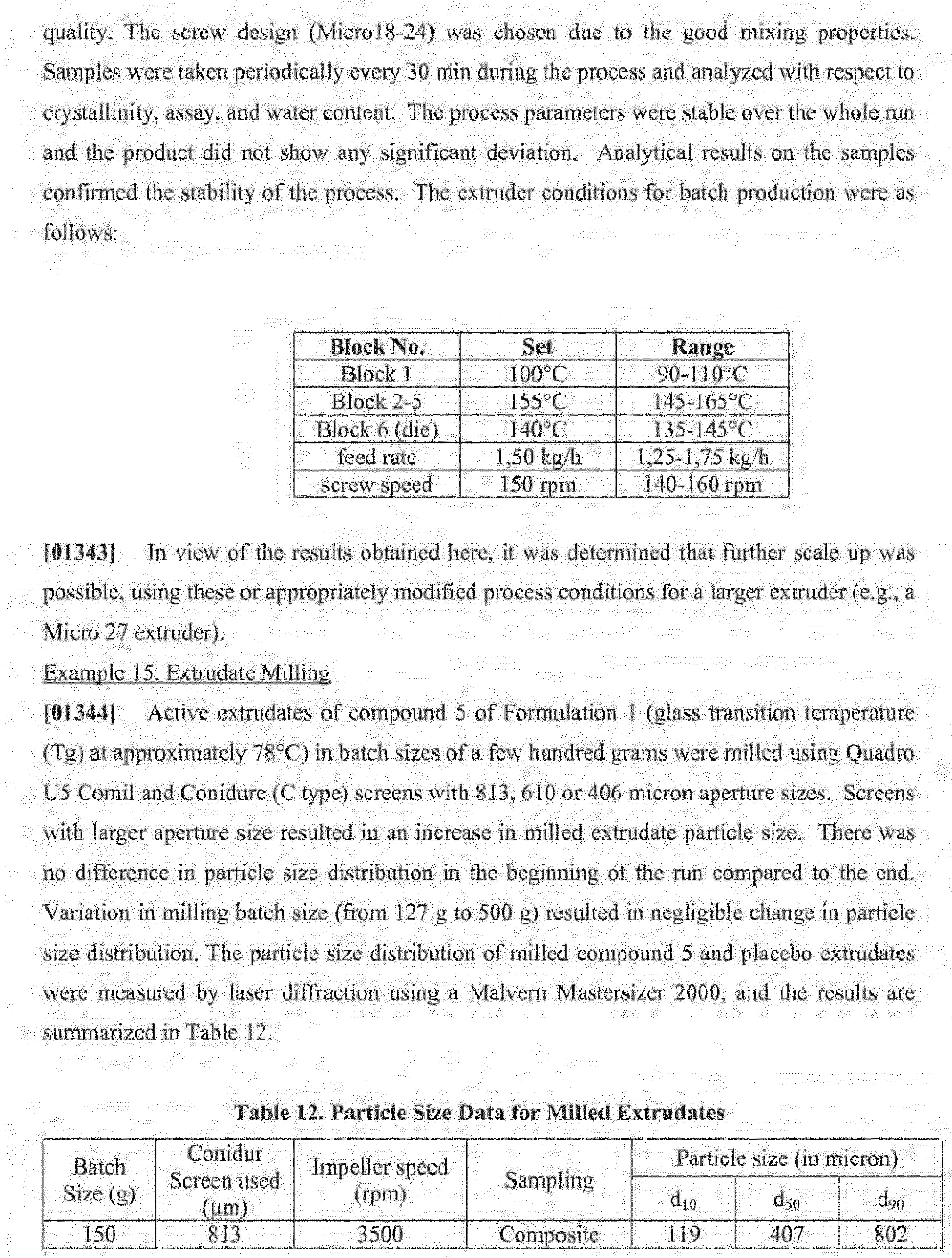

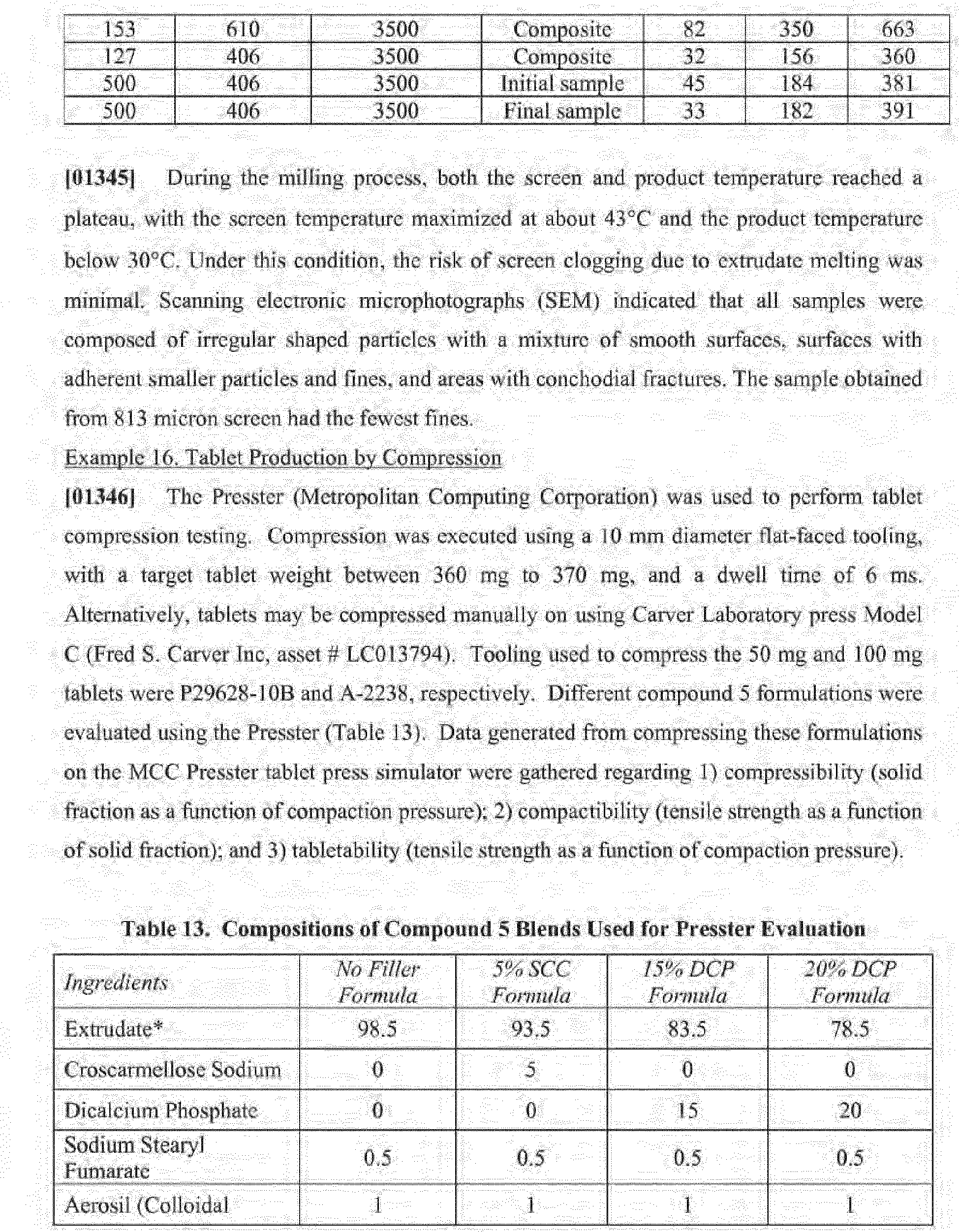

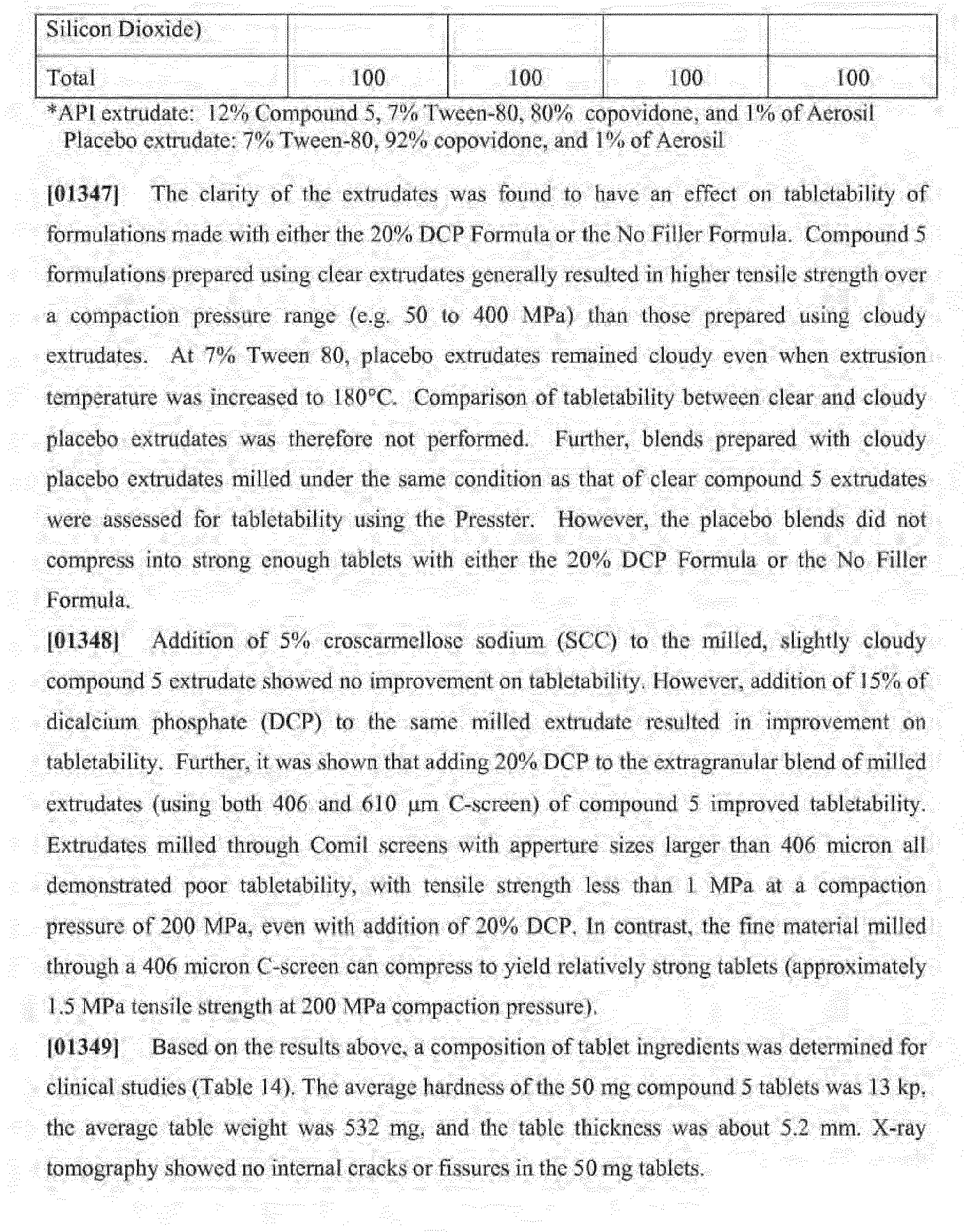

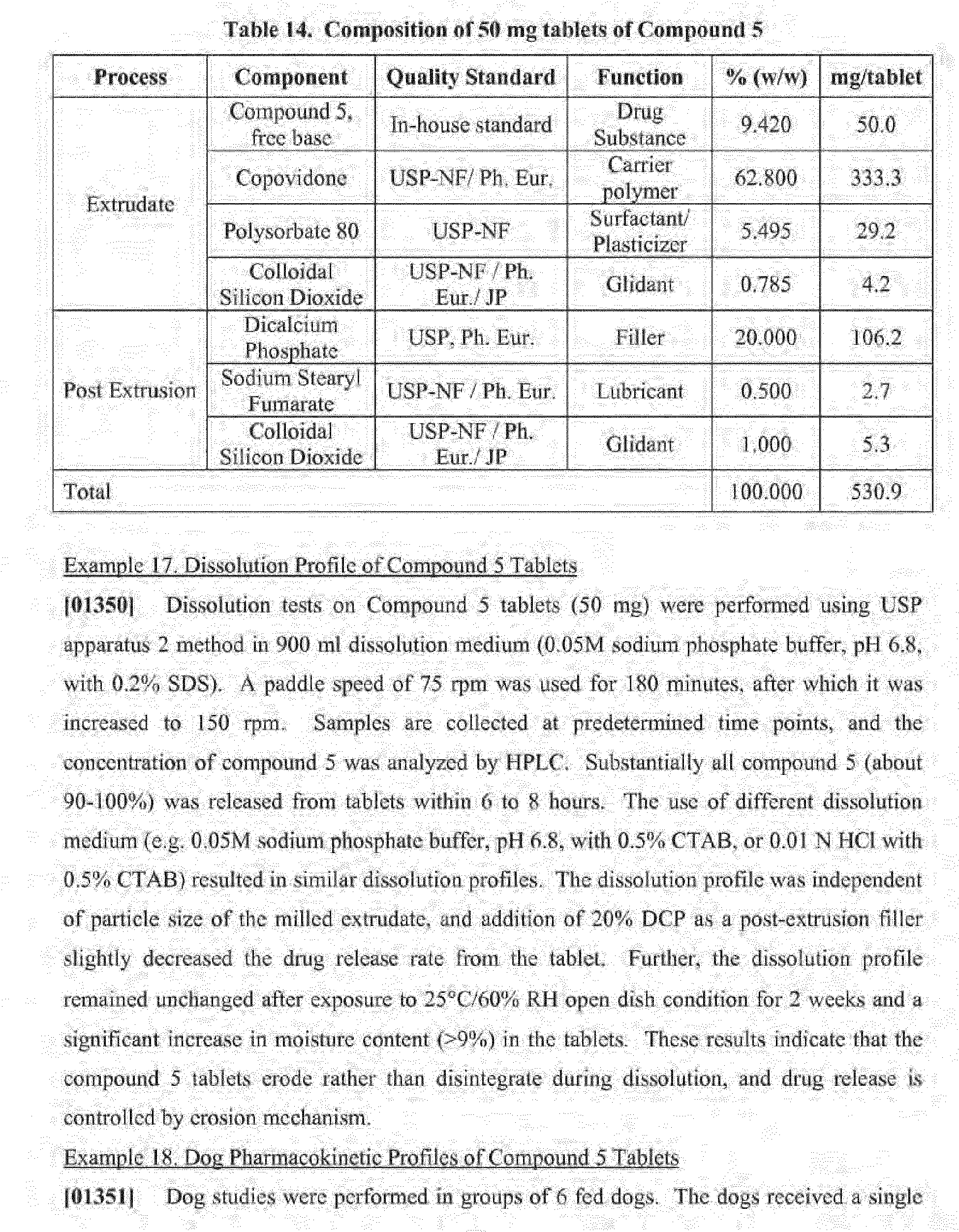

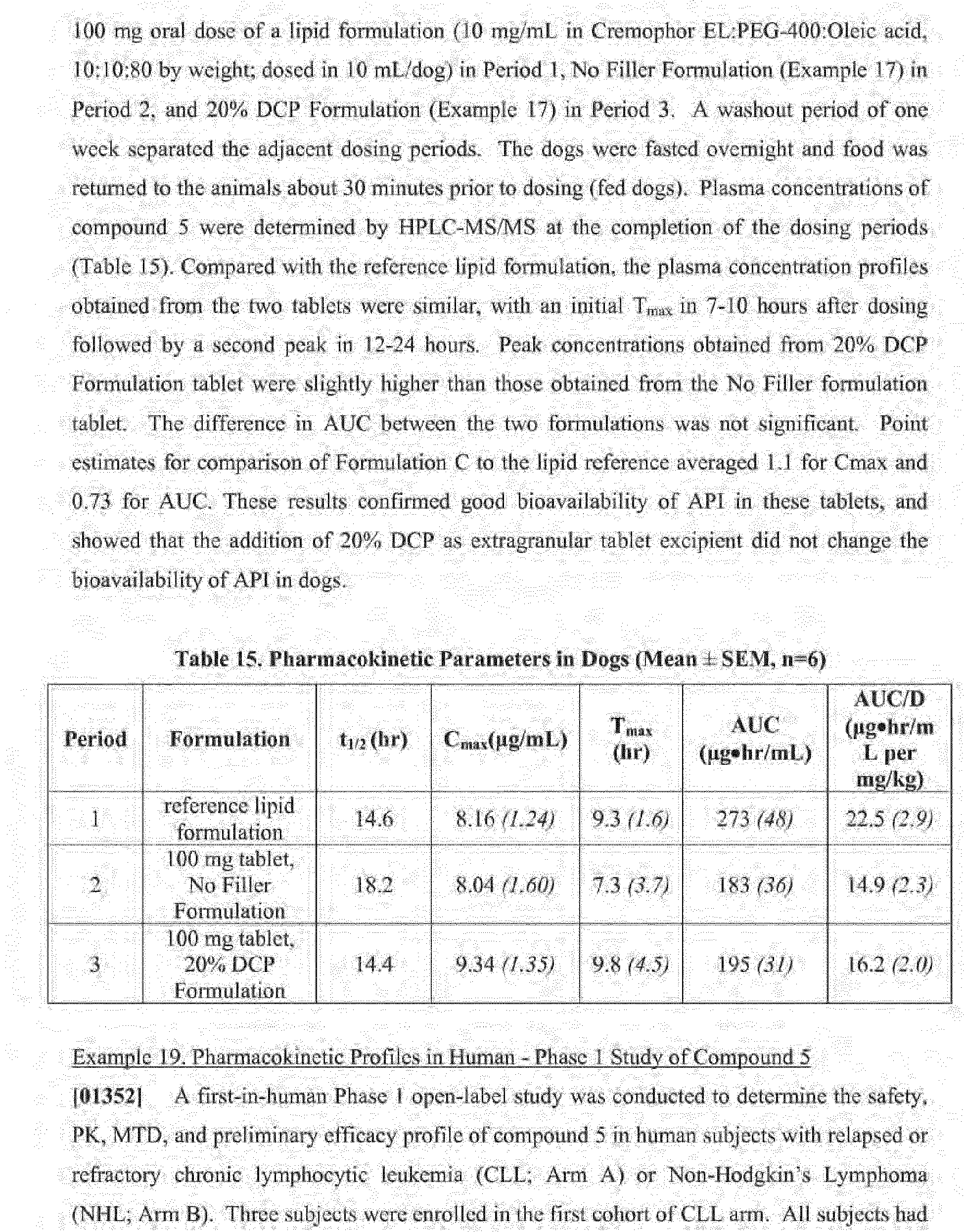

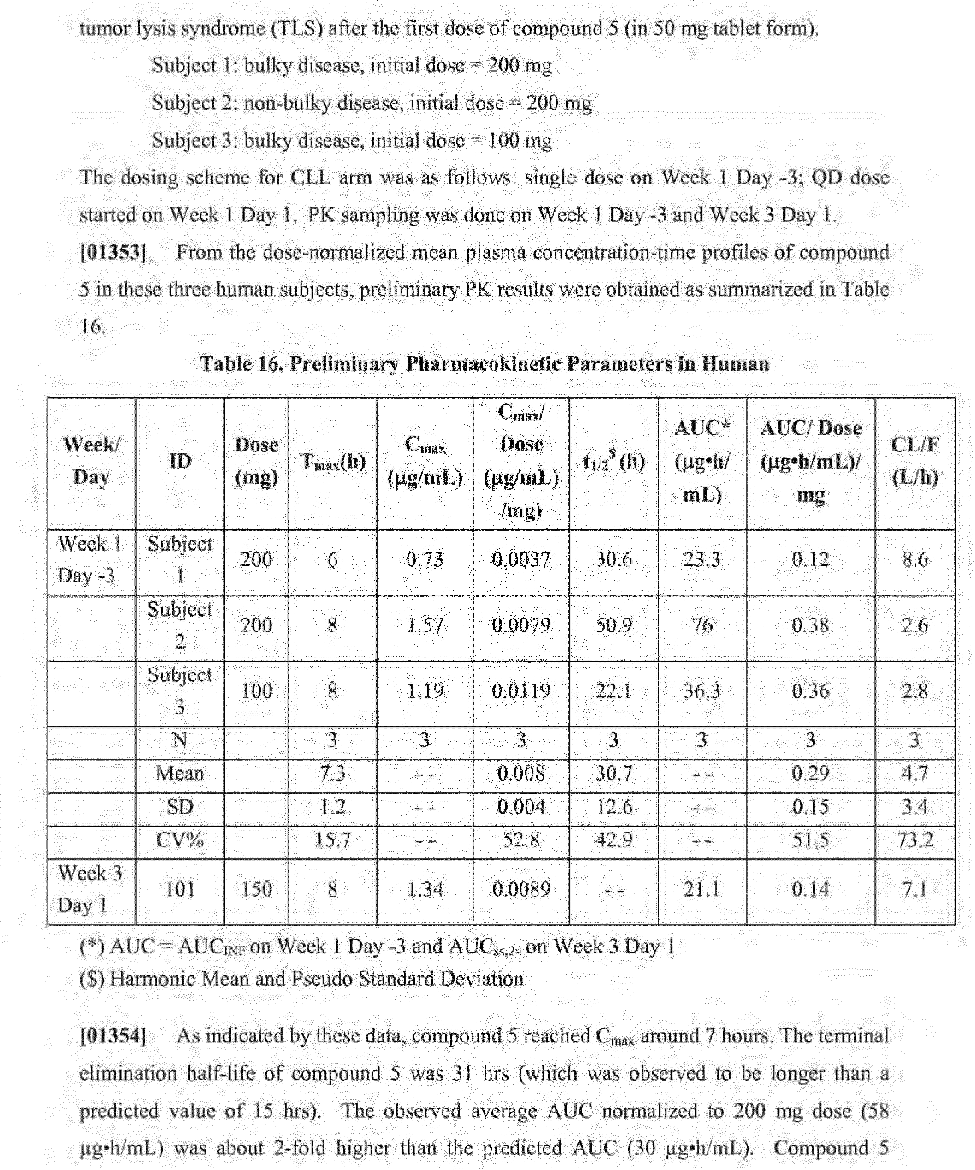

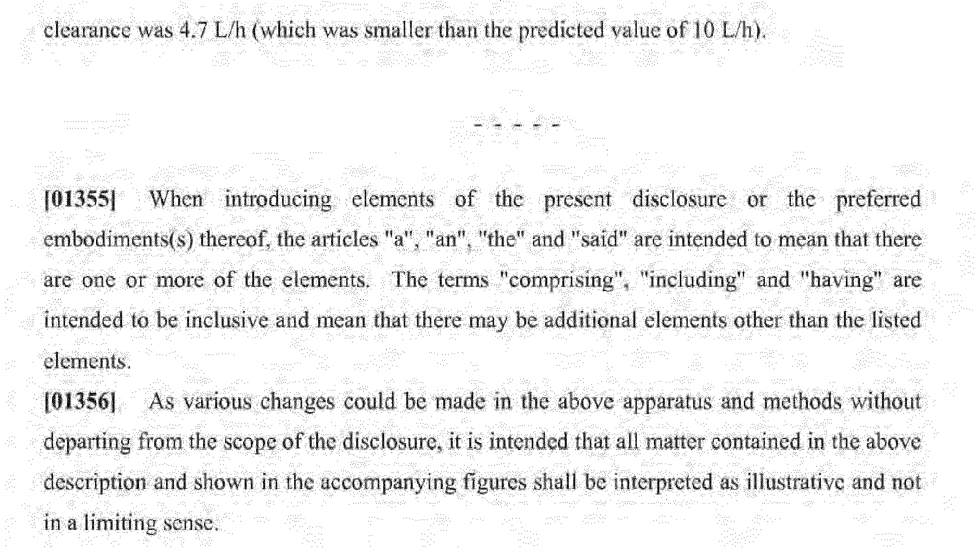

The present invention relates to the following items.
1. A solid dispersion comprising, in essentially non-crystalline form, a compound of Formula I 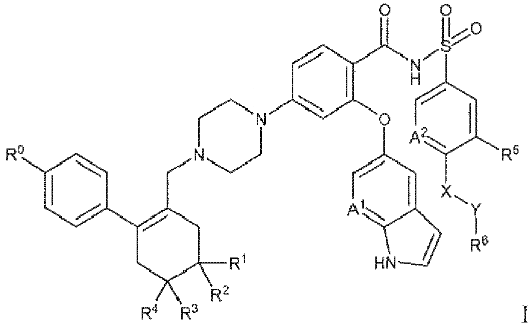 where:
   R⁰ is halo;
   R¹ and R² are H or are independently methyl or methoxy;
   R³ and R⁴ are independently methyl or methoxy if R¹ and R² are H, or are H if R¹ and R² are independently methyl or methoxy;
   A¹ and A² are each independently CH or N;
   R⁵ is C₁₋₄ alkyl or haloalkyl, C₁₋₄ alkylsulfonyl or haloalkylsulfonyl, halo, nitro or cyano;
   X is -O- or -NH-;
   Y is -(CH₂)ₙ- where n is 0, 1, 2 or 3; and
   R⁶ is an unsubstituted or substituted 3- to 7-membered carbocyclic or heterocyclic ring as defined herein, or is NR⁷R⁸;
      wherein, if R⁶ is NR⁷R⁸, R⁷ and R⁸ are each independently H or R⁹-(CH₂)ₘ-groups, no more than one of R⁷ and R⁸ being H, where each R⁹ is independently a 3- to 7-membered carbocyclic or heterocyclic ring, optionally substituted with no more than two Z¹ groups as defined below, and each m is independently 0 or 1; and
      wherein, if R⁶ is a substituted carbocyclic or heterocyclic ring, substituents thereon are no more than two Z¹ groups and/or no more than one Z² group, Z¹ groups being independently selected from (a) C₁₋₄ alkyl, C₂₋₄ alkenyl, C₁₋₄ alkoxy, C₁₋₄ alkylthio, C₁₋₄ alkylamino, C₁₋₄ alkylsulfonyl, C₁₋₄ alkylsulfonylamino, C₁₋₄ alkylcarbonyl, C₁₋₄ alkylcarbonylamino and C₁₋₄ alkylcarboxy, each optionally substituted with one or more substituents independently selected from halo, hydroxy, C₁₋₄ alkoxy, amino, C₁₋₄ alkylamino, di-(C₁₋₄ alkyl)amino and cyano, (b) halo, (e) hydroxy, (f) amino and (g) oxo groups, and Z² being (i) a further 3- to 6-membered carbocyclic or heterocyclic ring, optionally substituted with no more than two Z¹ groups as defined above, or (ii) NR⁷R⁸ where R⁷ and R⁸ are as defined above;
   or a pharmaceutically acceptable salt thereof; dispersed in a solid matrix that comprises (a) at least one pharmaceutically acceptable water-soluble polymeric carrier and (b) at least one pharmaceutically acceptable surfactant.
2. The solid dispersion of item 1, wherein, in the compound of Formula I, R⁰ is chloro.
3. The solid dispersion of item 2, wherein, in the compound of Formula I, R³ and R⁴ are each methyl.
4. The solid dispersion of item 3, wherein, in the compound of Formula I, R¹ and R² are each hydrogen.
5. The solid dispersion of item 4, wherein, in the compound of Formula I, A¹ is N and A² is CH.
6. The solid dispersion of item 5, wherein, in the compound of Formula I, R⁵ is nitro.
7. The solid dispersion of item 6, wherein, in the compound of Formula I, X is -NH-.
8. The solid dispersion of item 7, wherein, in the compound of Formula I, Y is -(CH₂)ₙ- where n is 1.
9. The solid dispersion of item 8, wherein, in the compound of Formula I, R⁶ is tetrahydropyran.
10. The solid dispersion of item 9, wherein the compound is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide.
11. The solid dispersion of item 1, wherein, in the compound of Formula I, R¹ is methyl or methoxy, R² is methyl, and R³ and R⁴ are each H.
12. The solid dispersion of item 1, wherein, in the compound of Formula I, if A² is -CH- then R⁵ is nitro; and if A² is -N- then R⁵ is bromo.
13. The solid dispersion of item 1, wherein, in the compound of Formula I, R⁶ is a 3- to 7-membered carbocyclic or heterocyclic ring, unsubstituted or substituted with no more than two Z¹ groups and/or no more than one Z² group.
14. The solid dispersion of item 13, wherein, in the compound of Formula 1, said carbocyclic or heterocyclic ring is a saturated ring.
15. The solid dispersion of item 14, wherein, in the compound of Formula I, said saturated ring is selected from the group consisting of cyclopropyl, cyclobutyl, cyclopentyl, cyclohexyl, azetidinyl, oxetanyl, thietanyl, pyrrolidinyl, imazolidinyl, pyrazolidinyl, tetrahydrofuranyl, oxazolidinyl, isoxazolidinyl, thiophanyl, thiazolidinyl, isothiazolidinyl, piperidinyl, piperazinyl, tetrahydropyranyl, 1,4-dioxanyl, morpholinyl and tetrahydrothiopyranyl rings.
16. The solid dispersion of item 1, wherein the compound is selected from the group consisting of
   4-(4-{[2-(4-chlorophenyl)-4,4-dimethylcyclohex-1-en-1-yl]methyl}piperazin-1-yl)-2-{1H-indol-5-yloxy)-N-({3-nitro-4-[(1-tetrahydro-2H-hyran-4-ylpiperidin-4-yl)amino]phenyl}sulfonyl)benzamide;
   4-(4-{[2-(4-chlorophenyl)-4,4-dimethylcyclohex-1-en-1-yl]methyl}piperazin-1-yl)-2-(1H-indol-5-yloxy)-N-({4-[(4-methylpiperazin-1-yl)amino]-3-nitrophenyl}sulfonyl)benzamide;
   4-(4-{[2-(4-chlorophenyl)-4,4-dimethylcyclohex-1-en-1-yl]methyl}piperazin-1-yl)-N-({3-mtro-4-[(tetrahydro-2H-pyran-4-ylmethyl)amino]phenyl}sulfonyl)-2-(1H-pyrrolo[2,3-b]pyridin-5-yloxy)benzamide;
   *trans-*4*-*(4*-*{[2-(4-chlorophenyl)-4,4-dimethylcyclohex-1-en-1-yl]methyl}piperazin-1-yl)-N-({4-[(4-morpholin-4-ylcyclohexyl)amino]-3-nitrophenyl}sulfonyl)-2-(1H-pyrrolo[2,3-b]pyridin-5-yloxy)benzamide;
   *cis*-4-(4-{[2-(4-chlorophenyl)-4,4-dimethylcyclohex-1-en-1-yl]methyl}piperazin-1-yl)-N-[(4-{[(4-methoxycyclohexyl)methyl]amino}-3-nitrophenyl)sulfonyl]-2-(1H-pyrrolo[2,3-b]pyridin-5-yloxy)benzamide;
   *trans-*4-(4-{[2-clorophenyl)-4,4-dimethylcyclohex-1-en-1-yl]methyl}piperazin-1-yl)-N-[(4-{[(4-methoxycyclohexyl)methyl]amino)-3-nitrophenyl)sulfonyl]-2-(1H-pyrrolo[2,3-b]pyridin-5-yloxy)benzamide;
   4-(4-{[2-(4-chlorophenyl)-4,4-dimethylcyclohex-1-en-1-yl]methyl}piperazin-1-yl)-N-({4-[(4-fluorotetrahydro-2H-pyran-4-yl)methoxy]-3-nitrophenyl}sulfonyl)-2-(1H-pyrrolo[2,3-b]pyridin-5-yloxy)benzamide;
   N-[(5-chloro-6-([4-fluoro-1-(oxetan-3-yl)piperidin-4-yl]metlioxy}pyridin-3-yl)-sulfonyl]-4-(4-{[2-(4-chlorophenyl)-4,4-dimethylcyclohex-1-en-1-yl]methyl}-piperazin-1-yl)-2-(1H-pyrrolo[2,3-b]pyridin-5-yloxy)benzamide;
   N-((5-bromo-6-[(1-tetrahydro-2H-pyran-4-ylpiperidin-4-yl)amino]pyridin-3-yl)-sulfonyl)-4-(4-{[2-(4-chlorophenyl)-4,4-dimethylcyclohex-1-en-1-yl]methyl}-piperazin-1-yl)-2-(1H-pyrrolo[2,3-b]pyridin-5-yloxy)benzamide;
   4-(4-{[2-(4-ch[orophenyl)-5-methoxy-5-methylcyclohex-1-en-1-yl]methyl}piperazin-1-yl)-N-({3-nitro-4-[(tetrahydro-2H-pyran-4-ylmethyl)amino]phenyl}-sulfonyl)-2-(1H-pyrrolo[2,3-b]pyridin-5-yloxy)benzamide;
   4-(4-{[2-(4-chlorophenyl)-4,4-dimethylcyclohex-1-en-1-yl]methyl}piperazin-1-yl)-N-[(4-{[(3R)-1-(methylsulfonyl)pyrrolidin-3-yl]amino}-3-nitrophenyl)-sulfonyl]-2-(1H-pyrrolo[2,3-b]pyridin-5-yloxy)benzamide;
   4-(4-{[2-(4-chlorophenyl)-4,4-dimethylcyclohex-1-en-1-yl]methyl)piperazin-1-yl)-N-[(4-{[(*trans*-4-hydroxy-4-methylcyclohexyl)methyl]amino}-3-nitrophenyl)-sulfonyl]-2-(1H-pyrrolo[2,3-b]pyridin-5-yloxy)benzamide;
   4-(4-{[2-(4-chlorophenyl)-4,4-dimethylcyclohex-1-en-1-yl]methyl}piperazin-1-yl)-N-[(4-{[(*cis*-4-hydroxy-4-methylcyclohexyl)methyl]amino}-3-nitrophenyl)-sulfonyl]-2-(1H-pyrrolo[2,3-b]pyridin-5-yloxy)bcnzamidc;
   4-(4-{[2-(4-chlorophenyl)-4,4-dimethylcyclohex-1-en-1-yl]methyl}piperazin-1-yl)-N-{[4-({3-[cyclopropyl(oxetan-3-yl)amino]propyl}amino)-3-nitrophenyl]-sulfonyl}-2-(1H-pyrrolo[2,3-b]pyridin-5-yloxy)benzamide;
   4-(4-{[2-(4-chlorophenyl)-4,4-dimethylcyclo-1-en-1-yl]methyl}piperazin-1-yl)-N-{[3-nitro-4-({[(3R)-1-tetrahydro-2H-pyran-4-ylpyrrolidin-3-yl]methyl} -amino)phenyl]sulfonyl}-2-{1H-pyrrolo[2,3-b]pyridin-5-yloxy)benzamide;
   4-(4-{[2-(4-chlorophenyl)-4,4-dimethylcyclohex-1-en-1-yl]methyl}piperazin-1-yl)-N-[(4-{[(4-methylmorpholin-2-yl)methyl]amino}-3-nitrophenyl)sulfonyl]-2-(1H-pyrrolo[2,3-b]pyridin-5-yloxy)benzamide;
   N-[(5-chloro-6-{[1-(cyanomethyl)piperidin-4-yl]metlaoxy}pyridin-3-yl)sulfonyl]-4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)benzamide;
   N-[{4-{[{4-aminotetrahydro-2H-pyran-4-yl)methyl]amino}-3-nitrophenyl}sulfonyl]-4-(4-{[2-(4-chlorophenyl)-4,4-dimethylcyclohex-1-en-1-yl]methyl)piperazin-1-yl)-2-(1H-pyrrolo[2,3-b]pyridin-5-yloxy)benzamide;
   4-(4-{[2-(4-chlorophenyl)-4,4-dimethylcyclohex-1-en-1-yl]methyl}piperazin-1-yl)-N-({4-[(4-methoxytetrahydro-2H-pyran-4-yl)methoxy]-3-nitrophenyl}sulfonyl)-2-{1H-pyrrolo[2,3-b]pyridin-5-yloxy)benzamide;
   and pharmaceutically acceptable salts thereof.
17. The solid dispersion of item 1, wherein the compound or salt is present in a parent-compound-equivalent amount of about 5% to about 40% by weight.
18. The solid dispersion of item 1, wherein the at least one polymeric carrier is present in an amount of about 40% to about 85% by weight and the at least one surfactant is present in an amount of about 5% to about 20% by weight.
19. The solid dispersion of item 1, wherein the at least one polymeric carrier is selected from the group consisting of homopolymers and copolymers of N-vinyl lactams, cellulose esters, cellulose ethers, high molecular weight polyalkylene oxides, polyacrylates, polymethacrylates, polyacrylamides, vinyl acetate polymers, graft copolymers of polyethylene glycol, polyvinyl caprolactam and polyvinyl acetate, oligo- and polysaccharides and mixtures thereof.
20. The solid dispersion of item 1, wherein the at least one polymeric carrier is selected from the group consisting of povidones, copovidones, HPMCs, polyethylene glycol/polyvinyl caprolactam/polyvinyl acetate graft copolymers and mixtures thereof.
21. The solid dispersion of item 1, wherein the at least one surfactant is non-ionic.
22. The solid dispersion of item 1, wherein the at least one surfactant is selected from the group consisting of polyoxyethylene glycerides, fatty acid monoesters of sorbitan, polysorbates, α-tocopheryl polyethylene glycol succinate (TPGS) and mixtures thereof.
23. The solid dispersion of item 1, further comprising at least one glidant.
24. The solid dispersion of item 23, wherein the at least one glidant comprises colloidal silicon dioxide.
25. The solid dispersion of item 1, wherein the compound or salt is present in a parent-compound-equivalent amount of about 5% to about 40% by weight, the at least one polymeric carrier is present in an amount of about 40% to about 85% by weight and the at least one surfactant is present in an amount of about 5% to about 20% by weight.
26. The solid dispersion of item 25, wherein the compound or salt is present in a parent-compound-equivalent amount of about 5% to about 15% by weight, the at least one polymeric carrier is present in an amount of about 70% to about 85% by weight and the at least one surfactant is present in an amount of about 5% to about 15% by weight.
27. The solid dispersion of item 26, wherein the compound is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide.
28. The solid dispersion of item 27, wherein the at least one polymeric carrier is a copovidone.
29. The solid dispersion of item 28, wherein the at least one surfactant is a polysorbate.
30. The solid dispersion of item 30, further comprising at least one glidant.
31. The solid dispersion of item 30, wherein the at least one glidant comprises colloidal silicon dioxide.
32. A process for preparing a solid dispersion, comprising:
   (a) subjecting to elevated temperature (i) an active pharmaceutical ingredient (API)
      that comprises a compound of Formula I 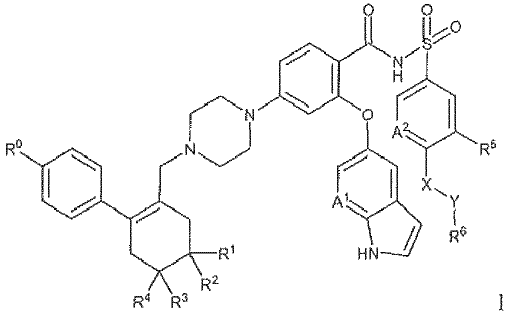 where:
      R⁰ is halo;
      R³ and R² are H or arc independently methyl or methoxy;
      R³ and R⁴ arc independently methyl or methoxy if R¹ and R² arc H, or are H if R¹ and R² arc independetttly methyl or methoxy;
      A¹ and A² are each independently CH or N;
      R⁵ is C₁₋₄ alkyl or haloalkyl, C₁₋₄ alkylsulfonyl or haloalkylsulfonyl, halo, nitro or cyano;
      X is -O- or -NH-;
      Y is -(CH₂)ₙ- where n is 0, 1, 2 or 3; and
      R⁶ is an unsubstituted or substituted 3- to 7-membered carbocyclic or heterocyclic ring as defined herein, or is NR⁷R⁸;
      wherein, if R⁶ is NR⁷R⁸, R⁷ and R⁸ arc each independently H or R⁹-(CH₂)ₘ-groups, no more than one of R⁷ and R⁸ being H, where each R⁹ is independently a 3- to 7-membered carbocyclic or heterocyclic ring, optionally substituted with no more than two Z¹ groups as defined below, and each m is independently 0 or 1; and
      wherein, if R⁶ is a substituted carbocyclic or heterocyclic ring, substituents thereon are no more than two Z¹ groups and/or no more than one Z² group, Z¹ groups being independently selected from (a) C₁₋₄ alkyl, C₂₋₄ alkenyl, C₁₋₄ alkoxy, C₁₋₄ alkylthio, C₁₋₄ alkylamino, C₁₋₄ alkylsulfonyl, C₁₋₄ alkylsulfonylamino, C₁₋₄ alkylcarbonyl, C₁₋₄ alkylcarbonylamino and C₁₋₄ alkylcarboxy, each optionally substituted with one or more substituents independently selected from halo, hydroxy, C₁₋₄ alkoxy, amino, C₁₋₄ alkylamino, di-(C₁₋₄ alkyl)amino and cyano, (b) halo, (e) hydroxy, (f) amino and (g) oxo groups, and Z² being (i) a further 3- to 6-membered carbocyclic or heterocyclic ring, optionally substituted with no more than two Z¹ groups as defined above, or (ii) NR⁷R⁸ where R⁷ and R⁸ are as defined above;
      or a pharmaceutically acceptable salt thereof, (ii) a pharmaceutically acceptable water-soluble polymeric carrier and (iii) a pharmaceutically acceptable surfactant to provide an extrudable semi-solid mixture;
   (b) extruding the semi-solid mixture; and
   (c) cooling the resulting extrudate to provide a solid matrix comprising the polymeric carrier and the surfactant and having the compound or salt thereof dispersed in an essentially non-crystalline form therein.
33. The process of item 32, wherein the API, polymeric carrier and surfactant arc mixed together before said subjecting to elevated temperature.
34. The process of item 32, wherein the API, polymeric carrier and surfactant are mixed together while subjecting to elevated temperature.
35. The process of item 32, wherein said elevated temperature is about 70° C. to about 250° C.
36. The process of item 32, wherein said elevated temperature is about 90° C. to about 160° C.
37. The process of item 32, further comprising calendering the extrudate before or while cooling.
38. The process of item 32, wherein API is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide.
39. The process of item 38, wherein the polymeric carrier is a copovidone.
40. The process of item 39, wherein the surfactant is a polysorbate.
41. A process for preparing a solid dispersion, comprising:
   (a) subjecting to elevated temperature (i) an active pharmaceutical ingredient (API) that comprises a compound of Formula I 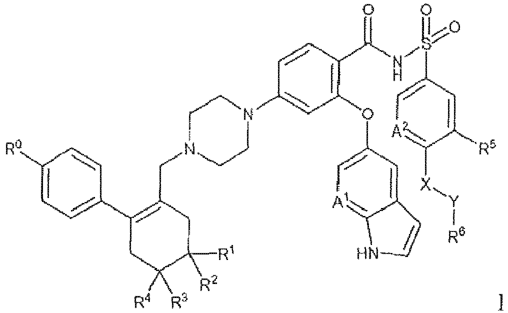 where:
      R⁰ is halo;
      R¹ and R² are H or are independently methyl or methoxy;
      R³ and R⁴ are independently methyl or methoxy if R¹ and R² are H, or are H if R¹ and R² are independently methyl or methoxy;
      A¹ and A² are each independently CH or N;
      R⁵ is C₁₋₄ alkyl or haloalkyl, C₁₋₄ alkylsulfonyl or haloalkylsulfonyl, halo, nitro or cyano;
      X is -O- or -NH-;
      Y is -(CH₂)ₙ- where n is 0, 1, 2 or 3; and
      R⁶ is an unsitbstituted or substituted 3- to 7-membered carbocyclic or heterocyclic ring as defined herein, or is NR⁷R⁸;
      wherein, if R⁶ is NR⁷R⁸, R⁷ and R⁸ are each independently H or R⁹-(CH₂)ₘ-groups, no more than one of R⁷ and R⁸ being H, where each R⁹ is independently a 3- to 7-membered carbocyclic or heterocyclic ring, optionally substituted with no more than two Z¹ groups as defined below, and each m is independently 0 or 1; and
      wherein, if R⁶ is a substituted carbocyclic or heterocyclic ring, substituents thereon are no more than two Z¹ groups and/or no more than one Z² group, Z¹ groups being independently selected from (a) C₁₋₄ alkyl, C₂₋₄ alkenyl, C₁₋₄ alkoxy, C₁₋₄ alkylthio, C₁₋₄ alkylamino, C₁₋₄ alkylsulfollyl, C₁₋₄ alkylsulfonylamino, C₁₋₄ alkylcarbonyl, C₁₋₄ alkylcarbonylamino and C₁₋₄ alkylcarboxy, each optionally substituted with one or more substituents independently selected from halo, hydroxy, C₁₋₄ alkoxy, amino, C₁₋₄ alkylamino, di-(C₁₋₄ alkyl)amino and cyano, (b) halo, (e) hydroxy, (f) amino and (g) oxo groups, and Z² being (i) a further 3- to 6-membered carbocyclic or heterocyclic ring, optionally substituted with no more than two Z¹ groups as defined above, or (ii) NR⁷R⁸ where R⁷ and R⁸ are as defined above;
      or a pharmaceutically acceptable salt thereof, (ii) a pharmaceutically acceptable water-soluble polymeric carrier and (iii) a pharmaceutically acceptable surfactant to provide an extrudable semi-solid mixtures;
   (b) extruding and calendering the semi-solid mixture; and
   (c) cooling the resulting extrudate to provide a solid matrix comprising the polymeric carrier and the surfactant and having the compound or salt thereof dispersed in an essentially non-crystalline form therein.
42. The process of item 41, wherein API is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide.
43. The process of item 42, wherein the polymeric carrier is a copovidone.
44. The process of item 43, wherein the surfactant is a polysorbate.
45. An orally deliverable pharmaceutical dosage form comprising the solid dispersion of item 1.
46. A method for treating a neoplastic, immune or autoimmune disease, comprising orally administering to a subject having the disease a therapeutically effective amount of the solid dispersion of item 1.
47. The method of item 46, wherein the disease is a neoplastic disease.
48. The method of item 47, wherein the neoplastic disease is selected from the group consisting of cancer, mesothelioma, bladder cancer, pancreatic cancer, skin cancer, cancer of the head or neck, cutaneous or intraocular melanoma, ovarian cancer, breast cancer, uterine cancer, carcinoma of the fallopian tubes, carcinoma of the endometrium, carcinoma of the cervix, carcinoma of the vagina, carcinoma of the vulva, bone cancer, colon cancer, rectal cancer, cancer of the anal region, stomach cancer, gastrointestinal (gastric, colorectal and/or duodenal) cancer, chronic lymphocytic leukemia, acute lymphocytic leukemia, esophageal cancer, cancer of the small intestine, cancer of the endocrine system, cancer of the thyroid gland, cancer of the parathyroid gland, cancer of the adrenal gland, sarcoma of soft tissue, cancer of the urethra, cancer of the penis, testicular cancer, hepatocellular (hepatic and/or biliary duct) cancer, primary or secondary central nervous system tumor, primary or secondary brain tumor, Hodgkin's disease, chronic or acute leukemia, chronic myeloid leukemia, lymphocytic lymphoma, lymphoblastic leukemia, follicular lymphoma, lymphoid malignancies of T-cell or B-cell origin, melanoma, multiple myeloma, oral cancer, non-small-cell lung cancer, prostate cancer, small-cell lung cancer, cancer of the kidney and/or ureter, renal cell carcinoma, carcinoma of the renal pelvis, neoplasms of the central nervous system, primary central nervous system lymphoma, non-Hodgkin's lymphoma, spinal axis tumors, brain stem glioma, pituitary adenoma, adrenocortical cancer, gall bladder cancer, cancer of the spleen, cholangiocarinoma, fibrosarcoma, neuroblastoma, retinoblastoma and combinations thereof.
49. The method of item 47, wherein the neoplastic disease is a lymphoid malignancy.
50. The method of item 49, wherein the lymphoid malignancy is non-Hodgkin's lymphoma.
51. The method of item 47, wherein the neoplastic disease is chronic lymphocytic leukemia or acute lymphocytic leukemia.
52. The method of item 46, wherein the disease is an immune or autoimmune disease.
53. The method of item 46, wherein the solid dispersion is administered in a parent-compound-equivalent dose of about 50 to about 500 mg per day of the compound of Formula I or salt thereof at an average treatment interval of about 3 hours to about 7 days.
54. The method of item 46, wherein the solid dispersion is administered once daily in a parent-compound-equvalent dose of about 50 to about 500 mg per day of the compound of Formula I or salt thereof.
55. The method of item 46, wherein the compound is selected from the group consisting of
   4-(4-{[2-(4-chlorophenyl)-4,4-dimethylcyclohex-1-en-yl]methyl}piperazin-1-yl)-2-(1H-indol-5-yloxy)-N-({3-nitro-4-[(1-tetrahydro-2H-pyran-4-ylpiperidin-4-yl)amino]phenyl}sulfonyl)benzamide;
   4-(4-{[2-(4-chlorophenyl)-4,4-dimethylcyclohex-1-en-1-yl]methyl}piperazin-1-yl)-2-(1H-indol-5-yloxy)-N-({4-[(4-methylpiperazin-1-yl)amino]-3-nitrophenyl}sulfonyl)benzamide;
   4-(4-{[2-(4-chlorophenyl)-4,4-dimethylcyclohex-1-en-1-yl]methyl}piperazin-1-yl)-N-{3-nitro-4-[(tetrahydro-2H-pyran-4-ylmethyl)amino]phenyl}sulfonyl)-2-(1H-pyrrolo[2,3-b]pyridin-5-yloxy)benzamide;
   *trans-*4-(4-{[2-(4-chlorophenyl)-4,4-dimethylcyclohex-1-en-1-yl]methyl}piperazin-1-yl)-N-({4-[(4-morpholin-4-ylcycohexyl)amino]-3-nitrophenyl}sulfonyl)-2-(1H-pyrrolo[2,3-b]pyridin-5-yloxy)benzamide;
   *cis*-4-(4-{[2-(4-chlorophenyl)-4,4-dimethylcyclohex-1-en-1-yl]methyl} piperazin-1-yl)-N-[(4-{[(4-methoxycyclohexyl)methyl]amino}-3-nitrophenyl)sulfonyl]-2-(1H-pyrrolo[2,3-b]pyridin-5-yloxy)benzamide;
   *trans*-4-(4-{[2-(4-chlorophenyl)-4,4-dimethylcychohex-1-en-1-yl]methyl}piperazin-1-yl)-N-[(4-{[{4-methoxycyclohexyl}methyl]amino}-3-nitrophenyl}sulfonyl]-2-(1H-pyrrolo[2,3-b]pyridin-5-yloxy)benzamide,
   4-(4-([2-(4-chlorophenyl)-4,4-dimethylcyclohex-1-en-1-yl]methyl)piperazin-1-yl)-N-((4-[(4-fluorotetrahydro-2H-pyran-4-yl)methoxy]-3-nitrophenyl}sulfonyl)-2-(1H-pyrrolo[2,3-b]pyridin-5-yloxy)benzamide;
   N-[(5-chloro-6-{[4-fluoro-1-(oxetan-3-yl)piperidin-4-yl]methoxy}pyridin-3-yl)-sulfonyl]-4-(4-{[2-(4-chlorophenyl)-4,4-dimethylcyclohex-1-en-1-yl]methyl}-piperazin-1-yl)-2-(1H-pyrrolo[2,3-b]pyridin-5-yloxy)benzamide;
   N-({5-bromo-6-[(1-tetrahydro-2H-pyran-4-ylpiperidin-4-yl)umino]pyridin-3-yl}-sulfonyl)-4-(4-[2-(4-chlorophenyl)-4,4-dimethylcyclohex-1-en-1-yl]methyl}-piperazin-1-yl)-2-(1H-pyrrolo[2,3-b]pyridin-5-yloxy)benzamide_{;}
   4-(4-{[2-(4-chlorophenyl)-5-methoxy-5-methylcyclohex-1-en-1-yl]methyl} piperazin-1-yl)-N-({3-nitro-4-[(tetrahydro-2H-pyran-4-ylmethyl)amino]phenyl}-sulfonyl)-2-(1H-pyrrolo[2,3-b]pyridin-5-yloxy)benzimde;
   4-(4-{[2-(4-chlorophenyl)-4,4-dimethylcyclohex-1-en-1-yl]methyl} piperazin-1-yl)-N-[(4-{[(3R)-1-(methylsulfonyl)pyrrolidin-3-yl]amino}-3-nitrophenyl)-sulfonyl]-2-(1)-]-pyrrolo[2,3-b]pyridin-5-yloxy)benzamide;
   4-(4-{[2-(4-chlorophenyl)-4,4-dimethylcyclohex-1-en-1-yl]methyl]piperazin-1-yl)-N-[(4-{[(*trans*-4-hydroxy-4-methylcyclohexyl)methyl]amino}-3-nitrophenyl)-suffonyl]-2-(1H-pyrrolo[2,3-b]pyridin-5-yloxy)benzamide;
   4-(4-{[2-(4-chlorophenyl)-4,4-dimethylcyclohex-1-en-1-yl]methyl}prperazin-1-yl)-N-[(4-([(*cis*-4-hydroxy-4-methylcyclohexyl)methyl]amino}-3-nitrophenyl)-sulfonyl]-2-(1H-pyrrolo[2,3-b]pyridin-5-yloxy)benzamide;
   4-(4-{[2-(4-chlorophenyl)-4,4-dimethylcyclohex-1-en-1-yl]methyl}piperazin-1-yl)-N-{[4-({3-[cyclopropyl(oxetan-3-yl)amino]propyl}amino)-3-nitrophenyl]-sulfonyl}-2-(1H-pyrrolo[2,3-b]pyridin-5-yloxy)benzamide;
   4-(4-{[2-(4-chlorophenyl)-4,4-dimethylcyclohex-1-en-1-yl)methyl}piperazin-1-yl)-N-{[3-nitro-4-({[(3R)-1-tetrahydro-2H-pyran-4-ylpyrrolidin-3-yl]methyl}-amino)phenyl]sulfonyl}-2-(1H-pyrrolo[2,3-b]pyridin-5-yloxy)benzamide;
   4-(4-{[2-(4-chlorophenyl)-4,4-dimethylcyclohex-1-en-1-yl-methyl} piperazin-1-yl)-N-[(4-{[(4-methylmorpholin-2-yl)methyl]amino}-3-nitrophenyl)sulfonyl]-2-(1H-pyrrolo[2,3-b]pyridin-5-yloxy)benzamide;
   N-{(5-chloro-6-{[1-(cyanomethyl)piperidin-4-ylmethoxy}pyridin-3-yl)sulfonyl]-4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)benzamide;
   N-[(4-{[(4-aminotetrahydro-2H-1-pyran-4-yl)methyl]amino}-3-nitrophenyl)sulfonyl]-4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)benzamide;
   4-(4-{[2-(4-chlorophenyl)-4,4-dimethylclohex-1-en-yl]methyl}piperazin-1-yl)-N-({4-[(4-methoxytetrahydro-2H-pyran-4-yl)methoxy]-3-nitrophenyl}sulfonyl)-2-(1H-pyrrolo[2,3-b]pyridin-5-yloxy)benzamide;
   and pharmaceutically acceptable salts thereof.
56. The method of item 46, wherein the compound is 4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide.

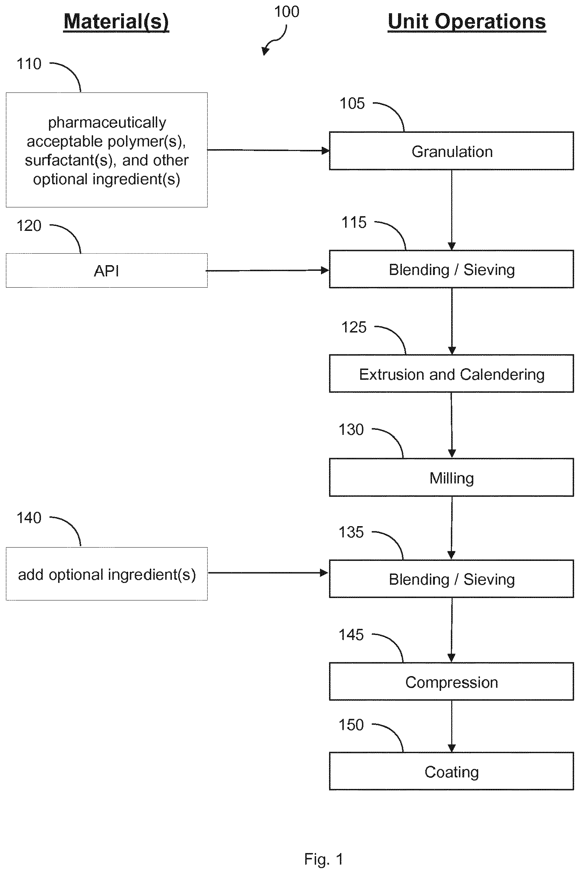

## Claims

1. A solid dispersion comprising, in essentially non-crystalline form such that no more than about 5% crystallinity is observed by X-ray diffraction analysis, a compound of Formula I 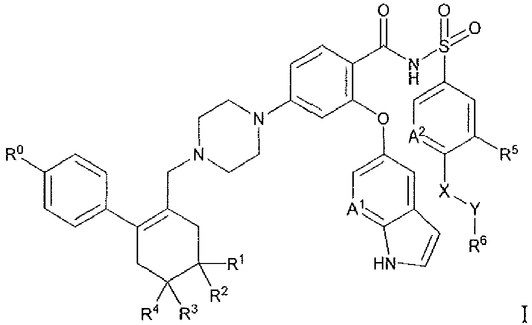 where:
R⁰ is halo;
R¹ and R² are H or are independently methyl or methoxy;
R³ and R⁴ are independently methyl or methoxy if R¹ and R² are H, or are H if R¹ and R² are independently methyl or methoxy;
A¹ and A² are each independently CH or N;
R⁵ is C₁₋₄ alkyl or haloalkyl, C₁₋₄ alkylsulfonyl or haloalkylsulfonyl, halo, nitro or cyano;
X is -O- or -NH-;
Y is -(CH₂)ₙ- where n is 0, 1, 2 or 3; and
R⁶ is an unsubstituted or substituted 3- to 7-membered carbocyclic or heterocyclic ring as defined herein, or is NR⁷R⁸;
wherein, if R⁶ is NR⁷R⁸, R⁷ and R⁸ are each independently H or R⁹ -(CH₂)ₘ- groups, no more than one of R⁷ and R⁸ being H, where each R⁹ is independently a 3- to 7-membered carbocyclic or heterocyclic ring, optionally substituted with no more than two Z¹ groups as defined below, and each m is independently 0 or 1; and
wherein, if R⁶ is a substituted carbocyclic or heterocyclic ring, substituents thereon are no more than two Z¹ groups and/or no more than one Z² group, Z¹ groups being independently selected from (a) C₁₋₄ alkyl, C₂₋₄ alkenyl, C₁₋₄ alkoxy, C₁₋₄ alkylthio, C₁₋₄ alkylamino, C₁₋₄ alkylsulfonyl, C₁₋₄ alkylsuifonylamitio, C₁₋₄ alkylcarbonyl, C₁₋₄ alkylcarbonylamino and C₁₋₄ alkylcarboxy, each optionally substituted with one or more substituents independently selected from halo, hydroxy, C₁₋₄ alkoxy, amino, C₁₋₄ alkylamino, di-(C₁₋₄ alkyl)amino and cyano, (b) halo, (e) hydroxy, (f) amino and (g) oxo groups, and Z² being (i) a further 3- to 6-membered carbocyclic or heterocyclic ring, optionally substituted with no more than two Z¹ groups as defined above, or (ii) NR⁷R⁸ where R⁷ and R⁸ are as defined above;
or a pharmaceutically acceptable salt thereof; wherein the compound of Formula I or the pharmaceutically acceptable salt thereof is dispersed in a solid matrix that comprises (a) at least one pharmaceutically acceptable water-soluble polymeric carrier and (b) at least one pharmaceutically acceptable surfactant.

2. The solid dispersion of Claim 1, wherein the compound is 4-(4-{[2-(4-chlorophenyl)-4,4-dimethylcyclohex-1-en-1-yl]methyl}piperazin-1-yl)-N-({4-[(1,4-dioxan-2-ylrnethyl)amino]-3-nitrophenyl}sulfonyl)-2-(1H-pyrrolo[2,3-b]pyridin-5-yloxy}benzamide, an enantiomer thereof, or a pharmaceutically acceptable salt thereof.

3. The solid dispersion of Claim 1, wherein, in the compound of Formula I, if R¹ is methyl or methoxy, R² is methyl, and R³ and R⁴ are each H, or if A² is -CH- then R³ is nitro; and if A² is -N- then R⁵ is bromo.

4. The solid dispersion of Claim 1, wherein, in the compound of Formula I, R⁶ is a 3- to 7-membered carbocyclic or heterocyclic ring, unsubstituted or substituted with no more than two Z¹ groups and/or no more than one Z² group.

5. The solid dispersion of Claim 1, wherein, in the compound of Formula I, R⁶ is selected from the group consisting of cyclopropyl, cyclobutyl, cyclopentyl, cyclohexyl, azetidinyl, oxetanyl, thietanyl, pyrrolidinyl, imazolidinyl, pyrazolidinyl, tetrahydrofuranyl, oxazolidinyl, isoxazolidinyl, thiophanyl, thiazolidinyl, isothiazolidinyl, piperidinyl, piperazinyl, tetrahydropyranyl, 1,4-dioxanyl, morpholinyl and tetrahydrothiopyranyl rings.

6. The solid dispersion of Claim 1, wherein the compound is selected from the group consisting of
4-(4-{[2-(4-chlorophenyl)-4,4-dimethylcyclohex-1-en-1-yl]methyl}piperazin-1-yl)-2-(1H-indol-5-yloxy)-N-({3-nitro-4-[(1-tetrahydro-2H-pyran-4-y]piperidin-4-yl)amino]phenyl}sulfonyl)benzamide;
4-(4-{[2-(4-chlorophenyl)-4,4-dimethylcyclohex-1-en-1-yl]methyl}piperazin-1-yl)-2-(1H-indo)-5-yloxy)-N-({4-[(4-methylpiperazin-1-yl)amino]-3-nitrophenyl}sulfonyl)benzamide;
*trans*-4-(4-{[2-(4-chlorophenyl)-4,4-dimethylcyclohex-1-en-1-yl]methyl}piperazin-1-yl)-N-({4-[(4-morpholin-4-ylcyclohexyl)amino]-3-nitrophenyl}sulfonyl)-2-(1H-pyrrolo[2,3-b]pyridin-5-yloxy)benzamide;
*cis*-4-(4-{[2-(4-chlorophenyl)-4,4-dimethylcyclohex-1-en-1-yl]methyl}piperazin-1-yl)-N-[(4-{[(4-methoxycyclohexyl)methyl]amino}-3-nitrophenyl}sulfonyl]-2-(1H-pyrrolo[2,3-b]pyridin-5-yloxy)benzamide;
*trans-*4-(4-{[2-(4-chtorophenyl)-4,4-dimethylcyclohex-1-en-1-yl]methyl}piperazin-1-yl)-N-[(4-{[(4-methoxycyclohexyl)methyl]amino}-3-nitrophenyl)sulfonyl]-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-({4-[(4-fluorotetrahydro-2H-pyran-4-yl)methoxy]-3-nitrophenyl}sulfonyl)-2-(1H-pyrrolo[2,3-b]pyridin-5-yloxy)benzamide;
N-[(5-chloro-6-{[4-fluoro-1-(oxetan-3-yl)piperidin-4-yl]methoxy}pyridin-3-yl)-sulfonyl]-4-(4-{[2-(4-chlorophenyl)-4,4-dimethy)cyclohex-1-en-1-yl]methyl}-piperazin-1-yl)-2-(1H-pyrrolo[2,3-b]pyridin-5-yloxy)benzamide;
N-({5-bromo-6-[(1-tetrahydro-2H-pyran-4-ylpiperidin-4-yl)amino]pyridin-3-yl}-sulfonyl)-4-(4-{[2-(4-chlorophenyl)-4,4-dimethylcyclohex-1-en-1-yl]methyl}-piperazin-1-yl)-2-(1H-pyrrolo[2,3-b]piridin-5-yloxy)benzamide;
4-(4-{[2-(4-chlorophenyl)-5-methoxy-5-methylcyclohex-1-en-1-yl]methyl}piperazin-1-yl)-N-({3-nitro-4-[(tetrahydro-2H-pyran-4-ylmethyl)amino]phenyl)-sulfonyl)-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-[(4-{[(3R)-1-(methylsulfonyl)pyrrolidin-3-yl]amino}-3-nitrophenyl)-sulfonyl]-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-{[4-({3-[cyclopropyl(oxetan-3-yl)amino]propyl)amino)-3-nitrophenyl]-sulfonyl}-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-{[3-nitro-4-(([(3R)-1-tetrahydro-2H-pyran-4-ylpyrrolidin-3-yl]methyl}-amino)phenyl]sulfonyl}-2-(1H-pyrrolo[2,3-b]pyridin-5-yloxy)benzamide;
4-(4-{[2-(4-chlorophenyl)-4,4-dimethylcyclohex-1-en-1-yl)methyl}piperazin-1-yl)-N-[(4-{[(4-methylmorpholin-2-yl)methyl]amino}-3-nitrophenyl)sulfonyl]-2-(1H-pyrrolo[2,3-b]pyridin-5-yloxy)benzamide;
N-[(5-chloro-6-{[1-(cyanomethyl)piperidin-4-yl]methoxy}pyridin-3-yl)sulfonyl]-4-(4-{[2-(4-chlorophenyl)-4,4-dimethylcyclohex-1-en-1-yl]methyl)piperazin-1-yl)-2-(1H-pyrrolo[2,3-b]pyridin-5-yloxy)benzamide;
N-[(4-{[(4-aminotetrahydro-2H-pyran-1-yl)methyl]amino}-3-1-nitrophenyl)sulfonyl]-4-(4-{[2-(4-chlorophenyl)-4,4-dimethylcyclohex-1-en-1-yl]methyl}piperazin-]-yl)-2-(1H-pyrrolo[2,3-b]pyridin-5-yloxy)benzamide;
4-(4-{[2-(4-chlorophenyl)-4,4-dimethy)cyclohex-1-en-1-yl]methyl}piperazin-1-yl)-N-({4-[(4-methoxytetrahydro-2H-pyran-4-yl)methoxy]-3-nitrophenyl)sulfonyl)-2-(1H-pyrrolo[2,3-b]pyridin-5-yloxy)benzamide;
and pharmaceutically acceptable salts thereof.

7. The solid dispersion of Claim 1, wherein the at least one polymeric carrier is selected from the group consisting of homopolymers and copolymers of N-vinyl lactams, cellulose esters, cellulose ethers, high molecular weight polyalkylene oxides, polyacrylates, polymethacrylates, polyacrylamides, vinyl acetate polymers, graft copolymers of polyethylene glycol, polyvinyl caprolactam and polyvinyl acetate, oligo- and polysaccharides and mixtures thereof.

8. The solid dispersion of Claim 1, wherein the at least one polymeric carrier is selected from the group consisting of povidones, copovidones, HPMCs, polyethylene glycol/polyvinyl caprolactam/polyvinyl acetate graft copolymers and mixtures thereof.

9. The solid dispersion of Claim 1, wherein the at least one surfactant is selected from the group consisting of polyoxyethylene glycerides, fatty acid monoesters of sorbitan, polysorbates, α-tocopheryl polyethylene glycol succinate (TPGS) and mixtures thereof.

10. The solid dispersion of Claim 1, further comprising colloidal silicon dioxide.

11. The solid dispersion of Claim 1, wherein the compound or salt is present in a parent-compound-equivalent amount of about 5% to about 40% by weight, the at least one polymeric carrier is present in an amount of about 40% to about 85% by weight and the at least one surfactant is present in an amount of about 5% to about 20% by weight.

12. The solid dispersion of Claim 11, wherein the compound or salt is present in a parent-compound-equivalent amount of about 5% to about 15% by weight, the at least one polymeric carrier is present in an amount of about 70% to about 85% by weight and the at least one surfactant is present in an amount of about 5% to about 15% by weight.

13. The solid dispersion of Claim 12, wherein the compound is 4-(4-{[2-(4-chlorophenyl)-4,4-dimethylcyclohex-1-en-1-yl]methyl}piperazin-1-yl)-N-({4-[(1,4-dioxan-2-ylmethyl)amino]-3-nitrophenyl}sulfonyl)-2-(1H-pyrrolo[2,3-b]pyridin-5-yloxy)benzamide, an enantiomer thereof, or a pharmaceutically acceptable salt thereof.

14. A process for preparing a solid dispersion, comprising:
(a) subjecting to elevated temperature (i) an active pharmaceutical ingredient (API) that comprises a compound of Formula I 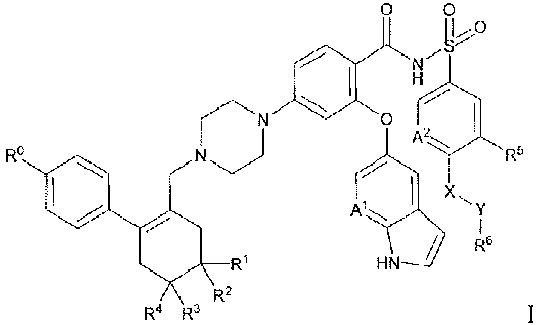 where:
R⁰ is halo;
R¹ and R² are H or are independently methyl or methoxy;
R³ and R⁴ are independently methyl or methoxy if R¹ and R² are H, or are H if R¹ and R² are independently methyl or methoxy;
A¹ and A² are each independently CH or N;
R⁵ is C₁₋₄ alkyl or haloalkyl, C₁₋₄ alkylsulfonyl or haloalkylsulfonyl, halo, nitro or cyano;
X is -O- or -NH-;
Y is -(CH₂)ₙ- where n is 0, 1, 2 or 3; and
R⁶ is an unsubstituted or substituted 3- to 7-membered carbocyclic or heterocyclic ring as defined herein, or is NR⁷R⁸;
wherein, if R⁶ is NR⁷R⁸, R⁷ and R⁸ arc each independently H or R⁹-(CH₂)ₘ₋ groups, no more than one of R⁷ and R⁸ being H, where each R⁹ is independently a 3- to 7-membered carbocyclic or heterocyclic ring, optionally substituted with no more than two Z¹ groups as defined below, and each m is independently 0 or 1; and
wherein, if R⁶ is a substituted carbocyclic or heterocyclic ring, substituents thereon are no more than two Z¹ groups and/or no more than one Z² group, Z¹ groups being independently selected from (a) C₁₋₄ alkyl, C₂₋₄ alkenyl, C₁₋₄ alkoxy, C₁₋₄ alkylthio, C₁₋₄ alkylamino, C₁₋₄ alkylsulfonyl, C₁₋₄ alkylsulfonylamino, C₁₋₄ alkylcarbonyl, C₁₋₄ alkylcarbonylamino and C₁₋₄ alkylcarboxy, each optionally substituted with one or more substituents independently selected from halo, hydroxy, C₁₋₄ alkoxy, amino, C₁₋₄ alkylamino, di-(C₁₋₄ alkyl)amino and cyano, (b) halo, (c) hydroxy, (f) amino and (g) oxo groups, and Z² being (i) a further 3- to 6-membered carbocyclic or heterocyclic ring, optionally substituted with no more than two Z¹ groups as defined above, or (ii) NR⁷R⁸ where R⁷ and R⁸ are as defined above;
or a pharmaceutically acceptable salt thereof, (ii) a pharmaceutically acceptable water-soluble polymeric carrier and (iii) a pharmaceutically acceptable surfactant to provide an extrudable semi-solid mixture;
(b) extruding the semi-solid mixture; and
(c) cooling the resulting extrudate to provide a solid matrix comprising the polymeric carrier and the surfactant and having the compound or salt thereof dispersed in an essentially non-crystalline form therein such that no more than about 5% crystallinity is observed by X-ray diffraction analysis.

15. The process of Claim 14, wherein the API, polymeric carrier and surfactant are mixed together before said subjecting to elevated temperature.

16. The process of Claim 14, wherein the API, polymeric carrier and surfactant are mixed together while subjecting to elevated temperature.

17. The process of Claim 14, wherein said elevated temperature is about 70°C to about 250°C, or is about 90°C to about 160°C.

18. The process of Claim 14, further comprising calendering the extrudate before or while cooling.

19. The process of Claim 14, wherein API is 4-(4-{[2-(4-chlorophenyl)-4,4-dimethylcyclohex-1-en-1-yl]methyl}piperazin-1-yl)-N-({4-[(1,4-dioxan-2-ylmethyl)amino]-3-nitrophenyl}sulfonyl)-2-(1H-pyrrolo[2,3-b]pyridin-5-yloxy)benzamide, an enantiomer thereof, or a pharmaceutically acceptable salt thereof.

20. The process of claim 14, wherein step (b) comprises extruding and calendering the semi-solid mixture.

21. An orally deliverable pharmaceutical dosage form comprising the solid dispersion of Claim 1.

22. The solid dispersion of claim 1 for use as a medicament.

23. The solid dispersion of Claim 22, for use in treating a neoplastic disease or an immune or autoimmune disease.

24. The solid dispersion of Claim 23, wherein the neoplastic disease is selected from the group consisting of cancer, mesothelioma, bladder cancer, pancreatic cancer, skin cancer, cancer of the head or neck, cutaneous or intraocular melanoma, ovarian cancer, breast cancer, uterine cancer, carcinoma of the fallopian tubes, carcinoma of the endometrium, carcinoma of the cervix, carcinoma of the vagina, carcinoma of the vulva, bone cancer, colon cancer, rectal cancer, cancer of the anal region, stomach cancer, gastrointestinal (gastric, colorectal and/or duodenal) cancer, chronic lymphocytic leukemia, acute lymphocytic leukemia, esophageal cancer, cancer of the small intestine, cancer of the endocrine system, cancer of the thyroid gland, cancer of the parathyroid gland, cancer of the adrenal gland, sarcoma of soft tissue, cancer of the urethra, cancer of the penis, testicular cancer, hepatocellular (hepatic and/or biliary duct) cancer, primary or secondary central nervous system tumor, primary or secondary brain tumor, Hodgkin's disease, chronic or acute leukemia, chronic myeloid leukemia, lymphocytic lymphoma, lymphoblastic leukemia, follicular lymphoma, lymphoid malignancies of T-cell or B-cell origin, melanoma, multiple myeloma, oral cancer, non-small-cell lung cancer, prostate cancer, small-cell lung cancer, cancer of the kidney and/or ureter, renal cell carcinoma, carcinoma of the renal pelvis, neoplasms of the central nervous system, primary central nervous system lymphoma, non-Hodgkin's lymphoma, spinal axis tumors, brain stem glioma, pituitary adenoma, adrenocortical cancer, gall bladder cancer, cancer of the spleen, cholangiocarcinoma, fibrosarcoma, neuroblastoma, retinoblastoma and combinations thereof.

25. The solid dispersion of Claim 22, wherein the neoplastic disease is non-Hodgkin's lymphoma, chronic lymphocytic leukemia, or acute lymphocytic leukemia.

26. The solid dispersion of Claim 22, wherein the solid dispersion is administered in a parent-compound-equivalent dose of about 50 to about 500 mg per day of the compound of Formula I or salt thereof at an average treatment interval of about 3 hours to about 7 days.

27. The solid dispersion of Claim 22, wherein the compound is selected from the group consisting of
4-(4-{[2-(4-chlorophenyl)-4,4-dimethylcyclohex-1-en-1-yl]methyl}piperazin-1-yl)-2-(1H-indol-5-yloxy)-N-({3-nitro-4-[(1-tetrahydro-2H-pyran-4-ylpiperidin-4-yl)amino]phenyl}sulfonyl)benzamide;
4-(4-{[2-(4-chlorophenyl)4,4-dimethylcyclohex-1-en-1-yl]methyl}piperazin-1-yl)-2-(1H-indol-5-yloxy)-N-({4-[(4-methylpiperazin-1-yl)amino]-3-nitrophenyl}sulfonyl)benzamide;
*trans-*4-(4-{[2-(4-chlorophenyl)-4,4-dimethylcyclohex-1-en-1-yl]methyl}piperazin-1-yl)-N-({4-[(4-morpholin-4-ylcyclohexyl)amino]-3-nitrophenyl}sulfonyl)-2-(1H-pyrrolo[2,3-b]pyridin-5-yloxy)benzamide;
*cis*-4-(4-{[2-(4-chlorophenyl)-4,4-dimethylcyclohex-1-en-1-yl]methyl}piperazin-1-yl)-N-[(4-{[(4-methoxycyclohexyl)methyl]amino}-3-nitrophenyl)sulfonyl]-2-(1H-pyrrolo[2,3-b]pyridin-5-yloxy)benzamide;
*trans*-4-(4-{[2-(4-chlorophenyl)-4,4-dimethylcyclohex-1-en-1-yl]methyl}piperazin-1-yl)-N-[{4-{[{4-methoxycyclohexyl)methyl]amino}-3-nitrophenyl)sulfonyl]-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-({4-[(4-fluorotetrahydro-2H-pyran-4-yl)methoxy]-3-nitrophenyl}sulfonyl)-2-(1H-pyrrolo[2,3-b]pyridin-5-yloxy)benzamide;
N-[(5-chloro-6-{[4-fluoro-1-(oxetan-3-yl)piperidin-4-yl]methoxy}pyridin-3-yl)-sulfonyl]-4-(4-{[2-(4-chlorophenyl)-4,4-dimethylcyclohex-1-en-1-yl]methyl}-piperazin-1-yl)-2-(1H-pyrrolo[2,3-b]pyridin-5-yloxy)benzamide;
N-({5-bromo-6-[(1-tetrahydro-2H-pyran-4-ylpiperidin-4-yl)amino]pyridin-3-yl}-sulfonyl)-4-(4-{[2-(4-chlorophenyl)-4,4-dimethylcyclohex-1-en-1-yl]methyl}-piperazin-1-yl)-2-(1H-pyrrolo[2,3-b]pyridin-5-yloxy)benzamide;
4-(4-{[2-(4-chlorophenyl)-5-methoxy-5-methylcyclohex-1-en-1-yl]methyl}piperazin-1-yl)-N-({3-nitro-4-[(tetrahydro-2H-pyran-4-ylmethyl)amino]phenyl}-sulfonyl)-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-[(4-{[(3R)-1-(methylsulfonyl)pyrrolidin-3-yl]amino}-3-)nitrophenyl)-sulfonyl]-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-{[4-({3-[cyclopropyl(oxetan-3-yl)amino]propyl}amino)-3-nitrophenyl]-sulfonyl}-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-{[3-nitro-4-({[(3R)-1-tetrahydro-2H-pyran-4-ylpyrrolidin-3-yl]methyl}-amino)phenyl]sulfonyl}-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-[(4-{[(4-methylmorpholin-2-yl)methyl]amino}-3-nitrophenyl)sulfonyl]-2-(1H-pyrrolo[2,3-b]pyridin-5-yloxy)benzamide;
N-[(5-chloro-6-{[1-(cyanomethyl)piperidin-4-yl]methoxy}pyridin-3-yl)sulfonyl]-4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)benzamide;
N-[(4-{[(4-aminotetrahydro-2H-pyran-4-yl)methyl]amino}-3-nitrophenyl)sulfonyl]-4-(4-{[2-(4-chlorophenyl)-4,4-dimethylcyclohex-1-en-1-yl]methyl}piperazin-1-yl)-2-(1H-pyrrolo[2,3-b]pyridin-5-yloxy)benzamide;
4-(4-{[2-(4-chlorophenyl)-4,4-dimethylcyclohex-1-en-1-yl]methyl}piperazin-1-yl)-N-({4-[(4-methoxytetrahydro-2H-pyran-4-yl)methoxy]-3-nitrophenyl}sulfonyl)-2-(1H-pyrrolo [2,3-b]pyridin-5-yloxy)benzamide;
and pharmaceutically acceptable salts thereof.
